# EUROPEAN PATENT APPLICATION

(11) **EP 4 840 855 A1**
(43) Date of publication of application: **18.11.2026**
(21) Application number: 25738490.9
(22) Date of filing: 09.01.2025
(51) Int. Cl.: C07D 401/14, C07D 403/12, C07D 405/14, C07D 407/14, C07D 417/14, C07D 409/14, A61K 31/50, A61K 31/4439, A61K 31/506, A61K 31/497, A61P 35/00

(54) **SULFONAMIDE DERIVATIVE, PREPARATION METHOD THEREFOR AND USE THEREOF**

(30) Priority: 11.01.2024 CN 202410040971; 03.04.2024 CN 202410398716; 14.05.2024 CN 202410595688; 10.07.2024 CN 202410919296; 16.08.2024 CN 202411127098; 30.10.2024 CN 202411526533
(71) Applicant: Zhejiang Hisun Pharmaceutical Co., Ltd., Taizhou, Zhejiang 318000 (CN); Shanghai Aryl Pharmtech Co., Ltd., Songjiang, Shanghai 201612 (CN)
(72) Inventor: HUANG, Xiangui, Shanghai 201612 (CN); YAN, Qingyan, Shanghai 201612 (CN); LIAO, Weiwei, Shanghai 201612 (CN); YU, Mingfeng, Shanghai 201612 (CN); ZHOU, Houjiang, Taizhou, Zhejiang 318000 (CN)
(74) Representative: Boult Wade Tennant LLP
(86) International application number: PCT/CN2025/071438
(87) International publication number: WO 2025/148961

(57) **Abstract**

The present invention relates to a sulfonamide derivative, a preparation method therefor, and a medical use of a pharmaceutical composition containing the derivative. Specifically, the present invention relates to a sulfonamide derivative represented by general formula (AAI), a preparation method therefor, and a pharmaceutically acceptable salt thereof, as well as a use thereof as a therapeutic agent, particularly as a CTPS1 inhibitor, the definitions of the substituents in the general formula (AAI) being the same as the definitions in the description.

## Description

### FIELD

The present disclosure relates to a sulfonamide derivative, a preparation method therefor, a pharmaceutical composition containing the derivative, and use thereof as a therapeutic agent, particularly as a CTPS1 inhibitor.

### BACKGROUND

Cytidine triphosphate (CTP) is an important precursor required for DNA, RNA, and phospholipid metabolism in human cells. CTP is produced through two pathways: a salvage synthesis pathway and a *de novo* synthesis pathway. CTP synthase (CTPS) is a rate-limiting enzyme for the *de novo* synthesis of CTP, and includes two subtypes: CTPS1 and CTPS2. CTPS1 and CTPS2 have 74% sequence homology but have different physiological roles. CTPS2 is uniformly expressed in various tissues, whereas CTPS1 is generally expressed at low levels in most tissues but is rapidly upregulated in activated T cells. By expressing wild-type CTPS1 or adding exogenous CTP or its nucleotide precursor cytidine, T cell proliferation is restored in CTPS1-deficient cells. It is indicated that CTPS1 plays an essential role in the proliferation of B cells and T cells and is necessary for an effective immune response.

Proliferating cells, such as activated lymphocytes, have a high demand for ribonucleotides, particularly CTP and GTP. CTPS1 is highly expressed after lymphocyte activation and catalyzes the synthesis of CTP, providing a material basis for lymphocyte proliferation. Meanwhile, CTPS1 has reduced sensitivity to CTP feedback inhibition compared to CTPS2, which confers on CTPS1 the ability to continuously catalyze CTP synthesis, ultimately expanding the CTP reserve required during lymphocyte proliferation. CTPS1 is upregulated in activated lymphocytes to expand the CTP pool, meeting the increased demand for nucleic acid and lipid synthesis. The demand for CTP in other tissues is met through the CTPS2 subtype and nucleotide salvage pathways. Therefore, selective inhibition of proliferative CTPS1 is desirable in the treatment of immune diseases and lymphocyte cancers, which makes CTPS1 an ideal target for immunosuppression.

Lymphoma is one of the most common malignancies in China, and is mainly divided into two categories: Hodgkin Lymphoma (HL) and non-Hodgkin Lymphoma (NHL). In actual clinical diagnosis, NHL is more common, with about 80% to 90% of diagnosed lymphomas being NHL. NHL mainly includes diffuse large B-cell lymphoma, peripheral T-cell lymphoma, mantle cell lymphoma, cutaneous T-cell lymphoma, indolent B-cell lymphoma, and NK cell lymphoma. In T-cell non-Hodgkin lymphoma and acute T-cell lymphoma, CTPS1 expression levels are relatively high, and CTPS1 overexpression provides a material basis for the excessive proliferation of these T-cell lymphomas, thus serving as a target for anticancer drugs.

To date, no novel CTPS1-targeting inhibitors have been marketed. Currently, STP-938 developed by Step Pharma has entered Phase I/II clinical studies for the treatment of relapsed/refractory B-cell and T-cell lymphomas, and a small molecule CTPS1 inhibitor developed by Nimbus is in the discovery stage. Overall, as a relatively cutting-edge research direction, there is still enormous room for exploration in CTPS1-related research, and it is highly necessary to continue developing new inhibitors.

### SUMMARY

In view of the foregoing, the present disclosure provides a sulfonamide derivative represented by formula (AAI): 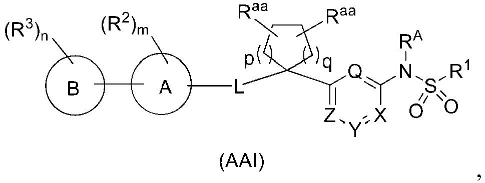
or a stereoisomer, a tautomer, or a pharmaceutically acceptable salt thereof;
wherein:
   any two R^{aa}, together with the atoms to which they are attached, form a ring C, wherein ring C is optionally further substituted by one or more R⁴;
   ring C is selected from the group consisting of a 6-membered aryl, a 3- to 5- membered cycloalkyl, a 5- to 7- membered heterocyclyl, and a 5- to 6- membered heteroaryl;
   R^{A} is selected from the group consisting of a hydrogen atom and a C₁₋₆ alkyl;
   ring A is selected from the group consisting of a 6-membered aryl, a 5- to 6- membered heteroaryl, and a 5- to 7- membered heterocyclyl;
   ring B is selected from the group consisting of a 6-membered aryl and a 5- to 10-membered heteroaryl;
   L is selected from the group consisting of -C(=O)-, -C(=O)NR^{a}-, and -NR^{b}C(=O)-;
   R^{a} and R^{b} are each independently selected from the group consisting of a hydrogen atom and a C₁₋₆ alkyl;
   X, Y, Z, and Q are each independently selected from the group consisting of N and CR^{c}; and at most two of X, Y, Z, and Q are simultaneously N atoms;
   alternatively, X and Y, Z and Y each independently form a 5- to 6- membered heteroaryl or a 5- to 6- membered heterocyclyl; wherein the 5- to 6- membered heteroaryl or 5- to 6-membered heterocyclyl is optionally further substituted by one or more substituents selected from the group consisting of halogen, hydroxy, cyano, and 3- to 5- membered cycloalkyl;
   R^{c} is selected from the group consisting of a hydrogen atom, halogen, hydroxy, cyano, C₁₋₆ alkyl, and C₁₋₆ alkoxy; wherein the C₁₋₆ alkyl or C₁₋₆ alkoxy is optionally further substituted by one or more substituents selected from the group consisting of halogen, hydroxy, cyano, and 3- to 5- membered cycloalkyl;
   R¹ is selected from the group consisting of C₁₋₆ alkyl and 3- to 5- membered cycloalkyl; wherein the C₁₋₆ alkyl or 3- to 5- membered cycloalkyl is optionally further substituted by one or more substituents selected from the group consisting of halogen, hydroxy, cyano, 3- to 5-membered cycloalkyl, C₁₋₆ alkyl, C₁₋₆ haloalkyl, C₁₋₆ alkoxy, and C₁₋₆ haloalkoxy;
   each R² is independently selected from the group consisting of a hydrogen atom, halogen, cyano, hydroxy, C₁₋₆ alkyl, and C₁₋₆ alkoxy; wherein the C₁₋₆ alkyl or C₁₋₆ alkoxy is optionally further substituted by one or more substituents selected from the group consisting of halogen, hydroxy, cyano, C₁₋₆ alkyl, and C₁₋₆ alkoxy;
   alternatively, two R², together with the same carbon atom to which they are attached, form a -C(O);
   R³ is each independently selected from the group consisting of a hydrogen atom, halogen, cyano, hydroxy, C₁₋₆ alkyl, C₁₋₆ alkoxy, 3- to 5- membered cycloalkyl, C₂₋₆ alkenyl, SF₅, -C(O)R⁵, -C(O)OR⁵, -NHC(O)R⁵, -NHC(O)OR⁵, -NR⁶R⁷, -C(O)NR⁶R⁷, -CH₂NHC(O)OR⁵, -CH₂NR⁶R⁷, and -S(O)ᵣR⁵; wherein the alkyl, alkoxy, alkenyl, or cycloalkyl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
   R⁴ is each independently selected from the group consisting of a hydrogen atom, cyano, halogen, alkyl, alkenyl, alkynyl, hydroxy, cycloalkyl, heterocyclyl, aryl, heteroaryl, -OR⁵, -C(O)R⁵, -C(O)OR⁵, -NHC(O)R⁵, -NHC(O)OR⁵, -NR⁶R⁷, -C(O)NR⁶R⁷, -S(O)₂NR⁶R⁷, -CH₂NHC(O)OR⁵, -CH₂NR⁶R⁷, and -S(O)ᵣR⁵; wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
   R⁵ is each independently selected from the group consisting of a hydrogen atom, alkyl, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
   R⁶ and R⁷ are each independently selected from the group consisting of a hydrogen atom, hydroxy, alkyl, alkoxy, cycloalkyl, heterocyclyl, aryl, and heteroaryl, wherein the alkyl, alkoxy, cycloalkyl, heterocyclyl, aryl, or heteroaryl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
   alternatively, R⁶ and R⁷, together with the atoms to which they are attached, form a 4- to 8- membered heterocyclyl, wherein the 4- to 8- membered heterocyclyl contains one or more N, O, or S(O)r, and the 4- to 8- membered heterocyclyl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
   R⁸, R⁹, and R¹⁰ are each independently selected from the group consisting of a hydrogen atom, alkyl, amino, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, amino, cyano, alkyl, alkoxy, cycloalkyl, heterocyclyl, aryl, heteroaryl, carboxy, and carboxylate;
   m is selected from the group consisting of 0, 1, and 2;
   n is selected from the group consisting of 0, 1, and 2;
   p is selected from the group consisting of 0, 1, and 2;
   q is selected from the group consisting of 0, 1, and 2; and
   r is each independently 0, 1, or 2.

In a preferred embodiment, the present disclosure provides a compound represented by formula (AAI), or a stereoisomer, a tautomer, or a pharmaceutically acceptable salt thereof, wherein 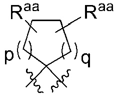 is selected from the group consisting of 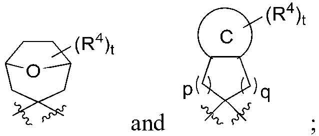 wherein t is selected from the group consisting of 0, 1, and 2; and ring C, R⁴, p, or q are as defined in formula (AAI).

In a preferred embodiment, the present disclosure provides a compound represented by formula (AAI), or a stereoisomer, a tautomer, or a pharmaceutically acceptable salt thereof, which is a compound represented by formula (AI): 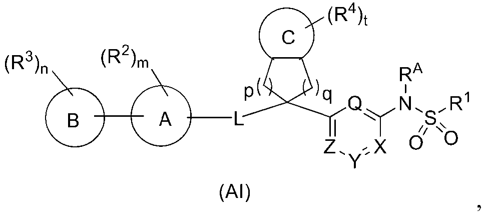
or a stereoisomer, a tautomer, or a pharmaceutically acceptable salt thereof;
wherein:
   X, Y, Z, and Q are each independently selected from the group consisting of N and CR^{c}; and at most two of X, Y, Z, and Q are simultaneously N atoms;
   alternatively, Z and Y form a 5- to 6- membered heteroaryl, wherein the 5- to 6-membered heteroaryl is optionally further substituted by one or more substituents selected from the group consisting of halogen, hydroxy, cyano, and 3- to 5- membered cycloalkyl;
   alternatively, X and Y form a 5- to 6- membered heteroaryl, wherein the 5- to 6-membered heteroaryl is optionally further substituted by one or more substituents selected from the group consisting of halogen, hydroxy, cyano, and 3- to 5- membered cycloalkyl;
   t is selected from the group consisting of 0, 1, and 2; and
   ring A, ring B, ring C, L, R¹ to R⁴, R^{A}, p, q, m, and n are as defined in formula (AAI).

In a preferred embodiment, the present disclosure provides a compound of formula (AAI) or (AI), or a stereoisomer, a tautomer, or a pharmaceutically acceptable salt thereof, which is a compound of formula (I): 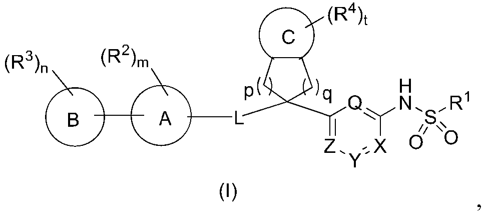
or a stereoisomer, a tautomer, or a pharmaceutically acceptable salt thereof;
wherein: ring A, ring B, ring C, L, R¹ to R⁴, X, Y, Z, Q, p, q, m, n, and t are as defined in formula (AI).

In a preferred embodiment, the present disclosure provides a compound of formula (AAI), (AI), or (I), or a stereoisomer, a tautomer, or a pharmaceutically acceptable salt thereof, wherein ring A is selected from the group consisting of: 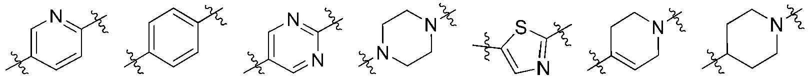 and

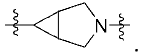

In a preferred embodiment, the present disclosure provides a compound of formula (AAI), (AI), or (I), or a stereoisomer, a tautomer, or a pharmaceutically acceptable salt thereof, wherein ring B is selected from the group consisting of:

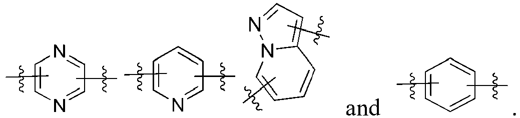

In a preferred embodiment, the present disclosure provides a compound of formula (AAI), (AI), or (I), or a stereoisomer, a tautomer, or a pharmaceutically acceptable salt thereof, wherein: L is selected from the group consisting of -C(=O)-, -C(=O)NH-, and -NHC(=O)-.

In a preferred embodiment, the present disclosure provides a compound of formula (AAI), (AI), or (I), or a stereoisomer, a tautomer, or a pharmaceutically acceptable salt thereof, which is a compound of formula (IIA) or (IIB): 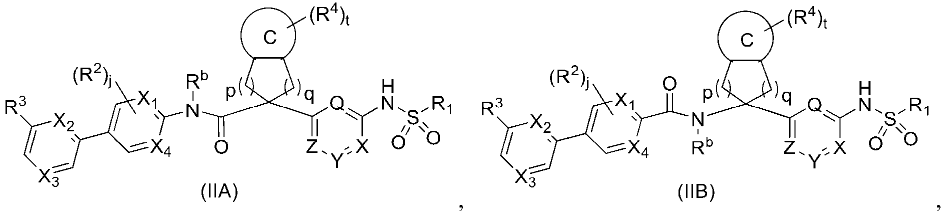
or a stereoisomer, a tautomer, or a pharmaceutically acceptable salt thereof;
wherein: each X₁ is independently selected from the group consisting of N and CR^{d};
X₄ is each independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy; and
ring C, X, Y, Z, Q, R¹ to R⁴, p, q, and t are as defined in formula (AI).

In a preferred embodiment, the present disclosure provides a compound of formula (AAI), (AI), or (I), or a stereoisomer, a tautomer, or a pharmaceutically acceptable salt thereof, which is a compound of formula (II) or (III): 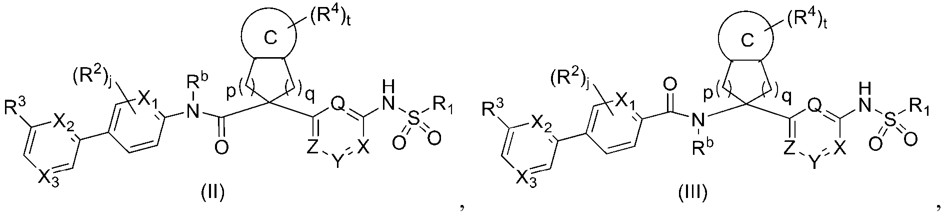
or a stereoisomer, a tautomer, or a pharmaceutically acceptable salt thereof;
wherein: each X₁ is independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy; and
ring C, X, Y, Z, Q, R¹ to R⁴, p, q, and t are as defined in formula (AI).

In a preferred embodiment, the present disclosure provides a compound of formula (AAI), or a stereoisomer, a tautomer, or a pharmaceutically acceptable salt thereof, which is a compound of formula (IV-1) or (IV-2): 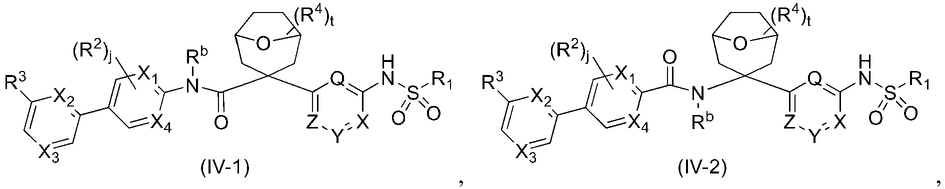
or a stereoisomer, a tautomer, or a pharmaceutically acceptable salt thereof
wherein: each X₁ is independently selected from the group consisting of N and CR^{d};
X₄ is each independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
t is selected from the group consisting of 0, 1, and 2; and
X, Y, Z, Q, and R¹ to R⁴ are as defined in formula (AAI).

In a preferred embodiment, the present disclosure provides a compound of formula (AAI), (AI), or (I), or a stereoisomer, a tautomer, or a pharmaceutically acceptable salt thereof which is a compound of formula (V-A), (V-B), (V-C), or (V-D): 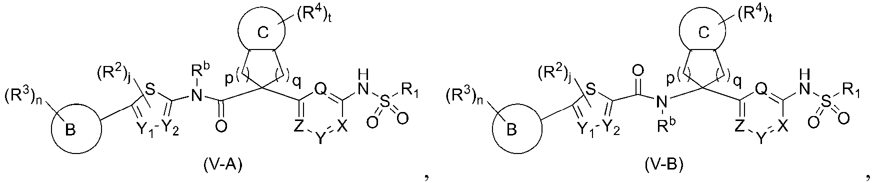 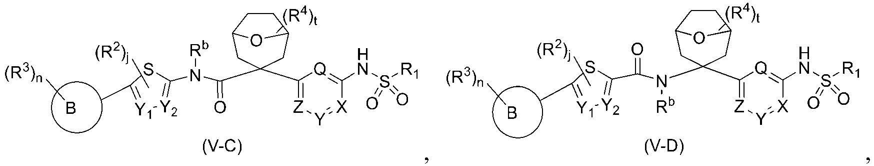
or a stereoisomer, a tautomer, or a pharmaceutically acceptable salt thereof;
wherein: each R^{b} is independently selected from the group consisting of a hydrogen atom and methyl;
ring B is each independently selected from the group consisting of 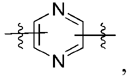 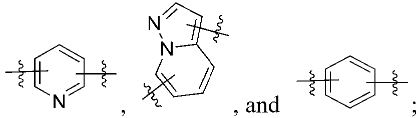
Y₁ and Y₂ are each independently selected from the group consisting of N and CH;
j is each independently selected from the group consisting of 0 and 1;
t is each independently selected from the group consisting of 0, 1, and 2; and
ring C, X, Y, Z, Q, R¹ to R⁴, p, q, and n are as defined in formula (AAI).

In a preferred embodiment, the present disclosure provides a compound of formula (AAI), (AI), (I), (V-A), (V-B), (V-C), or (V-D), or a stereoisomer, a tautomer, or a pharmaceutically acceptable salt thereof, which is a compound of formula (V-1) or (V-2): 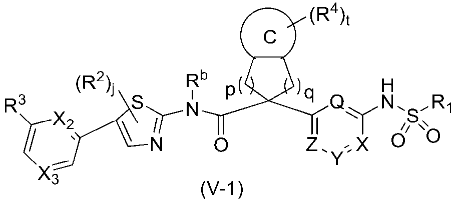 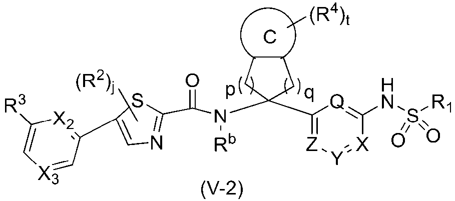
or a stereoisomer, a tautomer, or a pharmaceutically acceptable salt thereof
wherein: each R^{b} is independently selected from the group consisting of a hydrogen atom and methyl;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
t is selected from the group consisting of 0, 1, and 2; and
ring C, X, Y, Z, Q, R¹ to R⁴, p, and q are as defined in formula (AAI).

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein 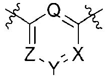 is selected from the group consisting of: 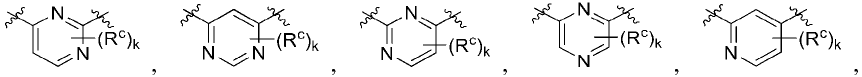 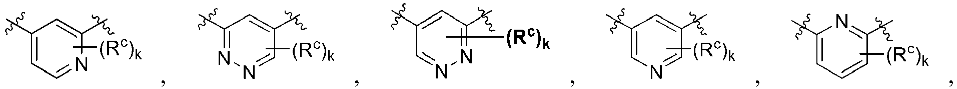 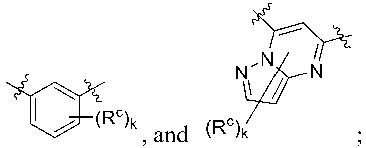
R^{c} is each independently selected from the group consisting of hydrogen atom, halogen, cyano, C₁₋₆ alkoxy, C₁₋₆ haloalkyl, and C₁₋₆ haloalkoxy; and
k is each independently selected from the group consisting of 0, 1, and 2.

In a preferred embodiment, the present disclosure provides a compound of formula (AAI), (AI), (I), (IIA), (IIB), (II), (III), (V-1), (V-2), (V-A), (V-B), (V-C), or (V-D), or a stereoisomer, a tautomer, or a pharmaceutically acceptable salt thereof, wherein ring C is selected from the group consisting of:

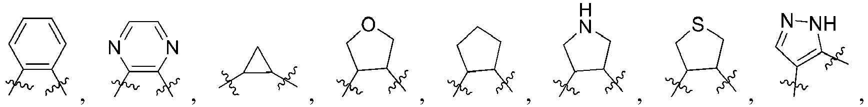

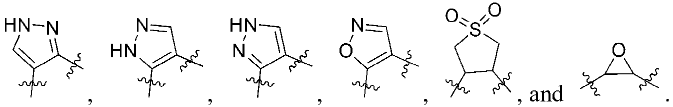

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R⁴ is selected from the group consisting of hydrogen atom, C₁₋₆ alkyl, -C(O)R⁵, -C(O)NR⁶R⁷, -S(O)₂R⁵, and -S(O)₂NR⁶R⁷, wherein the C₁₋₆ alkyl is optionally further substituted with one or more substituents selected from the group consisting of hydroxy, halogen, cyano, and -C(O)OR⁸;
R⁵ is each independently selected from the group consisting of C₁₋₆ alkyl, 3- to 5-membered cycloalkyl, and 5- to 6- membered heteroaryl, wherein the C₁₋₆ alkyl, 3- to 5-membered cycloalkyl, or 5- to 6- membered heteroaryl is optionally further substituted by one or more substituents selected from the group consisting of halogen and C₁₋₆ alkoxy;
R⁶ and R⁷ are each independently selected from the group consisting of a hydrogen atom and C₁₋₆ alkyl; and
R⁸ is selected from the group consisting of a hydrogen atom and C₁₋₆ alkyl.

In a preferred embodiment, the present disclosure provides a compound of formula (AI), (I), (IIA), (IIB), (II), (III), (V-1), (V-2), (V-A), (V-B), (V-C), or (V-D), or a stereoisomer, a tautomer, or a pharmaceutically acceptable salt thereof, wherein 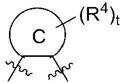 is selected from the group consisting of: 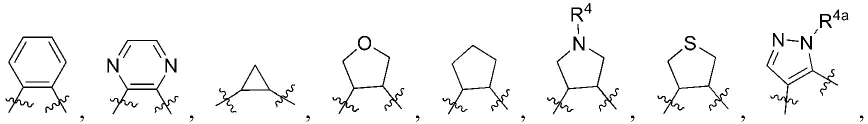 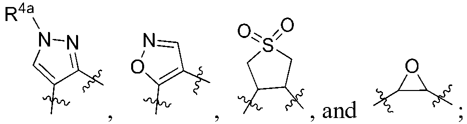
wherein: R^{4a} is each independently selected from the group consisting of a hydrogen atom and C₁₋₆ alkyl; and
R⁴ is as defined in formula (AI).

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R¹ is selected from the group consisting of C₁₋₆ alkyl and 3- to 5- membered cycloalkyl, wherein the C₁₋₆ alkyl or 3- to 5- membered cycloalkyl is optionally further substituted by one or more substituents selected from the group consisting of C₁₋₃ alkyl, C₁₋₃ alkoxy, halogen, hydroxy, and cyano.

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R¹ is selected from the group consisting of cyclopropyl, methyl, ethyl, isopropyl, difluoromethyl, trifluoromethyl,

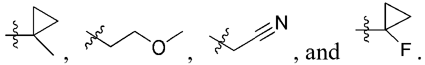

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R² is selected from the group consisting of hydrogen atom, halogen, cyano, C₁₋₆ alkyl, C₁₋₆ alkoxy, C₁₋₆ haloalkyl, and C₁₋₆ haloalkoxy;
alternatively, two R², together with the same carbon atom to which they are attached, form a -C(O).

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R³ is selected from the group consisting of hydrogen atom, halogen, cyano, hydroxy, C₁₋₆ alkyl, C₁₋₆ alkoxy, 3 to 5- membered cycloalkyl, SF₅, C₁₋₆ haloalkyl, and C₁₋₆ haloalkoxy.

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein R³ is selected from the group consisting of chloro, trifluoromethoxy, methyl, ethyl, and ethoxy.

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein p is 0, and q is 0.

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein p is 1, and q is 1 or 2.

In a preferred embodiment, the present disclosure provides a compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, wherein p is 2, and q is 0, 1, or 2.

In a preferred embodiment of the present disclosure, the compound of general formula is selected from the group consisting of:

| **Compound No.** | **Structure** | **Name** |
|---|---|---|
| Example 1 | 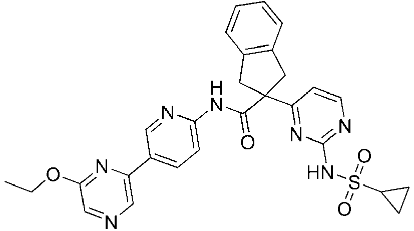 | 2-(2-(cyclopropanesulfonamido)pyr imidin-4-yl)-N-(5-(6-ethoxypyrazin -2-yl)pyridin-2-yl)-2,3-dihydro-1H-indene-2-carboxamide |
| Example 2 | 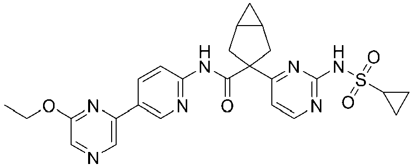 | 3-(2-(cyclopropanesulfonamido)pyr imidin-4-yl)-N-(5-(6-ethoxypyrazin -2-yl)pyridin-2-yl)bicyclo[3.1.0]he xane-3-carboxamide |
| Example 3 | 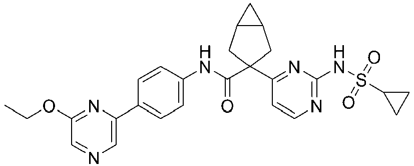 | 3-(2-(cyclopropanesulfonamido)pyr imidin-4-yl)-N-(4-(6-ethoxypyrazin -2-yl)phenyl)bicyclo[3.1.0]hexane-3-carboxamide |
| Example 4 | 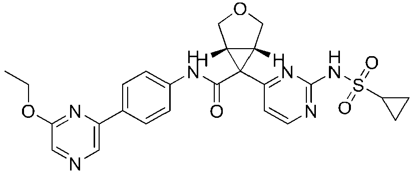 | (1R,5 S)-6-(2-(cyclopropanesulfona mido)pyrimidin-4-yl)-N-(4-(6-etho xypyrazin-2-yl)phenyl)-3-oxabicycl o[3.1.0]hexane-6-carboxamide |
| Example 5 | 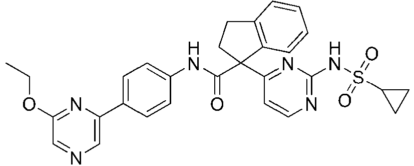 | 1-(2-(cyclopropanesulfonamido)pyr imidin-4-yl)-N-(4-(6-ethoxypyrazin -2-yl)phenyl)-2,3-dihydro-1H-inde ne-1-carboxamide |
| Example 6 | 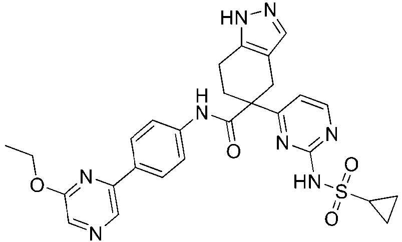 | 5-(2-(cyclopropanesulfonamido)pyr imidin-4-yl)-N-(4-(6-ethoxypyrazin -2-yl)phenyl)-4,5,6,7-tetrahydro-1H -indazole-5-carboxamide |
| Example 7 | 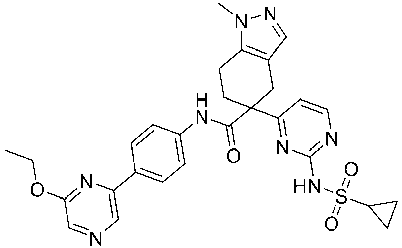 | 5-(2-(cyclopropanesulfonamido)pyr imidin-4-yl)-N-(4-(6-ethoxypyrazin -2-yl)phenyl)-1-methyl-4,5,6,7-tetr ahydro-1H-indazole-5-carboxamide |
| Example 8 | 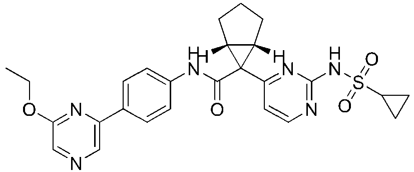 | (1R,5 S)-6-(2-(cyclopropanesulfona mido)pyrimidin-4-yl)-N-(4-(6-etho xypyrazin-2-yl)phenyl)bicyclo[3.1. 0]hexane-6-carboxamide |
| Example 9 | 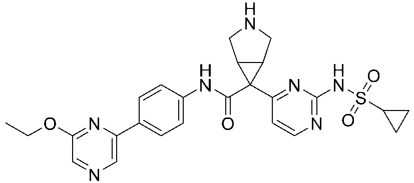 | 6-(2-(cyclopropanesulfonamido)pyr imidin-4-yl)-N-(4-(6-ethoxypyrazin -2-yl)phenyl)-3-azabicyclo[3.1.0]he xane-6-carboxamide |
| Example 10 | 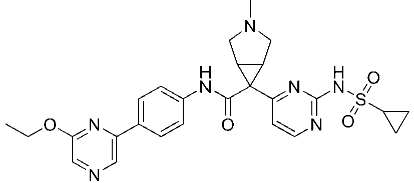 | 6-(2-(cyclopropanesulfonamido)pyr imidin-4-yl)-N-(4-(6-ethoxypyrazin -2-yl)phenyl)-3-methyl-3-azabicycl o[3.1.0]hexane-6-carboxamide |
| Example 11 | 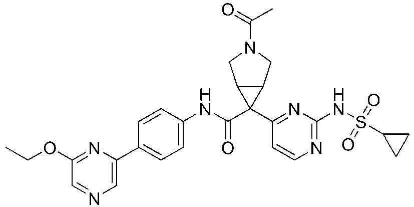 | 3-acetyl-6-(2-(cyclopropanesulfona mido)pyrimidin-4-yl)-N-(4-(6-etho xypyrazin-2-yl)phenyl)-3-azabicycl o[3.1.0]hexane-6-carboxamide |
| Example 12 | 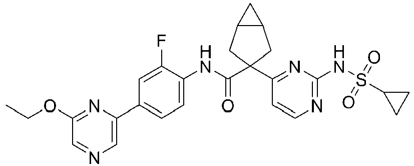 | 3-(2-(cyclopropanesulfonamido)pyr imidin-4-yl)-N-(4-(6-ethoxypyrazin -2-yl)-2-fluorophenyl)bicyclo[3.1.0 ]hexane-3-carboxamide |
| Example 13 | 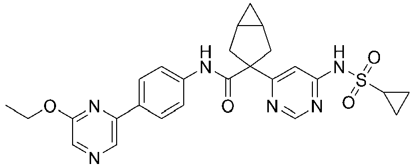 | 3-(6-(cyclopropanesulfonamido)pyr imidin-4-yl)-N-(4-(6-ethoxypyrazin -2-yl)phenyl)bicyclo[3.1.0]hexane-3-carboxamide |
| Example 14 | 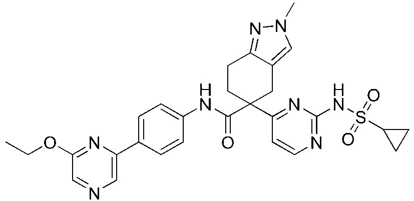 | 5-(2-(cyclopropanesulfonamido)pyr imidin-4-yl)-N-(4-(6-ethoxypyrazin -2-yl)phenyl)-2-methyl-4,5,6,7-tetr ahydro-2H-indazole-5-carboxamide |
| Example 15 | 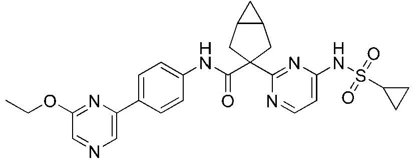 | 3-(4-(cyclopropanesulfonamido)pyr imidin-2-yl)-N-(4-(6-ethoxypyrazin -2-yl)phenyl)bicyclo[3.1.0]hexane-3-carboxamide |
| Example 16 | 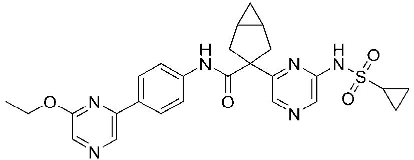 | 3-(6-(cyclopropanesulfonamido)pyr azin-2-yl)-N-(4-(6-ethoxypyrazin-2 -yl)phenyl)bicyclo[3.1.0]hexane-3-carboxamide |
| Example 17 | 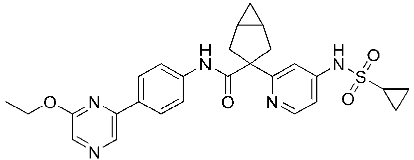 | 3-(4-(cyclopropanesulfonamido)pyr idin-2-yl)-N-(4-(6-ethoxypyrazin-2 -yl)phenyl)bicyclo[3.1.0]hexane-3-carboxamide |
| Example 18 | 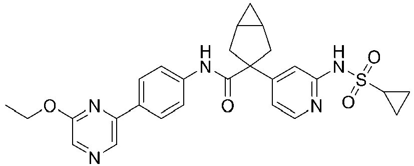 | 3-(2-(cyclopropanesulfonamido)pyr idin-4-yl)-N-(4-(6-ethoxypyrazin-2 -yl)phenyl)bicyclo[3.1.0]hexane-3-carboxamide |
| Example 19 | 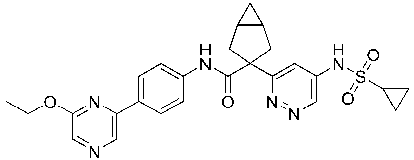 | 3-(5-(cyclopropanesulfonamido)pyr idazin-3 -yl)-N-(4-(6-ethoxypyrazin -2-yl)phenyl)bicyclo[3.1.0]hexane-3-carboxamide |
| Example 20 | 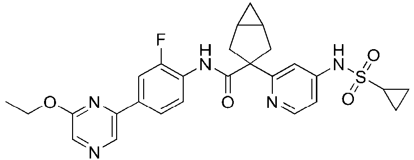 | 3-(4-(cyclopropanesulfonamido)pyr idin-2-yl)-N-(4-(6-ethoxypyrazin-2 -yl)-2-fluorophenyl)bicyclo[3.1.0]h exane-3-carboxamide |
| Example 21 | 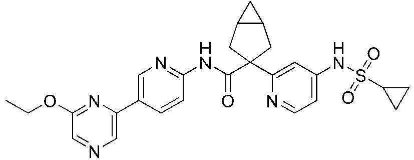 | 3-(4-(cyclopropanesulfonamido)pyr idin-2-yl)-N-(5-(6-ethoxypyrazin-2 -yl)pyridin-2-yl)bicyclo[3.1.0]hexa ne-3-carboxamide |
| Example 22 | 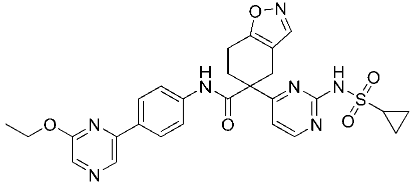 | 5-(2-(cyclopropanesulfonamido)pyr imidin-4-yl)-N-(4-(6-ethoxypyrazin -2-yl)phenyl)-4,5,6,7-tetrahydroben zo[d]isoxazole-5-carboxamide |
| Example 23 | 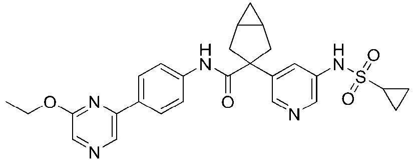 | 3-(5-(cyclopropanesulfonamido)pyr idin-3-yl)-N-(4-(6-ethoxypyrazin-2 -yl)phenyl)bicyclo[3.1.0]hexane-3-carboxamide |
| Example 24 | 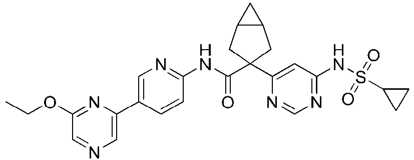 | 3-(6-(cyclopropanesulfonamido)pyr imidin-4-yl)-N-(5-(6-ethoxypyrazin -2-yl)pyridin-2-yl)bicyclo[3.1.0]he xane-3-carboxamide |
| Example 25 | 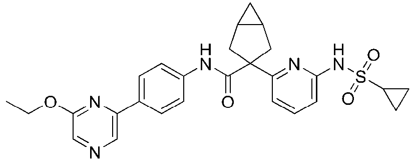 | 3-(6-(cyclopropanesulfonamido)pyr idin-2-yl)-N-(4-(6-ethoxypyrazin-2 -yl)phenyl)bicyclo[3.1.0]hexane-3-carboxamide |
| Example 26 | 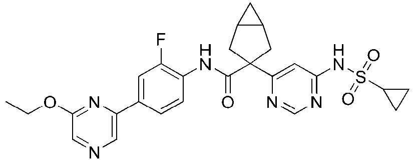 | 3-(6-(cyclopropanesulfonamido)pyr imidin-4-yl)-N-(4-(6-ethoxypyrazin -2-yl)-2-fluorophenyl)bicyclo[3.1.0 ]hexane-3-carboxamide |
| Example 27 | 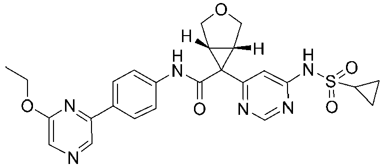 | (1R,5 S)-6-(6-(cyclopropanesulfona mido)pyrimidin-4-yl)-N-(4-(6-etho xypyrazin-2-yl)phenyl)-3-oxabicycl o[3.1.0]hexane-6-carboxamide |
| Example 28 | 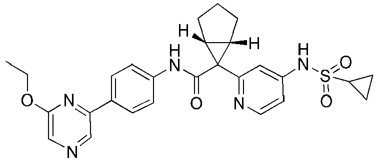 | (1R,5S)-6-(4-(cyclopropanesulfona mido)pyridin-2-yl)-N-(4-(6-ethoxy pyrazin-2-yl)phenyl)bicyclo[3.1.0] hexane-6-carboxamide |
| Example 29 | 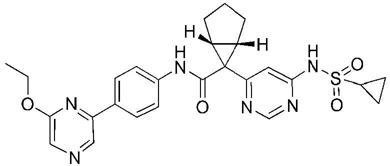 | (1R,5S)-6-(6-(cyclopropanesulfona mido)pyrimidin-4-yl)-N-(4-(6-etho xypyrazin-2-yl)phenyl)bicyclo[3.1. 0]hexane-6-carboxamide |
| Example 30 | 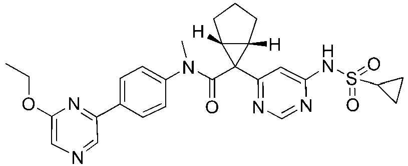 | (1R,5S)-6-(6-(cyclopropanesulfona mido)pyrimidin-4-yl)-N-(4-(6-etho xypyrazin-2-yl)phenyl)-N-methylbi cyclo[3.1.0]hexane-6-carboxamide |
| Example 31 | 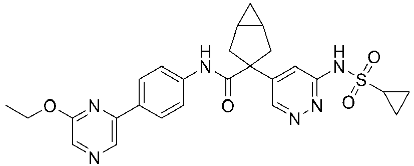 | 3-(6-(cyclopropanesulfonamido)pyr idazin-4-yl)-N-(4-(6-ethoxypyrazin -2-yl)phenyl)bicyclo[3.1.0]hexane-3-carboxamide |
| Example 32 | 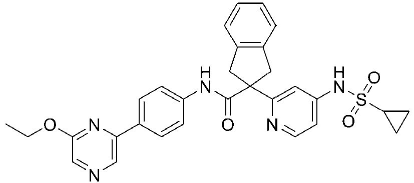 | 2-(4-(cyclopropanesulfonamido)pyr idin-2-yl)-N-(4-(6-ethoxypyrazin-2 -yl)phenyl)-2,3-dihydro-1H-indene-2-carboxamide |
| Example 33 | 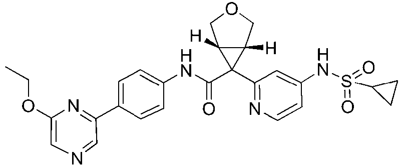 | (1R,5S)-6-(4-(cyclopropanesulfona mido)pyridin-2-yl)-N-(4-(6-ethoxy pyrazin-2-yl)phenyl)-3-oxabicyclo[ 3.1.0]hexane-6-carboxamide |
| Example 34 | 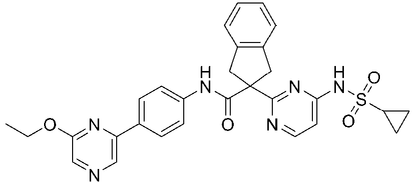 | 2-(4-(cyclopropanesulfonamido)pyr imidin-2-yl)-N-(4-(6-ethoxypyrazin -2-yl)phenyl)-2,3-dihydro-1H-inde ne-2-carboxamide |
| Example 35 | 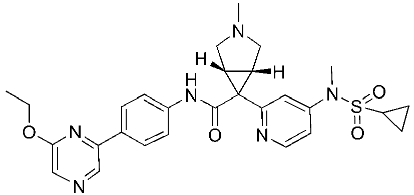 | (1R,5S)-N-(4-(6-ethoxypyrazin-2-y l)phenyl)-3-methyl-6-(4-(N-methyl cyclopropanesulfonamido)pyridin-2-yl)-3-azabicyclo[3.1.0]hexane-6-carboxamide |
| Example 36 | 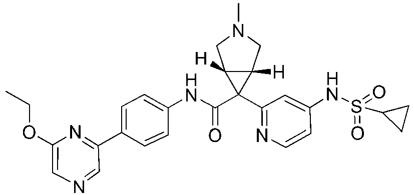 | (1R,5S)-6-(4-(cyclopropanesulfona mido)pyridin-2-yl)-N-(4-(6-ethoxy pyrazin-2-yl)phenyl)-3-methyl-3-az abicyclo[3.1.0]hexane-6-carboxami de |
| Example 37 | 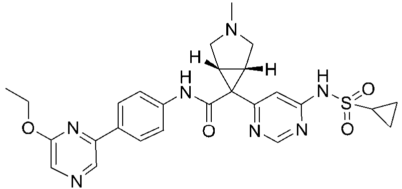 | (1R,5S)-6-(6-(cyclopropanesulfona mido)pyrimidin-4-yl)-N-(4-(6-etho xypyrazin-2-yl)phenyl)-3-methyl-3 -azabicyclo[3.1.0]hexane-6-carboxa mide |
| Example 38 | 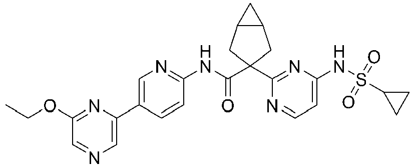 | 3-(4-(cyclopropanesulfonamido)pyr imidin-2-yl)-N-(5-(6-ethoxypyrazin -2-yl)pyridin-2-yl)bicyclo[3.1.0]he xane-3-carboxamide |
| | | |
| Example 39 | 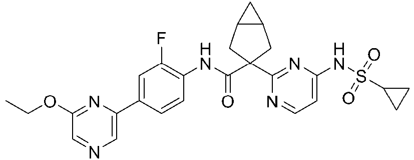 | 3-(4-(cyclopropanesulfonamido)pyr imidin-2-yl)-N-(4-(6-ethoxypyrazin -2-yl)-2-fluorophenyl)bicyclo[3.1.0 ]hexane-3-carboxamide |
| Example 40 | 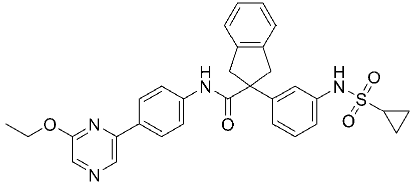 | 2-(3-(cyclopropanesulfonamido)ph enyl)-N-(4-(6-ethoxypyrazin-2-yl)p henyl)-2,3-dihydro-1H-indene-2-ca rboxamide |
| Example 41 | 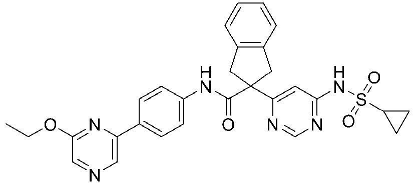 | 2-(6-(cyclopropanesulfonamido)pyr imidin-4-yl)-N-(4-(6-ethoxypyrazin -2-yl)phenyl)-2,3-dihydro-1H-inde ne-2-carboxamide |
| Example 42 | 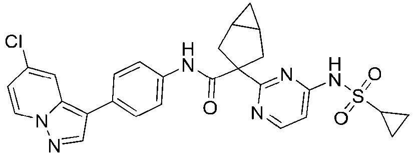 | N-(4-(5-chloropyrazolo[1,5-a]pyrid in-3-yl)phenyl)-3-(4-(cyclopropane sulfonamido)pyrimidin-2-yl)bicycl o[3.1.0]hexane-3-carboxamide |
| Example 43 | 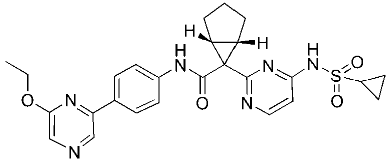 | (1R,5S)-6-(4-(cyclopropanesulfona mido)pyrimidin-2-yl)-N-(4-(6-etho xypyrazin-2-yl)phenyl)bicyclo[3.1. 0]hexane-6-carboxamide |
| Example 44 | 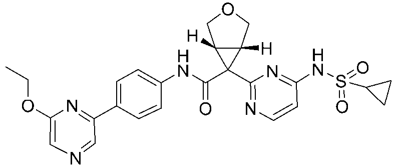 | (1R,5S)-6-(4-(cyclopropanesulfona mido)pyrimidin-2-yl)-N-(4-(6-etho xypyrazin-2-yl)phenyl)-3-oxabicycl o[3.1.0]hexane-6-carboxamide |
| Example 45 | 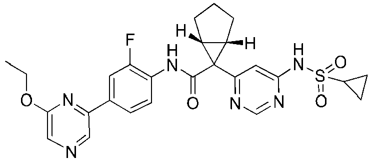 | (1R,5S)-6-(6-(cyclopropanesulfona mido)pyrimidin-4-yl)-N-(4-(6-etho xypyrazin-2-yl)-2-fluorophenyl)bic yclo[3.1.0]hexane-6-carboxamide |
| Example 46 | 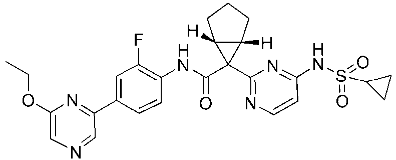 | (1R,5S)-6-(4-(cyclopropanesulfona mido)pyrimidin-2-yl)-N-(4-(6-etho xypyrazin-2-yl)-2-fluorophenyl)bic yclo[3.1.0]hexane-6-carboxamide |
| Example 47 | 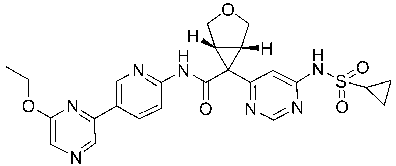 | (1R,5S)-6-(6-(cyclopropanesulfona mido)pyrimidin-4-yl)-N-(5-(6-etho xypyrazin-2-yl)pyridin-2-yl)-3-oxa bicyclo[3.1.0]hexane-6-carboxamid e |
| Example 48 | 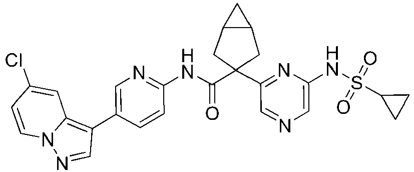 | N-(5-(5-chloropyrazolo[1,5-a]pyrid in-3-yl)pyridin-2-yl)-3-(6-(cyclopro panesulfonamido)pyrazin-2-yl)bicy clo[3.1.0]hexane-3-carboxamide |
| Example 49 | 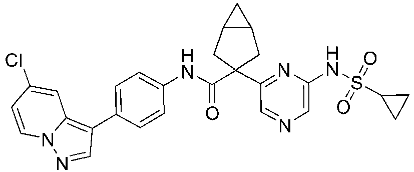 | N-(4-(5-chloropyrazolo[1,5-a]pyrid in-3-yl)phenyl)-3-(6-(cyclopropane sulfonamido)pyrazin-2-yl)bicyclo[3 .1.0]hexane-3-carboxamide |
| Example 50 | 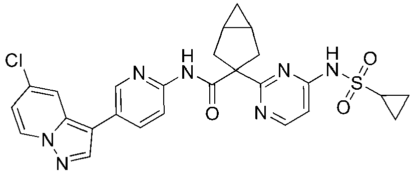 | N-(5-(5-chloropyrazolo[1,5-a]pyrid in-3-yl)pyridin-2-yl)-3-(4-(cyclopro panesulfonamido)pyrimidin-2-yl)bi cyclo[3.1.0]hexane-3-carboxamide |
| Example 51 | 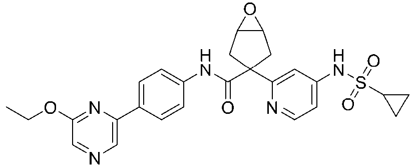 | 3-(4-(cyclopropanesulfonamido)pyr idin-2-yl)-N-(4-(6-ethoxypyrazin-2 -yl)phenyl)-6-oxabicyclo[3.1.0]hex ane-3-carboxamide |
| Example 52 | 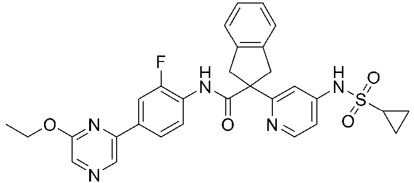 | 2-(4-(cyclopropanesulfonamido)pyr idin-2-yl)-N-(4-(6-ethoxypyrazin-2 -yl)-2-fluorophenyl)-2,3-dihydro-1 H-indene-2-carboxamide |
| Example 53 | 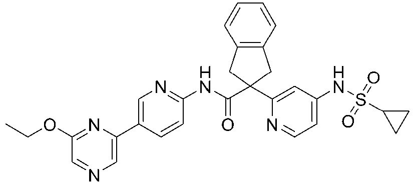 | 2-(4-(cyclopropanesulfonamido)pyr idin-2-yl)-N-(5-(6-ethoxypyrazin-2 -yl)pyridin-2-yl)-2,3-dihydro-1H-in dene-2-carboxamide |
| Example 54 | 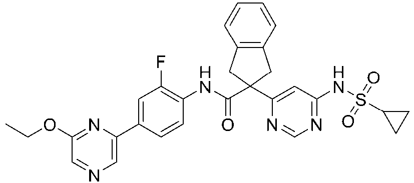 | 2-(6-(cyclopropanesulfonamido)pyr imidin-4-yl)-N-(4-(6-ethoxypyrazin -2-yl)-2-fluorophenyl)-2,3-dihydro-1H-indene-2-carboxamide |
| Example 55 | 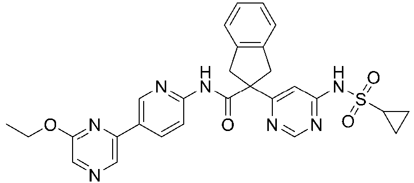 | 2-(6-(cyclopropanesulfonamido)pyr imidin-4-yl)-N-(5-(6-ethoxypyrazin -2-yl)pyridin-2-yl)-2,3-dihydro-1H-indene-2-carboxamide |
| Example 56 | 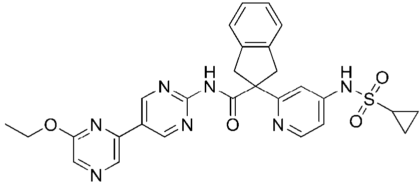 | 2-(4-(cyclopropanesulfonamido)pyr idin-2-yl)-N-(5-(6-ethoxypyrazin-2 -yl)pyrimidin-2-yl)-2,3-dihydro-1H -indene-2-carboxamide |
| Example 57 | 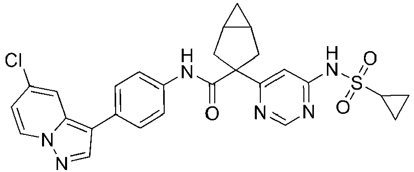 | N-(4-(5-chloropyrazolo[1,5-a]pyrid in-3-yl)phenyl)-3-(6-(cyclopropane sulfonamido)pyrimidin-4-yl)bicycl o[3.1.0]hexane-3-carboxamide |
| Example 58 | 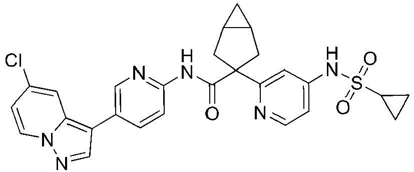 | N-(5-(5-chloropyrazolo[1,5-a]pyrid in-3-yl)pyridin-2-yl)-3-(4-(cyclopro panesulfonamido)pyridin-2-yl)bicy clo[3.1.0]hexane-3-carboxamide |
| Example 59 | 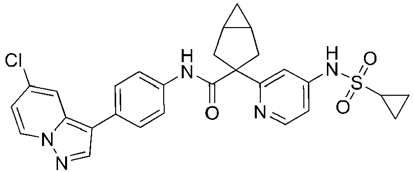 | N-(4-(5-chloropyrazolo[1,5-a]pyrid in-3-yl)phenyl)-3-(4-(cyclopropane sulfonamido)pyridin-2-yl)bicyclo[3 .1.0]hexane-3-carboxamide |
| Example 60 | 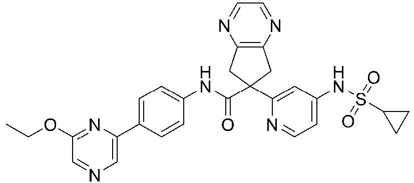 | 6-(4-(cyclopropanesulfonamido)pyr idin-2-yl)-N-(4-(6-ethoxypyrazin-2 -yl)phenyl)-6,7-dihydro-5H-cyclop enta[b]pyrazine-6-carboxamide |
| Example 61 | 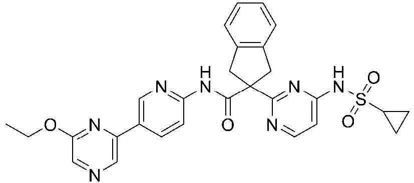 | 2-(4-(cyclopropanesulfonamido)pyr imidin-2-yl)-N-(5-(6-ethoxypyrazin -2-yl)pyridin-2-yl)-2,3-dihydro-1H-indene-2-carboxamide |
| Example 62 | 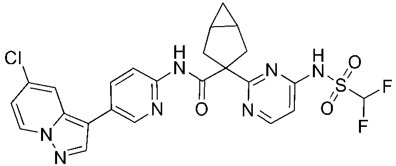 | N-(5-(5-chloropyrazolo[1,5-a]pyrid in-3-yl)pyridin-2-yl)-3-(4-((difluor omethyl)sulfonamido)pyrimidin-2-yl)bicyclo[3.1.0]hexane-3-carboxa mide |
| Example 63 | 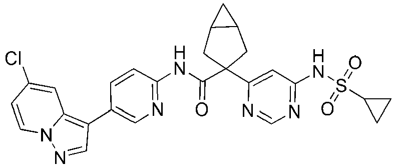 | N-(5-(5-chloropyrazolo[1,5-a]pyrid in-3-yl)pyridin-2-yl)-3-(6-(cyclopro panesulfonamido)pyrimidin-4-yl)bi cyclo[3.1.0]hexane-3-carboxamide |
| Example 64 | 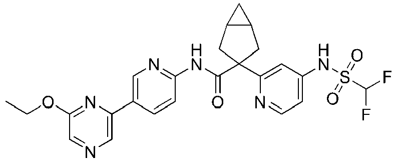 | 3-(4-((difluoromethyl)sulfonamido) pyridin-2-yl)-N-(5-(6-ethoxypyrazi n-2-yl)pyridin-2-yl)bicyclo[3.1.0]h exane-3-carboxamide |
| Example 65 | 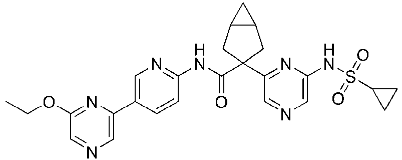 | 3-(6-(cyclopropanesulfonamido)pyr azin-2-yl)-N-(5-(6-ethoxypyrazin-2 -yl)pyridin-2-yl)bicyclo[3.1.0]hexa ne-3-carboxamide |
| Example 66 | 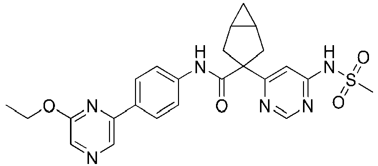 | N-(4-(6-ethoxypyrazin-2-yl)phenyl )-3-(6-(methylsulfonamido)pyrimid in-4-yl)bicyclo[3.1.0]hexane-3-carb oxamide |
| Example 67 | 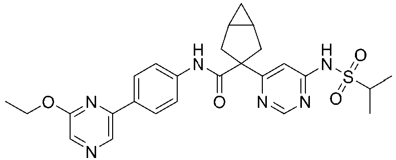 | N-(4-(6-ethoxypyrazin-2-yl)phenyl )-3-(6-((1-methylethyl)sulfonamido )pyrimidin-4-yl)bicyclo[3.1.0]hexa ne-3-carboxamide |
| Example 68 | 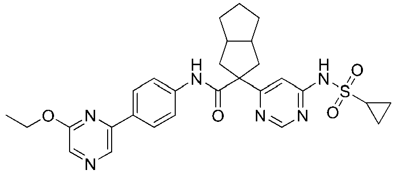 | 2-(6-(cyclopropanesulfonamido)pyr imidin-4-yl)-N-(4-(6-ethoxypyrazin -2-yl)phenyl)octahydropentalene-2-carboxamide |
| Example 69 | 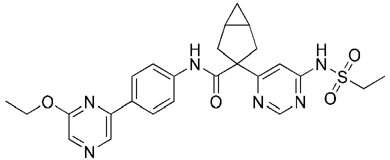 | N-(4-(6-ethoxypyrazin-2-yl)phenyl )-3-(6-(ethylsulfonamido)pyrimidin -4-yl)bicyclo[3.1.0]hexane-3-carbo xamide |
| Example 70 | 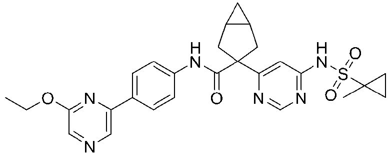 | N-(4-(6-ethoxypyrazin-2-yl)phenyl )-3-(6-((1-methylcyclopropane)-1-s ulfonamido)pyrimidin-4-yl)bicyclo [3.1.0]hexane-3-carboxamide |
| Example 71 | 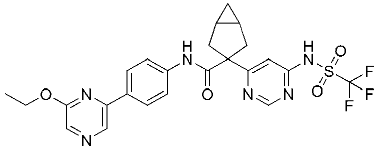 | N-(4-(6-ethoxypyrazin-2-yl)phenyl )-3-(6-((trifluoromethyl)sulfonamid o)pyrimidin-4-yl)bicyclo[3.1.0]hex ane-3-carboxamide |
| Example 72 | 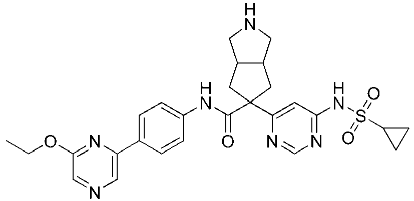 | 5-(6-(cyclopropanesulfonamido)pyr imidin-4-yl)-N-(4-(6-ethoxypyrazin -2-yl)phenyl)octahydrocyclopenta[ c]pyrrole-5-carboxamide |
| | | |
| Example 73 | 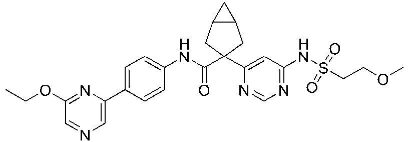 | N-(4-(6-ethoxypyrazin-2-yl)phenyl )-3-(6-((2-methoxyethyl)sulfonami do)pyrimidin-4-yl)bicyclo[3.1.0]he xane-3-carboxamide |
| Example 74 | 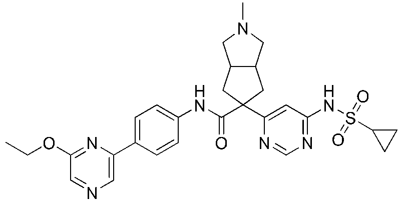 | 5-(6-(cyclopropanesulfonamido)pyr imidin-4-yl)-N-(4-(6-ethoxypyrazin -2-yl)phenyl)-2-methyloctahydrocy clopenta[c]pyrrole-5-carboxamide |
| Example 75 | 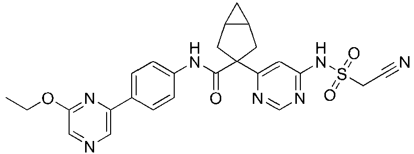 | 3-(6-((cyanomethyl)sulfonamido)p yrimidin-4-yl)-N-(4-(6-ethoxypyraz in-2-yl)phenyl)bicyclo[3.1.0]hexan e-3-carboxamide |
| Example 76 | 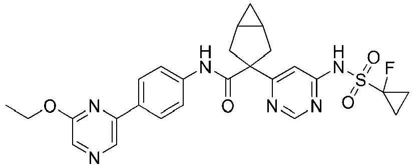 | N-(4-(6-ethoxypyrazin-2-yl)phenyl )-3-(6-((1-fluorocyclopropane)-1-su lfonamido)pyrimidin-4-yl)bicyclo[ 3.1.0]hexane-3-carboxamide |
| Example 77 | 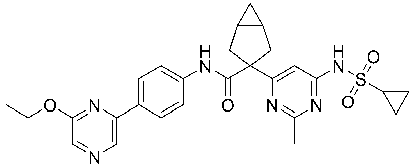 | 3-(6-(cyclopropanesulfonamido)-2-methylpyrimidin-4-yl)-N-(4-(6-eth oxypyrazin-2-yl)phenyl)bicyclo[3.1 .0]hexane-3-carboxamide |
| Example 78 | 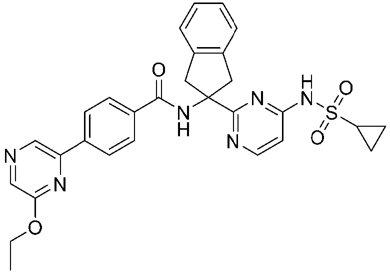 | N-(2-(4-(cyclopropanesulfonamido )pyrimidin-2-yl)-2,3-dihydro-1H-in den-2-yl)-4-(6-ethoxypyrazin-2-yl) benzamide |
| Example 79 | 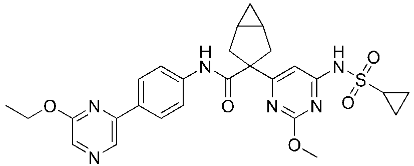 | 3-(6-(cyclopropanesulfonamido)-2-methoxypyrimidin-4-yl)-N-(4-(6-et hoxypyrazin-2-yl)phenyl)bicyclo[3. 1.0]hexane-3-carboxamide |
| Example 80 | 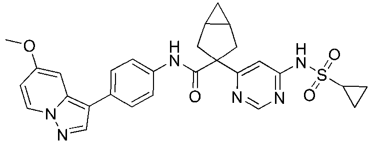 | 3-(6-(cyclopropanesulfonamido)pyr imidin-4-yl)-N-(4-(5-methoxypyraz olo[1,5-a]pyridin-3-yl)phenyl)bicyc lo[3.1.0]hexane-3-carboxamide |
| Example 81 | 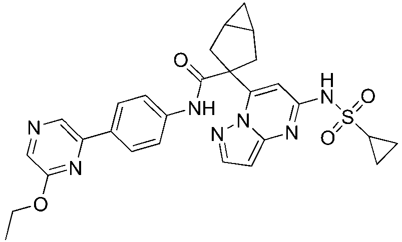 | 3-(5-(cyclopropanesulfonamido)pyr azolo[1,5-a]pyrimidin-7-yl)-N-(4-( 6-ethoxypyrazin-2-yl)phenyl)bicycl o[3.1.0]hexane-3-carboxamide |
| Example 82 | 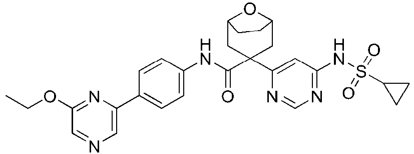 | 3-(6-(cyclopropanesulfonamido)pyr imidin-4-yl)-N-(4-(6-ethoxypyrazin -2-yl)phenyl)-8-oxabicyclo[3.2.1]o ctane-3-carboxamide |
| Example 83 | 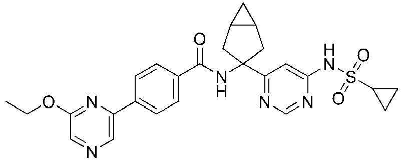 | N-(3-(6-(cyclopropanesulfonamido )pyrimidin-4-yl)bicyclo[3.1.0]hexa n-3-yl)-4-(6-ethoxypyrazin-2-yl)be nzamide |
| Example 84 | 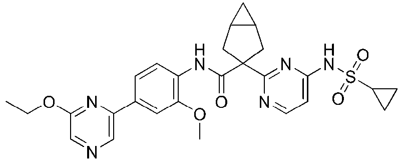 | 3-(4-(cyclopropanesulfonamido)pyr imidin-2-yl)-N-(4-(6-ethoxypyrazin -2-yl)-2-methoxyphenyl)bicyclo[3. 1.0]hexane-3-carboxamide |
| Example 85 | 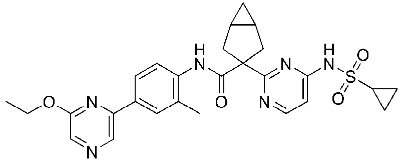 | 3-(4-(cyclopropanesulfonamido)pyr imidin-2-yl)-N-(4-(6-ethoxypyrazin -2-yl)-2-methylphenyl)bicyclo[3.1. 0]hexane-3-carboxamide |
| Example 86 | 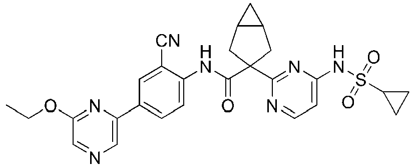 | N-(2-cyano-4-(6-ethoxypyrazin-2-y l)phenyl)-3-(4-(cyclopropanesulfon amido)pyrimidin-2-yl)bicyclo[3.1.0 ]hexane-3-carboxamide |
| Example 87 | 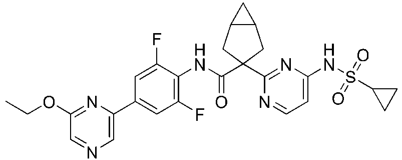 | 3-(4-(cyclopropanesulfonamido)pyr imidin-2-yl)-N-(4-(6-ethoxypyrazin -2-yl)-2,6-difluorophenyl)bicyclo[3 .1.0]hexane-3-carboxamide |
| Example 88 | 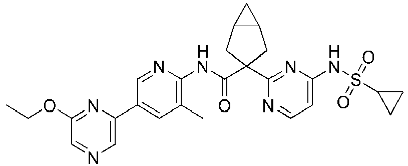 | 3-(4-(cyclopropanesulfonamido)pyr imidin-2-yl)-N-(5-(6-ethoxypyrazin -2-yl)-3-methylpyridin-2-yl)bicyclo [3.1.0]hexane-3-carboxamide |
| Example 89 | 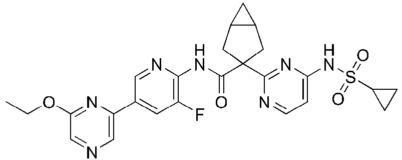 | 3-(4-(cyclopropanesulfonamido)pyr imidin-2-yl)-N-(5-(6-ethoxypyrazin -2-yl)-3-fluoropyridin-2-yl)bicyclo[ 3.1.0]hexane-3-carboxamide |
| Example 90 | 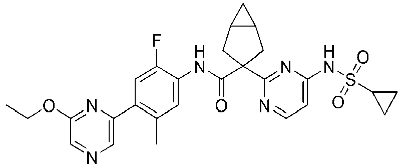 | 3-(4-(cyclopropanesulfonamido)pyr imidin-2-yl)-N-(4-(6-ethoxypyrazin -2-yl)-2-fluoro-5-methylphenyl)bic yclo[3.1.0]hexane-3-carboxamide |
| Example 91 | 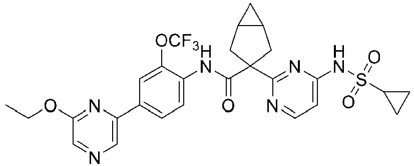 | 3-(4-(cyclopropanesulfonamido)pyr imidin-2-yl)-N-(4-(6-ethoxypyrazin -2-yl)-2-(trifluoromethoxy)phenyl) bicyclo[3.1.0]hexane-3-carboxamid e |
| Example 92 | 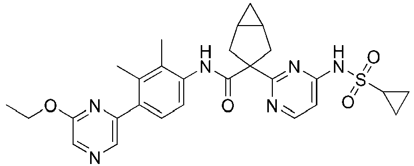 | 3-(4-(cyclopropanesulfonamido)pyr imidin-2-yl)-N-(4-(6-ethoxypyrazin -2-yl)-2,3-dimethylphenyl)bicyclo[ 3.1.0]hexane-3-carboxamide |
| Example 93 | 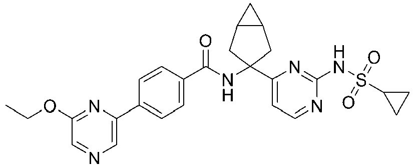 | N-(3-(2-(cyclopropanesulfonamido )pyrimidin-4-yl)bicyclo[3.1.0]hexa n-3-yl)-4-(6-ethoxypyrazin-2-yl)be nzamide |
| Example 94 | 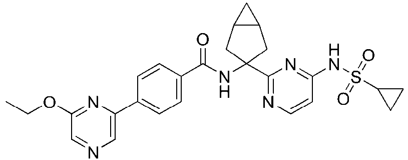 | N-(3-(4-(cyclopropanesulfonamido )pyrimidin-2-yl)bicyclo[3.1.0]hexa n-3-yl)-4-(6-ethoxypyrazin-2-yl)be nzamide |
| Example 95 | 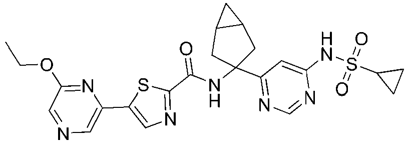 | N-(3-(6-(cyclopropanesulfonamido )pyrimidin-4-yl)bicyclo[3.1.0]hexa n-3-yl)-5-(6-ethoxypyrazin-2-yl)thi azole-2-carboxamide |
| Example 96 | 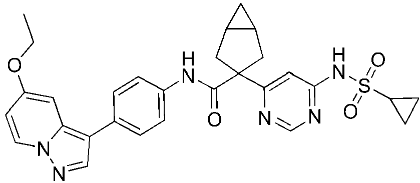 | 3-(6-(cyclopropanesulfonamido)pyr imidin-4-yl)-N-(4-(5-ethoxypyrazol o[1,5-a]pyridin-3-yl)phenyl)bicyclo [3.1.0]hexane-3-carboxamide |
| Example 97 | 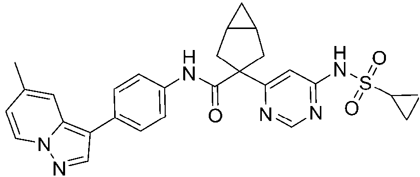 | 3-(6-(cyclopropanesulfonamido)pyr imidin-4-yl)-N-(4-(5-methylpyrazol o[1,5-a]pyridin-3-yl)phenyl)bicyclo [3.1.0]hexane-3-carboxamide |
| Example 98 | 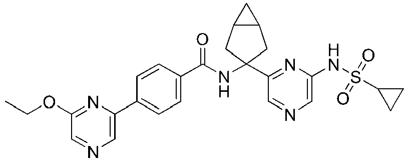 | N-(3-(6-(cyclopropanesulfonamido )pyrazin-2-yl)bicyclo[3.1.0]hexan-3-yl)-4-(6-ethoxypyrazin-2-yl)benz amide |
| Example 99 | 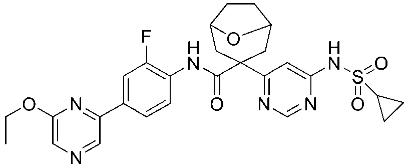 | 3-(6-(cyclopropanesulfonamido)pyr imidin-4-yl)-N-(4-(6-ethoxypyrazin -2-yl)-2-fluorophenyl)-8-oxabicycl o[3.2.1]octane-3-carboxamide |
| Example 100 | 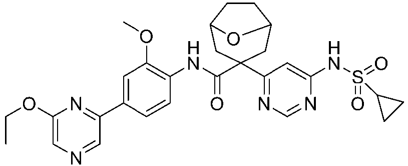 | 3-(6-(cyclopropanesulfonamido)pyr imidin-4-yl)-N-(4-(6-ethoxypyrazin -2-yl)-2-methoxyphenyl)-8-oxabicy clo[3.2.1]octane-3-carboxamide |
| Example 101 | 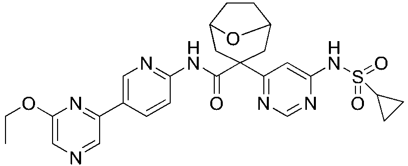 | 3-(6-(cyclopropanesulfonamido)pyr imidin-4-yl)-N-(5-(6-ethoxypyrazin -2-yl)pyridin-2-yl)-8-oxabicyclo[3. 2.1]octane-3-carboxamide |
| Example 102 | 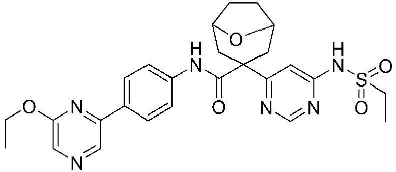 | N-(4-(6-ethoxypyrazin-2-yl)phenyl )-3-(6-(ethylsulfonamido)pyrimidin -4-yl)-8-oxabicyclo[3.2.1]octane-3-carboxamide |
| Example 103 | 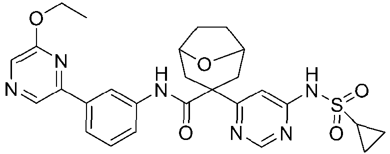 | 3-(6-(cyclopropanesulfonamido)pyr imidin-4-yl)-N-(3-(6-ethoxypyrazin -2-yl)phenyl)-8-oxabicyclo[3.2.1]o ctane-3-carboxamide |
| Example 104 | 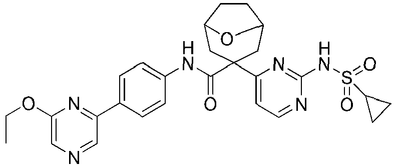 | 3-(2-(cyclopropanesulfonamido)pyr imidin-4-yl)-N-(4-(6-ethoxypyrazin -2-yl)phenyl)-8-oxabicyclo[3.2.1]o ctane-3-carboxamide |
| Example 105 | 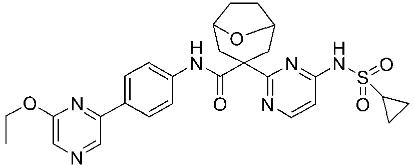 | 3-(4-(cyclopropanesulfonamido)pyr imidin-2-yl)-N-(4-(6-ethoxypyrazin -2-yl)phenyl)-8-oxabicyclo[3.2.1]o ctane-3-carboxamide |
| Example 106 | 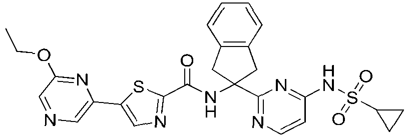 | N-(2-(4-(cyclopropanesulfonamido )pyrimidin-2-yl)-2,3-dihydro-1H-in den-2-yl)-5-(6-ethoxypyrazin-2-yl)t hiazole-2-carboxamide |
| Example 107 | 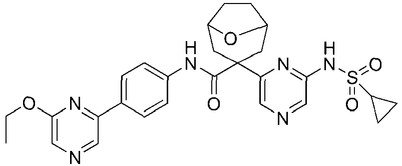 | 3-(6-(cyclopropanesulfonamido)pyr azin-2-yl)-N-(4-(6-ethoxypyrazin-2 -yl)phenyl)-8-oxabicyclo[3.2.1]octa ne-3-carboxamide |
| Example 108 | 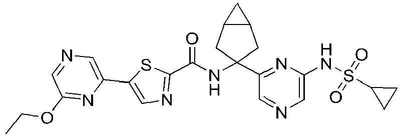 | N-(3-(6-(cyclopropanesulfonamido )pyrazin-2-yl)bicyclo[3.1.0]hexan-3-yl)-5-(6-ethoxypyrazin-2-yl)thiaz ole-2-carboxamide |
| Example 109 | 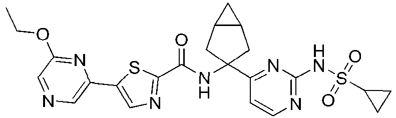 | N-(3-(2-(cyclopropanesulfonamido )pyrimidin-4-yl)bicyclo[3.1.0]hexa n-3-yl)-5-(6-ethoxypyrazin-2-yl)thi azole-2-carboxamide |
| Example 110 | 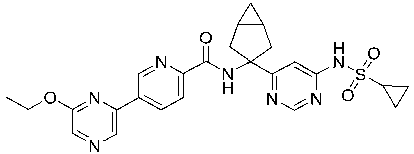 | N-(3-(6-(cyclopropanesulfonamido )pyrimidin-4-yl)bicyclo[3.1.0]hexa n-3-yl)-5-(6-ethoxypyrazin-2-yl)pic olinamide |
| Example 111 | 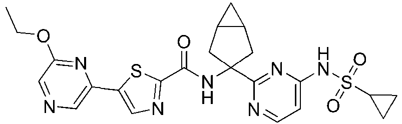 | N-(3-(4-(cyclopropanesulfonamido )pyrimidin-2-yl)bicyclo[3.1.0]hexa n-3-yl)-5-(6-ethoxypyrazin-2-yl)thi azole-2-carboxamide |
| Example 112 | 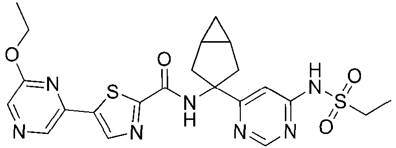 | 5-(6-ethoxypyrazin-2-yl)-N-(3-(6-( ethylsulfonamido)pyrimidin-4-yl)bi cyclo[3.1.0]hexan-3-yl)thiazole-2-c arboxamide |
| Example 113 | 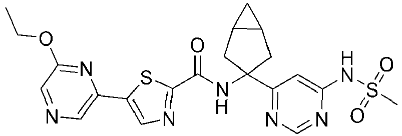 | 5-(6-ethoxypyrazin-2-yl)-N-(3-(6-( methylsulfonamido)pyrimidin-4-yl) bicyclo[3.1.0]hexan-3-yl)thiazole-2 -carboxamide |
| Example 114 | 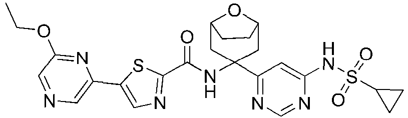 | N-((1R,5S)-3-(6-(cyclopropanesulf onamido)pyrimidin-4-yl)-8-oxabicy clo[3.2.1]octan-3-yl)-5-(6-ethoxypy razin-2-yl)thiazole-2-carboxamide |
| Example 115 | 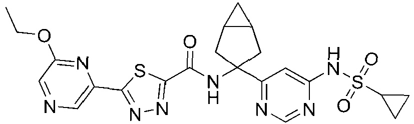 | N-(3-(6-(cyclopropanesulfonamido )pyrimidin-4-yl)bicyclo[3.1.0]hexa n-3-yl)-5-(6-ethoxypyrazin-2-yl)-1, 3,4-thiadiazole-2-carboxamide |
| Example 116 | 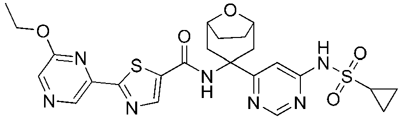 | N-((1R,5S)-3-(6-(cyclopropanesulf onamido)pyrimidin-4-yl)-8-oxabicy clo[3.2.1]octan-3-yl)-2-(6-ethoxypy razin-2-yl)thiazole-5-carboxamide |
| Example 117 | 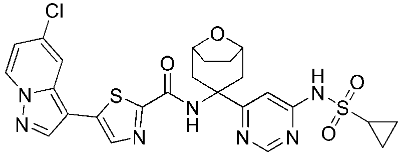 | 5-(5-chloropyrazolo[1,5-a]pyridin-3-yl)-N-((1R,5S)-3-(6-(cyclopropan esulfonamido)pyrimidin-4-yl)-8-ox abicyclo[3.2.1]octan-3-yl)thiazole-2-carboxamide |
| Example 118 | 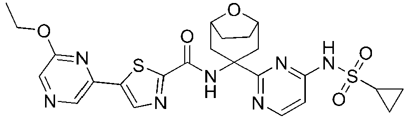 | N-((1R,5S)-3-(4-(cyclopropanesulf onamido)pyrimidin-2-yl)-8-oxabicy clo[3.2.1]octan-3-yl)-5-(6-ethoxypy razin-2-yl)thiazole-2-carboxamide |
| Example 119 | 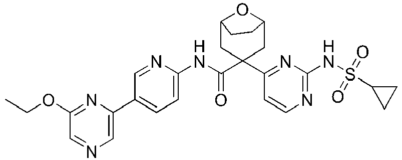 | (1R,5 S)-3-(2-(cyclopropanesulfonam ido)pyrimidin-4-yl)-N-(5-(6-ethoxyp yrazin-2-yl)pyridin-2-yl)-8-oxabicycl o[3.2.1]octane-3-carboxamide |
| Example 120 | 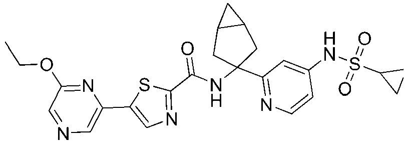 | N-(3-(4-(cyclopropanesulfonamido)p yridin-2-yl)bicyclo[3.1.0]hexan-3-yl) -5-(6-ethoxypyrazin-2-yl)thiazole-2-carboxamide |
| Example 121 | 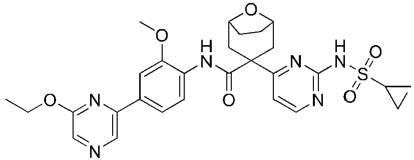 | (1R,5S)-3-(2-(cyclopropanesulfonam ido)pyrimidin-4-yl)-N-(4-(6-ethoxyp yrazin-2-yl)-2-methoxyphenyl)-8-ox abicyclo[3.2.1]octane-3-carboxamide |
| Example 122 | 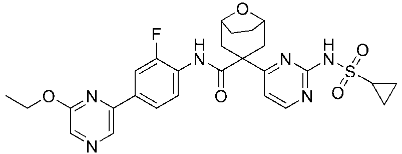 | (1R,5S)-3-(2-(cyclopropanesulfonam ido)pyrimidin-4-yl)-N-(4-(6-ethoxyp yrazin-2-yl)-2-fluorophenyl)-8-oxabi cyclo[3.2.1]octane-3-carboxamide |
| Example 123 | 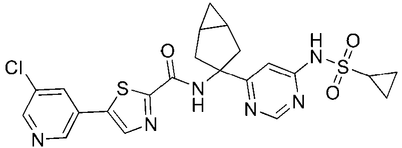 | 5-(5-chloropyridin-3-yl)-N-(3-(6-(cy clopropanesulfonamido)pyrimidin-4-yl)bicyclo[3.1.0]hexan-3-yl)thiazole-2-carboxamide |
| Example 124 | 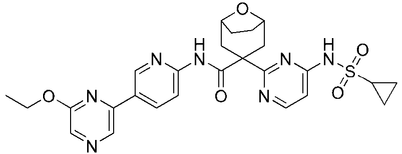 | (1R,5S)-3-(4-(cyclopropanesulfonam ido)pyrimidin-2-yl)-N-(5-(6-ethoxyp yrazin-2-yl)pyridin-2-yl)-8-oxabicycl o[3.2.1]octane-3-carboxamide |
| Example 125 | 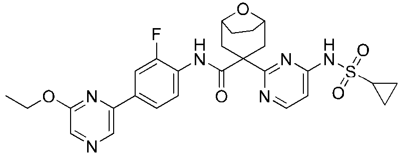 | (1R,5S)-3-(4-(cyclopropanesulfonam ido)pyrimidin-2-yl)-N-(4-(6-ethoxyp yrazin-2-yl)-2-fluorophenyl)-8-oxabi cyclo[3.2.1]octane-3-carboxamide |
| Example 126 | 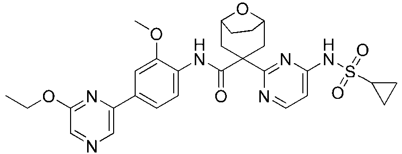 | (1R,5S)-3-(4-(cyclopropanesulfonam ido)pyrimidin-2-yl)-N-(4-(6-ethoxyp yrazin-2-yl)-2-methoxyphenyl)-8-ox abicyclo[3.2.1]octane-3-carboxamide |
| Example 127 | 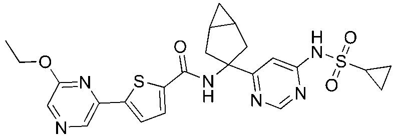 | N-(3-(6-(cyclopropanesulfonamido)p yrimidin-4-yl)bicyclo[3.1.0]hexan-3-yl)-5-(6-ethoxypyrazin-2-yl)thiophen e-2-carboxamide |
| Example 128 | 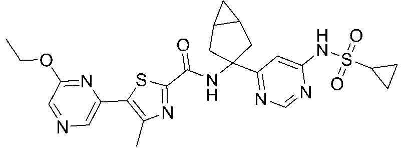 | N-(3-(6-(cyclopropanesulfonamido)p yrimidin-4-yl)bicyclo[3.1.0]hexan-3-yl)-5-(6-ethoxypyrazin-2-yl)-4-meth ylthiazole-2-carboxamide |
| Example 129 | 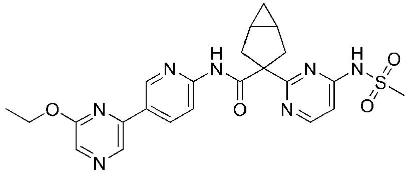 | N-(5-(6-ethoxypyrazin-2-yl)pyridin-2-yl)-3-(4-(methylsulfonamido)pyri midin-2-yl)bicyclo[3.1.0]hexane-3-c arboxamide |
| Example 130 | 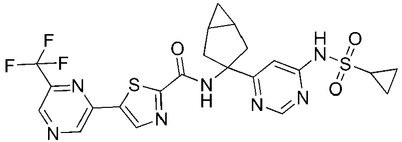 | N-(3-(6-(cyclopropanesulfonamido)p yrimidin-4-yl)bicyclo[3.1.0]hexan-3-yl)-5-(6-(trifluoromethyl)pyrazin-2-y l)thiazole-2-carboxamide |
| Example 131 | 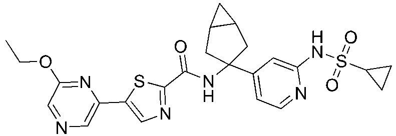 | N-(3-(2-(cyclopropanesulfonamido)p yridin-4-yl)bicyclo[3.1.0]hexan-3-yl) -5-(6-ethoxypyrazin-2-yl)thiazole-2-carboxamide |
| Example 132 | 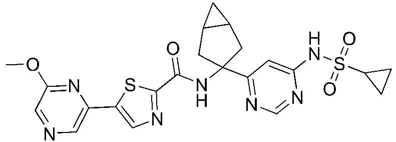 | N-(3-(6-(cyclopropanesulfonamido)p yrimidin-4-yl)bicyclo[3.1.0]hexan-3-yl)-5-(6-methoxypyrazin-2-yl)thiazol e-2-carboxamide |
| Example 133 | 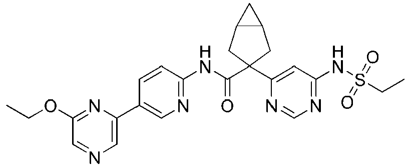 | N-(5-(6-ethoxypyrazin-2-yl)pyridin-2-yl)-3-(6-(ethylsulfonamido)pyrimi din-4-yl)bicyclo[3.1.0]hexane-3-carb oxamide |
| Example 134 | 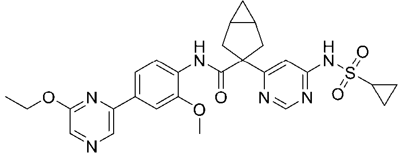 | 3-(6-(cyclopropanesulfonamido)pyri midin-4-yl)-N-(4-(6-ethoxypyrazin-2 -yl)-2-methoxyphenyl)bicyclo[3.1.0] hexane-3-carboxamide |
| Example 135 | 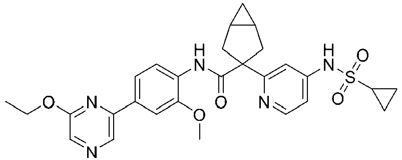 | 3-(4-(cyclopropanesulfonamido)pyri din-2-yl)-N-(4-(6-ethoxypyrazin-2-yl )-2-methoxyphenyl)bicyclo[3.1.0]he xane-3-carboxamide |
| Example 136 | 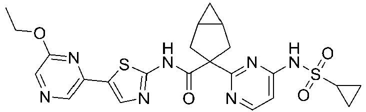 | 3-(4-(cyclopropanesulfonamido)pyri midin-2-yl)-N-(5-(6-ethoxypyrazin-2 -yl)thiazol-2-yl)bicyclo[3.1.0]hexane -3-carboxamide |

or a stereoisomer, a tautomer, or a pharmaceutically acceptable salt thereof.

Note: If there is a discrepancy between the drawn structure and the name given for the structure, the drawn structure shall prevail.

Further, the present disclosure provides a pharmaceutical composition comprising an effective dose of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, and a pharmaceutically acceptable carrier.

The present disclosure provides a use of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof, in the manufacture of a CTPS1 inhibitor.

The present disclosure further provides a use of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof, in the manufacture of a medicament for treating a disease mediated by CTPS1, wherein the disease mediated by CTPS1 is preferably lymphoma or solid tumor; more preferably relapsed/refractory B-cell or T-cell lymphoma; most preferably mantle cell lymphoma, diffuse large B-cell lymphoma, peripheral T-cell lymphoma, cutaneous T-cell lymphoma, or indolent B-cell lymphoma.

The present disclosure further provides a use of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof, in the manufacture of a medicament for treating lymphoma or solid tumor.

The present disclosure provides a use of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof, in the manufacture of a medicament for treating relapsed/refractory B-cell and T-cell lymphoma.

The present disclosure provides a use of the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof, in the manufacture of a medicament for treating mantle cell lymphoma, diffuse large B-cell lymphoma, peripheral T-cell lymphoma, cutaneous T-cell lymphoma, or indolent B-cell lymphoma.

Accordingly, the present disclosure further provides a method for preventing or treating a disease mediated by CTPS1, comprising administering to a subject in need thereof the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof; wherein the disease mediated by CTPS1 is preferably lymphoma or solid tumor; more preferably relapsed/refractory B-cell or T-cell lymphoma; most preferably mantle cell lymphoma, diffuse large B-cell lymphoma, peripheral T-cell lymphoma, cutaneous T-cell lymphoma, or indolent B-cell lymphoma.

The present disclosure further provides a method for preventing or treating lymphoma or solid tumor, comprising administering to a subject in need thereof the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof. The present disclosure further provides a method of preventing or treating relapsed/refractory B-cell and T-cell lymphoma, comprising administering to a subject in need thereof the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof. The present disclosure further provides a method for preventing or treating mantle cell lymphoma, diffuse large B-cell lymphoma, peripheral T-cell lymphoma, cutaneous T-cell lymphoma, or indolent B-cell lymphoma, comprising administering to a subject in need thereof the compound of formula (AAI), (AI), (I), (IIA), (IIB), (II), (III), (IV-1), (IV-2), (V-1), (V-2), (V-A), (V-B), (V-C), or (V-D), or a stereoisomer, a tautomer, or a pharmaceutically acceptable salt thereof, or the pharmaceutical composition thereof.

### DETAILED DESCRIPTION

Unless stated to the contrary, certain terms used in the specification and claims of the present disclosure are defined as follows:
"Alkyl," when used as a group or as part of a group, refers to a C₁-C₂₀ linear or branched aliphatic hydrocarbon group. It is preferably C₁-C₁₀ alkyl, more preferably C₁-C₆ alkyl or C₁-C₄ alkyl. Examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and the like. Alkyl may be substituted or unsubstituted.

"Alkenyl" refers to an alkyl group as defined above consisting of at least two carbon atoms and at least one carbon-carbon double bond, and representative examples include, but are not limited to, ethenyl, 1-propenyl, 2-propenyl, 1-, 2-, or 3-butenyl, 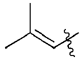 and the like; preferably C₂-C₄ alkenyl. Alkenyl may be optionally substituted or unsubstituted.

"Alkynyl" refers to an aliphatic hydrocarbon group containing one carbon-carbon triple bond, which may be linear or branched. Preferably C₂-C₁₀ alkynyl, more preferably C₂-C₆ alkynyl, most preferably C₂-C₄ alkynyl. Examples of alkynyl groups include, but are not limited to, ethynyl, 1-propynyl, 2-propynyl, 1-, 2-, or 3-butynyl, and the like. Alkynyl may be substituted or unsubstituted.

"Alkylene" refers to a saturated C₁-C₂₀ linear or branched aliphatic hydrocarbon group having two radicals derived by removing two hydrogen atoms from the same carbon atom or two different carbon atoms of a parent alkane, preferably C₁-C₁₀ alkylene, more preferably C₁-C₆ alkylene or C₁-C₄ alkylene. Examples of alkylene groups include, but are not limited to, methylene, 1,1-ethylene, 1,2-ethylene, 1,1-propylene, 1,2-propylene, 1,3-propylene, 1,4-butylene, and the like. Alkylene may be substituted or unsubstituted.

"Cycloalkyl" refers to a non-aromatic cyclic alkyl group in which one or more ring-forming atoms are carbon atoms, including monocyclic, polycyclic, fused, bridged, and spiro rings, preferably having 3- to 10- membered cycloalkyl or 3- to 8- membered cycloalkyl, for example 5- to 7- membered monocyclic or 7- to 10- membered bicyclic or tricyclic rings. Examples of "cycloalkyl" include, but are not limited to, cyclopropyl, cyclopentyl, cyclobutyl. Cycloalkyl may be substituted or unsubstituted.

"Spirocycloalkyl" refers to a 5- to 18- membered polycyclic group having two or more ring structures, wherein monocyclic rings share one carbon atom (called a spiro atom) with each other, containing one or more double bonds in the rings, but no ring having a fully conjugated π-electron aromatic system. Preferably 6- to 14- membered, more preferably 7- to 10- membered. According to the number of spiro atoms shared between rings, spirocycloalkyl is divided into monospiro, dispiro or polispirocycloalkyl, preferably monospiro and dispirocycloalkyl, preferably 4-/5-, 4-/6-, 5-/5- or 5-/6- membered. Non-limiting examples of "spirocycloalkyl" include, but are not limited to: spiro[4.5]decyl, spiro[4.4]nonyl, spiro[3.5]nonyl, spiro[2.4]heptyl.

"Fused cycloalkyl" refers to a 5- to 18- membered all-carbon polycyclic group containing two or more ring structures sharing a pair of carbon atoms with each other, wherein one or more rings may contain one or more double bonds, but no ring has a fully conjugated π-electron aromatic system, preferably 6- to 12- membered, more preferably 7- to 10- membered. According to the number of constituent rings, it may be divided into bicyclic, tricyclic, tetracyclic or polycyclic fused cycloalkyl, preferably bicyclic or tricyclic, more preferably 5-/5- or 5-/6-membered bicyclic alkyl. Non-limiting examples of "fused cycloalkyl" include, but are not limited to: bicyclo[3.1.0]hexyl, bicyclo[3.2.0]hept-1-enyl, bicyclo[3.2.0]heptyl, decahydronaphthyl or tetradecahydrophenanthryl.

"Bridged cycloalkyl" refers to a 5- to 18- membered all-carbon polycyclic group containing two or more ring structures sharing two non-directly connected carbon atoms with each other, wherein one or more rings may contain one or more double bonds, but no ring has a fully conjugated π-electron aromatic system, preferably 6- to 12- membered, more preferably 7-to 10- membered. Preferably 6- to 14- membered, more preferably 7- to 10- membered. According to the number of constituent rings, it may be divided into bicyclic, tricyclic, tetracyclic or polycyclic bridged cycloalkyl, preferably bicyclic, tricyclic or tetracyclic, more preferably bicyclic or tricyclic. Non-limiting examples of "bridged cycloalkyl" include, but are not limited to: (1s,4s)-bicyclo[2.2.1]heptyl, bicyclo[3.2.1]octyl, (1s,5s)-bicyclo[3.3.1]nonyl, bicyclo[2.2.2]octyl, (1r,5r)-bicyclo[3.3.2]decyl.

"Heterocyclyl," "heterocycloalkyl," "heterocycle," or "heterocyclic" are used interchangeably in the present disclosure and refer to a non-aromatic heterocyclic group in which one or more ring-forming atoms are heteroatoms selected from the group consisting of nitrogen, oxygen, and S(O)ᵣ (wherein t is selected from the group consisting of 0, 1, and 2), including monocyclic, polycyclic, fused, bridged, and spiro rings. Preferably having 5- to 7- membered monocyclic or 7- to 10- membered bicyclic or tricyclic rings, which may contain 1, 2, or 3 atoms selected from the group consisting of nitrogen, oxygen, and sulfur. Examples of "heterocyclyl" include, but are not limited to, morpholino, oxetanyl, thiomorpholino, tetrahydrofuranyl, tetrahydropyranyl, 1,1-dioxo-thiomorpholino, piperidinyl, 2-oxo-piperidinyl, pyrrolidinyl, 2-oxo-pyrrolidinyl, piperazin-2-one, 8-oxa-3-aza-bicyclo[3.2.1]octyl, piperazinyl, hexahydropyrimidinyl;
heterocyclyl may be substituted or unsubstituted.

"Spiraheterocyclyl" refers to a polycyclic group having 5 to 18 members and two or more ring structures, wherein the rings share a single atom with each other, and the rings contain one or more double bonds but no ring has a fully conjugated π-electron aromatic system, wherein one or more ring atoms are heteroatoms selected from the group consisting of nitrogen, oxygen, and S(O)t (where t is selected from the group consisting of 0, 1, and 2), and the remaining ring atoms are carbon. Preferably 6- to 14- membered, more preferably 7- to 10- membered. According to the number of spiro atoms shared between rings, spiroheterocyclyl is divided into monospiroheterocyclyl, dispiroheterocyclyl, or polyspiroheterocyclyl, preferably monospiroheterocyclyl or dispiroheterocyclyl. More preferably 4-/4- membered, 4-/5- membered, 3-/5- membered, 3-/6- membered, 4-/6- membered, 5-/5- membered, or 5-/6- membered monospiroheterocyclyl. Non-limiting examples of "spiraheterocyclyl" include, but are not limited to: 1,7-dioxaspiro[4.5]decanyl, 2-oxa-7-azaspiro[4.4]nonyl, 7-oxaspiro[3.5]nonyl, and 5-oxaspiro[2.4]heptyl.

"Fused heterocyclyl" refers to an all-carbon polycyclic group containing two or more ring structures sharing a pair of atoms with each other, wherein one or more rings may contain one or more double bonds but no ring has a fully conjugated π-electron aromatic system, wherein one or more ring atoms are heteroatoms selected from the group consisting of nitrogen, oxygen, and S(O)ᵣ (where t is selected from the group consisting of 0, 1, and 2), and the remaining ring atoms are carbon. Preferably 6- to 14- membered, more preferably 7- to 10- membered. According to the number of constituent rings, it can be divided into bicyclic, tricyclic, tetracyclic, or polycyclic fused heterocyclyl, preferably bicyclic or tricyclic, more preferably 5-/5- membered or 5-/6- membered bicyclic fused heterocyclyl. Non-limiting examples of "fused heterocyclyl" include, but are not limited to: octahydropyrrolo[3,4-c]pyrrolyl, octahydro-1H-isoindolyl, 3-azabicyclo[3.1.0]hexyl, and octahydrobenzo[b][1,4]dioxinyl.

"Bridged heterocyclyl" refers to a polycyclic group having 5 to 14 members or 5 to 18 members, and containing two or more ring structures sharing two non-adjacent atoms with each other, wherein one or more rings may contain one or more double bonds but no ring has a fully conjugated π-electron aromatic system, wherein one or more ring atoms are heteroatoms selected from the group consisting of nitrogen, oxygen, and S(O)t (where t is selected from the group consisting of 0, 1, and 2), and the remaining ring atoms are carbon. Preferably 6- to 14-membered, more preferably 7- to 10- membered. According to the number of constituent rings, it can be divided into bicyclic, tricyclic, tetracyclic, or polycyclic bridged heterocyclyl, preferably bicyclic, tricyclic, or tetracyclic, more preferably bicyclic or tricyclic. Non-limiting examples of "bridged heterocyclyl" include, but are not limited to: 2-azabicyclo[2.2.1]heptyl, 2-azabicyclo[2.2.2]octyl, and 2-azabicyclo[3.3.2]decyl.

"Aryl" refers to a carbocyclic aromatic system containing one or two rings, wherein the rings can be connected together in a fused manner. The term "aryl" includes monocyclic or bicyclic aryl groups, such as phenyl, naphthyl, and tetrahydronaphthyl aromatic groups. Preferably aryl is C₆-C₁₀ aryl, more preferably aryl is phenyl and naphthyl, most preferably naphthyl. Aryl may be substituted or unsubstituted.

"Heteroaryl" refers to an aromatic 5- to 6- membered monocycle or 8- to 10- membered bicycle, which can contain 1 to 4 atoms selected from the group consisting of nitrogen, oxygen, and sulfur. Examples of "heteroaryl" include, but are not limited to, furyl, pyridinyl, 2-oxo-1,2-dihydropyridinyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolinyl, indazolyl, benzisothiazolyl, benzoxazolyl, benzisoxazolyl, isothiazolyl, 1H-1,2,4-triazolyl, 4H-1,2,4-triazolyl, pyridinyl, pyrimidinyl, pyrazin-2(1H)-onyl, pyrimidin-4(3H)-onyl, pyridazin-3(2H)-onyl, 1H-indolyl, 1H-benzo[d]imidazolyl, 1H-pyrrolo[2,3-c]pyridinyl, 3H-imidazo[4,5-c]pyridinyl, isoquinolinyl, quinazolinyl, 2H-isoindolyl, furan[3,2-b]pyridinyl, furan[2,3-c]pyridinyl, thieno[2,3-c]pyridinyl, benzofuranyl, benzo[b]thienyl, 1H-pyrrolo[3,2-b]pyridinyl, and 2H-pyrrolo[3,4-c]pyridinyl.

Heteroaryl can be substituted or unsubstituted.

"Alkoxy" refers to a group of (alkyl-O-). wherein alkyl is as defined in the present disclosure, preferably C₁-C₆ alkoxy. Examples thereof include, but are not limited to: methoxy, ethoxy, n-propoxy, isopropoxy, n-butoxy, isobutoxy, and tert-butoxy.

"Nitro" refers to a -NO₂ group.

"Hydroxy" refers to a -OH group.

"Halogen" refers to fluorine, chlorine, bromine, and iodine.

"Amino" refers to -NH₂.

"Cyano" refers to -CN.

"Benzyl" refers to -CH₂-phenyl.

"Carboxy" refers to -C(O)OH.

"Carboxylate" group refers to -C(O)O-alkyl or -C(O)O-cycloalkyl, wherein alkyl and cycloalkyl are as defined above.

"Hydroxyalkyl" refers to an alkyl group substituted by hydroxy, wherein alkyl is as defined above.

"Aminoalkyl" refers to an alkyl group substituted by amino, wherein alkyl is as defined above.

"Haloalkyl" refers to an alkyl group substituted by a halogen, wherein alkyl is as defined above.

"Haloalkoxy" refers to an alkoxy group substituted by a halogen, wherein alkoxy is as defined above.

"DMSO" refers to dimethyl sulfoxide.

"BOC" refers to tert-butoxycarbonyl.

"Bn" refers to benzyl.

"THP" refers to 2-tetrahydropyranyl.

"TFA" refers to trifluoroacetic acid.

"Ts" refers to p-toluenesulfonyl.

"Leaving group" refers to an atom or functional group that dissociates from a larger molecule in a chemical reaction, and is a term applied in nucleophilic substitution reactions and elimination reactions. In a nucleophilic substitution reaction, the reactant attacked by a nucleophile is referred to as a substrate, and the atom or group that breaks away from the substrate molecule with a pair of electrons is referred to as a leaving group. Groups that readily accept electrons and have a strong ability to bear negative charge are good leaving groups. The smaller the pKa of the conjugate acid of a leaving group, the more easily the leaving group dissociates from other molecules. The reason is that when the pKa of its conjugate acid is smaller, the corresponding leaving group has an increased tendency to exist in the form of an anion (or an electrically neutral leaving group) without binding to other atoms. Common leaving groups include, but are not limited to, halogen, methylsulfonyl, -OTs, or -OH.

"Substituted" refers to one or more hydrogen atoms in a group, preferably up to 5, more preferably 1 to 3 hydrogen atoms, being independently replaced by a corresponding number of substituents. It is understood that substituents are only located at their possible chemical positions, and a person skilled in the art can determine possible or impossible substitutions (through experiment or theory) without undue effort. For example, an amino or hydroxy group having free hydrogen may be unstable when bound to a carbon atom having an unsaturated (e.g., olefinic) bond.

"Substitution" or "substituted" as described in the present disclosure, unless otherwise specified, refers to a group that may be substituted by one or more substituents selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, amino, haloalkyl, hydroxyalkyl, carboxy, carboxylate, =O, -OR⁶, -C(O)R⁶, -C(O)OR⁶, -NHC(O)R⁶, -NHC(O)OR⁶, -NR⁷R⁸, -C(0)NR⁷R⁸, -CH₂NHC(O)OR⁶, -CH₂NR⁷R⁸, and -S(O)rR⁶;
R⁶ is selected from the group consisting of a hydrogen atom, alkyl, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹, and -NR¹⁰C(O)R¹¹;
R⁷ and R⁸ are each independently selected from the group consisting of a hydrogen atom, hydroxy, halogen, alkyl, alkoxy, cycloalkyl, heterocyclyl, aryl, and heteroaryl, wherein the alkyl, alkoxy, cycloalkyl, heterocyclyl, aryl, or heteroaryl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹, and -NR¹⁰C(O)R¹¹;
alternatively, R⁷ and R⁸, together with the atoms to which they are attached, form a 4- to 8- membered heterocyclyl, wherein the 4- to 8- membered heterocyclyl contains one or more N, O, or S(O)r, and the 4- to 8- membered heterocyclyl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁹, -C(O)OR⁹, -OC(O)R⁹, -NR¹⁰R¹¹, -C(O)NR¹⁰R¹¹, -SO₂NR¹⁰R¹¹, and -NR¹⁰C(O)R¹¹;
R⁹, R¹⁰, and R¹¹ are each independently selected from the group consisting of a hydrogen atom, alkyl, amino, cycloalkyl, heterocyclyl, aryl, and heteroaryl, wherein the alkyl, cycloalkyl, heterocyclyl, aryl, or heteroaryl are optionally further substituted by one or more substituents selected from the group consisting of hydroxy, halogen, nitro, amino, cyano, alkyl, alkoxy, cycloalkyl, heterocyclyl, aryl, heteroaryl, carboxy, and carboxylate;
r is selected from the group consisting of 0, 1, and 2.

Compounds of the present disclosure may contain asymmetric centers or chiral centers and therefore exist in different stereoisomeric forms. It is intended that all stereoisomeric forms of the compounds of the present disclosure, including but not limited to diastereomers, enantiomers, and atropisomers and geometric (conformational) isomers and mixtures thereof, such as racemic mixtures, are within the scope of the present disclosure.

Unless otherwise specified, the structures described in the present disclosure also include all isomers of such structures (e.g., diastereomers, enantiomers, and atropisomers and geometric (conformational) isomer forms; for example, the R and S configurations at each asymmetric center, (Z) and (E) double bond isomers, and (Z) and (E) conformational isomers. Therefore, individual stereoisomers of the compounds of the present disclosure as well as enantiomer mixtures, diastereomer mixtures, and geometric (conformational) isomer mixtures are all within the scope of the present disclosure.

"Pharmaceutically acceptable salt" refers to certain salts of the above-mentioned compounds that retain the original biological activity and are suitable for pharmaceutical use. The pharmaceutically acceptable salt of the compound represented by formula (I) may be a metal salt or an amine salt formed with an appropriate acid.

"Pharmaceutical composition" refers to a mixture containing one or more compounds described in the present disclosure or physiologically pharmaceutically acceptable salts or prodrugs thereof with other chemical components, and other components such as physiologically pharmaceutically acceptable carriers and excipients. The purpose of the pharmaceutical composition is to facilitate administration to an organism and promote absorption of the active ingredient to exert biological activity.

### Synthesis methods of compounds of the present disclosure

In order to achieve the object of the present disclosure, the following technical solutions are adopted:

### Method 1:

The present disclosure provides a method for preparing a compound of formula (II) or a stereoisomer, a tautomer, or a pharmaceutically acceptable salt thereof, comprising: 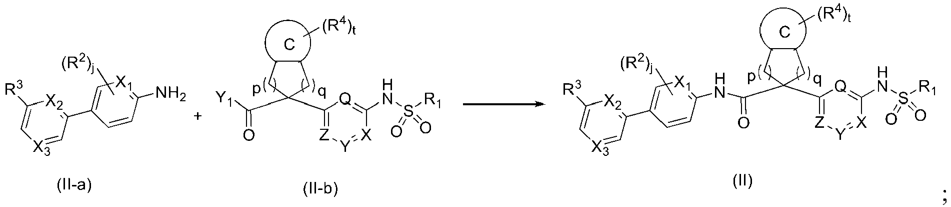
performing an acid-amine condensation reaction or an amine-ester exchange reaction with a compound of formula (II-a) and a compound of formula (II-b), and optionally further performing a substitution reaction, to obtain a compound of formula (II);
wherein:
   Y₁ is selected from the group consisting of hydroxy, methoxy, and ethoxy;
   X₁, X₂, X₃, X, Y, Z, Q, R¹ to R⁴, p, q, j, and t are as defined in formula (II).

### Method 2:

The present disclosure provides a method for preparing a compound of formula (III) or a stereoisomer, a tautomer, or a pharmaceutically acceptable salt thereof, comprising: 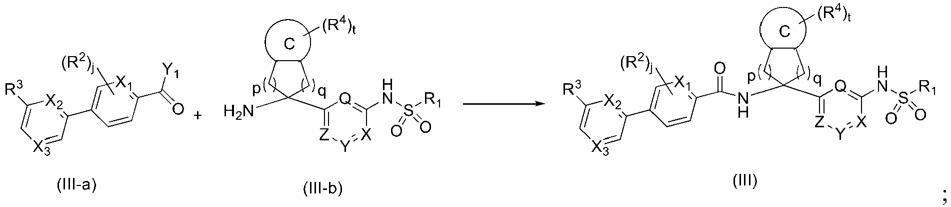
performing an acid-amine condensation reaction or an amine-ester exchange reaction with a compound of formula (III-a) and a compound of formula (III-b), and optionally further performing a substitution reaction, to obtain a compound of formula (III);
wherein:
   Y₁ is selected from the group consisting of hydroxy, methoxy, and ethoxy;
   X₁, X₂, X₃, X, Y, Z, Q, R¹ to R⁴, p, q, j, and t are as defined in formula (III).

### Detailed Description of Embodiments

The present disclosure is further described below in conjunction with examples, however, these examples are not intented to limit the scope of the present disclosure.

### Examples

The examples provide the preparation of representative compounds of formula (I) and relevant structural characterization data. It should be noted that the following examples are for illustrating the present disclosure and not limiting the present disclosure. ¹H NMR spectra were measured using a Bruker instrument (400 MHz), and chemical shifts were expressed in ppm. Tetramethylsilane was used as an internal standard (0.00 ppm). The representation of ¹H NMR: s = singlet, d = doublet, t = triplet, m = multiplet, br = broadened, dd = doublet of doublets, dt = doublet of triplets. When coupling constants were provided, the unit was Hz.

Mass spectra were measured using an LC/MS instrument, and the ionization mode may be ESI or APCI.

For thin layer chromatography (TLC), Yantai Huanghai HSGF254 or Qingdao GF254 silica gel plates were used. The specifications of silica gel plates used for thin-layer chromatography (TLC) were 0.15 mm to 0.2 mm, and the specification of those used for separation and purification by thin layer chromatography was 0.4 mm to 0.5 mm.

For column chromatography, Yantai Huanghai silica gel of 200 to 300 mesh was generally used as a carrier.

In the following examples, unless otherwise specified, all temperatures are presented in degrees Celsius. Unless otherwise specified, various starting materials and reagents are commercially available or are synthesized according to known methods. Commercially available starting materials and reagents are used directly without further purification. Unless otherwise specified, commercial manufacturers include, but are not limited to, Aldrich Chemical Company, ABCR GmbH & Co. KG, Acros Organics, Guangzan Chemical Technology Co., Ltd., and Jingyan Chemical Technology Co., Ltd.

CD₃OD: deuterated methanol.

CDCl₃: deuterated chloroform.

DMSO-*d*₆: deuterated dimethyl sulfoxide.

Argon atmosphere refers to a reaction flask connected to an argon balloon having a volume of about 1 L.

In the examples, unless otherwise specified, solutions in reactions referred to aqueous solutions.

Compounds were purified using silica gel column chromatography and reverse-phase column chromatography. The eluent systems were selected from the group consisting of A: petroleum ether and ethyl acetate system; B: dichloromethane and methanol system; C: dichloromethane: ethyl acetate; and D: trifluoroacetic acid aqueous solution and acetonitrile system. The volume ratios of solvents varied depending on the polarity of the compound, and small amounts of acidic or basic reagents such as acetic acid or triethylamine may also be added for adjustment.

### Example 1

### 2-(2-(cyclopropanesulfonamido)pyrimidin-4-yl)-N-(5-(6-ethoxypyrazin-2-yl)pyridin-2-yl)-2,3-di hydro-1H-indene-2-carboxamide

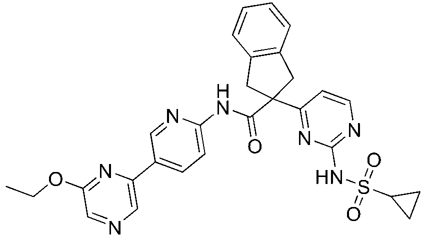

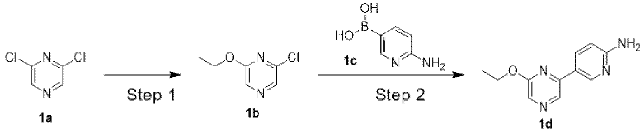

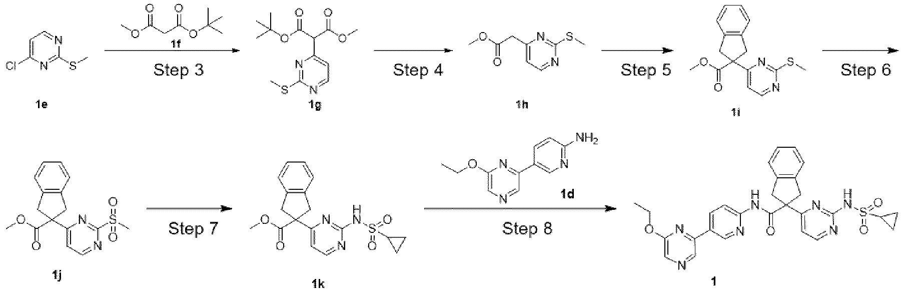

### Step 1

### 2-chloro-6-ethoxypyrazine

Under ice-water bath, sodium hydride (1.76 g, 40.61 mmol, 60% purity) was added to a solution of ethanol (1.62 g, 35.24 mmol) in tetrahydrofuran (50 mL). After 0.5 hours, 2,6-dichloropyrazine **1a** (5 g, 33.56 mmol, commercially available) was added. The mixture was heated to room temperature and stirred for 16 hours. Mass spectrometry showed that the starting material was basically completely reacted. Water (20 mL) was added to quench the reaction, and the mixture was extracted with ethyl acetate (50 mL × 3). The combined organic phase was washed with saturated sodium chloride solution (50 mL), dried over anhydrous sodium sulfate, filtered, and concentrated under reduced pressure. The obtained crude product was purified by silica gel column chromatography (eluent: System A) to obtain 2-chloro-6-ethoxypyrazine **1b** (4 g) with a yield of 75.15%.

MS m/z (ESI): 159.0 [M+1]

### Step 2

### 5-(6-ethoxypyrazin-2-yl)pyridin-2-amine

2-Chloro-6-ethoxypyrazine **1b** (2 g, 12.61 mmol), (6-aminopyridin-3-yl)boronic acid **1c** (2.09 g, 15.13 mmol, commercially available), 1,1-bis(diphenylphosphino)ferrocene palladium(II) dichloride (922.78 mg, 1.26 mmol), and potassium carbonate (5.23 g, 37.83 mmol) were added to 1,4-dioxane (60 mL) and water (15 mL). The mixture was subjected to argon exchange three times, heated to 95°C, and stirred for 3 hours. After the reaction was completed, the solvent was evaporated to dryness by rotation. The crude product was purified by silica gel column chromatography (eluent: System A) to obtain 5-(6-ethoxypyrazin-2-yl)pyridin-2-amine **1d** (3.5 g) with a yield of 96.26%.

MS m/z (ESI): 217.2 [M+1]

### Step 3

### 1-(tert-butyl) 3-methyl 2-(2-(methylthio)pyrimidin-4-yl)malonate

4-Chloro-2-(methylthio)pyrimidine **1e** (5 g, 31.13 mmol, commercially available), tert-butyl methyl malonate **1f** (7.05 g, 40.47 mmol, 6.84 mL, commercially available), and cesium carbonate (25.36 g, 77.82 mmol) were added to N,N-dimethylformamide (45.27 mL). The mixture was stirred at 70°C for 3 hours, cooled to room temperature, and quenched by adding water (50 mL). The mixture was extracted with ethyl acetate (50 mL × 3). The combined organic phase was washed with saturated sodium chloride solution (50 mL) to obtain 1-(tert-butyl) 3-methyl 2-(2-(methylthio)pyrimidin-4-yl)malonate **1g** (8 g) with a yield of 86.14%. The crude product was directly used in the next step.

MS m/z (ESI): 299.3 [M+1]

### Step 4

### methyl 2-(2-(methylthio)pyrimidin-4-yl)acetate

1-(Tert-butyl) 3-methyl 2-(2-(methylthio)pyrimidin-4-yl)malonate **1g** (9 g, 30.17 mmol) was added to dichloromethane (50 mL). Trifluoroacetic acid (34.39 g, 301.65 mmol, 23.24 mL) was slowly added dropwise, and the mixture was stirred at room temperature for 1 hour. After the reaction was completed, the mixture was concentrated under reduced pressure. The residue was purified by silica gel column chromatography (eluent: System A) to obtain methyl 2-(2-(methylthio)pyrimidin-4-yl)acetate **1h** (4 g) with a yield of 66.89%.

MS m/z (ESI): 199.2 [M+1]

### Step 5

### methyl 2-(2-(methylthio)pyrimidin-4-yl)-2,3-dihydro-1H-indene-2-carboxylate

Under ice-water bath, sodium hydride (443.90 mg, 11.10 mmol, 60% purity) was slowly added to a solution of methyl 2-(2-(methylthio)pyrimidin-4-yl)acetate **1h** (1 g, 5.04 mmol) in N,N-dimethylformamide (8 mL). After 0.5 hours, 1,2-bis(chloromethyl)benzene (971.34 mg, 5.55 mmol) was added, and the mixture was stirred at room temperature for 2 hours. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated. The residue was purified by silica gel column chromatography (eluent: System A) to obtain methyl 2-(2-(methylthio)pyrimidin-4-yl)-2,3-dihydro-1H-indene-2-carboxylate **1i** (900 mg) with a yield of 59.40%.

MS m/z (ESI): 301.0 [M+1]

### Step 6

### methyl 2-(2-(methylsulfonyl)pyrimidin-4-yl)-2,3-dihydro-1H-indene-2-carboxylate

At room temperature, meta-chloroperoxybenzoic acid (1.44 g, 8.32 mmol) was added to a solution of methyl 2-(2-(methylthio)pyrimidin-4-yl)-2,3-dihydro-1H-indene-2-carboxylate **1i** (1 g, 3.33 mmol) in dichloromethane (10 mL). After stirring at room temperature for 2 hours, saturated sodium bicarbonate solution was added to neutralize the mixture, and the mixture was extracted with dichloromethane (30 mL × 2). The combined organic phase was washed with saturated sodium chloride solution, dried over anhydrous sodium sulfate, and concentrated under reduced pressure. The residue was purified by silica gel column chromatography (eluent: System A) to obtain methyl 2-(2-(methylsulfonyl)pyrimidin-4-yl)-2,3-dihydro-1H-indene-2-carboxylate **1j** (950 mg) with a yield of 85.85%.

MS m/z (ESI): 333.1[M+1]

### Step 7

### methyl 2-(2-(cyclopropanesulfonamido)pyrimidin-4-yl)-2,3-dihydro-1H-indene-2-carboxylate

Methyl 2-(2-(methylsulfonyl)pyrimidin-4-yl)-2,3-dihydro-1H-indene-2-carboxylate **1j** (800 mg, 2.41 mmol), cyclopropanesulfonamide (437.43 mg, 3.61 mmol), and cesium carbonate (1.57 g, 4.81 mmol) were added to N-methylpyrrolidone (5 mL). The mixture was heated to 90°C and the reaction was performed for 2 hours, then cooled to room temperature. Water (30 mL) was added to the reaction mixture, and the mixture was extracted with ethyl acetate (30 mL × 2). The combined organic phase was dried over anhydrous sodium sulfate and concentrated under reduced pressure. The residue was purified by silica gel column chromatography (eluent: System A) to obtain methyl 2-(2-(cyclopropanesulfonamido)pyrimidin-4-yl)-2,3-dihydro-1H-indene-2-carboxylate **1k** (700 mg) with a yield of 77.88%.

MS m/z (ESI): 374.2 [M+1]

### Step 8

### 2-(2-(cyclopropanesulfonamido)pyrimidin-4-yl)-N-(5-(6-ethoxypyrazin-2-yl)pyridin-2-yl)-2,3-di hydro-1H-indene-2-carboxamide

Under ice-water bath, trimethylaluminum (2.0 M solution in toluene) (2 M, 187.45 µL) was added to a solution of 5-(6-ethoxypyrazin-2-yl)pyridin-2-amine **1d** (30.11 mg, 139.25 µmol) in toluene (1.95 mL). The mixture was stirred at room temperature for 1 hour, then methyl 2-(2-(cyclopropanesulfonamido)pyrimidin-4-yl)-2,3-dihydro-1H-indene-2-carboxylate **1k** (40 mg, 107.12 µmol) was added, and the mixture was heated to 90°C and the reaction was performed for 5 hours. A small amount of methanol and 1 N hydrochloric acid were added to the reaction mixture to quench the reaction, and the mixture was extracted with dichloromethane (30 mL × 2). The combined organic phase was dried over anhydrous sodium sulfate and concentrated under reduced pressure. The crude product was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 2-(2-(cyclopropanesulfonamido)pyrimidin-4-yl)-N-(5-(6-ethoxypyrazin-2-yl)pyridin-2-yl)-2,3-di hydro-1H-indene-2-carboxamide **1** (3.48 mg) with a yield of 4.60%.

MS m/z (ESI): 558.2 [M+1]
1H NMR (400 MHz, Chloroform-d) δ 9.52 (s, 1H), 8.88 (s, 1H), 8.62-8.42 (m, 2H), 8.40-8.23 (m, 2H), 8.17 (s, 1H), 7.25 (s, 1H), 7.22 - 7.14 (m, 2H), 7.07 (d, J = 5.1 Hz, 1H), 4.48 (q, J = 7.0 Hz, 2H), 4.00 (d, J = 16.0 Hz, 2H), 3.66 (d, J = 16.0 Hz, 2H), 3.21 - 3.03 (m, 1H), 1.46 (t, J = 7.0 Hz, 3H), 1.28 (s, 2H), 1.01 (d, J = 7.6 Hz, 2H).

### Example 2

### 3-(2-(cyclopropanesulfonamido)pyrimidin-4-yl)-N-(5-(6-ethoxypyrazin-2-yl)pyridin-2-yl)bicyclo [3.1.0]hexane-3-carboxamide

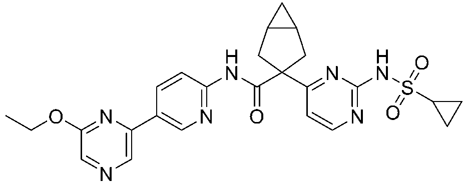

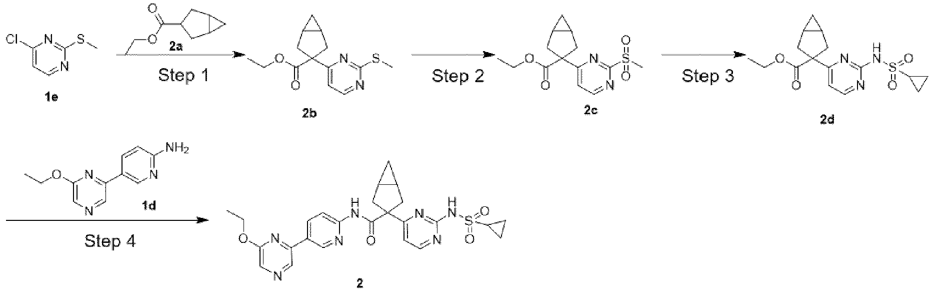

### Step 1

### ethyl 3-(2-(methylthio)pyrimidin-4-yl)bicyclo[3.1.0]hexane-3-carboxylate

At -30°C, lithium bis(trimethylsilyl)amide (1 M, 14.01 mL) was added to a solution of 4-chloro-2-(methylthio)pyrimidine **1e** (750 mg, 4.67 mmol) and ethyl bicyclo[3.1.0]hexane-3-carboxylate **2a** (900.04 mg, 5.84 mmol, commercially available) in tetrahydrofuran (10 mL). The mixture was heated to room temperature and stirred for 1 hour. After the reaction was completed, saturated ammonium chloride solution was added to quench the reaction, and the mixture was extracted with ethyl acetate (30 mL × 3). The combined organic phase was washed with saturated sodium chloride solution, dried over anhydrous sodium sulfate, and concentrated under reduced pressure. The residue was purified by silica gel column chromatography (eluent: System A) to obtain ethyl 3-(2-(methylthio)pyrimidin-4-yl)bicyclo[3.1.0]hexane-3-carboxylate **2b** (1.25 g) with a yield of 96.17%.

MS m/z (ESI): 278.9 [M+1]

### Step 2

### ethyl 3-(2-(methylsulfonyl)pyrimidin-4-yl)bicyclo[3.1.0]hexane-3-carboxylate

At room temperature, meta-chloroperoxybenzoic acid (1.12 g, 6.47 mmol) was added to a solution of ethyl 3-(2-(methylthio)pyrimidin-4-yl)bicyclo[3.1.0]hexane-3-carboxylate **2b** (600 mg, 2.16 mmol) in dichloromethane (10 mL). After stirring at room temperature for 2 hours, saturated sodium bicarbonate solution was added to neutralize the mixture, and the mixture was extracted with dichloromethane (30 mL × 3). The combined organic phase was washed with saturated sodium chloride solution, dried over anhydrous sodium sulfate, and concentrated under reduced pressure. The residue was purified by silica gel column chromatography (eluent: System A) to obtain ethyl 3-(2-(methylsulfonyl)pyrimidin-4-yl)bicyclo[3.1.0]hexane-3-carboxylate **2c** (330 mg) with a yield of 49.33%.

MS m/z (ESI): 311.0 [M+1]

### Step 3

### ethyl 3-(2-(cyclopropanesulfonamido)pyrimidin-4-yl)bicyclo[3.1.0]hexane-3-carboxylate

Ethyl 3-(2-(methylsulfonyl)pyrimidin-4-yl)bicyclo[3.1.0]hexane-3-carboxylate 2c (330 mg, 1.06 mmol) and cyclopropanesulfonamide (193.23 mg, 1.59 mmol) were added to N-methylpyrrolidone (3 mL). The mixture was heated to 80°C and stirred for 2 hours. After the reaction was completed, the mixture was cooled to room temperature. Water (30 mL) was added, and the mixture was extracted with ethyl acetate (30 mL × 3). The combined organic phase was washed with saturated sodium chloride solution, dried over anhydrous sodium sulfate, and concentrated under reduced pressure. The residue was purified by silica gel column chromatography (eluent: System A) to obtain ethyl 3-(2-(cyclopropanesulfonamido)pyrimidin-4-yl)bicyclo[3.1.0]hexane-3-carboxylate **2d** (250 mg) with a yield of 66.91%.

MS m/z (ESI): 352.0 [M+1]

### Step 4

### 3-(2-(cyclopropanesulfonamido)pyrimidin-4-yl)-N-(5-(6-ethoxypyrazin-2-yl)pyridin-2-yl)bicyclo [3.1.0]hexane-3-carboxamide

At room temperature, trimethylaluminum (2.0 M solution in toluene) (2 M, 958.54 µL) and 5-(6-ethoxypyrazin-2-yl)pyridin-2-amine **1d** (142.13 mg, 657.28 µmol) were added to toluene (2 mL). The mixture was stirred at room temperature for 1 hour. Ethyl 3-(2-(cyclopropanesulfonamido)pyrimidin-4-yl)bicyclo[3.1.0]hexane-3-carboxylate **2d** (192.49 mg, 547.74 µmol) was added, and the mixture was heated to 90°C and stirred for 2 hours. The mixture was cooled to room temperature, and a small amount of methanol was added to quench the reaction. The solvent was directly evaporated to dryness by rotation to obtain a crude product. Dilute hydrochloric acid and dichloromethane were added, and insoluble solids were removed by filtration. The filtrate was concentrated under reduced pressure. The crude product was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain

### 3-(2-(cyclopropanesulfonamido)pyrimidin-4-yl)-N-(5-(6-ethoxypyrazin-2-yl)pyridin-2-yl)bicyclo [3.1.0]hexane-3-carboxamide 2 (12 mg) with a yield of 3.70%.

MS m/z (ESI): 522.2 [M+1]
1H NMR (400 MHz, DMSO-d6) δ 10.26 (s, 1H), 9.02 (d, J = 2.4 Hz, 1H), 8.84 (s, 1H), 8.56-8.44 (m, 2H), 8.25 (s, 1H), 8.15 (d, J = 8.8 Hz, 1H), 7.13 (d, J = 5.3 Hz, 1H), 4.49-4.45 (m, 2H), 3.25 (dt, J = 8.2, 3.6 Hz, 1H), 2.87 (d, J = 13.8 Hz, 2H), 2.43 (dd, J = 13.8, 3.5 Hz, 2H), 1.40 (q, J = 7.0, 6.3 Hz, 5H), 1.07 (dt, J = 7.0, 3.5 Hz, 2H), 0.92 (td, J = 8.4, 5.9 Hz, 2H), 0.47-0.39 (m, 1H), 0.18-0.07 (m, 1H).

### Example 3

### 3-(2-(cyclopropanesulfonamido)pyrimidin-4-yl)-N-(4-(6-ethoxypyrazin-2-yl)phenyl)bicyclo[3.1. 0]hexane-3-carboxamide

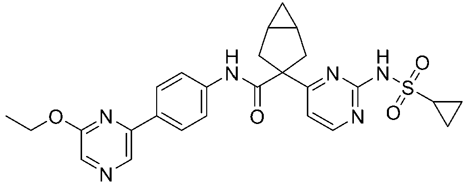

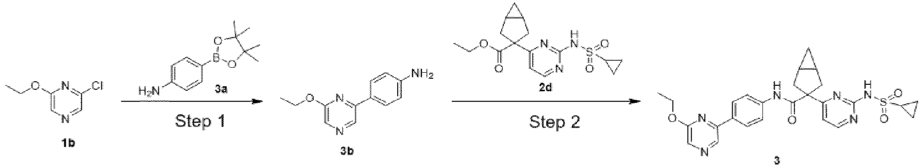

### Step 1

### 4-(6-ethoxypyrazin-2-yl)aniline

2-Chloro-6-ethoxypyrazine **1b** (1.52 g, 9.59 mmol), 4-(4,4,5,5-tetramethyl-1,3,2-dioxaborolan-2-yl)aniline **3a** (2 g, 9.13 mmol, commercially available), 1,1-bis(diphenylphosphino)ferrocene palladium(II) dichloride (667.95 mg, 912.88 µmol), and potassium carbonate (3.79 g, 27.39 mmol) were sequentially added to 1,4-dioxane (20 mL) and water (10 mL). The mixture was subjected to argon exchange three times and heated to 80°C with stirring for 2 hours. After the reaction was completed, the mixture was concentrated under reduced pressure. The residue was purified by silica gel column chromatography (eluent: System A) to obtain 4-(6-ethoxypyrazin-2-yl)aniline **3b** (1.5 g) with a yield of 76.34%.

MS m/z (ESI): 216.0 [M+1]

### Step 2

### 3-(2-(cyclopropanesulfonamido)pyrimidin-4-yl)-N-(4-(6-ethoxypyrazin-2-yl)phenyl)bicyclo[3.1. 0]hexane-3-carboxamide

At room temperature, trimethylaluminum (2.0 M toluene solution) (2 M, 958.54 µL) was added to a solution of 4-(6-ethoxypyrazin-2-yl)aniline **3b** (141.48 mg, 657.28 µmol) in toluene (2 mL). After stirring for 1 hour, ethyl 3-(2-(cyclopropanesulfonamido)pyrimidin-4-yl)bicyclo[3.1.0]hexane-3-carboxylate 2d (192.49 mg, 547.74 µmol) was added, and the mixture was heated to 90°C and stirred for 18 hours. After the reaction was completed, a small amount of methanol was added to quench the reaction, and the solvent was directly evaporated to dryness by rotation to obtain a crude product. Dilute hydrochloric acid (1 mL, 1 M) and dichloromethane (10 mL) were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(2-(cyclopropanesulfonamido)pyrimidin-4-yl)-N-(4-(6-ethoxypyrazin-2-yl)phenyl)bicyclo[3.1. 0]hexane-3-carboxamide **3** (65 mg) with a yield of 17.71%.

MS m/z (ESI): 521.2 [M+1]
1H NMR (400 MHz, DMSO-d6) δ 11.31 (s, 1H), 9.67 (s, 1H), 8.76 (s, 1H), 8.52 (d, J = 5.3 Hz, 1H), 8.18 (s, 1H), 8.12-8.03 (m, 2H), 7.75 (d, J = 8.7 Hz, 2H), 7.03 (d, J = 5.3 Hz, 1H), 4.47 (q, J = 7.0 Hz, 2H), 3.31 - 3.21 (m, 1H), 2.86 (d, J = 13.6 Hz, 2H), 2.49 - 2.38 (m, 2H), 1.41 (dt, J = 14.0, 5.5 Hz, 5H), 1.09 (dt, J = 6.7, 3.4 Hz, 2H), 0.96 (dt, J = 7.8, 3.6 Hz, 2H), 0.50 - 0.38 (m, 1H), 0.20 - 0.11 (m, 1H).

### Example 4

### (1R,5S)-6-(2-(cyclopropanesulfonamido)pyrimidin-4-yl)-N-(4-(6-ethoxypyrazin-2-yl)phenyl)-3-o xabicyclo[3.1.0]hexane-6-carboxamide

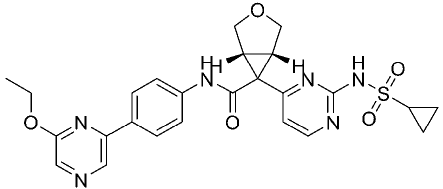

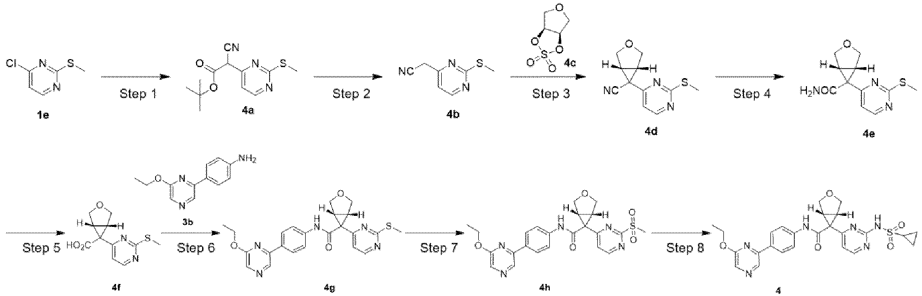

### Step 1

### tert-butyl 2-cyano-2-(2-(methylthio)pyrimidin-4-yl)acetate

Under an ice bath, a solution of tert-butyl 2-cyanoacetate (8.79 g, 62.26 mmol) in dimethyl sulfoxide (50 mL) was added dropwise to a solution of sodium hydride (2.54 g, 63.43 mmol, 60% purity) in dimethyl sulfoxide (50 mL) with continuous stirring for 20 minutes. The gas was released, and 4-chloro-2-(methylthio)pyrimidine **1e** (5 g, 31.13 mmol) was added. The mixture was heated to 70°C and stirred for 18 hours. After the reaction was completed, the generated solid was filtered, and the filter cake was dried to obtain tert-butyl 2-cyano-2-(2-(methylthio)pyrimidin-4-yl)acetate **4a** (7.6 g) with a yield of 92.02%. The crude product was directly used in the next step.

MS m/z (ESI): 266.3 [M+1]

### Step 2

### 2-(2-(methylthio)pyrimidin-4-yl)acetonitrile

At room temperature, trifluoroacetic acid (32.66 g, 286.43 mmol, 21.93 mL) was added to a solution of tert-butyl 2-cyano-2-(2-(methylthio)pyrimidin-4-yl)acetate **4a** (7.6 g, 28.64 mmol) in dichloromethane (100 mL). After stirring for 4 hours, the solvent was evaporated to dryness by rotation. Saturated sodium bicarbonate solution was added to neutralize the mixture to neutral, and the mixture was extracted with dichloromethane (30 mL × 3). The combined organic phase was washed with saturated sodium chloride solution, dried over anhydrous sodium sulfate, filtered, and evaporated to dryness by rotation. The residue was purified by silica gel column chromatography (eluent: System B) to obtain 2-(2-(methylthio)pyrimidin-4-yl)acetonitrile **4b** (3.2 g) with a yield of 67.62%.

MS m/z (ESI): 166.0 [M+1]

### Step 3

### (1R,5S)-6-(2-(methylthio)pyrimidin-4-yl)-3-oxabicyclo[3.1.0]hexane-6-carbonitrile

Under ice-water bath, (3aR,6aS)-tetrahydrofuro[3,4-d][1,3,2]dioxathiole 2,2-dioxide 4c (1.21 g, 7.26 mmol, prepared according to published patent "WO2005005398") was added to a solution of 2-(2-(methylthio)pyrimidin-4-yl)acetonitrile **4b** (1 g, 6.05 mmol) in anhydrous ethylene glycol dimethyl ether (8 mL). The mixture was stirred at room temperature for 1 hour, then heated to 60°C and stirred for 18 hours. After the reaction was completed, water (20 mL) was added to quench the reaction, and the mixture was extracted with dichloromethane (30 mL × 3). The combined organic phase was washed with saturated sodium chloride solution, dried over anhydrous sodium sulfate, filtered, and evaporated to dryness by rotation. The residue was purified by silica gel column chromatography (eluent: System B) to obtain (1R,5S)-6-(2-(methylthio)pyrimidin-4-yl)-3-oxabicyclo[3.1.0]hexane-6-carbonitrile **4d** (1.08 g) with a yield of 76.49%.

MS m/z (ESI): 234.1 [M+1]

### Step 4

### (1R,5S)-6-(2-(methylthio)pyrimidin-4-yl)-3-oxabicyclo[3.1.0]hexane-6-carboxamide

Under an ice bath, hydrogen peroxide (5 mL, 35%) was added to a solution of (1R,5S)-6-(2-(methylthio)pyrimidin-4-yl)-3-oxabicyclo[3.1.0]hexane-6-carbonitrile **4d** (1 g, 4.29 mmol) in dimethyl sulfoxide (10 mL). The mixture was stirred at room temperature for 16 hours, then poured into ice water. After stirring for 30 minutes, a solid precipitated. The solid was collected by filtration, and the filter cake was dried to obtain (1R,5S)-6-(2-(methylthio)pyrimidin-4-yl)-3-oxabicyclo[3.1.0]hexane-6-carboxamide **4e** (1 g) with a yield of 92.83%. The crude product was directly used in the next step.

MS m/z (ESI): 252.0 [M+1]

### Step 5

### (1R,5S)-6-(2-(methylthio)pyrimidin-4-yl)-3-oxabicyclo[3.1.0]hexane-6-carboxylic acid

Under ice-water bath, sodium nitrite (3.02 g, 43.77 mmol) was added to a mixed solution of acetic acid (4 mL) and acetic anhydride (8 mL) containing (1R,5S)-6-(2-(methylthio)pyrimidin-4-yl)-3-oxabicyclo[3.1.0]hexane-6-carboxamide 4e (1.1 g, 4.38 mmol). The mixture was stirred at room temperature for 16 hours, then poured into a small amount of water. A solid precipitated, and the mixture was filtered under reduced pressure. The filter cake was dried to obtain (1R,5S)-6-(2-(methylthio)pyrimidin-4-yl)-3-oxabicyclo[3.1.0]hexane-6-carboxylic acid 4f (600 mg) with a yield of 54.33%.

### Step 6

### (1R,5S)-N-(4-(6-ethoxypyrazin-2-yl)phenyl)-6-(2-(methylthio)pyrimidin-4-yl)-3-oxabicyclo[3.1. 0]hexane-6-carboxamide

Under ice-water bath, (1R,5S)-6-(2-(methylthio)pyrimidin-4-yl)-3-oxabicyclo[3.1.0]hexane-6-carboxylic acid **4f** (180 mg, 713.47 µmol), N,N,N',N'-tetramethylchloroformamidinium hexafluorophosphate (599.31 mg, 2.14 mmol), and N-methylimidazole (351.45 mg, 4.28 mmol) were added to anhydrous acetonitrile (5 mL). The mixture was stirred at room temperature for 0.5 hours, then 4-(6-ethoxypyrazin-2-yl)aniline **3b** (184 mg, 856.47 µmol) was added, and the mixture was heated to 60°C and stirred for 18 hours. After the reaction was completed, water (10 mL) was added, and the mixture was extracted with ethyl acetate (20 mL × 3). The combined organic phase was dried over anhydrous sodium sulfate, filtered, and concentrated under reduced pressure. The crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-N-(4-(6-ethoxypyrazin-2-yl)phenyl)-6-(2-(methylthio)pyrimidin-4-yl)-3-oxabicyclo[3.1. 0]hexane-6-carboxamide **4g** (120 mg) with a yield of 37.42%.

MS m/z (ESI): 449.8 [M+1]

### Step 7

### (1R,5S)-N-(4-(6-ethoxypyrazin-2-yl)phenyl)-6-(2-(methylsulfonyl)pyrimidin-4-yl)-3-oxabicyclo[ 3.1.0]hexane-6-carboxamide

At room temperature, (1R,5S)-N-(4-(6-ethoxypyrazin-2-yl)phenyl)-6-(2-(methylthio)pyrimidin-4-yl)-3-oxabicyclo[3.1. 0]hexane-6-carboxamide **4g** (120 mg, 266.95 µmol) and meta-chloroperoxybenzoic acid (108.39 mg, 533.90 µmol, 85% purity) were added to dichloromethane (3 mL). The mixture was stirred at room temperature for 18 hours. Saturated sodium bicarbonate solution was added to neutralize the mixture, and the mixture was extracted with dichloromethane (20 mL × 3). The combined organic phase was dried over anhydrous sodium sulfate, filtered, and concentrated under reduced pressure to obtain (1R,5S)-N-(4-(6-ethoxypyrazin-2-yl)phenyl)-6-(2-(methylsulfonyl)pyrimidin-4-yl)-3-oxabicyclo[ 3.1.0]hexane-6-carboxamide **4h** (60 mg) with a yield of 48.28%. The crude product was directly used in the next step.

MS m/z (ESI): 435.1 [M+1]

### Step 8

### (1R,5S)-6-(2-(cyclopropanesulfonamido)pyrimidin-4-yl)-N-(4-(6-ethoxypyrazin-2-yl)phenyl)-3-o xabicyclo[3.1.0]hexane-6-carboxamide

Cyclopropanesulfonamide (60.39 mg, 498.42 µmol), (1R,5S)-N-(4-(6-ethoxypyrazin-2-yl)phenyl)-6-(2-(methylsulfonyl)pyrimidin-4-yl)-3-oxabicyclo[ 3.1.0]hexane-6-carboxamide **4h** (120 mg, 249.21 µmol), and cesium carbonate (162.39 mg, 498.42 µmol) were added to N-methylpyrrolidone (2 mL). The mixture was heated to 90°C and stirred for 1 hour, then cooled to room temperature. The crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(2-(cyclopropanesulfonamido)pyrimidin-4-yl)-N-(4-(6-ethoxypyrazin-2-yl)phenyl)-3-o xabicyclo[3.1.0]hexane-6-carboxamide **4** (12.37 mg) with a yield of 9.50%.

MS m/z (ESI): 462.1 [M+1]
1H NMR (400 MHz, Chloroform-d) δ 8.54 (s, 1H), 8.40 (s, 1H), 8.19 - 7.96 (m, 3H), 7.73 (s, 2H), 7.00 (s, 1H), 4.54 (q, J = 7.0 Hz, 2H), 4.35 (d, J = 9.3 Hz, 2H), 3.97 (d, J = 9.2 Hz, 2H), 3.13 (s, 1H), 2.59 (s, 2H), 1.47 (t, J = 7.0 Hz, 3H), 1.33 (s, 4H).

### Example 5

### 1-(2-(cyclopropanesulfonamido)pyrimidin-4-yl)-N-(4-(6-ethoxypyrazin-2-yl)phenyl)-2,3-dihydro -1H-indene-1-carboxamide

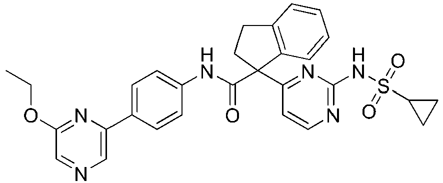

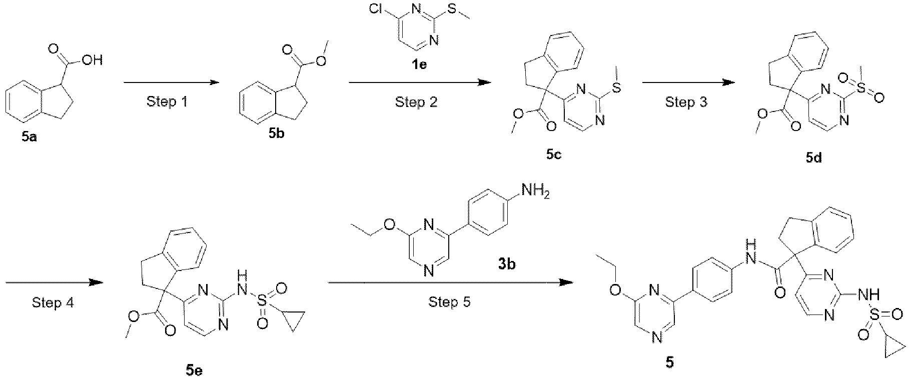

### Step 1

### methyl 2,3-dihydro-1H-indene-1-carboxylate

To a solution of 2,3-dihydro-1H-indene-1-carboxylic acid 5a (600 mg, 3.70 mmol, commercially available) in dichloromethane (9.73 mL) was added thionyl chloride (880.26 mg, 7.40 mmol, 536.74 µL). The mixture was stirred at room temperature for 2 hours. Part of the solvent was removed, and anhydrous methanol (9.73 mL) was added. The mixture was stirred at room temperature for 18 hours and concentrated under reduced pressure. Saturated sodium bicarbonate solution was added to neutralize the mixture, and the mixture was extracted with dichloromethane (20 mL × 3). The combined organic phase was dried over anhydrous sodium sulfate, filtered, and concentrated to obtain methyl 2,3-dihydro-1H-indene-1-carboxylate **5b** (560 mg) with a yield of 85.90%. The crude product was directly used in the next step.

### Step 2

### methyl 1-(2-(methylthio)pyrimidin-4-yl)-2,3-dihydro-1H-indene-1-carboxylate

At -30°C, lithium bis(trimethylsilyl)amide (1 M, 7.47 mL) was added to a solution of 4-chloro-2-(methylthio)pyrimidine **1e** (400 mg, 2.49 mmol) and methyl 2,3-dihydro-1H-indene-1-carboxylate **5b** (438.82 mg, 2.49 mmol) in tetrahydrofuran (23.07 mL). The mixture was slowly heated to room temperature and stirred for 1 hour. After the reaction was completed, saturated ammonium chloride solution was added to quench the reaction, and the mixture was extracted with ethyl acetate (20 mL × 3). The combined organic phase was dried over anhydrous sodium sulfate, filtered, and concentrated. The crude product was purified by silica gel column chromatography (eluent: System A) to obtain methyl 1-(2-(methylthio)pyrimidin-4-yl)-2,3-dihydro-1H-indene-1-carboxylate 5c (530 mg) with a yield of 70.85%.

MS m/z (ESI): 301.2 [M+1]

### Step 3

### methyl 1-(2-(methylsulfonyl)pyrimidin-4-yl)-2,3-dihydro-1H-indene-1-carboxylate

At room temperature, methyl 1-(2-(methylthio)pyrimidin-4-yl)-2,3-dihydro-1H-indene-1-carboxylate 5c (650 mg, 2.16 mmol) and meta-chloroperoxybenzoic acid (878.67 mg, 4.33 mmol, 85% purity) were added to dichloromethane (7 mL). The mixture was stirred at room temperature for 18 hours. After the reaction was completed, saturated sodium bicarbonate solution was added to neutralize the mixture, and the mixture was extracted with dichloromethane (20 mL × 3). The combined organic phase was dried over anhydrous sodium sulfate, filtered, and concentrated. The crude product was purified by silica gel column chromatography (eluent: System A) to obtain methyl 1-(2-(methylsulfonyl)pyrimidin-4-yl)-2,3-dihydro-1H-indene-1-carboxylate **5d** (460 mg) with a yield of 63.96%.

MS m/z (ESI): 333.1 [M+1]

### Step 4

### methyl 1-(2-(cyclopropanesulfonamido)pyrimidin-4-yl)-2,3-dihydro-1H-indene-1-carboxylate

Methyl 1-(2-(methylsulfonyl)pyrimidin-4-yl)-2,3-dihydro-1H-indene-1-carboxylate **5d** (460 mg, 1.38 mmol) and cyclopropanesulfonamide (335.36 mg, 2.77 mmol) were dissolved in N-methylpyrrolidone (3 mL). Cesium carbonate (901.86 mg, 2.77 mmol) was added subsequently. The mixture was heated to 90°C and stirred for 18 hours. After the reaction was completed, the mixture was cooled to room temperature, and water (20 mL) was added. The mixture was extracted with ethyl acetate (20 mL × 3). The combined organic phase was washed with saturated sodium chloride solution, dried over anhydrous sodium sulfate, filtered, and evaporated to dryness by rotation. The crude product was purified by silica gel column chromatography (eluent: System B) to obtain methyl 1-(2-(cyclopropanesulfonamido)pyrimidin-4-yl)-2,3-dihydro-1H-indene-1-carboxylate **5e** (350 mg) with a yield of 67.72%.

MS m/z (ESI): 374.1 [M+1]

### Step 5

### 1-(2-(cyclopropanesulfonamido)pyrimidin-4-yl)-N-(4-(6-ethoxypyrazin-2-yl)phenyl)-2,3-dihydro -1H-indene-1-carboxamide

4-(6-Ethoxypyrazin-2-yl)aniline **3b** (121.05 mg, 562.36 µmol) and trimethylaluminum (2.0 M toluene solution) (2 M, 702.95 µL) were dissolved in toluene (500.00 µL). The mixture was stirred at room temperature for 1 hour. Methyl 1-(2-(cyclopropanesulfonamido)pyrimidin-4-yl)-2,3-dihydro-1H-indene-1-carboxylate **5e** (175 mg, 468.63 µmol) was added, and the mixture was heated to 90°C and stirred for 5 hours. All starting materials were completely reacted. A small amount of methanol was added to quench the reaction, and the solvent was directly evaporated to dryness by rotation to obtain a crude product. Dilute hydrochloric acid (1 mL, 1 M) and dichloromethane (10 mL) were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 1-(2-(cyclopropanesulfonamido)pyrimidin-4-yl)-N-(4-(6-ethoxypyrazin-2-yl)phenyl)-2,3-dihydro -1H-indene-1-carboxamide 5 (42 mg) with a yield of 12.19%.

MS m/z (ESI): 556.8 [M+1]
1H NMR (400 MHz, Chloroform-d) δ 8.80 (s, 1H), 8.53 (d, J = 5.4 Hz, 2H), 8.06 (s, 1H), 7.97 (d, J = 8.4 Hz, 2H), 7.82 (s, 1H), 7.76 (d, J = 8.2 Hz, 2H), 7.34 (d, J = 2.9 Hz, 3H), 7.01 (d, J = 5.3 Hz, 1H), 4.55 (d, J = 7.1 Hz, 2H), 3.10 (dt, J = 17.3, 5.8 Hz, 3H), 2.98 - 2.86 (m, 2H), 1.47 (t, J = 7.0 Hz, 3H).

### Example 6

### 5-(2-(cyclopropanesulfonamido)pyrimidin-4-yl)-N-(4-(6-ethoxypyrazin-2-yl)phenyl)-4,5,6,7-tetr ahydro-1H-indazole-5-carboxamide

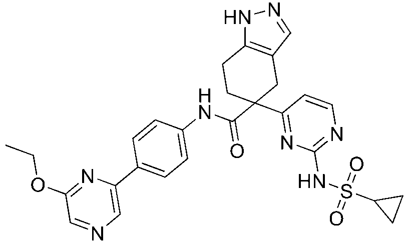

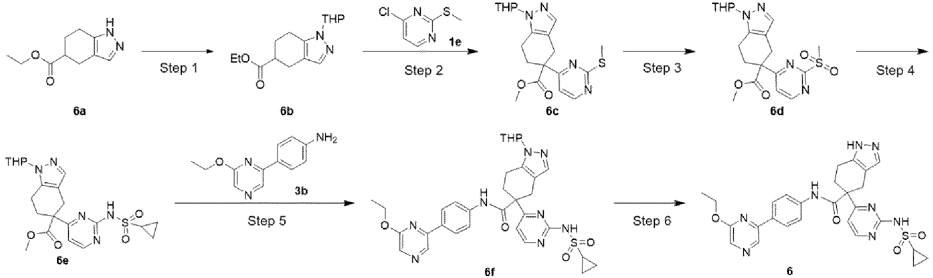

### Step 1

### ethyl 1-(tetrahydro-2H-pyran-2-yl)-4,5,6,7-tetrahydro-1H-indazole-5-carboxylate

Ethyl 4,5,6,7-tetrahydro-1H-indazole-5-carboxylate **6a** (1 g, 5.15 mmol, commercially available), 3,4-dihydro-2H-pyran (1.30 g, 15.45 mmol), and p-toluenesulfonic acid (98.85 mg, 514.85 µmol) were added to tetrahydrofuran (10 mL), and the mixture was heated to 80°C and stirred for 18 hours. After the reaction was completed, a saturated sodium bicarbonate solution was added to neutralize the mixture, followed by extraction with ethyl acetate (20 mL×3). The combined organic phases were washed with a saturated sodium chloride solution, dried over anhydrous sodium sulfate, and concentrated under reduced pressure. The crude product was purified by silica gel column chromatography (eluent: System A) to obtain ethyl 1-(tetrahydro-2H-pyran-2-yl)-4,5,6,7-tetrahydro-1H-indazole-5-carboxylate **6b** (1.2 g) with a yield of 83.74%.

MS m/z (ESI): 279.1 [M+1]

### Step 2

### methyl 5-(2-(methylthio)pyrimidin-4-yl)-1-(tetrahydro-2H-pyran-2-yl)-4,5,6,7-tetrahydro-1H-indazole-5 -carboxylate

At -30°C, lithium bis(trimethylsilyl)amide (1 M, 11.21 mL) was added to a solution of 4-chloro-2-(methylthio)pyrimidine **1e** (0.6 g, 3.74 mmol) and ethyl 1-(tetrahydro-2H-pyran-2-yl)-4,5,6,7-tetrahydro-1H-indazole-5-carboxylate 6b (1.25 g, 4.48 mmol) in tetrahydrofuran (7 mL), and then the mixture was heated to room temperature and stirred for 1 hour. After the starting materials were completely reacted, an aqueous solution of saturated ammonium chloride was added to quench the reaction, followed by extraction with ethyl acetate (20 mL×3). The combined organic phases were washed with a saturated sodium chloride solution, dried over anhydrous sodium sulfate, filtered, and evaporated to dryness by rotation. The crude product was purified by silica gel column chromatography (eluent: System A) to obtain methyl 5-(2-(methylthio)pyrimidin-4-yl)-1-(tetrahydro-2H-pyran-2-yl)-4,5,6,7-tetrahydro-1H-indazole-5 -carboxylate **6c** (831 mg) with a yield of 55.27%. MS m/z (ESI): 403.2 [M+1]

### Step 3

### methyl 5-(2-(methylsulfonyl)pyrimidin-4-yl)-1-(tetrahydro-2H-pyran-2-yl)-4,5,6,7-tetrahydro-1H-indazo le-5-carboxylate

Methyl 5-(2-(methylthio)pyrimidin-4-yl)-1-(tetrahydro-2H-pyran-2-yl)-4,5,6,7-tetrahydro-1H-indazole-5 -carboxylate **6c** (980 mg, 2.43 mmol) and meta-chloroperoxybenzoic acid (988.61 mg, 4.87 mmol, 85% purity) were dissolved in dichloromethane (10 mL) and stirred at room temperature for 18 hours. After the reaction was completed, a saturated sodium bicarbonate solution was added to neutralize the mixture, followed by extraction with dichloromethane (20 mL×3). The combined organic phases were dried over anhydrous sodium sulfate, filtered, and concentrated. The crude product was purified by silica gel column chromatography (eluent: System A) to obtain methyl 5-(2-(methylsulfonyl)pyrimidin-4-yl)-1-(tetrahydro-2H-pyran-2-yl)-4,5,6,7-tetrahydro-1H-indazo le-5-carboxylate 6d (500 mg) with a yield of 47.26%.

MS m/z (ESI): 435.1 [M+1]

### Step 4

### methyl 5-(2-(cyclopropanesulfonamido)pyrimidin-4-yl)-1-(tetrahydro-2H-pyran-2-yl)-4,5,6,7-tetrahydro-1H-indazole-5-carboxylate

Methyl 5-(2-(methylsulfonyl)pyrimidin-4-yl)-1-(tetrahydro-2H-pyran-2-yl)-4,5,6,7-tetrahydro-1H-indazo le-5-carboxylate **6d** (963.18 mg, 2.30 mmol) and cyclopropanesulfonamide (557.36 mg, 4.67 mmol) were added to N-methylpyrrolidone (3 mL), followed by addition of cesium carbonate (1.50 g, 4.60 mmol), and the mixture was heated to 90°C and stirred for 2 hours. After the reaction was completed, water (20 mL) was added, followed by extraction with ethyl acetate (20 mL×3). The combined organic phases were washed with a saturated sodium chloride solution, dried over anhydrous sodium sulfate, filtered, and evaporated to dryness by rotation. The crude product was purified by silica gel column chromatography (eluent: System A) to obtain methyl 5-(2-(cyclopropanesulfonamido)pyrimidin-4-yl)-1-(tetrahydro-2H-pyran-2-yl)-4,5,6,7-tetrahydro-1H-indazole-5-carboxylate **6e** (800 mg) with a yield of 75.32%.

MS m/z (ESI): 462.1 [M+1]

### Step 5

### 5-(2-(cyclopropanesulfonamido)pyrimidin-4-yl)-N-(4-(6-ethoxypyrazin-2-yl)phenyl)-1-(tetrahydr o-2H-pyran-2-yl)-4,5,6,7-tetrahydro-1H-indazole-5-carboxamide

4-(6-Ethoxypyrazin-2-yl)aniline **3b** (195.88 mg, 910.01 µmol) and trimethylaluminum (2.0 M solution in toluene) (2 M, 1.14 mL) were dissolved in toluene (65.0 µL) and stirred at room temperature for 1 hour, followed by addition of methyl 5-(2-(cyclopropanesulfonamido)pyrimidin-4-yl)-1-(tetrahydro-2H-pyran-2-yl)-4,5,6,7-tetrahydro-1H-indazole-5-carboxylate **6e** (350 mg, 758.34 µmol), and the mixture was heated to 90°C and stirred for 18 hours. After the reaction was completed, a small amount of methanol was added to quench the reaction, followed by addition of dilute hydrochloric acid and dichloromethane. The insoluble solid was removed by filtration, and the filtrate was concentrated under reduced pressure. The crude product was purified by preparative liquid chromatography (separation column: AKZONOBEL Kromasil; 250×21.2 mm I.D.; 5 µm, 20 mL/min; mobile phase A: 0.05% TFA+H₂O, mobile phase B: CH₃CN) to obtain 5-(2-(cyclopropanesulfonamido)pyrimidin-4-yl)-N-(4-(6-ethoxypyrazin-2-yl)phenyl)-1-(tetrahydr o-2H-pyran-2-yl)-4,5,6,7-tetrahydro-1H-indazole-5-carboxamide **6f** (150 mg) with a yield of 30.68%.

MS m/z (ESI): 645.1 [M+1]

### Step 6

### 5-(2-(cyclopropanesulfonamido)pyrimidin-4-yl)-N-(4-(6-ethoxypyrazin-2-yl)phenyl)-4,5,6,7-tetr ahydro-1H-indazole-5-carboxamide

At room temperature, 5-(2-(cyclopropanesulfonamido)pyrimidin-4-yl)-N-(4-(6-ethoxypyrazin-2-yl)phenyl)-1-(tetrahydr o-2H-pyran-2-yl)-4,5,6,7-tetrahydro-1H-indazole-5-carboxamide **6f** (100 mg, 155.10 µmol) and trifluoroacetic acid (1 mL) were added to dichloromethane (1 mL), and the mixture was stirred at room temperature for 4 hours. After the reaction was completed, the solvent was removed by concentration under reduced pressure, and the crude product was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain 5-(2-(cyclopropanesulfonamido)pyrimidin-4-yl)-N-(4-(6-ethoxypyrazin-2-yl)phenyl)-4,5,6,7-tetr ahydro-1H-indazole-5-carboxamide 6 (27 mg) with a yield of 24.51%.

MS m/z (ESI): 560.8 [M+1]
1H NMR (400 MHz, DMSO-d6) δ 9.53 (s, 1H), 8.74 (s, 1H), 8.58 (d, J = 5.3 Hz, 1H), 8.17 (s, 1H), 8.05 (d, J = 8.4 Hz, 2H), 7.73 (d, J = 8.4 Hz, 2H), 7.54 (s, 1H), 7.15 (d, J = 5.3 Hz, 1H), 4.46 (q, J = 7.1 Hz, 3H), 3.35 - 3.12 (m, 3H), 2.71 (s, 1H), 2.57 (d, J = 18.1 Hz, 3H), 1.39 (t, J = 7.1 Hz, 3H), 1.05 (dq, J = 10.4, 5.0 Hz, 2H), 0.94 - 0.74 (m, 2H).

### Example 7

### 5-(2-(cyclopropanesulfonamido)pyrimidin-4-yl)-N-(4-(6-ethoxypyrazin-2-yl)phenyl)-1-methyl-4, 5,6,7-tetrahydro-1H-indazole-5-carboxamide

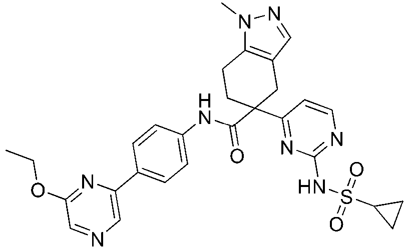

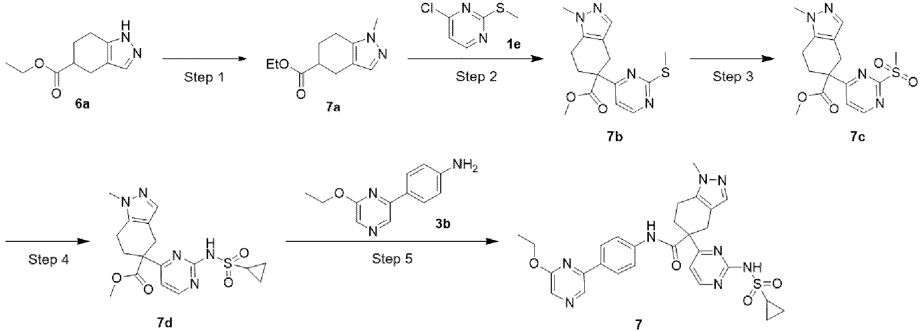

### Step 1

### ethyl 1-methyl-4,5,6,7-tetrahydro-1H-indazole-5-carboxylate

Ethyl 4,5,6,7-tetrahydro-1H-indazole-5-carboxylate 6a (1.10 g, 7.72 mmol, 480.77 µL), iodomethane (1.1 g, 7.72 mmol), and cesium carbonate (3.35 g, 10.30 mmol) were added to N,N-dimethylformamide (5 mL), and the mixture was stirred at room temperature for 18 hours. After the reaction was completed, water (20 mL) was added, and the mixture was extracted with ethyl acetate (20 mL×3). The combined organic phase was washed with saturated sodium chloride solution, dried over anhydrous sodium sulfate, filtered, and evaporated to dryness by rotation. The crude product was separated by silica gel column chromatography (eluent: System A) to obtain ethyl 1-methyl-4,5,6,7-tetrahydro-1H-indazole-5-carboxylate **7a** (1 g) with a yield of 93.26%.

MS m/z (ESI): 209.1 [M+1]

### Step 2

### methyl 1-methyl-5-(2-(methylthio)pyrimidin-4-yl)-4,5,6,7-tetrahydro-1H-indazole-5-carboxylate

At -30°C, lithium bis(trimethylsilyl)amide (1 M, 16.81 mL) was added to a solution of 4-chloro-2-(methylthio)pyrimidine **1e** (900 mg, 5.60 mmol) and ethyl 1-methyl-4,5,6,7-tetrahydro-1H-indazole-5-carboxylate **7a** (1.17 g, 5.60 mmol) in tetrahydrofuran (10 mL), and the mixture was slowly heated to room temperature and stirred for 1 hour. After the reaction was completed, saturated ammonium chloride solution (20 mL) was added to quench the reaction, and the mixture was extracted with ethyl acetate (20 mL×3). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 1-methyl-5-(2-(methylthio)pyrimidin-4-yl)-4,5,6,7-tetrahydro-1H-indazole-5-carboxylate **7b** (800 mg) with a yield of 42.95%.

MS m/z (ESI): 333.9 [M+1]

### Step 3

### methyl 1-methyl-5-(2-(methylsulfonyl)pyrimidin-4-yl)-4,5,6,7-tetrahydro-1H-indazole-5-carboxylate

Methyl 1-methyl-5-(2-(methylthio)pyrimidin-4-yl)-4,5,6,7-tetrahydro-1H-indazole-5-carboxylate 7b (800 mg, 2.41 mmol) and meta-chloroperoxybenzoic acid (977.19 mg, 4.81 mmol, 85% purity) were dissolved in dichloromethane (10 mL), and the mixture was stirred at room temperature for 18 hours. After the reaction was completed, saturated sodium bicarbonate solution was added to neutralize the mixture, and the mixture was extracted with dichloromethane (20 mL×3). The combined organic phase was washed with saturated sodium chloride solution, dried over anhydrous sodium sulfate, filtered, and evaporated to dryness by rotation to obtain methyl 1-methyl-5-(2-(methylsulfonyl)pyrimidin-4-yl)-4,5,6,7-tetrahydro-1H-indazole-5-carboxylate **7c** (400 mg) with a yield of 45.61%, and the crude product was directly used in the next step.

MS m/z (ESI): 365.1 [M+1]

### Step 4

### methyl 5-(2-(cyclopropanesulfonamido)pyrimidin-4-yl)-1-methyl-4,5,6,7-tetrahydro-1H-indazole-5-carb oxylate

Cyclopropanesulfonamide (532.4 mg, 4.40 mmol) and methyl 1-methyl-5-(2-(methylsulfonyl)pyrimidin-4-yl)-4,5,6,7-tetrahydro-1H-indazole-5-carboxylate **7c** (764.88 mg, 2.20 mmol) were dissolved in N-methylpyrrolidone (4 mL), followed by addition of cesium carbonate (1.43 g, 4.39 mmol). The reaction was heated to 90°C and stirred for 1 hour. After the reaction was complete, the mixture was cooled to room temperature, water (20 mL) was added, and the mixture was extracted with ethyl acetate (20 mL × 3). The combined organic phases were washed with saturated sodium chloride solution, dried over anhydrous sodium sulfate, filtered, and evaporated to dryness by rotation. The crude product was purified by silica gel column chromatography (eluent: System A) to obtain methyl 5-(2-(cyclopropanesulfonamido)pyrimidin-4-yl)-1-methyl-4,5,6,7-tetrahydro-1H-indazole-5-carb oxylate **7d** (750 mg) with a yield of 84.26%.

MS m/z (ESI): 374.1 [M+1]

### Step 5

### 5-(2-(cyclopropanesulfonamido)pyrimidin-4-yl)-N-(4-(6-ethoxypyrazin-2-yl)phenyl)-1-methyl-4, 5,6,7-tetrahydro-1H-indazole-5-carboxamide

At room temperature, 4-(6-ethoxypyrazin-2-yl)aniline **3b** (195.88 mg, 910.01 µmol) was dissolved in toluene (3 mL), followed by addition of trimethylaluminum (2.0 M solution in toluene) (2 M, 1.14 mL). After stirring at room temperature for 1 hour, methyl 5-(2-(cyclopropanesulfonamido)pyrimidin-4-yl)-1-methyl-4,5,6,7-tetrahydro-1H-indazole-5-carb oxylate 7d (296.85 mg, 758.34 µmol) was added. The mixture was heated to 90°C and stirred for 18 hours. After the reaction was complete, a small amount of methanol was added to quench the reaction, and the solvent was directly evaporated to dryness by rotation to obtain the crude product. Dilute hydrochloric acid (1 mL, 1 M) and dichloromethane (10 mL) were added, insoluble solid was removed by filtration, and the filtrate was concentrated under reduced pressure. The crude product was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 5-(2-(cyclopropanesulfonamido)pyrimidin-4-yl)-N-(4-(6-ethoxypyrazin-2-yl)phenyl)-1-methyl-4, 5,6,7-tetrahydro-1H-indazole-5-carboxamide 7 (98 mg) with a yield of 16.49%.

MS m/z (ESI): 574.8 [M+1]

### Example 8

### (1R,5S)-6-(2-(cyclopropanesulfonamido)pyrimidin-4-yl)-N-(4-(6-ethoxypyrazin-2-yl)phenyl)bic yclo[3.1.0]hexane-6-carboxamide

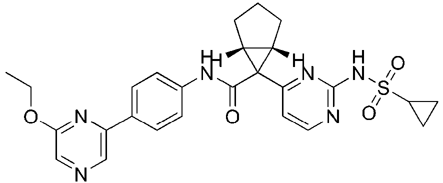

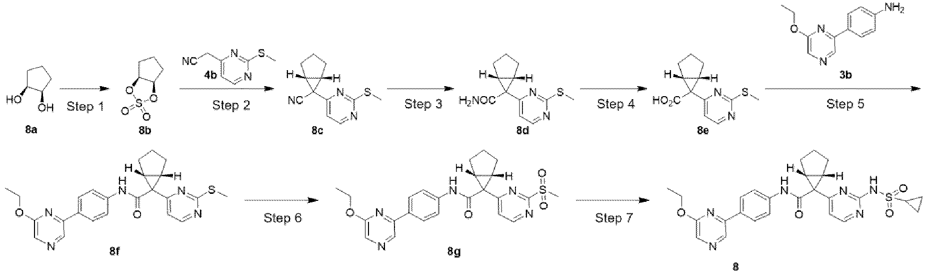

### Step 1

### (3aR,6aS)-tetrahydro-4H-cyclopenta[d][1,3,2]dioxathiole 2,2-dioxide

To a solution of (1R,2S)-cyclopentane-1,2-diol **8a** (1.96 g, 19.21 mmol, commercially available) in ethyl acetate (20 mL) was added triethylamine (9.72 g, 96.06 mmol, 13.50 mL). The reaction was cooled to -50°C, and a solution of sulfuryl chloride (3.37 g, 24.97 mmol, 2.02 mL) in ethyl acetate (2 mL) was added dropwise. The reaction was slowly heated to room temperature and stirred for 18 hours. Solid was removed by filtration, and the filtrate was evaporated to dryness by rotation. The crude product was purified by silica gel column chromatography (eluent: System A) to obtain (3aR,6aS)-tetrahydro-4H-cyclopenta[d][1,3,2]dioxathiole 2,2-dioxide **8b** (1.4 g) with a yield of 44.39%.

### Step 2

### (1R,5S)-6-(2-(methylthio)pyrimidin-4-yl)bicyclo[3.1.0]hexane-6-carbonitrile

Under ice-water bath, sodium hydride (387.37 mg, 9.68 mmol, 1.61 µL, 60% purity) was added to a solution of 2-(2-(methylthio)pyrimidin-4-yl)acetonitrile 4b (800 mg, 4.84 mmol) in anhydrous dimethoxyethane (32 mL). After stirring for 10 minutes, (3aR,6aS)-tetrahydro-4H-cyclopenta[d][1,3,2]dioxathiole 2,2-dioxide **8b** (953.98 mg, 5.81 mmol) was added. The mixture was stirred at room temperature for 1 hour, then heated to 60°C and stirred for an additional 18 hours. Water (10 mL) was added to quench the reaction, and the mixture was extracted with dichloromethane (20 mL × 3). The combined organic phases were washed with saturated sodium chloride solution, dried over anhydrous sodium sulfate, filtered, and evaporated to dryness by rotation. The crude product was purified by silica gel column chromatography (eluent: System A) to obtain (1R,5S)-6-(2-(methylthio)pyrimidin-4-yl)bicyclo[3.1.0]hexane-6-carbonitrile 8c (790 mg) with a yield of 70.53%.

MS m/z (ESI): 231.9 [M+1]

### Step 3

### (1R,5S)-6-(2-(methylthio)pyrimidin-4-yl)bicyclo[3.1.0]hexane-6-carboxamide

Under ice-water bath, (1R,5S)-6-(2-(methylthio)pyrimidin-4-yl)bicyclo[3.1.0]hexane-6-carbonitrile **8c** (980 mg, 4.24 mmol) was dissolved in dimethyl sulfoxide (10 mL), followed by addition of hydrogen peroxide (5 mL, 35%). The mixture was stirred at room temperature for 18 hours, then heated to 50°C and stirred for an additional 2 hours, after which the starting material was completely consumed. The reaction mixture was poured into ice-water (20 mL), and a solid precipitated after stirring. The mixture was filtered under reduced pressure, and the filter cake was dried to obtain (1R,5S)-6-(2-(methylthio)pyrimidin-4-yl)bicyclo[3.1.0]hexane-6-carboxamide **8d** (700 mg) with a yield of 66.27%. The crude product was directly used in the next step.

MS m/z (ESI): 249.8 [M+1]

### Step 4

### (1R,5S)-6-(2-(methylthio)pyrimidin-4-yl)bicyclo[3.1.0]hexane-6-carboxylic acid

To a solution of (1R,5S)-6-(2-(methylthio)pyrimidin-4-yl)bicyclo[3.1.0]hexane-6-carboxamide **8d** (700 mg, 2.81 mmol) in acetic acid (3 mL) and acetic anhydride (6 mL) in ice-water bath was added sodium nitrite (1.94 g, 28.08 mmol), and the mixture was stirred at room temperature for 18 hours. Water (10 mL) was added to the reaction mixture, and the mixture was extracted with ethyl acetate (20 mL×3). The combined organic phases were washed with saturated sodium chloride solution, dried over anhydrous sodium sulfate, filtered, and evaporated to dryness by rotation to obtain (1R,5S)-6-(2-(methylthio)pyrimidin-4-yl)bicyclo[3.1.0]hexane-6-carboxylic acid **8e** (650 mg) with a yield of 92.49%. The crude product was used directly in the next step.

MS m/z (ESI): 251.2 [M+1]

### Step 5

### (1R,5S)-N-(4-(6-ethoxypyrazin-2-yl)phenyl)-6-(2-(methylthio)pyrimidin-4-yl)bicyclo[3.1.0]hexa ne-6-carboxamide

(1R,5S)-6-(2-(methylthio)pyrimidin-4-yl)bicyclo[3.1.0]hexane-6-carboxylic acid **8e** (250 mg, 998.74 µmol), N,N,N',N'-tetramethylchloroformamidinium hexafluorophosphate (838.94 mg, 3.00 mmol), and N-methylimidazole (327.99 mg, 3.99 mmol) were added to N-methylpyrrolidone (3 mL) at room temperature. After stirring for half an hour, 4-(6-ethoxypyrazin-2-yl)aniline **3b** (257 mg, 1.2 mmol) was added, and the reaction was heated to 60°C and stirred for 18 hours, then heated to 120°C and stirred for 3 hours. After completion of the reaction, water (20 mL) was added to the reaction mixture, and the mixture was extracted with ethyl acetate (20 mL×3). The combined organic phases were washed with saturated sodium chloride solution, dried over anhydrous sodium sulfate, filtered, and evaporated to dryness by rotation. The crude product was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (1R,5S)-N-(4-(6-ethoxypyrazin-2-yl)phenyl)-6-(2-(methylthio)pyrimidin-4-yl)bicyclo[3.1.0]hexa ne-6-carboxamide **8f** (50 mg) with a yield of 11.19%.

MS m/z (ESI): 448.2 [M+1]

### Step 6

### (1R,5S)-N-(4-(6-ethoxypyrazin-2-yl)phenyl)-6-(2-(methylsulfonyl)pyrimidin-4-yl)bicyclo[3.1.0] hexane-6-carboxamide

(1R,5S)-N-(4-(6-ethoxypyrazin-2-yl)phenyl)-6-(2-(methylthio)pyrimidin-4-yl)bicyclo[3 .1.0]hexane-6-carboxamide **8f** (50 mg, 111.72 µmol) and meta-chloroperoxybenzoic acid (45.36 mg, 223.44 µmol, 85% purity) were added to dichloromethane (2 mL) and stirred at room temperature for 18 hours. After completion of the reaction, saturated sodium bicarbonate solution was added to neutralize the mixture until the mixture became neutral, and the mixture was extracted with dichloromethane (20 mL×3). The combined organic phases were washed with saturated sodium chloride solution, dried over anhydrous sodium sulfate, filtered, and evaporated to dryness by rotation to obtain (1R,5S)-N-(4-(6-ethoxypyrazin-2-yl)phenyl)-6-(2-(methylsulfonyl)pyrimidin-4-yl)bicyclo[3.1.0] hexane-6-carboxamide **8g** (51 mg) with a yield of 98.48%. The crude product was used directly in the next step.

MS m/z (ESI): 464.9 [M+1]

### Step 7

### (1R,5S)-6-(2-(cyclopropanesulfonamido)pyrimidin-4-yl)-N-(4-(6-ethoxypyrazin-2-yl)phenyl)bic yclo[3.1.0]hexane-6-carboxamide

Cyclopropanesulfonamide (26.66 mg, 220.04 µmol), (1R,5S)-N-(4-(6-ethoxypyrazin-2-yl)phenyl)-6-(2-(methylsulfonyl)pyrimidin-4-yl)bicyclo[3.1.0] hexane-6-carboxamide **8g** (51 mg, 110.02 µmol), and cesium carbonate (71.69 mg, 220.04 µmol) were added to N-methylpyrrolidone (2 mL), and the reaction was heated to 90°C and stirred for 1 hour. After completion of the reaction, the mixture was cooled to room temperature, and water (20 mL) was added. The mixture was extracted with ethyl acetate (20 mL×3). The combined organic phases were washed with saturated sodium chloride solution, dried over anhydrous sodium sulfate, filtered, and evaporated to dryness by rotation. The crude product was purified by preparative liquid chromatography (separation column AKZONOBEL Kromasil; 250×21.2 mm I.D.; 5 µm, 20 mL/min; mobile phase A: 0.05% TFA+H₂O, mobile phase B: CH₃CN) to obtain (1R,5S)-6-(2-(cyclopropanesulfonamido)pyrimidin-4-yl)-N-(4-(6-ethoxypyrazin-2-yl)phenyl)bic yclo[3.1.0]hexane-6-carboxamide **8** (30 mg) with a yield of 40.22%.

MS m/z (ESI): 520.9 [M+1]
1H NMR (400 MHz, DMSO-d6) δ 11.16 (s, 1H), 10.49 (s, 1H), 8.76 (s, 1H), 8.41 (d, J = 5.5 Hz, 1H), 8.19 (s, 1H), 8.10 (d, J = 8.5 Hz, 2H), 7.84 (d, J = 8.4 Hz, 2H), 6.87 (d, J = 5.4 Hz, 1H), 4.48 (q, J = 7.0 Hz, 2H), 3.20 (dt, J = 8.2, 3.6 Hz, 1H), 2.25 (d, J = 2.9 Hz, 2H), 2.13 (dd, J = 13.3, 8.5 Hz, 2H), 2.06 - 1.95 (m, 2H), 1.74 - 1.58 (m, 1H), 1.40 (t, J = 7.0 Hz, 3H), 1.24 (d, J = 9.0 Hz, 2H), 1.04 (q, J = 4.2 Hz, 2H), 0.94 (dq, J = 7.6, 4.9, 4.5 Hz, 2H).

### Example 9

### 6-(2-(cyclopropanesulfonamido)pyrimidin-4-yl)-N-(4-(6-ethoxypyrazin-2-yl)phenyl)-3-azabicycl o[3.1.0]hexane-6-carboxamide

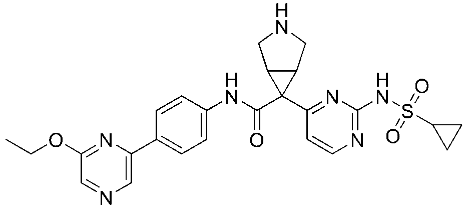

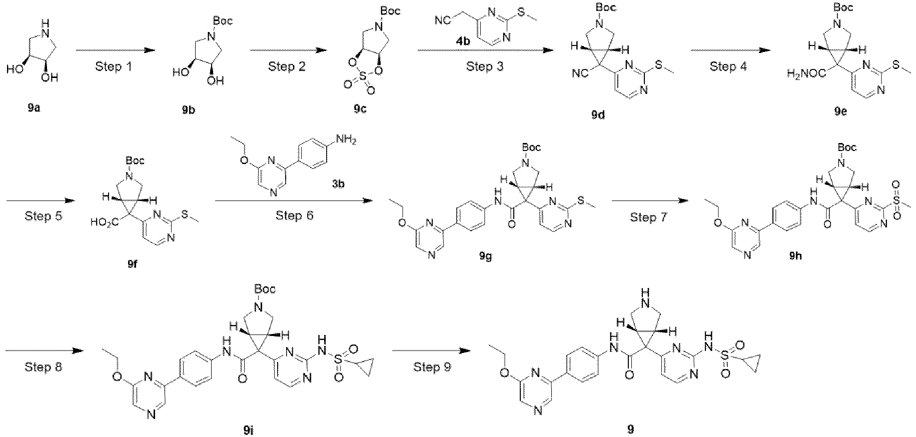

### Step 1

### tert-butyl (3S,4R)-3,4-dihydroxypyrrolidine-1-carboxylate

(3S,4R)-pyrrolidin-3,4-diol **9a** (3 g, 21.49 mmol, commercially available), di-tert-butyl dicarbonate (7.04 g, 32.24 mmol), and sodium bicarbonate (18.06 g, 214.93 mmol) were dissolved in 1,4-dioxane (30 mL) and water (30 mL) at room temperature and stirred at room temperature for 18 hours. Insoluble solid was removed by filtration, and the filtrate was directly evaporated to dryness by rotation. The crude product was purified by silica gel column chromatography (eluent: System A) to obtain tert-butyl (3S,4R)-3,4-dihydroxypyrrolidine-1-carboxylate **9b** (3.9 g) with a yield of 89.28%.

MS m/z (ESI): 147.1 [M+1-56]

### Step 2

### tert-butyl (3aR,6aS)-tetrahydro-5H-[1,3,2]dioxathiolo[4,5-c]pyrrole-5-carboxylate 2,2-dioxide

At -50°C, a solution of sulfonyl chloride (3.37 g, 24.97 mmol, 2.02 mL) in ethyl acetate (5 mL) was added dropwise to a solution of tert-butyl (3S,4R)-3,4-dihydroxypyrrolidine-1-carboxylate **9b** (3.90 g, 19.21 mmol) and triethylamine (9.72 g, 96.06 mmol, 13.50 mL) in ethyl acetate (20 mL). The reaction mixture was allowed to slowly warm to room temperature and was stirred for 18 h. Upon completion of the reaction, solids were removed by filtration, and the filtrate was concentrated under reduced pressure. The crude product was purified by silica gel column chromatography (eluent: System A) to obtain tert-butyl (3aR,6aS)-tetrahydro-5H-[1,3,2]dioxathiolo[4,5-c]pyrrole-5-carboxylate 2,2-dioxide **9c** (3 g) with a yield of 58.86%.

### Step 3

### tert-butyl (1R,5S)-6-cyano-6-(2-(methylthio)pyrimidin-4-yl)-3-azabicyclo[3.1.0]hexane-3-carboxylate

Under ice-water bath, sodium hydride (314.74 mg, 7.87 mmol, 1.61 µL, 60% purity) was added to a solution of 2-(2-(methylthio)pyrimidin-4-yl)acetonitrile **4b** (650 mg, 3.93 mmol) in anhydrous dimethoxyethane (28 mL). After stirring for ten minutes, tert-butyl (3aR,6aS)-tetrahydro-5H-[1,3,2]dioxathiolo[4,5-c]pyrrole-5-carboxylate 2,2-dioxide **9c** (1.57 g, 5.90 mmol) was added. The mixture was stirred at room temperature for 1 h, then heated to 60°C and stirred for 18 h. Upon completion of the reaction, water (10 mL) was added to quench the reaction, and the mixture was extracted with dichloromethane (20 mL × 3). The combined organic phases were washed with saturated sodium chloride solution, dried over anhydrous sodium sulfate, filtered, and concentrated under reduced pressure. The crude product was purified by silica gel column chromatography (eluent: System A) to obtain tert-butyl (1R,5S)-6-cyano-6-(2-(methylthio)pyrimidin-4-yl)-3-azabicyclo[3.1.0]hexane-3-carboxylate **9d** (550 mg) with a yield of 29.44%.

MS m/z (ESI): 333.1 [M+1]

### Step 4

### tert-butyl (1R,5S)-6-carbamoyl-6-(2-(methylthio)pyrimidin-4-yl)-3-azabicyclo[3.1.0]hexane-3-carboxylate

Under ice-water bath, sodium hydroxide (144.40 mg, 3.61 mmol) was added to a solution of tert-butyl (1R,5S)-6-cyano-6-(2-(methylthio)pyrimidin-4-yl)-3-azabicyclo[3.1.0]hexane-3-carboxylate **9d** (600 mg, 1.80 mmol) in dimethyl sulfoxide (3 mL). After stirring for ten minutes, hydrogen peroxide (1.5 mL, 35%) was added, and the mixture was stirred at room temperature for 3 h. Water (20 mL) was added to quench the reaction, and the mixture was filtered under reduced pressure. The filter cake was dried, and the crude product was purified by silica gel column chromatography (eluent: System A) to obtain tert-butyl (1R,5S)-6-carbamoyl-6-(2-(methylthio)pyrimidin-4-yl)-3-azabicyclo[3.1.0]hexane-3-carboxylate **9e** (410 mg) with a yield of 64.82%.

MS m/z (ESI): 351.0 [M+1]

### Step 5

### (1R,5S)-3-(tert-butoxycarbonyl)-6-(2-(methylthio)pyrimidin-4-yl)-3-azabicyclo[3.1.0]hexane-6-c arboxylic acid

Under ice-water bath, sodium nitrite (511.94 mg, 7.42 mmol) was added to a solution of tert-butyl (1R,5S)-6-carbamoyl-6-(2-(methylthio)pyrimidin-4-yl)-3-azabicyclo[3.1.0]hexane-3-carboxylate **9e** (260 mg, 741.94 µmol), acetic acid (1.50 mL), and acetic anhydride (3 mL) in dimethyl sulfoxide (3 mL). The mixture was stirred at room temperature for 18 h. Upon completion of the reaction, the reaction mixture was poured into water, neutralized with saturated sodium bicarbonate solution, acidified with citric acid, and extracted with ethyl acetate (20 mL × 3). The combined organic phases were washed with saturated sodium chloride solution, dried over anhydrous sodium sulfate, filtered, and concentrated. The crude product was purified by silica gel column chromatography (eluent: System A) to obtain (1R,5S)-3-(tert-butoxycarbonyl)-6-(2-(methylthio)pyrimidin-4-yl)-3-azabicyclo[3.1.0]hexane-6-c arboxylic acid **9f** (150 mg) with a yield of 57.53%.

### Step 6

### tert-butyl (1R,5S)-6-((4-(6-ethoxypyrazin-2-yl)phenyl)carbamoyl)-6-(2-(methylthio)pyrimidin-4-yl)-3-azab icyclo[3.1.0]hexane-3-carboxylate

Under ice-water bath, (1R,5S)-3-(tert-butoxycarbonyl)-6-(2-(methylthio)pyrimidin-4-yl)-3-azabicyclo[3.1.0]hexane-6-c arboxylic acid **9f** (170 mg, 483.75 µmol) was dissolved in N-methylpyrrolidone (2 mL), followed by addition of N,N,N',N'-tetramethylchloroformamidinium hexafluorophosphate (406.35 mg, 1.45 mmol) and N-methylimidazole (238.30 mg, 2.90 mmol). The mixture was stirred at room temperature for half an hour, then 4-(6-ethoxypyrazin-2-yl)aniline **3b** (114 mg, 532.75 µmol) was added. The temperature was raised to 100°C, and the mixture was stirred for 18 h. Upon completion of the reaction, water (20 mL) was added to the reaction mixture, which was extracted with ethyl acetate (20 mL × 3). The combined organic phases were washed with saturated sodium chloride solution, dried over anhydrous sodium sulfate, filtered, and evaporated to dryness by rotation. The crude product was purified by silica gel column chromatography (eluent: System A) to obtain tert-butyl (1R,5S)-6-((4-(6-ethoxypyrazin-2-yl)phenyl)carbamoyl)-6-(2-(methylthio)pyrimidin-4-yl)-3-azab icyclo[3.1.0]hexane-3-carboxylate **9g** (200 mg) with a yield of 75.35%.

MS m/z (ESI): 549.1 [M+1]

### Step 7

### tert-butyl (1R,5S)-6-((4-(6-ethoxypyrazin-2-yl)phenyl)carbamoyl)-6-(2-(methylsulfonyl)pyrimidin-4-yl)-3-azabicyclo[3.1.0]hexane-3-carboxylate

At room temperature, tert-butyl (1R,5S)-6-((4-(6-ethoxypyrazin-2-yl)phenyl)carbamoyl)-6-(2-(methylthio)pyrimidin-4-yl)-3-azab icyclo[3.1.0]hexane-3-carboxylate **9g** (200 mg, 364.53 µmol) and meta-chloroperoxybenzoic acid (162.82 mg, 801.96 µmol, 85% purity) were dissolved in dichloromethane (2 mL), and the mixture was stirred at room temperature for 2 h. Upon completion of the reaction, saturated sodium bicarbonate solution was added to neutralize, and the mixture was extracted with dichloromethane (20 mL × 3). The combined organic phases were washed with saturated sodium chloride solution, dried over anhydrous sodium sulfate, filtered, and evaporated to dryness by rotation to obtain tert-butyl (1R,5S)-6-((4-(6-ethoxypyrazin-2-yl)phenyl)carbamoyl)-6-(2-(methylsulfonyl)pyrimidin-4-yl)-3-azabicyclo[3.1.0]hexane-3-carboxylate **9h** (50 mg) with a yield of 24.29%. The crude product was directly used in the next step.

MS m/z (ESI): 565.1/581.1 [M+1]

### Step 8

### tert-butyl (1R,5S)-6-(2-(cyclopropanesulfonamido)pyrimidin-4-yl)-6-((4-(6-ethoxypyrazin-2-yl)phenyl)car bamoyl)-3-azabicyclo[3.1.0]hexane-3-carboxylate

Tert-butyl (1R,5S)-6-((4-(6-ethoxypyrazin-2-yl)phenyl)carbamoyl)-6-(2-(methylsulfonyl)pyrimidin-4-yl)-3-azabicyclo[3.1.0]hexane-3-carboxylate **9h** (200 mg, 354.20 µmol), cyclopropanesulfonamide (51.50 mg, 425.04 µmol), and cesium carbonate (230.81 mg, 708.40 µmol) were dissolved in N-methylpyrrolidone (1 mL), heated to 90°C, and stirred for 1 hour. After the reaction was complete, the mixture was cooled to room temperature, water (20 mL) was added, and the mixture was extracted with ethyl acetate (20 mL × 3). The combined organic phases were washed with saturated sodium chloride solution, dried over anhydrous sodium sulfate, filtered, and evaporated to dryness by rotation. The crude product was purified by preparative liquid chromatography (column AKZONOBEL Kromasil; 250 × 21.2 mm I.D.; 5 µm, 20 mL/min; mobile phase A: 0.05% TFA + H₂O, mobile phase B: CH₃CN) to obtain tert-butyl (1R,5S)-6-(2-(cyclopropanesulfonamido)pyrimidin-4-yl)-6-((4-(6-ethoxypyrazin-2-yl)phenyl)car bamoyl)-3-azabicyclo[3.1.0]hexane-3-carboxylate **9i** (30 mg) with a yield of 13.62%.

MS m/z (ESI): 622.1 [M+1]

### Step 9

### 6-(2-(cyclopropanesulfonamido)pyrimidin-4-yl)-N-(4-(6-ethoxypyrazin-2-yl)phenyl)-3-azabicycl o[3.1.0]hexane-6-carboxamide

Tert-butyl (1R,5S)-6-(2-(cyclopropanesulfonamido)pyrimidin-4-yl)-6-((4-(6-ethoxypyrazin-2-yl)phenyl)car bamoyl)-3-azabicyclo[3.1.0]hexane-3-carboxylate **9i** (20 mg, 32.17 µmol) and trifluoroacetic acid (0.5 mL) were dissolved in dichloromethane (1.5 mL) and stirred at room temperature for 1 hour. After the reaction was complete, the solvent was removed under reduced pressure. The crude product was purified by preparative liquid chromatography (column AKZONOBEL Kromasil; 250 × 21.2 mm I.D.; 5 µm, 20 mL/min; mobile phase A: 0.05% TFA + H₂O, mobile phase B: CH₃CN) to obtain 6-(2-(cyclopropanesulfonamido)pyrimidin-4-yl)-N-(4-(6-ethoxypyrazin-2-yl)phenyl)-3-azabicycl o[3.1.0]hexane-6-carboxamide **9** (10 mg) with a yield of 44.02%.

MS m/z (ESI): 522.1 [M+1]
1H NMR (400 MHz, DMSO-d6) δ 10.85 (s, 1H), 9.71 (s, 1H), 8.79 (s, 1H), 8.52 (d, J = 5.4 Hz, 2H), 8.25 - 8.06 (m, 3H), 7.86 (d, J = 8.4 Hz, 2H), 6.98 (d, J = 5.3 Hz, 1H), 4.48 (q, J = 7.0 Hz, 3H), 3.77 (s, 2H), 3.64 - 3.56 (m, 2H), 3.16 (d, J = 4.8 Hz, 1H), 2.76 (d, J = 3.5 Hz, 2H), 1.40 (t, J = 7.0 Hz, 3H), 1.07 (q, J = 4.1 Hz, 2H).

### Example 10

### 6-(2-(cyclopropanesulfonamido)pyrimidin-4-yl)-N-(4-(6-ethoxypyrazin-2-yl)phenyl)-3-methyl-3-azabicyclo[3.1.0]hexane-6-carboxamide

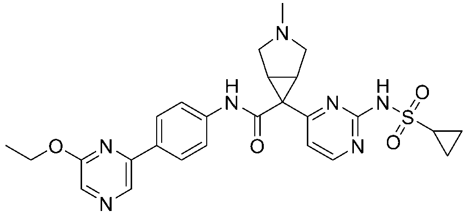

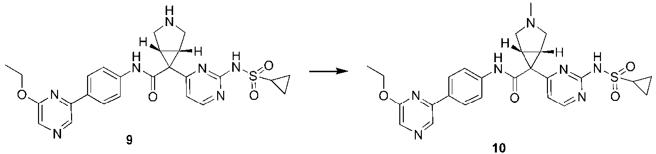

6-(2-(Cyclopropanesulfonamido)pyrimidin-4-yl)-N-(4-(6-ethoxypyrazin-2-yl)phenyl)-3 -azabicyclo[3.1.0]hexane-6-carboxamide **9** (7 mg, 13.42 µmol) and sodium hydroxide (536.82 µg, 13.42 µmol) were added to tetrahydrofuran (1 mL) at room temperature and stirred for 1 hour. Subsequently, paraformaldehyde (523.92 µg, 17.45 µmol) and formic acid (1.54 mg, 33.55 µmol) were added, and the mixture was heated to 70°C and stirred for 5 hours. After the reaction was complete, the solvent was removed under reduced pressure. The crude product was purified by preparative liquid chromatography (column AKZONOBEL Kromasil; 250 × 21.2 mm I.D.; 5 µm, 20 mL/min; mobile phase A: 0.05% TFA + H₂O, mobile phase B: CH₃CN) to obtain 6-(2-(cyclopropanesulfonamido)pyrimidin-4-yl)-N-(4-(6-ethoxypyrazin-2-yl)phenyl)-3-methyl-3-azabicyclo[3.1.0]hexane-6-carboxamide **10** (4.02 mg) with a yield of 44.13%.

MS m/z (ESI): 536.1 [M+1]

### Example 11

### 3-acetyl-6-(2-(cyclopropanesulfonamido)pyrimidin-4-yl)-N-(4-(6-ethoxypyrazin-2-yl)phenyl)-3-azabicyclo[3.1.0]hexane-6-carboxamide

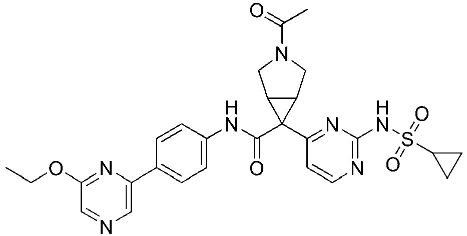

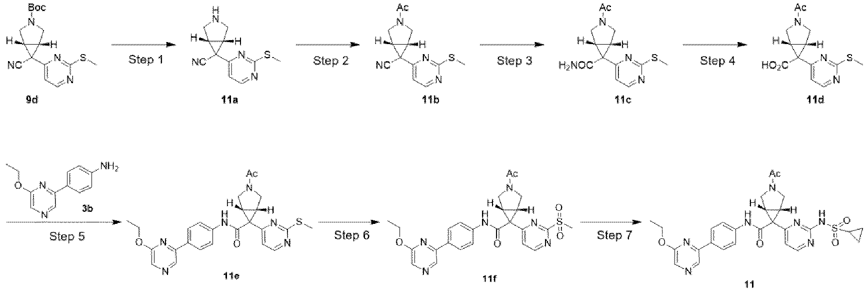

### Step 1

### (1R,5S)-6-(2-(methylthio)pyrimidin-4-yl)-3-azabicyclo[3.1.0]hexane-6-carbonitrile

A solution of hydrogen chloride in dioxane (15 mL, 4 M) was added to a solution of tert-butyl (1R,5S)-6-cyano-6-(2-(methylthio)pyrimidin-4-yl)-3-azabicyclo[3.1.0]hexane-3-carboxylate 9d (2 g, 3.01 mmol) in dichloromethane (20 mL) at room temperature, and the mixture was stirred at room temperature for 5 hours. After the reaction was complete, the solvent was removed under reduced pressure, and the residue was neutralized by addition of saturated sodium bicarbonate solution. The mixture was extracted with dichloromethane (20 mL × 3). The combined organic phases were washed with saturated sodium chloride solution, dried over anhydrous sodium sulfate, filtered, and evaporated to dryness by rotation. The crude product was purified by silica gel column chromatography (eluent: System B) to obtain (1R,5S)-6-(2-(methylthio)pyrimidin-4-yl)-3-azabicyclo[3.1.0]hexane-6-carbonitrile **11a** (500 mg) with a yield of 71.55%.

MS m/z (ESI): 232.9 [M+1]

### Step 2

### (1R,5S)-3-acetyl-6-(2-(methylthio)pyrimidin-4-yl)-3-azabicyclo[3.1.0]hexane-6-carbonitrile

To a solution of (1R,5S)-6-(2-(methylthio)pyrimidin-4-yl)-3-azabicyclo[3.1.0]hexane-6-carbonitrile **11a** (350 mg, 1.51 mmol) and triethylamine (381.14 mg, 3.77 mmol, 529.37 µL) in dichloromethane (3 mL) in ice-water bath was added acetyl chloride (141.93 mg, 1.81 mmol, 128.56 µL), and the mixture was stirred at room temperature for 18 hours. After the reaction was completed, water (20 mL) was added, and the mixture was extracted with dichloromethane (20 mL×3). The combined organic phases were washed with saturated sodium chloride solution, dried over anhydrous sodium sulfate, filtered, and evaporated to dryness by rotation. The crude product was purified by silica gel column chromatography (eluent: System B) to obtain (1R,5S)-3-acetyl-6-(2-(methylthio)pyrimidin-4-yl)-3-azabicyclo[3.1.0]hexane-6-carbonitrile **11b** (400 mg) with a yield of 96.77%.

MS m/z (ESI): 274.3 [M+1]

### Step 3

### (1R,5S)-3-acetyl-6-(2-(methylthio)pyrimidin-4-yl)-3-azabicyclo[3.1.0]hexane-6-carboxamide

(1R,5S)-3-acetyl-6-(2-(methylthio)pyrimidin-4-yl)-3-azabicyclo[3.1.0]hexane-6-carboni trile **11b** (300 mg, 1.09 mmol) was dissolved in dimethyl sulfoxide (3 mL). Sodium hydroxide (87.48 mg, 2.19 mmol) was added in ice-water bath, and the mixture was stirred for 10 minutes. Hydrogen peroxide (1.5 mL) was added, and the mixture was stirred at room temperature for 3 hours. Water (20 mL) was added to quench the reaction, and the mixture was filtered under reduced pressure. The filter cake was dried to obtain (1R,5S)-3-acetyl-6-(2-(methylthio)pyrimidin-4-yl)-3-azabicyclo[3.1.0]hexane-6-carboxamide **11c** (300 mg) with a yield of 93.84%.

MS m/z (ESI): 293.0 [M+1]

### Step 4

### (1R,5S)-3-acetyl-6-(2-(methylthio)pyrimidin-4-yl)-3-azabicyclo[3.1.0]hexane-6-carboxylic acid

To a solution of (1R,5S)-3-acetyl-6-(2-(methylthio)pyrimidin-4-yl)-3-azabicyclo[3.1.0]hexane-6-carboxamide **11c** (400 mg, 1.37 mmol), acetic acid (1.50 mL), and acetic anhydride (3 mL) in dimethyl sulfoxide (3 mL) in ice-water bath was added sodium nitrite (944.05 mg, 13.68 mmol), and the mixture was stirred at room temperature for 18 hours. After the reaction was completed, the reaction mixture was poured into water (5 mL), neutralized with saturated sodium bicarbonate solution, acidified with citric acid, and extracted with ethyl acetate. Most of the product was in the aqueous phase. The aqueous phase was purified by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain (1R,5S)-3-acetyl-6-(2-(methylthio)pyrimidin-4-yl)-3-azabicyclo[3.1.0]hexane-6-carboxylic acid **11d** (180 mg) with a yield of 44.85%.

MS m/z (ESI): 394.0 [M+1]

### Step 5

### (1R,5S)-3-acetyl-N-(4-(6-ethoxypyrazin-2-yl)phenyl)-6-(2-(methylthio)pyrimidin-4-yl)-3-azabic yclo[3.1.0]hexane-6-carboxamide

To a solution of (1R,5S)-3-acetyl-6-(2-(methylthio)pyrimidin-4-yl)-3-azabicyclo[3.1.0]hexane-6-carboxylic acid **11d** (180 mg, 613.62 µmol) in N-methylpyrrolidone (2 mL) in ice-water bath were added N,N,N',N'-tetramethylchloroformamidinium hexafluorophosphate (343.63 mg, 1.23 mmol) and N-methylimidazole (201.51 mg, 2.45 mmol). The mixture was stirred at room temperature for half an hour. Then 4-(6-ethoxypyrazin-2-yl)aniline **3b** (145 mg, 672.62 µmol) was added, and the mixture was heated to 100°C and stirred for 18 hours. Water (20 mL) was added to the reaction mixture, and the mixture was extracted with ethyl acetate (20 mL×3). The combined organic phases were washed with saturated sodium chloride solution, dried over anhydrous sodium sulfate, filtered, and evaporated to dryness by rotation. The crude product was purified by silica gel column chromatography (eluent: System A) to obtain (1R,5S)-3-acetyl-N-(4-(6-ethoxypyrazin-2-yl)phenyl)-6-(2-(methylthio)pyrimidin-4-yl)-3-azabic yclo[3.1.0]hexane-6-carboxamide **11e** (300 mg) with a yield of 99.66%.

MS m/z (ESI): 549.1 [M+1]

### Step 6

### (1R,5S)-3-acetyl-N-(4-(6-ethoxypyrazin-2-yl)phenyl)-6-(2-(methylsulfonyl)pyrimidin-4-yl)-3-az abicyclo[3.1.0]hexane-6-carboxamide

(1R,5S)-3-acetyl-N-(4-(6-ethoxypyrazin-2-yl)phenyl)-6-(2-(methylthio)pyrimidin-4-yl)-3-azabicyclo[3.1.0]hexane-6-carboxamide **11e** (300 mg, 611.53 µmol) and meta-chloroperoxybenzoic acid (273.14 mg, 1.35 mmol, 85% purity) were dissolved in dichloromethane (3 mL) and stirred at room temperature for 2 hours. After the reaction was completed, saturated sodium bicarbonate solution was added to neutralize, and the mixture was extracted with dichloromethane (20 mL×3). The combined organic phases were washed with saturated sodium chloride solution, dried over anhydrous sodium sulfate, filtered, and evaporated to dryness by rotation to obtain (1R,5S)-3-acetyl-N-(4-(6-ethoxypyrazin-2-yl)phenyl)-6-(2-(methylsulfonyl)pyrimidin-4-yl)-3-az abicyclo[3.1.0]hexane-6-carboxamide **11f** (300 mg) with a yield of 96.84%. The crude product was directly used in the next step. MS m/z (ESI): 506.8 [M+1]

### Step 7

### 3-acetyl-6-(2-(cyclopropanesulfonamido)pyrimidin-4-yl)-N-(4-(6-ethoxypyrazin-2-yl)phenyl)-3-azabicyclo[3.1.0]hexane-6-carboxamide

(1R,5S)-3-acetyl-N-(4-(6-ethoxypyrazin-2-yl)phenyl)-6-(2-(methylsulfonyl)pyrimidin-4 -yl)-3-azabicyclo[3.1.0]hexane-6-carboxamide **11f** (300 mg, 592.21 µmol), cyclopropanesulfonamide (86.10 mg, 710.65 µmol), and cesium carbonate (385.91 mg, 1.18 mmol) were added to N-methylpyrrolidone (1 mL). The mixture was heated to 90°C and stirred for 1 hour, then cooled to room temperature. Water (20 mL) was added, and the mixture was extracted with ethyl acetate (20 mL×3). The combined organic phases were washed with saturated sodium chloride solution, dried over anhydrous sodium sulfate, filtered, and evaporated to dryness by rotation. The crude product was purified by preparative liquid chromatography (separation column AKZONOBEL Kromasil; 250×21.2 mm I.D.; 5 µm, 20 mL/min; mobile phase A: 0.05% TFA + H₂O, mobile phase B: CH₃CN) to obtain 3-acetyl-6-(2-(cyclopropanesulfonamido)pyrimidin-4-yl)-N-(4-(6-ethoxypyrazin-2-yl)phenyl)-3-azabicyclo[3.1.0]hexane-6-carboxamide **11** (55 mg) with a yield of 13.29%.

MS m/z (ESI): [M+1] 563.8
1H NMR (400 MHz, DMSO-d6) δ 11.23 (s, 1H), 10.54 (s, 1H), 8.77 (s, 1H), 8.47 (d, J = 5.4 Hz, 1H), 8.19 (s, 1H), 8.13 - 8.02 (m, 2H), 7.71 (d, J = 8.7 Hz, 2H), 6.92 (d, J = 5.5 Hz, 1H), 4.48 (q, J = 7.0 Hz, 2H), 4.11 (d, J = 12.3 Hz, 1H), 3.96 (d, J = 11.2 Hz, 1H), 3.76 (d, J = 10.3 Hz, 1H), 3.41 (s, 3H), 3.18 (s, 1H), 2.06 - 1.94 (m, 1H), 1.70 (s, 3H), 1.40 (t, J = 7.0 Hz, 3H), 1.24 (s, 2H), 1.09 - 0.92 (m, 4H).

### Example 12

### 3-(2-(cyclopropanesulfonamido)pyrimidin-4-yl)-N-(4-(6-ethoxypyrazin-2-yl)-2-fluorophenyl)bic yclo[3.1.0]hexane-3-carboxamide

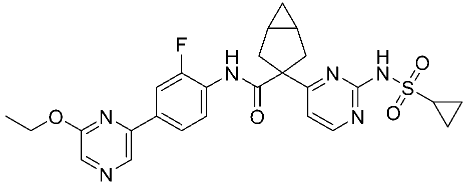

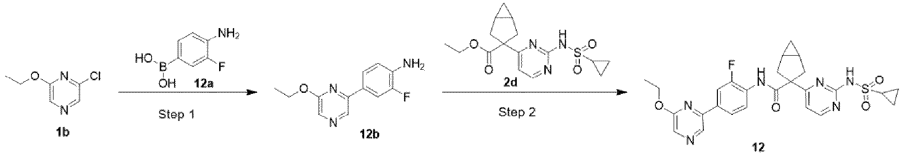

### Step 1

### 4-(6-ethoxypyrazin-2-yl)-2-fluoroaniline

2-Chloro-6-ethoxypyrazine **1b** (500 mg, 3.15 mmol), (4-amino-3-fluorophenyl)boronic acid **12a** (586.19 mg, 3.78 mmol, commercially available), 1,1'-bis(diphenylphosphino)ferrocene palladium(II) dichloride (230.70 mg, 315.29 µmol), and potassium carbonate (1.31 g, 9.46 mmol) were added to 1,4-dioxane (10 mL) and water (2.5 mL). The mixture was subjected to argon exchange three times, and the reaction mixture was heated to 95°C and stirred for 2 hours. After the reaction was completed, the solvent was removed under reduced pressure, and the crude product was isolated by silica gel column chromatography (eluent: System A) to obtain 4-(6-ethoxypyrazin-2-yl)-2-fluoroaniline **12b** (590 mg) with a yield of 80.23%.

MS m/z (ESI): 234.1 [M+1]

### Step 2

### 3-(2-(cyclopropanesulfonamido)pyrimidin-4-yl)-N-(4-(6-ethoxypyrazin-2-yl)-2-fluorophenyl)bic yclo[3.1.0]hexane-3-carboxamide

4-(6-Ethoxypyrazin-2-yl)-2-fluoroaniline **12b** (100 mg, 428.74 µmol) and trimethylaluminum (2.0 M toluene solution) (2 M, 643.11 µL) were added to toluene (1 mL) and stirred at room temperature for 1 hour, followed by addition of ethyl 3-(2-(cyclopropanesulfonamido)pyrimidin-4-yl)bicyclo[3.1.0]hexane-3-carboxylate **2d** (150.67 mg, 428.74 µmol), and the reaction mixture was heated to 90°C and stirred for 5 hours. After the reaction was completed, a small amount of methanol was added to quench the reaction, and the solvent was directly removed by rotary evaporation to obtain a crude product. Dilute hydrochloric acid (1 mL, 1 M) and dichloromethane (10 mL) were added, insoluble solid was removed by filtration, the filtrate was concentrated under reduced pressure, and the crude product was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 3-(2-(cyclopropanesulfonamido)pyrimidin-4-yl)-N-(4-(6-ethoxypyrazin-2-yl)-2-fluorophenyl)bic yclo[3.1.0]hexane-3-carboxamide **12** (22 mg) with a yield of 7.71%.

MS m/z (ESI): 538.8 [M+1]
1H NMR (400 MHz, DMSO-d6) δ 11.06 (s, 1H), 9.32 (s, 1H), 8.59 (s, 1H), 8.30 (d, J = 5.4 Hz, 1H), 8.01 (s, 1H), 7.73 (t, J = 11.5 Hz, 2H), 7.35 (d, J = 8.2 Hz, 1H), 6.82 (d, J = 5.4 Hz, 1H), 4.24 (q, J = 7.1 Hz, 2H), 3.04 (s, 1H), 2.62 (d, J = 13.6 Hz, 2H), 2.22 (d, J = 13.0 Hz, 2H), 1.16 (q, J = 7.4 Hz, 5H), 0.94 - 0.74 (m, 4H), 0.19 (d, J = 7.0 Hz, 1H), -0.00 (d, J = 5.7 Hz, 1H).

### Example 13

### 3-(6-(cyclopropanesulfonamido)pyrimidin-4-yl)-N-(4-(6-ethoxypyrazin-2-yl)phenyl)bicyclo[3.1. 0]hexane-3-carboxamide

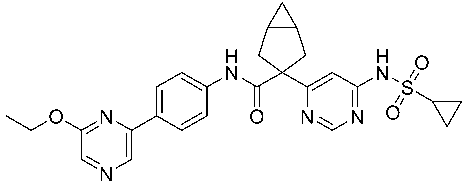

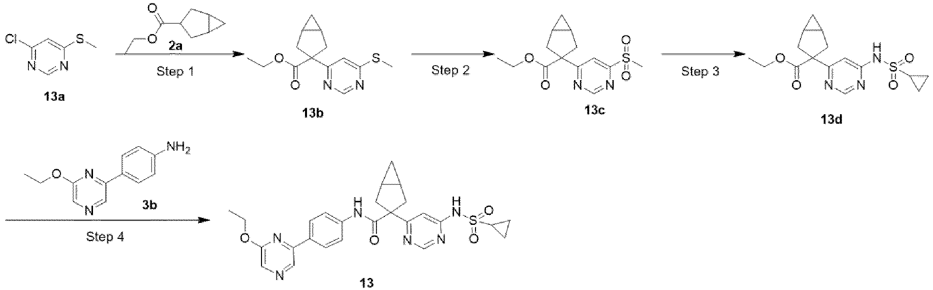

### Step 1

### ethyl 3-(6-(methylthio)pyrimidin-4-yl)bicyclo[3.1.0]hexane-3-carboxylate

At -30°C, lithium bis(trimethylsilyl)amide (1 M, 5.60 mL) was added to a tetrahydrofuran (3.00 mL) solution of 4-chloro-6-(methylthio)pyrimidine **13a** (300 mg, 1.87 mmol, commercially available) and ethyl bicyclo[3.1.0]hexane-3-carboxylate **2a** (302.41 mg, 1.96 mmol), and the reaction mixture was slowly heated to room temperature and the reaction was performed for 1 hour. After the reaction was completed, a saturated ammonium chloride solution was added to quench the reaction, followed by extraction with ethyl acetate (20 mL × 3). The combined organic phases were washed with saturated sodium chloride solution, dried over anhydrous sodium sulfate, filtered, and evaporated to dryness by rotation. The crude product was purified by silica gel column chromatography (eluent: System A) to obtain ethyl 3-(6-(methylthio)pyrimidin-4-yl)bicyclo[3.1.0]hexane-3-carboxylate **13b** (375 mg) with a yield of 72.13%.

MS m/z (ESI): 278.8 [M+1]

### Step 2

### ethyl 3-(6-(methylsulfonyl)pyrimidin-4-yl)bicyclo[3.1.0]hexane-3-carboxylate

At room temperature, ethyl 3-(6-(methylthio)pyrimidin-4-yl)bicyclo[3.1.0]hexane-3-carboxylate **13b** (500 mg, 1.80 mmol) and meta-chloroperoxybenzoic acid (802.27 mg, 3.95 mmol, 85% purity) were dissolved in dichloromethane (5 mL) and stirred for 2 hours. After the reaction was completed, a saturated sodium bicarbonate solution was added to neutralize the mixture, followed by extraction with dichloromethane (20 mL × 3). The combined organic phases were washed with saturated sodium chloride solution, dried over anhydrous sodium sulfate, filtered, and evaporated to dryness by rotation to obtain ethyl 3-(6-(methylsulfonyl)pyrimidin-4-yl)bicyclo[3.1.0]hexane-3-carboxylate **13c** (50 mg) with a yield of 9.46%. The crude product was directly used in the next step without further purification.

MS m/z (ESI): 310.8 [M+1]

### Step 3

### ethyl 3-(6-(cyclopropanesulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate **13c** (550 mg, 1.77 mmol) and cyclopropanesulfonamide (279.11 mg, 2.30 mmol) were dissolved in N-methylpyrrolidone (3 mL), and the mixture was heated to 80°C with stirring for 2 hours. After the reaction was complete, the mixture was cooled to room temperature, and water was added. The mixture was extracted with ethyl acetate (30 mL×3). The combined organic phase was washed with saturated sodium chloride solution, dried over anhydrous sodium sulfate, and concentrated under reduced pressure. The residue was purified by silica gel column chromatography (eluent: System A) to obtain ethyl 3-(6-(cyclopropanesulfonamido)pyrimidin-4-yl)bicyclo[3.1.0]hexane-3-carboxylate **13d** (620 mg) with a yield of 99.56%.

MS m/z (ESI): 352.0 [M+1]

### Step 4

### 3-(6-(cyclopropanesulfonamido)pyrimidin-4-yl)-N-(4-(6-ethoxypyrazin-2-yl)phenyl)bicyclo[3.1. 0]hexane-3-carboxamide

At room temperature, 4-(6-ethoxypyrazin-2-yl)aniline **3b** (126.72 mg, 588.71 µmol) and trimethylaluminum (2.0 M solution in toluene) (2 M, 883.06 µL) were added to toluene (1 mL) and stirred for 1 hour. Subsequently, ethyl 3-(6-(cyclopropanesulfonamido)pyrimidin-4-yl)bicyclo[3.1.0]hexane-3-carboxylate **13d** (172.40 mg, 490.59 µmol) was added, and the mixture was heated to 90°C with stirring for 2 hours. The mixture was cooled to room temperature, and a small amount of methanol was added to quench the reaction. The solvent was directly evaporated to dryness by rotation to obtain a crude product. Dilute hydrochloric acid and dichloromethane were added, and insoluble solid was removed by filtration. The filtrate was concentrated under reduced pressure, and the crude product was purified by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 3-(6-(cyclopropanesulfonamido)pyrimidin-4-yl)-N-(4-(6-ethoxypyrazin-2-yl)phenyl)bicyclo[3.1. 0]hexane-3-carboxamide **13** (60 mg) with a yield of 18.12%.

MS m/z (ESI): [M+1] 520.8
1H NMR (400 MHz, DMSO-d6) δ 11.23 (s, 1H), 9.67 (s, 1H), 8.65 (d, J = 6.7 Hz, 2H), 8.07 (s, 1H), 7.96 (d, J = 8.4 Hz, 2H), 7.65 (d, J = 8.3 Hz, 2H), 6.82 (s, 1H), 4.36 (q, J = 7.1 Hz, 2H), 2.93 (s, 1H), 2.74 (d, J = 13.6 Hz, 2H), 2.30 (d, J = 13.4 Hz, 2H), 1.42 - 1.22 (m, 5H), 1.00 - 0.81 (m, 4H), 0.31 (d, J = 7.1 Hz, 1H), 0.06 - -0.05 (m, 1H).

### Example 14

### 5-(2-(cyclopropanesulfonamido)pyrimidin-4-yl)-N-(4-(6-ethoxypyrazin-2-yl)phenyl)-2-methyl-4, 5,6,7-tetrahydro-2H-indazole-5-carboxamide

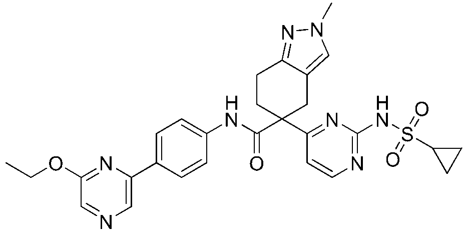

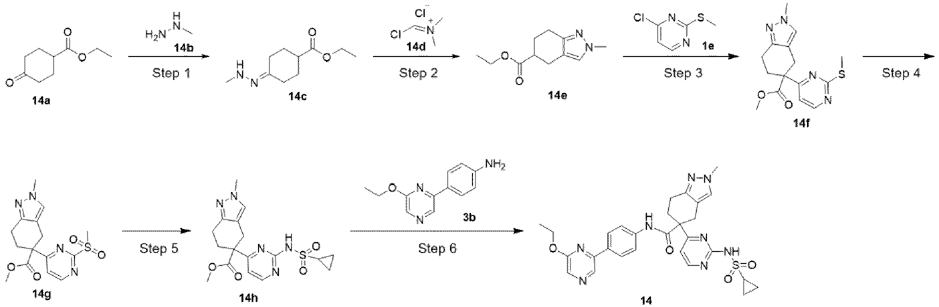

### Step 1

### ethyl 4-(2-methylhydrazineylidene)cyclohexane-1-carboxylate

Ethyl 4-oxocyclohexane-1-carboxylate **14a** (2 g, 11.75 mmol, commercially available), methylhydrazine **14b** (2.03 g, 14.10 mmol, commercially available), and N,N-diisopropylethylamine (3.80 g, 29.38 mmol, 5.27 mL) were dissolved in ethanol (20 mL), and the reaction was heated to 90°C with stirring for 18 hours. After the reaction was complete, the reaction mixture was concentrated under reduced pressure to remove the solvent to obtain ethyl 4-(2-methylhydrazineylidene)cyclohexane-1-carboxylate **14c** (2 g), and the crude product was directly used in the next step.

### Step 2

### ethyl 2-methyl-4,5,6,7-tetrahydro-2H-indazole-5-carboxylate

Ethyl 4-(2-methylhydrazineylidene)cyclohexane-1-carboxylate **14c** (2 g, 10.09 mmol) and (chloromethylene)dimethyliminium chloride **14d** (1.29 g, 10.09 mmol, commercially available) were dissolved in N,N-dimethylformamide (5 mL), and the mixture was heated to 90°C with stirring for 18 hours. After the reaction was complete, the solvent was removed by concentration under reduced pressure. Saturated sodium bicarbonate solution was added, and the mixture was extracted with ethyl acetate (30 mL×3). The combined organic phase was washed with saturated sodium chloride solution, dried over anhydrous sodium sulfate, filtered, and evaporated to dryness by rotation. The crude product was purified by silica gel column chromatography (eluent: System A) to obtain ethyl 2-methyl-4,5,6,7-tetrahydro-2H-indazole-5-carboxylate **14e** (500 mg) with a yield of 23.80%.

MS m/z (ESI): 208.9 [M+1]

### Step 3

### methyl 2-methyl-5-(2-(methylthio)pyrimidin-4-yl)-4,5,6,7-tetrahydro-2H-indazole-5-carboxylate

At -30°C, lithium bis(trimethylsilyl)amide (1 M, 3.60 mL) was added to a solution of 4-chloro-2-(methylthio)pyrimidine **1e** (385.64 mg, 2.40 mmol) and ethyl 2-methyl-4,5,6,7-tetrahydro-2H-indazole-5-carboxylate **14e** (500 mg, 1.20 mmol) in tetrahydrofuran (36.42 mL), and the mixture was slowly heated to room temperature with stirring for 1 hour. After the reaction was complete, saturated ammonium chloride solution was added to quench the reaction. The mixture was extracted with ethyl acetate (30 mL×3). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 2-methyl-5-(2-(methylthio)pyrimidin-4-yl)-4,5,6,7-tetrahydro-2H-indazole-5-carboxylate **14f** (350 mg) with a yield of 87.71%.

MS m/z (ESI): 333.9 [M+1]

### Step 4

### methyl 2-methyl-5-(2-(methylsulfonyl)pyrimidin-4-yl)-4,5,6,7-tetrahydro-2H-indazole-5-carboxylate

Methyl 2-methyl-5-(2-(methylthio)pyrimidin-4-yl)-4,5,6,7-tetrahydro-2H-indazole-5-carboxylate **14f** (350 mg, 1.05 mmol) and meta-chloroperoxybenzoic acid (470.27 mg, 2.32 mmol, 85% purity) were added to dichloromethane (5 mL). The mixture was stirred at room temperature for 2 h, then neutralized by adding saturated sodium bicarbonate solution. The mixture was extracted with dichloromethane (30 mL × 3). The combined organic phases were washed with saturated sodium chloride solution, dried over anhydrous sodium sulfate, filtered, and evaporated to dryness by rotation. The crude product was purified by silica gel column chromatography (eluent: System B) to obtain methyl 2-methyl-5-(2-(methylsulfonyl)pyrimidin-4-yl)-4,5,6,7-tetrahydro-2H-indazole-5-carboxylate **14g** (100 mg) with a yield of 27.26%.

MS m/z (ESI): 349.1 [M+1]

### Step 5

### methyl 5-(2-(cyclopropanesulfonamido)pyrimidin-4-yl)-2-methyl-4,5,6,7-tetrahydro-2H-indazole-5-carb oxylate

Methyl 2-methyl-5-(2-(methylsulfonyl)pyrimidin-4-yl)-4,5,6,7-tetrahydro-2H-indazole-5-carboxylate **14g** (500 mg, 1.44 mmol) and cyclopropanesulfonamide (226.03 mg, 1.87 mmol) were added to N-methylpyrrolidone (2 mL). The mixture was heated to 80°C and stirred for 2 h. After the reaction was complete, the mixture was cooled to room temperature, and water (20 mL) was added to the reaction mixture. The mixture was extracted with ethyl acetate (30 mL × 3). The combined organic phases were washed with saturated sodium chloride solution, dried over anhydrous sodium sulfate, filtered, and evaporated to dryness by rotation. The crude product was purified by silica gel column chromatography (eluent: System B) to obtain methyl 5-(2-(cyclopropanesulfonamido)pyrimidin-4-yl)-2-methyl-4,5,6,7-tetrahydro-2H-indazole-5-carb oxylate **14h** (300 mg) with a yield of 51.56%.

MS m/z (ESI): 406.1 [M+1]

### Step 6

### 5-(2-(cyclopropanesulfonamido)pyrimidin-4-yl)-N-(4-(6-ethoxypyrazin-2-yl)phenyl)-2-methyl-4, 5,6,7-tetrahydro-2H-indazole-5-carboxamide

Trimethylaluminum (2.0 M solution in toluene) (2 M, 665.89 µL) was added to a solution of 4-(6-ethoxypyrazin-2-yl)aniline **3b** (95.56 mg, 443.93 µmol) in toluene (1.22 mL). The mixture was stirred at room temperature for 1 h, then methyl 5-(2-(cyclopropanesulfonamido)pyrimidin-4-yl)-2-methyl-4,5,6,7-tetrahydro-2H-indazole-5-carb oxylate **14h** (150 mg, 369.94 µmol) was added. The mixture was heated to 90°C and stirred for 5 h. After the reaction was complete, a small amount of methanol was added to quench the reaction, and the solvent was directly evaporated to dryness by rotation to obtain the crude product. Dilute hydrochloric acid and dichloromethane were added, and the insoluble solid was removed by filtration. The filtrate was concentrated under reduced pressure, and the crude product was purified by preparative liquid chromatography (column: AKZONOBEL Kromasil; 250 × 21.2 mm I.D.; 5 µm, 20 mL/min; mobile phase A: 0.05% TFA + H₂O, mobile phase B: CH₃CN) to obtain 5-(2-(cyclopropanesulfonamido)pyrimidin-4-yl)-N-(4-(6-ethoxypyrazin-2-yl)phenyl)-2-methyl-4, 5,6,7-tetrahydro-2H-indazole-5-carboxamide **14** (6.9 mg) with a yield of 2.52%.

MS m/z (ESI): 574.8 [M+1]

### Example 15

### 3-(4-(cyclopropanesulfonamido)pyrimidin-2-yl)-N-(4-(6-ethoxypyrazin-2-yl)phenyl)bicyclo[3.1. 0]hexane-3-carboxamide

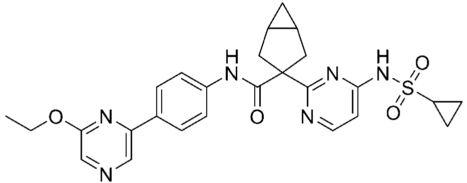

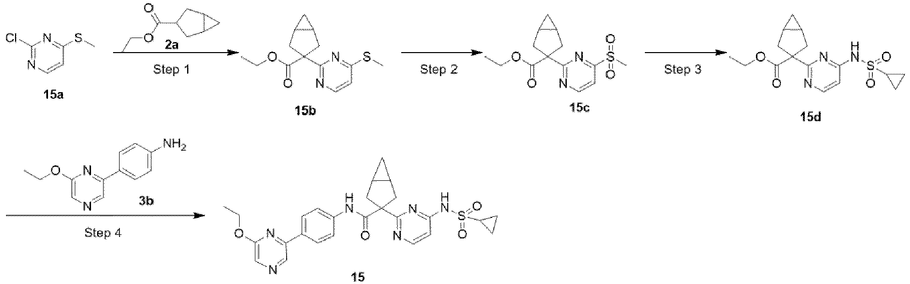

### Step 1

### ethyl 3-(4-(methylthio)pyrimidin-2-yl)bicyclo[3.1.0]hexane-3-carboxylate

Lithium bis(trimethylsilyl)amide (1 M, 5.84 mL) was added to a solution of 2-chloro-4-(methylthio)pyrimidine **15a** (374.98 mg, 2.33 mmol, commercially available) and ethyl bicyclo[3.1.0]hexane-3-carboxylate 2a (300 mg, 1.95 mmol) in tetrahydrofuran (3.00 mL) at -30°C. The mixture was slowly heated to room temperature over 1 h. After the reaction was complete, the mixture was quenched by adding saturated ammonium chloride solution. The mixture was extracted with ethyl acetate (20 mL × 3). The combined organic phases were washed with saturated sodium chloride solution, dried over anhydrous sodium sulfate, filtered, and evaporated to dryness by rotation. The crude product was purified by silica gel column chromatography (eluent: System A) to obtain ethyl 3-(4-(methylthio)pyrimidin-2-yl)bicyclo[3.1.0]hexane-3-carboxylate **15b** (300 mg) with a yield of 55.40%.

MS m/z (ESI): 278.9 [M+1]

### Step 2

### ethyl 3-(4-(methylsulfonyl)pyrimidin-2-yl)bicyclo[3.1.0]hexane-3-carboxylate

Ethyl 3-(4-(methylthio)pyrimidin-2-yl)bicyclo[3.1.0]hexane-3-carboxylate 15b (500 mg, 1.80 mmol) and meta-chloroperoxybenzoic acid (962.72 mg, 4.74 mmol, 85% purity) were dissolved in dichloromethane (5 mL) at room temperature and stirred for 2 h. After the reaction was complete, saturated sodium bicarbonate solution was added to neutralize the mixture. The mixture was extracted with dichloromethane (20 mL × 3). The combined organic phases were washed with saturated sodium chloride solution, dried over anhydrous sodium sulfate, filtered, and concentrated under reduced pressure to obtain ethyl 3-(4-(methylsulfonyl)pyrimidin-2-yl)bicyclo[3.1.0]hexane-3-carboxylate **15c** (600 mg) with a yield of 89.69%. The crude product was directly used in the next step without further purification.

MS m/z (ESI): 310.8 [M+1]

### Step 3

### ethyl 3-(4-(cyclopropanesulfonamido)pyrimidin-2-yl)bicyclo[3.1.0]hexane-3-carboxylate

ethyl 3-(4-(methylsulfonyl)pyrimidin-2-yl)bicyclo[3.1.0]hexane-3-carboxylate **15c** (660.00 mg, 2.13 mmol) and cyclopropanesulfonamide (309.17 mg, 2.55 mmol) were dissolved in N-methylpyrrolidone (3 mL), the temperature was raised to 80°C and the mixture was stirred for 2 hours. After the reaction was completed, the mixture was cooled to room temperature, water was added, and extraction was performed with ethyl acetate (30 mL×3). The combined organic phases were washed with saturated sodium chloride solution, dried over anhydrous sodium sulfate, and concentrated under reduced pressure. The residue was purified by silica gel column chromatography (eluent: System A) to obtain ethyl 3-(4-(cyclopropanesulfonamido)pyrimidin-2-yl)bicyclo[3.1.0]hexane-3-carboxylate **15d** (600 mg) in a yield of 80.29%.

MS m/z (ESI): 352.0 [M+1]

### Step 4

### 3-(4-(cyclopropanesulfonamido)pyrimidin-2-yl)-N-(4-(6-ethoxypyrazin-2-yl)phenyl)bicyclo[3.1. 0]hexane-3-carboxamide

At room temperature, 4-(6-ethoxypyrazin-2-yl)aniline **3b** (110.25 mg, 512.21 µmol) and trimethylaluminum (2.0 M toluene solution) (2 M, 768.31 µL) were added to toluene (2 mL) and stirred for 1 hour. Subsequently, ethyl 3-(4-(cyclopropanesulfonamido)pyrimidin-2-yl)bicyclo[3.1.0]hexane-3-carboxylate **15d** (150 mg, 426.84 µmol) was added, the temperature was raised to 90°C and the mixture was stirred for 2 hours. The mixture was cooled to room temperature, quenched by adding a small amount of methanol, and the solvent was directly evaporated to dryness by rotation to obtain a crude product. Dilute hydrochloric acid and dichloromethane were added, insoluble solid was removed by filtration, and the filtrate was concentrated under reduced pressure. The crude product was purified by preparative liquid chromatography (separation column: AKZONOBEL Kromasil; 250×21.2 mm I.D.; 5 µm, 20 mL/min; mobile phase A: 0.05% TFA + H₂O, mobile phase B: CH₃CN) to obtain 3-(4-(cyclopropanesulfonamido)pyrimidin-2-yl)-N-(4-(6-ethoxypyrazin-2-yl)phenyl)bicyclo[3.1. 0]hexane-3-carboxamide **15** (73 mg) in a yield of 26.53%.

MS m/z (ESI): [M+1] 520.8
1H NMR (400 MHz, DMSO-d6) δ 11.25 (s, 1H), 9.63 (s, 1H), 8.75 (s, 1H), 8.49 (d, J = 5.7 Hz, 1H), 8.17 (s, 1H), 8.05 (d, J = 8.5 Hz, 2H), 7.77 (d, J = 8.5 Hz, 2H), 6.78 (d, J = 5.7 Hz, 1H), 4.47 (q, J = 7.0 Hz, 2H), 3.16 - 3.08 (m, 1H), 2.83 (d, J = 13.5 Hz, 2H), 2.64 - 2.55 (m, 2H), 1.39 (t, J = 7.0 Hz, 5H), 1.05 (dt, J = 7.0, 3.5 Hz, 2H), 0.83 (td, J = 7.4, 4.7 Hz, 2H), 0.43 - 0.33 (m, 1H), 0.15 (q, J = 4.3 Hz, 1H).

### Example 16

### 3-(6-(cyclopropanesulfonamido)pyrazin-2-yl)-N-(4-(6-ethoxypyrazin-2-yl)phenyl)bicyclo[3.1.0] hexane-3-carboxamide

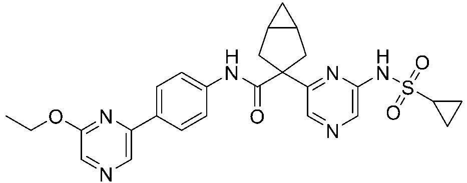

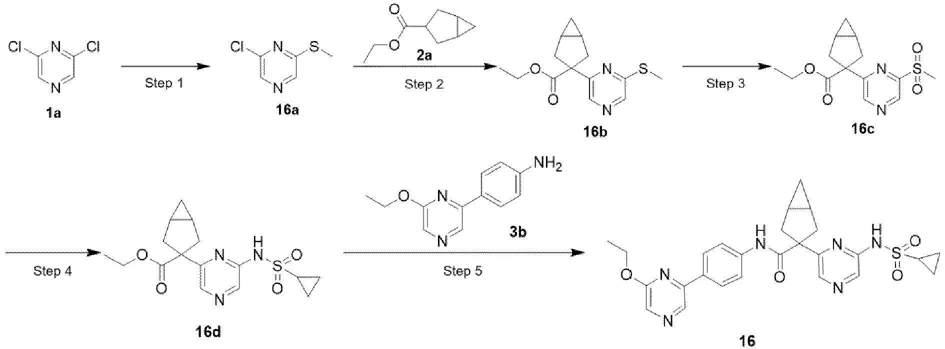

### Step 1

### 2-chloro-6-(methylthio)pyrazine

2,6-dichloropyrazine **1a** (5 g, 33.56 mmol), potassium carbonate (4.64 g, 33.56 mmol), and sodium thiomethoxide (10.59 g, 30.21 mmol, 20% purity) were added to N,N-dimethylformamide (30 mL) and stirred at room temperature for 18 hours. Water (30 mL) was added, and extraction was performed with ethyl acetate (30 mL×3). The combined organic phases were washed with saturated sodium chloride solution, dried over anhydrous sodium sulfate, and concentrated under reduced pressure. The residue was purified by silica gel column chromatography (eluent: System A) to obtain 2-chloro-6-(methylthio)pyrazine **16a** (3.2 g) in a yield of 59.36%.

MS m/z (ESI): 161.2 [M+1]

### Step 2

### ethyl 3-(6-(methylthio)pyrazin-2-yl)bicyclo[3.1.0]hexane-3-carboxylate

At -30°C, lithium bis(trimethylsilyl)amide (1 M, 6.81 mL) was added to a solution of 2-chloro-6-(methylthio)pyrazine 16a (437.48 mg, 2.72 mmol) and ethyl bicyclo[3.1.0]hexane-3-carboxylate 2a (350 mg, 2.27 mmol) in tetrahydrofuran (3 mL). The mixture was heated to room temperature and stirred for 3 hours. Saturated ammonium chloride solution was added to quench the reaction, and extraction was performed with ethyl acetate (20 mL×3). The combined organic phases were washed with saturated sodium chloride solution, dried over anhydrous sodium sulfate, filtered, and evaporated to dryness by rotation. The crude product was purified by silica gel column chromatography (eluent: System A) to obtain ethyl 3-(6-(methylthio)pyrazin-2-yl)bicyclo[3.1.0]hexane-3-carboxylate 16b (370 mg) in a yield of 58.56%.

MS m/z (ESI): 278.9 [M+1]

### Step 3

### ethyl 3-(6-(methylsulfonyl)pyrazin-2-yl)bicyclo[3.1.0]hexane-3-carboxylate

At room temperature, ethyl 3-(6-(methylthio)pyrazin-2-yl)bicyclo[3.1.0]hexane-3-carboxylate 16b (370 mg, 1.33 mmol) and meta-chloroperoxybenzoic acid (593.68 mg, 2.92 mmol, 85% purity) were dissolved in dichloromethane (5 mL) and stirred for 2 h. After the reaction was completed, a saturated sodium bicarbonate solution was added to neutralize, and the mixture was extracted with dichloromethane (20 mL × 3). The combined organic phases were washed with a saturated sodium chloride solution, dried over anhydrous sodium sulfate, filtered, and evaporated to dryness by rotation to obtain ethyl 3-(6-(methylsulfonyl)pyrazin-2-yl)bicyclo[3.1.0]hexane-3-carboxylate 16c (400 mg) with a yield of 96.96%. The crude product was directly used in the next step.

MS m/z (ESI): 310.8 [M+1]

### Step 4

### ethyl 3-(6-(cyclopropanesulfonamido)pyrazin-2-yl)bicyclo[3.1.0]hexane-3-carboxylate

Ethyl 3-(6-(methylsulfonyl)pyrazin-2-yl)bicyclo[3.1.0]hexane-3-carboxylate 16c (660.00 mg, 2.13 mmol) and cyclopropanesulfonamide (309.17 mg, 2.55 mmol) were dissolved in N-methylpyrrolidone (3 mL), heated to 80°C, and stirred for 2 h. After the reaction was completed, the mixture was cooled to room temperature, and water (30 mL) was added. The mixture was extracted with ethyl acetate (30 mL × 3). The combined organic phases were washed with a saturated sodium chloride solution, dried over anhydrous sodium sulfate, and concentrated under reduced pressure. The residue was purified by silica gel column chromatography (eluent: System A) to obtain ethyl 3-(6-(cyclopropanesulfonamido)pyrazin-2-yl)bicyclo[3.1.0]hexane-3-carboxylate **16d** (350 mg) with a yield of 46.84%.

MS m/z (ESI): 352.0 [M+1]

### Step 5

### 3-(6-(cyclopropanesulfonamido)pyrazin-2-yl)-N-(4-(6-ethoxypyrazin-2-yl)phenyl)bicyclo[3.1.0] hexane-3-carboxamide

4-(6-Ethoxypyrazin-2-yl)aniline **3b** (110.25 mg, 512.21 µmol) and trimethylaluminum (2.0 M toluene solution) (2 M, 768.31 µL) were dissolved in toluene (1.5 mL) and stirred at room temperature for 1 h. Ethyl 3-(6-(cyclopropanesulfonamido)pyrazin-2-yl)bicyclo[3.1.0]hexane-3-carboxylate **16d** (150.00 mg, 426.84 µmol) was added, and the mixture was heated to 90°C and stirred for 5 h. The mixture was cooled to room temperature, and a small amount of methanol was added to quench the reaction. The solvent was removed by concentration under reduced pressure. Dilute hydrochloric acid and dichloromethane were added, and the insoluble solid was removed by filtration. The filtrate was concentrated under reduced pressure, and the crude product was purified by preparative liquid chromatography (separation column: AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyrazin-2-yl)-N-(4-(6-ethoxypyrazin-2-yl)phenyl)bicyclo[3.1.0] hexane-3-carboxamide **16** (12 mg) with a yield of 5.24%.

MS m/z (ESI): 520.8 [M+1]
1H NMR (400 MHz, DMSO-d6) δ 10.93 (s, 1H), 9.46 (s, 1H), 8.56 (s, 1H), 8.09 (s, 1H), 7.99 (s, 2H), 7.89 (d, J = 8.4 Hz, 2H), 7.57 (d, J = 8.4 Hz, 2H), 4.28 (q, J = 7.2 Hz, 2H), 2.97 (s, 1H), 2.76 (d, J = 13.6 Hz, 2H), 2.27 (d, J = 13.4 Hz, 2H), 1.23 (dt, J = 14.3, 7.3 Hz, 5H), 0.94 (s, 2H), 0.77 (d, J = 8.0 Hz, 2H), 0.27 (d, J = 7.3 Hz, 1H), 0.00 (s, 1H).

### Example 17

### 3-(4-(cyclopropanesulfonamido)pyridin-2-yl)-N-(4-(6-ethoxypyrazin-2-yl)phenyl)bicyclo[3.1.0]h exane-3-carboxamide

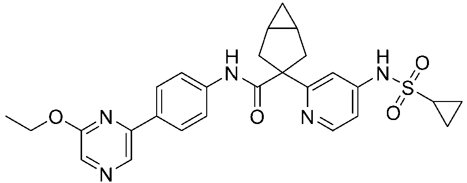

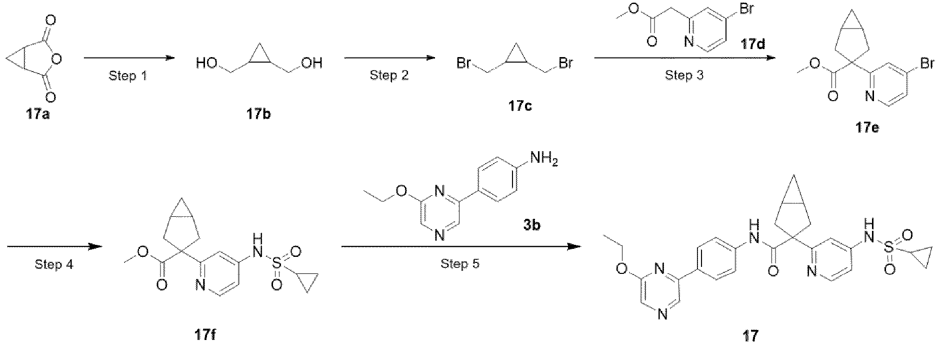

### Step 1

### cyclopropane-1,2-diyldimethanol

Under ice-water bath, a solution of 3-oxabicyclo[3.1.0]hexane-2,4-dione 17a (15 g, 133.83 mmol) in tetrahydrofuran (100 mL) was slowly added to a suspension of lithium aluminum hydride (6.81 g, 200.74 mmol) in tetrahydrofuran (250 mL). The mixture was slowly heated to room temperature and then heated to 70°C with stirring for 18 h. After the reaction was completed, the mixture was cooled with ice water, and water (6.81 mL) and 15% sodium hydroxide solution (6.81 mL) were added. Ethyl acetate (200 mL) was subsequently added, and the mixture was stirred for 30 min. The insoluble solid was removed by filtration, and the filtrate was dried over anhydrous sodium sulfate, filtered, and concentrated to obtain cyclopropane-1,2-diyldimethanol **17b** (7 g) with a yield of 51.21%.

### Step 2

### 1,2-bis(bromomethyl)cyclopropane

Under ice-water bath, triphenylphosphine (37.25 g, 122.39 mmol) was dissolved in anhydrous acetonitrile (300 mL). Liquid bromine (19.56 g, 122.39 mmol, 6.72 mL) was added dropwise, and the mixture was stirred for 30 min. A solution of cyclopropane-1,2-diyldimethanol **17b** (5 g, 48.96 mmol) in acetonitrile (50 mL) was added, and the mixture was slowly heated to room temperature and stirred for 18 h. The reaction mixture was filtered to remove the insoluble solid, and the filtrate was concentrated under reduced pressure. Ethyl acetate/petroleum ether (100 mL, 2/1) was added, and the mixture was stirred for 1 h. The insoluble solid was removed by filtration, and the filtrate was concentrated under reduced pressure to obtain 1,2-bis(bromomethyl)cyclopropane **17c** (10.1 g) with a yield of 90.51%. The crude product was directly used in the next step.

### Step 3

### methyl 3-(4-bromopyridin-2-yl)bicyclo[3.1.0]hexane-3-carboxylate

Methyl 2-(4-bromopyridin-2-yl)acetate **17d** (500 mg, 2.17 mmol, commercially available) was added to N,N-dimethylformamide (6 mL), and the mixture was subjected to argon exchange three times. Under cooling with ice-water bath, sodium hydroxide (199.95 mg, 5.00 mmol) was added. After stirring for 0.5 hours, 1,2-bis(bromomethyl)cyclopropane **17c** (643.97 mg, 2.83 mmol) was added, and the mixture was stirred at room temperature for 23 hours. After the reaction was completed, water (30 mL) was added, and the mixture was extracted with ethyl acetate (30 mL × 3). The combined organic phase was washed with saturated sodium chloride solution, dried over anhydrous sodium sulfate, and concentrated under reduced pressure. The residue was purified by silica gel column chromatography (eluent: System A) to obtain methyl 3-(4-bromopyridin-2-yl)bicyclo[3.1.0]hexane-3-carboxylate **17e** (490 mg) with a yield of 76.13%.

### Step 4

### methyl 3-(4-(cyclopropanesulfonamido)pyridin-2-yl)bicyclo[3.1.0]hexane-3-carboxylate

Methyl 3-(4-bromopyridin-2-yl)bicyclo[3.1.0]hexane-3-carboxylate 17e (100 mg, 337.66 µmol), cyclopropanesulfonamide (61.36 mg, 506.48 µmol), cesium carbonate (220.03 mg, 675.31 µmol), allylpalladium chloride (12.35 mg, 33.77 µmol), and 2-di-tert-butylphosphino-2',4',6'-triisopropylbiphenyl (28.68 mg, 67.53 µmol) were sequentially added to 1,4-dioxane (2 mL). The mixture was subjected to argon exchange 3 times and heated to 90°C with stirring for 2 hours. After the reaction was completed, dichloromethane (30 mL) and methanol (5 mL) were added, and insoluble solid was removed by filtration. The filtrate was concentrated under reduced pressure, and the crude product was purified by silica gel column chromatography (eluent: System A) to obtain methyl 3-(4-(cyclopropanesulfonamido)pyridin-2-yl)bicyclo[3.1.0]hexane-3-carboxylate 17f (50 mg) with a yield of 44.02%.

MS m/z (ESI): 336.2 [M+1]

### Step 5

### 3-(4-(cyclopropanesulfonamido)pyridin-2-yl)-N-(4-(6-ethoxypyrazin-2-yl)phenyl)bicyclo[3.1.0]h exane-3-carboxamide

4-(6-Ethoxypyrazin-2-yl)aniline **3b** (110.25 mg, 512.21 µmol) and trimethylaluminum (2.0 M toluene solution) (2 M, 768.31 µL) were dissolved in toluene (1.5 mL) and stirred at room temperature for 1 hour. Methyl 3-(4-(cyclopropanesulfonamido)pyridin-2-yl)bicyclo[3.1.0]hexane-3-carboxylate 17f (143.59 mg, 426.84 µmol) was added, and the mixture was heated to 90°C and stirred for 5 hours. After the reaction was completed, a small amount of methanol was added to quench the reaction, and the solvent was removed by concentration under reduced pressure. Dilute hydrochloric acid and dichloromethane were added, and insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyridin-2-yl)-N-(4-(6-ethoxypyrazin-2-yl)phenyl)bicyclo[3.1.0]h exane-3-carboxamide **17** (50 mg) with a yield of 17.03%.

MS m/z (ESI): 519.8 [M+1]
1H NMR (400 MHz, DMSO-d6) δ 9.47 (s, 1H), 8.80 (s, 1H), 8.48 (d, J = 5.9 Hz, 1H), 8.23 (s, 1H), 8.10 (d, J = 8.5 Hz, 2H), 7.78 (d, J = 8.5 Hz, 2H), 7.35 (s, 1H), 7.23 (d, J = 6.2 Hz, 1H), 4.52 (q, J = 7.0 Hz, 2H), 2.95 - 2.78 (m, 3H), 2.65 (d, J = 13.7 Hz, 2H), 1.46 (dt, J = 14.1, 5.8 Hz, 5H), 1.08 (ddd, J = 16.8, 7.2, 3.9 Hz, 4H), 0.45 (d, J = 4.9 Hz, 1H), -0.00 (d, J = 4.2 Hz, 1H).

### Example 18

### 3-(2-(cyclopropanesulfonamido)pyridin-4-yl)-N-(4-(6-ethoxypyrazin-2-yl)phenyl)bicyclo[3.1.0]h exane-3-carboxamide

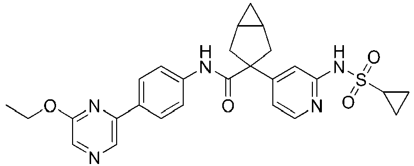

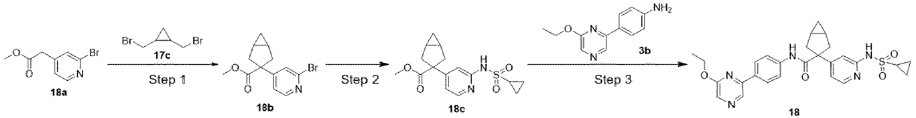

### Step 1

### methyl 3-(2-bromopyridin-4-yl)bicyclo[3.1.0]hexane-3-carboxylate

Methyl 2-(2-bromopyridin-4-yl)acetate **18a** (900 mg, 3.91 mmol, commercially available) was added to N,N-dimethylformamide (6 mL), and the mixture was subjected to argon exchange three times. Under cooling with ice-water bath, sodium hydroxide (359.91 mg, 9.00 mmol) was slowly added. After stirring for 0.5 hours, 1,2-bis(bromomethyl)cyclopropane **17c** (1.16 g, 5.09 mmol) was added. The mixture was stirred at room temperature for 23 hours. After the reaction was completed, water (30 mL) was added, and the mixture was extracted with ethyl acetate (30 mL × 3). The combined organic phase was washed with saturated sodium chloride solution, dried over anhydrous sodium sulfate, and concentrated under reduced pressure. The residue was purified by silica gel column chromatography (eluent: System A) to obtain methyl 3-(2-bromopyridin-4-yl)bicyclo[3.1.0]hexane-3-carboxylate **18b** (800 mg) with a yield of 69.05%.

MS m/z (ESI): 296.3 [M+1]

### Step 2

### methyl 3-(2-(cyclopropanesulfonamido)pyridin-4-yl)bicyclo[3.1.0]hexane-3-carboxylate

Methyl 3-(2-bromopyridin-4-yl)bicyclo[3.1.0]hexane-3-carboxylate **18b** (1 g, 3.38 mmol), cyclopropanesulfonamide (613.65 mg, 5.06 mmol), cesium carbonate (2.20 g, 6.75 mmol), 2-di-tert-butylphosphino-2',4',6'-triisopropylbiphenyl (143.77 mg, 337.83 µmol), and allylpalladium(II) chloride dimer (61.77 mg, 168.83 µmol) were sequentially added to 1,4-dioxane (10 mL). The mixture was subjected to argon exchange 3 times and heated to 90°C with stirring for 2 hours. After the reaction was completed, dichloromethane (30 mL) and methanol (5 mL) were added, and insoluble solid was removed by filtration. The filtrate was concentrated under reduced pressure, and the crude product was purified by silica gel column chromatography (eluent: System A) to obtain methyl 3-(2-(cyclopropanesulfonamido)pyridin-4-yl)bicyclo[3.1.0]hexane-3-carboxylate **18c** (900 mg) with a yield of 79.23%.

MS m/z (ESI): 336.2 [M+1]

### Step 3

### 3-(2-(cyclopropanesulfonamido)pyridin-4-yl)-N-(4-(6-ethoxypyrazin-2-yl)phenyl)bicyclo[3.1.0]h exane-3-carboxamide

4-(6-Ethoxypyrazin-2-yl)aniline **3b** (122.85 mg, 570.74 µmol) and trimethylaluminum (2.0 M toluene solution) (2 M, 856.11 µL) were dissolved in toluene (1.5 mL) and stirred at room temperature for 1 hour. Subsequently, methyl 3-(2-(cyclopropanesulfonamido)pyridin-4-yl)bicyclo[3.1.0]hexane-3-carboxylate **18c** (160 mg, 475.62 µmol) was added, and the mixture was heated to 90°C and stirred for 5 hours. After the reaction was completed, a small amount of methanol was added to quench the reaction, and the solvent was removed by concentration under reduced pressure. Dilute hydrochloric acid and dichloromethane were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(2-(cyclopropanesulfonamido)pyridin-4-yl)-N-(4-(6-ethoxypyrazin-2-yl)phenyl)bicyclo[3.1.0]h exane-3-carboxamide **18** (54 mg) with a yield of 17.32%.

MS m/z (ESI): 520.8 [M+1]
1H NMR (400 MHz, DMSO-d6) δ 9.56 (s, 1H), 8.63 (d, J = 2.1 Hz, 1H), 8.13-7.87 (m, 4H), 7.60 (dd, J = 8.5, 5.5 Hz, 2H), 6.99-6.84 (m, 2H), 4.35 (q, J = 7.0 Hz, 2H), 2.98 2.78 (m, 2H), 2.70 (dd, J = 14.5, 3.9 Hz, 1H), 2.15 (d, J = 13.9 Hz, 1H), 2.03 (d, J = 13.1 Hz, 1H), 1.28 (q, J = 7.1, 6.6 Hz, 5H), 0.86 (dd, J = 25.5, 6.6 Hz, 4H), 0.31 (d, J = 7.1 Hz, 1H), 0.09 - -0.10 (m, 1H).

### Example 19

### 3-(5-(cyclopropanesulfonamido)pyridazin-3-yl)-N-(4-(6-ethoxypyrazin-2-yl)phenyl)bicyclo[3.1.0 ]hexane-3-carboxamide

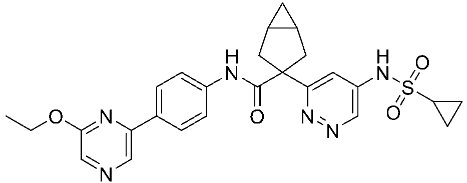

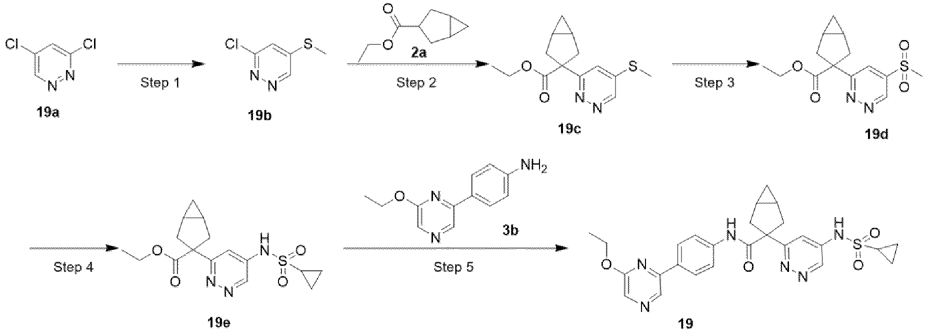

### Step 1

### 3-chloro-5-(methylthio)pyridazine

3,5-Dichloropyridazine **19a** (2.2 g, 14.77 mmol, commercially available) and sodium thiomethoxide (6.21 g, 17.72 mmol, 20% purity) were dissolved in tetrahydrofuran (10 mL) and stirred at room temperature for 1 hour. After the reaction was completed, water (30 mL) was added, and the mixture was extracted with ethyl acetate (30 mL × 3). The combined organic phase was washed with saturated sodium chloride solution, dried over anhydrous sodium sulfate, and concentrated under reduced pressure. The crude product was added to ethyl acetate/petroleum ether = 1/1 (30 mL) mixed solvent and stirred for 0.5 hours. The mixture was filtered under reduced pressure, and the filter cake was dried to obtain 3-chloro-5-(methylthio)pyridazine **19b** (1.63 g) with a yield of 68.72%.

MS m/z (ESI): 161.0 [M+1]

### Step 2

### ethyl 3-(5-(methylthio)pyridazin-3-yl)bicyclo[3.1.0]hexane-3-carboxylate

At -30°C, lithium bis(trimethylsilyl)amide (1 M, 7.78 mL) was added to a solution of 3-chloro-5-(methylthio)pyridazine **19b** (499.98 mg, 3.11 mmol) and ethyl bicyclo[3.1.0]hexane-3-carboxylate **2a** (400 mg, 2.59 mmol) in tetrahydrofuran (2 mL). The mixture was slowly heated to room temperature and stirred for 3 hours. After the reaction was completed, saturated ammonium chloride aqueous solution (20 mL) was added to quench the reaction, and the mixture was extracted with ethyl acetate (20 mL × 3). The combined organic phase was washed with saturated sodium chloride solution, dried over anhydrous sodium sulfate, filtered, and evaporated to dryness by rotation. The crude product was purified by silica gel column chromatography (eluent: System A) to obtain ethyl 3-(5-(methylthio)pyridazin-3-yl)bicyclo[3.1.0]hexane-3-carboxylate **19c** (350 mg) with a yield of 48.47%.

MS m/z (ESI): 278.9 [M+1]

### Step 3

### ethyl 3-(5-(methylsulfonyl)pyridazin-3-yl)bicyclo[3.1.0]hexane-3-carboxylate

Ethyl 3-(5-(methylthio)pyridazin-3-yl)bicyclo[3.1.0]hexane-3-carboxylate **19c** (370 mg, 1.33 mmol) and meta-chloroperoxybenzoic acid (431.76 mg, 2.13 mmol, 85% purity) were dissolved in dichloromethane (3 mL) and stirred at room temperature for 2 hours. After the reaction was completed, saturated sodium bicarbonate solution was added to neutralize the mixture, and the mixture was extracted with dichloromethane (20 mL × 3). The combined organic phase was washed with saturated sodium chloride solution, dried over anhydrous sodium sulfate, filtered, and evaporated to dryness by rotation to obtain ethyl 3-(5-(methylsulfonyl)pyridazin-3-yl)bicyclo[3.1.0]hexane-3-carboxylate **19d** (350 mg) with a yield of 89.45%. The crude product was directly used in the next step.

MS m/z (ESI): 294.9 [M+1]

### Step 4

### ethyl 3-(5-(cyclopropanesulfonamido)pyridazin-3-yl)bicyclo[3.1.0]hexane-3-carboxylate

Ethyl 3-(5-(methylsulfonyl)pyridazin-3-yl)bicyclo[3.1.0]hexane-3-carboxylate **19d** (400 mg, 1.36 mmol) and cyclopropanesulfonamide (197.56 mg, 1.63 mmol) were dissolved in N-methylpyrrolidone (3 mL). The mixture was heated to 80°C and stirred for 2 hours. After the reaction was completed, the mixture was cooled to room temperature, and water (30 mL) was added. The mixture was extracted with ethyl acetate (30 mL × 3). The combined organic phase was washed with saturated sodium chloride solution, dried over anhydrous sodium sulfate, and concentrated under reduced pressure. The residue was purified by silica gel column chromatography (eluent: System A) to obtain ethyl 3-(5-(cyclopropanesulfonamido)pyridazin-3-yl)bicyclo[3.1.0]hexane-3-carboxylate **19e** (300 mg) with a yield of 62.82%.

MS m/z (ESI): 352.0 [M+1]

### Step 5

### 3-(5-(cyclopropanesulfonamido)pyridazin-3-yl)-N-(4-(6-ethoxypyrazin-2-yl)phenyl)bicyclo[3.1.0 ]hexane-3-carboxamide

4-(6-Ethoxypyrazin-2-yl)aniline **3b** (122.85 mg, 570.74 µmol) and trimethylaluminum (2.0 M toluene solution) (2 M, 856.11 µL) were dissolved in toluene (2 mL). The mixture was stirred at room temperature for 1 hour. Ethyl 3-(5-(cyclopropanesulfonamido)pyridazin-3-yl)bicyclo[3.1.0]hexane-3-carboxylate **19e** (160.47 mg, 475.62 µmol) was added, and the mixture was heated to 90°C and stirred for 5 hours. After the reaction was completed, a small amount of methanol was added to quench the reaction. The mixture was concentrated under reduced pressure to remove the solvent. Dilute hydrochloric acid and dichloromethane were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(5-(cyclopropanesulfonamido)pyridazin-3-yl)-N-(4-(6-ethoxypyrazin-2-yl)phenyl)bicyclo[3.1.0 ]hexane-3-carboxamide **19** (8 mg) with a yield of 2.39%.

MS m/z (ESI): 520.8 [M+1]
1H NMR (400 MHz, DMSO-d6) δ 9.85 (s, 1H), 8.76 (d, J = 4.4 Hz, 1H), 8.18 (d, J = 5.1 Hz, 1H), 8.07 (t, J = 6.0 Hz, 2H), 7.70 (d, J = 8.5 Hz, 3H), 4.46 (q, J = 7.1 Hz, 2H), 3.09 - 2.88 (m, 2H), 2.62 (s, 1H), 2.19 (t, J = 13.5 Hz, 2H), 1.41 (dt, J = 14.2, 5.6 Hz, 5H), 0.92 (t, J = 8.2 Hz, 4H), 0.46 (t, J = 7.3 Hz, 1H), 0.12 (d, J = 4.6 Hz, 1H).

### Example 20

### 3-(4-(cyclopropanesulfonamido)pyridin-2-yl)-N-(4-(6-ethoxypyrazin-2-yl)-2-fluorophenyl)bicycl o[3.1.0]hexane-3-carboxamide

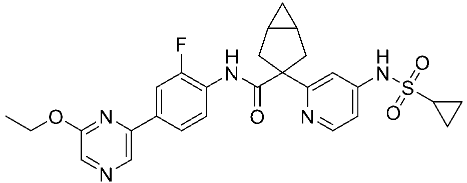

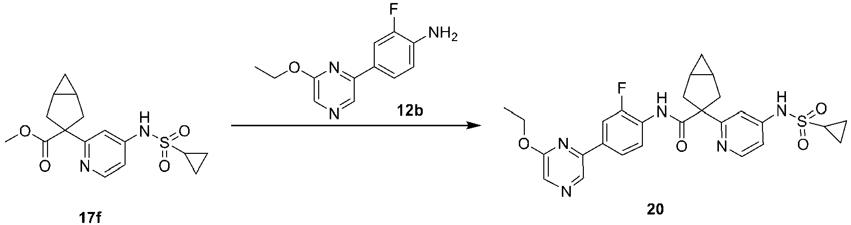

4-(6-Ethoxypyrazin-2-yl)-2-fluoroaniline **12b** (41.60 mg, 178.36 µmol) and trimethylaluminum (2.0 M toluene solution) (2 M, 267.53 µL) were dissolved in toluene (2.00 mL). The mixture was stirred at room temperature for 1 hour. Methyl 3-(4-(cyclopropanesulfonamido)pyridin-2-yl)bicyclo[3.1.0]hexane-3-carboxylate **17f** (50 mg, 148.63 µmol) was added, and the mixture was heated to 90°C and stirred for 5 hours. After the reaction was completed, a small amount of methanol was added to quench the reaction. The mixture was concentrated under reduced pressure to remove the solvent. Dilute hydrochloric acid and dichloromethane were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyridin-2-yl)-N-(4-(6-ethoxypyrazin-2-yl)-2-fluorophenyl)bicycl o[3.1.0]hexane-3-carboxamide **20** (21 mg) with a yield of 20.00%.

MS m/z (ESI): 537.8 [M+1]
1H NMR (400 MHz, DMSO-d6) δ 9.30 (s, 1H), 8.81 (s, 1H), 8.48 (d, J = 5.8 Hz, 1H), 8.23 (s, 1H), 8.02-7.90 (m, 2H), 7.82 (d, J = 8.2 Hz, 1H), 7.30 (d, J = 2.1 Hz, 1H), 7.22 (d, J = 5.9 Hz, 1H), 4.46 (q, J = 7.0 Hz, 2H), 2.89 (s, 1H), 2.82 - 2.72 (m, 2H), 2.61 (d, J = 13.7 Hz, 2H), 1.46 - 1.35 (m, 5H), 1.04 (ddt, J = 10.5, 4.6, 2.4 Hz, 4H), 0.37 (d, J = 5.7 Hz, 1H), -0.16 (d, J = 4.4 Hz, 1H).

### Example 21

### 3-(4-(cyclopropanesulfonamido)pyridin-2-yl)-N-(5-(6-ethoxypyrazin-2-yl)pyridin-2-yl)bicyclo[3. 1.0]hexane-3-carboxamide

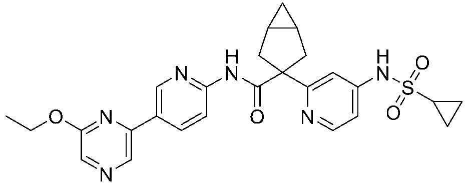

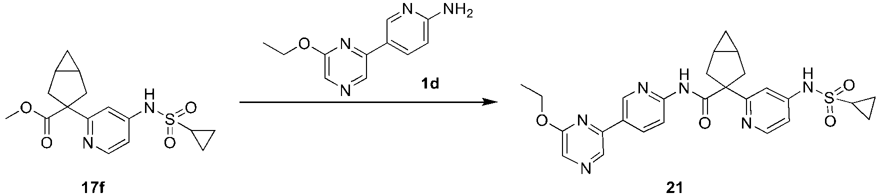

5-(6-Ethoxypyrazin-2-yl)pyridin-2-amine **1d** (38.57 mg, 178.36 µmol) and trimethylaluminum (2.0 M toluene solution) (2 M, 267.53 µL) were dissolved in toluene (2.00 mL). The mixture was stirred at room temperature for 1 hour. Methyl 3-(4-(cyclopropanesulfonamido)pyridin-2-yl)bicyclo[3.1.0]hexane-3-carboxylate **17f** (50 mg, 148.63 µmol) was added, and the mixture was heated to 90°C and stirred for 5 hours. After the reaction was completed, a small amount of methanol was added to quench the reaction. The mixture was concentrated under reduced pressure to remove the solvent. Dilute hydrochloric acid and dichloromethane were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyridin-2-yl)-N-(5-(6-ethoxypyrazin-2-yl)pyridin-2-yl)bicyclo[3. 1.0]hexane-3-carboxamide **21** (11 mg) with a yield of 11.00%.

MS m/z (ESI): 520.8 [M+1]
1H NMR (400 MHz, DMSO-d6) δ 9.65 (s, 1H), 8.99 (d, J = 2.4 Hz, 1H), 8.81 (s, 1H), 8.48 (dd, J = 8.5, 2.5 Hz, 2H), 8.24 (s, 1H), 8.10 (d, J = 8.8 Hz, 1H), 7.30 (d, J = 2.1 Hz, 1H), 7.19 (dd, J = 5.8, 2.1 Hz, 1H), 4.46 (q, J = 7.0 Hz, 2H), 2.89 (dq, J = 10.2, 3.9, 2.8 Hz, 1H), 2.78 (dd, J = 13.9, 4.0 Hz, 2H), 2.61 (d, J = 13.7 Hz, 2H), 1.40 (dt, J = 14.1, 7.2 Hz, 5H), 1.02 (ddt, J = 10.8, 8.4, 2.4 Hz, 4H), 0.37 (q, J = 7.2 Hz, 1H), -0.17 (d, J = 4.4 Hz, 1H).

### Example 22

### 5-(2-(cyclopropanesulfonamido)pyrimidin-4-yl)-N-(4-(6-ethoxypyrazin-2-yl)phenyl)-4,5,6,7-tetr ahydrobenzo[d]isoxazole-5-carboxamide

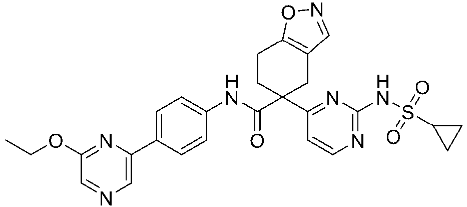

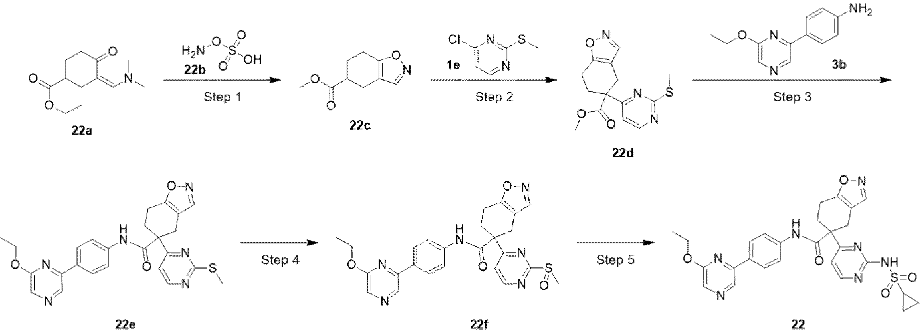

### Step 1

### methyl 4,5,6,7-tetrahydrobenzo[d]isoxazole-5-carboxylate

Ethyl 3-((dimethylamino)methylene)-4-oxocyclohexane-1-carboxylate **22a** (10 g, 44.39 mmol, prepared according to published patent "WO2008011130") and (aminooxy)sulfonic acid **22b** (5.52 g, 48.83 mmol, commercially available) were added to methanol (10 mL). The reaction was sealed and heated to 100°C with stirring for 18 hours. After the reaction was completed, the solvent was removed by concentration under reduced pressure. Saturated sodium bicarbonate solution was added to neutralize the mixture, and the mixture was extracted with dichloromethane (30 mL × 2). The combined organic phase was washed with saturated sodium chloride solution, dried over anhydrous sodium sulfate, and concentrated under reduced pressure. The residue was purified by silica gel column chromatography (eluent: System A) to obtain methyl 4,5,6,7-tetrahydrobenzo[d]isoxazole-5-carboxylate **22c** (3.2 g) with a yield of 39.79%.

MS m/z (ESI): 181.9 [M+1]

### Step 2

### methyl 5-(2-(methylthio)pyrimidin-4-yl)-4,5,6,7-tetrahydrobenzo[d]isoxazole-5-carboxylate

4-Chloro-2-(methylthio)pyrimidine **1e** (4 g, 24.90 mmol) and methyl 4,5,6,7-tetrahydrobenzo[d]isoxazole-5-carboxylate **22c** (6.32 g, 34.86 mmol) were dissolved in tetrahydrofuran (30 mL). The mixture was cooled to -30°C, and lithium bis(trimethylsilyl)amide (1 M, 74.71 mL) was added. The reaction was slowly heated to room temperature and stirred for 1 hour. The starting material was completely reacted. Saturated ammonium chloride solution (20 mL) was added to quench the reaction, and the mixture was extracted with ethyl acetate (20 mL × 3). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 5-(2-(methylthio)pyrimidin-4-yl)-4,5,6,7-tetrahydrobenzo[d]isoxazole-5-carboxylate **22d** (4.2 g) with a yield of 55.23%.

MS m/z (ESI): 306.2 [M+1]

### Step 3

### N-(4-(6-ethoxypyrazin-2-yl)phenyl)-5-(2-(methylthio)pyrimidin-4-yl)-4,5,6,7-tetrahydrobenzo[d] isoxazole-5-carboxamide

4-(6-Ethoxypyrazin-2-yl)aniline **3b** (504.02 mg, 2.34 mmol) and trimethylaluminum (2.0 M toluene solution) (2 M, 3.51 mL) were added to toluene (10 mL). The mixture was stirred at room temperature for 1 hour. Methyl 5-(2-(methylthio)pyrimidin-4-yl)-4,5,6,7-tetrahydrobenzo[d]isoxazole-5-carboxylate **22d** (858.00 mg, 2.81 mmol) was added, and the mixture was heated to 90°C with stirring for 18 hours. After the reaction was completed, a small amount of methanol was added to quench the reaction, and the solvent was removed by concentration under reduced pressure. Dilute hydrochloric acid and dichloromethane were added, and the insoluble solid was removed by filtration. The filtrate was concentrated under reduced pressure, and the crude product was purified by silica gel column chromatography (eluent: System B) to obtain N-(4-(6-ethoxypyrazin-2-yl)phenyl)-5-(2-(methylthio)pyrimidin-4-yl)-4,5,6,7-tetrahydrobenzo[d] isoxazole-5-carboxamide **22e** (200 mg) with a yield of 17.48%.

MS m/z (ESI): 489.1 [M+1]

### Step 4

### N-(4-(6-ethoxypyrazin-2-yl)phenyl)-5-(2-(methylsulfinyl)pyrimidin-4-yl)-4,5,6,7-tetrahydrobenz o[d]isoxazole-5-carboxamide

N-(4-(6-ethoxypyrazin-2-yl)phenyl)-5-(2-(methylthio)pyrimidin-4-yl)-4,5,6,7-tetrahydr obenzo[d]isoxazole-5-carboxamide **22e** (200 mg, 409.37 µmol) and meta-chloroperoxybenzoic acid (182.84 mg, 900.60 µmol, 85% purity) were dissolved in dichloromethane (5 mL). The mixture was stirred at room temperature for 18 hours. After the reaction was completed, the solvent was removed by concentration under reduced pressure. The crude product was purified by silica gel column chromatography (eluent: System B) to obtain N-(4-(6-ethoxypyrazin-2-yl)phenyl)-5-(2-(methylsulfinyl)pyrimidin-4-yl)-4,5,6,7-tetrahydrobenz o[d]isoxazole-5-carboxamide **22f** (120 mg) with a yield of 58.10%.

MS m/z (ESI): 505.5 [M+1]

### Step 5

### 5-(2-(cyclopropanesulfonamido)pyrimidin-4-yl)-N-(4-(6-ethoxypyrazin-2-yl)phenyl)-4,5,6,7-tetr ahydrobenzo[d]isoxazole-5-carboxamide

Under ice-water bath, cyclopropanesulfonamide (57.63 mg, 475.66 µmol) and sodium hydride (23.78 mg, 594.58 µmol, 60% purity) were dissolved in N,N-dimethylformamide (2 mL). The mixture was stirred for 0.5 hours. N-(4-(6-ethoxypyrazin-2-yl)phenyl)-5-(2-(methylsulfinyl)pyrimidin-4-yl)-4,5,6,7-tetrahydrobenz o[d]isoxazole-5-carboxamide **22f** (120 mg, 237.83 µmol) was added, and the mixture was slowly heated to room temperature and stirred for 5 hours. After the reaction was completed, water (10 mL) was added, and the mixture was extracted with ethyl acetate (10 mL × 3). The combined organic phase was washed with saturated sodium chloride solution, dried over anhydrous sodium sulfate, and concentrated under reduced pressure. The crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 5-(2-(cyclopropanesulfonamido)pyrimidin-4-yl)-N-(4-(6-ethoxypyrazin-2-yl)phenyl)-4,5,6,7-tetr ahydrobenzo[d]isoxazole-5-carboxamide **22** (4.25 mg) with a yield of 1.59%.

MS m/z (ESI): 561.7 [M+1]

### Example 23

### 3-(5-(cyclopropanesulfonamido)pyridin-3-yl)-N-(4-(6-ethoxypyrazin-2-yl)phenyl)bicyclo[3.1.0]h exane-3-carboxamide

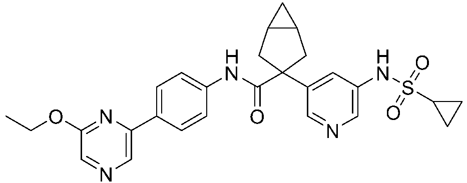

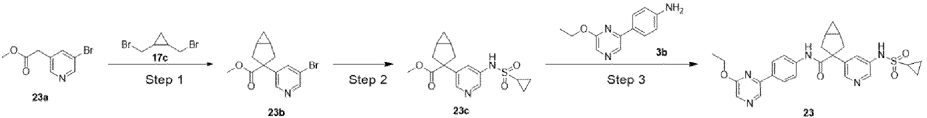

### Step 1

### methyl 3-(5-bromopyridin-3-yl)bicyclo[3.1.0]hexane-3-carboxylate

Methyl 2-(5-bromopyridin-3-yl)acetate **23a** (500 mg, 2.17 mmol, commercially available) was added to N,N-dimethylformamide (6 mL). The mixture was subjected to argon exchange three times. Under cooling with ice-water bath, sodium hydroxide (199.95 mg, 5.00 mmol) was slowly added. After stirring for 0.5 hours, 1,2-bis(bromomethyl)cyclopropane **17c** (643.97 mg, 2.83 mmol) was added, and the mixture was slowly heated to room temperature and stirred for 23 hours. After the reaction was completed, water (20 mL) was added to the reaction mixture, and the mixture was extracted with ethyl acetate (20 mL × 3). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 3-(5-bromopyridin-3-yl)bicyclo[3.1.0]hexane-3-carboxylate **23b** (600 mg) with a yield of 55.93%.

MS m/z (ESI): 296.3 [M+1]

### Step 2

### methyl 3-(5-(cyclopropanesulfonamido)pyridin-3-yl)bicyclo[3.1.0]hexane-3-carboxylate

Methyl 3-(5-bromopyridin-3-yl)bicyclo[3.1.0]hexane-3-carboxylate **23b** (800 mg, 2.70 mmol), cyclopropanesulfonamide (392.73 mg, 3.24 mmol), cesium carbonate (1.76 g, 5.40 mmol), 2-di-tert-butylphosphino-2',4',6'-triisopropylbiphenyl (114 mg, 270.06 µmol), and allylpalladium(II) chloride dimer (49.42 mg, 135.06 µmol) were sequentially added to 1,4-dioxane (10 mL). The mixture was subjected to argon exchange 3 times and heated to 90°C with stirring for 2 hours. After the reaction was completed, dichloromethane (30 mL) and methanol (5 mL) were added, and insoluble solid was removed by filtration. The filtrate was concentrated under reduced pressure, and the crude product was purified by silica gel column chromatography (eluent: System B) to obtain methyl 3-(5-(cyclopropanesulfonamido)pyridin-3-yl)bicyclo[3.1.0]hexane-3-carboxylate **23c** (230 mg) with a yield of 20.25%.

MS m/z (ESI): 336.2 [M+1]

### Step 3

### 3-(5-(cyclopropanesulfonamido)pyridin-3-yl)-N-(4-(6-ethoxypyrazin-2-yl)phenyl)bicyclo[3.1.0]h exane-3-carboxamide

4-(6-Ethoxypyrazin-2-yl)aniline **3b** (102.38 mg, 475.62 µmol) and trimethylaluminum (2.0 M toluene solution) (2 M, 856.11 µL) were dissolved in toluene (1.41 mL) and stirred at room temperature for 1 hour. Subsequently, methyl 3-(5-(cyclopropanesulfonamido)pyridin-3-yl)bicyclo[3.1.0]Thexane-3-carboxylate **23c** (160.00 mg, 475.62 µmol) was added, and the mixture was heated to 90°C and stirred for 5 hours. After the reaction was completed, a small amount of methanol was added to quench the reaction, and the solvent was removed by concentration under reduced pressure. Dilute hydrochloric acid and dichloromethane were added, and insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(5-(cyclopropanesulfonamido)pyridin-3-yl)-N-(4-(6-ethoxypyrazin-2-yl)phenyl)bicyclo[3.1.0]h exane-3-carboxamide **23** (55 mg) with a yield of 16.79%.

MS m/z (ESI): 520.8 [M+1]
1H NMR (400 MHz, DMSO-d6) δ 10.00 (s, 1H), 9.45 (d, J = 97.4 Hz, 1H), 8.42 (dd, J = 108.2, 46.5 Hz, 3H), 8.17-7.71 (m, 4H), 7.58 (d, J = 7.7 Hz, 2H), 4.31 (p, J = 7.3 Hz, 2H), 3.03 (d, J = 14.2 Hz, 1H), 2.78 (d, J = 13.5 Hz, 1H), 2.59-2.47 (m, 1H), 2.10 (dd, J = 34.2, 13.6 Hz, 2H), 1.29-1.08 (m, 5H), 0.77 (d, J = 6.5 Hz, 4H), 0.32 (t, J = 16.5 Hz, 1H), 0.05 (m, 1H).

### Example 24

### 3-(6-(cyclopropanesulfonamido)pyrimidin-4-yl)-N-(5-(6-ethoxypyrazin-2-yl)pyridin-2-yl)bicyclo [3.1.0]hexane-3-carboxamide

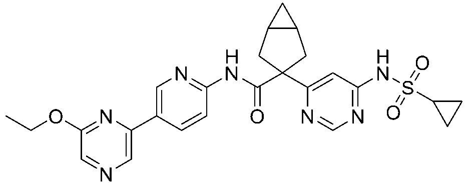

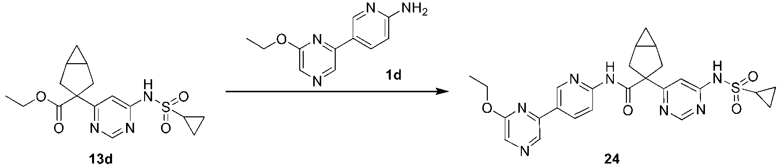

5-(6-Ethoxypyrazin-2-yl)pyridin-2-amine **1d** (40 mg, 184.98 µmol) and trimethylaluminum (2.0 M toluene solution) (2 M, 277.47 µL) were added to toluene (2 mL) and stirred at room temperature for 1 hour. Ethyl 3-(6-(cyclopropanesulfonamido)pyrimidin-4-yl)bicyclo[3.1.0]hexane-3-carboxylate **13d** (62.41 mg, 184.98 µmol) was added, and the mixture was heated to 90°C and stirred for 5 hours. After the reaction was completed, the mixture was cooled to room temperature, and a small amount of methanol was added to quench the reaction. The solvent was removed by concentration under reduced pressure. Dilute hydrochloric acid (1 mL, 1 M) and dichloromethane (10 mL) were added, and insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyrimidin-4-yl)-N-(5-(6-ethoxypyrazin-2-yl)pyridin-2-yl)bicyclo [3.1.0]hexane-3-carboxamide **24** (5.46 mg) with a yield of 4.46%.

MS m/z (ESI): 521.8 [M+1]
1H NMR (400 MHz, DMSO-d6) δ 10.18 (s, 1H), 9.00 (d, J = 2.4 Hz, 1H), 8.83 (s, 1H), 8.52-8.40 (m, 2H), 8.30-8.15 (m, 2H), 6.80 (d, J = 5.7 Hz, 1H), 4.48 (d, J = 7.0 Hz, 2H), 3.13-3.07 (m, 1H), 2.80 (d, J = 13.6 Hz, 2H), 2.58 (dd, J = 13.0, 10.0 Hz, 2H), 1.40 (t, J = 7.0 Hz, 5H), 1.06 (dt, J = 6.7, 3.4 Hz, 2H), 0.91-0.82 (m, 3H), 0.39 (d, J = 6.6 Hz, 1H), 0.13 (d, J = 4.7 Hz, 1H).

### Example 25

### 3-(6-(cyclopropanesulfonamido)pyridin-2-yl)-N-(4-(6-ethoxypyrazin-2-yl)phenyl)bicyclo[3.1.0]h exane-3-carboxamide

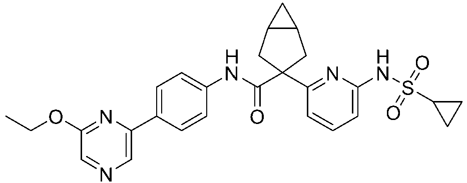

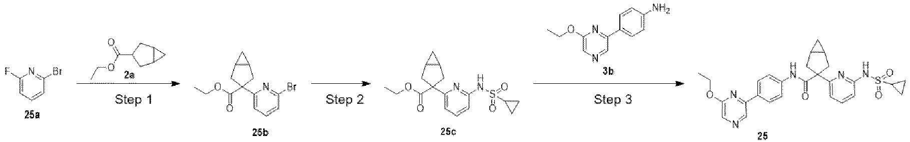

### Step 1

### ethyl 3-(6-bromopyridin-2-yl)bicyclo[3.1.0]hexane-3-carboxylate

At -30°C, lithium hexamethyldisilazide (1.2 M tetrahydrofuran solution) (1 M, 7.78 mL) was added to a solution of 2-bromo-6-fluoropyridine **25a** (547.80 mg, 3.11 mmol, commercially available) and ethyl bicyclo[3.1.0]hexane-3-carboxylate **2a** (400 mg, 2.59 mmol) in tetrahydrofuran (5 mL). The mixture was slowly heated to room temperature and stirred for 3 hours. After the reaction was completed, saturated ammonium chloride solution (20 mL) was added to quench the reaction, and the mixture was extracted with ethyl acetate (20 mL × 3). The combined organic phase was washed with saturated sodium chloride solution, dried over anhydrous sodium sulfate, filtered, and evaporated to dryness by rotation. The crude product was purified by silica gel column chromatography (eluent: System A) to obtain ethyl 3-(6-bromopyridin-2-yl)bicyclo[3.1.0]hexane-3-carboxylate **25b** (700 mg) with a yield of 87.00%.

MS m/z (ESI): 357.1 [M+1]

### Step 2

### ethyl 3-(6-(cyclopropanesulfonamido)pyridin-2-yl)bicyclo[3.1.0]hexane-3-carboxylate

Ethyl 3-(6-bromopyridin-2-yl)bicyclo[3.1.0]hexane-3-carboxylate **25b** (800 mg, 2.58 mmol), cyclopropanesulfonamide (468.72 mg, 3.87 mmol), cesium carbonate (1.68 g, 5.16 mmol), 2-di-tert-butylphosphino-2',4',6'-triisopropylbiphenyl (109 mg, 257 µmol), and allylpalladium(II) chloride dimer (47.18 mg, 128.96 µmol) were sequentially added to 1,4-dioxane (10 mL). The mixture was subjected to argon exchange three times and heated to 90°C with stirring for 2 hours. After the reaction was completed, the solvent was removed by concentration under reduced pressure. The crude product was purified by silica gel column chromatography (eluent: System B) to obtain ethyl 3-(6-(cyclopropanesulfonamido)pyridin-2-yl)bicyclo[3.1.0]hexane-3-carboxylate **25c** (450 mg) with a yield of 49.79%.

MS m/z (ESI): 350.9 [M+1]

### Step 3

### 3-(6-(cyclopropanesulfonamido)pyridin-2-yl)-N-(4-(6-ethoxypyrazin-2-yl)phenyl)bicyclo[3.1.0]h exane-3-carboxamide

4-(6-Ethoxypyrazin-2-yl)aniline **3b** (99.82 mg, 463.73 µmol) and trimethylaluminum (2.0 M toluene solution) (2 M, 642.08 µL) were added to toluene (1.5 mL) and stirred at room temperature for 1 hour. Subsequently, ethyl 3-(6-(cyclopropanesulfonamido)pyridin-2-yl)bicyclo[3.1.0]hexane-3-carboxylate **25c** (125.00 mg, 356.71 µmol) was added, and the mixture was heated to 90°C and stirred for 5 hours. After the reaction was completed, the mixture was cooled to room temperature, and a small amount of methanol was added to quench the reaction. The solvent was removed by concentration under reduced pressure. Dilute hydrochloric acid (1 mL, 1 M) and dichloromethane (10 mL) were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyridin-2-yl)-N-(4-(6-ethoxypyrazin-2-yl)phenyl)bicyclo[3.1.0]h exane-3-carboxamide **25** (80 mg) with a yield of 34.64%.

MS m/z (ESI): 519.8 [M+1]
1H NMR (400 MHz, DMSO-d6) δ 10.51 (s, 1H), 9.32 (s, 1H), 8.60 (s, 1H), 8.02 (s, 1H), 7.90 (d, J = 8.3 Hz, 2H), 7.60 (d, J = 8.4 Hz, 2H), 7.51 (t, J = 7.8 Hz, 1H), 6.81 (d, J = 7.7 Hz, 1H), 6.63 (d, J = 8.1 Hz, 1H), 4.31 (q, J = 7.0 Hz, 2H), 3.13 - 3.04 (m, 1H), 2.77 (d, J = 13.4 Hz, 2H), 2.27 (d, J = 13.2 Hz, 2H), 1.23 (t, J = 6.9 Hz, 5H), 0.98 - 0.89 (m, 2H), 0.83 (d, J = 7.4 Hz, 2H), 0.24 (q, J = 7.0 Hz, 1H), -0.00 (q, J = 4.3 Hz, 1H).

### Example 26

### 3-(6-(cyclopropanesulfonamido)pyrimidin-4-yl)-N-(4-(6-ethoxypyrazin-2-yl)-2-fluorophenyl)bic yclo[3.1.0]hexane-3-carboxamide

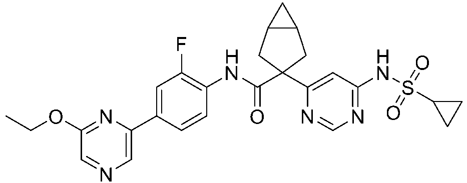

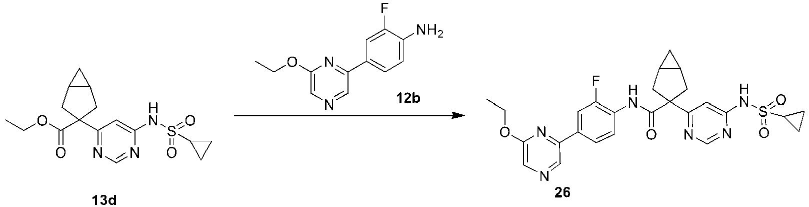

4-(6-Ethoxypyrazin-2-yl)-2-fluoroaniline **12b** (82.96 mg, 355.67 µmol) and trimethylaluminum (2.0 M toluene solution) (2 M, 533.50 µL) were dissolved in toluene (1.73 mL) and stirred at room temperature for 1 hour. Subsequently, ethyl 3-(6-(cyclopropanesulfonamido)pyrimidin-4-yl)bicyclo[3.1.0]hexane-3-carboxylate 13d (100 mg, 296.39 µmol) was added, and the mixture was heated to 90°C and stirred for 5 hours. After the reaction was completed, the mixture was cooled to room temperature, and a small amount of methanol was added to quench the reaction. The solvent was removed by concentration under reduced pressure. Dilute hydrochloric acid (1 mL, 1 M) and dichloromethane (10 mL) were added, and the insoluble solid was removed by filtration. The filtrate was concentrated under reduced pressure, and the crude product was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyrimidin-4-yl)-N-(4-(6-ethoxypyrazin-2-yl)-2-fluorophenyl)bic yclo[3.1.0]hexane-3-carboxamide **26** (49.42 mg) with a yield of 24.70%.

MS m/z (ESI): 538.8 [M+1]
1H NMR (400 MHz, DMSO-d6) δ 9.43 (s, 1H), 8.57 (d, J = 17.4 Hz, 2H), 7.73 (t, J = 10.3 Hz, 2H), 7.36 (t, J = 8.0 Hz, 1H), 6.71 (s, 1H), 4.23 (q, J = 7.1 Hz, 2H), 2.84 (s, 1H), 2.60 (d, J = 13.5 Hz, 2H), 2.17 (d, J = 13.3 Hz, 2H), 1.20 - 1.12 (m, 5H), 0.90 - 0.77 (m, 4H), 0.18 (d, J = 7.0 Hz, 1H), -0.01 (d, J = 5.9 Hz, 1H).

### Example 27

### (1R,5S)-6-(6-(cyclopropanesulfonamido)pyrimidin-4-yl)-N-(4-(6-ethoxypyrazin-2-yl)phenyl)-3-o xabicyclo[3.1.0]hexane-6-carboxamide

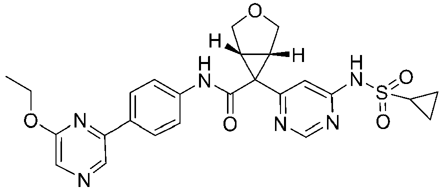

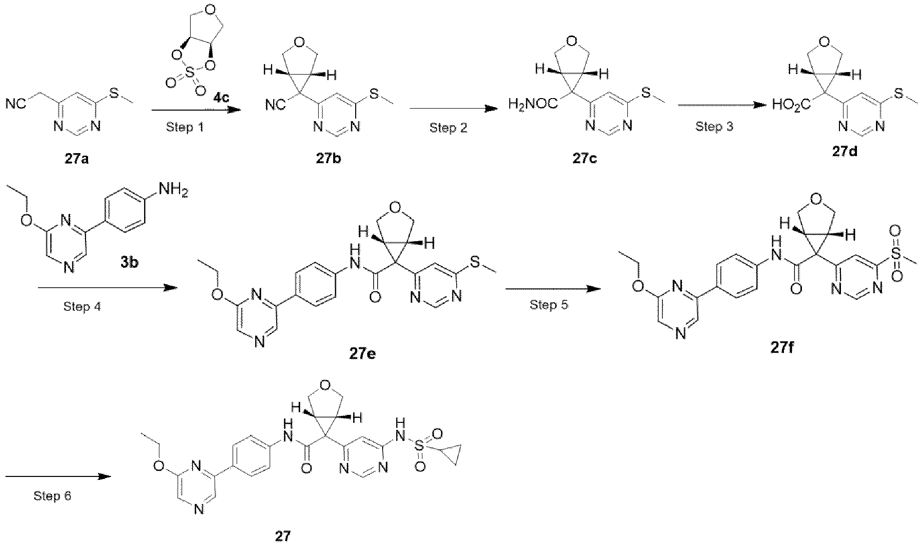

### Step 1

### (1R,5S)-6-(6-(methylthio)pyrimidin-4-yl)-3-oxabicyclo[3.1.0]hexane-6-carbonitrile

Under ice-water bath, 2-(6-(methylthio)pyrimidin-4-yl)acetonitrile **27a** (700 mg, 4.24 mmol, prepared according to published patent "WO 2008042639A1") was dissolved in anhydrous dimethoxyethane (28 mL). Sodium hydride (372.85 mg, 9.32 mmol, 60% purity) was added, and the mixture was stirred at room temperature for 0.5 hours. (3aR,6aS)-Tetrahydrofuro[3,4-d][1,3,2]dioxathiole 2,2-dioxide **4c** (844.76 mg, 5.08 mmol) was added, and the mixture was heated to 60°C and stirred for 18 hours. Water (50 mL) and ethyl acetate (50 mL) were added, and the mixture was partitioned. The organic phase was dried over anhydrous sodium sulfate, filtered, and evaporated to dryness by rotation. The residue was purified by silica gel column chromatography (eluent: System B) to obtain (1R,5S)-6-(6-(methylthio)pyrimidin-4-yl)-3-oxabicyclo[3.1.0]hexane-6-carbonitrile **27b** (520 mg) with a yield of 52.61%.

MS m/z (ESI): 233.2 [M+1]

### Step 2

### (1R,5S)-6-(6-(methylthio)pyrimidin-4-yl)-3-oxabicyclo[3.1.0]hexane-6-carboxamide

(1R,5S)-6-(6-(methylthio)pyrimidin-4-yl)-3-oxabicyclo[3.1.0]hexane-6-carbonitrile **27b** (520 mg, 2.23 mmol) was added to dimethyl sulfoxide (3 mL). Under ice-water bath, sodium hydroxide (178.32 mg, 4.46 mmol) was added, and the mixture was stirred for 10 minutes. Hydrogen peroxide (1.5 mL) was added, and the mixture was stirred at room temperature for 3 hours. Water (10 mL) was added to quench the reaction, and the solid was collected by filtration and dried to obtain (1R,5S)-6-(6-(methylthio)pyrimidin-4-yl)-3-oxabicyclo[3.1.0]hexane-6-carboxamide 27c (500 mg) with a yield of 89.26%.

MS m/z (ESI): 251.0 [M+1]

### Step 3

### (1R,5S)-6-(6-(methylthio)pyrimidin-4-yl)-3-oxabicyclo[3.1.0]hexane-6-carboxylic acid

(1R,5S)-6-(6-(methylthio)pyrimidin-4-yl)-3-oxabicyclo[3.1.0]hexane-6-carboxamide **27c** (560 mg, 2.23 mmol), acetic acid (1.50 mL), and acetic anhydride (3 mL) were added to dimethyl sulfoxide (3 mL). Under ice-water bath, sodium nitrite (1.54 g, 22.28 mmol) was added, and the mixture was stirred at room temperature overnight. The reaction mixture was poured into water (10 mL) and neutralized with saturated sodium bicarbonate solution. Saturated citric acid solution was added to adjust the pH to acidic. The mixture was extracted with ethyl acetate (20 mL × 3). The aqueous phase was collected and purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(methylthio)pyrimidin-4-yl)-3-oxabicyclo[3.1.0]hexane-6-carboxylic acid 27d (210 mg) with a yield of 37.35%.

MS m/z (ESI): 253.0 [M+1]

### Step 4

### (1R,5S)-N-(4-(6-ethoxypyrazin-2-yl)phenyl)-6-(6-(methylthio)pyrimidin-4-yl)-3-oxabicyclo[3.1. 0]hexane-6-carboxamide

Under ice-water bath, (1R,5S)-6-(6-(methylthio)pyrimidin-4-yl)-3-oxabicyclo[3.1.0]hexane-6-carboxylic acid **27d** (210 mg, 832.38 µmol), N-methylpyrrolidone (2 mL), N,N,N',N'-tetramethylchloroformamidinium hexafluorophosphate (466.13 mg, 1.66 mmol), and N-methylimidazole (273.35 mg, 3.33 mmol) were added to a 15 mL single-neck flask. The reaction was performed at room temperature for 0.5 hours. 4-(6-Ethoxypyrazin-2-yl)aniline **3b** (197.09 mg, 915.62 µmol) was added, and the mixture was heated to 100°C and stirred for 5 hours. After the reaction was completed, water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain (1R,5S)-N-(4-(6-ethoxypyrazin-2-yl)phenyl)-6-(6-(methylthio)pyrimidin-4-yl)-3-oxabicyclo[3.1. 0]hexane-6-carboxamide **27e** (350 mg) with a yield of 93.54%.

MS m/z (ESI): 450.1 [M+1]

### Step 5

### (1R,5S)-N-(4-(6-ethoxypyrazin-2-yl)phenyl)-6-(6-(methylsulfonyl)pyrimidin-4-yl)-3-oxabicyclo[ 3.1.0]hexane-6-carboxamide

(1R,5S)-N-(4-(6-ethoxypyrazin-2-yl)phenyl)-6-(6-(methylthio)pyrimidin-4-yl)-3-oxabic yclo[3.1.0]hexane-6-carboxamide **27e** (350 mg, 778.60 µmol) and meta-chloroperoxybenzoic acid (316.15 mg, 1.56 mmol, 85% purity) were added to dichloromethane (5 mL). The mixture was stirred at room temperature overnight. Saturated sodium bicarbonate solution was added to neutralize the mixture, and the mixture was extracted with dichloromethane (20 mL × 3). The combined organic phase was dried over anhydrous sodium sulfate, filtered, and concentrated under reduced pressure to obtain (1R,5S)-N-(4-(6-ethoxypyrazin-2-yl)phenyl)-6-(6-(methylsulfonyl)pyrimidin-4-yl)-3-oxabicyclo[ 3.1.0]hexane-6-carboxamide **27f (350** mg) with a yield of 96.56%.

MS m/z (ESI): 465.5 [M+1]

### Step 6

### (1R,5S)-6-(6-(cyclopropanesulfonamido)pyrimidin-4-yl)-N-(4-(6-ethoxypyrazin-2-yl)phenyl)-3-o xabicyclo[3.1.0]hexane-6-carboxamide

(1R,5S)-N-(4-(6-ethoxypyrazin-2-yl)phenyl)-6-(6-(methylsulfonyl)pyrimidin-4-yl)-3-ox abicyclo[3.1.0]hexane-6-carboxamide **27f** (360 mg, 773.32 µmol), cyclopropanesulfonamide (93.69 mg, 773.32 µmol), and cesium carbonate (503.93 mg, 1.55 mmol) were added to N-methylpyrrolidone (1.5 mL). The mixture was heated to 100°C and the reaction was performed for 2 hours. The reaction mixture was directly added to water (2 mL) and methanol (2 mL) and purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(cyclopropanesulfonamido)pyrimidin-4-yl)-N-(4-(6-ethoxypyrazin-2-yl)phenyl)-3-o xabicyclo[3.1.0]hexane-6-carboxamide **27** (80 mg) with a yield of 15.83%.

MS m/z (ESI): 523.1 [M+1].

¹H NMR (400 MHz, DMSO-d₆) δ 10.42 (s, 1H), 8.76 (d, J = 21.9 Hz, 2H), 8.20 (s, 1H), 8.11 (d, J = 8.3 Hz, 2H), 7.82 (d, J = 8.3 Hz, 2H), 7.01 (s, 1H), 4.48 (q, J = 7.0 Hz, 2H), 4.11 (d, J = 8.9 Hz, 2H), 3.83 (d, J = 8.8 Hz, 2H), 3.04 (s, 1H), 1.40 (t, J = 7.0 Hz, 3H), 1.03 (dd, J = 12.9, 6.6 Hz, 4H).

### Example 28

### (1R,5S)-6-(4-(cyclopropanesulfonamido)pyridin-2-yl)-N-(4-(6-ethoxypyrazin-2-yl)phenyl)bicycl o[3.1.0]hexane-6-carboxamide

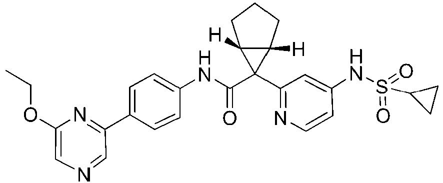

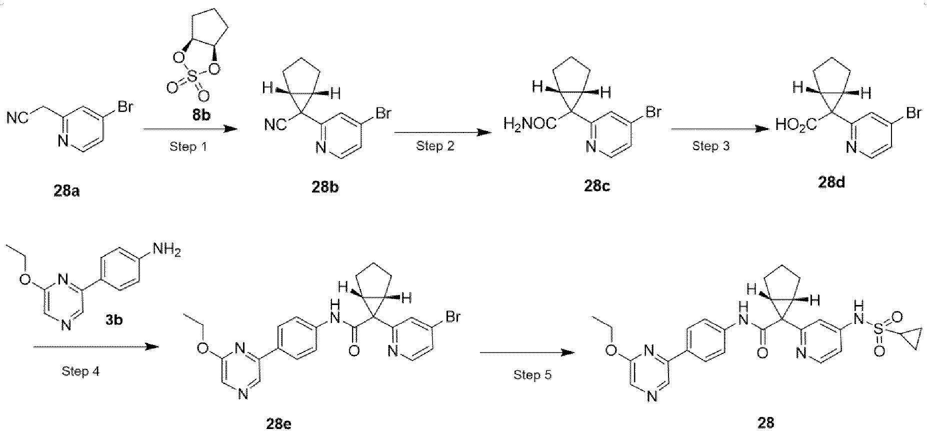

### Step 1

### (1R,5S)-6-(4-bromopyridin-2-yl)bicyclo[3.1.0]hexane-6-carbonitrile

Under ice-water bath, 2-(4-bromopyridin-2-yl)acetonitrile **28a** (500 mg, 2.54 mmol) was added to anhydrous dimethoxyethane (20 mL). Sodium hydride (223.31 mg, 5.58 mmol, 60% purity) was added, and the mixture was stirred at room temperature for 0.5 hours. (3aR,6aS)-Tetrahydro-4H-cyclopenta[d][1,3,2]dioxathiole 2,2-dioxide **8b** (499.96 mg, 3.05 mmol) was added, and the mixture was heated to 60°C and stirred for 18 hours. Water (50 mL) and ethyl acetate (50 mL) were added, and the mixture was separated. The organic phase was dried over anhydrous sodium sulfate, filtered, and evaporated to dryness by rotation. The residue was purified by silica gel column chromatography (eluent: System B) to obtain (1R,5S)-6-(4-bromopyridin-2-yl)bicyclo[3.1.0]hexane-6-carbonitrile **28b** (260 mg) with a yield of 94%.

MS m/z (ESI): 263.0 [M+1]

### Step 2

### (1R,5S)-6-(4-bromopyridin-2-yl)bicyclo[3.1.0]hexane-6-carboxamide

(1R,5S)-6-(4-Bromopyridin-2-yl)bicyclo[3.1.0]hexane-6-carbonitrile **28b** (260 mg, 988.09 µmol), sodium hydroxide (79.05 mg, 1.98 mmol), and hydrogen peroxide (1.5 mL) were sequentially added to dimethyl sulfoxide (1.5 mL). The mixture was heated to 40°C and stirred for 3 hours. Water (10 mL) was added to quench the reaction, and the mixture was stirred for 0.5 hours. The solid was collected by filtration. The aqueous phase was extracted with ethyl acetate (10 mL × 3). The organic phase was dried over anhydrous sodium sulfate, filtered, and evaporated to dryness by rotation, then combined with the collected solid and dried to obtain (1R,5S)-6-(4-bromopyridin-2-yl)bicyclo[3.1.0]hexane-6-carboxamide **28c** (260 mg) with a yield of 93.59%.

MS m/z (ESI): 281.5 [M+1]

### Step 3

### (1R,5S)-6-(4-bromopyridin-2-yl)bicyclo[3.1.0]hexane-6-carboxylic acid

(1R,5S)-6-(4-bromopyridin-2-yl)bicyclo[3.1.0]hexane-6-carboxamide **28c** (293.18 mg, 1.04 mmol), acetic acid (1.50 mL), and acetic anhydride (3 mL) were added to dimethyl sulfoxide (3 mL). Under ice-water bath, sodium nitrite (719.53 mg, 10.43 mmol) was added, and the mixture was stirred at room temperature overnight. The reaction mixture was poured into water (10 mL) and neutralized with saturated sodium bicarbonate solution. Saturated citric acid solution was added to adjust the pH to acidic. The mixture was extracted with ethyl acetate (20 mL × 3). The aqueous phase was collected and purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-bromopyridin-2-yl)bicyclo[3.1.0]hexane-6-carboxylic acid **28d** (210 mg) with a yield of 71.38%.

MS m/z (ESI): 282.0 [M+1]

### Step 4

### (1R,5S)-6-(4-bromopyridin-2-yl)-N-(4-(6-ethoxypyrazin-2-yl)phenyl)bicyclo[3.1.0]hexane-6-car boxamide

Under ice-water bath, (1R,5S)-6-(4-bromopyridin-2-yl)bicyclo[3.1.0]hexane-6-carboxylic acid **28d** (180 mg, 638.00 µmol), N-methylpyrrolidone (1 mL), N,N,N',N'-tetramethylchloroformamidinium hexafluorophosphate (357.28 mg, 1.28 mmol), and N-methylimidazole (209.52 mg, 2.55 mmol) were added to a 15 mL single-neck flask. The reaction was performed at room temperature for 0.5 hours. 4-(6-Ethoxypyrazin-2-yl)aniline **3b** (144.20 mg, 669.90 µmol) was added, and the mixture was heated to 65°C and stirred for 5 hours. Water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain (1R,5S)-6-(4-bromopyridin-2-yl)-N-(4-(6-ethoxypyrazin-2-yl)phenyl)bicyclo[3.1.0]hexane-6-car boxamide **28e** (50 mg) with a yield of 16.35%.

MS m/z (ESI): 480.1 [M+1]

### Step 5

### (1R,5S)-6-(4-(cyclopropanesulfonamido)pyridin-2-yl)-N-(4-(6-ethoxypyrazin-2-yl)phenyl)bicycl o[3.1.0]hexane-6-carboxamide

(1R,5S)-6-(4-bromopyridin-2-yl)-N-(4-(6-ethoxypyrazin-2-yl)phenyl)bicyclo[3.1.0] hexane-6-carboxamide **28e** (200 mg, 417.22 µmol), cyclopropanesulfonamide (75.82 mg, 625.82 µmol), cesium carbonate (271.87 mg, 834.43 µmol), 2-di-tert-butylphosphino-2',4',6'-triisopropylbiphenyl (17.72 mg, 41.72 µmol), and allylpalladium(II) chloride dimer (7.63 mg, 20.86 µmol) were sequentially added to 1,4-dioxane (3 mL). The mixture was subjected to argon exchange three times and heated to 90°C with stirring for 2 hours. The mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-(cyclopropanesulfonamido)pyridin-2-yl)-N-(4-(6-ethoxypyrazin-2-yl)phenyl)bicycl o[3.1.0]hexane-6-carboxamide **28** (26 mg) with a yield of 97.6%.

MS m/z (ESI): 350.9 [M+1]
¹H NMR (400 MHz, DMSO-d₆) δ 10.53 (s, 1H), 8.77 (s, 1H), 8.39 (d, J = 6.1 Hz, 1H), 8.24 - 8.06 (m, 3H), 7.84 (d, J = 8.4 Hz, 2H), 7.36 (s, 1H), 7.22 (d, J = 6.1 Hz, 1H), 4.48 (q, J = 7.1 Hz, 2H), 2.86 (d, J = 7.3 Hz, 1H), 2.25 - 2.10 (m, 4H), 2.01 (q, J = 10.9 Hz, 2H), 1.68 (d, J = 10.3 Hz, 1H), 1.40 (t, J = 7.0 Hz, 3H), 1.31 - 1.15 (m, 1H), 1.16 - 0.94 (m, 5H).

### Example 29

### (1R,5S)-6-(6-(cyclopropanesulfonamido)pyrimidin-4-yl)-N-(4-(6-ethoxypyrazin-2-yl)phenyl)bic yclo[3.1.0]hexane-6-carboxamide

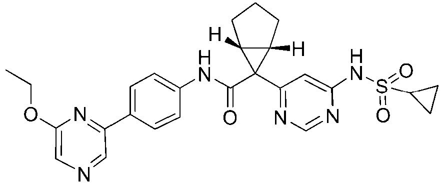

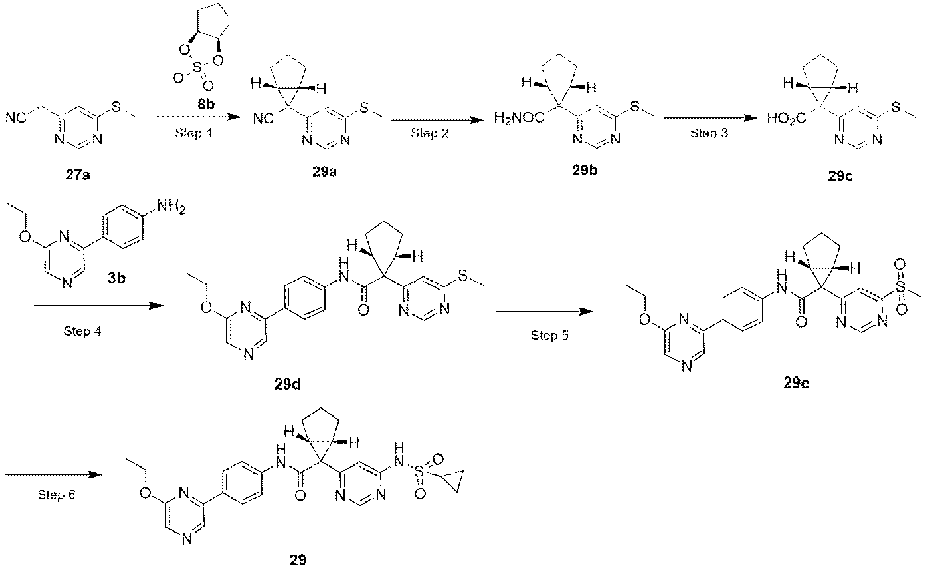

### Step 1

### (1R,5S)-6-(6-(methylthio)pyrimidin-4-yl)bicyclo[3.1.0]hexane-6-carbonitrile

Under ice-water bath, 2-(2-(methylthio)pyrimidin-4-yl)acetonitrile 27a (800 mg, 4.84 mmol) was dissolved in anhydrous dimethoxyethane (20 mL). Sodium hydride (426.11 mg, 10.65 mmol, 60% purity) was added, and the mixture was stirred at room temperature for 0.5 hours. (3aR,6aS)-Tetrahydro-4H-cyclopenta[d][1,3,2]dioxathiole 2,2-dioxide 8b (953.98 mg, 5.81 mmol) was added, and the mixture was heated to 60°C and stirred for 18 hours. Water (50 mL) and ethyl acetate (50 mL) were added, and the mixture was separated. The organic phase was dried over anhydrous sodium sulfate, filtered, and evaporated to dryness by rotation. The residue was purified by silica gel column chromatography (eluent: System B) to obtain (1R,5S)-6-(6-(methylthio)pyrimidin-4-yl)bicyclo[3.1.0]hexane-6-carbonitrile **29a** (600 mg) with a yield of 53.57%.

MS m/z (ESI): 231.2 [M+1]

### Step 2

### (1R,5S)-6-(6-(methylthio)pyrimidin-4-yl)bicyclo[3.1.0]hexane-6-carboxamide

(1R,5S)-6-(6-(methylthio)pyrimidin-4-yl)bicyclo[3.1.0]hexane-6-carbonitrile **29a** (600 mg, 2.59 mmol) was added to dimethyl sulfoxide (3 mL). Under ice-water bath, sodium hydroxide (207.51 mg, 5.19 mmol) was added. After stirring for 10 minutes, hydrogen peroxide (1.5 mL) was added, and the mixture was stirred at room temperature for 3 hours. Water (10 mL) was added to quench the reaction. The solid was collected by filtration and dried to obtain (1R,5S)-6-(6-(methylthio)pyrimidin-4-yl)bicyclo[3.1.0]hexane-6-carboxamide **29b** (550 mg) with a yield of 85.04%. MS m/z (ESI): 249.0 [M+1]

### Step 3

### (1R,5S)-6-(6-(methylthio)pyrimidin-4-yl)bicyclo[3.1.0]hexane-6-carboxylic acid

(1R,5S)-6-(6-(methylthio)pyrimidin-4-yl)bicyclo[3.1.0]hexane-6-carboxamide **29b** (550 mg, 2.21 mmol), acetic acid (1.50 mL), and acetic anhydride (3 mL) were added to dimethyl sulfoxide (3 mL). Under ice-water bath, sodium nitrite (1.52 g, 22.06 mmol) was added, and the mixture was stirred at room temperature overnight. The reaction mixture was poured into water (10 mL) and neutralized with saturated sodium bicarbonate solution. Saturated citric acid solution was added to adjust the pH to acidic. The mixture was extracted with ethyl acetate (20 mL × 3). The aqueous phase was collected and purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(methylthio)pyrimidin-4-yl)bicyclo[3.1.0]hexane-6-carboxylic acid **29c** (350 mg) with a yield of 63.39%.

MS m/z (ESI): 251.0 [M+1]

### Step 4

### (1R,5S)-N-(4-(6-ethoxypyrazin-2-yl)phenyl)-6-(6-(methylthio)pyrimidin-4-yl)bicyclo[3.1.0]hexa ne-6-carboxamide

Under ice-water bath, (1R,5S)-6-(6-(methylthio)pyrimidin-4-yl)bicyclo[3.1.0]hexane-6-carboxylic acid **29c** (80 mg, 319.60 µmol), N-methylpyrrolidone (1 mL), N,N,N',N'-tetramethylchloroformamidinium hexafluorophosphate (178.97 mg, 639.19 µmol), and N-methylimidazole (104.96 mg, 1.28 mmol) were added to a 15 mL single-neck flask. The reaction was performed at room temperature for 0.5 hours. 4-(6-Ethoxypyrazin-2-yl)aniline **3b** (72.23 mg, 335.58 µmol) was added, and the mixture was heated to 100°C and stirred for 18 hours. After the reaction was completed, water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain (1R,5S)-N-(4-(6-ethoxypyrazin-2-yl)phenyl)-6-(6-(methylthio)pyrimidin-4-yl)bicyclo[3.1.0]hexa ne-6-carboxamide **29d** (130 mg) with a yield of 90.89%.

MS m/z (ESI): 448.1 [M+1]

### Step 5

### (1R,5S)-N-(4-(6-ethoxypyrazin-2-yl)phenyl)-6-(6-(methylsulfonyl)pyrimidin-4-yl)bicyclo[3.1.0] hexane-6-carboxamide

(1R,5S)-N-(4-(6-ethoxypyrazin-2-yl)phenyl)-6-(6-(methylthio)pyrimidin-4-yl)bicyclo[3 .1.0]hexane-6-carboxamide **29d** (140 mg, 312.81 µmol) and meta-chloroperoxybenzoic acid (101.61 mg, 500.50 µmol, 85% purity) were added to dichloromethane (2 mL) and stirred at room temperature overnight. Saturated sodium bicarbonate solution was added to neutralize the mixture, and the mixture was extracted with dichloromethane (10 mL × 3). The combined organic phase was dried over anhydrous sodium sulfate, filtered, and concentrated under reduced pressure to obtain (1R,5S)-N-(4-(6-ethoxypyrazin-2-yl)phenyl)-6-(6-(methylsulfonyl)pyrimidin-4-yl)bicyclo[3.1.0] hexane-6-carboxamide 29e (140 mg) with a yield of 96.55%.

MS m/z (ESI): 464.1 [M+1]

### Step 6

### (1R,5S)-6-(6-(cyclopropanesulfonamido)pyrimidin-4-yl)-N-(4-(6-ethoxypyrazin-2-yl)phenyl)bic yclo[3.1.0]hexane-6-carboxamide

(1R,5S)-N-(4-(6-ethoxypyrazin-2-yl)phenyl)-6-(6-(methylsulfonyl)pyrimidin-4-yl)bicyc lo[3.1.0]hexane-6-carboxamide **29e** (140 mg, 302.02 µmol), cyclopropanesulfonamide (43.91 mg, 362.42 µmol), and cesium carbonate (196.81 mg, 604.03 µmol) were added to N-methylpyrrolidone (1 mL). The mixture was heated to 100°C and the reaction was performed for 2 hours. The reaction mixture was cooled to room temperature and directly purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(cyclopropanesulfonamido)pyrimidin-4-yl)-N-(4-(6-ethoxypyrazin-2-yl)phenyl)bic yclo[3.1.0]hexane-6-carboxamide **29** (40 mg) with a yield of 19.62%.

MS m/z (ESI): 521.3 [M+1].

¹H NMR (400 MHz, DMSO-d₆) δ 10.47 (s, 1H), 8.77 (s, 1H), 8.68 (s, 1H), 8.19 (s, 1H), 8.11 (d, J = 8.4 Hz, 2H), 7.83 (d, J = 8.4 Hz, 2H), 6.95 (s, 1H), 4.48 (d, J = 7.0 Hz, 2H), 3.02 (s, 1H), 2.21 (d, J = 3.0 Hz, 2H), 2.10 (dd, J = 13.3, 8.5 Hz, 2H), 2.00 (d, J = 10.2 Hz, 2H), 1.72 - 1.61 (m, 1H), 1.40 (t, J = 7.0 Hz, 3H), 1.10 - 0.93 (m, 5H).

### Example 30

### (1R,5S)-6-(6-(cyclopropanesulfonamido)pyrimidin-4-yl)-N-(4-(6-ethoxypyrazin-2-yl)phenyl)-N-methylbicyclo[3.1.0]hexane-6-carboxamide

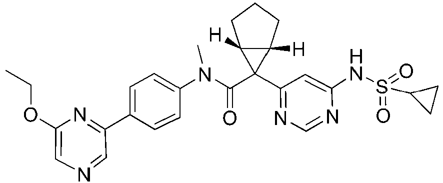

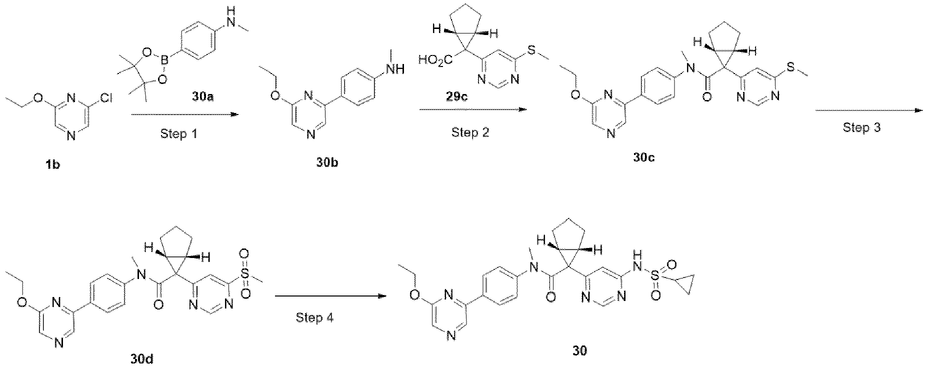

### Step 1

### 4-(6-ethoxypyrazin-2-yl)-N-methylaniline

2-Chloro-6-ethoxypyrazine **1b** (340.15 mg, 2.14 mmol), N-methyl-4-(4,4,5,5-tetramethyl-1,3,2-dioxaborolan-2-yl)aniline **30a** (500 mg, 2.14 mmol, commercially available), 1,1-bis(diphenylphosphino)ferrocene palladium(II) dichloride (156.94 mg, 214.49 µmol), and potassium carbonate (889.33 mg, 6.43 mmol) were sequentially added to a mixed solvent of 1,4-dioxane (6 mL) and water (3 mL). The mixture was subjected to argon exchange three times and heated to 80°C with stirring for 2 hours. The reaction mixture was concentrated under reduced pressure, and the residue was purified by silica gel column chromatography (eluent: System B) to obtain 4-(6-ethoxypyrazin-2-yl)-N-methylaniline **30b** (450 mg) with a yield of 91.51%.

MS m/z (ESI): 230.0 [M+1]

### Step 2

### (1R,5S)-N-(4-(6-ethoxypyrazin-2-yl)phenyl)-N-methyl-6-(6-(methylthio)pyrimidin-4-yl)bicyclo[ 3.1.0]hexane-6-carboxamide

Under ice-water bath, (1R,5S)-6-(6-(methylthio)pyrimidin-4-yl)bicyclo[3.1.0]hexane-6-carboxylic acid **29c** (80 mg, 319.60 µmol), N-methylpyrrolidone (1 mL), N,N,N',N'-tetramethylchloroformamidinium hexafluorophosphate (178.97 mg, 639.19 µmol), and N-methylimidazole (104.96 mg, 1.28 mmol) were added to a 15 mL single-neck flask. The reaction was performed at room temperature for 0.5 hours. 4-(6-Ethoxypyrazin-2-yl)-N-methylaniline **30b** (76.94 mg, 335.58 µmol) was added, and the mixture was heated to 100°C and stirred for 18 hours. After the reaction was completed, water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain (1R,5S)-N-(4-(6-ethoxypyrazin-2-yl)phenyl)-N-methyl-6-(6-(methylthio)pyrimidin-4-yl)bicyclo[ 3.1.0]hexane-6-carboxamide **30c** (80 mg) with a yield of 54.23%.

MS m/z (ESI): 462.1 [M+1]

### Step 3

### (1R,5S)-N-(4-(6-ethoxypyrazin-2-yl)phenyl)-N-methyl-6-(6-(methylsulfonyl)pyrimidin-4-yl)bicy clo[3.1.0]hexane-6-carboxamide

(1R,5S)-N-(4-(6-ethoxypyrazin-2-yl)phenyl)-N-methyl-6-(6-(methylthio)pyrimidin-4-yl )bicyclo[3.1.0]hexane-6-carboxamide **30c** (60 mg, 129.99 µmol) and meta-chloroperoxybenzoic acid (42.23 mg, 207.98 µmol, 85% purity) were added to dichloromethane (2 mL). The mixture was stirred at room temperature overnight. Saturated sodium bicarbonate solution was added to neutralize the mixture, and the mixture was extracted with dichloromethane (10 mL × 3). The combined organic phase was dried over anhydrous sodium sulfate, filtered, and concentrated under reduced pressure to obtain (1R,5S)-N-(4-(6-ethoxypyrazin-2-yl)phenyl)-N-methyl-6-(6-(methylsulfonyl)pyrimidin-4-yl)bicy clo[3.1.0]hexane-6-carboxamide **30d** (40 mg) with a yield of 64.43%.

MS m/z (ESI): 478.1 [M+1]

### Step 4

### (1R,5S)-6-(6-(cyclopropanesulfonamido)pyrimidin-4-yl)-N-(4-(6-ethoxypyrazin-2-yl)phenyl)-N-methylbicyclo[3.1.0]hexane-6-carboxamide

(1R,5S)-N-(4-(6-ethoxypyrazin-2-yl)phenyl)-N-methyl-6-(6-(methylsulfonyl)pyrimidin-4-yl)bicyclo[3.1.0]hexane-6-carboxamide **30d** (60 mg, 125.63 mmol), cyclopropanesulfonamide (18.27 g, 150.76 mmol), and cesium carbonate (81.87 g, 251.27 mmol) were added to N-methylpyrrolidone (1 mL). The mixture was heated to 90°C and the reaction was performed for 1 hour. The reaction mixture was cooled to room temperature and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(cyclopropanesulfonamido)pyrimidin-4-yl)-N-(4-(6-ethoxypyrazin-2-yl)phenyl)-N-methylbicyclo[3.1.0]hexane-6-carboxamide 30 (6 mg) with a yield of 7.36%.

MS m/z (ESI): 535.1 [M+1]

¹H NMR (400 MHz, DMSO-d₆) δ 8.84 (s, 1H), 8.69 (d, J = 10.8 Hz, 1H), 8.26 (s, 1H), 8.19 (d, J = 8.2 Hz, 2H), 7.54 (d, J = 8.2 Hz, 2H), 6.97 (d, J = 14.3 Hz, 1H), 4.49 (q, J = 7.4 Hz, 2H), 3.32 (s, 3H), 3.05 (s, 1H), 2.55 (t, J = 5.5 Hz, 2H), 2.12 (t, J = 13.0 Hz, 4H), 1.40 (q, J = 6.4, 5.8 Hz, 4H), 1.14 - 1.00 (m, 5H).

### Example 31

### 3-(6-(cyclopropanesulfonamido)pyridazin-4-yl)-N-(4-(6-ethoxypyrazin-2-yl)phenyl)bicyclo[3.1.0 ]hexane-3-carboxamide

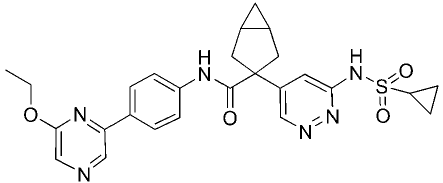

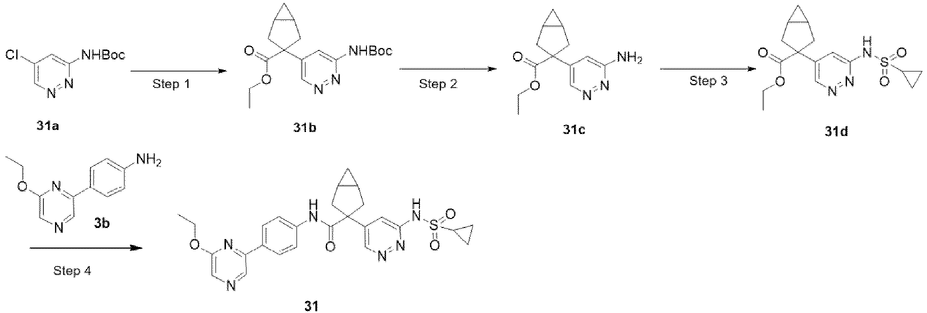

### Step 1

### ethyl 3-(6-((tert-butoxycarbonyl)amino)pyridazin-4-yl)bicyclo[3.1.0]hexane-3-carboxylate

Tert-butyl (5-chloropyridazin-3-yl)carbamate **31a** (700 mg, 3.05 mmol, prepared according to published patent "WO2022128584 A1") and ethyl bicyclo[3.1.0]hexane-3-carboxylate (564.01 mg, 3.66 mmol, commercially available) were added to tetrahydrofuran (4.69 mL). The mixture was cooled to -78°C, and lithium bis(trimethylsilyl)amide (1 M, 9.14 mL) was added. The mixture was heated to room temperature and stirred for 1 hour. Saturated ammonium chloride solution was added to the reaction mixture, and the organic phase was separated, dried over anhydrous sodium sulfate, and concentrated under reduced pressure. The residue was purified by silica gel column chromatography (eluent: System B) to obtain ethyl 3-(6-((tert-butoxycarbonyl)amino)pyridazin-4-yl)bicyclo[3.1.0]hexane-3-carboxylate **31b** (900 mg) with a yield of 85.00%.

MS m/z (ESI): 348.1[M+1]

### Step 2

### ethyl 3-(6-aminopyridazin-4-yl)bicyclo[3.1.0]hexane-3-carboxylate

Ethyl 3-(6-((tert-butoxycarbonyl)amino)pyridazin-4-yl)bicyclo[3.1.0]hexane-3-carboxylate **31b** (550 mg, 1.58 mmol) was added to a 4 M solution of hydrochloric acid in dioxane (5 mL) and stirred at room temperature overnight. The reaction mixture was concentrated under reduced pressure to obtain ethyl 3-(6-aminopyridazin-4-yl)bicyclo[3.1.0]hexane-3-carboxylate **31c** (350 mg) with a yield of 89.97%.

MS m/z (ESI): 248.0 [M+1]

### Step 3

### ethyl 3-(6-(cyclopropanesulfonamido)pyridazin-4-yl)bicyclo[3.1.0]hexane-3-carboxylate

Ethyl 3-(6-aminopyridazin-4-yl)bicyclo[3.1.0]hexane-3-carboxylate **31c** (350 mg, 1.42 mmol), 4-dimethylaminopyridine (345.82 mg, 2.83 mmol), cyclopropanesulfonyl chloride (795.92 mg, 5.66 mmol), and pyridine (419.95 µL) were mixed. The mixture was heated to 70°C and stirred for 2 hours, then heated to 100°C and stirred for 18 hours. The mixture was concentrated under reduced pressure and the solvent was evaporated to dryness by rotation. Water (10 mL) and saturated citric acid solution (10 mL) were added, and the mixture was extracted with dichloromethane (20 mL × 3). The combined organic phase was dried over anhydrous sodium sulfate, filtered, and concentrated under reduced pressure. The residue was purified by silica gel column chromatography (eluent: System B) to obtain ethyl 3-(6-(cyclopropanesulfonamido)pyridazin-4-yl)bicyclo[3.1.0]hexane-3-carboxylate **31d** (150 mg) with a yield of 30.16%.

MS m/z (ESI): 352.1 [M+1]

### Step 4

### 3-(6-(cyclopropanesulfonamido)pyridazin-4-yl)-N-(4-(6-ethoxypyrazin-2-yl)phenyl)bicyclo[3.1.0 ]hexane-3-carboxamide

Ethyl 3-(6-(cyclopropanesulfonamido)pyridazin-4-yl)bicyclo[3.1.0]hexane-3-carboxylate **31d** (66.15 mg, 307.32 µmol), toluene (1.81 mL), and a solution of trimethylaluminum in toluene (2 M, 460.99 µL) were mixed and stirred at room temperature for 1 hour. 4-(6-Ethoxypyrazin-2-yl)aniline **3b** (90 mg, 256.10 µmol) was added, and the mixture was heated to 90°C and stirred for 5 hours. Methanol (1 mL) was added to quench the reaction, and the mixture was directly concentrated under reduced pressure. Dilute hydrochloric acid (1 M, 2 mL) and dichloromethane (5 mL) were added to the residue. Insoluble solid was removed by filtration, and the filtrate was separated. The dichloromethane phase was collected, dried over anhydrous sodium sulfate, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyridazin-4-yl)-N-(4-(6-ethoxypyrazin-2-yl)phenyl)bicyclo[3.1.0 ]hexane-3-carboxamide **31** (25 mg) with a yield of 14.92%.

MS m/z (ESI): 521.8 [M+1]
¹H NMR (400 MHz, DMSO-d₆) δ 9.71 (s, 1H), 8.63 (s, 1H), 8.06 (s, 1H), 7.95 (d, J = 8.4 Hz, 2H), 7.57 (d, J = 8.4 Hz, 3H), 4.34 (q, J = 7.1 Hz, 2H), 2.87 (d, J = 13.5 Hz, 2H), 2.08 (d, J = 13.4 Hz, 2H), 1.36 - 1.20 (m, 6H), 0.78 (d, J = 11.6 Hz, 4H), 0.34 (q, J = 7.3 Hz, 1H), -0.00 (q, J = 4.4 Hz, 1H).

### Example 32

### 2-(4-(cyclopropanesulfonamido)pyridin-2-yl)-N-(4-(6-ethoxypyrazin-2-yl)phenyl)-2,3-dihydro-1 H-indene-2-carboxamide

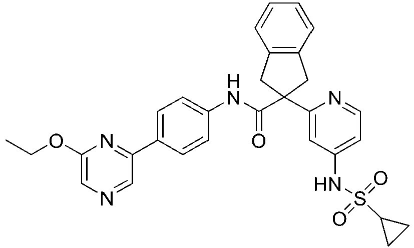

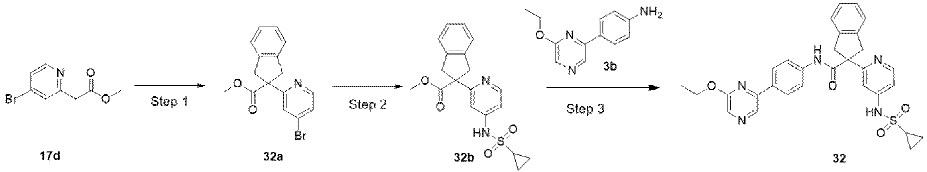

### Step 1

### methyl 2-(4-bromopyridin-2-yl)-2,3-dihydro-1H-indene-2-carboxylate

Methyl 2-(4-bromopyridin-2-yl)acetate **17d** (500 mg, 2.17 mmol) was added to N,N-dimethylformamide (1 mL). The mixture was subjected to argon exchange three times. Under cooling with ice-water bath, sodium hydroxide (199.95 mg, 5.00 mmol) was slowly added, and the mixture was stirred for 0.5 hours. 1,2-Bis(chloromethyl)benzene (494.59 mg, 2.83 mmol) was added, and the mixture was stirred at room temperature for 23 hours. After the reaction was completed, ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated. The residue was purified by silica gel column chromatography (eluent: System B) to obtain methyl 2-(4-bromopyridin-2-yl)-2,3-dihydro-1H-indene-2-carboxylate **32a** (280 mg) with a yield of 38.78%.

MS m/z (ESI): 333.0 [M+1]

### Step 2

### methyl 2-(4-(cyclopropanesulfonamido)pyridin-2-yl)-2,3-dihydro-1H-indene-2-carboxylate

Methyl 2-(4-bromopyridin-2-yl)-2,3-dihydro-1H-indene-2-carboxylate **32a** (230 mg, 692.37 µmol), cyclopropanesulfonamide (125.83 mg, 1.04 mmol), cesium carbonate (451.18 mg, 1.38 mmol), 2-di-tert-butylphosphino-2',4',6'-triisopropylbiphenyl (58.80 mg, 138.47 µmol), and allylpalladium(II) chloride dimer (25.33 mg, 69.24 µmol) were sequentially added to 1,4-dioxane (2 mL). The mixture was subjected to argon exchange 3 times and heated to 90°C with stirring for 2 hours. The mixture was concentrated under reduced pressure. The residue was purified by silica gel column chromatography (eluent: System B) to obtain methyl 2-(4-(cyclopropanesulfonamido)pyridin-2-yl)-2,3-dihydro-1H-indene-2-carboxylate **32b** (100 mg) with a yield of 38.78%.

MS m/z (ESI): 373.1 [M+1]

### Step 3

### 2-(4-(cyclopropanesulfonamido)pyridin-2-yl)-N-(4-(6-ethoxypyrazin-2-yl)phenyl)-2,3-dihydro-1 H-indene-2-carboxamide

4-(6-Ethoxypyrazin-2-yl)aniline **3b** (57.80 mg, 268.50 µmol) was added to toluene (1 mL). The mixture was subjected to argon exchange three times. A toluene solution of trimethylaluminum (2 M, 469.88 µL) was slowly added dropwise, and the mixture was stirred for 1 hour. Methyl 2-(4-(cyclopropanesulfonamido)pyridin-2-yl)-2,3-dihydro-1H-indene-2-carboxylate **32b** (100 mg, 268.50 µmol) was added, and the mixture was heated to 90°C with stirring for 5 hours. After the reaction was completed, the mixture was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(4-(cyclopropanesulfonamido)pyridin-2-yl)-N-(4-(6-ethoxypyrazin-2-yl)phenyl)-2,3-dihydro-1 H-indene-2-carboxamide 32 (64.54 mg) with a yield of 33.74%.

MS m/z (ESI): 556.2 [M+1]
¹H NMR (400 MHz, DMSO-d₆) δ 10.13 (s, 1H), 8.77 (s, 1H), 8.51 (d, J = 6.2 Hz, 1H), 8.19 (s, 1H), 8.09 (d, J = 8.4 Hz, 2H), 7.80 (d, J = 8.4 Hz, 2H), 7.40 (s, 1H), 7.35 (d, J = 6.2 Hz, 1H), 7.29 (d, J = 4.2 Hz, 2H), 7.20 (d, J = 4.3 Hz, 2H), 4.45 (t, J = 7.1 Hz, 2H), 3.98 (d, J = 16.2 Hz, 2H), 3.58 (s, 2H), 2.88 (s, 1H), 1.39 (t, J = 7.0 Hz, 3H), 0.99 (d, J = 9.0 Hz, 4H).

### Example 33

### (1R,5S)-6-(4-(cyclopropanesulfonamido)pyridin-2-yl)-N-(4-(6-ethoxypyrazin-2-yl)phenyl)-3-oxa bicyclo[3.1.0]hexane-6-carboxamide

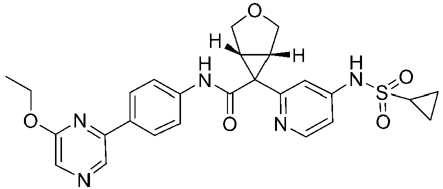

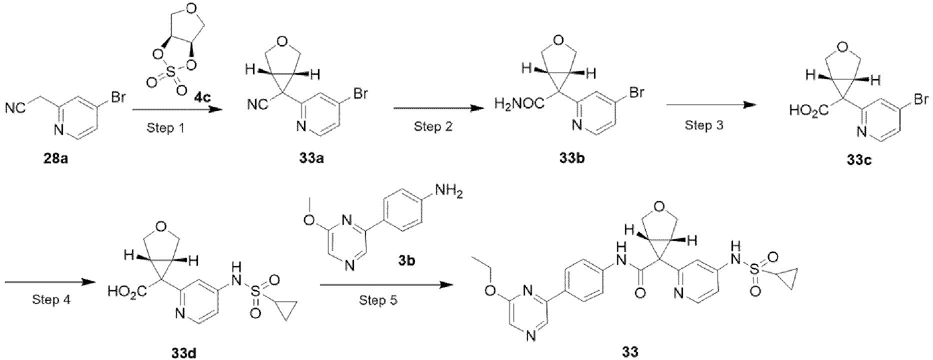

### Step 1

### (1R,5S)-6-(4-bromopyridin-2-yl)-3-oxabicyclo[3.1.0]hexane-6-carbonitrile

Under ice-water bath, 2-(4-bromopyridin-2-yl)acetonitrile **28a** (1 g, 5.08 mmol) was added to anhydrous dimethoxyethane (40 mL). Sodium hydride (446.63 mg, 11.17 mmol, 60% purity) was added, and the mixture was stirred at room temperature for 0.5 hours. (3aR,6aS)-Tetrahydrofuro[3,4-d][1,3,2]dioxathiole 2,2-dioxide **4c** (1.26 g, 7.61 mmol) was added, and the mixture was heated to 60°C and stirred for 18 hours. Water (50 mL) and ethyl acetate (50 mL) were added, and the mixture was partitioned. The organic phase was dried over anhydrous sodium sulfate, filtered, and evaporated to dryness by rotation. The residue was purified by silica gel column chromatography (eluent: System B) to obtain (1R,5S)-6-(4-bromopyridin-2-yl)-3-oxabicyclo[3.1.0]hexane-6-carbonitrile **33a** (800 mg) with a yield of 59.46%.

MS m/z (ESI): 265.1 [M+1]

### Step 2

### (1R,5S)-6-(4-bromopyridin-2-yl)-3-oxabicyclo[3.1.0]hexane-6-carboxamide

(1R,5S)-6-(4-bromopyridin-2-yl)-3-oxabicyclo[3.1.0]hexane-6-carbonitrile **33a** (1.6 g, 6.04 mmol) was added to dimethyl sulfoxide (3 mL). Under ice-water bath, sodium hydroxide (482.83 mg, 12.07 mmol) was added. After stirring for 10 minutes, hydrogen peroxide (1.5 mL) was added, and the mixture was stirred at room temperature for 3 hours. Water (10 mL) was added to quench the reaction, and the mixture was extracted with ethyl acetate (10 mL × 3). The organic phase was dried over anhydrous sodium sulfate, filtered, and concentrated under reduced pressure to obtain (1R,5S)-6-(4-bromopyridin-2-yl)-3-oxabicyclo[3.1.0]hexane-6-carboxamide 33b (1.2 g) with a yield of .23%.

MS m/z (ESI): 283.0 [M+1]

### Step 3

### (1R,5S)-6-(4-bromopyridin-2-yl)-3-oxabicyclo[3.1.0]hexane-6-carboxylic acid

(1R,5S)-6-(4-bromopyridin-2-yl)-3-oxabicyclo[3.1.0]hexane-6-carboxamide **33b** (1.2 g, 4.24 mmol), acetic acid (1.50 mL), and acetic anhydride (3 mL) were added to dimethyl sulfoxide (3 mL). Under ice-water bath, sodium nitrite (2.92 g, 42.38 mmol) was added, and the mixture was stirred at room temperature overnight. The reaction mixture was poured into water (10 mL) and neutralized with saturated sodium bicarbonate solution. Saturated citric acid solution was added to adjust the pH to acidic, and the mixture was extracted with ethyl acetate (20 mL × 3). The aqueous phase was collected and purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-bromopyridin-2-yl)-3-oxabicyclo[3.1.0]hexane-6-carboxylic acid **33c** (200 mg) with a yield of 16.61%.

MS m/z (ESI): 284.0 [M+1]

### Step 4

### (1R,5S)-6-(4-(cyclopropanesulfonamido)pyridin-2-yl)-3-oxabicyclo[3.1.0]hexane-6-carboxylic acid

(1R,5S)-6-(4-bromopyridin-2-yl)-3-oxabicyclo[3.1.0]hexane-6-carboxylic acid 33c (750 mg, 2.64 mmol), cyclopropanesulfonamide (383.81 mg, 3.17 mmol), cesium carbonate (1.72 g, 5.28 mmol), 2-diter-butylphosphino-2',4',6'-triisopropylbiphenyl (112 mg, 263 µmol), and allylpalladium(II) chloride dimer (48.29 mg, 131.99 µmol) were sequentially added to 1,4-dioxane (10 mL). The mixture was subjected to argon exchange 3 times and heated to 90°C with stirring for 2 hours. The reaction mixture was cooled to room temperature, and insoluble material was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-(cyclopropanesulfonamido)pyridin-2-yl)-3-oxabicyclo[3.1.0]hexane-6-carboxylic acid 33d (110 mg) with a yield of 12.85%.

MS m/z (ESI): 325.0 [M+1]

### Step 5

### (1R,5S)-6-(4-(cyclopropanesulfonamido)pyridin-2-yl)-N-(4-(6-ethoxypyrazin-2-yl)phenyl)-3-oxa bicyclo[3.1.0]hexane-6-carboxamide

Under ice-water bath, (1R,5S)-6-(4-(cyclopropanesulfonamido)pyridin-2-yl)-3-oxabicyclo[3.1.0]hexane-6-carboxylic acid **33d** (110 mg, 339.14 µmol), N-methylpyrrolidone (1 mL), N,N,N',N'-tetramethylchloroformamidinium hexafluorophosphate (189.92 mg, 678.28 µmol), and N-methylimidazole (111.37 mg, 1.36 mmol) were added to a 15 mL single-neck flask. The reaction was performed at room temperature for 0.5 hours. 4-(6-Ethoxypyrazin-2-yl)aniline **3b** (87.60 mg, 406.97 µmol) was added, and the mixture was heated to 90°C and the reaction was performed for 2 hours. The reaction mixture was cooled to room temperature. Water (10 mL) and ethyl acetate (10 mL) were added, and the mixture was extracted and partitioned. The organic phase was dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-(cyclopropanesulfonamido)pyridin-2-yl)-N-(4-(6-ethoxypyrazin-2-yl)phenyl)-3-oxa bicyclo[3.1.0]hexane-6-carboxamide 33 (25 mg) with a yield of 11.50%.

MS m/z (ESI): 522.2 [M+1]
¹H NMR (400 MHz, DMSO-d₆) δ 10.39 (s, 1H), 8.77 (s, 1H), 8.39 (d, J = 6.1 Hz, 1H), 8.23 - 8.02 (m, 3H), 7.82 (d, J = 8.3 Hz, 2H), 7.34 (s, 1H), 7.17 (d, J = 5.9 Hz, 1H), 4.48 (q, J = 7.5 Hz, 2H), 4.13 (d, J = 8.9 Hz, 2H), 3.82 (d, J = 8.8 Hz, 2H), 2.82 (s, 1H), 2.48 (s, 2H), 1.40 (t, J = 7.1 Hz, 3H), 1.24 (s, 1H).

### Example 34

### 2-(4-(cyclopropanesulfonamido)pyrimidin-2-yl)-N-(4-(6-ethoxypyrazin-2-yl)phenyl)-2,3-dihydro -1H-indene-2-carboxamide

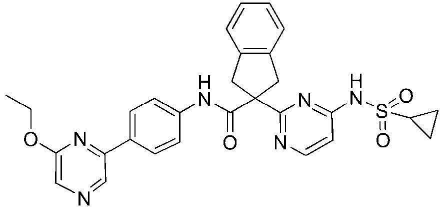

According to the synthesis method of Example 15 of the present disclosure, ethyl bicyclo[3.1.0]hexane-3-carboxylate 2a as the starting material in Example 15 was replaced with methyl 2,3-dihydro-1H-indene-2-carboxylate to obtain 2-(4-(cyclopropanesulfonamido)pyrimidin-2-yl)-N-(4-(6-ethoxypyrazin-2-yl)phenyl)-2,3-dihydro -1H-indene-2-carboxamide 34.

MS m/z (ESI): 557.1 [M+1]
1H NMR (400 MHz, DMSO-d6) δ 9.79 (s, 1H), 8.75 (s, 1H), 8.51 (s, 1H), 8.16 (s, 1H), 8.06 (d, J = 8.8 Hz, 2H), 7.78 (d, J = 8.9 Hz, 2H), 7.23 (dd, J = 5.4, 3.3 Hz, 2H), 7.13 (dd, J = 5.5, 3.2 Hz, 2H), 6.81 (d, J = 5.7 Hz, 1H), 4.47 (q, J = 7.0 Hz, 2H), 3.84 (d, J = 16.3 Hz, 2H), 3.74 (d, J = 16.3 Hz, 2H), 2.83 (td, J = 8.1, 4.8 Hz, 1H), 1.39 (t, J = 7.0 Hz, 3H), 1.05 - 0.96 (m, 2H), 0.81 - 0.69 (m, 2H).

### Examples 35 and 36

### (1R,5S)-N-(4-(6-ethoxypyrazin-2-yl)phenyl)-3-methyl-6-(4-(N-methylcyclopropanesulfonamido) pyridin-2-yl)-3-azabicyclo[3.1.0]hexane-6-carboxamide 35

### (1R,5S)-6-(4-(cyclopropanesulfonamido)pyridin-2-yl)-N-(4-(6-ethoxypyrazin-2-yl)phenyl)-3-met hyl-3-azabicyclo[3.1.0]hexane-6-carboxamide 36

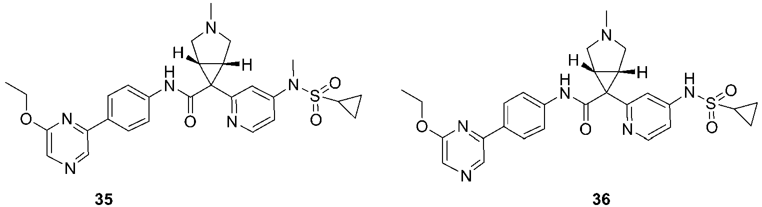

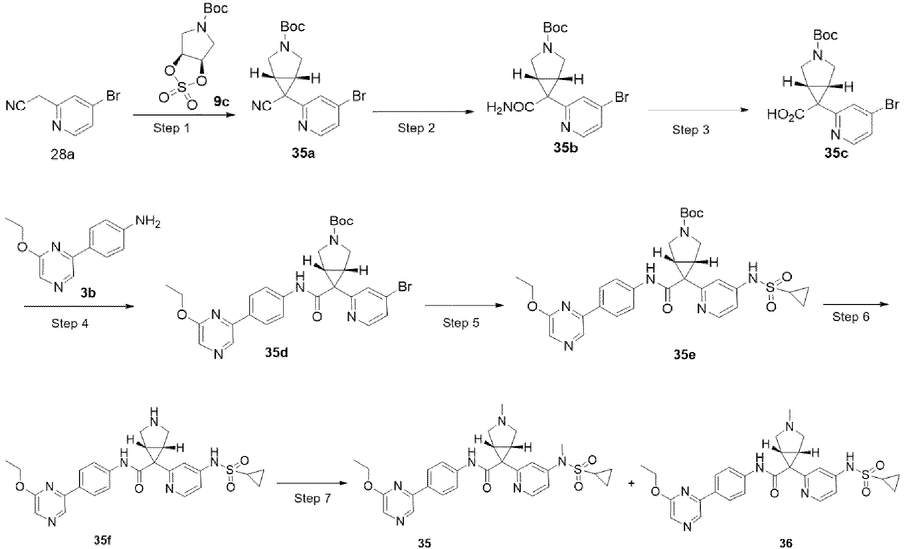

### Step 1

### tert-butyl (1R,5S)-6-(4-bromopyridin-2-yl)-6-cyano-3-azabicyclo[3.1.0]hexane-3-carboxylate

Under ice-water bath, 2-(4-bromopyridin-2-yl)acetonitrile **28a** (1 g, 5.08 mmol) was added to anhydrous dimethoxyethane (40 mL). Sodium cyanide (446.63 mg, 11.17 mmol, 60% purity) was added, and the mixture was stirred at room temperature for 0.5 hours. (3aR,6aS)-tert-butyl tetrahydro-5H-[1,3,2]dithiolo[4,5-c]pyrrole-5-carboxylate 2,2-dioxide **9c** (1.88 g, 7.11 mmol) was added, and the mixture was heated to 70°C and stirred for 6 hours. Water (50 mL) and ethyl acetate (50 mL) were added, and the mixture was partitioned. The organic phase was dried over anhydrous sodium sulfate, filtered, and evaporated to dryness by rotation. The residue was purified by silica gel column chromatography (eluent: System B) to obtain tert-butyl (1R,5S)-6-(4-bromopyridin-2-yl)-6-cyano-3-azabicyclo[3.1.0]hexane-3-carboxylate **35a** (1.1 g) with a yield of 59.50%.

### Step 2

### tert-butyl (1R,5S)-6-(4-bromopyridin-2-yl)-6-carbamoyl-3-azabicyclo[3.1.0]hexane-3-carboxylate

Tert-butyl (1R,5S)-6-(4-bromopyridin-2-yl)-6-cyano-3-azabicyclo[3.1.0]hexane-3-carboxylate **35a** (1.1 g, 3.02 mmol) and sodium hydroxide (241.60 mg, 6.04 mmol) were added to dimethyl sulfoxide (3 mL). Hydrogen peroxide (1.5 mL) was added dropwise at room temperature, and the mixture was stirred at room temperature for 2 hours. Water (50 mL) and ethyl acetate (50 mL) were added, and the mixture was partitioned. The organic phase was dried over anhydrous sodium sulfate, filtered, and concentrated under reduced pressure to obtain tert-butyl (1R,5S)-6-(4-bromopyridin-2-yl)-6-carbamoyl-3-azabicyclo[3.1.0]hexane-3-carboxylate **35b** (1 g) with a yield of 86.62%.

MS m/z (ESI): 383.0 [M+1]

### Step 3

### (1R,5S)-6-(4-bromopyridin-2-yl)-3-(tert-butoxycarbonyl)-3-azabicyclo[3.1.0]hexane-6-carboxyli c acid

Tert-butyl (1R,5S)-6-(4-bromopyridin-2-yl)-6-carbamoyl-3-azabicyclo[3.1.0]hexane-3-carboxylate **35b** (1.1 g, 2.88 mmol), acetic acid (3 mL), and acetic anhydride (3 mL) were mixed. Sodium nitrite (1.99 g, 28.78 mmol) was added, and the mixture was heated to 45°C and stirred for 5 hours. Water (3 mL) was added to dissolve the mixture, and the mixture was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-bromopyridin-2-yl)-3-(tert-butoxycarbonyl)-3-azabicyclo[3.1.0]hexane-6-carboxyli c acid **35c** (700 mg) with a yield of 63.47%.

MS m/z (ESI): 383.0 [M+1]

### Step 4

### tert-butyl (1R,5S)-6-(4-bromopyridin-2-yl)-6-((4-(6-ethoxypyrazin-2-yl)phenyl)carbamoyl)-3-azabicyclo[3 .1.0]hexane-3-carboxylate

Under ice-water bath, (1R,5S)-6-(4-bromopyridin-2-yl)-3-(tert-butoxycarbonyl)-3-azabicyclo[3.1.0]hexane-6-carboxyli c acid 35c (340 mg, 887.18 µmol), N-methylpyrrolidone (3 mL), N,N,N',N'-tetramethylchloroformamidinium hexafluorophosphate (496.82 mg, 1.77 mmol), and N-methylimidazole (291.35 mg, 3.55 mmol) were added to a 15 mL single-neck flask. The reaction was performed at room temperature for 0.5 hours. 4-(6-Ethoxypyrazin-2-yl)aniline **3b** (210.06 mg, 975.90 µmol) was added, and the mixture was heated to 50°C and stirred for 18 hours. Water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain tert-butyl (1R,5S)-6-(4-bromopyridin-2-yl)-6-((4-(6-ethoxypyrazin-2-yl)phenyl)carbamoyl)-3-azabicyclo[3 .1.0]hexane-3-carboxylate **35d** (400 mg) with a yield of 77.67%.

MS m/z (ESI): 580.1 [M+1]

### Step 5

### tert-butyl (1R,5S)-6-(4-(cyclopropanesulfonamido)pyridin-2-yl)-6-((4-(6-ethoxypyrazin-2-yl)phenyl)carba moyl)-3-azabicyclo[3.1.0]hexane-3-carboxylate

Tert-butyl (1R,5S)-6-(4-bromopyridin-2-yl)-6-((4-(6-ethoxypyrazin-2-yl)phenyl)carbamoyl)-3-azabicyclo[3 .1.0]hexane-3-carboxylate **35d** (150 mg, 258.41 µmol), cyclopropanesulfonamide (37.57 mg, 310.09 µmol), cesium carbonate (168.39 mg, 516.82 µmol), 2-di-tert-butylphosphino-2',4',6'-triisopropylbiphenyl (10.97 mg, 25.84 µmol), and allylpalladium(II) chloride dimer (4.73 mg, 12.92 µmol) were sequentially added to 1,4-dioxane (4 mL). The mixture was subjected to argon exchange 3 times, heated to 90°C, and stirred for 2 hours. The mixture was concentrated under reduced pressure, and the residue was filtered to remove insoluble solid. The residue was purified by silica gel column chromatography (eluent: System B) to obtain tert-butyl (1R,5S)-6-(4-(cyclopropanesulfonamido)pyridin-2-yl)-6-((4-(6-ethoxypyrazin-2-yl)phenyl)carba moyl)-3-azabicyclo[3.1.0]hexane-3-carboxylate **35e** (100 mg) with a yield of 62.34%.

MS m/z (ESI): 621.0 [M+1]

### Step 6

### (1R,5S)-6-(4-(cyclopropanesulfonamido)pyridin-2-yl)-N-(4-(6-ethoxypyrazin-2-yl)phenyl)-3-aza bicyclo[3.1.0]hexane-6-carboxamide

Tert-butyl (1R,5S)-6-(4-(cyclopropanesulfonamido)pyridin-2-yl)-6-((4-(6-ethoxypyrazin-2-yl)phenyl)carba moyl)-3-azabicyclo[3.1.0]hexane-3-carboxylate **35e** (100 mg, 161.10 µmol) was added to a solution of hydrogen chloride in dioxane (4 M, 5 mL). The mixture was stirred at room temperature overnight. The reaction mixture was concentrated under reduced pressure, and the residue was directly used in the next step.

MS m/z (ESI): 521.1 [M+1]

### Step 7

### (1R,5S)-N-(4-(6-ethoxypyrazin-2-yl)phenyl)-3-methyl-6-(4-(N-methylcyclopropanesulfonamido) pyridin-2-yl)-3-azabicyclo[3.1.0]hexane-6-carboxamide 35

### (1R,5S)-6-(4-(cyclopropanesulfonamido)pyridin-2-yl)-N-(4-(6-ethoxypyrazin-2-yl)phenyl)-3-met hyl-3-azabicyclo[3.1.0]hexane-6-carboxamide 36

(1R,5S)-6-(4-(cyclopropanesulfonamido)pyridin-2-yl)-N-(4-(6-ethoxypyrazin-2-yl)phen yl)-3-azabicyclo[3.1.0]hexane-6-carboxamide **35f** (60 mg, 115.25 µmol), sodium hydroxide (4.61 mg, 115.25 µmol), paraformaldehyde (3.46 mg, 115.25 µmol), and formic acid (13.25 mg, 288.13 µmol) were added to tetrahydrofuran (5 mL). The mixture was heated to 80°C and stirred for 3 hours. The reaction mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-N-(4-(6-ethoxypyrazin-2-yl)phenyl)-3-methyl-6-(4-(N-methylcyclopropanesulfonamido) pyridin-2-yl)-3-azabicyclo[3.1.0]hexane-6-carboxamide 35 and (1R,5S)-6-(4-(cyclopropanesulfonamido)pyridin-2-yl)-N-(4-(6-ethoxypyrazin-2-yl)phenyl)-3-met hyl-3-azabicyclo[3.1.0]hexane-6-carboxamide 36.

**Example 35:** 5 mg, with a yield of 6.28%.

MS m/z (ESI): 549.2 [M+1]
¹H NMR (400 MHz, DMSO-*d*₆) δ 8.77 (s, 1H), 8.41 (d, J = 6.0 Hz, 1H), 8.19 (s, 1H), 8.07 (d, J = 8.3 Hz, 2H), 7.88 (s, 1H), 7.72 (d, J = 8.3 Hz, 2H), 7.32 (s, 1H), 7.21 (d, J = 5.8 Hz, 1H), 4.48 (q, J = 7.0 Hz, 2H), 4.15 (d, J = 12.2 Hz, 1H), 4.05 (d, J = 11.3 Hz, 1H), 3.77 (dd, J = 11.4, 3.9 Hz, 1H), 3.39 - 3.31 (m, 1H), 2.85 (s, 1H), 2.50 (s, 6H), 2.48 - 2.35 (m, 2H), 1.40 (t, J = 7.0 Hz, 3H), 1.00 (dd, J = 12.8, 4.9 Hz, 4H).

**Example 36:** 20 mg, with a yield of 26.22%.

MS m/z (ESI): 535.2 [M+1]
¹H NMR (400 MHz, DMSO-*d*₆) δ 10.96 (s, 1H), 10.81 (s, 1H), 8.80 (s, 1H), 8.38 (d, J = 5.6 Hz, 1H), 8.21 (s, 1H), 8.15 (d, J = 8.4 Hz, 2H), 7.87 (d, J = 8.4 Hz, 2H), 7.26 (s, 1H), 7.17 - 7.09 (m, 1H), 4.48 (q, J = 7.0 Hz, 2H), 4.20 (s, 1H), 3.92 - 3.80 (m, 1H), 3.67 (d, J = 10.7 Hz, 1H), 3.16 (d, J = 11.1 Hz, 1H), 2.89 (s, 1H), 2.67 - 2.60 (m, 1H), 2.51 - 2.48 (m, 3H), 1.41 (t, J = 7.0 Hz, 3H), 1.03 - 0.85 (m, 4H).

### Example 37

### (1R,5S)-6-(6-(cyclopropanesulfonamido)pyrimidin-4-yl)-N-(4-(6-ethoxypyrazin-2-yl)phenyl)-3-methyl-3-azabicyclo[3.1.0]hexane-6-carboxamide

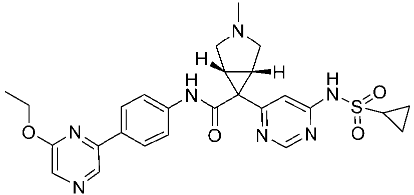

According to the synthesis methods of Examples 9 and 10 of the present disclosure,the material 2-(2-(methylthio)pyrimidin-4-yl)acetonitrile **4b** in Example 9 was replaced with 2-(6-(methylthio)pyrimidin-4-yl)acetonitrile **27a** to obtain (1R,5S)-6-(6-(cyclopropanesulfonamido)pyrimidin-4-yl)-N-(4-(6-ethoxypyrazin-2-yl)phenyl)-3-methyl-3-azabicyclo[3.1.0]hexane-6-carboxamide **37.**

MS m/z (ESI): 536.2 [M+1]
¹H NMR (400 MHz, DMSO-*d*₆) δ 10.81 (s, 1H), 9.50 (s, 1H), 8.21 (s, 1H), 8.15 (d, J = 8.4 Hz, 2H), 7.87 (d, J = 8.4 Hz, 2H), 7.26 (s, 1H), 7.17 - 7.09 (m, 1H), 4.48 (q, J = 7.0 Hz, 2H), 4.20 (s, 1H), 3.92 - 3.80 (m, 1H), 3.67 (d, J = 10.7 Hz, 1H), 3.16 (d, J = 11.1 Hz, 1H), 2.89 (s, 1H), 2.67 - 2.60 (m, 1H), 2.51 - 2.48 (m, 3H), 1.41 (t, J = 7.0 Hz, 3H), 1.03 - 0.85 (m, 4H).

### Example 38

### 3-(4-(cyclopropanesulfonamido)pyrimidin-2-yl)-N-(5-(6-ethoxypyrazin-2-yl)pyridin-2-yl)bicyclo [3.1.0]hexane-3-carboxamide

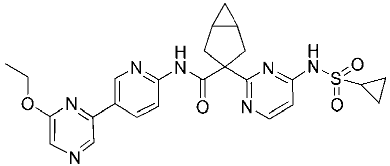

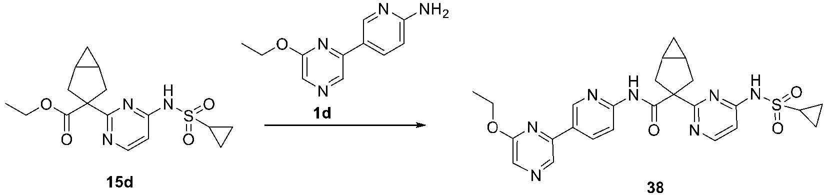

Ethyl 3-(4-(cyclopropanesulfonamido)pyrimidin-2-yl)bicyclo[3.1.0]hexane-3-carboxylate **15d** (54.15 mg, 250.41 µmol) was added to toluene (1.86 mL). A solution of trimethylaluminum in toluene (2 M, 409.77 µL) was added, and the mixture was stirred at room temperature for 1 hour. 5-(6-Ethoxypyrazin-2-yl)pyridin-2-amine **1d** (80 mg, 227.65 µmol) was added, and the mixture was heated to 90°C and stirred for 1.5 hours. The reaction mixture was cooled to room temperature, and methanol (1 mL) was added to quench the reaction. The mixture was concentrated under reduced pressure. Dilute hydrochloric acid (1 M, 2 mL) and dichloromethane (5 mL) were added to the residue. The insoluble solid was removed by filtration, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyrimidin-2-yl)-N-(5-(6-ethoxypyrazin-2-yl)pyridin-2-yl)bicyclo [3.1.0]hexane-3-carboxamide 38 (13 mg) with a yield of 8.86%.

MS m/z (ESI): 522.1 [M+1].

¹H NMR (400 MHz, DMSO-*d*₆) δ 10.03 (s, 1H), 8.92 - 8.81 (m, 1H), 8.69 (s, 1H), 8.35 (d, J = 6.4 Hz, 2H), 8.14 - 7.97 (m, 2H), 6.67 (d, J = 5.7 Hz, 1H), 4.32 (d, J = 7.1 Hz, 2H), 2.97 (dt, J = 8.0, 3.7 Hz, 1H), 2.67 (d, J = 13.7 Hz, 2H), 2.47 (d, J = 13.5 Hz, 2H), 1.26 (t, J = 6.9 Hz, 5H), 0.93 (p, J = 4.9 Hz, 2H), 0.79 - 0.69 (m, 2H), 0.30 - 0.20 (m, 1H), -0.00 (q, J = 4.3 Hz, 1H).

### Example 39

### 3-(4-(cyclopropanesulfonamido)pyrimidin-2-yl)-N-(4-(6-ethoxypyrazin-2-yl)-2-fluorophenyl)bic yclo[3.1.0]hexane-3-carboxamide

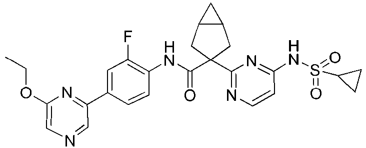

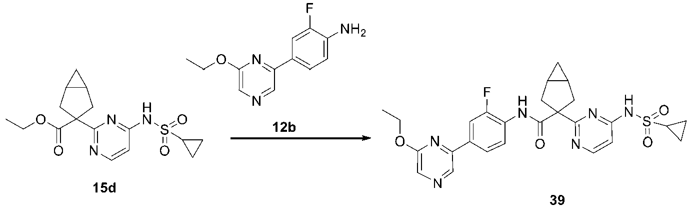

Ethyl 3-(4-(cyclopropanesulfonamido)pyrimidin-2-yl)bicyclo[3.1.0]hexane-3-carboxylate **15d** (58.41 mg, 250.41 µmol) was added to toluene (1.86 mL). A solution of trimethylaluminum in toluene (2 M, 409.77 µL) was added, and the mixture was stirred at room temperature for 1 hour. 4-(6-Ethoxypyrazin-2-yl)-2-fluoroaniline **12b** (80 mg, 227.65 µmol) was added, and the mixture was heated to 90°C and stirred for 1.5 hours. Methanol (1 mL) was added to quench the reaction, and the mixture was concentrated under reduced pressure. Dilute hydrochloric acid (1 M, 2 mL) and dichloromethane (5 mL) were added to the residue. The insoluble solid was removed by filtration, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyrimidin-2-yl)-N-(4-(6-ethoxypyrazin-2-yl)-2-fluorophenyl)bic yclo[3.1.0]hexane-3-carboxamide **39** (13 mg) with a yield of 7.44%.

MS m/z (ESI): 521.8 [M+1].

¹H NMR (400 MHz, DMSO-*d*₆) δ 11.28 (s, 1H), 9.41 (s, 1H), 8.83 (s, 1H), 8.52 (s, 1H), 8.24 (s, 1H), 7.95 (s, 2H), 7.80 (s, 1H), 6.84 (s, 1H), 4.48 (s, 2H), 3.16 (s, 1H), 2.81 (s, 2H), 2.60 (d, J = 13.7 Hz, 2H), 1.40 (s, 5H), 1.13 (s, 2H), 1.01 (s, 2H), 0.40 (s, 1H), 0.26 (s, 1H).

### Example 40

### 2-(3-(cyclopropanesulfonamido)phenyl)-N-(4-(6-ethoxypyrazin-2-yl)phenyl)-2,3-dihydro-1H-ind ene-2-carboxamide

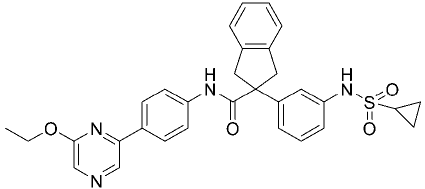

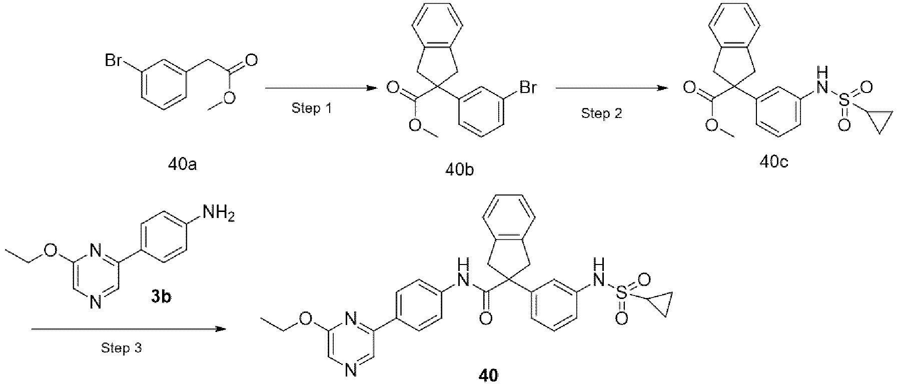

### Step 1

### methyl 2-(3-bromophenyl)-2,3-dihydro-1H-indene-2-carboxylate

Methyl 2-(3-bromophenyl)acetate **40a** (2.5 g, 10.91 mmol), 1,2-bis(chloromethyl)benzene (2.87 g, 16.37 mmol), and sodium hydride (1.09 g, 27.28 mmol, 60% purity) were added to tetrahydrofuran (50 mL). The mixture was heated to 60°C and stirred for 18 hours. The reaction mixture was cooled to room temperature. Ethyl acetate (50 mL) and water (30 mL) were added, and the organic phase was separated. The aqueous phase was extracted with ethyl acetate (50 mL × 2). The combined organic phase was washed with saturated sodium chloride solution (30 mL × 2), dried over anhydrous sodium sulfate, filtered, and concentrated. The residue was purified by silica gel column chromatography (eluent: System A) to obtain methyl 2-(3-bromophenyl)-2,3-dihydro-1H-indene-2-carboxylate **40b** (2.2 g) with a yield of 60.86%.

MS m/z (ESI): 332.1 [M+1].

### Step 2

### methyl 2-(3-(cyclopropanesulfonamido)phenyl)-2,3-dihydro-1H-indene-2-carboxylate

Methyl 2-(3-bromophenyl)-2,3-dihydro-1H-indene-2-carboxylate **40b** (1.1 g, 3.32 mmol), cyclopropanesulfonamide (482.87 mg, 3.99 mmol), cesium carbonate (2.16 g, 6.64 mmol), 2-di-tert-butylphosphino-2',4',6'-triisopropylbiphenyl (141.03 mg, 332.12 µmol), and allylpalladium(II) chloride dimer (60.76 mg, 166.06 µmol) were sequentially added to 1,4-dioxane (4 mL). The mixture was subjected to argon exchange 3 times and heated to 100°C with stirring for 1 hour. The reaction mixture was cooled to room temperature and concentrated under reduced pressure. Methanol (10 mL) was added to the residue, and the insoluble material was removed by filtration. The filtrate was concentrated under reduced pressure, and the residue was purified by silica gel column chromatography (eluent: System A) to obtain methyl 2-(3-(cyclopropanesulfonamido)phenyl)-2,3-dihydro-1H-indene-2-carboxylate **40c** (800 mg) with a yield of 64.85%.

MS m/z (ESI): 389.1 [M+18].

### Step 3

### 2-(3-(cyclopropanesulfonamido)phenyl)-N-(4-(6-ethoxypyrazin-2-yl)phenyl)-2,3-dihydro-1H-ind ene-2-carboxamide

Methyl 2-(3-(cyclopropanesulfonamido)phenyl)-2,3-dihydro-1H-indene-2-carboxylate **40c** (122.85 mg, 570.72 µmol) was added to toluene (1.24 mL). A solution of trimethylaluminum in toluene (2 M, 1.03 mL) was added, and the mixture was stirred at room temperature for 1 hour. 4-(6-Ethoxypyrazin-2-yl)aniline **3b** (220 mg, 570.72 µmol) was added, and the mixture was heated to 90°C and stirred for 5 hours. Methanol (1 mL) was added to quench the reaction, and the mixture was concentrated under reduced pressure. Dilute hydrochloric acid (1 M, 2 mL) and dichloromethane (5 mL) were added to the residue. The insoluble solid was removed by filtration, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(3-(cyclopropanesulfonamido)phenyl)-N-(4-(6-ethoxypyrazin-2-yl)phenyl)-2,3-dihydro-1H-ind ene-2-carboxamide **40** (160 mg) with a yield of 40.75%.

MS m/z (ESI): 555.1 [M+1]
¹H NMR (400 MHz, DMSO-*d*₆) δ 9.71 (s, 1H), 9.63 (s, 1H), 8.73 (s, 1H), 8.16 (s, 1H), 8.05 (d, J = 8.6 Hz, 2H), 7.76 (d, J = 8.6 Hz, 2H), 7.31 - 7.20 (m, 3H), 7.22 - 7.10 (m, 3H), 7.08 (dd, J = 7.9, 2.1 Hz, 1H), 4.46 (q, J = 7.0 Hz, 2H), 3.90 (d, J = 15.9 Hz, 2H), 3.36 (d, J = 16.0 Hz, 2H), 2.45 (d, J = 4.6 Hz, 1H), 1.38 (t, J = 7.0 Hz, 3H), 0.89 - 0.70 (m, 4H).

### Example 41

### 2-(6-(cyclopropanesulfonamido)pyrimidin-4-yl)-N-(4-(6-ethoxypyrazin-2-yl)phenyl)-2,3-dihydro -1H-indene-2-carboxamide

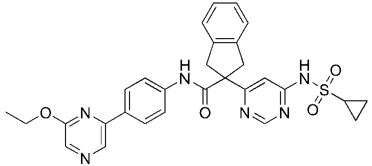

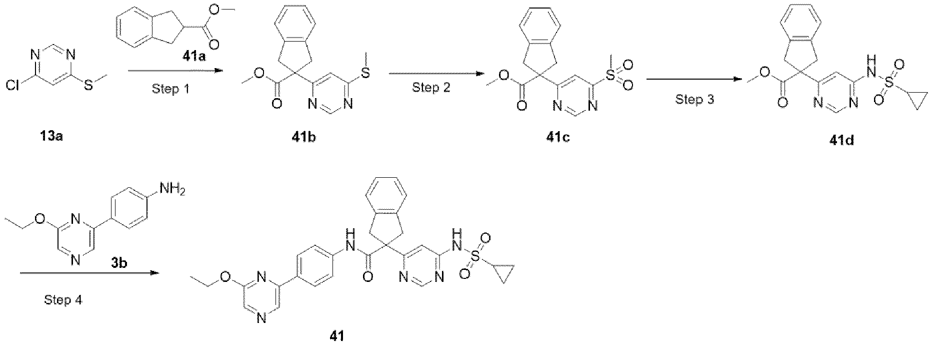

### Step 1

### methyl 2-(6-(methylthio)pyrimidin-4-yl)-2,3-dihydro-1H-indene-2-carboxylate

4-Chloro-6-(methylthio)pyrimidine **13a** (328.16 mg, 2.04 mmol) and methyl 2,3-dihydro-1H-indene-2-carboxylate **41a** (300 mg, 1.70 mmol, commercially available) were sequentially added to tetrahydrofuran (1 mL). The mixture was subjected to argon exchange three times. At -30°C, a solution of lithium bis(trimethylsilyl)amide in tetrahydrofuran (1 M, 5.11 mL) was slowly added dropwise, and the mixture was stirred at room temperature for 16 hours. The reaction mixture was concentrated under reduced pressure, and the residue was purified by silica gel column chromatography (eluent: System A) to obtain methyl 2-(6-(methylthio)pyrimidin-4-yl)-2,3-dihydro-1H-indene-2-carboxylate **41b** (290 mg) with a yield of 56.71%.

MS m/z (ESI): 301.0 [M+1]

### Step 2

### methyl 2-(6-(methylsulfonyl)pyrimidin-4-yl)-2,3-dihydro-1H-indene-2-carboxylate

Methyl 2-(6-(methylthio)pyrimidin-4-yl)-2,3-dihydro-1H-indene-2-carboxylate **41b** (290 mg, 965.46 µmol) and meta-chloroperoxybenzoic acid (366.53 mg, 2.12 mmol) were sequentially added to dichloromethane (0.5 mL). The mixture was subjected to argon exchange three times and stirred at room temperature for 16 hours. Ethyl acetate (30 mL) and water (15 mL) were added to the reaction mixture, and the mixture was partitioned. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure to obtain methyl 2-(6-(methylsulfonyl)pyrimidin-4-yl)-2,3-dihydro-1H-indene-2-carboxylate 41c (220 mg) with a yield of 68.56%.

MS m/z (ESI): 333.1 [M+1]

### Step 3

### methyl 2-(6-(cyclopropanesulfonamido)pyrimidin-4-yl)-2,3-dihydro-1H-indene-2-carboxylate

Methyl 2-(6-(methylsulfonyl)pyrimidin-4-yl)-2,3-dihydro-1H-indene-2-carboxylate 41c (630 mg, 1.90 mmol), cyclopropanesulfonamide (275.58 mg, 2.27 mmol), and cesium carbonate (1.24 g, 3.79 mmol) were sequentially added to N-methylpyrrolidone (2 mL). The mixture was subjected to argon exchange three times and heated to 80°C with stirring for 2 hours. The reaction mixture was cooled to room temperature and concentrated under reduced pressure. The residue was purified by silica gel column chromatography (eluent: System A) to obtain methyl 2-(6-(cyclopropanesulfonamido)pyrimidin-4-yl)-2,3-dihydro-1H-indene-2-carboxylate **41d** (398 mg) with a yield of 56.23%.

MS m/z (ESI): 374.1 [M+1]

### Step 4

### 2-(6-(cyclopropanesulfonamido)pyrimidin-4-yl)-N-(4-(6-ethoxypyrazin-2-yl)phenyl)-2,3-dihydro -1H-indene-2-carboxamide

4-(6-Ethoxypyrazin-2-yl)aniline **3b** (69.17 mg, 321.35 µmol) was added to toluene (1.24 µL). The mixture was subjected to argon exchange three times. A solution of trimethylaluminum in toluene (2 M, 468.63 µL) was slowly added dropwise, and the mixture was stirred for 0.5 hours. Methyl 2-(6-(cyclopropanesulfonamido)pyrimidin-4-yl)-2,3-dihydro-1H-indene-2-carboxylate **41d** (100.00 mg, 267.79 µmol) was added, and the mixture was heated to 90°C and stirred for 5 hours. The reaction mixture was cooled to room temperature and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(cyclopropanesulfonamido)pyrimidin-4-yl)-N-(4-(6-ethoxypyrazin-2-yl)phenyl)-2,3-dihydro -1H-indene-2-carboxamide **41** (81 mg) with a yield of 41.90%.

MS m/z (ESI): 557.1 [M+1]
¹H NMR (400 MHz, DMSO-*d*₆) δ 9.76 (s, 1H), 8.75 (s, 2H), 8.17 (s, 1H), 8.07 (d, J = 8.8 Hz, 2H), 7.77 (d, J = 8.8 Hz, 2H), 7.26 (dd, J = 5.4, 3.3 Hz, 2H), 7.17 (dd, J = 5.5, 3.2 Hz, 2H), 6.99 (s, 1H), 4.47 (q, J = 7.0 Hz, 2H), 3.85 (s, 2H), 3.81 (s, 2H), 3.50 (d, J = 16.4 Hz, 2H), 3.01 (s, 1H), 1.39 (t, J = 7.1 Hz, 3H), 1.07-0.92 (m, 4H).

### Example 42

### N-(4-(5-chloropyrazolo[1,5-a]pyridin-3-yl)phenyl)-3-(4-(cyclopropanesulfonamido)pyrimidin-2-yl)bicyclo[3.1.0]hexane-3-carboxamide

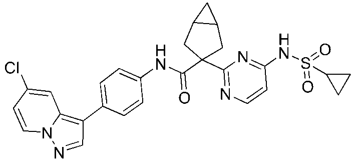

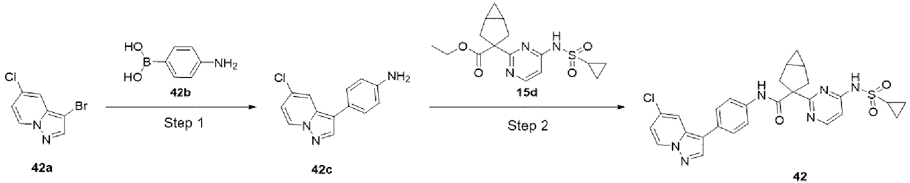

### Step 1

### 4-(5-chloropyrazolo[1,5-a]pyridin-3-yl)aniline

3-Bromo-5-chloropyrazolo[1,5-a]pyridine **42a** (230 mg, 993.62 µmol, prepared according to published patent WO2022087634 A1), (4-aminophenyl)boronic acid **42b** (149.68 mg, 1.09 mmol, commercially available), 1,1-bis(diphenylphosphino)ferrocene palladium(II) dichloride (72.70 mg, 99.36 µmol), and potassium carbonate (411.98 mg, 2.98 mmol) were added to a mixed solvent of 1,4-dioxane (2 mL) and water (0.5 mL). The mixture was subjected to argon exchange three times and heated to 95°C with stirring for 1.5 hours. The reaction mixture was cooled to room temperature and concentrated under reduced pressure. The residue was purified by silica gel column chromatography (eluent: System A) to obtain 4-(5-chloropyrazolo[1,5-a]pyridin-3-yl)aniline **42c** (130 mg) with a yield of 53.69%.

MS m/z (ESI): 244.1 [M+1]

### Step 2

### N-(4-(5-chloropyrazolo[1,5-a]pyridin-3-yl)phenyl)-3-(4-(cyclopropanesulfonamido)pyrimidin-2-yl)bicyclo[3.1.0]hexane-3-carboxamide

4-(5-Chloropyrazolo[1,5-a]pyridin-3-yl)aniline **42c** (61.02 mg, 250.41 µmol) was added to toluene (1.86 mL). A toluene solution of trimethylaluminum (2 M, 409.77 µL) was added, and the mixture was stirred at room temperature for 1 hour. Ethyl 3-(4-(cyclopropanesulfonamido)pyrimidin-2-yl)bicyclo[3.1.0]hexane-3-carboxylate **15d** (80 mg, 227.65 µmol) was added, and the mixture was heated to 90°C with stirring for 1.5 hours. Methanol (1 mL) was added to quench the reaction, and the mixture was concentrated under reduced pressure. Dilute hydrochloric acid (1 M, 2 mL) and dichloromethane (5 mL) were added to the residue. The insoluble solid was removed by filtration, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(5-chloropyrazolo[1,5-a]pyridin-3-yl)phenyl)-3-(4-(cyclopropanesulfonamido)pyrimidin-2-yl)bicyclo[3.1.0]hexane-3-carboxamide 42 (25 mg) with a yield of 15.57%.

MS m/z (ESI): 549.1 [M+1]
¹H NMR (400 MHz, DMSO-*d*₆) δ 11.09 (s, 1H), 9.30 (s, 1H), 8.59 (d, J = 7.4 Hz, 1H), 8.32 (d, J = 5.8 Hz, 1H), 8.21 (s, 1H), 7.84 (d, J = 2.3 Hz, 1H), 7.54 (d, J = 8.2 Hz, 2H), 7.43 (d, J = 8.3 Hz, 2H), 6.80 (dd, J = 7.4, 2.3 Hz, 1H), 6.61 (d, J = 5.7 Hz, 1H), 3.03-2.95 (m, 1H), 2.67 (d, J = 13.4 Hz, 2H), 2.40 (d, J = 13.4 Hz, 2H), 1.22 (dd, J = 7.8, 4.0 Hz, 2H), 0.88 (d, J = 4.2 Hz, 2H), 0.68 (dt, J = 7.9, 3.5 Hz, 2H), 0.20 (d, J = 6.8 Hz, 1H), -0.00 (d, J = 4.4 Hz, 1H).

### Example 43

### (1R,5S)-6-(4-(cyclopropanesulfonamido)pyrimidin-2-yl)-N-(4-(6-ethoxypyrazin-2-yl)phenyl)bic yclo[3.1.0]hexane-6-carboxamide

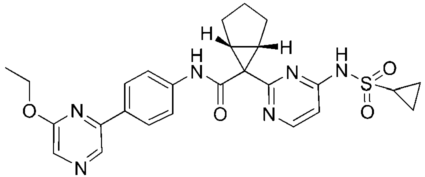

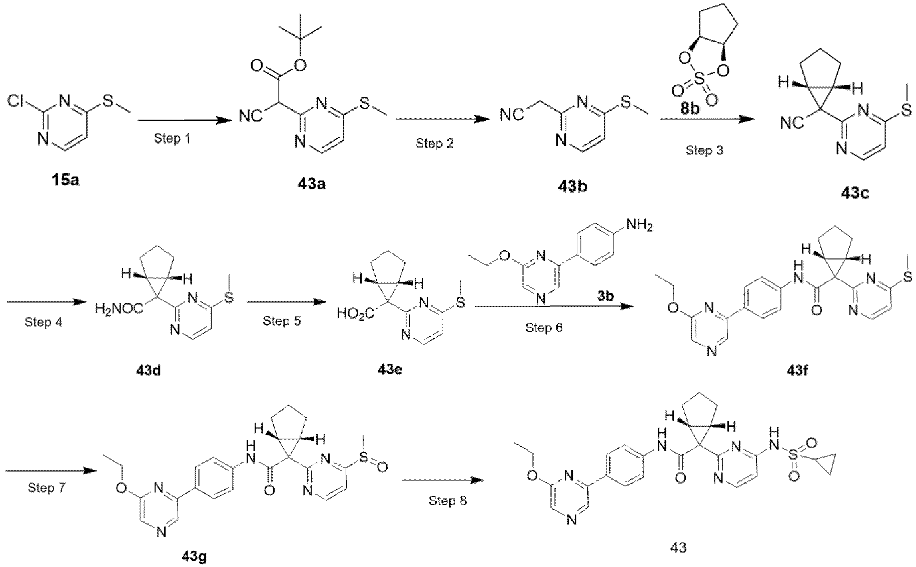

### Step 1

### tert-butyl 2-cyano-2-(4-(methylthio)pyrimidin-2-yl)acetate

Under ice-water bath, tert-butyl 2-cyanoacetate (11.86 g, 84.05 mmol, commercially available) was added to tetrahydrofuran (100 mL). Sodium hydride (4.48 g, 112.06 mmol, 60% purity) was added in batches, and the mixture was stirred at room temperature for 0.5 hours. 2-Chloro-4-(methylthio)pyrimidine 15a (9.00 g, 56.03 mmol) was added, and the mixture was heated to 75°C with stirring for 18 hours. Water (100 mL) and ethyl acetate (100 mL) were added, and the mixture was partitioned. The organic phase was dried over anhydrous sodium sulfate, filtered, and concentrated under reduced pressure to obtain tert-butyl 2-cyano-2-(4-(methylthio)pyrimidin-2-yl)acetate **43a** (14 g) with a yield of 47.08%.

MS m/z (ESI): 266.3 [M+1]

### Step 2

### 2-(4-(methylthio)pyrimidin-2-yl)acetonitrile

Tert-butyl 2-cyano-2-(4-(methylthio)pyrimidin-2-yl)acetate **43a** (14.87 g, 28.02 mmol) and trifluoroacetic acid (10 mL) were added to dichloromethane (30 mL), and the mixture was stirred at room temperature overnight. The reaction mixture was concentrated under reduced pressure. Saturated sodium bicarbonate solution was added to the residue to adjust pH to 7 to 8, and the mixture was extracted with ethyl acetate (50 mL × 3). The organic phase was dried over anhydrous sodium sulfate, filtered, and evaporated to dryness by rotation. The residue was purified by silica gel column chromatography (eluent: System B) to obtain 2-(4-(methylthio)pyrimidin-2-yl)acetonitrile 43b (4.1 g) with a yield of 88.56%.

MS m/z (ESI): 166.2 [M+1]

### Step 3

### (1R,5S)-6-(4-(methylthio)pyrimidin-2-yl)bicyclo[3.1.0]hexane-6-carbonitrile

Under ice-water bath, 2-(4-(methylthio)pyrimidin-2-yl)acetonitrile **43b** (1 g, 6.05 mmol) was added to anhydrous dimethoxyethane (40 mL). Sodium hydride (532.64 mg, 13.32 mmol, 60% purity) was added, and the mixture was stirred at room temperature for 0.5 hours. (3aR,6aS)-Tetrahydro-4H-cyclopenta[d][1,3,2]dioxathiole 2,2-dioxide **8b** (1.49 g, 9.08 mmol) was added, and the mixture was heated to 65°C and stirred for 6 hours. Water (50 mL) and ethyl acetate (50 mL) were added, and the mixture was separated. The organic phase was dried over anhydrous sodium sulfate, filtered, and evaporated to dryness by rotation. The residue was purified by silica gel column chromatography (eluent: System B) to obtain (1R,5S)-6-(4-(methylthio)pyrimidin-2-yl)bicyclo[3.1.0]hexane-6-carbonitrile **43c** (700 mg) with a yield of 50.00%.

MS m/z (ESI): 232.0 [M+1]

### Step 4

### (1R,5S)-6-(4-(methylthio)pyrimidin-2-yl)bicyclo[3.1.0]hexane-6-carboxamide

(1R,5S)-6-(4-(methylthio)pyrimidin-2-yl)bicyclo[3.1.0]hexane-6-carbonitrile **43c** (700 mg, 3.03 mmol) and sodium hydroxide (242.09 mg, 6.05 mmol) were added to dimethyl sulfoxide (3 mL). Hydrogen peroxide (3 mL) was added, and the mixture was heated to 30°C and stirred for 18 hours. Water (30 mL) was added, and the mixture was extracted with ethyl acetate (30 mL × 3). The organic phases were combined, dried over anhydrous sodium sulfate, filtered, and concentrated under reduced pressure. The residue was purified by silica gel column chromatography (eluent: System B) to obtain (1R,5S)-6-(4-(methylthio)pyrimidin-2-yl)bicyclo[3.1.0]hexane-6-carboxamide **43d** (430 mg) with a yield of 56.99%.

MS m/z (ESI): 250.0 [M+1]

### Step 5

### (1R,5S)-6-(4-(methylthio)pyrimidin-2-yl)bicyclo[3.1.0]hexane-6-carboxylic acid

(1R,5S)-6-(4-(methylthio)pyrimidin-2-yl)bicyclo[3.1.0]hexane-6-carboxamide **43d** (430 mg, 1.72 mmol), acetic acid (2 mL), and acetic anhydride (4 mL) were mixed. Sodium nitrite (1.19 g, 17.25 mmol) was added, and the mixture was stirred at room temperature for 2 hours. Water (5 mL) was added to dissolve the reaction mixture, and the mixture was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-(methylthio)pyrimidin-2-yl)bicyclo[3.1.0]hexane-6-carboxylic acid **43e** (170 mg) with a yield of 39.38%.

MS m/z (ESI): 251.0 [M+1]

### Step 6

### (1R,5S)-N-(4-(6-ethoxypyrazin-2-yl)phenyl)-6-(4-(methylthio)pyrimidin-2-yl)bicyclo[3.1.0]hexa ne-6-carboxamide

Under ice-water bath, (1R,5S)-6-(4-(methylthio)pyrimidin-2-yl)bicyclo[3.1.0]hexane-6-carboxylic acid **43e** (88 mg, 351.56 µmol), N-methylpyrrolidone (2 mL), N,N,N',N'-tetramethylchloroformamidinium hexafluorophosphate (196.87 mg, 703.11 µmol), and N-methylimidazole (115.45 mg, 1.41 mmol) were added to a 15 mL single-neck flask. The reaction was performed at room temperature for 0.5 hours. 4-(6-Ethoxypyrazin-2-yl)aniline **3b** (83.24 mg, 386.71 µmol) was added, and the mixture was heated to 90°C and stirred for 18 hours. After the reaction was completed, water (20 mL) was added, and the mixture was extracted with ethyl acetate (20 mL × 3). The combined organic phase was dried over anhydrous sodium sulfate, filtered, and concentrated under reduced pressure. The residue was purified by silica gel column chromatography (eluent: System B) to obtain (1R,5S)-N-(4-(6-ethoxypyrazin-2-yl)phenyl)-6-(4-(methylthio)pyrimidin-2-yl)bicyclo[3.1.0]hexa ne-6-carboxamide **43f** (120 mg) with a yield of 76.27%.

MS m/z (ESI): 448.0 [M+1]

### Step 7

### (1R,5S)-N-(4-(6-ethoxypyrazin-2-yl)phenyl)-6-(4-(methylsulfinyl)pyrimidin-2-yl)bicyclo[3.1.0]h exane-6-carboxamide

(1R,5S)-N-(4-(6-ethoxypyrazin-2-yl)phenyl)-6-(4-(methylthio)pyrimidin-2-yl)bicyclo[3 .1.0]hexane-6-carboxamide **43f** (120 mg, 268.13 µmol) and meta-chloroperoxybenzoic acid (97.98 mg, 482.63 µmol, 85% purity) were added to dichloromethane (2 mL), and the mixture was stirred at room temperature for 2 hours. Saturated sodium bicarbonate solution was added to neutralize the mixture, and the mixture was extracted with dichloromethane (10 mL × 3). The combined organic phase was dried over anhydrous sodium sulfate, filtered, and concentrated under reduced pressure to obtain (1R,5S)-N-(4-(6-ethoxypyrazin-2-yl)phenyl)-6-(4-(methylsulfinyl)pyrimidin-2-yl)bicyclo[3.1.0]h exane-6-carboxamide **43g** (100 mg) with a yield of 80.46%.

MS m/z (ESI): [M+1] 464.0

### Step 8

### (1R,5S)-6-(4-(cyclopropanesulfonamido)pyrimidin-2-yl)-N-(4-(6-ethoxypyrazin-2-yl)phenyl)bic yclo[3.1.0]hexane-6-carboxamide

(1R,5S)-N-(4-(6-ethoxypyrazin-2-yl)phenyl)-6-(4-(methylsulfinyl)pyrimidin-2-yl)bicycl o[3.1.0]hexane-6-carboxamide **43g** (120 mg, 258.87 µmol), cyclopropanesulfonamide (47.05 mg, 388.31 µmol), and cesium carbonate (168.69 mg, 517.74 µmol) were added to N-methylpyrrolidone (1 mL). The mixture was heated to 90°C and the reaction was performed for 1 hour. The reaction mixture was cooled to room temperature, and water (20 mL) and ethyl acetate (20 mL) were added. The pH was adjusted to 6 to 7 with 1 M dilute hydrochloric acid. The mixture was extracted with ethyl acetate (20 mL × 3). The organic phase was dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(4-(cyclopropanesulfonamido)pyrimidin-2-yl)-N-(4-(6-ethoxypyrazin-2-yl)phenyl)bic yclo[3.1.0]hexane-6-carboxamide **43** (25 mg) with a yield of 14.76%.

MS m/z (ESI): 521.2 [M+1]
¹H NMR (400 MHz, DMSO-*d*₆) δ 11.14 (s, 1H), 10.31 (s, 1H), 8.76 (s, 1H), 8.42 (d, J = 5.6 Hz, 1H), 8.18 (s, 1H), 8.07 (d, J = 8.3 Hz, 2H), 7.83 (d, J = 8.4 Hz, 2H), 6.70 (d, J = 5.6 Hz, 1H), 4.48 (q, J = 7.1 Hz, 2H), 3.26 - 3.15 (m, 1H), 2.21 (s, 2H), 2.12 (dd, J = 13.2, 8.5 Hz, 2H), 2.00 (t, J = 11.2 Hz, 2H), 1.64 (d, J = 11.1 Hz, 1H), 1.40 (t, J = 7.1 Hz, 3H), 1.09 (d, J = 12.2 Hz, 1H), 1.05 - 0.95 (m, 2H), 0.81 (d, J = 7.7 Hz, 2H).

### Example 44

### (1R,5S)-6-(4-(cyclopropanesulfonamido)pyrimidin-2-yl)-N-(4-(6-ethoxypyrazin-2-yl)phenyl)-3-o xabicyclo[3.1.0]hexane-6-carboxamide

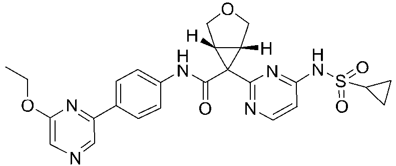

According to the synthesis method of Example 43 of the present disclosure, (3aR,6aS)-tetrahydro-4H-cyclopenta[d][1,3,2]dioxathiole 2,2-dioxide **8b** in Example 43 was replaced with (3aR,6aS)-tetrahydrofuro[3,4-d][1,3,2]dioxathiole 2,2-dioxide 4c to obtain (1R,5S)-6-(4-(cyclopropanesulfonamido)pyrimidin-2-yl)-N-(4-(6-ethoxypyrazin-2-yl)phenyl)-3-o xabicyclo[3.1.0]hexane-6-carboxamide 44.

MS m/z (ESI): 523.2 [M+1].

¹H NMR (400 MHz, DMSO-d6) δ 11.25 (s, 1H), 10.29 (s, 1H), 8.80 (s, 1H), 8.50 (d, J = 5.6 Hz, 1H), 8.22 (s, 1H), 8.12 (d, J = 8.3 Hz, 2H), 7.85 (d, J = 8.4 Hz, 2H), 6.78 (d, J = 5.7 Hz, 1H), 4.53 (q, J = 7.0 Hz, 2H), 4.17 (d, J = 8.8 Hz, 2H), 3.88 (d, J = 8.7 Hz, 2H), 3.37 (s, 2H), 3.30 - 3.20 (m, 1H), 1.45 (t, J = 7.0 Hz, 3H), 1.05 (dd, J = 7.2, 4.3 Hz, 2H), 0.86 (tt, J = 7.6, 5.1, 4.6 Hz, 2H).

### Example 45

### (1R,5S)-6-(6-(cyclopropanesulfonamido)pyrimidin-4-yl)-N-(4-(6-ethoxypyrazin-2-yl)-2-fluoroph enyl)bicyclo[3.1.0]hexane-6-carboxamide

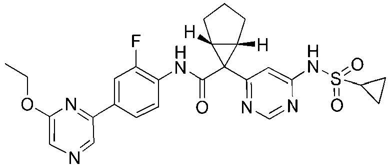

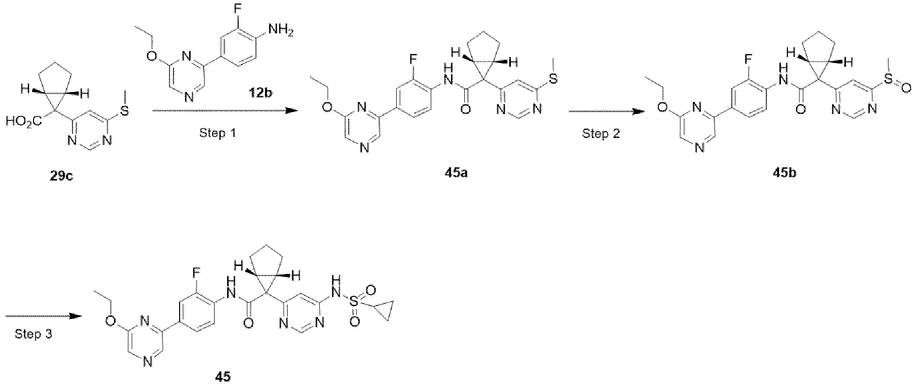

### Step 1

### (1R,5S)-N-(4-(6-ethoxypyrazin-2-yl)-2-fluorophenyl)-6-(6-(methylthio)pyrimidin-4-yl)bicyclo[3. 1.0]hexane-6-carboxamide

Under ice-water bath, (1R,5S)-6-(6-(methylthio)pyrimidin-4-yl)bicyclo[3.1.0]hexane-6-carboxylic acid **29c** (85 mg, 339.57 µmol), N-methylpyrrolidone (2 mL), N,N,N',N'-tetramethylchloroformamidinium hexafluorophosphate (190.16 mg, 679.14 µmol), and N-methylimidazole (111.52 mg, 1.36 mmol) were added to a 15 mL single-neck flask. The reaction was performed at room temperature for 0.5 hours. 4-(6-Ethoxypyrazin-2-yl)-2-fluoroaniline **12b** (87.12 mg, 373.53 µmol) was added, and the mixture was heated to 90°C and stirred for 18 hours. The reaction mixture was cooled to room temperature, and water (20 mL) and ethyl acetate (20 mL) were added. The mixture was extracted with ethyl acetate (20 mL × 3). The organic phase was dried over anhydrous sodium sulfate, filtered, and concentrated under reduced pressure to obtain (1R,5S)-N-(4-(6-ethoxypyrazin-2-yl)-2-fluorophenyl)-6-(6-(methylthio)pyrimidin-4-yl)bicyclo[3. 1.0]hexane-6-carboxamide **45a** (60 mg) with a yield of 18.98%.

MS m/z (ESI): 466.0 [M+1]

### Step 2

### (1R,5S)-N-(4-(6-ethoxypyrazin-2-yl)-2-fluorophenyl)-6-(6-(methylsulfinyl)pyrimidin-4-yl)bicycl o[3.1.0]hexane-6-carboxamide

(1R,5S)-N-(4-(6-ethoxypyrazin-2-yl)-2-fluorophenyl)-6-(6-(methylthio)pyrimidin-4-yl) bicyclo[3.1.0]hexane-6-carboxamide **45a** (60 mg, 128.88 µmol) and meta-chloroperoxybenzoic acid (41.87 mg, 206.21 µmol, 85% purity) were added to dichloromethane (2 mL). The mixture was stirred at room temperature for 3 hours. Saturated sodium bicarbonate solution was added to neutralize the mixture, and the mixture was extracted with dichloromethane (10 mL × 3). The combined organic phase was dried over anhydrous sodium sulfate, filtered, and concentrated under reduced pressure to obtain (1R,5S)-N-(4-(6-ethoxypyrazin-2-yl)-2-fluorophenyl)-6-(6-(methylsulfinyl)pyrimidin-4-yl)bicycl o[3.1.0]hexane-6-carboxamide **45b** (60 mg) with a yield of 96.68%.

MS m/z (ESI): 482.0 [M+1]

### Step 3

### (1R,5S)-6-(6-(cyclopropanesulfonamido)pyrimidin-4-yl)-N-(4-(6-ethoxypyrazin-2-yl)-2-fluoroph enyl)bicyclo[3.1.0]hexane-6-carboxamide

(1R,5S)-N-(4-(6-ethoxypyrazin-2-yl)-2-fluorophenyl)-6-(6-(methylsulfinyl)pyrimidin-4 -yl)bicyclo[3.1.0]hexane-6-carboxamide **45b** (60 mg, 124.60 µmol), cyclopropanesulfonamide (22.64 mg, 186.90 µmol), and cesium carbonate (81.19 mg, 249.20 µmol) were added to N-methylpyrrolidone (1 mL). The mixture was heated to 90°C and stirred for 1 hour. The reaction mixture was cooled to room temperature. Water (20 mL) and ethyl acetate (20 mL) were added, and the pH was adjusted to 6 to 7 with 1 M dilute hydrochloric acid. The mixture was extracted with ethyl acetate (20 mL × 3). The organic phase was dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain (1R,5S)-6-(6-(cyclopropanesulfonamido)pyrimidin-4-yl)-N-(4-(6-ethoxypyrazin-2-yl)-2-fluoroph enyl)bicyclo[3.1.0]hexane-6-carboxamide **45** (3.53 mg) with a yield of 4.26%.

MS m/z (ESI): 539.1 [M+1]
¹H NMR (400 MHz, DMSO-*d*₆) δ 10.27 (s, 1H), 8.86 (s, 1H), 8.68 (s, 1H), 8.26 (s, 1H), 8.07 - 7.93 (m, 3H), 7.01 (s, 1H), 4.49 (q, J = 7.0 Hz, 2H), 2.21 (d, J = 3.3 Hz, 2H), 2.12 (dd, J = 13.3, 8.4 Hz, 2H), 2.00 (d, J = 8.4 Hz, 3H), 1.41 (t, J = 7.0 Hz, 3H), 1.17 (t, J = 7.1 Hz, 2H), 1.05 (d, J = 14.8 Hz, 4H).

### Example 46

### (1R,5S)-6-(4-(cyclopropanesulfonamido)pyrimidin-2-yl)-N-(4-(6-ethoxypyrazin-2-yl)-2-fluoroph enyl)bicyclo[3.1.0]hexane-6-carboxamide

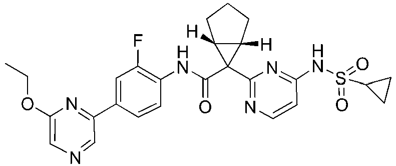

According to the synthesis method of Example 45 of the present disclosure, (1R,5S)-6-(6-(methylthio)pyrimidin-4-yl)bicyclo[3.1.0]hexane-6-carboxylic acid **29c** in Example 45 was replaced with (1R,5S)-6-(4-(methylthio)pyrimidin-2-yl)bicyclo[3.1.0]hexane-6-carboxylic acid **43e** to obtain (1R,5S)-6-(4-(cyclopropanesulfonamido)pyrimidin-2-yl)-N-(4-(6-ethoxypyrazin-2-yl)-2-fluoroph enyl)bicyclo[3.1.0]hexane-6-carboxamide **46.**

MS m/z (ESI): 539.1 [M+1]
¹H NMR (400 MHz, DMSO-*d*₆) δ 11.12 (s, 1H), 10.06 (s, 1H), 8.83 (s, 1H), 8.41 (d, J = 5.6 Hz, 1H), 8.23 (d, J = 2.7 Hz, 2H), 7.97 (d, J = 10.2 Hz, 2H), 6.70 (d, J = 5.6 Hz, 1H), 4.49 (q, J = 7.0 Hz, 2H), 3.21 (d, J = 8.2 Hz, 1H), 2.20 (d, J = 3.0 Hz, 2H), 2.13 (dd, J = 13.2, 8.4 Hz, 2H), 1.98 (d, J = 11.3 Hz, 2H), 1.64 (s, 1H), 1.41 (t, J = 7.0 Hz, 3H), 1.17 (d, J = 6.0 Hz, 1H), 1.05 (t, J = 3.3 Hz, 2H), 0.92 (h, J = 5.2, 4.7 Hz, 2H).

### Example 47

### (1R,5S)-6-(6-(cyclopropanesulfonamido)pyrimidin-4-yl)-N-(5-(6-ethoxypyrazin-2-yl)pyridin-2-y l)-3-oxabicyclo[3.1.0]hexane-6-carboxamide

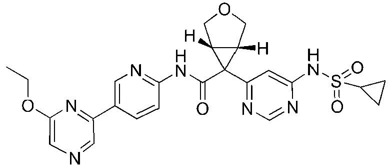

According to the synthesis method of Example 45 of the present disclosure, 4-(6-ethoxypyrazin-2-yl)-2-fluoroaniline **12b** in Example 45 was replaced with 5-(6-ethoxypyrazin-2-yl)pyridin-2-amine **1d** to obtain (1R,5S)-6-(6-(cyclopropanesulfonamido)pyrimidin-4-yl)-N-(5-(6-ethoxypyrazin-2-yl)pyridin-2-y l)-3-oxabicyclo[3.1.0]hexane-6-carboxamide **47.**

MS m/z (ESI): 524.2 [M+1]
¹H NMR (400 MHz, DMSO-*d*₆) δ 11.07 (s, 1H), 9.08 (d, J = 2.5 Hz, 1H), 8.85 (s, 1H), 8.71 (s, 1H), 8.53 (dd, J = 8.7, 2.5 Hz, 1H), 8.34 - 8.20 (m, 2H), 6.95 (s, 1H), 4.49 (q, J = 7.0 Hz, 2H), 4.11 (d, J = 9.0 Hz, 2H), 3.82 (d, J = 8.8 Hz, 2H), 3.04 (s, 1H), 1.41 (t, J = 7.0 Hz, 3H), 1.31 - 1.11 (m, 2H), 1.11 - 0.91 (m, 4H).

### Example 48

### N-(5-(5-chloropyrazolo[1,5-a]pyridin-3-yl)pyridin-2-yl)-3-(6-(cyclopropanesulfonamido)pyrazin-2-yl)bicyclo[3.1.0]hexane-3-carboxamide

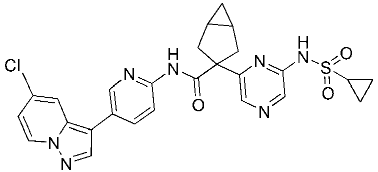

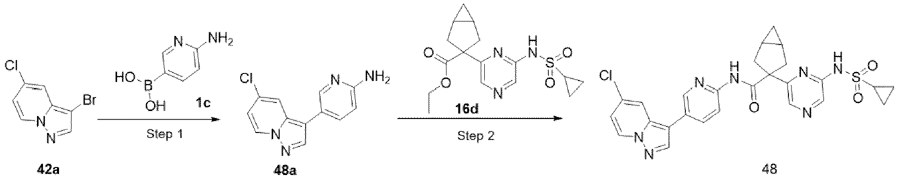

### Step 1

### 5-(5-chloropyrazolo[1,5-a]pyridin-3-yl)pyridin-2-amine

3-Bromo-5-chloropyrazolo[1,5-a]pyridine **42a** (1.1 g, 4.75 mmol), (6-aminopyridin-3-yl)boronic acid **1c** (721.01 mg, 5.23 mmol), 1,1-bis(diphenylphosphino)ferrocene palladium(II) dichloride (347.71 mg, 475.21 µmol), and potassium carbonate (1.97 g, 14.26 mmol) were added to a mixed solvent of 1,4-dioxane (2 mL) and water (0.5 mL). The mixture was subjected to argon exchange three times and heated to 95°C with stirring for 1.5 hours. The reaction mixture was concentrated under reduced pressure. The residue was purified by silica gel column chromatography (eluent: System B) to obtain 5-(5-chloropyrazolo[1,5-a]pyridin-3-yl)pyridin-2-amine **48a** (900 mg, 3.68 mmol, 77.40% yield). MS m/z (ESI): [M+1] 245.1

### Step 2

### N-(5-(5-chloropyrazolo[1,5-a]pyridin-3-yl)pyridin-2-yl)-3-(6-(cyclopropanesulfonamido)pyrazin-2-yl)bicyclo[3.1.0]hexane-3-carboxamide

5-(5-Chloropyrazolo[1,5-a]pyridin-3-yl)pyridin-2-amine **48a** (114.88 mg, 469.52 µmol) was added to toluene (2 mL). A solution of trimethylaluminum in toluene (2 M, 768.31 µL) was added, and the mixture was stirred at room temperature for 1 hour. Ethyl 3-(6-(cyclopropanesulfonamido)pyrazin-2-yl)bicyclo[3.1.0]hexane-3-carboxylate 16d (150 mg, 426.84 µmol) was added, and the mixture was heated to 90°C and stirred for 1.5 hours. Methanol (1 mL) was added to quench the reaction, and the mixture was concentrated under reduced pressure. Dilute hydrochloric acid (1 M, 2 mL) and dichloromethane (5 mL) were added to the residue, and insoluble solid was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(5-(5-chloropyrazolo[1,5-a]pyridin-3-yl)pyridin-2-yl)-3-(6-(cyclopropanesulfonamido)pyrazin-2-yl)bicyclo[3.1.0]hexane-3-carboxamide **48** (3.5 mg) with a yield of 1.10%.

MS m/z (ESI): 550.1 [M+1]
¹H NMR (400 MHz, DMSO-d6) δ 10.86 (s, 1H), 9.87 (s, 1H), 8.63 (d, J = 7.4 Hz, 1H), 8.51 - 8.41 (m, 1H), 8.32 (s, 1H), 8.18 (s, 1H), 8.04 - 7.82 (m, 4H), 6.86 (dd, J = 7.4, 2.2 Hz, 1H), 2.99 - 2.93 (m, 1H), 2.77 (d, J = 13.7 Hz, 2H), 2.27 (d, J = 13.8 Hz, 2H), 1.27 (dd, J = 8.0, 4.2 Hz, 2H), 0.94 - 0.88 (m, 2H), 0.80 - 0.72 (m, 2H), 0.33 - 0.24 (m, 1H), 0.03 - 0.05 (m, 1H).

### Example 49

### N-(4-(5-chloropyrazolo[1,5-a]pyridin-3-yl)phenyl)-3-(6-(cyclopropanesulfonamido)pyrazin-2-yl) bicyclo[3.1.0]hexane-3-carboxamide

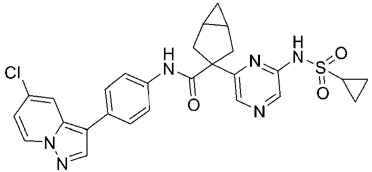

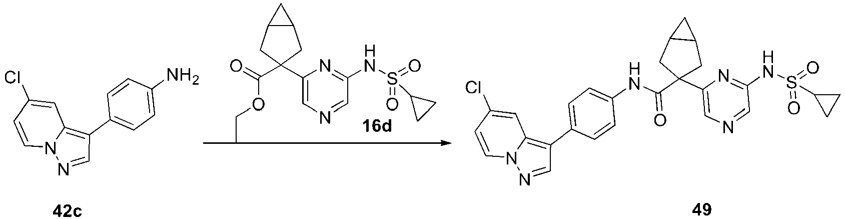

4-(5-Chloropyrazolo[1,5-a]pyridin-3-yl)aniline **42c** (106.79 mg, 438.22 µmol) was added to toluene (1.55 mL). A solution of trimethylaluminum in toluene (2 M, 717.09 µL) was added, and the mixture was stirred at room temperature for 1 hour. Ethyl 3-(6-(cyclopropanesulfonamido)pyrazin-2-yl)bicyclo[3.1.0]hexane-3-carboxylate 16d (140 mg, 398.38 µmol) was added, and the mixture was heated to 90°C and stirred for 1.5 hours. Methanol (1 mL) was added to quench the reaction, and the mixture was concentrated under reduced pressure. Dilute hydrochloric acid (1 M, 2 mL) and dichloromethane (5 mL) were added to the residue, and insoluble solid was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(5-chloropyrazolo[1,5-a]pyridin-3-yl)phenyl)-3-(6-(cyclopropanesulfonamido)pyrazin-2-yl) bicyclo[3.1.0]hexane-3-carboxamide **49** (10 mg) with a yield of 3.53%.

MS m/z (ESI): 549.1 [M+1]
¹H NMR (400 MHz, DMSO-*d*₆) δ 11.08 (s, 1H), 9.50 (s, 1H), 8.76 (d, J = 7.4 Hz, 1H), 8.39 (s, 1H), 8.25 (s, 1H), 8.15 (s, 1H), 8.02 (d, J = 2.3 Hz, 1H), 7.69 - 7.60 (m, 4H), 6.98 (dd, J = 7.4, 2.3 Hz, 1H), 3.19 - 3.09 (m, 1H), 2.93 (d, J = 13.6 Hz, 2H), 2.43 (d, J = 13.6 Hz, 2H), 1.47 - 1.39 (m, 2H), 1.10 (dt, J = 6.6, 3.3 Hz, 2H), 0.96 (dt, J = 7.8, 3.6 Hz, 2H), 0.44 (d, J = 6.4 Hz, 1H), 0.18 (d, J = 4.5 Hz, 1H).

### Example 50

### N-(5-(5-chloropyrazolo[1,5-a]pyridin-3-yl)pyridin-2-yl)-3-(4-(cyclopropanesulfonamido)pyrimid in-2-yl)bicyclo[3.1.0]hexane-3-carboxamide

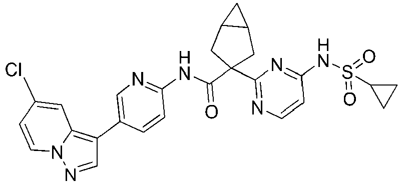

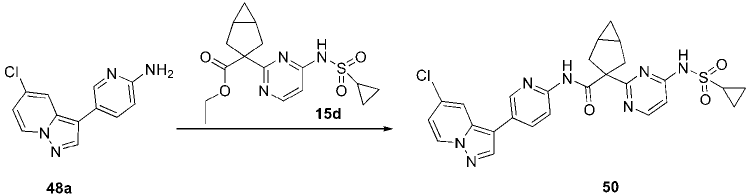

5-(5-Chloropyrazolo[1,5-a]pyridin-3-yl)pyridin-2-amine **48a** (104.44 mg, 426.84 µmol) was added to toluene (2 mL). A solution of trimethylaluminum in toluene (2 M, 768.31 µL) was added, and the mixture was stirred at room temperature for 1 hour. Ethyl 3-(4-(cyclopropanesulfonamido)pyrimidin-2-yl)bicyclo[3.1.0]hexane-3-carboxylate **15d** (150.00 mg, 426.84 µmol) was added, and the mixture was heated to 90°C and stirred for 1.5 hours. Methanol (1 mL) was added to quench the reaction, and the mixture was concentrated under reduced pressure. Dilute hydrochloric acid (1 M, 2 mL) and dichloromethane (5 mL) were added to the residue, and insoluble solid was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(5-(5-chloropyrazolo[1,5-a]pyridin-3-yl)pyridin-2-yl)-3-(4-(cyclopropanesulfonamido)pyrimid in-2-yl)bicyclo[3.1.0]hexane-3-carboxamide **50** (35 mg) with a yield of 11.11%.

MS m/z (ESI): 550.1 [M+1]
¹H NMR (400 MHz, DMSO-*d*₆) δ 11.08 (s, 1H), 9.75 (s, 1H), 8.63 (d, J = 7.4 Hz, 1H), 8.45 (d, J = 1.7 Hz, 1H), 8.38 - 8.29 (m, 2H), 7.94 (dd, J = 21.1, 2.0 Hz, 3H), 6.86 (dd, J = 7.4, 2.2 Hz, 1H), 6.65 (d, J = 5.7 Hz, 1H), 2.98 (s, 1H), 2.66 (d, J = 13.6 Hz, 2H), 2.44 (dd, J = 13.5, 3.3 Hz, 2H), 1.24 (dt, J = 7.8, 3.6 Hz, 2H), 0.91 (dd, J = 4.7, 2.4 Hz, 2H), 0.74 (dd, J = 7.9, 2.7 Hz, 2H), 0.23 (td, J = 8.0, 5.1 Hz, 1H), 0.01 (d, J = 4.3 Hz, 1H).

### Example 51

### 3-(4-(cyclopropanesulfonamido)pyridin-2-yl)-N-(4-(6-ethoxypyrazin-2-yl)phenyl)-6-oxabicyclo[ 3.1.0]hexane-3-carboxamide

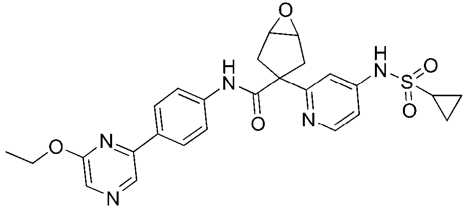

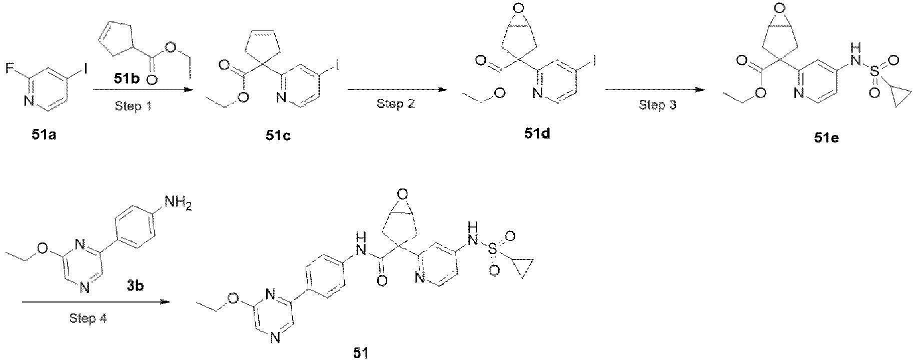

### Step 1

### ethyl 1-(4-iodopyridin-2-yl)cyclopent-3-ene-1-carboxylate

Under ice-water bath, 2-fluoro-4-iodopyridine **51a** (279.38 mg, 1.25 mmol, commercially available) and ethyl cyclopent-3-ene-1-carboxylate **51b** (263.45 mg, 1.88 mmol, commercially available) were added to tetrahydrofuran (3.81 mL). A solution of lithium bis(trimethylsilyl)amide in tetrahydrofuran (1 M, 5.64 mL) was added, and the mixture was stirred at room temperature for 1 hour. Saturated ammonium chloride solution (3 mL) was added to quench the reaction, and the mixture was extracted with ethyl acetate (10 mL × 3). The mixture was dried over anhydrous sodium sulfate, filtered, and concentrated under reduced pressure. The residue was purified by silica gel column chromatography (eluent: System B) to obtain ethyl 1-(4-iodopyridin-2-yl)cyclopent-3-ene-1-carboxylate **51c** (190 mg) with a yield of 26.51%.

MS m/z (ESI): 344.1 [M+1]

### Step 2

### ethyl 3-(4-iodopyridin-2-yl)-6-oxabicyclo[3.1.0]hexane-3-carboxylate

Ethyl 1-(4-iodopyridin-2-yl)cyclopent-3-ene-1-carboxylate **51c** (2 g, 3.50 mmol) and meta-chloroperoxybenzoic acid (1.42 g, 6.99 mmol, 85% purity) were added to dichloromethane (20 mL), and the reaction was performed at room temperature overnight. Saturated sodium bicarbonate solution (10 mL) was added, and the mixture was partitioned. The organic phase was dried over anhydrous sodium sulfate, filtered, and concentrated under reduced pressure. The residue was purified by silica gel column chromatography (eluent: System B) to obtain ethyl 3-(4-iodopyridin-2-yl)-6-oxabicyclo[3.1.0]hexane-3-carboxylate **51d** (780 mg) with a yield of 62.10%.

MS m/z (ESI): 360.0 [M+1]

### Step 3

### ethyl 3-(4-(cyclopropanesulfonamido)pyridin-2-yl)-6-oxabicyclo[3.1.0]hexane-3-carboxylate

Ethyl 3-(4-iodopyridin-2-yl)-6-oxabicyclo[3.1.0]hexane-3-carboxylate **51d** (780 mg, 2.17 mmol), cyclopropanesulfonamide (315.75 mg, 2.61 mmol), cesium carbonate (1.42 g, 4.34 mmol), and allylpalladium chloride (39.73 mg, 108.59 µmol) were sequentially added to 1,4-dioxane (10 mL). The mixture was subjected to argon exchange 3 times and heated to 90°C with stirring for 2 hours. The reaction mixture was cooled to room temperature, and insoluble solid was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain ethyl 3-(4-(cyclopropanesulfonamido)pyridin-2-yl)-6-oxabicyclo[3.1.0]hexane-3-carboxylate **51e** (270 mg) with a yield of 35.28%.

MS m/z (ESI): 353.0 [M+1]

### Step 4

### 3-(4-(cyclopropanesulfonamido)pyridin-2-yl)-N-(4-(6-ethoxypyrazin-2-yl)phenyl)-6-oxabicyclo[ 3.1.0]hexane-3-carboxamide

Ethyl 3-(4-(cyclopropanesulfonamido)pyridin-2-yl)-6-oxabicyclo[3.1.0]hexane-3-carboxylate **51e** (100 mg, 464.57 µmol) was added to toluene (1.83 mL). A solution of trimethylaluminum in toluene (2 M, 836.23 µL) was added, and the mixture was stirred at room temperature for 1 hour. 4-(6-Ethoxypyrazin-2-yl)aniline **3b** (163.72 mg, 464.57 µmol) was added, and the mixture was heated to 90°C with stirring for 1.5 hours. Methanol (1 mL) was added to quench the reaction, and the mixture was concentrated under reduced pressure. Dilute hydrochloric acid (1 M, 2 mL) and dichloromethane (5 mL) were added to the residue, and insoluble solid was removed by filtration. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-(cyclopropanesulfonamido)pyridin-2-yl)-N-(4-(6-ethoxypyrazin-2-yl)phenyl)-6-oxabicyclo[ 3.1.0]hexane-3-carboxamide 51 (2.3 mg) with a yield of 0.73%.

MS m/z (ESI): 552.1 [M+1]

### Example 52

### 2-(4-(cyclopropanesulfonamido)pyridin-2-yl)-N-(4-(6-ethoxypyrazin-2-yl)-2-fluorophenyl)-2,3-d ihydro-1H-indene-2-carboxamide

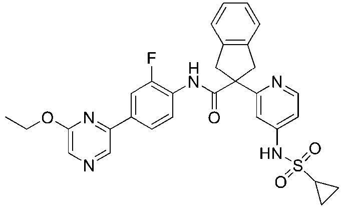

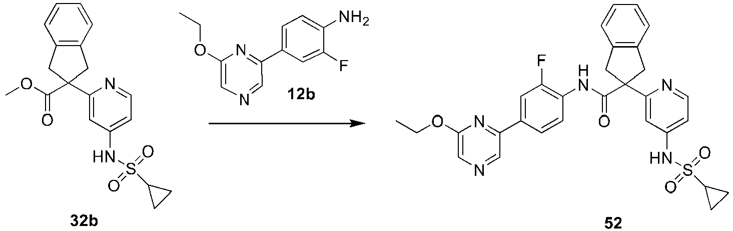

4-(6-Ethoxypyrazin-2-yl)-2-fluoroaniline **12b** (31.31 mg, 134.25 µmol) was added to toluene (1.23 mL). The mixture was subjected to argon exchange 3 times. A solution of trimethylaluminum in toluene (2 M, 234.94 µL) was slowly added dropwise, and the mixture was stirred for 1 hour. Methyl 2-(4-(cyclopropanesulfonamido)pyridin-2-yl)-2,3-dihydro-1H-indene-2-carboxylate 32b (50 mg, 134.25 µmol) was added, and the mixture was heated to 90°C with stirring for 5 hours. The reaction mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(4-(cyclopropanesulfonamido)pyridin-2-yl)-N-(4-(6-ethoxypyrazin-2-yl)-2-fluorophenyl)-2,3-d ihydro-1H-indene-2-carboxamide **52** (22.2 mg) with a yield of 22.27%.

MS m/z (ESI): 556.2 [M+1]
¹H NMR (400 MHz, DMSO-*d*₆) δ 9.91 (s, 1H), 8.82 (s, 1H), 8.50 (d, J = 5.8 Hz, 1H), 8.24 (s, 1H), 8.02-7.92 (m, 2H), 7.81 (t, J = 8.2 Hz, 1H), 7.37 (s, 1H), 7.28 (dt, J = 8.4, 4.1 Hz, 3H), 7.18 (dd, J = 5.5, 3.2 Hz, 2H), 4.47 (q, J = 7.0 Hz, 3H), 3.92 (d, J = 16.2 Hz, 2H), 3.59 (d, J = 16.2 Hz, 2H), 2.83 (q, J = 6.6 Hz, 1H), 1.39 (t, J = 7.0 Hz, 3H), 1.06-0.93 (m, 4H).

### Example 53

### 2-(4-(cyclopropanesulfonamido)pyridin-2-yl)-N-(5-(6-ethoxypyrazin-2-yl)pyridin-2-yl)-2,3-dihy dro-1H-indene-2-carboxamide

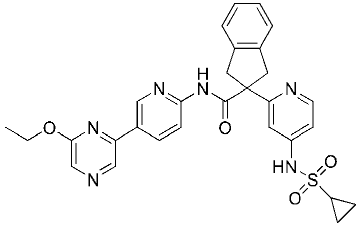

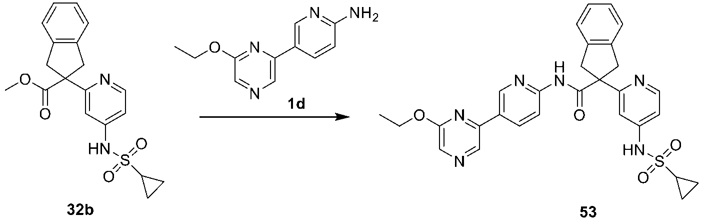

5-(6-Ethoxypyrazin-2-yl)pyridin-2-amine **1d** (29.03 mg, 134.25 µmol) was added to toluene (1.23 mL). The mixture was subjected to argon exchange 3 times. A solution of trimethylaluminum in toluene (2 M, 234.94 µL) was slowly added dropwise, and the mixture was stirred for 1 hour. Methyl 2-(4-(cyclopropanesulfonamido)pyridin-2-yl)-2,3-dihydro-1H-indene-2-carboxylate **32b** (50 mg, 134.25 µmol) was added, and the mixture was heated to 90°C with stirring for 5 hours. The reaction mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(4-(cyclopropanesulfonamido)pyridin-2-yl)-N-(5-(6-ethoxypyrazin-2-yl)pyridin-2-yl)-2,3-dihy dro-1H-indene-2-carboxamide **53** (10.2 mg) with a yield of 9.52%.

MS m/z (ESI): 556.2 [M+1]
¹H NMR (400 MHz, DMSO-*d*₆) δ 10.38 (s, 1H), 9.02 (d, J = 2.4 Hz, 1H), 8.83 (s, 1H), 8.48 (dd, J = 8.8, 2.5 Hz, 1H), 8.43 (d, J = 5.8 Hz, 1H), 8.24 (s, 1H), 8.17 (d, J = 8.8 Hz, 1H), 7.26 (td, J = 5.5, 2.6 Hz, 3H), 7.15 (ddd, J = 10.4, 5.7, 2.6 Hz, 3H), 4.47 (d, J = 7.1 Hz, 2H), 3.88 (d, J = 16.3 Hz, 2H), 3.56 (d, J = 16.3 Hz, 2H), 2.79 - 2.71 (m, 1H), 1.39 (t, J = 7.0 Hz, 3H), 0.98 - 0.91 (m, 4H).

### Example 54

### 2-(6-(cyclopropanesulfonamido)pyrimidin-4-yl)-N-(4-(6-ethoxypyrazin-2-yl)-2-fluorophenyl)-2, 3-dihydro-1H-indene-2-carboxamide

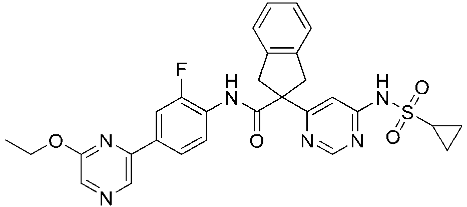

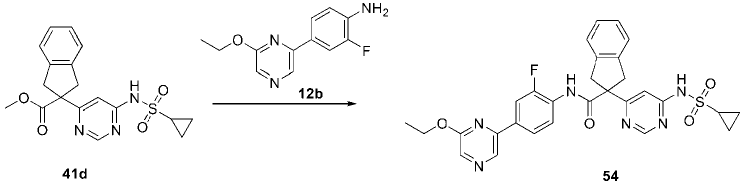

4-(6-Ethoxypyrazin-2-yl)-2-fluoroaniline **12b** (74.95 mg, 321.35 µmol) was added to toluene (2 mL). The mixture was subjected to argon exchange three times. A toluene solution of trimethylaluminum (2 M, 468.63 µL) was slowly added dropwise, and the mixture was stirred for 1 hour. Methyl 2-(6-(cyclopropanesulfonamido)pyrimidin-4-yl)-2,3-dihydro-1H-indene-2-carboxylate **41d** (100.00 mg, 267.79 µmol) was added, and the mixture was heated to 90°C and stirred for 5 hours. The reaction mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(cyclopropanesulfonamido)pyrimidin-4-yl)-N-(4-(6-ethoxypyrazin-2-yl)-2-fluorophenyl)-2, 3-dihydro-1H-indene-2-carboxamide **54** (23.9 mg) with a yield of 12.12%.

MS m/z (ESI): 575.0 [M+1]
^{1H} NMR (400 MHz, DMSO-*d*₆) δ 9.68 (s, 1H), 8.82 (d, J = 10.4 Hz, 2H), 8.24 (s, 1H), 8.02 - 7.93 (m, 2H), 7.76 (t, J = 8.1 Hz, 1H), 7.27 (dd, J = 5.4, 3.3 Hz, 2H), 7.17 (dd, J = 5.5, 3.2 Hz, 2H), 7.04 (s, 1H), 4.48 (q, J = 7.0 Hz, 2H), 3.83 (d, J = 16.3 Hz, 2H), 3.07 (d, J = 28.3 Hz, 1H), 1.39 (t, J = 7.0 Hz, 3H), 1.12 - 0.91 (m, 4H).

### Example 55

### 2-(6-(cyclopropanesulfonamido)pyrimidin-4-yl)-N-(5-(6-ethoxypyrazin-2-yl)pyridin-2-yl)-2,3-di hydro-1H-indene-2-carboxamide

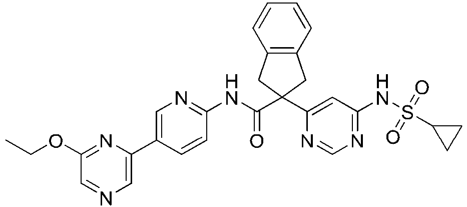

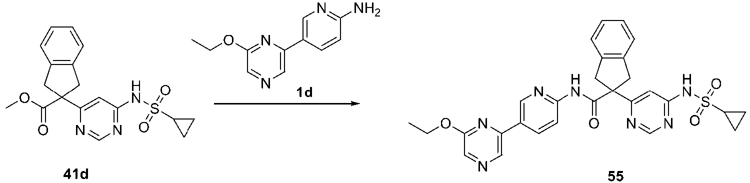

5-(6-Ethoxypyrazin-2-yl)pyridin-2-amine **1d** (69.49 mg, 321.35 µmol) was added to toluene (2 mL). The mixture was subjected to argon exchange three times. A solution of trimethylaluminum (2 M, 468.63 µL) in toluene was slowly added dropwise, and the mixture was stirred for 1 hour. Methyl 2-(6-(cyclopropanesulfonamido)pyrimidin-4-yl)-2,3-dihydro-1H-indene-2-carboxylate **41d** (100.00 mg, 267.79 µmol) was added, and the mixture was heated to 90°C and stirred for 5 hours. The reaction mixture was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(cyclopropanesulfonamido)pyrimidin-4-yl)-N-(5-(6-ethoxypyrazin-2-yl)pyridin-2-yl)-2,3-di hydro-1H-indene-2-carboxamide **55** (1.91 mg) with a yield of 1.01%.

MS m/z (ESI): 558.1 [M+1]
¹H NMR (400 MHz, DMSO-*d*₆) δ 10.48 (s, 1H), 9.03 (d, J = 2.4 Hz, 1H), 8.83 (s, 1H), 8.76 (s, 1H), 8.49 (dd, J = 8.8, 2.5 Hz, 1H), 8.26 - 8.18 (m, 2H), 7.26 (dd, J = 5.4, 3.3 Hz, 2H), 7.17 (dd, J = 5.6, 3.2 Hz, 3H), 6.97 (s, 1H), 4.51 - 4.45 (m, 2H), 3.83 (d, J = 16.4 Hz, 2H), 3.51 (d, J = 16.5 Hz, 2H), 3.04 (d, J = 19.6 Hz, 1H), 1.39 (t, J = 7.0 Hz, 3H), 1.05 - 0.95 (m, 4H).

### Example 56

### 2-(4-(cyclopropanesulfonamido)pyridin-2-yl)-N-(5-(6-ethoxypyrazin-2-yl)pyrimidin-2-yl)-2,3-di hydro-1H-indene-2-carboxamide

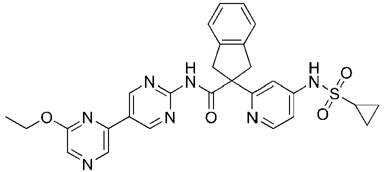

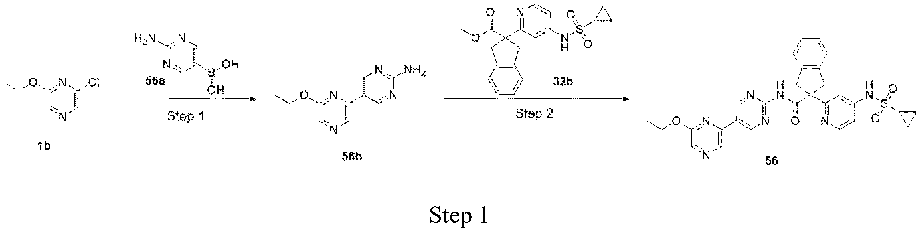

### 5-(6-ethoxypyrazin-2-yl)pyrimidin-2-amine

2-Chloro-6-ethoxypyrazine **1b** (500 mg, 3.15 mmol), (2-aminopyrimidin-5-yl)boronic acid **56a** (525.60 mg, 3.78 mmol, commercially available), 1,1-bis(diphenylphosphino)ferrocene palladium(II) dichloride (230.70 mg, 315.29 µmol), and potassium carbonate (1.31 g, 9.46 mmol) were sequentially added to 1,4-dioxane (2 mL) and water (0.5 mL). The mixture was subjected to argon exchange three times and stirred at 95°C for 3 hours. The reaction mixture was concentrated under reduced pressure, and the resulting residue was purified by silica gel column chromatography (eluent: System B) to obtain 5-(6-ethoxypyrazin-2-yl)pyrimidin-2-amine **56b** (400 mg) with a yield of 58.40%.

MS m/z (ESI): 218.0 [M+1]

### Step 2

### 2-(4-(cyclopropanesulfonamido)pyridin-2-yl)-N-(5-(6-ethoxypyrazin-2-yl)pyrimidin-2-yl)-2,3-di hydro-1H-indene-2-carboxamide

5-(6-Ethoxypyrazin-2-yl)pyrimidin-2-amine **56b** (58.33 mg, 268.50 µmol) was added to toluene (1 mL). The mixture was subjected to argon exchange three times. A solution of trimethylaluminum in toluene (2 M, 469.88 µL) was slowly added dropwise. After stirring for 1 hour, methyl 2-(4-(cyclopropanesulfonamido)pyridin-2-yl)-2,3-dihydro-1H-indene-2-carboxylate **32b** (100 mg, 268.50 µmol) was added, and the mixture was stirred at 90°C for 5 hours. The reaction mixture was concentrated under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(4-(cyclopropanesulfonamido)pyridin-2-yl)-N-(5-(6-ethoxypyrazin-2-yl)pyrimidin-2-yl)-2,3-di hydro-1H-indene-2-carboxamide **56** (17 mg) with a yield of 9.05%.

MS m/z (ESI): 558.1 [M+1]
¹H NMR (400 MHz, DMSO-*d*₆) δ 10.60 (s, 1H), 9.30 (s, 2H), 8.87 (s, 1H), 8.43 (d, J = 5.8 Hz, 1H), 8.30 (s, 1H), 7.29-7.24 (m, 3H), 7.16 (ddt, J = 8.5, 5.8, 3.2 Hz, 3H), 4.48 (q, J = 7.0 Hz, 2H), 3.88 (d, J = 16.3 Hz, 2H), 3.54 (d, J = 16.3 Hz, 2H), 2.76 (tt, J = 7.6, 5.2 Hz, 1H), 1.39 (t, J = 7.0 Hz, 3H), 0.98-0.93 (m, 4H).

### Example 57

### N-(4-(5-chloropyrazolo[1,5-a]pyridin-3-yl)phenyl)-3-(6-(cyclopropanesulfonamido)pyrimidin-4-yl)bicyclo[3.1.0]hexane-3-carboxamide

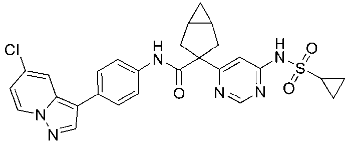

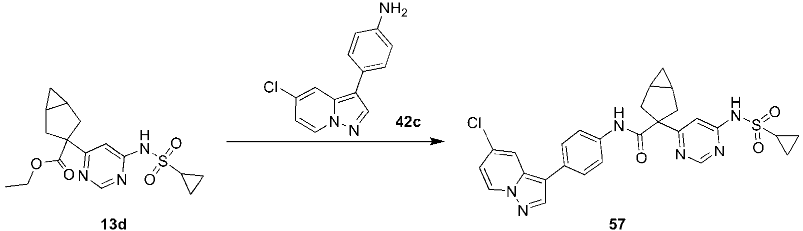

4-(5-chloropyrazolo[1,5-a]pyridin-3-yl)aniline **42c** (91.54 mg, 375.62 µmol) and trimethylaluminum (2.0 M toluene solution) (2 M, 614.65 µL) were added to toluene (2.1 mL). The mixture was stirred at room temperature for 1 hour. Ethyl 3-(6-(cyclopropanesulfonamido)pyrimidin-4-yl)bicyclo[3.1.0]hexane-3-carboxylate 13d (120 mg, 341.47 µmol) was added, and the mixture was heated to 90°C and stirred for 1.5 hours. A small amount of methanol (1 mL) was added to quench the reaction, and the solvent was directly evaporated to dryness by rotation. Dilute hydrochloric acid (1 mL, 1 M) and dichloromethane (5 mL) were added to the residue. The mixture was filtered, and the filtrate was concentrated under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(5-chloropyrazolo[1,5-a]pyridin-3-yl)phenyl)-3-(6-(cyclopropanesulfonamido)pyrimidin-4-yl)bicyclo[3.1.0]hexane-3-carboxamide **57** (25 mg) with a yield of 10.71%.

MS m/z (ESI): [M+1] 549.1.

¹H NMR (400 MHz, DMSO-*d*₆) δ 9.51 (s, 1H), 8.63 (d, J = 8.5 Hz, 2H), 8.26 (s, 1H), 7.88 (s, 1H), 7.59-7.46 (m, 4H), 6.90-6.78 (m, 2H), 2.92 (s, 1H), 2.73 (d, J = 13.5 Hz, 2H), 2.27 (d, J = 13.3 Hz, 2H), 1.28 (s, 2H), 0.97-0.84 (m, 5H), 0.28 (d, J = 7.5 Hz, 1H).

### Example 58

### N-(5-(5-chloropyrazolo[1,5-a]pyridin-3-yl)pyridin-2-yl)-3-(4-(cyclopropanesulfonamido)pyridin-2-yl)bicyclo[3.1.0]hexane-3-carboxamide

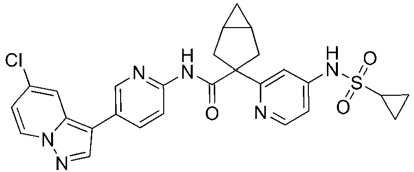

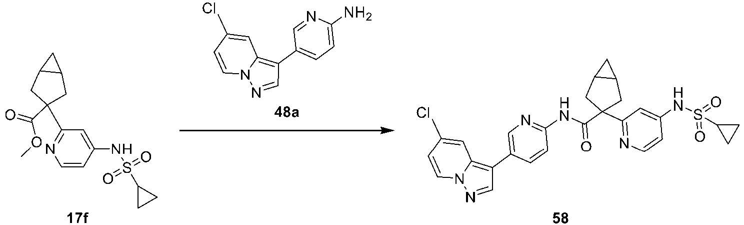

5-(5-Chloropyrazolo[1,5-a]pyridin-3-yl)pyridin-2-amine **48a** (69.12 mg, 282.51 µmol) and trimethylaluminum (2.0 M toluene solution) (2 M, 462.29 µL) were dissolved in toluene (2.3 mL). The mixture was stirred at room temperature for 1 hour. Methyl 3-(4-(cyclopropanesulfonamido)pyridin-2-yl)bicyclo[3.1.0]hexane-3-carboxylate 17f (90 mg, 256.83 µmol) was added, and the mixture was heated to 90°C and stirred for 1.5 hours. A small amount of methanol (1 mL) was added to quench the reaction, and the solvent was directly evaporated to dryness by rotation. Dilute hydrochloric acid (1 mL, 1 M) and dichloromethane (5 mL) were added. The mixture was filtered, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(5-(5-chloropyrazolo[1,5-a]pyridin-3-yl)pyridin-2-yl)-3-(4-(cyclopropanesulfonamido)pyridin-2-yl)bicyclo[3.1.0]hexane-3-carboxamide **58** (6 mg) with a yield of 3.31%.

MS m/z (ESI): [M+1] 549.1.

¹H NMR (400 MHz, DMSO-*d*₆) δ 9.90 (s, 1H), 8.62 (d, J = 7.2 Hz, 1H), 8.48 (s, 1H), 8.29 (d, J = 16.5 Hz, 2H), 8.04-7.85 (m, 3H), 7.07 (s, 2H), 6.85 (d, J = 7.3 Hz, 1H), 2.83-2.66 (m, 3H), 2.24 (d, J = 13.4 Hz, 2H), 1.26 (s, 2H), 0.85 (d, J = 11.3 Hz, 4H), 0.29 (d, J = 7.8 Hz, 1H), -0.00 (s, 1H).

### Example 59

### N-(4-(5-chloropyrazolo[1,5-a]pyridin-3-yl)phenyl)-3-(4-(cyclopropanesulfonamido)pyridin-2-yl) bicyclo[3.1.0]hexane-3-carboxamide

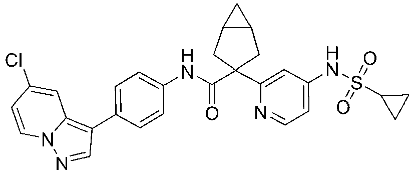

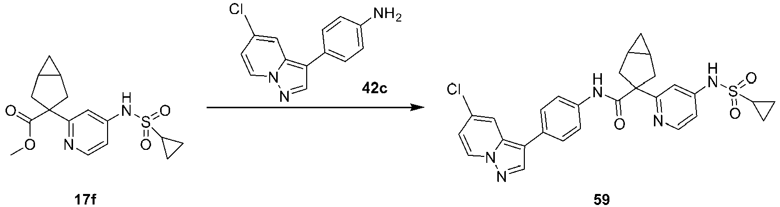

4-(5-chloropyrazolo[1,5-a]pyridin-3-yl)aniline **42c** (107.09 mg, 439.46 µmol) and trimethylaluminum (2.0 M toluene solution) (2 M, 719.11 µL) were added to toluene (2.05 mL). The mixture was stirred at room temperature for 1 hour. Methyl 3-(4-(cyclopropanesulfonamido)pyridin-2-yl)bicyclo[3.1.0]hexane-3-carboxylate 17f (140 mg, 399.51 µmol) was added, and the mixture was heated to 90°C and stirred for 1.5 hours. A small amount of methanol (1 mL) was added to quench the reaction, and the solvent was directly evaporated. Dilute hydrochloric acid (1 mL, 1 M) and dichloromethane (5 mL) were added. The mixture was filtered, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(5-chloropyrazolo[1,5-a]pyridin-3-yl)phenyl)-3-(4-(cyclopropanesulfonamido)pyridin-2-yl) bicyclo[3.1.0]hexane-3-carboxamide 59 (4 mg) with a yield of 1.45%.

MS m/z (ESI): [M+1] 548.1.

1H NMR (400 MHz, DMSO-d6) δ 9.49 (s, 1H), 8.62 (d, J = 7.4 Hz, 1H), 8.26 (d, J = 5.3 Hz, 2H), 7.87 (s, 1H), 7.58-7.45 (m, 4H), 7.02 (d, J = 6.7 Hz, 2H), 6.84 (d, J = 7.4 Hz, 1H), 2.84 (d, J = 13.5 Hz, 2H), 2.66 (d, J = 8.1 Hz, 1H), 2.26 (d, J = 13.3 Hz, 2H), 1.32-1.24 (m, 2H), 0.94-0.78 (m, 4H), 0.27 (d, J = 7.0 Hz, 1H), 0.00 (d, J = 4.9 Hz, 1H).

### Example 60

### 6-(4-(cyclopropanesulfonamido)pyridin-2-yl)-N-(4-(6-ethoxypyrazin-2-yl)phenyl)-6,7-dihydro-5 H-cyclopenta[b]pyrazine-6-carboxamide

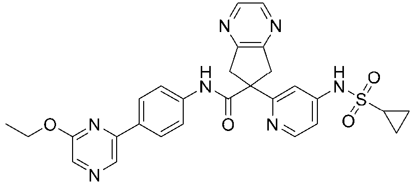

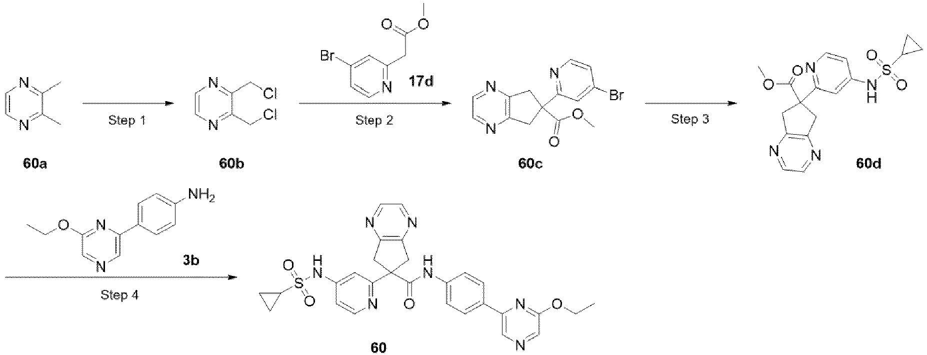

### Step 1

### 2,3-bis(chloromethyl)pyrazine

2,3-Dimethylpyrazine **60a** (500 mg, 4.62 mmol, commercially available), N-chlorosuccinimide (1.54 g, 11.56 mmol), and benzoyl peroxide (112.00 mg, 462.36 µmol) were sequentially added to carbon tetrachloride (1 mL). The mixture was subjected to argon exchange for 2 minutes and heated to 100°C with stirring for 16 hours. The mixture was concentrated under reduced pressure. The residue was purified by silica gel column chromatography (eluent: System A) to obtain 2,3-bis(chloromethyl)pyrazine **60b** (400 mg) with a yield of 48.87%.

MS m/z (ESI): 176.9 [M+1]

### Step 2

### methyl 6-(4-bromopyridin-2-yl)-6,7-dihydro-5H-cyclopenta[b]pyrazine-6-carboxylate

Methyl 2-(4-bromopyridin-2-yl)acetate **17d** (320 mg, 1.39 mmol) was added to N,N-dimethylformamide (16 mL). The mixture was subjected to argon exchange three times. Under cooling with ice-water bath, sodium hydroxide (255.94 mg, 6.40 mmol) was slowly added. After stirring for 0.5 hours, 2,3-bis(chloromethyl)pyrazine **60b** (246.24 mg, 1.39 mmol) was added. The mixture was stirred at room temperature for 23 hours. The reaction mixture was cooled to room temperature. Water (20 mL) and ethyl acetate (20 mL) were added, and the mixture was extracted with ethyl acetate (20 mL × 3). The combined organic phase was washed with saturated sodium chloride solution, dried over anhydrous sodium sulfate, filtered, and concentrated under reduced pressure. The residue was purified by silica gel column chromatography (eluent: System B) to obtain methyl 6-(4-bromopyridin-2-yl)-6,7-dihydro-5H-cyclopenta[b]pyrazine-6-carboxylate **60c** (240 mg) with a yield of 51.63%.

MS m/z (ESI): 333.9 [M+1]

### Step 3

### methyl 6-(4-(cyclopropanesulfonamido)pyridin-2-yl)-6,7-dihydro-5H-cyclopenta[b]pyrazine-6-carboxyla te

Methyl 6-(4-bromopyridin-2-yl)-6,7-dihydro-5H-cyclopenta[b]pyrazine-6-carboxylate 60c (270 mg, 807.98 µmol), cyclopropanesulfonamide (146.84 mg, 1.21 mmol), cesium carbonate (526.51 mg, 1.62 mmol), 2-di-tert-butylphosphino-2',4',6'-triisopropylbiphenyl (68.62 mg, 161.60 µmol), and allylpalladium(II) chloride dimer (29.56 mg, 80.80 µmol) were sequentially added to 1,4-dioxane (2 mL). The mixture was subjected to argon exchange three times and heated to 90°C with stirring for 2 hours. Water (20 mL) and ethyl acetate (20 mL) were added, and the mixture was extracted with ethyl acetate (20 mL × 3). The combined organic phase was washed with saturated sodium chloride solution (20 mL), dried over anhydrous sodium sulfate, filtered, and concentrated under reduced pressure. The residue was purified by silica gel column chromatography (eluent: System B) to obtain methyl 6-(4-(cyclopropanesulfonamido)pyridin-2-yl)-6,7-dihydro-5H-cyclopenta[b]pyrazine-6-carboxyla te 60d (260 mg) with a yield of 85.95%.

MS m/z (ESI): 375.0 [M+1]

### Step 4

### 6-(4-(cyclopropanesulfonamido)pyridin-2-yl)-N-(4-(6-ethoxypyrazin-2-yl)phenyl)-6,7-dihydro-5 H-cyclopenta[b]pyrazine-6-carboxamide

4-(6-Ethoxypyrazin-2-yl)aniline **3b** (50 mg, 232.29 µmol) was added to toluene (2.83 mL). The mixture was subjected to argon exchange three times. Trimethylaluminum (2.0 M toluene solution) (2 M, 406.50 µL) was slowly added dropwise. After stirring for 1 hour, methyl 6-(4-(cyclopropanesulfonamido)pyridin-2-yl)-6,7-dihydro-5H-cyclopenta[b]pyrazine-6-carboxyla te **60d** (86.97 mg, 232.29 µmol) was added, and the mixture was heated to 90°C with stirring for 5 hours. The mixture was concentrated under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 6-(4-(cyclopropanesulfonamido)pyridin-2-yl)-N-(4-(6-ethoxypyrazin-2-yl)phenyl)-6,7-dihydro-5 H-cyclopenta[b]pyrazine-6-carboxamide **60** (35 mg) with a yield of 21.74%.

MS m/z (ESI): 558.2 [M+1]
1H NMR (400 MHz, DMSO-d6) δ 10.58 (s, 1H), 9.88 (s, 1H), 8.76 (s, 1H), 8.36 (d, J = 10.3 Hz, 3H), 8.17 (s, 1H), 8.08 (d, J = 8.8 Hz, 2H), 7.79 (d, J = 8.9 Hz, 2H), 7.22 (s, 1H), 7.10 (dd, J = 5.6, 2.1 Hz, 1H), 4.47 (q, J = 7.0 Hz, 2H), 3.94 (d, J = 17.2 Hz, 2H), 3.68 (d, J = 17.2 Hz, 2H), 1.39 (t, J = 7.0 Hz, 3H), 0.98-0.91 (m, 4H).

### Example 61

### 2-(4-(cyclopropanesulfonamido)pyrimidin-2-yl)-N-(5-(6-ethoxypyrazin-2-yl)pyridin-2-yl)-2,3-di hydro-1H-indene-2-carboxamide

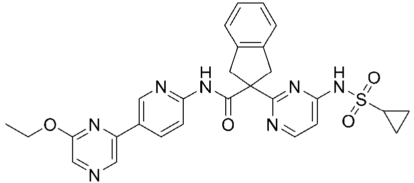

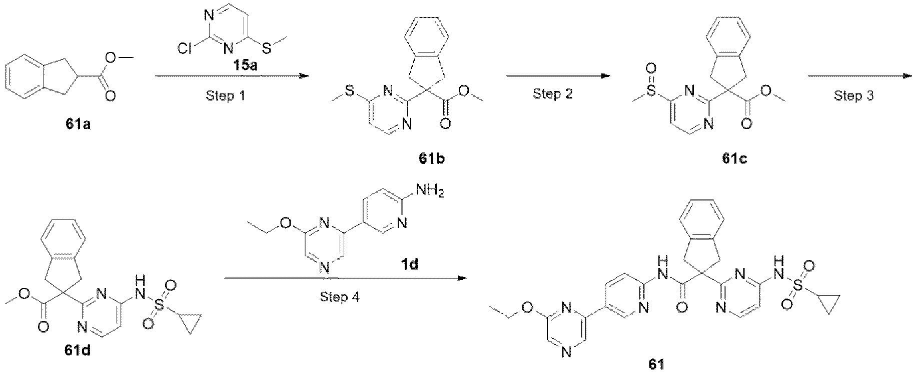

### Step 1

### methyl 2-(4-(methylthio)pyrimidin-2-yl)-2,3-dihydro-1H-indene-2-carboxylate

2-Chloro-4-(methylthio)pyrimidine **15a** (250 mg, 1.56 mmol) and methyl 2,3-dihydro-1H-indene-2-carboxylate **61a** (822.78 mg, 4.67 mmol, commercially available) were sequentially added to tetrahydrofuran (3 mL). The mixture was subjected to argon exchange three times. At -30°C, lithium bis(trimethylsilyl)amide (1.2 M tetrahydrofuran solution) (1.2 M, 4.67 mL) was slowly added dropwise, and then the mixture was stirred at room temperature for 16 hours. The reaction mixture was concentrated under reduced pressure, and the resulting residue was purified by silica gel column chromatography (eluent: System A) to obtain methyl 2-(4-(methylthio)pyrimidin-2-yl)-2,3-dihydro-1H-indene-2-carboxylate **61b** (350 mg) with a yield of 74.86%.

MS m/z (ESI): 301.0 [M+1]

### Step 2

### methyl 2-(4-(methylsulfinyl)pyrimidin-2-yl)-2,3-dihydro-1H-indene-2-carboxylate

Methyl 2-(4-(methylthio)pyrimidin-2-yl)-2,3-dihydro-1H-indene-2-carboxylate **61b** (450 mg, 1.50 mmol) and meta-chloroperoxybenzoic acid (568.77 mg, 3.30 mmol) were sequentially added to dichloromethane (0.5 mL). The mixture was subjected to argon exchange three times and stirred at room temperature for 16 hours. The reaction mixture was concentrated under reduced pressure to obtain methyl 2-(4-(methylsulfinyl)pyrimidin-2-yl)-2,3-dihydro-1H-indene-2-carboxylate 61c (473 mg), which was directly used in the next step.

MS m/z (ESI): 317.1 [M+1]

### Step 3

### methyl 2-(4-(cyclopropanesulfonamido)pyrimidin-2-yl)-2,3-dihydro-1H-indene-2-carboxylate

Methyl 2-(4-(methylsulfinyl)pyrimidin-2-yl)-2,3-dihydro-1H-indene-2-carboxylate **61c** (474 mg, 1.50 mmol), cyclopropanesulfonamide (217.83 mg, 1.80 mmol), and cesium carbonate (976.30 mg, 3.00 mmol) were sequentially added to N-methylpyrrolidone (2 mL). The mixture was subjected to argon exchange three times, heated to 80°C, and stirred for 2 hours. Water (20 mL) and ethyl acetate (20 mL) were added to the reaction mixture, and the mixture was extracted with ethyl acetate (20 mL × 3). The combined organic phase was washed with saturated sodium chloride solution, dried over anhydrous sodium sulfate, filtered, and concentrated under reduced pressure. The residue was purified by silica gel column chromatography (eluent: System B) to obtain methyl 2-(4-(cyclopropanesulfonamido)pyrimidin-2-yl)-2,3-dihydro-1H-indene-2-carboxylate **61d** (230 mg) with a yield of 41.11%.

MS m/z (ESI): 374.1 [M+1]

### Step 4

### 2-(4-(cyclopropanesulfonamido)pyrimidin-2-yl)-N-(5-(6-ethoxypyrazin-2-yl)pyridin-2-yl)-2,3-di hydro-1H-indene-2-carboxamide

5-(6-Ethoxypyrazin-2-yl)pyridin-2-amine **1d** (50 mg, 231.23 µmol) was added to toluene (2 mL). The mixture was subjected to argon exchange three times. Trimethylaluminum (2.0 M toluene solution) (2 M, 404.65 µL) was slowly added dropwise. After stirring for 1 hour, methyl 2-(4-(cyclopropanesulfonamido)pyrimidin-2-yl)-2,3-dihydro-1H-indene-2-carboxylate **61d** (86.35 mg, 231.23 µmol) was added, and the mixture was heated to 90°C and stirred for 5 hours. The reaction mixture was cooled to room temperature and concentrated under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(4-(cyclopropanesulfonamido)pyrimidin-2-yl)-N-(5-(6-ethoxypyrazin-2-yl)pyridin-2-yl)-2,3-di hydro-1H-indene-2-carboxamide **61** (6.3 mg) with a yield of 3.93%.

MS m/z (ESI): 556.2 [M+1]
1H NMR (400 MHz, DMSO) δ 10.42 (s, 1H), 9.02 (d, J=2.4 Hz, 1H), 8.83 (s, 1H), 8.57-8.44 (m, 2H), 8.24 (s, 1H), 8.18 (d, J=8.8 Hz, 1H), 7.22 (dt, J=7.5, 3.7 Hz, 2H), 7.13 (dd, J=5.5, 3.2 Hz, 2H), 6.83 (d, J=5.7 Hz, 1H), 4.47 (q, J=7.0 Hz, 2H), 3.85 (d, J=16.4 Hz, 4H), 2.87 (tt, J=8.3, 4.8 Hz, 1H), 1.39 (t, J=7.0 Hz, 3H), 1.01 (dt, J=6.9, 3.5 Hz, 2H), 0.82-0.74 (m, 2H).

### Example 62

### N-(5-(5-chloropyrazolo[1,5-a]pyridin-3-yl)pyridin-2-yl)-3-(4-((difluoromethyl)sulfonamido)pyri midin-2-yl)bicyclo[3.1.0]hexane-3-carboxamide

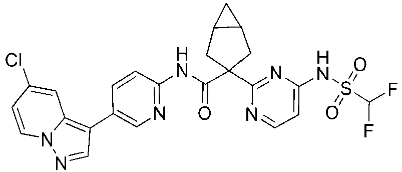

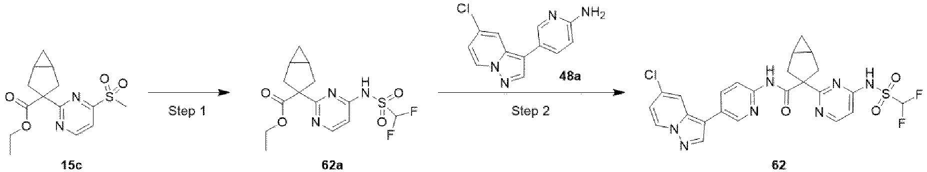

### Step 1

### ethyl 3-(4-((difluoromethyl)sulfonamido)pyrimidin-2-yl)bicyclo[3.1.0]hexane-3-carboxylate

Ethyl 3-(4-(methylsulfonyl)pyrimidin-2-yl)bicyclo[3.1.0]hexane-3-carboxylate 15c (110 mg, 354.42 µmol), difluoromethanesulfonamide (46.46 mg, 354.42 µmol), and cesium carbonate (230.95 mg, 708.84 µmol) were added to N-methylpyrrolidone (1 mL). The mixture was heated to 90°C with stirring for 1 hour, cooled to room temperature, and adjusted to pH 6 using 1 M dilute hydrochloric acid. Water (20 mL) was added, and the mixture was extracted with ethyl acetate (20 mL × 3). The combined organic phase was washed with saturated sodium chloride solution, dried over anhydrous sodium sulfate, filtered, and concentrated under reduced pressure. The residue was purified by silica gel column chromatography (eluent: System A) to obtain ethyl 3-(4-((difluoromethyl)sulfonamido)pyrimidin-2-yl)bicyclo[3.1.0]hexane-3-carboxylate 62a (60 mg) with a yield of 46.85%.

MS m/z (ESI): 362.0 [M+1]

### Step 2

### N-(5-(5-chloropyrazolo[1,5-a]pyridin-3-yl)pyridin-2-yl)-3-(4-((difluoromethyl)sulfonamido)pyri midin-2-yl)bicyclo[3.1.0]hexane-3-carboxamide

5-(5-Chloropyrazolo[1,5-a]pyridin-3-yl)pyridin-2-amine **48a** (45.95 mg, 187.81 µmol) was dissolved in toluene (2.5 mL). Trimethylaluminum (2.0 M toluene solution) (2 M, 307.32 µL) was added, and the mixture was stirred at room temperature for 1 hour. Ethyl 3-(4-((difluoromethyl)sulfonamido)pyrimidin-2-yl)bicyclo[3.1.0]hexane-3-carboxylate **62a** (61.70 mg, 170.74 µmol) was added, and the mixture was heated to 90°C and the reaction was performed for 1.5 hours. The mixture was cooled to room temperature, and a small amount of methanol (1 mL) was added to quench the reaction. The solvent was directly evaporated to dryness by rotation. Dilute hydrochloric acid (1 mL, 1 M) and dichloromethane (5 mL) were added, and the mixture was filtered. The filtrate was evaporated to dryness by rotation, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(5-(5-chloropyrazolo[1,5-a]pyridin-3-yl)pyridin-2-yl)-3-(4-((difluoromethyl)sulfonamido)pyri midin-2-yl)bicyclo[3.1.0]hexane-3-carboxamide 62 (14 mg) with a yield of 10.95%.

MS m/z (ESI): 560.1 [M+1]
¹H NMR (400 MHz, DMSO-d6) δ 10.30 (s, 1H), 8.80 (d, J = 7.4 Hz, 1H), 8.65 (d, J = 2.5 Hz, 1H), 8.50 (s, 1H), 8.28 (s, 1H), 8.17 (dd, J = 8.8, 2.7 Hz, 1H), 8.14-8.06 (m, 2H), 7.02 (dd, J = 7.4, 2.3 Hz, 1H), 6.94-6.80 (m, 2H), 2.83 (d, J = 13.9 Hz, 2H), 1.42 (dt, J = 22.6, 4.5 Hz, 3H), 1.10 (t, J = 6.9 Hz, 1H), 0.46-0.41 (m, 1H), 0.15 (d, J = 4.4 Hz, 1H).

### Example 63

### N-(5-(5-chloropyrazolo[1,5-a]pyridin-3-yl)pyridin-2-yl)-3-(6-(cyclopropanesulfonamido)pyrimid in-4-yl)bicyclo[3.1.0]hexane-3-carboxamide

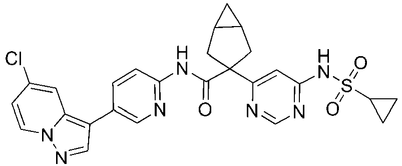

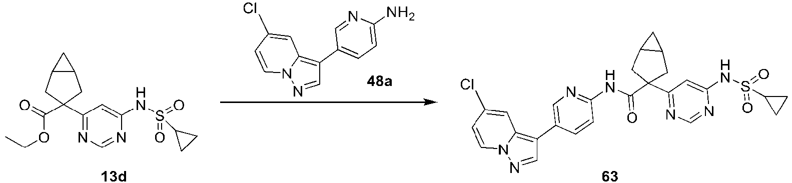

Trimethylaluminum (2.0 M toluene solution) (2 M, 1.02 mL) was added to a solution of 5-(5-chloropyrazolo[1,5-a]pyridin-3-yl)pyridin-2-amine **48a** (153.18 mg, 626.03 µmol) in toluene (2 mL). The mixture was stirred at room temperature for 1 hour. Ethyl 3-(6-(cyclopropanesulfonamido)pyrimidin-4-yl)bicyclo[3.1.0]hexane-3-carboxylate 13d (200 mg, 569.12 µmol) was added, and the mixture was heated to 90°C and the reaction was performed for 18 hours. The mixture was cooled to room temperature, and a small amount of methanol (1 mL) was added to quench the reaction. The solvent was directly evaporated. Dilute hydrochloric acid (1 mL, 1 M) and dichloromethane (5 mL) were added to the residue, and the mixture was filtered. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(5-(5-chloropyrazolo[1,5-a]pyridin-3-yl)pyridin-2-yl)-3-(6-(cyclopropanesulfonamido)pyrimid in-4-yl)bicyclo[3.1.0]hexane-3-carboxamide 63 (3 mg) with a yield of 0.96%.

MS m/z (ESI): 550.1 [M+1]
¹H NMR (400 MHz, DMSO-*d*₆) δ 10.09 (s, 1H), 8.81-8.74 (m, 2H), 8.64 (d, J = 2.5 Hz, 1H), 8.49 (s, 1H), 8.15 (dd, J = 8.7, 2.5 Hz, 1H), 8.08 (dd, J = 5.5, 3.2 Hz, 2H), 7.05-6.91 (m, 2H), 3.08 (s, 1H), 2.82 (d, J = 13.7 Hz, 2H), 2.45-2.37 (m, 2H), 1.41 (dt, J = 7.9, 3.6 Hz, 2H), 1.11-0.99 (m, 4H), 0.45 (d, J = 6.4 Hz, 1H), 0.15 (d, J = 4.5 Hz, 1H).

### Example 64

### 3-(4-((difluoromethyl)sulfonamido)pyridin-2-yl)-N-(5-(6-ethoxypyrazin-2-yl)pyridin-2-yl)bicycl o[3.1.0]hexane-3-carboxamide

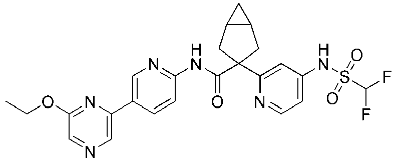

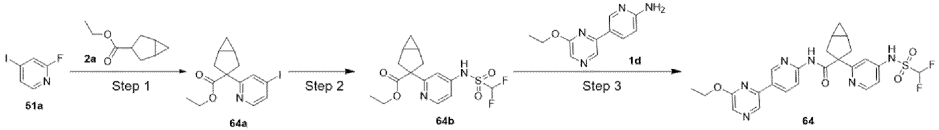

### Step 1

### ethyl 3-(4-iodopyridin-2-yl)bicyclo[3.1.0]hexane-3-carboxylate

2-Fluoro-4-iodopyridine **51a** (867.62 mg, 3.89 mmol) and ethyl bicyclo[3.1.0]hexane-3-carboxylate **2a** (500 mg, 3.24 mmol) were added to tetrahydrofuran (8 mL). The mixture was cooled to -30°C, and lithium hexamethyldisilazide (1.2 M tetrahydrofuran solution) (1 M, 9.73 mL) was added. The mixture was heated to room temperature and stirred for 18 hours. Saturated ammonium chloride solution was added to quench the reaction, and water (20 mL) was added. The mixture was extracted with ethyl acetate (20 mL × 3). The combined organic phase was washed with saturated sodium chloride solution, dried over anhydrous sodium sulfate, filtered, and concentrated under reduced pressure. The residue was purified by silica gel column chromatography (eluent: System A) to obtain ethyl 3-(4-iodopyridin-2-yl)bicyclo[3.1.0]hexane-3-carboxylate **64a** (600 mg) with a yield of 51.81%.

MS m/z (ESI): 357.1 [M+1]

### Step 2

### ethyl 3-(4-((difluoromethyl)sulfonamido)pyridin-2-yl)bicyclo[3.1.0]hexane-3-carboxylate

Ethyl 3-(4-iodopyridin-2-yl)bicyclo[3.1.0]hexane-3-carboxylate **64a** (300 mg, 967.16 µmol), difluoromethanesulfonamide (507.19 mg, 3.87 mmol), cesium carbonate (630.24 mg, 1.93 mmol), di-tert-butyl-[2-(2,4,6-triisopropylphenyl)phenyl]phosphine (41.07 mg, 96.72 µmol), and allylpalladium chloride (17.69 mg, 48.36 µmol) were sequentially added to 1,4-dioxane (3 mL). The mixture was subjected to argon exchange 3 times and stirred at 100°C for 2 hours. Water (20 mL) was added, and the mixture was extracted with ethyl acetate (20 mL × 3). The combined organic phase was washed with saturated sodium chloride solution, dried over anhydrous sodium sulfate, filtered, and concentrated under reduced pressure. The residue was purified by silica gel column chromatography (eluent: System A) to obtain ethyl 3-(4-((difluoromethyl)sulfonamido)pyridin-2-yl)bicyclo[3.1.0]hexane-3-carboxylate **64b** (50 mg, 138.74 µmol) with a yield of 14.35%.

MS m/z (ESI): 361.0 [M+1]

### Step 3

### 3-(4-((difluoromethyl)sulfonamido)pyridin-2-yl)-N-(5-(6-ethoxypyrazin-2-yl)pyridin-2-yl)bicycl o[3.1.0]hexane-3-carboxamide

Trimethylaluminum (2.0 M toluene solution) (2 M, 499.48 µL) was added to a solution of 5-(6-ethoxypyrazin-2-yl)pyridin-2-amine **1d** (72.00 mg, 332.99 µmol) in toluene (2 mL). The mixture was stirred at room temperature for 1 hour. Subsequently, ethyl 3-(4-((difluoromethyl)sulfonamido)pyridin-2-yl)bicyclo[3.1.0]hexane-3-carboxylate **64b** (100 mg, 277.49 µmol) was added, and the mixture was heated to 90°C and stirred for 1.5 hours. The mixture was cooled to room temperature, and a small amount of methanol was added to quench the reaction. The solvent was directly evaporated to obtain a crude product. Dilute hydrochloric acid (1 mL, 1 M) and dichloromethane (5 mL) were added, and the mixture was filtered. The filtrate was concentrated under reduced pressure, and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(4-((difluoromethyl)sulfonamido)pyridin-2-yl)-N-(5-(6-ethoxypyrazin-2-yl)pyridin-2-yl)bicycl o[3.1.0]hexane-3-carboxamide **64** (13 mg) with a yield of 6.92%.

MS m/z (ESI): 531.1 [M+1]
¹H NMR (400 MHz, DMSO-*d*₆) δ 10.60 (s, 1H), 8.97 (d, J = 2.4 Hz, 1H), 8.75 (s, 1H), 8.41 (dd, J = 8.8, 2.5 Hz, 1H), 8.16 (s, 1H), 8.01 (d, J = 8.8 Hz, 1H), 7.93 (d, J = 7.1 Hz, 1H), 7.09 (dd, J = 6.9, 2.3 Hz, 1H), 7.00 (d, J = 2.3 Hz, 1H), 6.56 (t, J = 53.4 Hz, 1H), 4.38 (q, J = 7.0 Hz, 2H), 3.03 (d, J = 13.6 Hz, 2H), 2.20 (d, J = 13.3 Hz, 2H), 1.40-1.25 (m, 5H), 0.35 (d, J = 7.1 Hz, 1H), -0.01 (d, J = 4.5 Hz, 1H).

### Example 65

### 3-(6-(cyclopropanesulfonamido)pyrazin-2-yl)-N-(5-(6-ethoxypyrazin-2-yl)pyridin-2-yl)bicyclo[3 .1.0]hexane-3-carboxamide

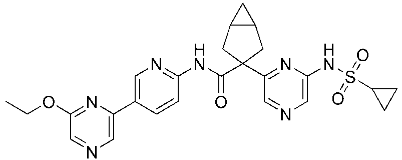

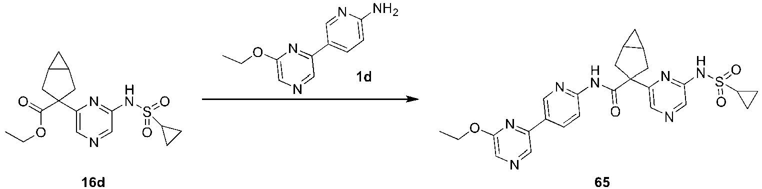

5-(6-Ethoxypyrazin-2-yl)pyridin-2-amine **1d** (59.07 mg, 273.18 µmol) and trimethylaluminum (2.0 M toluene solution) (2 M, 409.77 µL) were dissolved in toluene (2 mL). The mixture was stirred at room temperature for 1 hour. Subsequently, ethyl 3-(6-(cyclopropanesulfonamido)pyrazin-2-yl)bicyclo[3.1.0]hexane-3-carboxylate 16d (80 mg, 227.65 µmol) was added, and the mixture was heated to 90°C and stirred for 1.5 hours. The mixture was cooled to room temperature, and a small amount of methanol was added to quench the reaction. The solvent was directly evaporated. Dilute hydrochloric acid (1 mL, 1 M) and dichloromethane (5 mL) were added, and the mixture was filtered. The filtrate was concentrated under reduced pressure, and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyrazin-2-yl)-N-(5-(6-ethoxypyrazin-2-yl)pyridin-2-yl)bicyclo[3 .1.0]hexane-3-carboxamide (8 mg) with a yield of 5.31%.

MS m/z (ESI): 522.1 [M+1]
¹H NMR (400 MHz, DMSO-d6) δ 10.85 (s, 1H), 10.05 (s, 1H), 8.85 (d, J = 2.4 Hz, 1H), 8.67 (s, 1H), 8.32 (dd, J = 8.8, 2.5 Hz, 1H), 8.19 (s, 1H), 8.08 (s, 1H), 8.01-7.94 (m, 2H), 4.32 (t, J = 7.0 Hz, 2H), 2.94 (s, 1H), 2.75 (d, J = 13.8 Hz, 2H), 2.32-2.24 (m, 2H), 1.25 (dt, J = 14.1, 7.6 Hz, 5H), 0.91 (dd, J = 4.7, 2.5 Hz, 2H), 0.75 (dd, J = 7.9, 2.7 Hz, 2H), 0.30 (td, J = 8.0, 5.3 Hz, 1H), -0.01 (d, J = 4.4 Hz, 1H).

### Example 66

### N-(4-(6-ethoxypyrazin-2-yl)phenyl)-3-(6-(methylsulfonamido)pyrimidin-4-yl)bicyclo[3.1.0]hexa ne-3-carboxamide

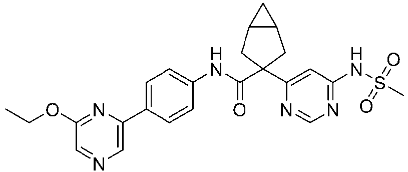

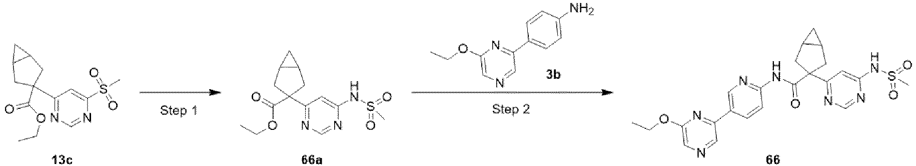

### Step 1

### ethyl 3-(6-(methylsulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (200 mg, 679.42 µmol), methanesulfonamide (129.25 mg, 1.36 mmol), and cesium carbonate (442.74 mg, 1.36 mmol) were dissolved in N-methylpyrrolidone (1 mL). The mixture was heated to 90°C and stirred for 3 hours. The solvent was removed by concentration under reduced pressure, and the residue was purified by silica gel column chromatography (eluent: System A) to obtain ethyl 3-(6-(methylsulfonamido)pyrimidin-4-yl)bicyclo[3.1.0]hexane-3-carboxylate 66a (200 mg) with a yield of 90.47%.

MS m/z (ESI): 326.0 [M+1]

### Step 2

### N-(4-(6-ethoxypyrazin-2-yl)phenyl)-3-(6-(methylsulfonamido)pyrimidin-4-yl)bicyclo[3.1.0]hexa ne-3-carboxamide

Trimethylaluminum (2.0 M toluene solution) (2 M, 442.56 µL) was added to a solution of 4-(6-ethoxypyrazin-2-yl)aniline **3b** (63.51 mg, 295.04 µmol) in toluene (1 mL). The mixture was stirred at room temperature for 1 hour. Subsequently, ethyl 3-(6-(methylsulfonamido)pyrimidin-4-yl)bicyclo[3.1.0]hexane-3-carboxylate **66a** (80 mg, 245.86 µmol) was added, and the mixture was heated to 90°C and stirred for 1.5 hours. The mixture was cooled to room temperature, and a small amount of methanol was added to quench the reaction. The solvent was directly evaporated. Dilute hydrochloric acid (1 mL, 1 M) and dichloromethane (5 mL) were added, and the mixture was filtered and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-(methylsulfonamido)pyrimidin-4-yl)bicyclo[3.1.0]hexa ne-3-carboxamide **66** (9 mg) with a yield of 5.68%.

MS m/z (ESI): 495.1 [M+1]
¹H NMR (400 MHz, DMSO-*d6*) δ 9.63 (s, 1H), 8.65 (d, J=15.9 Hz, 2H), 8.06 (s, 1H), 7.95 (d, J=8.4 Hz, 2H), 7.64 (d, J=8.4 Hz, 2H), 6.75 (s, 1H), 4.35 (q, J=7.0 Hz, 2H), 3.21 (s, 3H), 2.73 (d, J=13.5 Hz, 2H), 2.35-2.26 (m, 2H), 1.28 (t, J=7.0 Hz, 5H), 0.30 (d, J=6.5 Hz, 1H), -0.01 (d, J=4.4 Hz, 1H).

### Example 67

### N-(4-(6-ethoxypyrazin-2-yl)phenyl)-3-(6-((1-methylethyl)sulfonamido)pyrimidin-4-yl)bicyclo[3. 1.0]hexane-3-carboxamide

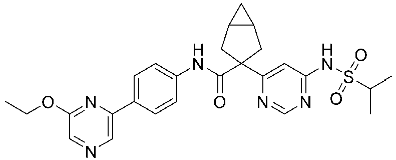

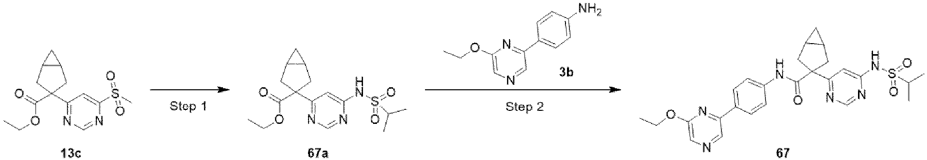

### Step 1

### ethyl 3-(6-((1-methylethyl)sulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (158.15 mg, 509.57 µmol), propane-2-sulfonamide (94.15 mg, 764.35 µmol), and cesium carbonate (332.05 mg, 1.02 mmol) were added to N-methylpyrrolidone (2 mL). The mixture was heated to 90°C and stirred for 3 hours. The solvent was removed by concentration under reduced pressure, and the residue was purified by silica gel column chromatography (eluent: System A) to obtain ethyl 3-(6-((1-methylethyl)sulfonamido)pyrimidin-4-yl)bicyclo[3.1.0]hexane-3-carboxylate **67a** (170 mg) with a yield of 94.39%.

MS m/z (ESI): 354.0 [M+1]

### Step 2

### N-(4-(6-ethoxypyrazin-2-yl)phenyl)-3-(6-((1-methylethyl)sulfonamido)pyrimidin-4-yl)bicyclo[3. 1.0]hexane-3-carboxamide

Trimethylaluminum (1.0 M n-hexane solution) (1 M, 1.02 mL) was added to a solution of 4-(6-ethoxypyrazin-2-yl)aniline **3b** (73.08 mg, 339.52 µmol) in toluene (3 mL). The mixture was stirred at room temperature for 1 hour. Ethyl 3-(6-((1-methylethyl)sulfonamido)pyrimidin-4-yl)bicyclo[3.1.0]hexane-3-carboxylate **67a** (100 mg, 282.94 µmol) was added, and the mixture was heated to 105°C and stirred for 18 hours. The mixture was cooled to room temperature, and a small amount of methanol was added to quench the reaction. The solvent was directly evaporated to dryness by rotation. Dilute hydrochloric acid (1 mL, 1 M) and dichloromethane (5 mL) were added, and the mixture was filtered. The filtrate was concentrated under reduced pressure, and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-((1-methylethyl)sulfonamido)pyrimidin-4-yl)bicyclo[3. 1.0]hexane-3-carboxamide **67** (37 mg) with a yield of 20.13%.

MS m/z (ESI): 523.1 [M+1]
¹H NMR (400 MHz, DMSO-d6) δ 9.76 (s, 1H), 8.75 (s, 2H), 8.18 (s, 1H), 8.07 (d, J=8.8 Hz, 2H), 7.76 (d, J=8.6 Hz, 2H), 6.93 (s, 1H), 4.47 (q, J=7.0 Hz, 2H), 2.93-2.72 (m, 3H), 2.44-2.34 (m, 2H), 1.39 (t, J=7.0 Hz, 5H), 1.24 (d, J=6.7 Hz, 6H), 0.41 (d, J=6.0 Hz, 1H), 0.11 (d, J=4.4 Hz, 1H).

### Example 68

### 2-(6-(cyclopropanesulfonamido)pyrimidin-4-yl)-N-(4-(6-ethoxypyrazin-2-yl)phenyl)octahydrope ntalene-2-carboxamide

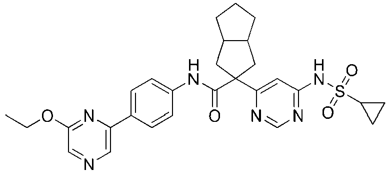

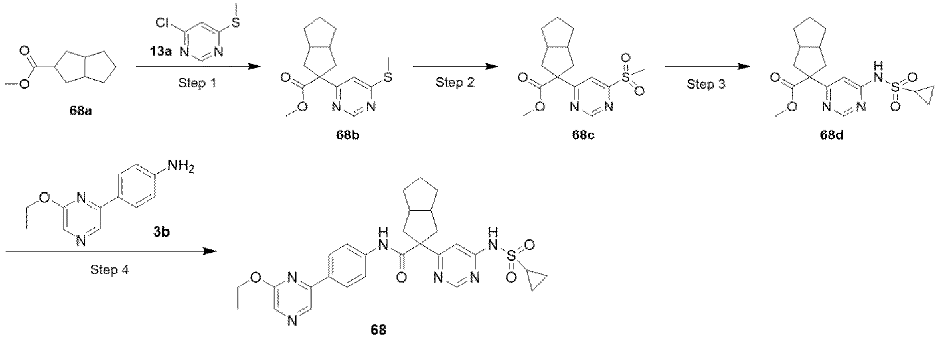

### Step 1

### methyl 2-(6-(methylthio)pyrimidin-4-yl)octahydropentalene-2-carboxylate

4-Chloro-6-(methylthio)pyrimidine **13a** (572.87 mg, 3.57 mmol) and methyl octahydropentalene-2-carboxylate **68a** (500 mg, 2.97 mmol, commercially available) were dissolved in tetrahydrofuran (7 mL). The mixture was cooled to -30°C, and lithium bis(trimethylsilyl)amide (1 M, 8.92 mL) was added. The mixture was heated to room temperature and stirred for 4 hours. Saturated ammonium chloride solution (50 mL) was added to quench the reaction, and the mixture was extracted with ethyl acetate (50 mL × 3). The combined organic phase was washed with saturated sodium chloride solution, dried over anhydrous sodium sulfate, filtered, and evaporated to dryness by rotation. The residue was purified by silica gel column chromatography (eluent: System A) to obtain methyl 2-(6-(methylthio)pyrimidin-4-yl)octahydropentalene-2-carboxylate 68b (630 mg) with a yield of 72.50%.

MS m/z (ESI): 293.0 [M+1]

### Step 2

### methyl 2-(6-(methylsulfonyl)pyrimidin-4-yl)octahydropentalene-2-carboxylate

Methyl 2-(6-(methylthio)pyrimidin-4-yl)octahydropentalene-2-carboxylate **68b** (750 mg, 2.57 mmol) and meta-chloroperoxybenzoic acid (1.04 g, 5.13 mmol, 85% purity) were dissolved in dichloromethane (10 mL) and stirred at room temperature for 4 hours. After the reaction was completed, saturated sodium bicarbonate solution (50 mL) was added, and the mixture was extracted with dichloromethane (50 mL × 3). The combined organic phase was washed with saturated sodium chloride solution, dried over anhydrous sodium sulfate, filtered, and evaporated to dryness by rotation. The residue was methyl 2-(6-(methylsulfonyl)pyrimidin-4-yl)octahydropentalene-2-carboxylate 68c (830 mg) with a yield of 99.75%. The crude product was directly used in the next step.

MS m/z (ESI): 325.0 [M+1]

### Step 3

### methyl 2-(6-(cyclopropanesulfonamido)pyrimidin-4-yl)octahydropentalene-2-carboxylate

Methyl 2-(6-(methylsulfonyl)pyrimidin-4-yl)octahydropentalene-2-carboxylate 68c (830 mg, 2.69 mmol), cyclopropanesulfonamide (423.90 mg, 3.50 mmol), and cesium carbonate (1.75 g, 5.38 mmol) were dissolved in N-methylpyrrolidone (8 mL). The mixture was heated to 90°C with stirring for 2 hours. The reaction mixture was concentrated under reduced pressure. The residue was purified by silica gel column chromatography (eluent: System A) to obtain methyl 2-(6-(cyclopropanesulfonamido)pyrimidin-4-yl)octahydropentalene-2-carboxylate 68d (600 mg) with a yield of 61.00%.

MS m/z (ESI): 366.0 [M+1]

### Step 4

### 2-(6-(cyclopropanesulfonamido)pyrimidin-4-yl)-N-(4-(6-ethoxypyrazin-2-yl)phenyl)octahydrope ntalene-2-carboxamide

Trimethylaluminum (2.0 M toluene solution) (2 M, 492.55 µL) was added to a solution of 4-(6-ethoxypyrazin-2-yl)aniline 3b (70.68 mg, 328.37 µmol) in toluene (3 mL) at room temperature. After stirring for 1 hour, methyl 2-(6-(cyclopropanesulfonamido)pyrimidin-4-yl)octahydropentalene-2-carboxylate **68d** (100 mg, 273.64 µmol) was added, and the mixture was heated to 105°C with stirring for 18 hours. The mixture was cooled to room temperature, and a small amount of methanol was added to quench the reaction. The solvent was directly evaporated to dryness by rotation. Dilute hydrochloric acid (1 mL, 1 M) and dichloromethane (5 mL) were added, and the mixture was filtered. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 2-(6-(cyclopropanesulfonamido)pyrimidin-4-yl)-N-(4-(6-ethoxypyrazin-2-yl)phenyl)octahydrope ntalene-2-carboxamide 68 (11 mg) with a yield of 5.94%.

MS m/z (ESI): 549.1 [M+1]
¹H NMR (400 MHz, DMSO-*d*₆) δ 9.34 (s, 1H), 8.75 (s, 2H), 8.17 (s, 1H), 8.05 (d, J = 8.4 Hz, 2H), 7.74 (d, J = 8.3 Hz, 2H), 7.17 (s, 1H), 4.47 (q, J = 7.2 Hz, 2H), 3.09 (s, 1H), 2.62 (d, J = 10.3 Hz, 3H), 1.87 (t, J = 10.5 Hz, 2H), 1.55 (s, 3H), 1.41 (q, J = 9.7, 6.9 Hz, 6H), 1.14-1.00 (m, 4H).

### Example 69

### N-(4-(6-ethoxypyrazin-2-yl)phenyl)-3-(6-(ethylsulfonamido)pyrimidin-4-yl)bicyclo[3.1.0]hexane -3-carboxamide

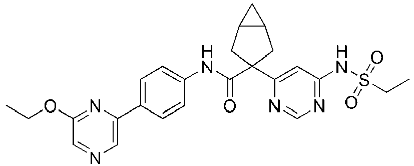

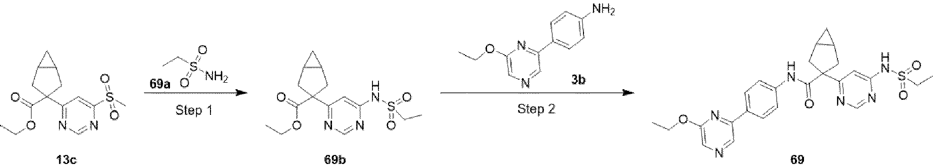

### Step 1

### ethyl 3-(6-(ethylsulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (200 mg, 679.42 µmol), ethylsulfonamide 69a (148.31 mg, 1.36 mmol, commercially available), and cesium carbonate (442.74 mg, 1.36 mmol) were added to N-methylpyrrolidone (2 mL). The mixture was heated to 90°C with stirring for 3 hours. The solvent was removed by concentration under reduced pressure. The residue was purified by silica gel column chromatography (eluent: System A) to obtain ethyl 3-(6-(ethylsulfonamido)pyrimidin-4-yl)bicyclo[3.1.0]hexane-3-carboxylate 69b (200 mg) with a yield of 86.73%.

MS m/z (ESI): 340.0 [M+1]

### Step 2

### N-(4-(6-ethoxypyrazin-2-yl)phenyl)-3-(6-(ethylsulfonamido)pyrimidin-4-yl)bicyclo[3.1.0]hexane -3-carboxamide

Trimethylaluminum (1.0 M n-hexane solution) (1 M, 848.53 µL) was added to a solution of 4-(6-ethoxypyrazin-2-yl)aniline 3b (60.88 mg, 282.84 µmol) in toluene (3 mL) at room temperature. After stirring for 1 hour, ethyl 3-(6-(ethylsulfonamido)pyrimidin-4-yl)bicyclo[3.1.0]hexane-3-carboxylate 69b (80 mg, 235.70 µmol) was added, and the mixture was heated to 105°C with stirring for 18 hours. The mixture was cooled to room temperature, and a small amount of methanol was added to quench the reaction. The solvent was evaporated to dryness by rotation. Dilute hydrochloric acid (1 mL, 1 M) and dichloromethane (5 mL) were added, and the mixture was filtered. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-(ethylsulfonamido)pyrimidin-4-yl)bicyclo[3.1.0]hexane -3-carboxamide 69 (23 mg) with a yield of 15.05%.

MS m/z (ESI): 509.1 [M+1]
¹H NMR (400 MHz, DMSO-*d*₆) δ 9.64 (s, 1H), 8.65 (d, J = 9.5 Hz, 2H), 8.06 (s, 1H), 7.95 (d, J = 8.5 Hz, 2H), 7.64 (d, J = 8.4 Hz, 2H), 6.77 (s, 1H), 4.35 (q, J = 7.0 Hz, 2H), 3.27 (m, 2H), 2.72 (d, J = 13.5 Hz, 2H), 2.29 (dd, J = 13.2, 3.3 Hz, 2H), 1.28 (t, J = 7.0 Hz, 5H), 1.07 (t, J = 7.3 Hz, 3H), 0.30 (d, J = 6.2 Hz, 1H), -0.01 (d, J = 4.4 Hz, 1H).

### Example 70

### N-(4-(6-ethoxypyrazin-2-yl)phenyl)-3-(6-((1-methylcyclopropane)-1-sulfonamido)pyrimidin-4-yl )bicyclo[3.1.0]hexane-3-carboxamide

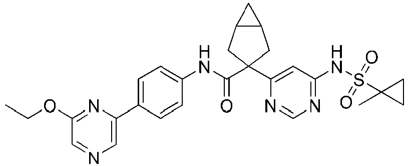

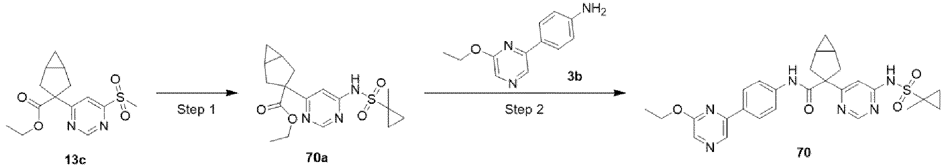

### Step 1

### ethyl 3-(6-((1-methylcyclopropane)-1-sulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (158.15 mg, 509.57 µmol), 1-methylcyclopropanesulfonamide (137.77 mg, 1.02 mmol), and cesium carbonate (332.05 mg, 1.02 mmol) were added to N-methylpyrrolidone (2 mL). The mixture was heated to 90°C and stirred for 3 hours. The mixture was concentrated under reduced pressure, and the residue was purified by silica gel column chromatography (eluent: System A) to obtain ethyl 3-(6-((1-methylcyclopropane)-1-sulfonamido)pyrimidin-4-yl)bicyclo[3.1.0]hexane-3-carboxylate 70a (170 mg) with a yield of 91.29%.

MS m/z (ESI): 366.0 [M+1]

### Step 2

### N-(4-(6-ethoxypyrazin-2-yl)phenyl)-3-(6-((1-methylcyclopropane)-1-sulfonamido)pyrimidin-4-yl )bicyclo[3.1.0]hexane-3-carboxamide

Trimethylaluminum (1.0 M n-hexane solution) (1 M, 848.53 µL) was added to a solution of 4-(6-ethoxypyrazin-2-yl)aniline 3b (60.88 mg, 282.84 µmol) in toluene (2 mL). The mixture was stirred at room temperature for 1 hour. Ethyl 3-(6-((1-methylcyclopropane)-1-sulfonamido)pyrimidin-4-yl)bicyclo[3.1.0]hexane-3-carboxylate 70a (86.14 mg, 235.70 µmol) was added, and the mixture was heated to 105°C and stirred for 18 hours. The reaction mixture was cooled to room temperature, and a small amount of methanol was added to quench the reaction. The solvent was evaporated to dryness by rotation. Dilute hydrochloric acid (1 mL, 1 M) and dichloromethane (5 mL) were added, and the mixture was filtered. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-((1-methylcyclopropane)-1-sulfonamido)pyrimidin-4-yl )bicyclo[3.1.0]hexane-3-carboxamide 70 (12 mg) with a yield of 7.53%.

MS m/z (ESI): 535.1 [M+1]
¹H NMR (400 MHz, DMSO-*d*₆) δ 9.65 (s, 1H), 8.64 (s, 2H), 8.06 (s, 1H), 7.96 (d, J=8.4 Hz, 2H), 7.65 (d, J=8.4 Hz, 2H), 6.96 (s, 1H), 4.36 (q, J=7.0 Hz, 2H), 2.73 (d, J=13.5 Hz, 2H), 2.30 (d, J=13.2 Hz, 2H), 1.35-1.15 (m, 10H), 0.73 (s, 2H), 0.31 (d, J=6.8 Hz, 1H), 0.04-0.07 (m, 1H).

### Example 71

### N-(4-(6-ethoxypyrazin-2-yl)phenyl)-3-(6-((trifluoromethyl)sulfonamido)pyrimidin-4-yl)bicyclo[ 3.1.0]hexane-3-carboxamide

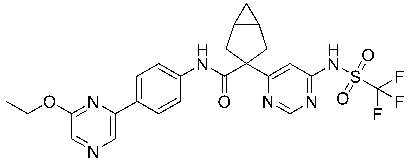

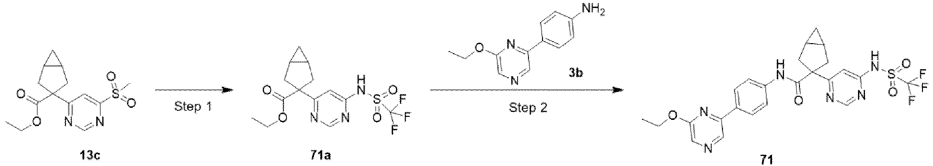

### Step 1

### ethyl 3-(6-((trifluoromethyl)sulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (150 mg, 483.30 µmol), trifluoromethanesulfonamide (144.11 mg, 966.59 µmol), and cesium carbonate (314.94 mg, 966.59 µmol) were added to N-methylpyrrolidone (2 mL). The mixture was heated to 120°C and stirred for 2 hours. The solvent was removed under reduced pressure, and the residue was purified by silica gel column chromatography (eluent: System A) to obtain ethyl 3-(6-((trifluoromethyl)sulfonamido)pyrimidin-4-yl)bicyclo[3.1.0]hexane-3-carboxylate 71a (80 mg) with a yield of 43.63%.

MS m/z (ESI): 380.0 [M+1]

### Step 2

### N-(4-(6-ethoxypyrazin-2-yl)phenyl)-3-(6-((trifluoromethyl)sulfonamido)pyrimidin-4-yl)bicyclo[ 3.1.0]hexane-3-carboxamide

Trimethylaluminum (1.0 M n-hexane solution) (1 M, 948.98 µL) was added to a solution of 4-(6-ethoxypyrazin-2-yl)aniline 3b (68.09 mg, 316.33 µmol) in toluene (3 mL). The mixture was stirred at room temperature for 1 hour. Ethyl 3-(6-((trifluoromethyl)sulfonamido)pyrimidin-4-yl)bicyclo[3.1.0]hexane-3-carboxylate **71a** (100 mg, 263.61 µmol) was added, and the mixture was heated to 105°C and stirred for 18 hours. A small amount of methanol was added to quench the reaction, and the solvent was evaporated to dryness by rotation. Dilute hydrochloric acid (1 mL, 1 M) and dichloromethane (5 mL) were added, and the mixture was filtered. The filtrate was concentrated under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-((trifluoromethyl)sulfonamido)pyrimidin-4-yl)bicyclo[ 3.1.0]hexane-3-carboxamide (35 mg) with a yield of 19.44%.

MS m/z (ESI): 549.1 [M+1]
¹H NMR (400 MHz, DMSO-*d*₆) δ 9.62 (s, 1H), 8.62 (s, 2H), 8.03 (s, 1H), 7.93 (d, J = 8.5 Hz, 2H), 7.57 (d, J = 8.6 Hz, 2H), 7.20 (s, 1H), 4.33 (q, J = 7.0 Hz, 2H), 2.71 (d, J = 13.6 Hz, 2H), 2.26 2.16 (m, 2H), 1.27 (dt, J = 14.0, 5.5 Hz, 5H), 0.32 (d, J = 5.6 Hz, 1H), -0.01 (d, J = 4.6 Hz, 1H).

### Example 72

### 5-(6-(cyclopropanesulfonamido)pyrimidin-4-yl)-N-(4-(6-ethoxypyrazin-2-yl)phenyl)octahydrocy clopenta[c]pyrrole-5-carboxamide

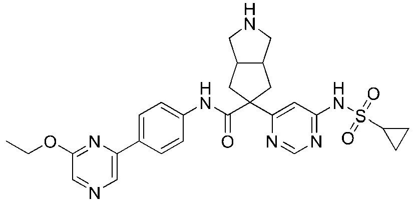

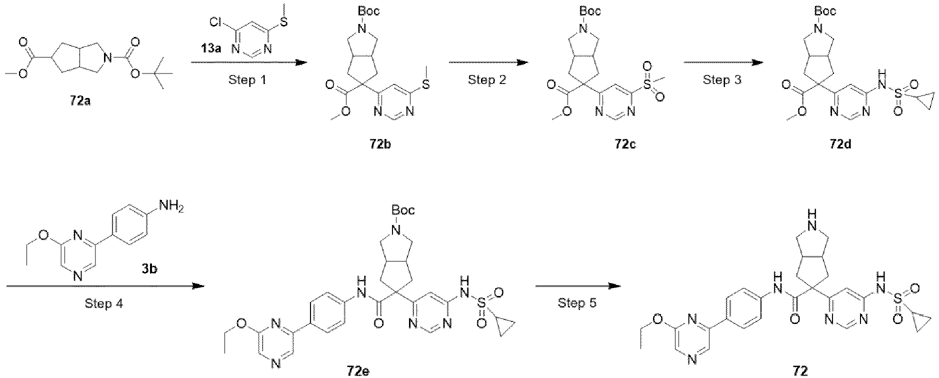

### Step 1

### 2-(tert-butyl) 5-methyl 5-(6-(methylthio)pyrimidin-4-yl)hexahydrocyclopenta[c]pyrrole-2,5(1H)-dicarboxylate

4-Chloro-6-(methylthio)pyrimidine **13a** (524.81 mg, 3.27 mmol) and 2-(tert-butyl) 5-methyl hexahydrocyclopenta[c]pyrrole-2,5(1H)-dicarboxylate 72a (800 mg, 2.97 mmol) were dissolved in tetrahydrofuran (4.77 mL). The mixture was cooled to -30°C, and lithium bis(trimethylsilyl)amide (1 M, 8.91 mL) was added. The mixture was heated to room temperature and stirred for 4 hours. Saturated ammonium chloride solution (50 mL) was added to quench the reaction, and the mixture was extracted with ethyl acetate (50 mL × 3). The combined organic phase was washed with saturated sodium chloride solution, dried over anhydrous sodium sulfate, filtered, and evaporated to dryness by rotation. The residue was purified by silica gel column chromatography (eluent: System A) to obtain 2-(tert-butyl) 5-methyl 5-(6-(methylthio)pyrimidin-4-yl)hexahydrocyclopenta[c]pyrrole-2,5(1H)-dicarboxylate 72b (360 mg) with a yield of 30.80%.

MS m/z (ESI): 394.0 [M+1]

### Step 2

### 2-(tert-butyl) 5-methyl

### 5-(6-(methylsulfonyl)pyrimidin-4-yl)hexahydrocyclopenta[c]pyrrole-2,5(1H)-dicarboxylate

2-(Tert-butyl) 5-methyl 5-(6-(methylthio)pyrimidin-4-yl)hexahydrocyclopenta[c]pyrrole-2,5(1H)-dicarboxylate 72b (320 mg, 813.22 µmol) and meta-chloroperoxybenzoic acid (330.20 mg, 1.63 mmol, 85% purity) were dissolved in dichloromethane (4 mL). The reaction was performed at room temperature for 2 hours. After the reaction was completed, saturated sodium bicarbonate solution (50 mL) was added, and the mixture was extracted with dichloromethane (50 mL × 3). The combined organic phase was washed with saturated sodium chloride solution, dried over anhydrous sodium sulfate, filtered, and evaporated to dryness by rotation to obtain 2-(tert-butyl) 5-methyl 5-(6-(methylsulfonyl)pyrimidin-4-yl)hexahydrocyclopenta[c]pyrrole-2,5(1H)-dicarboxylate 72c (250 mg) with a yield of 75.07%. The crude product was directly used in the next step.

MS m/z (ESI): 354.0 [M+1-56]

### Step 3

### 2-(tert-butyl) 5-methyl 5-(6-(cyclopropanesulfonamido)pyrimidin-4-yl)hexahydrocyclopenta[c]pyrrole-2,5(1H)-dicarbox ylate

2-(Tert-butyl) 5-methyl 5-(6-(methylsulfonyl)pyrimidin-4-yl)hexahydrocyclopenta[c]pyrrole-2,5(1H)-dicarboxylate 72c (360 mg, 879.12 µmol), cyclopropanesulfonamide (213.03 mg, 1.76 mmol), and cesium carbonate (572.87 mg, 1.76 mmol) were dissolved in N-methylpyrrolidone (1 mL). The mixture was heated to 90°C and stirred for 2 hours. The solvent was removed by concentration under reduced pressure. The residue was purified by silica gel column chromatography (eluent: System A) to obtain 2-(tert-butyl) 5-methyl 5-(6-(cyclopropanesulfonamido)pyrimidin-4-yl)hexahydrocyclopenta[c]pyrrole-2,5(1H)-dicarbox ylate 72d (280 mg) with a yield of 68.27%.

MS m/z (ESI): 467.0 [M+1]

### Step 4

### tert-butyl 5-(6-(cyclopropanesulfonamido)pyrimidin-4-yl)-5-((4-(6-ethoxypyrazin-2-yl)phenyl)carbamoyl) hexahydrocyclopenta[c]pyrrole-2(1H)-carboxylate

Trimethylaluminum (1.0 M n-hexane solution) (1 M, 1.39 mL) was added to a solution of 4-(6-ethoxypyrazin-2-yl)aniline 3b (107.96 mg, 501.55 µmol) in toluene (3 mL). The mixture was stirred at room temperature for 1 hour. 2-(tert-butyl) 5-methyl 5-(6-(cyclopropanesulfonamido)pyrimidin-4-yl)hexahydrocyclopenta[c]pyrrole-2,5(1H)-dicarbox ylate 72d (180 mg, 385.81 µmol) was added, and the mixture was heated to 105°C and stirred for 18 hours. The mixture was cooled to room temperature, and a small amount of methanol was added to quench the reaction. The solvent was evaporated to dryness by rotation. Dilute hydrochloric acid (1 mL, 1 M) and dichloromethane (5 mL) were added, and the mixture was filtered. The filtrate was concentrated under reduced pressure, and the residue was purified by silica gel column chromatography (eluent: System A) to obtain tert-butyl 5-(6-(cyclopropanesulfonamido)pyrimidin-4-yl)-5-((4-(6-ethoxypyrazin-2-yl)phenyl)carbamoyl) hexahydrocyclopenta[c]pyrrole-2(1H)-carboxylate 72e (180 mg) with a yield of 71.80%.

MS m/z (ESI): 550.1 [M+1-100]

### Step 5

### 5-(6-(cyclopropanesulfonamido)pyrimidin-4-yl)-N-(4-(6-ethoxypyrazin-2-yl)phenyl)octahydrocy clopenta[c]pyrrole-5-carboxamide

Tert-butyl 5-(6-(cyclopropanesulfonamido)pyrimidin-4-yl)-5-(((4-(6-ethoxypyrazin-2-yl)phenyl)carbamoyl) hexahydrocyclopenta[c]pyrrole-2(1H)-carboxylate 72e (180 mg, 277.03 µmol) was dissolved in a solution of hydrogen chloride in 1,4-dioxane (10 mL, 4 M) and stirred at room temperature for 4 hours. The mixture was concentrated under reduced pressure, the solvent was evaporated to dryness by rotation and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 5-(6-(cyclopropanesulfonamido)pyrimidin-4-yl)-N-(4-(6-ethoxypyrazin-2-yl)phenyl)octahydrocy clopenta[c]pyrrole-5-carboxamide 72 (150 mg) with a yield of 79.96%.

MS m/z (ESI): 550.1 [M+1]
1H NMR (400 MHz, DMSO-d6) δ 9.57 (s, 1H), 8.97 (s, 2H), 8.75 (s, 1H), 8.18 (s, 1H), 8.07 (d, J=8.4 Hz, 2H), 7.76 (d, J=8.4 Hz, 2H), 7.30-6.98 (m, 2H), 4.47 (d, J=7.1 Hz, 2H), 3.18 (d, J=10.3 Hz, 5H), 2.86 (s, 2H), 2.68 (s, 2H), 2.25 (s, 2H), 1.39 (t, J=7.0 Hz, 3H), 1.06 (dd, J=23.4, 6.3 Hz, 4H).

### Example 73

### N-(4-(6-ethoxypyrazin-2-yl)phenyl)-3-(6-((2-methoxyethyl)sulfonamido)pyrimidin-4-yl)bicyclo[ 3.1.0]hexane-3-carboxamide

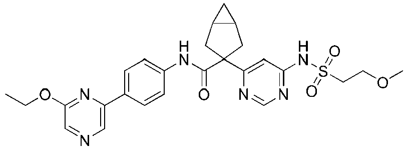

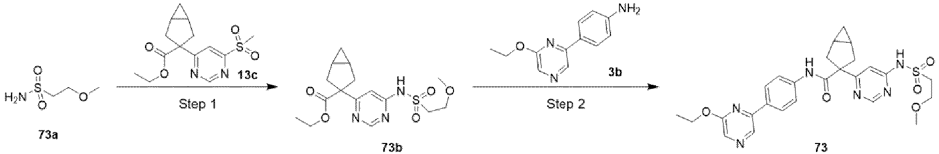

### Step 1

### ethyl3-(6-((2-methoxyethyl)sulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (150 mg, 483.30 µmol), 2-methoxyethanesulfonamide 73a (134.52 mg, 966.59 µmol, commercially available), and cesium carbonate (314.94 mg, 966.59 µmol) were added to N-methylpyrrolidone (2 mL). The mixture was heated to 120°C with stirring for 2 hours. The solvent was removed under reduced pressure, and the residue was purified by silica gel column chromatography (eluent: System A) to obtain ethyl 3-(6-((2-methoxyethyl)sulfonamido)pyrimidin-4-yl)bicyclo[3.1.0]hexane-3-carboxylate 73b (130 mg) with a yield of 72.81%.

MS m/z (ESI): 370.0 [M+1]

### Step 2

### N-(4-(6-ethoxypyrazin-2-yl)phenyl)-3-(6-((2-methoxyethyl)sulfonamido)pyrimidin-4-yl)bicyclo[ 3.1.0]hexane-3-carboxamide

Trimethylaluminum (1.0 M n-hexane solution) (1 M, 848.53 µL) was added to a solution of 4-(6-ethoxypyrazin-2-yl)aniline 3b (60.88 mg, 282.84 µmol) in toluene (2 mL). After stirring at room temperature for 1 hour, ethyl 3-(6-((2-methoxyethyl)sulfonamido)pyrimidin-4-yl)bicyclo[3.1.0]hexane-3-carboxylate 73b (87.08 mg, 235.70 µmol) was added, and the mixture was heated to 105°C and stirred for 18 hours. The reaction mixture was cooled to room temperature, and a small amount of methanol was added to quench the reaction. The solvent was directly evaporated to dryness by rotation. Dilute hydrochloric acid (1 mL, 1 M) and dichloromethane (5 mL) were added, and the mixture was filtered. The filtrate was concentrated under reduced pressure, and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain N-(4-(6-ethoxypyrazin-2-yl)phenyl)-3-(6-((2-methoxyethyl)sulfonamido)pyrimidin-4-yl)bicyclo[ 3.1.0]hexane-3-carboxamide (23 mg) with a yield of 14.20%.

MS m/z (ESI): 539.1 [M+1]
¹H NMR (400 MHz, DMSO-*d*₆) δ 9.63 (s, 1H), 8.65 (d, J=8.6 Hz, 2H), 8.06 (s, 1H), 7.95 (d, J=8.4 Hz, 2H), 7.64 (d, J=8.4 Hz, 2H), 6.77 (s, 1H), 4.35 (q, J=7.0 Hz, 2H), 3.70-3.53 (m, 4H), 3.01 (s, 3H), 2.73 (d, J=13.5 Hz, 2H), 2.29 (d, J=13.3 Hz, 2H), 1.28 (t, J=7.1 Hz, 5H), 0.30 (d, J=6.8 Hz, 1H), -0.01 (d, J=4.2 Hz, 1H).

### Example 74

### 5-(6-(cyclopropanesulfonamido)pyrimidin-4-yl)-N-(4-(6-ethoxypyrazin-2-yl)phenyl)-2-methyloc tahydrocyclopenta[c]pyrrole-5-carboxamide

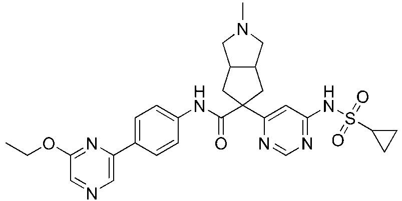

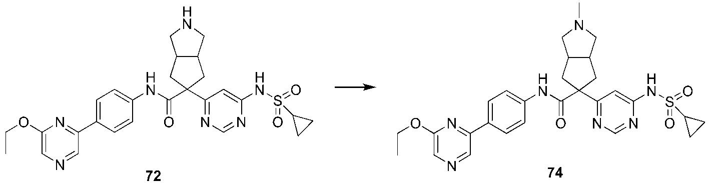

5-(6-(cyclopropanesulfonamido)pyrimidin-4-yl)-N-(4-(6-ethoxypyrazin-2-yl)phenyl)oct ahydrocyclopenta[c]pyrrole-5-carboxamide 72 (90 mg, 163.74 µmol), formic acid (18.83 mg, 409.36 µmol), paraformaldehyde (14.74 mg, 491.23 µmol), and sodium hydroxide (6.55 mg, 163.74 µmol) were dissolved in tetrahydrofuran (5 mL). The mixture was heated to 75°C with stirring for 3 hours. The solvent was evaporated to dryness by rotation, and the residue was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 5-(6-(cyclopropanesulfonamido)pyrimidin-4-yl)-N-(4-(6-ethoxypyrazin-2-yl)phenyl)-2-methyloc tahydrocyclopenta[c]pyrrole-5-carboxamide 74 (1.29 mg) with a yield of 1.15%.

MS m/z (ESI): 564.2 [M+1]
1H NMR (400 MHz, DMSO-d6) δ 9.89 (s, 1H), 9.57 (d, J=40.9 Hz, 1H), 8.78 (d, J=22.1 Hz, 2H), 8.18 (s, 1H), 8.08 (d, J=8.5 Hz, 2H), 7.76 (d, J=8.4 Hz, 2H), 7.09 (d, J=14.2 Hz, 1H), 4.47 (d, J=7.1 Hz, 2H), 4.13-3.94 (m, 1H), 3.74 (s, 1H), 3.48 (d, J=10.8 Hz, 1H), 3.10 (d, J=14.4 Hz, 2H), 2.88 (d, J=43.6 Hz, 7H), 2.21 (s, 2H), 1.38 (d, J=7.1 Hz, 3H), 1.12-0.96 (m, 4H).

### Example 75

### 3-(6-((cyanomethyl)sulfonamido)pyrimidin-4-yl)-N-(4-(6-ethoxypyrazin-2-yl)phenyl)bicyclo[3.1 .0]hexane-3-carboxamide

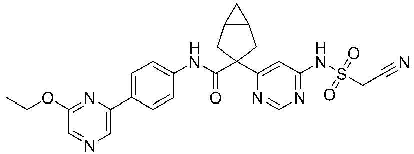

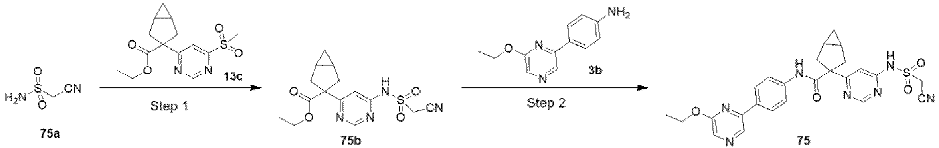

### Step 1

### ethyl 3-(6-((cyanomethyl)sulfonamido)pyrimidin-4-yl)bicyclo[3.1.0]hexane-3-carboxylate

Ethyl 3-(6-(methylsulfonyl)pyrimidin-4-yl)bicyclo[3.1.0]hexane-3-carboxylate 13c (200 mg, 644.40 µmol), cyanomethanesulfonamide 75a (154.82 mg, 1.29 mmol, commercially available), and cesium carbonate (419.91 mg, 1.29 mmol) were added to N-methylpyrrolidone (2 mL). The mixture was heated to 90°C and stirred for 3 hours. The solvent was removed by concentration under reduced pressure. The residue was purified by silica gel column chromatography (eluent: System A) to obtain ethyl 3-(6-((cyanomethyl)sulfonamido)pyrimidin-4-yl)bicyclo[3.1.0]hexane-3-carboxylate 75b (150 mg) with a yield of 66.43%.

MS m/z (ESI): 351.0 [M+1]

### Step 2

### 3-(6-((cyanomethyl)sulfonamido)pyrimidin-4-yl)-N-(4-(6-ethoxypyrazin-2-yl)phenyl)bicyclo[3.1 .0]hexane-3-carboxamide

Trimethylaluminum (1.0 M n-hexane solution) (1 M, 848.53 µL) was added to a solution of 4-(6-ethoxypyrazin-2-yl)aniline 3b (60.88 mg, 282.84 µmol) in toluene (2 mL). The mixture was stirred at room temperature for 1 hour. Ethyl 3-(6-((cyanomethyl)sulfonamido)pyrimidin-4-yl)bicyclo[3.1.0]hexane-3-carboxylate 75b (82.59 mg, 235.70 µmol) was added, and the mixture was heated to 105°C and stirred for 18 hours. The mixture was cooled to room temperature, and a small amount of methanol was added to quench the reaction. The solvent was directly evaporated to dryness by rotation to obtain a crude product. Dilute hydrochloric acid (1 mL, 1 M) and dichloromethane (5 mL) were added. The mixture was filtered, and the filtrate was concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-((cyanomethyl)sulfonamido)pyrimidin-4-yl)-N-(4-(6-ethoxypyrazin-2-yl)phenyl)bicyclo[3.1 .0]hexane-3-carboxamide 75 (1.07 mg) with a yield of 0.85%.

MS m/z (ESI): 520.1 [M+1]
1H NMR (400 MHz, DMSO-d6) δ 9.74 (s, 1H), 8.75 (s, 1H), 8.67 (s, 1H), 8.18 (s, 1H), 8.07 (d, J = 8.5 Hz, 2H), 7.73 (d, J = 8.6 Hz, 2H), 7.10 (s, 1H), 4.86 (s, 2H), 4.47 (q, J = 7.0 Hz, 2H), 2.84 (d, J = 13.6 Hz, 2H), 2.41-2.31 (m, 2H), 1.40 (q, J = 7.2 Hz, 5H), 0.44 (d, J = 6.5 Hz, 1H), 0.13 (d, J = 4.4 Hz, 1H).

### Example 76

### N-(4-(6-ethoxypyrazin-2-yl)phenyl)-3-(6-((1-fluorocyclopropane)-1-sulfonamido)pyrimidin-4-yl) bicyclo[3.1.0]hexane-3-carboxamide

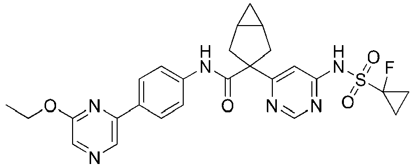

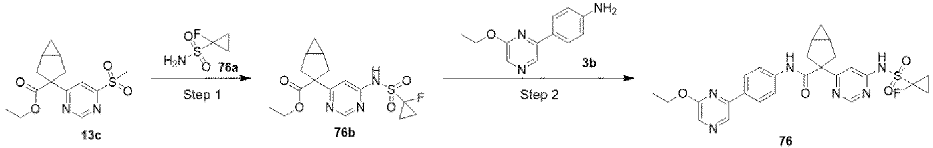

### Step 1

### ethyl 3-(6-((1-fluorocyclopropane)-1-sulfonamido)pyrimidin-4-yl)bicyclo [3.1.0]hexane-3-carboxylate

Ethyl 3-(6-(methylsulfonyl)pyrimidin-4-yl)bicyclo[3.1.0]hexane-3-carboxylate 13c (170 mg, 547.74 µmol), 1-fluorocyclopropanesulfonamide 76a (76.22 mg, 547.74 µmol), and cesium carbonate (356.93 mg, 1.10 mmol) were dissolved in N-methylpyrrolidone (1.5 mL). The mixture was heated to 90°C and stirred for 1 hour. The solvent was evaporated to dryness by rotation using an oil pump. The residue was purified by silica gel column chromatography (eluent: System A) to obtain ethyl 3-(6-((1-fluorocyclopropane)-1-sulfonamido)pyrimidin-4-yl)bicyclo[3.1.0]hexane-3-carboxylate 76b (150 mg) with a yield of 74.13%.

MS m/z (ESI): 370.0 [M+1]

### Step 2

### N-(4-(6-ethoxypyrazin-2-yl)phenyl)-3-(6-((1-fluorocyclopropane)-1-sulfonamido)pyrimidin-4-yl) bicyclo[3.1.0]hexane-3-carboxamide

4-(6-Ethoxypyrazin-2-yl)aniline 3b (60.88 mg, 282.84 µmol) and trimethylaluminum (1.0 M n-hexane solution) (1 M, 848.53 µL) were added to toluene (2 mL). The mixture was stirred at room temperature for 1 hour. Ethyl 3-(6-((1-fluorocyclopropane)-1-sulfonamido)pyrimidin-4-yl)bicyclo[3.1.0]hexane-3-carboxylate 76b (87.07 mg, 235.70 µmol) was added, and the mixture was heated to 105°C and stirred for 18 hours. A small amount of methanol was added to quench the reaction, and the solvent was directly evaporated to dryness by rotation to obtain a crude product. Dilute hydrochloric acid was added, and the mixture was stirred for 10 minutes. The mixture was concentrated under reduced pressure. The residue was dissolved in methanol and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(4-(6-ethoxypyrazin-2-yl)phenyl)-3-(6-((1-fluorocyclopropane)-1-sulfonamido)pyrimidin-4-yl) bicyclo[3.1.0]hexane-3-carboxamide 76 (6 mg) with a yield of 3.62%.

MS m/z (ESI): 539.1 [M+1]
1H NMR (400 MHz, DMSO-d6) δ 9.76 (s, 1H), 8.75 (s, 1H), 8.66 (s, 1H), 8.18 (s, 1H), 8.07 (d, J=8.4 Hz, 2H), 7.74 (d, J=8.4 Hz, 2H), 7.22 (s, 1H), 4.47 (q, J=7.0 Hz, 2H), 2.83 (d, J=13.5 Hz, 2H), 2.38 (d, J=13.4 Hz, 2H), 1.51-1.36 (m, 9H), 0.43 (d, J=6.6 Hz, 1H), 0.12 (t, J=4.4 Hz, 1H).

### Example 77

### 3-(6-(cyclopropanesulfonamido)-2-methylpyrimidin-4-yl)-N-(4-(6-ethoxypyrazin-2-yl)phenyl)bi cyclo[3.1.0]hexane-3-carboxamide

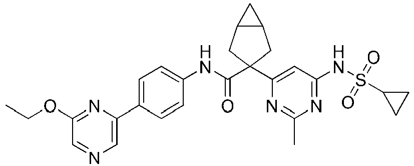

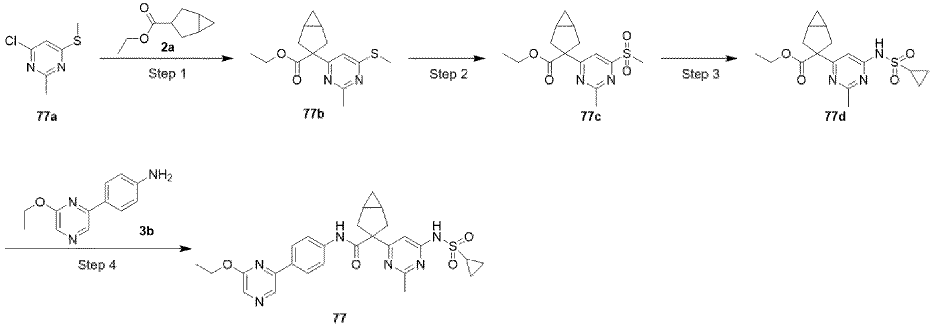

### Step 1

### ethyl 3-(2-methyl-6-(methylthio)pyrimidin-4-yl)bicyclo[3.1.0]hexane-3-carboxylate

At -70°C, lithium bis(trimethylsilyl)amide (1 M, 5.84 mL) was added to a solution of 4-chloro-2-methyl-6-(methylamino)pyrimidine 77a (679.55 mg, 3.89 mmol, commercially available) and ethyl bicyclo[3.1.0]hexane-3-carboxylate 2a (300 mg, 1.95 mmol) in tetrahydrofuran (10 mL). The mixture was slowly heated to room temperature and stirred for 2 hours. Saturated ammonium chloride solution was added to quench the reaction, and the mixture was extracted with ethyl acetate (50 mL × 3). The combined organic phase was washed with saturated sodium chloride solution, dried over anhydrous sodium sulfate, filtered, and concentrated under reduced pressure. The crude product was purified by silica gel column chromatography (eluent: System A) to obtain ethyl 3-(2-methyl-6-(methylthio)pyrimidin-4-yl)bicyclo[3.1.0]hexane-3-carboxylate 77b (160 mg) with a yield of 28.13%.

MS m/z (ESI): 293.1 [M+1]

### Step 2

### ethyl 3-(2-methyl-6-(methylsulfonyl)pyrimidin-4-yl)bicyclo[3.1.0]hexane-3-carboxylate

Ethyl 3-(2-methyl-6-(methylthio)pyrimidin-4-yl)bicyclo[3.1.0]hexane-3-carboxylate 77b (220 mg, 752.41 µmol) was added to a solution of meta-chloroperoxybenzoic acid (305.51 mg, 1.50 mmol, 85% purity) in dichloromethane (4 mL). The mixture was stirred at room temperature for 3 hours. Saturated sodium bicarbonate solution was added to the reaction mixture, and the mixture was extracted with dichloromethane (10 mL × 3). The combined organic phase was washed with saturated sodium chloride solution, dried over anhydrous sodium sulfate, filtered, and concentrated under reduced pressure to obtain ethyl 3-(2-methyl-6-(methylsulfonyl)pyrimidin-4-yl)bicyclo[3.1.0]hexane-3-carboxylate 77c (244 mg) with a yield of 99.97%. The crude product was directly used in the next step.

MS m/z (ESI): 325.0 [M+1]

### Step 3

### ethyl 3-(6-(cyclopropanesulfonamido)-2-methylpyrimidin-4-yl)bicyclo[3.1.0]hexane-3-carboxylate

Ethyl 3-(2-methyl-6-(methylsulfonyl)pyrimidin-4-yl)bicyclo[3.1.0]hexane-3-carboxylate 77c (255.63 mg, 828.89 µmol), cyclopropanesulfonamide (120.51 mg, 994.67 µmol), and cesium carbonate (540.14 mg, 1.66 mmol) were dissolved in N-methylpyrrolidone (1.5 mL). The mixture was heated to 100°C and stirred for 1 hour. The solvent was removed by an oil pump, and the crude product was purified by silica gel column chromatography (eluent: System A) to obtain ethyl 3-(6-(cyclopropanesulfonamido)-2-methylpyrimidin-4-yl)bicyclo[3.1.0]hexane-3-carboxylate 77d (200 mg) with a yield of 66.02%.

MS m/z (ESI): 366.0 [M+1]

### Step 4

### 3-(6-(cyclopropanesulfonamido)-2-methylpyrimidin-4-yl)-N-(4-(6-ethoxypyrazin-2-yl)phenyl)bi cyclo[3.1.0]hexane-3-carboxamide

4-(6-Ethoxypyrazin-2-yl)aniline 3b (60.88 mg, 282.84 µmol) and trimethylaluminum (1.0 M n-hexane solution) (1 M, 848.53 µL) were dissolved in toluene (2 mL) and stirred at room temperature for 1 hour. Ethyl 3-(6-(cyclopropanesulfonamido)-2-methylpyrimidin-4-yl)bicyclo[3.1.0]hexane-3-carboxylate 77d (86.14 mg, 235.70 µmol) was added, and the mixture was heated to 105°C and stirred for 18 hours. A small amount of methanol was added to quench the reaction, and the solvent was directly evaporated to dryness by rotation to obtain a crude product. Dilute hydrochloric acid was added, and the mixture was stirred for 10 minutes and concentrated under reduced pressure. The residue was dissolved in methanol and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)-2-methylpyrimidin-4-yl)-N-(4-(6-ethoxypyrazin-2-yl)phenyl)bi cyclo[3.1.0]hexane-3-carboxamide 77 (36 mg) with a yield of 22.82%.

MS m/z (ESI): 535.1 [M+1]
1H NMR (400 MHz, DMSO-d6) δ 9.64 (s, 1H), 8.64 (s, 1H), 8.07 (s, 1H), 7.96 (d, J=8.3 Hz, 2H), 7.64 (d, J=8.4 Hz, 2H), 6.70 (s, 1H), 4.36 (q, J=7.0 Hz, 2H), 2.69 (d, J=13.5 Hz, 2H), 2.39 (s, 6H), 1.29 (t, J=6.7 Hz, 5H), 0.87 (d, J=23.0 Hz, 4H), 0.29 (d, J=6.9 Hz, 1H), -0.00 (d, J=4.7 Hz, 1H).

### Example 78

### N-(2-(4-(cyclopropanesulfonamido)pyrimidin-2-yl)-2,3-dihydro-1H-inden-2-yl)-4-(6-ethoxypyra zin-2-yl)benzamide

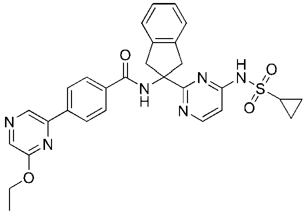

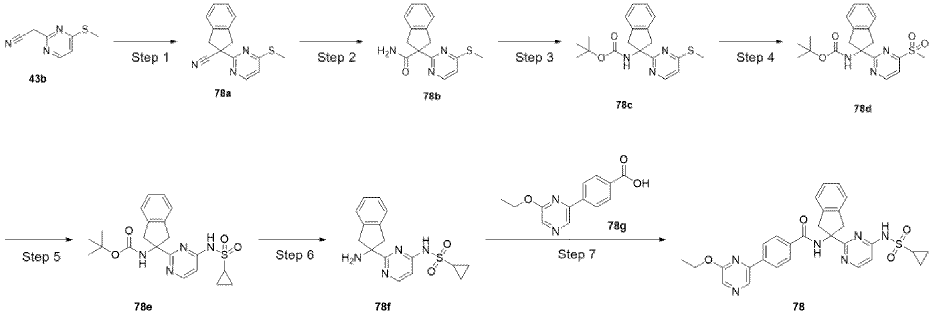

### Step 1

### 2-(4-(methylthio)pyrimidin-2-yl)-2,3-dihydro-1H-indene-2-carbonitrile

2-(4-(Methylthio)pyrimidin-2-yl)acetonitrile **43b** (600 mg, 3.63 mmol) was added to N,N-dimethylformamide (10 mL). The mixture was subjected to argon exchange three times. Under cooling in an ice bath, sodium hydroxide (334.11 mg, 8.35 mmol) was slowly added. After stirring for 0.5 hours, 1,2-bis(chloromethyl)benzene (826.45 mg, 4.72 mmol) was added. The mixture was stirred at room temperature for 23 hours. Water (100 mL) was added to the reaction mixture, and the mixture was extracted with ethyl acetate (100 mL × 3). The combined organic phase was washed with saturated sodium chloride solution (100 mL), dried over anhydrous sodium sulfate, filtered, and concentrated under reduced pressure. The residue was purified by silica gel column chromatography (eluent: System A) to obtain 2-(4-(methylthio)pyrimidin-2-yl)-2,3-dihydro-1H-indene-2-carbonitrile 78a (700 mg) with a yield of 72%.

MS m/z (ESI): 268.1 [M+1]

### Step 2

### 2-(4-(methylthio)pyrimidin-2-yl)-2,3-dihydro-1H-indene-2-carboxamide

2-(4-(Methylthio)pyrimidin-2-yl)-2,3-dihydro-1H-indene-2-carbonitrile **78a** (700 mg, 2.62 mmol) and potassium carbonate (144.75 mg, 1.05 mmol) were sequentially added to dimethyl sulfoxide (20 mL). Under cooling in an ice bath, hydrogen peroxide (2 mL) was added dropwise, and the mixture was stirred at room temperature for 2 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure. The resulting residue was purified by silica gel column chromatography (eluent: System A) to obtain 2-(4-(methylthio)pyrimidin-2-yl)-2,3-dihydro-1H-indene-2-carboxamide 78b (500 mg) with a yield of 65%.

MS m/z (ESI): 286.0 [M+1]

### Step 3

### tert-butyl (2-(4-(methylthio)pyrimidin-2-yl)-2,3-dihydro-1H-inden-2-yl)carbamate

2-(4-(Methylthio)pyrimidin-2-yl)-2,3-dihydro-1H-indene-2-carboxamide 78b (200 mg, 700.86 µmol) and lead tetraacetate (1.24 g, 2.80 mmol) were sequentially added to tert-butanol (5 mL). The mixture was subjected to argon exchange three times and heated to 100°C with continuous stirring for 7 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, and the filtrate was concentrated under reduced pressure. The resulting residue was purified by silica gel column chromatography (eluent: System A) to obtain tert-butyl (2-(4-(methylthio)pyrimidin-2-yl)-2,3-dihydro-1H-inden-2-yl)carbamate 78c (150 mg) with a yield of 60%.

MS m/z (ESI): 358.0 [M+1]

### Step 4

### tert-butyl (2-(4-(methylsulfonyl)pyrimidin-2-yl)-2,3-dihydro-1H-inden-2-yl)carbamate

Tert-butyl (2-(4-(methylthio)pyrimidin-2-yl)-2,3-dihydro-1H-inden-2-yl)carbamate 78c (150 mg, 419.62 µmol) and meta-chloroperoxybenzoic acid (127.79 mg, 629.43 µmol) were sequentially added to dichloromethane (3 mL). The mixture was stirred continuously at room temperature for 2 hours. An aqueous sodium sulfite solution was added to quench the reaction. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure to dryness to obtain crude tert-butyl (2-(4-(methylsulfonyl)pyrimidin-2-yl)-2,3-dihydro-1H-inden-2-yl)carbamate 78d (160 mg). The crude product was directly used in the next step.

MS m/z (ESI): 290.1 [M+1-100]

### Step 5

### tert-butyl (2-(4-(cyclopropanesulfonamido)pyrimidin-2-yl)-2,3-dihydro-1H-inden-2-yl)carbamate

Tert-butyl (2-(4-(methylsulfonyl)pyrimidin-2-yl)-2,3-dihydro-1H-inden-2-yl)carbamate 78d (160 mg, 410.82 µmol), cyclopropanesulfonamide (59.73 mg, 492.98 µmol), and cesium carbonate (267.70 mg, 821.63 µmol) were sequentially added to N-methylpyrrolidone (2 mL). The mixture was heated to 100°C with continuous stirring for 2 hours and concentrated under reduced pressure to dryness. The residue was purified by silica gel column chromatography (eluent: System A) to obtain tert-butyl (2-(4-(cyclopropanesulfonamido)pyrimidin-2-yl)-2,3-dihydro-1H-inden-2-yl)carbamate 78e (120 mg) with a yield of 68%.

MS m/z (ESI): 431.1 [M+1]

### Step 6

### N-(2-(2-amino-2,3-dihydro-1H-inden-2-yl)pyrimidin-4-yl)cyclopropanesulfonamide

Tert-butyl (2-(4-(cyclopropanesulfonamido)pyrimidin-2-yl)-2,3-dihydro-1H-inden-2-yl)carbamate 78e (120 mg, 278.73 µmol) was added to a 1 M solution of hydrochloric acid in dioxane (0.5 mL) and stirred continuously at room temperature for 2 hours. The mixture was concentrated to dryness under reduced pressure to obtain crude N-(2-(2-amino-2,3-dihydro-1H-inden-2-yl)pyrimidin-4-yl)cyclopropanesulfonamide 78f (90 mg) with a yield of 97%.

MS m/z (ESI): 331.0 [M+1]

### Step 7

### N-(2-(4-(cyclopropanesulfonamido)pyrimidin-2-yl)-2,3-dihydro-1H-inden-2-yl)-4-(6-ethoxypyra zin-2-yl)benzamide

4-(6-Ethoxypyrazin-2-yl)benzoic acid 78g (70 mg, 286.60 µmol, prepared according to published patent "WO2022087634"), N-(2-(2-amino-2,3-dihydro-1H-inden-2-yl)pyrimidin-4-yl)cyclopropanesulfonamide 78f (94.69 mg, 286.60 µmol), O-(7-azabenzotriazol-1-yl)-N,N,N',N'-tetramethyluronium hexafluorophosphate (130.77 mg, 343.92 µmol), and N,N-diisopropylethylamine (150.16 µL) were added sequentially to dichloromethane (3 mL) and stirred continuously at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(2-(4-(cyclopropanesulfonamido)pyrimidin-2-yl)-2,3-dihydro-1H-inden-2-yl)-4-(6-ethoxypyra zin-2-yl)benzamide 78 (64 mg) with a yield of 39%.

MS m/z (ESI): 557.2 [M+1]
1H NMR (400 MHz, DMSO-d6) δ 9.10 (s, 1H), 8.88 (s, 1H), 8.45 (d, J = 5.7 Hz, 1H), 8.28 (s, 1H), 8.20 (d, J = 8.4 Hz, 2H), 8.02 (d, J = 8.4 Hz, 2H), 7.24 (dd, J = 5.4, 3.3 Hz, 2H), 7.19-7.14 (m, 2H), 6.76 (d, J = 5.7 Hz, 1H), 4.49 (q, J = 7.0 Hz, 2H), 3.82 (d, J = 16.6 Hz, 2H), 3.63 (s, 2H), 2.86 (tt, J = 8.3, 3.9 Hz, 1H), 1.41 (t, J = 7.0 Hz, 3H), 1.00-0.93 (m, 2H), 0.69-0.61 (m, 2H).

### Example 79

### 3-(6-(cyclopropanesulfonamido)-2-methoxypyrimidin-4-yl)-N-(4-(6-ethoxypyrazin-2-yl)phenyl) bicyclo[3.1.0]hexane-3-carboxamide

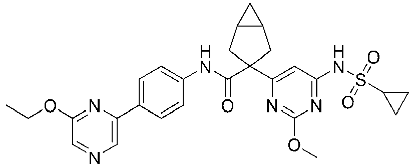

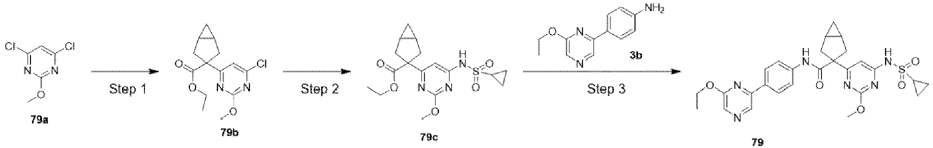

### Step 1

### ethyl 3-(6-chloro-2-methoxypyrimidin-4-yl)bicyclo[3.1.0]hexane-3-carboxylate

At -70°C, lithium bis(trimethylsilyl)amide (1 M, 5.84 mL) was added to a solution of 4,6-dichloro-2-methoxypyrimidine 79a (413.79 mg, 2.88 mmol) and ethyl bicyclo[3.1.0]hexane-3-carboxylate (300 mg, 1.95 mmol) in tetrahydrofuran (5 mL). The mixture was heated to room temperature and stirred for 2 hours. A saturated aqueous ammonium chloride solution (50 mL) was added to the reaction mixture to quench the reaction, and the mixture was extracted with ethyl acetate (50 mL × 3). The combined organic phase was washed with saturated sodium chloride solution, dried over anhydrous sodium sulfate, filtered, and concentrated under reduced pressure. The crude product was purified by silica gel column chromatography (eluent: System A) to obtain ethyl 3-(6-chloro-2-methoxypyrimidin-4-yl)bicyclo[3.1.0]hexane-3-carboxylate 79b (570 mg) with a yield of 98.73%.

MS m/z (ESI): 297.1 [M+1]

### Step 2

### ethyl 3-(6-(cyclopropanesulfonamido)-2-methoxypyrimidin-4-yl)bicyclo[3.1.0]hexane-3-carboxylate

Ethyl 3-(6-chloro-2-methoxypyrimidin-4-yl)bicyclo[3.1.0]hexane-3-carboxylate 79b (100 mg, 336.99 µmol), cyclopropanesulfonamide (61.24 mg, 505.48 µmol), cesium carbonate (219.59 mg, 673.97 µmol), di-tert-butyl-[2-(2,4,6-triisopropylphenyl)phenyl]phosphine (14.31 mg, 33.70 µmol), and allylpalladium(II) chloride dimer (6.16 mg, 16.85 µmol) were added sequentially to 1,4-dioxane (2 mL). The mixture was subjected to argon exchange 3 times and heated to 100°C with stirring for 2 hours. The solvent was removed by concentration under reduced pressure, and the crude product was purified by silica gel column chromatography (eluent: System A) to obtain ethyl 3-(6-(cyclopropanesulfonamido)-2-methoxypyrimidin-4-yl)bicyclo[3.1.0]hexane-3-carboxylate 79c (50 mg) with a yield of 38.90%.

MS m/z (ESI): 382.0 [M+1]

### Step 3

### 3-(6-(cyclopropanesulfonamido)-2-methoxypyrimidin-4-yl)-N-(4-(6-ethoxypyrazin-2-yl)phenyl) bicyclo[3.1.0]hexane-3-carboxamide

Trimethylaluminum (2.0 M toluene solution) (2 M, 424.27 µL) was added to a solution of 4-(6-ethoxypyrazin-2-yl)aniline 3b (60.88 mg, 282.84 µmol) in toluene (1.58 mL) and stirred at room temperature for 1 hour. Ethyl 3-(6-(cyclopropanesulfonamido)-2-methoxypyrimidin-4-yl)bicyclo[3.1.0]hexane-3-carboxylate 79c (89.91 mg, 235.70 µmol) was added, and the mixture was heated to 105°C and stirred for 18 hours. A small amount of methanol was added to the reaction mixture to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)-2-methoxypyrimidin-4-yl)-N-(4-(6-ethoxypyrazin-2-yl)phenyl) bicyclo[3.1.0]hexane-3-carboxamide 79 (20 mg) with a yield of 10.60%. MS m/z (ESI): 551.1 [M+1]

1H NMR (400 MHz, DMSO-d6) δ 9.60 (s, 1H), 8.65 (s, 1H), 8.07 (s, 1H), 7.97 (d, J = 8.5 Hz, 2H), 7.66 (d, J = 8.6 Hz, 2H), 6.40 (s, 1H), 4.41-4.34 (m, 2H), 3.80 (s, 3H), 2.99 (tt, J = 8.0, 4.9 Hz, 1H), 2.69 (d, J = 13.5 Hz, 2H), 2.36-2.28 (m, 2H), 1.29 (t, J = 6.9 Hz, 5H), 0.99-0.90 (m, 4H), 0.30 (td, J = 8.0, 5.4 Hz, 1H).

### Example 80

### 3-(6-(cyclopropanesulfonamido)pyrimidin-4-yl)-N-(4-(5-methoxypyrazolo[1,5-a]pyridin-3-yl)ph enyl)bicyclo[3.1.0]hexane-3-carboxamide

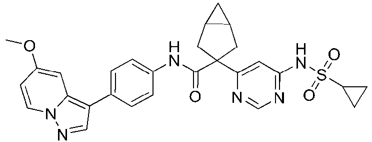

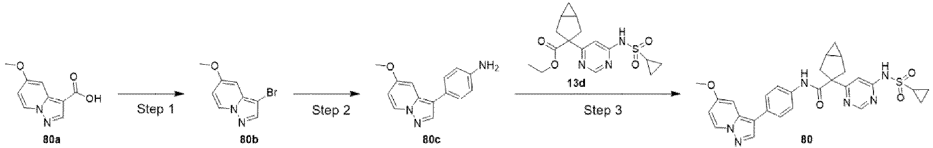

### Step 1

### 3-bromo-5-methoxy-pyrazolo[1,5-a]pyridine

5-Methoxypyrazolo[1,5-a]pyridine-3-carboxylic acid 80a (530 mg, 2.76 mmol, commercially available) and N-bromosuccinimide (589.03 mg, 3.31 mmol) were dissolved in N,N-dimethylformamide (5 mL) and stirred at room temperature for 1 hour. Water (50 mL) was added to the reaction solution, and the mixture was extracted with ethyl acetate (50 mL × 3). The combined organic phase was washed with saturated sodium chloride solution, dried over anhydrous sodium sulfate, filtered, and concentrated under reduced pressure to obtain 3-bromo-5-methoxy-pyrazolo[1,5-a]pyridine 80b (600 mg) with a yield of 95.81%.

MS m/z (ESI): 227.1 [M+1]

### Step 2

### 4-(5-methoxypyrazolo[1,5-a]pyridin-3-yl)aniline

3-Bromo-5-methoxy-pyrazolo[1,5-a]pyridine 80b (350 mg, 1.54 mmol), 4-(4,4,5,5-tetramethyl-1,3,2-dioxaborolan-2-yl)aniline (439.03 mg, 2.00 mmol), [1,1'-bis(diphenylphosphino)ferrocene]palladium(II) dichloride (112.79 mg, 154.15 µmol), and potassium carbonate (639.13 mg, 4.62 mmol) were dissolved in 1,4-dioxane (5 mL) and water (1 mL). The mixture was subjected to argon exchange three times and heated to 95°C with stirring for 1.5 hours. The mixture was concentrated under reduced pressure, and the solvent was directly evaporated to dryness by rotation. The crude product was purified by silica gel column chromatography (eluent: System A) to obtain 4-(5-methoxypyrazolo[1,5-a]pyridin-3-yl)aniline 80c (350 mg) with a yield of 94.90%.

MS m/z (ESI): 240.1 [M+1]

### Step 3

### 3-(6-(cyclopropanesulfonamido)pyrimidin-4-yl)-N-(4-(5-methoxypyrazolo[1,5-a]pyridin-3-yl)ph enyl)bicyclo[3.1.0]hexane-3-carboxamide

Trimethylaluminum (2.0 M n-hexane solution) (2 M, 512.21 µL) was added to a solution of 4-(5-methoxypyrazolo[1,5-a]pyridin-3-yl)aniline 80c (74.90 mg, 313.02 µmol) in toluene (2 mL). The mixture was stirred at room temperature for 1 hour, then ethyl 3-(6-(cyclopropanesulfonamido)pyrimidin-4-yl)bicyclo[3.1.0]hexane-3-carboxylate 13d (100 mg, 284.56 µmol) was added, and the mixture was heated to 90°C with stirring for 1.5 hours. A small amount of methanol was added to quench the reaction, and the solvent was directly evaporated to dryness by rotation to obtain a crude product. Dilute hydrochloric acid and dichloromethane were added, and insoluble solid was removed by filtration. After removal of the organic phase from the filtrate, the crude product was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(5-methoxypyrazolo[1,5-a]pyridin-3-yl)ph enyl)bicyclo[3.1.0]hexane-3-carboxamide 80 (44 mg) with a yield of20.66%.

MS m/z (ESI): 545.2 [M+1]
1H NMR (400 MHz, DMSO-d6) δ 9.46 (s, 1H), 8.64 (s, 1H), 8.42 (d, J=7.6 Hz, 1H), 8.06 (s, 1H), 7.53 (d, J=8.3 Hz, 2H), 7.45 (d, J=8.5 Hz, 2H), 6.98 (d, J=2.7 Hz, 1H), 6.79 (s, 1H), 6.48 (dd, J=7.6, 2.7 Hz, 1H), 3.75 (s, 3H), 2.92 (s, 1H), 2.73 (d, J=13.5 Hz, 2H), 2.27 (d, J=13.6 Hz, 2H), 1.27 (dt, J=7.8, 3.4 Hz, 2H), 0.91 (dt, J=23.6, 5.1 Hz, 4H), 0.31-0.23 (m, 1H), -0.01 (d, J=4.5 Hz, 1H).

### Example 81

### 3-(5-(cyclopropanesulfonamido)pyrazolo[1,5-a]pyrimidin-7-yl)-N-(4-(6-ethoxypyrazin-2-yl)phen yl)bicyclo[3.1.0]hexane-3-carboxamide

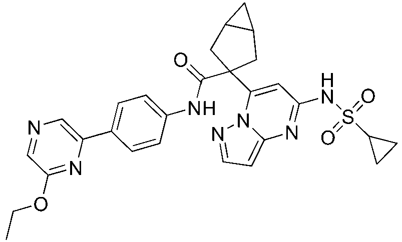

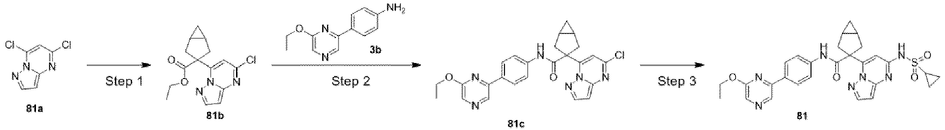

### Step 1

### ethyl 3-(5-chloropyrazolo[1,5-a]pyrimidin-7-yl)bicyclo[3.1.0]hexane-3-carboxylate

At -70°C, lithium bis(trimethylsilyl)amide (1 M, 9.73 mL) was added to a solution of 5,7-dichloropyrazolo[1,5-a]pyrimidine 81a (731.54 mg, 3.89 mmol, commercially available) in tetrahydrofuran (10 mL). The mixture was slowly heated to -30°C and stirred for 1 hour. A saturated aqueous ammonium chloride solution (20 mL) was added to the reaction solution, and the mixture was extracted with ethyl acetate (20 mL × 3). The combined organic phase was washed with saturated sodium chloride solution, dried over anhydrous sodium sulfate, filtered, and concentrated under reduced pressure. The crude product was purified by silica gel column chromatography (eluent: System A) to obtain ethyl 3-(5-chloropyrazolo[1,5-a]pyrimidin-7-yl)bicyclo[3.1.0]hexane-3-carboxylate 81b (750 mg) with a yield of 75.65%.

MS m/z (ESI): 306.1 [M+1]

### Step 2

### 3-(5-chloropyrazolo[1,5-a]pyrimidin-7-yl)-N-[4-(6-ethoxypyrazin-2-yl)phenyl]bicyclo[3.1.0]hex ane-3-carboxamide

Under ice-water bath, trimethylaluminum (2.0 M n-hexane solution) (2 M, 4.12 mL) was added to a solution of 4-(6-ethoxypyrazin-2-yl)aniline 3b (591.35 mg, 2.75 mmol) in toluene (5 mL). The mixture was stirred at room temperature for 1 hour. A solution of ethyl 3-(5-chloropyrazolo[1,5-a]pyrimidin-7-yl)bicyclo[3.1.0]hexane-3-carboxylate 81b (700 mg, 2.29 mmol) in toluene was added, and the mixture was heated to 100°C and stirred for 5 hours. A small amount of methanol was added to quench the reaction, and the solvent was directly evaporated to dryness by rotation to obtain a crude product. Dilute hydrochloric acid was added to pH 3-4, and ethyl acetate was added. The mixture was stirred for 0.5 hours, then neutralized with saturated sodium bicarbonate solution (20 mL). Insoluble solid was removed by filtration, and the organic phase was separated. The organic phase was dried over anhydrous sodium sulfate, filtered, and concentrated under reduced pressure. The crude product was purified by silica gel column chromatography (eluent: System A) to obtain 3-(5-chloropyrazolo[1,5-a]pyrimidin-7-yl)-N-[4-(6-ethoxypyrazin-2-yl)phenyl]bicyclo[3.1.0]hex ane-3-carboxamide 81c (100 mg) with a yield of 9.20%.

MS m/z (ESI): 475.2 [M+1]

### Step 3

### 3-(5-(cyclopropanesulfonamido)pyrazolo[1,5-a]pyrimidin-7-yl)-N-(4-(6-ethoxypyrazin-2-yl)phen yl)bicyclo[3.1.0]hexane-3-carboxamide

3-(5-Chloropyrazolo[1,5-a]pyrimidin-7-yl)-N-[4-(6-ethoxypyrazin-2-yl)phenyl]bicyclo[ 3.1.0]hexane-3-carboxamide 81c (100 mg, 210.55 µmol), cyclopropanesulfonamide (33.16 mg, 273.72 µmol), cesium carbonate (137.20 mg, 421.10 µmol), di-tert-butyl-(2-(2,4,6-triisopropylphenyl)phenyl)phosphine (8.94 mg, 21.06 µmol), and allylpalladium(II) chloride dimer (3.85 mg, 10.53 µmol) were sequentially added to 1,4-dioxane (2 mL). The mixture was subjected to argon exchange 3 times and heated to 100°C with stirring for 2 hours. The solvent was evaporated to dryness by rotation, and insoluble solid was filtered from the crude product. The product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(5-(cyclopropanesulfonamido)pyrazolo[1,5-a]pyrimidin-7-yl)-N-(4-(6-ethoxypyrazin-2-yl)phen yl)bicyclo[3.1.0]hexane-3-carboxamide 81 (25 mg) with a yield of 15.83%.

MS m/z (ESI): 560.1 [M+1]
1H NMR (400 MHz, DMSO-d6) δ 11.18 (s, 1H), 9.48 (s, 1H), 8.72 (s, 1H), 8.16 (s, 1H), 8.07-7.94 (m, 3H), 7.59 (d, J=8.4 Hz, 2H), 6.81 (s, 1H), 6.42 (s, 1H), 4.45 (q, J=7.0 Hz, 2H), 3.22 (s, 1H), 2.63-2.53 (m, 4H), 1.53-1.45 (m, 2H), 1.38 (t, J=7.0 Hz, 3H), 1.19-1.07 (m, 4H), 0.81 (d, J=6.9 Hz, 1H), 0.46 (d, J=4.5 Hz, 1H).

### Example 82

### 3-(6-(cyclopropanesulfonamido)pyrimidin-4-yl)-N-(4-(6-ethoxypyrazin-2-yl)phenyl)-8-oxabicycl o[3.2.1]octane-3-carboxamide

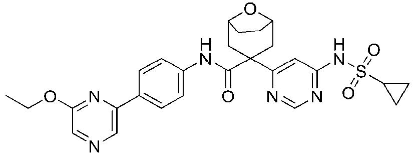

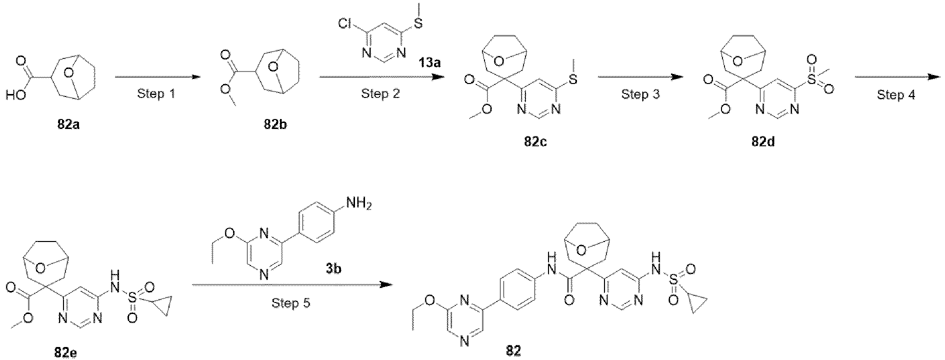

### Step 1

### methyl 8-oxabicyclo[3.2.1]octane-3-carboxylate

8-Oxabicyclo[3.2.1]octane-3-carboxylic acid 82a (1.7 g, 10.88 mmol, commercially available), potassium carbonate (4.51 g, 32.65 mmol), and iodomethane (4.64 g, 32.65 mmol, 2.03 mL) were dissolved in N,N-dimethylformamide (10 mL). The mixture was heated to 60°C and stirred for 18 hours. Water (100 mL) and ethyl acetate (100 mL) were added to the reaction mixture, and the organic phase was separated. The organic phase was dried over anhydrous sodium sulfate, filtered, and concentrated under reduced pressure. The crude product was separated by silica gel column chromatography (eluent: System A) to obtain methyl 8-oxabicyclo[3.2.1]octane-3-carboxylate 82b (1.1 g) with a yield of 59.37%.

### Step 2

### methyl 3-(6-(methylthio)pyrimidin-4-yl)-8-oxabicyclo[3.2.1]octane-3-carboxylate

At -30°C, lithium bis(trimethylsilyl)amide (1 M, 19.39 mL) was added to a solution of 4-chloro-6-methylaminopyrimidine 13a (1.14 g, 7.11 mmol) and methyl 8-oxabicyclo[3.2.1]octane-3-carboxylate 82b (1.1 g, 6.46 mmol) in tetrahydrofuran (15 mL). The mixture was heated to room temperature and stirred for 1 hour. Saturated ammonium chloride solution (100 mL) was added to quench the reaction, and the organic phase was separated. The organic phase was dried over anhydrous sodium sulfate, filtered, and concentrated under reduced pressure. The residue was separated by silica gel column chromatography (eluent: System A) to obtain methyl 3-(6-(methylthio)pyrimidin-4-yl)-8-oxabicyclo[3.2.1]octane-3-carboxylate 82c (1.2 g) with a yield of 63.08%.

MS m/z (ESI): 295.1 [M+1]

### Step 3

### methyl 3-(6-(methylsulfonyl)pyrimidin-4-yl)-8-oxabicyclo[3.2.1]octane-3-carboxylate

Methyl 3-(6-(methylthio)pyrimidin-4-yl)-8-oxabicyclo[3.2.1]octane-3-carboxylate 82c (1.2 g, 4.08 mmol) and meta-chloroperoxybenzoic acid (1.66 g, 8.15 mmol) were dissolved in dichloromethane (10 mL) and stirred at room temperature for 2 hours. Saturated sodium bicarbonate solution (50 mL) was added, and the mixture was extracted with dichloromethane (50 mL × 3). The combined organic phase was washed with saturated sodium chloride solution, dried over anhydrous sodium sulfate, filtered, and evaporated to dryness by rotation to obtain methyl 3-(6-(methylsulfonyl)pyrimidin-4-yl)-8-oxabicyclo[3.2.1]octane-3-carboxylate 82d (1.2 g) with a yield of 90.20%. The crude product was directly used in the next step.

MS m/z (ESI): 327.0 [M+1]

### Step 4

### methyl 3-(6-(cyclopropanesulfonamido)pyrimidin-4-yl)-8-oxabicyclo[3.2.1]octane-3-carboxylate

Methyl 3-(6-(methylsulfonyl)pyrimidin-4-yl)-8-oxabicyclo[3.2.1]octane-3-carboxylate 82d (1.1 g, 3.37 mmol), cyclopropanesulfonamide (490.03 mg, 4.04 mmol), and cesium carbonate (2.20 g, 6.74 mmol) were dissolved in N-methylpyrrolidone (8 mL). The mixture was heated to 100°C and stirred for 1 hour. Water (50 mL) was added to the reaction mixture, and the mixture was extracted with ethyl acetate (50 mL × 3). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 3-(6-(cyclopropanesulfonamido)pyrimidin-4-yl)-8-oxabicyclo[3.2.1]octane-3-carboxylate 82e (1 g) with a yield of 80.75%.

MS m/z (ESI): 368.0 [M+1]

### Step 5

### 3-(6-(cyclopropanesulfonamido)pyrimidin-4-yl)-N-(4-(6-ethoxypyrazin-2-yl)phenyl)-8-oxabicycl o[3.2.1]octane-3-carboxamide

Under ice-water bath, trimethylaluminum (2.0 M n-hexane solution) (2 M, 2.45 mL) was added to a solution of 4-(6-ethoxypyrazin-2-yl)aniline 3b (351.51 mg, 1.63 mmol) in toluene (5 mL). The mixture was stirred at room temperature for 1 hour, and then a solution of methyl 3-(6-(cyclopropanesulfonamido)pyrimidin-4-yl)-8-oxabicyclo[3.2.1]octane-3-carboxylate 82e (500 mg, 1.36 mmol) in toluene was added. The mixture was heated to 100°C and stirred for 5 hours. A small amount of methanol was added to quench the reaction, and the solvent was directly evaporated to obtain a crude product. Dilute hydrochloric acid was added, and the mixture was stirred for 10 minutes. The crude product was directly evaporated to dryness by rotation, then dissolved in methanol, and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(6-ethoxypyrazin-2-yl)phenyl)-8-oxabicycl o[3.2.1]octane-3-carboxamide 82 (65 mg) with a yield of 6.90%.

MS m/z (ESI): 551.1 [M+1]
1H NMR (400 MHz, DMSO-d6) δ 9.84 (s, 1H), 8.83 (d, J=15.8 Hz, 2H), 8.24 (s, 1H), 8.14 (d, J=8.4 Hz, 2H), 7.82 (d, J=8.4 Hz, 2H), 7.00 (s, 1H), 4.52 (q, J=7.0 Hz, 2H), 4.46 (s, 2H), 3.11 (s, 1H), 2.80 (d, J=14.3 Hz, 2H), 2.28 (dd, J=14.5, 3.9 Hz, 2H), 1.94 (d, J=7.2 Hz, 2H), 1.81 (dd, J=8.3, 4.4 Hz, 2H), 1.45 (t, J=7.0 Hz, 3H), 1.09 (dd, J=23.0, 6.4 Hz, 4H).

### Example 83

### N-(3-(6-(cyclopropanesulfonamido)pyrimidin-4-yl)bicyclo[3.1.0]hexan-3-yl)-4-(6-ethoxypyrazin -2-yl)benzamide

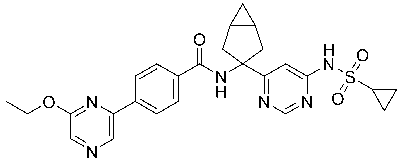

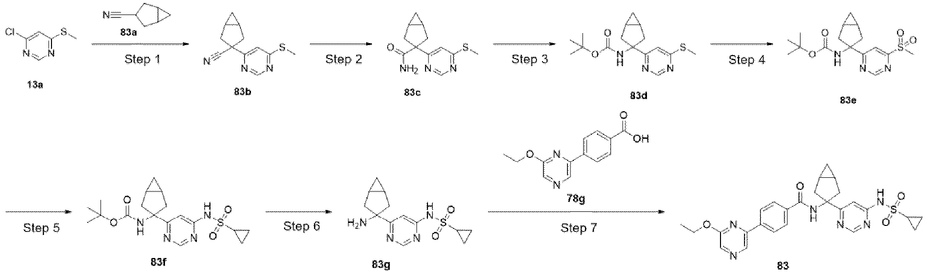

### Step 1

### 3-(6-(methylthio)pyrimidin-4-yl)bicyclo[3.1.0]hexane-3-carbonitrile

4-Chloro-6-(methylthio)pyrimidine **13a** (296.81 mg, 1.85 mmol, commercially available) and bicyclo[3.1.0]hexane-3-carbonitrile 83a (180 mg, 1.68 mmol, commercially available) were added to anhydrous tetrahydrofuran (5 mL). The mixture was cooled to -30°C, and 1 M lithium bis(trimethylsilyl)amide (5.0 mL) was added. The mixture was heated to room temperature and the reaction was performed for 2 hours. Saturated ammonium chloride aqueous solution was added to quench the reaction, and the mixture was extracted with ethyl acetate (100 mL). The organic phase was washed with saturated sodium chloride aqueous solution (100 mL × 3), dried over anhydrous sodium sulfate, filtered, and concentrated under reduced pressure. The residue was separated by silica gel column chromatography (eluent: System A) to obtain 3-(6-(methylthio)pyrimidin-4-yl)bicyclo[3.1.0]hexane-3-carbonitrile **83b** (222.0 mg) with a yield of 57%.

MS m/z (ESI): 232.1 [M+1]

### Step 2

### 3-(6-(methylthio)pyrimidin-4-yl)bicyclo[3.1.0]hexane-3-carboxamide

3-(6-(methylthio)pyrimidin-4-yl)bicyclo[3.1.0]hexane-3-carbonitrile 83b (220 mg, 951.08 µmol) and 2.5 M sodium hydroxide aqueous solution (0.5 mL) were sequentially added to dimethyl sulfoxide (2.0 mL). Hydrogen peroxide (0.5 mL) was added dropwise under an ice bath, and the mixture was stirred at room temperature for 1 hour. Saturated sodium sulfite aqueous solution was added to quench the reaction, and ethyl acetate (30 mL) and water (15 mL) were added. The mixture was separated, and the aqueous phase was extracted with ethyl acetate (30 mL × 2). The combined organic phase was washed with saturated sodium chloride solution (30 mL × 2), dried over anhydrous sodium sulfate, and concentrated under reduced pressure. The resulting residue was purified by silica gel column chromatography (eluent: System A) to obtain 3-(6-(methylthio)pyrimidin-4-yl)bicyclo[3.1.0]hexane-3-carboxamide 83c (230 mg) with a yield of 97%.

MS m/z (ESI): 250.0 [M+1]

### Step 3

### tert-butyl (3-(6-(methylthio)pyrimidin-4-yl)bicyclo[3.1.0]hexan-3-yl)carbamate

3-(6-(Methylthio)pyrimidin-4-yl)bicyclo[3.1.0]hexane-3-carboxamide **83c** (230 mg, 922.47 µmol) and lead tetraacetate (1.64 g, 3.69 mmol) were sequentially added to tert-butanol (3 mL). The mixture was subjected to argon exchange three times and heated to 100°C with stirring for 4 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure to dryness. The residue was purified by silica gel column chromatography (eluent: System A) to obtain tert-butyl (3-(6-(methylthio)pyrimidin-4-yl)bicyclo[3.1.0]hexan-3-yl)carbamate 83d (166 mg) with a yield of 56%.

MS m/z (ESI): 322.1 [M+1]

### Step 4

### tert-butyl (3-(6-(methylsulfonyl)pyrimidin-4-yl)bicyclo[3.1.0]hexan-3-yl)carbamate

Tert-butyl (3-(6-(methylthio)pyrimidin-4-yl)bicyclo[3.1.0]hexan-3-yl)carbamate 83d (166 mg, 516.43 µmol) and meta-chloroperoxybenzoic acid (209.70 mg, 1.03 mmol) were sequentially added to dichloromethane (5 mL). The mixture was stirred at room temperature for 2 hours. A sodium sulfite aqueous solution was added to quench the reaction. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure to dryness to obtain tert-butyl (3-(6-(methylsulfonyl)pyrimidin-4-yl)bicyclo[3.1.0]hexan-3-yl)carbamate 83e (180 mg) with a yield of 98.6%. The crude product was directly used in the next step.

MS m/z (ESI): 298.0 [M+1-56]

### Step 5

### tert-butyl (3-(6-(cyclopropanesulfonamido)pyrimidin-4-yl)bicyclo[3.1.0]hexan-3-yl)carbamate

Tert-butyl (3-(6-(methylsulfonyl)pyrimidin-4-yl)bicyclo[3.1.0]hexan-3-yl)carbamate 83e (180 mg, 509.29 µmol), cyclopropanesulfonamide (74.04 mg, 611.14 µmol), and cesium carbonate (331.87 mg, 1.02 mmol) were sequentially added to N-methylpyrrolidone (2 mL). The mixture was heated to 100°C with stirring for 2 hours and concentrated under reduced pressure to dryness. The resulting residue was purified by silica gel column chromatography (eluent: System A) to obtain tert-butyl (3-(6-(cyclopropanesulfonamido)pyrimidin-4-yl)bicyclo[3.1.0]hexan-3-yl)carbamate 83f (200 mg) with a yield of 99.5%.

MS m/z (ESI): [M+1] 395.2

### Step 6

### N-(6-(3-aminobicyclo[3.1.0]hexan-3-yl)pyrimidin-4-yl)cyclopropanesulfonamide

Tert-butyl (3-(6-(cyclopropanesulfonamido)pyrimidin-4-yl)bicyclo[3.1.0]hexan-3-yl)carbamate 83f (200 mg, 506.99 µmol) was added to a 1 M solution of hydrochloric acid in dioxane (0.5 mL). The mixture was stirred at room temperature for 2 hours and concentrated under reduced pressure to dryness to obtain N-(6-(3-aminobicyclo[3.1.0]hexan-3-yl)pyrimidin-4-yl)cyclopropanesulfonamide 83g (90 mg). The crude product was directly used in the next step.

MS m/z (ESI): 295.0 [M+1]

### Step 7

### N-(3-(6-(cyclopropanesulfonamido)pyrimidin-4-yl)bicyclo[3.1.0]hexan-3-yl)-4-(6-ethoxypyrazin -2-yl)benzamide

4-(6-Ethoxypyrazin-2-yl)benzoic acid 78g (50 mg, 204.71 µmol), N-(6-(3-aminobicyclo[3.1.0]hexan-3-yl)pyrimidin-4-yl)cyclopropanesulfonamide 83g (60.26 mg, 204.71 µmol), O-(7-azabenzotriazol-1-yl)-N,N,N',N'-tetramethyluronium hexafluorophosphate (93.41 mg, 245.65 µmol), and N,N-diisopropylethylamine (110 µL) were sequentially added to dichloromethane (3 mL). The mixture was stirred at room temperature for 2 hours and concentrated under reduced pressure to dryness. The resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-4-(6-ethoxypyrazin -2-yl)benzamide 83 (3.1 mg) with a yield of 3%.

MS m/z (ESI): 521.2 [M+1]
1H NMR (400 MHz, DMSO-d6) δ 8.90 (s, 1H), 8.75 (s, 1H), 8.71 (s, 1H), 8.30 (s, 1H), 8.25 (d, J = 8.1 Hz, 2H), 7.97 (d, J = 8.1 Hz, 2H), 6.99 (s, 1H), 4.51 (q, J = 7.0 Hz, 2H), 3.01 (s, 1H), 2.61 (dd, J = 14.1, 3.3 Hz, 4H), 1.47 (dt, J = 8.0, 3.8 Hz, 2H), 1.42 (t, J = 7.0 Hz, 3H), 1.07 - 0.98 (m, 4H), 0.63 (q, J = 4.2 Hz, 1H), 0.56 (td, J = 8.3, 4.9 Hz, 1H).

### Example 84

### 3-(4-(cyclopropanesulfonamido)pyrimidin-2-yl)-N-(4-(6-ethoxypyrazin-2-yl)-2-methoxyphenyl) bicyclo[3.1.0]hexane-3-carboxamide

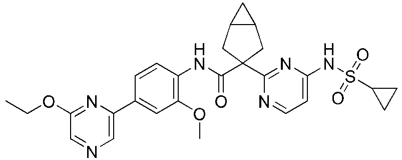

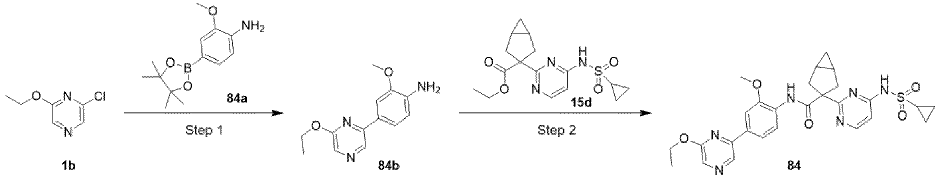

### Step 1

### 4-(6-ethoxypyrazin-2-yl)-2-methoxyaniline

2-Chloro-6-ethoxypyrazine **1b** (360.00 mg, 2.27 mmol, commercially available), 2-methoxy-4-(4,4,5,5-tetramethyl-1,3,2-dioxaborolan-2-yl)aniline 84a (471.25 mg, 1.89 mmol, commercially available), [1,1'-bis(diphenylphosphino)ferrocene]palladium(II) dichloride (138.42 mg, 189.17 µmol), and potassium carbonate (784.37 mg, 5.68 mmol) were dissolved in 1,4-dioxane (8 mL) and water (2 mL). The mixture was subjected to argon exchange three times and heated to 95°C with stirring for 3 hours. The solvent was removed by concentration under reduced pressure, and the residue was separated by silica gel column chromatography (eluent: System A) to obtain 4-(6-ethoxypyrazin-2-yl)-2-methoxyaniline 84b (300 mg) with a yield of 64.66%.

MS m/z (ESI): 246.1 [M+1]

### Step 2

### 3-(4-(cyclopropanesulfonamido)pyrimidin-2-yl)-N-(4-(6-ethoxypyrazin-2-yl)-2-methoxyphenyl) bicyclo[3.1.0]hexane-3-carboxamide

Trimethylaluminum (2.0 M n-hexane solution) (2 M, 332.94 µL) was added to a solution of 4-(6-ethoxypyrazin-2-yl)-2-methoxyaniline 84b (49.90 mg, 203.46 µmol) in toluene (3 mL), and the mixture was stirred at room temperature for 1 hour. Ethyl 3-(4-(cyclopropanesulfonamido)pyrimidin-2-yl)bicyclo[3.1.0]hexane-3-carboxylate 15d (65 mg, 184.96 µmol) was added, and the mixture was heated to 90°C with stirring for 5 hours. A small amount of methanol was added to quench the reaction, and the solvent was directly evaporated to obtain a crude product. Dilute hydrochloric acid and dichloromethane were added, and insoluble solid was removed by filtration. The solvent was evaporated to dryness by rotation, and the residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2-methoxyphenyl) bicyclo[3.1.0]hexane-3-carboxamide 84 (10 mg) with a yield of 7.37%.

MS m/z (ESI): 551.2 [M+1]
1H NMR (400 MHz, DMSO-d6) δ 11.08 (s, 1H), 8.74 (s, 1H), 8.56 (s, 1H), 8.33 (d, J=5.7 Hz, 1H), 7.94 (s, 1H), 7.86 (d, J=8.6 Hz, 1H), 7.46 (d, J=7.1 Hz, 2H), 6.63 (d, J=5.7 Hz, 1H), 4.23 (q, J=7.0 Hz, 2H), 3.68 (s, 3H), 2.89 (s, 1H), 2.53 (d, J=13.7 Hz, 2H), 2.47-2.36 (m, 2H), 1.15 (t, J=7.0 Hz, 5H), 0.89 (q, J=4.7, 4.0 Hz, 2H), 0.79 (dt, J=7.8, 3.4 Hz, 2H), 0.19 (d, J=6.6 Hz, 1H), 0.00 (d, J=4.3 Hz, 1H).

### Example 85

### 3-(4-(cyclopropanesulfonamido)pyrimidin-2-yl)-N-(4-(6-ethoxypyrazin-2-yl)-2-methylphenyl)bi cyclo[3.1.0]hexane-3-carboxamide

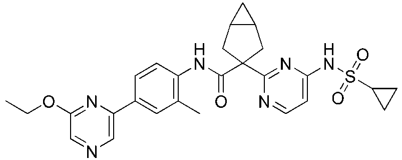

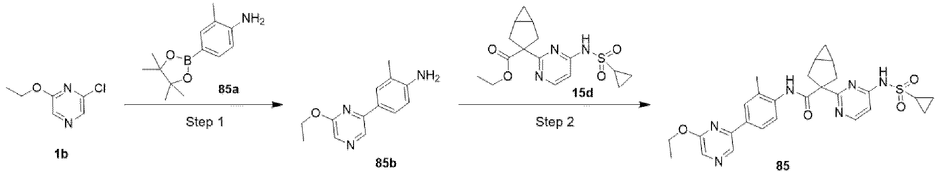

### Step 1

### 4-(6-ethoxypyrazin-2-yl)-2-methylaniline

2-Chloro-6-ethoxy-pyrazine **1b** (450.00 mg, 2.84 mmol), 2-methyl-4-(4,4,5,5-tetramethyl-1,3,2-dioxaborolan-2-yl)aniline 85a (440.99 mg, 1.89 mmol, commercially available), [1,1'-bis(diphenylphosphino)ferrocene]palladium(II) dichloride (138.42 mg, 189.17 µmol), and potassium carbonate (784.37 mg, 5.68 mmol) were dissolved in 1,4-dioxane (8 mL) and water (2 mL). The mixture was subjected to argon exchange three times and heated to 95°C with stirring for 3 hours. The solvent was removed by concentration under reduced pressure, and the crude product was separated by silica gel column chromatography (eluent: System A) to obtain 4-(6-ethoxypyrazin-2-yl)-2-methylaniline 85b (370 mg) with a yield of 85.31%.

MS m/z (ESI): 230.1 [M+1]

### Step 2

### 3-(4-(cyclopropanesulfonamido)pyrimidin-2-yl)-N-(4-(6-ethoxypyrazin-2-yl)-2-methylphenyl)bi cyclo[3.1.0]hexane-3-carboxamide

Trimethylaluminum (2.0 M n-hexane solution) (2 M, 332.94 µL) was added to a solution of 4-(6-ethoxypyrazin-2-yl)-2-methylaniline 85b (46.65 mg, 203.46 µmol) in toluene (3 mL), and the mixture was stirred at room temperature for 1 hour. Ethyl 3-(4-(cyclopropanesulfonamido)pyrimidin-2-yl)bicyclo[3.1.0]hexane-3-carboxylate 15d (65 mg, 184.96 µmol) was added, and the mixture was heated to 90°C with stirring for 5 hours. A small amount of methanol was added to quench the reaction, and the solvent was directly evaporated to obtain a crude product. Dilute hydrochloric acid and dichloromethane were added, and insoluble solid was removed by filtration. The crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2-methylphenyl)bi cyclo[3.1.0]hexane-3-carboxamide 85 (32 mg) with a yield of 25.75%.

MS m/z (ESI): 535.1 [M+1]
1H NMR (400 MHz, DMSO-d6) δ 11.28 (s, 1H), 8.99 (s, 1H), 8.75 (s, 1H), 8.53 (d, J=5.7 Hz, 1H), 8.19 (s, 1H), 7.99-7.83 (m, 2H), 7.48 (d, J=8.3 Hz, 1H), 6.87 (d, J=5.7 Hz, 1H), 4.47 (q, J=7.0 Hz, 2H), 3.18 (s, 1H), 2.85 (d, J=13.6 Hz, 2H), 2.61 (d, J=13.5 Hz, 2H), 2.17 (s, 3H), 1.40 (t, J=6.6 Hz, 5H), 1.18-0.99 (m, 4H), 0.44 (s, 1H), 0.27 (s, 1H).

### Example 86

### N-(2-cyano-4-(6-ethoxypyrazin-2-yl)phenyl)-3-(4-(cyclopropanesulfonamido)pyrimidin-2-yl)bic yclo[3.1.0]hexane-3-carboxamide

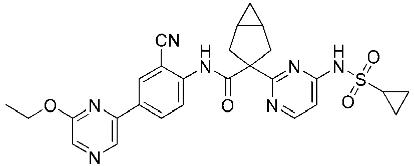

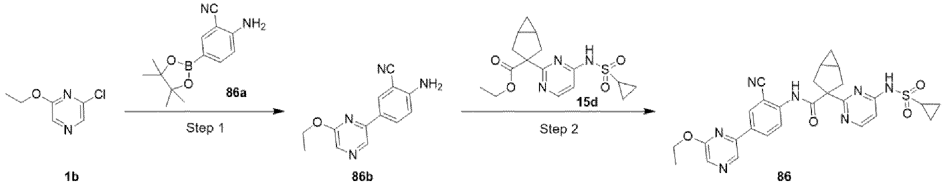

### Step 1

### 2-amino-5-(6-ethoxypyrazin-2-yl)benzonitrile

2-Chloro-6-ethoxypyrazine **1b** (390.00 mg, 2.46 mmol), 2-amino-5-(4,4,5,5-tetramethyl-1,3,2-dioxaborolan-2-yl)benzonitrile 86a (461.76 mg, 1.89 mmol, commercially available), [1,1'-bis(diphenylphosphino)ferrocene]palladium(II) dichloride (138.42 mg, 189.17 µmol), and potassium carbonate (784.37 mg, 5.68 mmol) were dissolved in 1,4-dioxane (8 mL) and water (2 mL). The mixture was subjected to argon exchange three times and heated to 95°C with stirring for 3 hours. The solvent was evaporated, and the crude product was separated by silica gel column chromatography (eluent: System A) to obtain 2-amino-5-(6-ethoxypyrazin-2-yl)benzonitrile 86b (430 mg) with a yield of 94.61%.

MS m/z (ESI): 241.1 [M+1]

### Step 2

### N-(2-cyano-4-(6-ethoxypyrazin-2-yl)phenyl)-3-(4-(cyclopropanesulfonamido)pyrimidin-2-yl)bic yclo[3.1.0]hexane-3-carboxamide

Trimethylaluminum (2.0 M n-hexane solution) (2 M, 332.94 µL) was added to a solution of 2-amino-5-(6-ethoxypyrazin-2-yl)benzonitrile 86b (48.88 mg, 203.46 µmol) in toluene (6.98 mL). The mixture was stirred at room temperature for 1 hour, then ethyl 3-(4-(cyclopropanesulfonamido)pyrimidin-2-yl)bicyclo[3.1.0]hexane-3-carboxylate 15d (65 mg, 184.96 µmol) was added, and the mixture was heated to 90°C with stirring for 5 hours. A small amount of methanol was added to quench the reaction, and the solvent was directly evaporated to dryness by rotation to obtain a crude product. Dilute hydrochloric acid and dichloromethane were added, and the insoluble solid was removed by filtration. The crude product was separated by preparative liquid chromatography (separation column AKZONOBEL Kromasil; 250×21.2 mm I.D.; 5 µm, 20 mL/min; mobile phase A: 0.05% TFA + H2O, mobile phase B: CH3CN) to obtain N-(2-cyano-4-(6-ethoxypyrazin-2-yl)phenyl)-3-(4-(cyclopropanesulfonamido)pyrimidin-2-yl)bic yclo[3.1.0]hexane-3-carboxamide 86 (8 mg) with a yield of 6.25%.

MS m/z (ESI): 546.1 [M+1]
1H NMR (400 MHz, DMSO-d6) δ 11.27 (s, 1H), 9.88 (s, 1H), 8.88 (s, 1H), 8.56-8.46 (m, 2H), 8.39 (dd, J=8.7, 2.2 Hz, 1H), 8.27 (s, 1H), 7.67 (d, J=8.7 Hz, 1H), 6.85 (d, J=5.7 Hz, 1H), 4.50 (q, J=7.0 Hz, 2H), 3.16-3.07 (m, 1H), 2.83 (d, J=13.6 Hz, 2H), 2.64 (dd, J=13.4, 3.4 Hz, 2H), 1.40 (t, J=7.0 Hz, 5H), 1.14 (q, J=4.8, 4.0 Hz, 2H), 1.03 (dt, J=7.7, 3.5 Hz, 2H), 0.39 (dp, J=13.5, 5.2, 4.8 Hz, 2H).

### Example 87

### 3-(4-(cyclopropanesulfonamido)pyrimidin-2-yl)-N-(4-(6-ethoxypyrazin-2-yl)-2,6-difluorophenyl) bicyclo[3.1.0]hexane-3-carboxamide

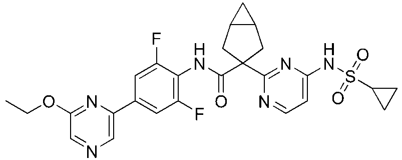

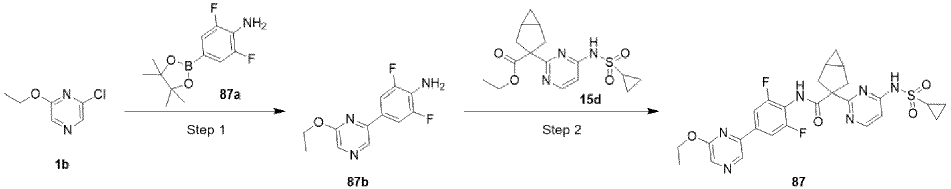

### Step 1

### 4-(6-ethoxypyrazin-2-yl)-2,6-difluoro-aniline

2-Chloro-6-ethoxypyrazine **1b** (404.13 mg, 2.55 mmol), 2,6-difluoro-4-(4,4,5,5-tetramethyl-1,3,2-dioxaborolan-2-yl)aniline 87a (500 mg, 1.96 mmol, commercially available), [1,1'-bis(diphenylphosphino)ferrocene]palladium(II) dichloride (143.43 mg, 196.03 µmol), and potassium carbonate (812.78 mg, 5.88 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 4-(6-ethoxypyrazin-2-yl)-2,6-difluoro-aniline 87b (150 mg) with a yield of 30.46%.

MS m/z (ESI): 251.1 [M+1]

### Step 2

### 3-(4-(cyclopropanesulfonamido)pyrimidin-2-yl)-N-(4-(6-ethoxypyrazin-2-yl)-2,6-difluorophenyl) bicyclo[3.1.0]hexane-3-carboxamide

Trimethylaluminum (2.0 M n-hexane solution) (2 M, 332.94 µL) was added to a solution of 4-(6-ethoxypyrazin-2-yl)-2,6-difluoro-aniline 87b (51.12 mg, 203.46 µmol) in toluene (6.98 mL). The mixture was stirred at room temperature for 1 hour, then ethyl 3-(4-(cyclopropanesulfonamido)pyrimidin-2-yl)bicyclo[3.1.0]hexane-3-carboxylate 15d (65 mg, 184.96 µmol) was added, and the mixture was heated to 90°C with stirring for 5 hours. A small amount of methanol was added to quench the reaction, and the solvent was directly evaporated to dryness by rotation to obtain a crude product. Dilute hydrochloric acid and dichloromethane were added, and the insoluble solid was removed by filtration. The organic solvent was removed from the filtrate, and the residue was separated by preparative liquid chromatography (separation column AKZONOBEL Kromasil; 250×21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2,6-difluorophenyl) bicyclo[3.1.0]hexane-3-carboxamide 87 (5 mg) with a yield of 3.93%.

MS m/z (ESI): 535.1 [M+1]
1H NMR (400 MHz, DMSO-d6) δ 11.26 (s, 1H), 9.33 (s, 1H), 8.88 (s, 1H), 8.29 (s, 1H), 7.87 (d, J=9.6 Hz, 2H), 6.88 (s, 1H), 4.49 (s, 2H), 3.17 (s, 1H), 2.85 (d, J=12.3 Hz, 2H), 2.60 (s, 2H), 1.40 (s, 5H), 1.22-0.98 (m, 4H), 0.37 (d, J=24.3 Hz, 2H).

### Example 88

### 3-(4-(cyclopropanesulfonamido)pyrimidin-2-yl)-N-(5-(6-ethoxypyrazin-2-yl)-3-methylpyridin-2-yl)bicyclo[3.1.0]hexane-3-carboxamide

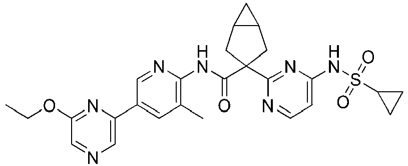

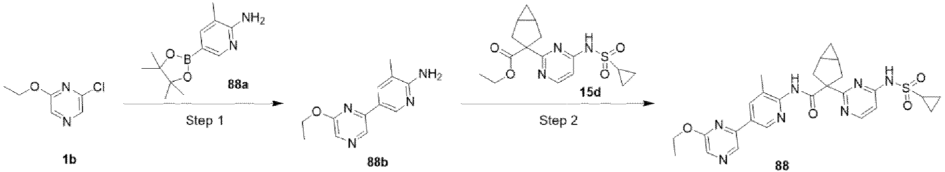

### Step 1

### 4-(6-ethoxypyrazin-2-yl)-2,6-difluoro-aniline

2-Chloro-6-ethoxypyrazine **1b** (404.13 mg, 2.55 mmol), 2,6-difluoro-4-(4,4,5,5-tetramethyl-1,3,2-dioxaborolan-2-yl)aniline 88a (500 mg, 1.96 mmol, commercially available), [1,1'-bis(diphenylphosphino)ferrocene]palladium(II) dichloride (143.43 mg, 196.03 µmol), and potassium carbonate (812.78 mg, 5.88 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 4-(6-ethoxypyrazin-2-yl)-2,6-difluoro-aniline 88b (150 mg) with a yield of 30.46%.

MS m/z (ESI): 251.1 [M+1]

### Step 2

### 3-(4-(cyclopropanesulfonamido)pyrimidin-2-yl)-N-(5-(6-ethoxypyrazin-2-yl)-3-methylpyridin-2-yl)bicyclo[3.1.0]hexane-3-carboxamide

Trimethylaluminum (2.0 M n-hexane solution) (2 M, 332.94 µL) was added to a solution of 4-(6-ethoxypyrazin-2-yl)-2,6-difluoro-aniline 88b (51.12 mg, 203.46 µmol) in toluene (6.98 mL). The mixture was stirred at room temperature for 1 hour. Ethyl 3-(4-(cyclopropanesulfonamido)pyrimidin-2-yl)bicyclo[3.1.0]hexane-3-carboxylate 15d (65 mg, 184.96 µmol) was added, and the mixture was heated to 90°C and stirred for 5 hours. A small amount of methanol was added to quench the reaction, and the solvent was directly evaporated to dryness by rotation to obtain a crude product. Dilute hydrochloric acid and dichloromethane were added, and the insoluble solid was removed by filtration. After removing the organic phase from the filtrate, the crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(5-(6-ethoxypyrazin-2-yl)-3-methylpyridin-2-yl)bicyclo[3.1.0]hexane-3-carboxamide 88 (5 mg) with a yield of 3.93%.

MS m/z (ESI): 535.1 [M+1]
1H NMR (400 MHz, DMSO-d6) δ 9.60 (s, 1H), 8.92 (s, 1H), 8.84 (s, 1H), 8.52 (d, J=5.7 Hz, 1H), 8.30 (d, J=19.1 Hz, 2H), 6.87 (d, J=5.7 Hz, 1H), 4.48 (d, J=7.0 Hz, 2H), 3.23 (s, 1H), 2.86 (d, J=13.6 Hz, 2H), 2.60 (d, J=12.9 Hz, 2H), 2.16 (s, 3H), 1.40 (t, J=6.8 Hz, 5H), 1.13 (d, J=4.6 Hz, 2H), 1.06 (d, J=7.7 Hz, 2H), 0.42 (s, 1H), 0.32 (d, J=4.3 Hz, 1H).

### Example 89

### 3-(4-(cyclopropanesulfonamido)pyrimidin-2-yl)-N-(5-(6-ethoxypyrazin-2-yl)-3-fluoropyridin-2-y l)bicyclo[3.1.0]hexane-3-carboxamide

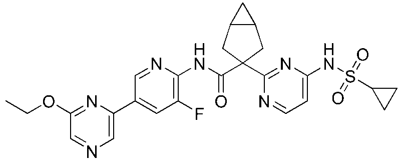

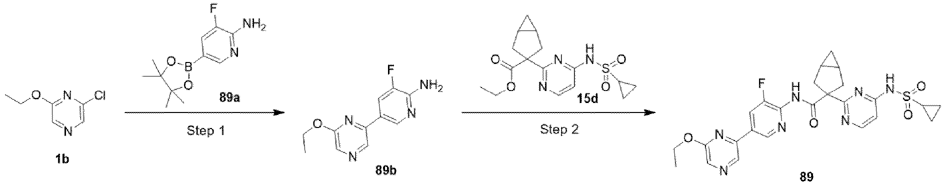

### Step 1

### 5-(6-ethoxypyrazin-2-yl)-3-fluoro-pyridin-2-amine

2-Chloro-6-ethoxypyrazine **1b** (399.68 mg, 2.52 mmol), 3-fluoro-5-(4,4,5,5-tetramethyl-1,3,2-dioxaborolan-2-yl)pyridin-2-amine 89a (500 mg, 2.10 mmol, commercially available), [1,1'-bis(diphenylphosphino)ferrocene]palladium(II) dichloride (153.68 mg, 210.03 µmol), and potassium carbonate (870.83 mg, 6.30 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 5-(6-ethoxypyrazin-2-yl)-3-fluoro-pyridin-2-amine 89b (350 mg) with a yield of 71.15%.

MS m/z (ESI): 251.1 [M+1]

### Step 2

### 3-(4-(cyclopropanesulfonamido)pyrimidin-2-yl)-N-(5-(6-ethoxypyrazin-2-yl)-3-fluoropyridin-2-y l)bicyclo[3.1.0]hexane-3-carboxamide

Trimethylaluminum (2.0 M n-hexane solution) (2 M, 512.21 µL) was added to a solution of 5-(6-ethoxypyrazin-2-yl)-3-fluoro-pyridin-2-amine 89b (79.98 mg, 341.47 µmol) in toluene (3 mL). The mixture was stirred at room temperature for 1 hour. Ethyl 3-(4-(cyclopropanesulfonamido)pyrimidin-2-yl)bicyclo[3.1.0]hexane-3-carboxylate 15d (100 mg, 284.56 µmol) was added, and the mixture was heated to 90°C and stirred for 5 hours. A small amount of methanol was added to quench the reaction, and the solvent was directly evaporated to obtain a crude product. Dilute hydrochloric acid and dichloromethane were added, and the insoluble solid was removed by filtration. The solvent was evaporated to dryness by rotation, and the crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(5-(6-ethoxypyrazin-2-yl)-3-fluoropyridin-2-y l)bicyclo[3.1.0]hexane-3-carboxamide 89 (24 mg) with a yield of 12.49%.

MS m/z (ESI): 540.2 [M+1]
1H NMR (400 MHz, DMSO-d6) δ 11.24 (s, 1H), 9.99 (s, 1H), 8.92 (d, J=13.1 Hz, 2H), 8.51 (d, J=5.7 Hz, 1H), 8.38 (d, J=11.1 Hz, 1H), 8.30 (s, 1H), 6.85 (d, J=5.7 Hz, 1H), 4.50 (q, J=7.0 Hz, 2H), 3.19 (dd, J=9.3, 4.5 Hz, 1H), 2.85 (d, J=13.6 Hz, 2H), 2.58 (d, J=13.9 Hz, 2H), 1.40 (t, J=7.0 Hz, 5H), 1.12 (d, J=4.7 Hz, 2H), 1.05 (d, J=8.2 Hz, 2H), 0.38 (q, J=5.8, 5.1 Hz, 2H).

### Example 90

### 3-(4-(cyclopropanesulfonamido)pyrimidin-2-yl)-N-(4-(6-ethoxypyrazin-2-yl)-2-fluoro-5-methylp henyl)bicyclo[3.1.0]hexane-3-carboxamide

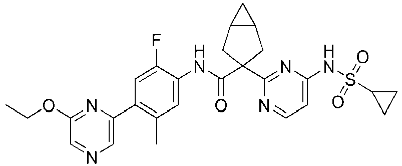

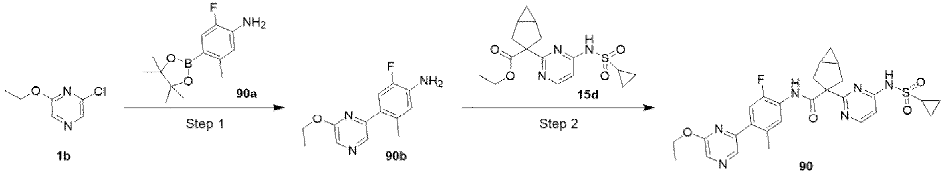

### Step 1

### 4-(6-ethoxypyrazin-2-yl)-2-fluoro-5-methylaniline

2-Chloro-6-ethoxypyrazine **1b** (378.93 mg, 2.39 mmol), 2-fluoro-5-methyl-4-(4,4,5,5-tetramethyl-1,3,2-dioxaborolan-2-yl)aniline 90a (600 mg, 2.39 mmol, prepared according to published patent WO2010002655), [1,1'-bis(diphenylphosphino)ferrocene]palladium(II) dichloride (174.84 mg, 238.94 µmol), and potassium carbonate (990.74 mg, 7.17 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 4-(6-ethoxypyrazin-2-yl)-2-fluoro-5-methylaniline 90b (400 mg) with a yield of 67.70%.

MS m/z (ESI): 248.1 [M+1]

### Step 2

### 3-(4-(cyclopropanesulfonamido)pyrimidin-2-yl)-N-(4-(6-ethoxypyrazin-2-yl)-2-fluoro-5-methylp henyl)bicyclo[3.1.0]hexane-3-carboxamide

Trimethylaluminum (2.0 M n-hexane solution) (2 M, 358.55 µL) was added to a solution of 4-(6-ethoxypyrazin-2-yl)-2-fluoro-5-methylaniline 90b (59.10 mg, 239.03 µmol) in toluene (2.97 mL). The mixture was stirred at room temperature for 1 hour. Ethyl 3-(4-(cyclopropanesulfonamido)pyrimidin-2-yl)bicyclo[3.1.0]hexane-3-carboxylate 15d (70 mg, 199.19 µmol) was added, and the mixture was heated to 90°C and stirred for 5 hours. A small amount of methanol was added to quench the reaction, and the solvent was directly evaporated to dryness by rotation to obtain a crude product. Dilute hydrochloric acid and dichloromethane were added, and the insoluble solid was removed by filtration. The solvent was evaporated to dryness by rotation, and the crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2-fluoro-5-methylp henyl)bicyclo[3.1.0]hexane-3-carboxamide 90 (15 mg) with a yield of 10.87%.

MS m/z (ESI): 553.2 [M+1]
1H NMR (400 MHz, Chloroform-d) δ 8.74 (d, J=3.2 Hz, 1H), 8.45 (d, J=5.7 Hz, 1H), 8.16-8.00 (m, 3H), 7.12 (d, J=11.6 Hz, 1H), 6.97 (d, J=5.7 Hz, 1H), 4.33 (q, J=7.1 Hz, 2H), 2.81 (t, J=13.2 Hz, 3H), 2.64 (dd, J=13.8, 3.4 Hz, 2H), 2.31 (s, 3H), 1.38-1.24 (m, 7H), 1.02 (dt, J=7.9, 3.6 Hz, 2H), 0.44 (d, J=7.0 Hz, 1H), 0.32-0.22 (m, 1H).

### Example 91

### 3-(4-(cyclopropanesulfonamido)pyrimidin-2-yl)-N-(4-(6-ethoxypyrazin-2-yl)-2-(trifluoromethox y)phenyl)bicyclo[3.1.0]hexane-3-carboxamide

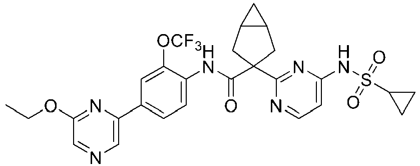

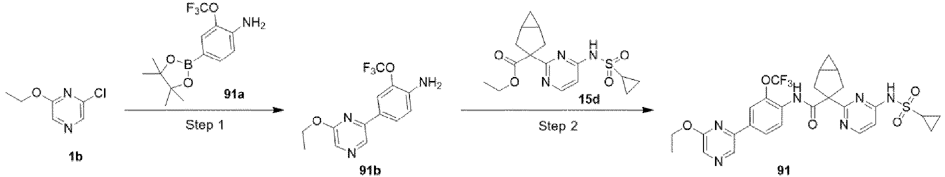

### Step 1

### 4-(6-ethoxypyrazin-2-yl)-2-(trifluoromethoxy)aniline

2-Chloro-6-ethoxypyrazine **1b** (338.71 mg, 2.14 mmol, commercially available), 4-(4,4,5,5-tetramethyl-1,3,2-dioxaborolan-2-yl)-2-(trifluoromethoxy)aniline 91a (647.34 mg, 2.14 mmol, commercially available), [1,1'-bis(diphenylphosphino)ferrocene]palladium(II) dichloride (156.28 mg, 213.58 µmol), and potassium carbonate (885.58 mg, 6.41 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 4-(6-ethoxypyrazin-2-yl)-2-(trifluoromethoxy)aniline 91b (250 mg) with a yield of 39.11%.

MS m/z (ESI): 300.1 [M+1]

### Step 2

### 3-(4-(cyclopropanesulfonamido)pyrimidin-2-yl)-N-(4-(6-ethoxypyrazin-2-yl)-2-(trifluoromethox y)phenyl)bicyclo[3.1.0]hexane-3-carboxamide

Trimethylaluminum (2.0 M n-hexane solution) (2 M, 358.55 µL) was added to a solution of 4-(6-ethoxypyrazin-2-yl)-2-(trifluoromethoxy)aniline 91b (71.53 mg, 239.03 µmol) in toluene (2.97 mL). The mixture was stirred at room temperature for 1 hour. Ethyl 3-(4-(cyclopropanesulfonamido)pyrimidin-2-yl)bicyclo[3.1.0]hexane-3-carboxylate 15d (70 mg, 199.19 µmol) was added, and the mixture was heated to 90°C and stirred for 5 hours. A small amount of methanol was added to quench the reaction, and the solvent was directly evaporated to obtain a crude product. Dilute hydrochloric acid and dichloromethane were added, and the insoluble solid was removed by filtration. The solvent was evaporated to dryness by rotation, and the crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2-(trifluoromethox y)phenyl)bicyclo[3.1.0]hexane-3-carboxamide 91 (45 mg) with a yield of 31.01%.

MS m/z (ESI): 605.2 [M+1]
1H NMR (400 MHz, DMSO-d6) δ 11.06 (s, 1H), 9.19 (s, 1H), 8.60 (s, 1H), 8.26 (d, J=5.8 Hz, 1H), 8.01 (s, 1H), 7.91-7.78 (m, 2H), 7.66 (d, J=8.5 Hz, 1H), 6.61 (d, J=5.7 Hz, 1H), 4.23 (q, J=7.0 Hz, 2H), 2.90 (dd, J=8.4, 4.0 Hz, 1H), 2.56 (d, J=13.6 Hz, 2H), 2.38 (d, J=13.3 Hz, 2H), 1.16 (t, J=6.9 Hz, 5H), 0.89 (q, J=4.7, 4.0 Hz, 2H), 0.79 (dt, J=7.8, 3.4 Hz, 2H), 0.16 (d, J=6.5 Hz, 1H), 0.04--0.07 (m, 1H).

### Example 92

### 3-(4-(cyclopropanesulfonamido)pyrimidin-2-yl)-N-(4-(6-ethoxypyrazin-2-yl)-2,3-dimethylphenyl )bicyclo[3.1.0]hexane-3-carboxamide

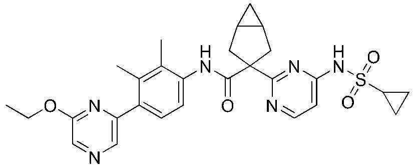

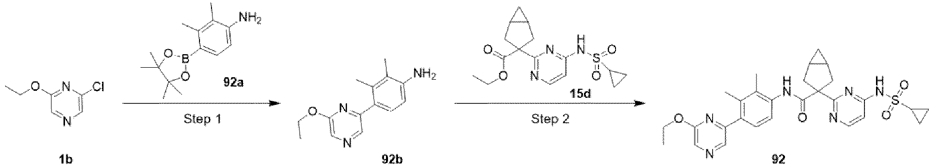

### Step 1

### 4-(6-ethoxypyrazin-2-yl)-2,3-dimethyl-aniline

2-Chloro-6-ethoxypyrazine **1b** (378.93 mg, 2.39 mmol), 2,3-dimethyl-4-(4,4,5,5-tetramethyl-1,3,2-dioxaborolan-2-yl)aniline 92a (590.53 mg, 2.39 mmol, prepared according to the published literature "ACS Chemical Biology (2016), 11(8), 2195-2205", [1,1'-bis(diphenylphosphino)ferrocene]palladium(II) dichloride (174.84 mg, 238.94 µmol), and potassium carbonate (990.74 mg, 7.17 mmol) were dissolved in 1,4-dioxane (8 mL) and water (2 mL). The mixture was subjected to argon exchange three times and heated to 95°C with stirring for 3 hours. The solvent was evaporated, and the crude product was separated by silica gel column chromatography (eluent: System A) to obtain 4-(6-ethoxypyrazin-2-yl)-2,3-dimethylaniline 92b (240 mg) with a yield of 41.28%.

MS m/z (ESI): 241.1 [M+1]

### Step 2

### 3-(4-(cyclopropanesulfonamido)pyrimidin-2-yl)-N-(4-(6-ethoxypyrazin-2-yl)-2,3-dimethylphenyl )bicyclo[3.1.0]hexane-3-carboxamide

Trimethylaluminum (2.0 M n-hexane solution) (2 M, 358.55 µL) was added to a solution of 4-(6-ethoxypyrazin-2-yl)-2,3-dimethylaniline 92b (58.16 mg, 239.03 µmol) in toluene (2.97 mL). The mixture was stirred at room temperature for 1 hour. Ethyl 3-(4-(cyclopropanesulfonamido)pyrimidin-2-yl)bicyclo[3.1.0]hexane-3-carboxylate 15d (70 mg, 199.19 µmol) was added, and the mixture was heated to 90°C and stirred for 5 hours. A small amount of methanol was added to quench the reaction, and the solvent was directly evaporated to dryness by rotation to obtain a crude product. Dilute hydrochloric acid and dichloromethane were added, and the insoluble solid was removed by filtration. The solvent was evaporated to dryness by rotation, and the crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)-2,3-dimethylphenyl )bicyclo[3.1.0]hexane-3-carboxamide 92 (15 mg) with a yield of 9.92%.

MS m/z (ESI): 549.2 [M+1]
1H NMR (400 MHz, DMSO-d6) δ 9.06 (s, 1H), 8.52 (d, J=5.7 Hz, 1H), 8.24 (d, J=6.2 Hz, 2H), 7.24-7.08 (m, 2H), 6.87 (d, J=5.7 Hz, 1H), 4.36 (q, J=7.0 Hz, 2H), 3.18 (t, J=4.3 Hz, 1H), 2.86 (d, J=13.5 Hz, 2H), 2.59 (d, J=14.3 Hz, 2H), 2.20 (s, 3H), 2.01 (s, 3H), 1.36 (dt, J=14.0, 6.0 Hz, 5H), 1.15 (p, J=4.5 Hz, 2H), 1.10-1.01 (m, 2H), 0.41 (d, J=6.6 Hz, 1H), 0.28 (d, J=4.2 Hz, 1H).

### Example 93

### N-(3-(2-(cyclopropanesulfonamido)pyrimidin-4-yl)bicyclo[3.1.0]hexan-3-yl)-4-(6-ethoxypyrazin -2-yl)benzamide

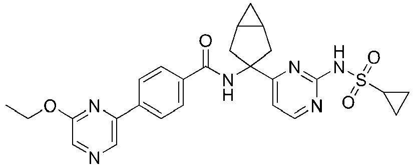

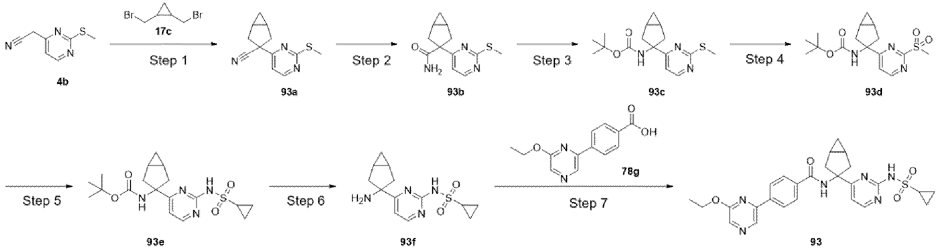

### Step 1

### 3-(2-(methylthio)pyrimidin-4-yl)bicyclo[3.1.0]hexane-3-carbonitrile

2-(2-(Methylthio)pyrimidin-4-yl)acetonitrile 4b (540 mg, 3.27 mmol) was added to N,N-dimethylformamide (10 mL). The mixture was subjected to argon exchange three times. Under cooling with an ice bath, sodium hydroxide (300.70 mg, 7.52 mmol) was slowly added. After stirring for 0.5 hours, 1,2-bis(bromomethyl)cyclopropane **17c** (931.20 mg, 3.27 mmol) was added. The mixture was stirred at room temperature for 23 hours. Water (100 mL) was added, and the mixture was extracted with ethyl acetate (100 mL × 3). The combined organic phase was washed with saturated brine (100 mL), dried over anhydrous sodium sulfate, and concentrated under reduced pressure. The resulting residue was purified by silica gel column chromatography (eluent: System A) to obtain 3-(2-(methylthio)pyrimidin-4-yl)bicyclo[3.1.0]hexane-3-carbonitrile **93a** (300 mg) with a yield of 40%.

MS m/z (ESI): 232.0 [M+1]

### Step 2

### 3-(2-(methylthio)pyrimidin-4-yl)bicyclo[3.1.0]hexane-3-carboxamide

3-(2-(Methylthio)pyrimidin-4-yl)bicyclo[3.1.0]hexane-3-carbonitrile **93a** (300 mg, 1.30 mmol) and 2.5 M aqueous sodium hydroxide solution (1 mL) were sequentially added to dimethyl sulfoxide (2 mL). Hydrogen peroxide (0.5 mL) was added dropwise under an ice bath, and the mixture was stirred at room temperature for 2 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL × 2), dried over anhydrous sodium sulfate, and concentrated under reduced pressure. The residue was purified by silica gel column chromatography (eluent: System A) to obtain 3-(2-(methylthio)pyrimidin-4-yl)bicyclo[3.1.0]hexane-3-carboxamide 93b (290 mg) with a yield of 89%.

MS m/z (ESI): 250.0 [M+1]

### Step 3

### tert-butyl (3-(2-(methylthio)pyrimidin-4-yl)bicyclo[3.1.0]hexan-3-yl)carbamate

3-(2-(Methylthio)pyrimidin-4-yl)bicyclo[3.1.0]hexane-3-carboxamide **93b** (500 mg, 2.01 mmol) and lead(IV) acetate (3.56 g, 8.02 mmol) were sequentially added to tert-butanol (5 mL). The mixture was subjected to argon exchange three times and heated to 100°C with continuous stirring for 7 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL × 2), dried over anhydrous sodium sulfate, and concentrated under reduced pressure. The residue was purified by silica gel column chromatography (eluent: System A) to obtain tert-butyl (3-(2-(methylthio)pyrimidin-4-yl)bicyclo[3.1.0]hexan-3-yl)carbamate **93c** (220 mg) with a yield of 34%.

MS m/z (ESI): 322.1 [M+1]

### Step 4

### tert-butyl (3-(2-(methylsulfonyl)pyrimidin-4-yl)bicyclo[3.1.0]hexan-3-yl)carbamate

Tert-butyl (3-(2-(methylthio)pyrimidin-4-yl)bicyclo[3.1.0]hexan-3-yl)carbamate **93c** (220 mg, 684.43 µmol) and meta-chloroperoxybenzoic acid (250.12 mg, 1.23 mmol) were sequentially added to dichloromethane (3 mL). The mixture was stirred continuously at room temperature for 2 hours. An aqueous sodium sulfite solution was added to quench the reaction. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure to obtain crude tert-butyl (3-(2-(methylsulfonyl)pyrimidin-4-yl)bicyclo[3.1.0]hexan-3-yl)carbamate **93d** (240 mg). The crude product was directly used in the next step.

MS m/z (ESI): 354.0 [M+1]

### Step 5

### tert-butyl (3-(2-(cyclopropanesulfonamido)pyrimidin-4-yl)bicyclo[3.1.0]hexan-3-yl)carbamate

tert-butyl (3-(2-(methylsulfonyl)pyrimidin-4-yl)bicyclo[3.1.0]hexan-3-yl)carbamate 93d (240 mg, 679.05 µmol), cyclopropanesulfonyl amine (98.73 mg, 814.86 µmol), and cesium carbonate (442.50 mg, 1.36 mmol) were sequentially added to N-methylpyrrolidone (5 mL). The mixture was heated to 100°C with continuous stirring for 2 hours and concentrated to dryness under reduced pressure. The resulting residue was purified by silica gel column chromatography (eluent: System A) to obtain tert-butyl (3-(2-(cyclopropanesulfonamido)pyrimidin-4-yl)bicyclo[3.1.0]hexan-3-yl)carbamate **93e** (200 mg) with a yield of 75%.

MS m/z (ESI): 395.0 [M+1]

### Step 6

### N-(4-(3-aminobicyclo[3.1.0]hexan-3-yl)pyrimidin-2-yl)cyclopropanesulfonamide

tert-butyl (3-(2-(cyclopropanesulfonamido)pyrimidin-4-yl)bicyclo[3.1.0]hexan-3-yl)carbamate **93e** (200 mg, 506.99 µmol) was dissolved in dichloromethane (2 mL). 1 M hydrochloric acid in 1,4-dioxane solution (0.5 mL) was added, and the mixture was stirred continuously at room temperature for 2 hours. The mixture was concentrated to dryness under reduced pressure to obtain N-(4-(3-aminobicyclo[3.1.0]hexan-3-yl)pyrimidin-2-yl)cyclopropanesulfonamide **93f** (140 mg) with a yield of 94%.

MS m/z (ESI): 295.0 [M+1]

### Step 7

### N-(3-(2-(cyclopropanesulfonamido)pyrimidin-4-yl)bicyclo[3.1.0]hexan-3-yl)-4-(6-ethoxypyrazin -2-yl)benzamide

4-(6-Ethoxypyrazin-2-yl)benzoic acid **78g** (50 mg, 204 µmol), N-(4-(3-aminobicyclo[3.1.0]hexan-3-yl)pyrimidin-2-yl)cyclopropanesulfonamide **93f** (60 mg, 204 µmol), O-(7-azabenzotriazol-1-yl)-N,N,N',N'-tetramethyluronium hexafluorophosphate (93.4 mg, 245.6 µmol), and N,N-diisopropylethylamine (110 µL) were sequentially added to dichloromethane (3 mL). The mixture was stirred at room temperature for 2 hours and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(2-(cyclopropanesulfonamido)pyrimidin-4-yl)bicyclo[3.1.0]hexan-3-yl)-4-(6-ethoxypyrazin -2-yl)benzamide **93** (21.0 mg) with a yield of 20%.

MS m/z (ESI): 521.2 [M+1]
1H NMR (400 MHz, DMSO-d6) δ 8.89 (d, J=2.0 Hz, 1H), 8.73 (s, 1H), 8.48-8.42 (m, 1H), 8.29 (s, 1H), 8.23 (d, J=8.0 Hz, 2H), 8.00 (dd, J=15.9, 8.2 Hz, 2H), 7.01 (dd, J=21.4, 5.3 Hz, 1H), 4.50 (q, J=7.0 Hz, 2H), 3.22 (dd, J=13.5, 5.4 Hz, 1H), 2.65 (dd, J=13.2, 3.3 Hz, 2H), 2.45 (s, 1H), 2.22 (d, J=14.1 Hz, 1H), 1.49 (dq, J=12.6, 5.1, 4.0 Hz, 2H), 1.41 (t, J=7.0 Hz, 3H), 1.12 (td, J=7.8, 4.3 Hz, 2H), 0.99 (qd, J=7.4, 4.8 Hz, 2H), 0.81 (td, J=8.1, 4.4 Hz, 1H), 0.61 (ddt, J=21.2, 8.2, 4.6 Hz, 1H).

### Example 94

### N-(3-(4-(cyclopropanesulfonamido)pyrimidin-2-yl)bicyclo[3.1.0]hexan-3-yl)-4-(6-ethoxypyrazin -2-yl)benzamide

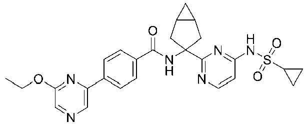

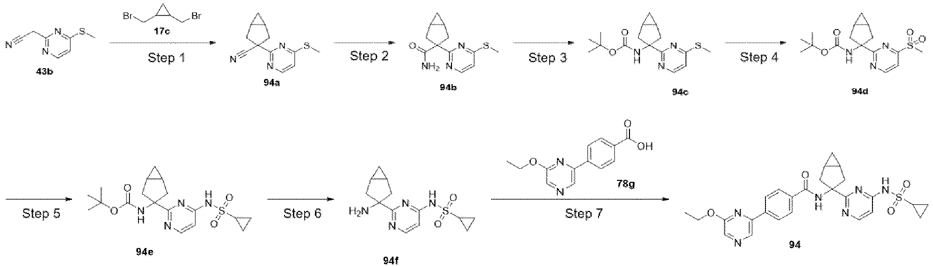

### Step 1

### 3-(4-(methylthio)pyrimidin-2-yl)bicyclo[3.1.0]hexane-3-carbonitrile

2-(4-(Methylthio)pyrimidin-2-yl)acetonitrile **43b** (250 mg, 1.51 mmol) was added to N,N-dimethylformamide (10 mL). The mixture was subjected to argon exchange three times. Under cooling with an ice bath, sodium hydroxide (139.21 mg, 3.48 mmol) was slowly added. After stirring for 0.5 hours, 1,2-bis(bromomethyl)cyclopropane (700.00 mg, 2.46 mmol) was added. The mixture was stirred at room temperature for 23 hours. Water (100 mL) was added, and the mixture was extracted with ethyl acetate (100 mL × 3). The combined organic phase was washed with saturated brine (100 mL), dried over anhydrous sodium sulfate, and concentrated under reduced pressure. The resulting residue was purified by silica gel column chromatography (eluent: System A) to obtain 3-(4-(methylthio)pyrimidin-2-yl)bicyclo[3.1.0]hexane-3-carbonitrile **94a** (140 mg) with a yield of 40%.

MS m/z (ESI): 232.0 [M+1]

### Step 2

### 3-(4-(methylthio)pyrimidin-2-yl)bicyclo[3.1.0]hexane-3-carboxamide

3-(4-(Methylthio)pyrimidin-2-yl)bicyclo[3.1.0]hexane-3-carbonitrile **94a** (140 mg, 605.23 µmol) and 2.5 M aqueous sodium hydroxide solution (1 mL) were sequentially added to dimethyl sulfoxide (2 mL). Under an ice bath, hydrogen peroxide (0.5 mL) was added dropwise. The mixture was stirred at room temperature for 2 hours. Ethyl acetate (30 mL) and water (15 mL) were added, and the mixture was partitioned. The aqueous phase was extracted with ethyl acetate (30 mL × 3). The combined organic phase was washed with saturated sodium chloride solution (30 mL × 2), dried over anhydrous sodium sulfate, and concentrated under reduced pressure. The residue was purified by silica gel column chromatography (eluent: System A) to obtain 3-(4-(methylthio)pyrimidin-2-yl)bicyclo[3.1.0]hexane-3-carboxamide **94b** (120 mg) with a yield of 79%.

MS m/z (ESI): 250.0 [M+1]

### Step 3

### tert-butyl (3-(4-(methylthio)pyrimidin-2-yl)bicyclo[3.1.0]hexan-3-yl)carbamate

3-(4-(Methylthio)pyrimidin-2-yl)bicyclo[3.1.0]hexane-3-carboxamide **94b** (120 mg, 481.29 µmol) and lead tetraacetate (853.57 mg, 1.93 mmol) were sequentially added to tert-butanol (5 mL). The mixture was subjected to argon exchange 3 times and heated to 100°C with stirring for 7 hours. Ethyl acetate (30 mL) and water (15 mL) were added, and the mixture was partitioned. The aqueous phase was extracted with ethyl acetate (30 mL × 3). The combined organic phase was washed with saturated sodium chloride solution (30 mL × 2), dried over anhydrous sodium sulfate. The filtrate was concentrated under reduced pressure. The resulting residue was purified by silica gel column chromatography (eluent: System A) to obtain tert-butyl (3-(4-(methylthio)pyrimidin-2-yl)bicyclo[3.1.0]hexan-3-yl)carbamate **94c** (83 mg) with a yield of 53%.

MS m/z (ESI): 322.1 [M+1]

### Step 4

### tert-butyl (3-(4-(methylsulfonyl)pyrimidin-2-yl)bicyclo[3.1.0]hexan-3-yl)carbamate

Tert-Butyl (3-(4-(methylthio)pyrimidin-2-yl)bicyclo[3.1.0]hexan-3-yl)carbamate **94c** (83 mg, 258.22 µmol) and meta-chloroperoxybenzoic acid (94 mg, 464.79 µmol) were sequentially added to dichloromethane (3 mL). The mixture was stirred at room temperature for 2 hours. An aqueous sodium sulfite solution was added to quench the reaction. Ethyl acetate (30 mL) and water (15 mL) were added, and the mixture was partitioned. The aqueous phase was extracted with ethyl acetate (30 mL × 3). The combined organic phase was washed with saturated sodium chloride solution (30 mL × 2), dried over anhydrous sodium sulfate, filtered, and concentrated to dryness under reduced pressure to obtain crude tert-butyl (3-(4-(methylsulfonyl)pyrimidin-2-yl)bicyclo[3.1.0]hexan-3-yl)carbamate 94d (90 mg). The crude product was directly used in the next step.

MS m/z (ESI): 354.0 [M+1]

### Step 5

### tert-butyl (3-(4-(cyclopropanesulfonamido)pyrimidin-2-yl)bicyclo[3.1.0]hexan-3-yl)carbamate

Tert-butyl (3-(4-(methylsulfonyl)pyrimidin-2-yl)bicyclo[3.1.0]hexan-3-yl)carbamate 94d (90 mg, 254.64 µmol), cyclopropanesulfonamide (37.02 mg, 305.57 µmol), and cesium carbonate (165.94 mg, 509.29 µmol) were sequentially added to N-methylpyrrolidone (2 mL). The mixture was heated to 100°C with stirring for 2 hours and concentrated to dryness under reduced pressure. The resulting residue was purified by silica gel column chromatography (eluent: System A) to obtain tert-butyl (3-(4-(cyclopropanesulfonamido)pyrimidin-2-yl)bicyclo[3.1.0]hexan-3-yl)carbamate 94e (65 mg) with a yield of 65%.

MS m/z (ESI): 395.2 [M+1]

### Step 6

### N-(2-(3-aminobicyclo[3.1.0]hexan-3-yl)pyrimidin-4-yl)cyclopropanesulfonamide

Tert-butyl (3-(4-(cyclopropanesulfonamido)pyrimidin-2-yl)bicyclo[3.1.0]hexan-3-yl)carbamate **94e** (65 mg, 164.77 µmol) was dissolved in dichloromethane (2 mL). 1M hydrochloric acid dioxane solution (0.5 mL) was added, and the mixture was stirred continuously at room temperature for 2 hours. The mixture was concentrated to dryness under reduced pressure to obtain crude N-(2-(3-aminobicyclo[3.1.0]hexan-3-yl)pyrimidin-4-yl)cyclopropanesulfonamide **94f** (48 mg) with a yield of 98%.

MS m/z (ESI): 295.0 [M+1]

### Step 7

### N-(3-(4-(cyclopropanesulfonamido)pyrimidin-2-yl)bicyclo[3.1.0]hexan-3-yl)-4-(6-ethoxypyrazin -2-yl)benzamide

4-(6-Ethoxypyrazin-2-yl)benzoic acid **78g** (40 mg, 163.77 µmol), N-(2-(3-aminobicyclo[3.1.0]hexan-3-yl)pyrimidin-4-yl)cyclopropanesulfonamide **94f** (48.21 mg, 163.77 µmol), O-(7-azabenzotriazol-1-yl)-N,N,N',N'-tetramethyluronium hexafluorophosphate (74.72 mg, 196.52 µmol), and N,N-diisopropylethylamine (88 µL) were sequentially added to dichloromethane (2 mL). The mixture was stirred continuously at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(4-(cyclopropanesulfonamido)pyrimidin-2-yl)bicyclo[3.1.0]hexan-3-yl)-4-(6-ethoxypyrazin -2-yl)benzamide 94 (1.6 mg) with a yield of 1.8%.

MS m/z (ESI): 521.2 [M+1]
1H NMR (400 MHz, DMSO-d6) δ 8.89 (s, 1H), 8.63 (s, 1H), 8.42 (d, J=5.8 Hz, 1H), 8.29 (s, 1H), 8.21(d, J=8.1 Hz, 2H), 7.99 (d, J=8.2 Hz, 2H), 6.75 (d, J=5.9 Hz, 1H), 4.51 (q, J=7.0 Hz, 2H), 3.08 (s,1H), 2.75 (dd, J=14.0, 3.6 Hz, 2H), 2.48-2.34 (m, 2H), 1.44 (dt, J=14.1, 5.6 Hz, 5H), 1.02 (dt, J=6.9,3.5 Hz, 2H), 0.81-0.74 (m, 2H), 0.68 (q, J=4.2 Hz, 1H), 0.56 (td, J=8.2, 4.6 Hz, 1H).

### Example 95

### N-(3-(6-(cyclopropanesulfonamido)pyrimidin-4-yl)bicyclo[3.1.0]hexan-3-yl)-5-(6-ethoxypyrazin -2-yl)thiazole-2-carboxamide

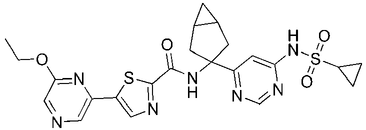

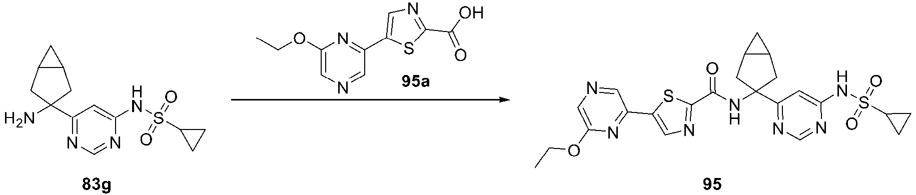

5-(6-Ethoxypyrazin-2-yl)thiazole-2-carboxylic acid **95a** (50 mg, 199.00 µmol, prepared according to published patent WO2022087634), N-(6-(3-aminobicyclo[3.1.0]hexan-3-yl)pyrimidin-4-yl)cyclopropanesulfonamide **83g** (58.6 mg, 199.00 µmol), O-(7-azabenzotriazol-1-yl)-N,N,N',N'-tetramethyluronium hexafluorophosphate (90.80 mg, 238.8 µmol), and N,N-diisopropylethylamine (107 µL) were sequentially added to dichloromethane (3 mL). The mixture was stirred continuously at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-ethoxypyrazin -2-yl)thiazole-2-carboxamide **95** (16.7 mg) with a yield of 15.7%.

MS m/z (ESI): 528.2 [M+1]
1H NMR (400 MHz, DMSO-d6) δ 9.05 (s, 1H), 8.92 (s, 1H), 8.79 (d, J=15.9 Hz, 2H), 8.28 (s, 1H), 7.00(s, 1H), 4.42 (q, J=7.1 Hz, 2H), 3.03 (d, J=8.8 Hz, 1H), 2.59 - 2.51(m, 4H), 1.54-1.33 (m, 5H), 1.12 -0.94 (m, 4H), 0.66-0.47 (m, 2H).

### Example 96

### 3-(6-(cyclopropanesulfonamido)pyrimidin-4-yl)-N-(4-(5-ethoxypyrazolo[1,5-a]pyridin-3-yl)phen yl)bicyclo[3.1.0]hexane-3-carboxamide

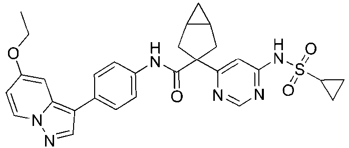

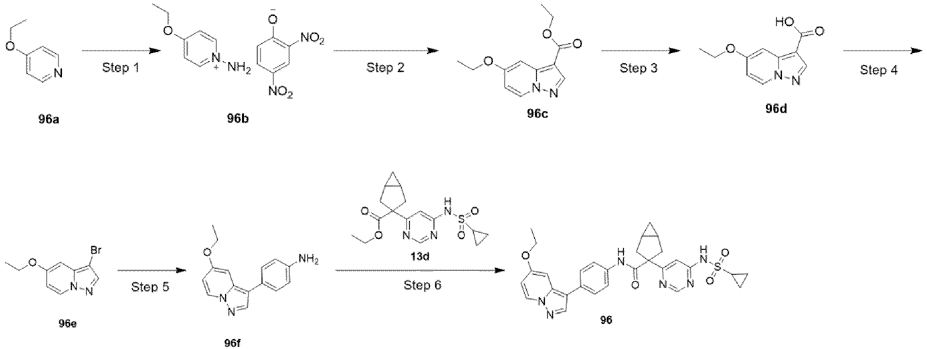

### Step 1

### 1-amino-4-ethoxypyridin-1-ium 2,4-dinitrophenolate

4-Ethoxypyridine 96a (2 g, 16.24 mmol, commercially available) and O-(2,4-dinitrophenyl)hydroxylamine (3.56 g, 17.86 mmol) were dissolved in acetonitrile (40 mL). The mixture was heated to 70°C and stirred for 4 hours. The reaction mixture was concentrated, then washed with methyl tert-butyl ether (100 mL). The solid was collected by filtration and dried under vacuum to obtain 1-amino-4-ethoxypyridin-1-ium 2,4-dinitrophenolate 96b (5.1 g) with a yield of 97.34%.

MS m/z (ESI): 139.1 [M+1]

### Step 2

### ethyl 5-ethoxypyrazolo[1,5-a]pyridine-3-carboxylate

1-Amino-4-ethoxypyridin-1-ium 2,4-dinitrophenolate **96b** (5.1 g, 15.81 mmol), ethyl propiolate (1.55 g, 15.81 mmol), and potassium carbonate (4.37 g, 31.61 mmol) were dissolved in N,N-dimethylformamide (20 mL) and stirred at room temperature for 18 hours. The solvent was evaporated under vacuum. Water (200 mL) and ethyl acetate (200 mL) were added. Insoluble solid was removed by filtration, and the organic phase was separated. The organic phase was washed with saturated sodium chloride solution, dried over anhydrous sodium sulfate, filtered, and concentrated to dryness. The residue was purified by silica gel column chromatography (eluent: System A) to obtain ethyl 5-ethoxypyrazolo[1,5-a]pyridine-3-carboxylate **96c** (1.2 g) with a yield of 32.41%.

MS m/z (ESI): 235.1 [M+1]

### Step 3

### 5-ethoxypyrazolo[1,5-a]pyridine-3-carboxylic acid

Ethyl 5-ethoxypyrazolo[1,5-a]pyridine-3-carboxylate **96c** (1.2 g, 5.12 mmol) and sodium hydroxide (614.73 mg, 15.37 mmol) were dissolved in ethanol (30 mL) and water (15 mL). The mixture was heated to 85°C and stirred for 5 hours. The organic solvent was removed under reduced pressure. The aqueous phase was adjusted to pH 1 by adding 12 N hydrochloric acid. The solid was collected by filtration and dried to obtain 5-ethoxypyrazolo[1,5-a]pyridine-3-carboxylic acid **96d** (1 g) with a yield of 94.67%. The crude product was directly used in the next step.

MS m/z (ESI): 207.1 [M+1]

### Step 4

### 3-bromo-5-ethoxypyrazolo[1,5-a]pyridine

5-Ethoxypyrazolo[1,5-a]pyridine-3-carboxylic acid **96d** (1 g, 4.85 mmol) and N-bromosuccinimide (1.04 g, 5.82 mmol) were dissolved in N,N-dimethylformamide (2 mL) and stirred at room temperature for 1 hour. Water (50 mL) was added to the reaction mixture, and the mixture was extracted with ethyl acetate (50 mL × 3). The combined organic phase was washed with saturated sodium chloride solution, dried over anhydrous sodium sulfate, filtered, and evaporated to dryness by rotation to obtain 3-bromo-5-ethoxypyrazolo[1,5-a]pyridine **96e** (1 g, 2.49 mmol) with a yield of 51.32%. The crude product was directly used in the next step.

MS m/z (ESI): 241.1 [M+1]

### Step 5

### 4-(5-ethoxypyrazolo[1,5-a]pyridin-3-yl)aniline

3-Bromo-5-ethoxypyrazolo[1,5-a]pyridine **96e** (500 mg, 2.07 mmol), 4-(4,4,5,5-tetramethyl-1,3,2-dioxaborolan-2-yl)aniline (499.82 mg, 2.28 mmol), [1,1'-bis(diphenylphosphino)ferrocene]palladium(II) dichloride (151.75 mg, 207.40 µmol), and potassium carbonate (859.93 mg, 6.22 mmol) were dissolved in a mixed solution of 1,4-dioxane (8 mL) and water (2 mL). The mixture was subjected to argon exchange three times and heated to 95°C with stirring for 3 hours. The solvent was removed under reduced pressure. The crude product was purified by silica gel column chromatography (eluent: System A) to obtain 4-(5-ethoxypyrazolo[1,5-a]pyridin-3-yl)aniline **96f** (450 mg, 1.78 mmol) with a yield of 85.66%.

MS m/z (ESI): 254.1 [M+1]

### Step 6

### 3-(6-(cyclopropanesulfonamido)pyrimidin-4-yl)-N-(4-(5-ethoxypyrazolo[1,5-a]pyridin-3-yl)phen yl)bicyclo[3.1.0]hexane-3-carboxamide

Trimethylaluminum (2.0 M n-hexane solution) (2 M, 512.21 µL) was added to a solution of 4-(5-ethoxypyrazolo[1,5-a]pyridin-3-yl)aniline **96f** (79.29 mg, 313.02 µmol) in toluene (2 mL) and stirred at room temperature for 1 hour. Ethyl 3-(6-(cyclopropanesulfonamido)pyrimidin-4-yl)bicyclo[3.1.0]hexane-3-carboxylate **13d** (100 mg, 284.56 µmol) was added, and the mixture was heated to 90°C and stirred for 5 hours. A small amount of methanol was added to quench the reaction, and the solvent was evaporated to dryness by rotation. Dilute hydrochloric acid (1 N) was added to adjust the pH to 5-6, and the mixture was stirred for 10 minutes. The mixture was dissolved in a small amount of methanol and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(5-ethoxypyrazolo[1,5-a]pyridin-3-yl)phen yl)bicyclo[3.1.0]hexane-3-carboxamide **96** (16 mg) with a yield of 7.45%.

MS m/z (ESI): 549.2 [M+1]
1H NMR (400 MHz, DMSO-d6) δ 9.46 (s, 1H), 8.64 (s, 1H), 8.42 (d, J = 7.5 Hz, 1H), 8.05 (s, 1H), 7.53 (d, J = 8.3 Hz, 2H), 7.44 (d, J = 8.4 Hz, 2H), 6.96 (s, 1H), 6.79 (s, 1H), 6.47 (d, J = 7.6 Hz, 1H), 4.03 (q, J = 7.2 Hz, 2H), 2.92 (s, 1H), 2.73 (d, J = 13.5 Hz, 2H), 2.27 (d, J = 13.3 Hz, 2H), 1.25 (q, J = 7.4 Hz, 5H), 0.98 - 0.83 (m, 4H), 0.27 (d, J = 6.8 Hz, 1H), 0.05 - -0.08 (m, 1H).

### Example 97

### 3-(6-(cyclopropanesulfonamido)pyrimidin-4-yl)-N-(4-(5-methylpyrazolo[1,5-a]pyridin-3-yl)phen yl)bicyclo[3.1.0]hexane-3-carboxamide

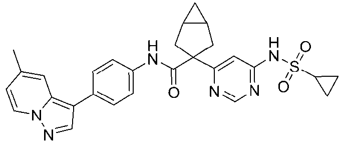

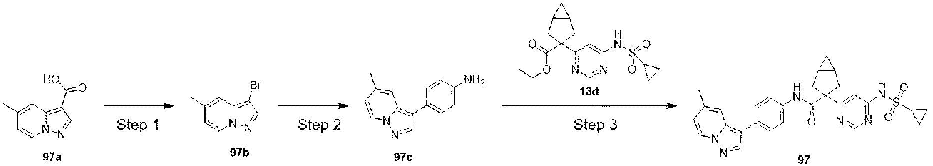

### Step 1

### 3-bromo-5-methylpyrazolo[1,5-a]pyridine

5-Methylpyrazolo[1,5-a]pyridine-3-carboxylic acid **97a** (2 g, 11.35 mmol, prepared according to published patent "WO2009008748") and N-bromosuccinimide (2.42 g, 13.62 mmol) were dissolved in N,N-dimethylformamide (2 mL) and stirred at room temperature for 1 hour. Water (50 mL) was added, and the mixture was extracted with ethyl acetate (50 mL × 3). The combined organic phase was washed with saturated sodium chloride solution, dried over anhydrous sodium sulfate, filtered, and evaporated to dryness by rotation to obtain 3-bromo-5-methylpyrazolo[1,5-a]pyridine **97b** (1.9 g) with a yield of 47.58%.

MS m/z (ESI): 211.1 [M+1]

### Step 2

### 4-(5-methylpyrazolo[1,5-a]pyridin-3-yl)aniline

3-Bromo-5-methylpyrazolo[1,5-a]pyridine **97b** (1.9 g, 9.00 mmol), 4-(4,4,5,5-tetramethyl-1,3,2-dioxaborolan-2-yl)aniline (2.17 g, 9.90 mmol), [1,1'-bis(diphenylphosphino)ferrocene]palladium(II) dichloride (658.70 mg, 900.23 µmol), and potassium carbonate (3.73 g, 27.01 mmol) were dissolved in 1,4-dioxane (8 mL) and water (2 mL). The mixture was subjected to argon exchange three times and heated to 95°C with stirring for 3 hours. The solvent was evaporated to dryness by rotation, and the crude product was separated by silica gel column chromatography (eluent: System A) to obtain 4-(5-methylpyrazolo[1,5-a]pyridin-3-yl)aniline **97c** (1.6 g) with a yield of 79.60%.

MS m/z (ESI): 224.1 [M+1]

### Step 3

### 3-(6-(cyclopropanesulfonamido)pyrimidin-4-yl)-N-(4-(5-methylpyrazolo[1,5-a]pyridin-3-yl)phen yl)bicyclo[3.1.0]hexane-3-carboxamide

Trimethylaluminum (2.0 M n-hexane solution) (2 M, 512.21 µL) was added to a solution of 4-(5-methylpyrazolo[1,5-a]pyridin-3-yl)aniline **97c** (69.89 mg, 313.02 µmol) in toluene (2 mL). The mixture was stirred at room temperature for 1 hour, then ethyl 3-(6-(cyclopropanesulfonamido)pyrimidin-4-yl)bicyclo[3.1.0]hexane-3-carboxylate **13d** (100 mg, 284.56 µmol) was added, and the mixture was heated to 90°C and stirred for 5 hours. A small amount of methanol was added to quench the reaction, and dilute hydrochloric acid (1 N) was added to adjust the pH to 5-6. The mixture was stirred for 10 minutes, then dissolved with a small amount of methanol and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(5-methylpyrazolo[1,5-a]pyridin-3-yl)phen yl)bicyclo[3.1.0]hexane-3-carboxamide **97** (25 mg) with a yield of 11.98%.

MS m/z (ESI): 529.2 [M+1]
1H NMR (400 MHz, DMSO-d6) δ 11.09 (s, 1H), 9.47 (s, 1H), 8.64 (s, 1H), 8.45 (d, J = 7.1 Hz, 1H), 8.11 (s, 1H), 7.63 - 7.51 (m, 3H), 7.46 (d, J = 8.7 Hz, 2H), 6.79 (s, 1H), 6.63 (d, J = 7.1 Hz, 1H), 2.92 (s, 1H), 2.73 (d, J = 13.5 Hz, 2H), 2.27 (d, J = 10.1 Hz, 5H), 1.26 (dd, J = 8.1, 4.0 Hz, 2H), 0.97 - 0.82 (m, 4H), 0.27 (q, J = 7.2 Hz, 1H), -0.01 (d, J = 4.5 Hz, 1H).

### Example 98

### N-(3-(6-(cyclopropanesulfonamido)pyrazin-2-yl)bicyclo[3.1.0]hexan-3-yl)-4-(6-ethoxypyrazin-2 -yl)benzamide

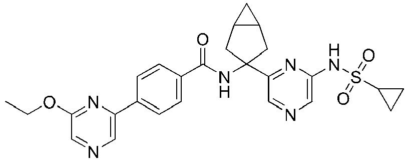

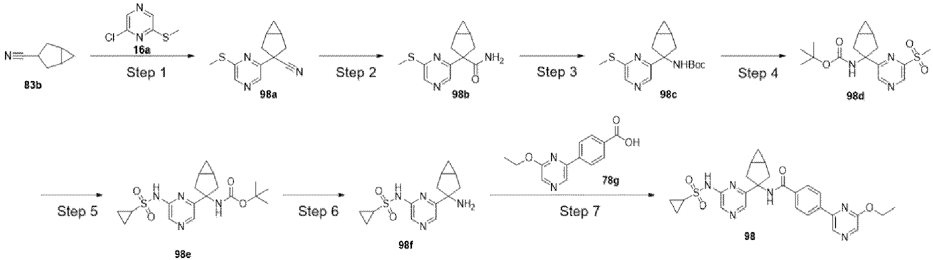

### Step 1

### 3-(6-(methylthio)pyrazin-2-yl)bicyclo[3.1.0]hexane-3-carbonitrile

Bicyclo[3.1.0]hexane-3-carbonitrile **83b** (200 mg, 1.87 mmol) and 2-chloro-6-(methylthio)pyrazine **16a** (299.80 mg, 1.87 mmol) were added to anhydrous tetrahydrofuran (5 mL). The mixture was cooled to -30°C, and lithium bis(trimethylsilyl)amide (5.6 mL, 1 M) was added, then the mixture was heated to room temperature and the reaction was performed for 2 hours. Saturated ammonium chloride solution was added to quench the reaction, and the mixture was extracted with ethyl acetate (100 mL × 3). The organic phase was washed with saturated sodium chloride solution (100 mL × 2), dried over anhydrous sodium sulfate, filtered, and concentrated under reduced pressure. The resulting residue was purified by silica gel column chromatography (eluent: System A) to obtain 3-(6-(methylthio)pyrazin-2-yl)bicyclo[3.1.0]hexane-3-carbonitrile **98a** (300.0 mg) with a yield of 69%.

MS m/z (ESI): 232.1 [M+1]

### Step 2

### 3-(6-(methylthio)pyrazin-2-yl)bicyclo[3.1.0]hexane-3-carboxamide

3-(6-(Methylthio)pyrazin-2-yl)bicyclo[3.1.0]hexane-3-carbonitrile **98a** (300 mg, 1.30 mmol) and aqueous sodium hydroxide solution (1.0 mL, 2.5 M) were sequentially added to dimethyl sulfoxide (2.0 mL). Under an ice bath, hydrogen peroxide (0.5 mL) was added dropwise, and the mixture was stirred at room temperature for 1 hour. Saturated aqueous sodium sulfite solution was added to quench the reaction. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, and concentrated under reduced pressure. The resulting residue was purified by silica gel column chromatography (eluent: System A) to obtain 3-(6-(methylthio)pyrazin-2-yl)bicyclo[3.1.0]hexane-3-carboxamide **98b** (320 mg) with a yield of 98%.

MS m/z (ESI): 250.0 [M+1]

### Step 3

### tert-butyl (3-(6-(methylthio)pyrazin-2-yl)bicyclo[3.1.0]hexan-3-yl)carbamate

3-(6-(Methylthio)pyrazin-2-yl)bicyclo[3.1.0]hexane-3-carboxamide **98b** (320 mg, 1.28 mmol) and lead tetraacetate (2.28 g, 5.13 mmol) were sequentially added to tert-butanol (5 mL). The mixture was subjected to argon exchange three times and heated to 100°C with continuous stirring for 4 hours. Ethyl acetate (30 mL) and water (15 mL) were added, and the mixture was partitioned. The aqueous phase was extracted with ethyl acetate twice (30 mL × 2). The combined organic phase was washed with saturated sodium chloride solution (30 mL × 2), dried over anhydrous sodium sulfate, filtered, and concentrated under reduced pressure to dryness. The residue was purified by silica gel column chromatography (eluent: System A) to obtain tert-butyl (3-(6-(methylthio)pyrazin-2-yl)bicyclo[3.1.0]hexan-3-yl)carbamate **98c** (250 mg) with a yield of 60%.

MS m/z (ESI): 322.1 [M+1]

### Step 4

### tert-butyl (3-(6-(methylsulfonyl)pyrazin-2-yl)bicyclo[3.1.0]hexan-3-yl)carbamate

Tert-butyl (3-(6-(methylthio)pyrazin-2-yl)bicyclo[3.1.0]hexan-3-yl)carbamate **98c** (250 mg, 777.7 µmol) and meta-chloroperoxybenzoic acid (315.81 mg, 1.56 mmol) were sequentially added to dichloromethane (5 mL). The mixture was stirred continuously at room temperature for 2 hours. Aqueous sodium sulfite solution was added to quench the reaction. Ethyl acetate (30 mL) and water (15 mL) were added, and the mixture was partitioned. The aqueous phase was extracted with ethyl acetate twice (30 mL × 2). The combined organic phase was washed with saturated sodium chloride solution (30 mL × 2), dried over anhydrous sodium sulfate, filtered, and concentrated under reduced pressure to dryness to obtain tert-butyl (3-(6-(methylsulfonyl)pyrazin-2-yl)bicyclo[3.1.0]hexan-3-yl)carbamate **98d** (270 mg) with a yield of 98.2%. The product was directly used in the next step.

MS m/z (ESI): 254.0 [M+1-100]

### Step 5

### tert-butyl (3-(6-(cyclopropanesulfonamido)pyrazin-2-yl)bicyclo[3.1.0]hexan-3-yl)carbamate

Tert-butyl (3-(6-(methylsulfonyl)pyrazin-2-yl)bicyclo[3.1.0]hexan-3-yl)carbamate **98d** (270 mg, 763.93 µmol), cyclopropanesulfonamide (138.83 mg, 1.15 mmol), and cesium carbonate (497.81 mg, 1.53 mmol) were sequentially added to N-methylpyrrolidone (2 mL). The mixture was heated to 100°C with continuous stirring for 2 hours and concentrated under reduced pressure to dryness. The resulting residue was purified by silica gel column chromatography (eluent: System A) to obtain tert-butyl (3-(6-(cyclopropanesulfonamido)pyrazin-2-yl)bicyclo[3.1.0]hexan-3-yl)carbamate **98e** (150 mg) with a yield of 49.7%.

MS m/z (ESI): 395.2 [M+1]

### Step 6

### N-(6-(3-aminobicyclo[3.1.0]hexan-3-yl)pyrazin-2-yl)cyclopropanesulfonamide

Tert-butyl (3-(6-(cyclopropanesulfonamido)pyrazin-2-yl)bicyclo[3.1.0]hexan-3-yl)carbamate **98e** (50 mg, 126.75 µmol) was added to a solution of hydrogen chloride in dioxane (0.5 mL, 1 M). The mixture was stirred continuously at room temperature for 1 hour and concentrated under reduced pressure to dryness to obtain N-(6-(3-aminobicyclo[3.1.0]hexan-3-yl)pyrazin-2-yl)cyclopropanesulfonamide **98f** (90 mg) with a yield of 80.4%.

MS m/z (ESI): 295.1 [M+1]

### Step 7

### N-(3-(6-(cyclopropanesulfonamido)pyrazin-2-yl)bicyclo[3.1.0]hexan-3-yl)-4-(6-ethoxypyrazin-2 -yl)benzamide

4-(6-Ethoxypyrazin-2-yl)benzoic acid **78g** (30 mg, 122.83 µmol), N-(6-(3-aminobicyclo[3.1.0]hexan-3-yl)pyrazin-2-yl)cyclopropanesulfonamide **98f** (36.1 mg, 122.8 µmol), O-(7-azabenzotriazol-1-yl)-N,N,N',N'-tetramethyluronium hexafluorophosphate (56.0 mg, 147.4 µmol), and N,N-diisopropylethylamine (66 µL) were sequentially added to dichloromethane (1 mL). The mixture was stirred at room temperature for 2 hours and concentrated under reduced pressure to dryness. The resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrazin-2-yl)bicyclo[3.1.0]hexan-3-yl)-4-(6-ethoxypyrazin-2 -yl)benzamide **98** (20.0 mg) with a yield of 29.2%.

MS m/z (ESI): 521.2 [M+1]
1H NMR (400 MHz, Chloroform-d) δ 8.62 (s, 1H), 8.53 (s, 1H), 8.41 (s, 1H), 8.18 (s, 1H), 8.10 (d, J = 7.3 Hz, 2H), 7.88 (d, J = 7.6 Hz, 2H), 6.77 (s, 1H), 4.53 (q, J = 7.0 Hz, 2H), 2.73 (d, J = 15.8 Hz, 3H), 2.52 (d, J = 14.0 Hz, 2H), 1.59 (s, 2H), 1.48 (t, J = 7.0 Hz, 3H), 1.28 (d, J = 14.6 Hz, 2H), 1.01 (d, J = 7.2 Hz, 2H), 0.84 - 0.69 (m, 2H).

### Example 99

### 3-(6-(cyclopropanesulfonamido)pyrimidin-4-yl)-N-(4-(6-ethoxypyrazin-2-yl)-2-fluorophenyl)-8-oxabicyclo[3.2.1]octane-3-carboxamide

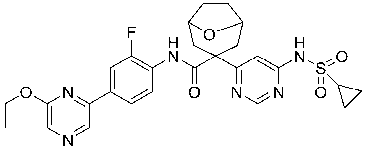

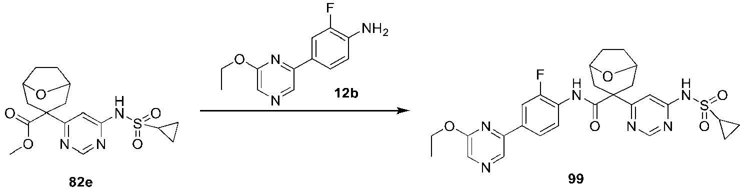

Under ice-water bath, trimethylaluminum (2.0 M n-hexane solution) (489.90 µL) was added to a solution of 4-(6-ethoxypyrazin-2-yl)-2-fluoroaniline **12b** (76.18 mg, 326.60 µmol) in toluene (2 mL). The mixture was stirred at room temperature for 1 hour. A solution of methyl 3-(6-(cyclopropanesulfonamido)pyrimidin-4-yl)-8-oxabicyclo[3.2.1]octane-3-carboxylate **82e** (100 mg, 272.17 µmol) in toluene was added, and the mixture was heated to 100°C and stirred for 5 hours. A small amount of methanol was added to quench the reaction, and the solvent was evaporated to dryness by rotation. Dilute hydrochloric acid was added, and the mixture was stirred for 10 minutes. The crude product was evaporated to dryness by rotation, dissolved in methanol, and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(6-ethoxypyrazin-2-yl)-2-fluorophenyl)-8-oxabicyclo[3.2.1]octane-3-carboxamide **99** (5 mg) with a yield of 2.52%.

MS m/z (ESI): 569.1 [M+1]
1H NMR (400 MHz, DMSO-d6) δ 9.70 (s, 1H), 8.82 (d, J = 9.5 Hz, 2H), 8.25 (s, 1H), 7.98 (t, J = 9.5 Hz, 2H), 7.54 (s, 1H), 6.97 (s, 1H), 4.50 - 4.37 (m, 4H), 3.08 (s, 1H), 2.72 (d, J = 14.3 Hz, 2H), 2.27 - 2.19 (m, 2H), 2.00 (d, J = 7.6 Hz, 2H), 1.79 (d, J = 7.2 Hz, 2H), 1.40 (t, J = 7.0 Hz, 3H), 1.14 - 1.01 (m, 4H).

### Example 100

### 3-(6-(cyclopropanesulfonamido)pyrimidin-4-yl)-N-(4-(6-ethoxypyrazin-2-yl)-2-methoxyphenyl)-8-oxabicyclo[3.2.1]octane-3-carboxamide

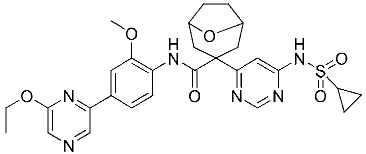

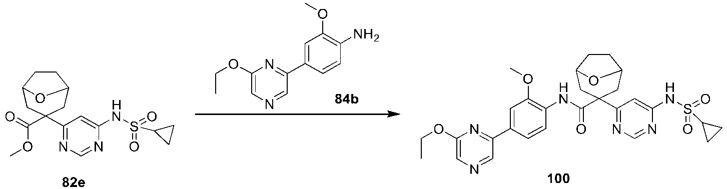

Under ice-water bath, trimethylaluminum (2.0 M n-hexane solution) (489.90 µL) was added to a solution of 4-(6-ethoxypyrazin-2-yl)-2-methoxyaniline **84b** (80.11 mg, 326.60 µmol) in toluene (2 mL). The mixture was stirred at room temperature for 1 hour. A solution of methyl 3-(6-(cyclopropanesulfonamido)pyrimidin-4-yl)-8-oxabicyclo[3.2.1]octane-3-carboxylate **82e** (100 mg, 272.17 µmol) in toluene was added, and the mixture was heated to 90°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was evaporated to dryness by rotation, dissolved in methanol, and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(6-ethoxypyrazin-2-yl)-2-methoxyphenyl)-8-oxabicyclo[3.2.1]octane-3-carboxamide **100** (8 mg) with a yield of 4.10%.

MS m/z (ESI): 581.1 [M+1]
1H NMR (400 MHz, DMSO-d6) δ 9.19 (s, 1H), 8.83 (d, J = 6.8 Hz, 2H), 8.21 (s, 1H), 7.86 (d, J = 8.2 Hz, 1H), 7.72 (d, J = 8.4 Hz, 2H), 6.95 (s, 1H), 4.52 - 4.38 (m, 4H), 3.91 (s, 3H), 3.15 (m, 1H), 2.71 (d, J = 14.3 Hz, 2H), 2.22 (s, 2H), 1.97 (d, J = 7.5 Hz, 2H), 1.78 (s, 2H), 1.40 (t, J = 7.0 Hz, 3H), 1.23 (s, 2H), 1.03 (d, J = 7.5 Hz, 2H).

### Example 101

### 3-(6-(cyclopropanesulfonamido)pyrimidin-4-yl)-N-(5-(6-ethoxypyrazin-2-yl)pyridin-2-yl)-8-oxa bicyclo[3.2.1]octane-3-carboxamide

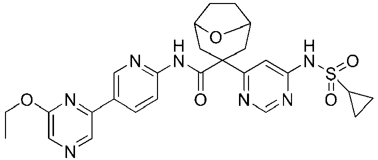

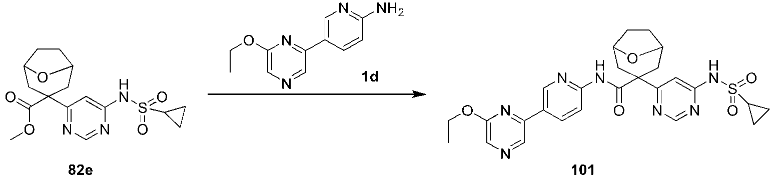

Under ice-water bath, trimethylaluminum (2.0 M n-hexane solution) (489.90 µL) was added to a solution of 5-(6-ethoxypyrazin-2-yl)pyridin-2-amine **1d** (70.62 mg, 326.60 µmol) in toluene (2 mL). The mixture was stirred at room temperature for 1 hour. A solution of methyl 3-(6-(cyclopropanesulfonamido)pyrimidin-4-yl)-8-oxabicyclo[3.2.1]octane-3-carboxylate **82e** (100 mg, 272.17 µmol) in toluene was added, and the mixture was heated to 120°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was evaporated to dryness by rotation, dissolved in methanol, and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(5-(6-ethoxypyrazin-2-yl)pyridin-2-yl)-8-oxa bicyclo[3.2.1]octane-3-carboxamide **101** (4 mg) with a yield of 1.98%.

MS m/z (ESI): 552.1 [M+1]
1H NMR (400 MHz, DMSO-d6) δ 9.84 (s, 1H), 8.83 (d, J = 15.8 Hz, 2H), 8.24 (s, 1H), 8.14 (d, J = 8.4 Hz, 2H), 7.82 (d, J = 8.4 Hz, 2H), 7.00 (s, 1H), 4.52 (q, J = 7.0 Hz, 2H), 4.46 (s, 2H), 3.11 (s, 1H), 2.80 (d, J = 14.3 Hz, 2H), 2.28 (dd, J = 14.5, 3.9 Hz, 2H), 1.94 (d, J = 7.2 Hz, 2H), 1.81 (dd, J = 8.3, 4.4 Hz, 2H), 1.45 (t, J = 7.0 Hz, 3H), 1.09 (dd, J = 23.0, 6.4 Hz, 4H).

### Example 102

### N-(4-(6-ethoxypyrazin-2-yl)phenyl)-3-(6-(ethylsulfonamido)pyrimidin-4-yl)-8-oxabicyclo [3.2.1] octane-3-carboxamide

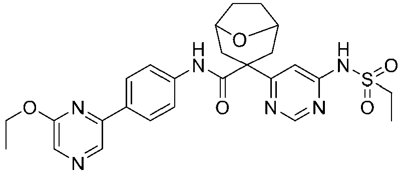

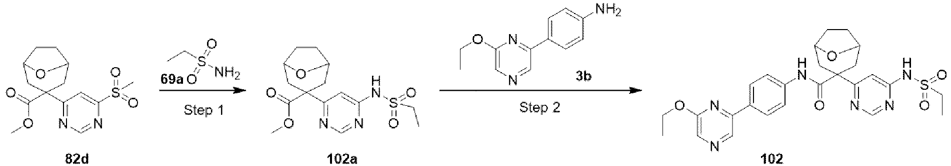

### Step 1

### methyl 3-(6-(ethylsulfonamido)pyrimidin-4-yl)-8-oxabicyclo[3.2.1]octane-3-carboxylate

Methyl 3-(6-(methylsulfonyl)pyrimidin-4-yl)-8-oxabicyclo[3.2.1]octane-3-carboxylate **82d** (80 mg, 245.12 µmol), ethanesulfonamide **69a** (32.11 mg, 294.15 µmol), and cesium carbonate (159.73 mg, 490.25 µmol) were dissolved in N-methylpyrrolidone (1 mL). The mixture was heated to 100°C and stirred for 1 hour. The mixture was cooled to room temperature, and water (30 mL) was added. The mixture was extracted with ethyl acetate (30 mL × 2). The combined organic phase was washed with saturated sodium chloride solution, dried over anhydrous sodium sulfate, filtered, and evaporated to dryness by rotation. The crude product was purified by silica gel column chromatography (eluent: System A) to obtain methyl 3-(6-(ethylsulfonamido)pyrimidin-4-yl)-8-oxabicyclo[3.2.1]octane-3-carboxylate **102a** (80 mg) with a yield of 91.83%.

MS m/z (ESI): 356.0 [M+1]

### Step 2

### N-(4-(6-ethoxypyrazin-2-yl)phenyl)-3-(6-(ethylsulfonamido)pyrimidin-4-yl)-8-oxabicyclo[3.2.1] octane-3-carboxamide

Under ice-water bath, trimethylaluminum (2.0 M n-hexane solution) (703.41 µL) was added to a solution of 4-(6-ethoxypyrazin-2-yl)aniline **3b** (72.68 mg, 337.64 µmol) in toluene (2 mL). The mixture was stirred at room temperature for 1 hour. A solution of methyl 3-(6-(ethylsulfonamido)pyrimidin-4-yl)-8-oxabicyclo[3.2.1]octane-3-carboxylate **102a** (100 mg, 281.37 µmol) in toluene was added. The mixture was heated to 120°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol. The crude product was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(4-(6-ethoxypyrazin-2-yl)phenyl)-3-(6-(ethylsulfonamido)pyrimidin-4-yl)-8-oxabicyclo[3.2.1] octane-3-carboxamide **102** (3 mg) with a yield of 1.51%.

MS m/z (ESI): 539.1 [M+1]
1H NMR (400 MHz, DMSO-d6) δ 9.78 (s, 1H), 8.78 (d, J = 17.3 Hz, 2H), 8.18 (s, 1H), 8.08 (d, J = 8.4 Hz, 2H), 7.76 (d, J = 8.5 Hz, 2H), 6.90 (s, 1H), 4.47 (d, J = 7.1 Hz, 2H), 4.40 (s, 2H), 2.74 (d, J = 14.3 Hz, 2H), 2.28 - 2.16 (m, 2H), 1.89 (d, J = 7.3 Hz, 2H), 1.76 (d, J = 7.7 Hz, 2H), 1.40 (t, J = 7.0 Hz, 3H), 1.20 (dd, J = 15.8, 8.6 Hz, 5H).

### Example 103

### 3-(6-(cyclopropanesulfonamido)pyrimidin-4-yl)-N-(3-(6-ethoxypyrazin-2-yl)phenyl)-8-oxabicycl o[3.2.1]octane-3-carboxamide

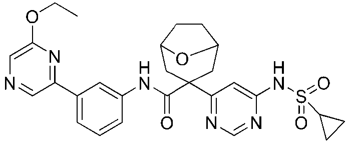

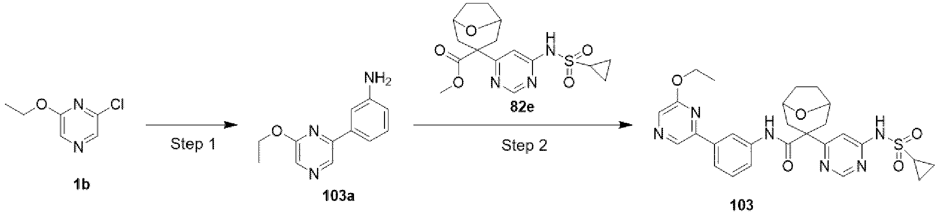

### Step 1

### 3-(6-ethoxypyrazin-2-yl)aniline

2-Chloro-6-ethoxypyrazine **1b** (650 mg, 4.10 mmol), 3-(4,4,5,5-tetramethyl-1,3,2-dioxaborolan-2-yl)aniline (1.08 g, 4.92 mmol), [1,1'-bis(diphenylphosphino)ferrocene]palladium(II) dichloride (299.90 mg, 409.87 µmol), and potassium carbonate (1.70 g, 12.30 mmol) were added to 1,4-dioxane (8 mL) and water (2 mL). The mixture was subjected to argon exchange three times and heated to 95°C with stirring for 3 hours. The solvent was evaporated to dryness by rotation. The crude product was separated by silica gel column chromatography (eluent: System A) to obtain 3-(6-ethoxypyrazin-2-yl)aniline **103a** (800 mg) with a yield of 90.68%.

MS m/z (ESI): 232.1 [M+1]

### Step 2

### 3-(6-(cyclopropanesulfonamido)pyrimidin-4-yl)-N-(3-(6-ethoxypyrazin-2-yl)phenyl)-8-oxabicycl o[3.2.1]octane-3-carboxamide

Under ice-water bath, trimethylaluminum (2.0 M n-hexane solution) (391.92 µL) was added to a solution of 3-(6-ethoxypyrazin-2-yl)aniline **103a** (56.24 mg, 261.28 µmol) in toluene (7.06 mL). The mixture was stirred at room temperature for 1 hour. A solution of methyl 3-(6-(cyclopropanesulfonamido)pyrimidin-4-yl)-8-oxabicyclo[3.2.1]octane-3-carboxylate **82e** (80 mg, 217.74 µmol) in toluene was added. The mixture was heated to 100°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol. The crude product was separated by preparative liquid chromatography (separation column AKZONOBEL Kromasil; 250 × 21.2 mm I.D.; 5 µm, 20 mL/min; mobile phase A: 0.05% TFA + H₂O, mobile phase B: CH₃CN) to obtain 3-(6-(cyclopropanesulfonamido)pyrimidin-4-yl)-N-(3-(6-ethoxypyrazin-2-yl)phenyl)-8-oxabicycl o[3.2.1]octane-3-carboxamide **103** (17 mg) with a yield of 11.05%.

MS m/z (ESI): 551.1 [M+1]
1H NMR (400 MHz, DMSO-d6) δ 9.76 (s, 1H), 8.80 (s, 1H), 8.70 (s, 1H), 8.26 (d, J = 10.8 Hz, 2H), 7.83 (d, J = 7.8 Hz, 1H), 7.75 (d, J = 8.1 Hz, 1H), 7.47 (s, 1H), 6.97 (s, 1H), 4.48 (q, J = 7.0 Hz, 2H), 4.40 (s, 2H), 3.04 (s, 1H), 2.76 (d, J = 14.3 Hz, 2H), 2.23 (dd, J = 14.6, 3.9 Hz, 2H), 1.91 (t, J = 6.8 Hz, 2H), 1.80 - 1.71 (m, 2H), 1.41 (t, J = 7.0 Hz, 3H), 1.10 - 0.96 (m, 4H).

### Example 104

### 3-(2-(cyclopropanesulfonamido)pyrimidin-4-yl)-N-(4-(6-ethoxypyrazin-2-yl)phenyl)-8-oxabicycl o[3.2.1]octane-3-carboxamide

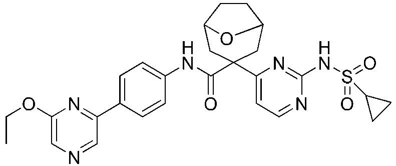

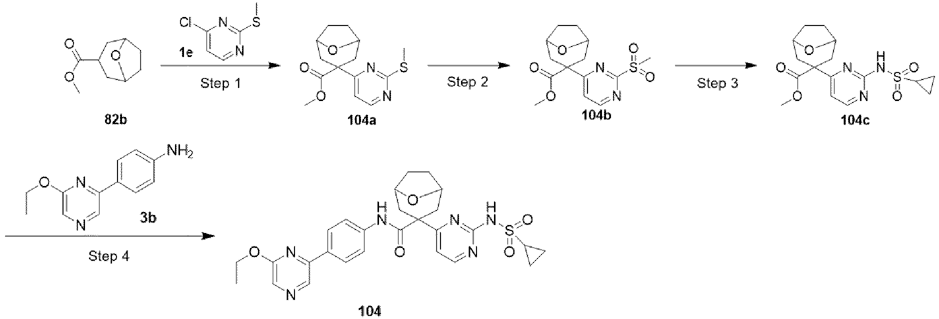

### Step 1

### methyl3-(2-(methylthio)pyrimidin-4-yl)-8-oxabicyclo[3.2.1]octane-3-carboxylate

At -30°C, lithium bis(trimethylsilyl)amide (1 M, 3.53 mL) was added to a solution of 4-chloro-2-(methylthio)pyrimidine 1e (207.62 mg, 1.29 mmol) and methyl 8-oxabicyclo[3.2.1]octane-3-carboxylate **82b** (200 mg, 1.18 mmol) in tetrahydrofuran (46.73 mL). The mixture was heated to room temperature and stirred for 1 hour. Saturated ammonium chloride solution was added to quench the reaction, and the aqueous phase was extracted with ethyl acetate (50 mL × 3). The combined organic phase was washed with saturated sodium chloride solution (30 mL × 2), dried over anhydrous sodium sulfate, filtered, and concentrated under reduced pressure. The crude product was separated by silica gel column chromatography (eluent: System A) to obtain methyl 3-(2-(methylthio)pyrimidin-4-yl)-8-oxabicyclo[3.2.1]octane-3-carboxylate **104a** (190 mg) with a yield of 54.93%.

MS m/z (ESI): 295.1 [M+1]

### Step 2

### methyl 3-(2-(methylsulfonyl)pyrimidin-4-yl)-8-oxabicyclo[3.2.1]octane-3-carboxylate

Meta-Chloroperoxybenzoic acid (288.29 mg, 1.42 mmol, 85% purity) was added to a solution of methyl 3-(2-(methylthio)pyrimidin-4-yl)-8-oxabicyclo[3.2.1]octane-3-carboxylate **104a** (190 mg, 645.45 µmol) in dichloromethane (5 mL). The mixture was stirred at room temperature for 2 hours. Saturated sodium bicarbonate solution (50 mL) was added, and the mixture was extracted with dichloromethane (50 mL × 3). The combined organic phase was washed with saturated sodium chloride solution (30 mL × 2), dried over anhydrous sodium sulfate, filtered, and concentrated to dryness under reduced pressure to obtain methyl 3-(2-(methylsulfonyl)pyrimidin-4-yl)-8-oxabicyclo[3.2.1]octane-3-carboxylate **104b** (200 mg) with a yield of 94.94%. The crude product was directly used in the next step.

MS m/z (ESI): 327.0 [M+1]

### Step 3

### methyl 3-(2-(cyclopropanesulfonamido)pyrimidin-4-yl)-8-oxabicyclo[3.2.1]octane-3-carboxylate

Methyl 3-(2-(methylsulfonyl)pyrimidin-4-yl)-8-oxabicyclo[3.2.1]octane-3-carboxylate **104b** (210 mg, 643.45 µmol), cyclopropanesulfonamide (93.55 mg, 772.14 µmol), and cesium carbonate (419.30 mg, 1.29 mmol) were added to N-methylpyrrolidone (1 mL). The mixture was heated to 100°C and stirred for 2 hours. The mixture was cooled to room temperature, and water (50 mL) was added. The mixture was extracted with ethyl acetate (50 mL × 3). The combined organic phase was washed with saturated sodium chloride solution (30 mL × 2), dried over anhydrous sodium sulfate, filtered, and concentrated to dryness under reduced pressure to obtain methyl 3-(2-(cyclopropanesulfonamido)pyrimidin-4-yl)-8-oxabicyclo[3.2.1]octane-3-carboxylate **104c** (150 mg) with a yield of 63.45%.

MS m/z (ESI): 368.1 [M+1]

### Step 4

### 3-(2-(cyclopropanesulfonamido)pyrimidin-4-yl)-N-(4-(6-ethoxypyrazin-2-yl)phenyl)-8-oxabicycl o[3.2.1]octane-3-carboxamide

Under ice-water bath, trimethylaluminum (2.0 M n-hexane solution) (489.90 µL) was added to a solution of 4-(6-ethoxypyrazin-2-yl)aniline **3b** (70.30 mg, 326.60 µmol) in toluene (2 mL). The mixture was stirred at room temperature for 1 hour. A toluene solution of methyl 3-(2-(cyclopropanesulfonamido)pyrimidin-4-yl)-8-oxabicyclo[3.2.1]octane-3-carboxylate **104c** (100 mg, 272.17 µmol) was added, and the mixture was heated to 100°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol, and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(2-(cyclopropanesulfonamido)pyrimidin-4-yl)-N-(4-(6-ethoxypyrazin-2-yl)phenyl)-8-oxabicycl o[3.2.1]octane-3-carboxamide **104** (4 mg) with a yield of 2.16%.

MS m/z (ESI): 551.1 [M+1]
1H NMR (400 MHz, DMSO-d6) δ 11.31 (s, 1H), 9.63 (s, 1H), 8.75 (s, 1H), 8.56 (s, 1H), 8.18 (s, 1H), 8.08 (d, J = 8.3 Hz, 2H), 7.74 (d, J = 8.4 Hz, 2H), 7.06 (s, 1H), 4.46 (dd, J = 19.7, 12.6 Hz, 4H), 3.31 (s, 1H), 2.75 (d, J = 14.5 Hz, 2H), 2.26 (d, J = 14.2 Hz, 2H), 1.90 (d, J = 7.2 Hz, 2H), 1.77 (s, 2H), 1.39 (t, J = 7.1 Hz, 3H), 1.10 (s, 2H), 0.96 (d, J = 7.9 Hz, 2H).

### Example 105

### 3-(4-(cyclopropanesulfonamido)pyrimidin-2-yl)-N-(4-(6-ethoxypyrazin-2-yl)phenyl)-8-oxabicycl o[3.2.1]octane-3-carboxamide

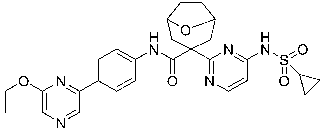

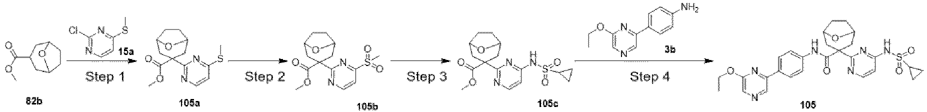

### Step 1

### methyl 3-(4-(methylthio)pyrimidin-2-yl)-8-oxabicyclo[3.2.1]octane-3-carboxylate

At -30°C, lithium bis(trimethylsilyl)amide (1 M, 3.53 mL) was added to a solution of 2-chloro-4-(methylthio)pyrimidine **15a** (207.62 mg, 1.29 mmol) and methyl 8-oxabicyclo[3.2.1]octane-3-carboxylate **82b** (200 mg, 1.18 mmol) in tetrahydrofuran (1.47 mL). The mixture was heated to room temperature and stirred for 1 hour. Saturated ammonium chloride was added to quench the reaction, and the aqueous phase was extracted with ethyl acetate (50 mL × 3). The combined organic phase was washed with saturated sodium chloride solution (30 mL × 2), dried over anhydrous sodium sulfate, filtered, and concentrated to dryness under reduced pressure. The crude product was purified by silica gel column chromatography (eluent: System A) to obtain methyl 3-(4-(methylthio)pyrimidin-2-yl)-8-oxabicyclo[3.2.1]octane-3-carboxylate **105a** (210 mg) with a yield of 60.71%.

MS m/z (ESI): 295.1 [M+1]

### Step 2

### methyl 3-(4-(methylsulfonyl)pyrimidin-2-yl)-8-oxabicyclo[3.2.1]octane-3-carboxylate

Meta-chloroperoxybenzoic acid (289.67 mg, 1.43 mmol, 85% purity) was added to a solution of methyl 3-(4-(methylthio)pyrimidin-2-yl)-8-oxabicyclo[3.2.1]octane-3-carboxylate **105a** (210 mg, 713.39 µmol) in dichloromethane (2 mL). The mixture was stirred at room temperature for 2 hours. Saturated sodium bicarbonate solution (50 mL) was added, and the mixture was extracted with dichloromethane (50 mL × 3). The combined organic phase was washed with saturated sodium chloride solution (30 mL × 2), dried over anhydrous sodium sulfate, filtered, and concentrated to dryness under reduced pressure to dryness to obtain methyl 3-(4-(methylsulfonyl)pyrimidin-2-yl)-8-oxabicyclo[3.2.1]octane-3-carboxylate **105b** (230 mg) with a yield of 98.79%. The product was not isolated and was directly used in the next step.

MS m/z (ESI): 327.0 [M+1]

### Step 3

### methyl 3-(4-(cyclopropanesulfonamido)pyrimidin-2-yl)-8-oxabicyclo[3.2.1]octane-3-carboxylate

Methyl 3-(4-(methylsulfonyl)pyrimidin-2-yl)-8-oxabicyclo[3.2.1]octane-3-carboxylate **105b** (232 mg, 710.86 µmol), cyclopropanesulfonamide (103.35 mg, 853.03 µmol), and cesium carbonate (463.22 mg, 1.42 mmol) were added to N-methylpyrrolidone (1 mL). The mixture was heated to 100°C and stirred for 2 hours. Water (50 mL) was added, and the mixture was extracted with ethyl acetate (50 mL × 3). The combined organic phase was washed with saturated sodium chloride solution (30 mL × 2), dried over anhydrous sodium sulfate, filtered, and concentrated to dryness under reduced pressure to dryness. The crude product was purified by silica gel column chromatography (eluent: System A) to obtain methyl 3-(4-(cyclopropanesulfonamido)pyrimidin-2-yl)-8-oxabicyclo[3.2.1]octane-3-carboxylate **105c** (200 mg) with a yield of 76.57%.

MS m/z (ESI): 368.1 [M+1]

### Step 4

### 3-(4-(cyclopropanesulfonamido)pyrimidin-2-yl)-N-(4-(6-ethoxypyrazin-2-yl)phenyl)-8-oxabicycl o[3.2.1]octane-3-carboxamide

Under ice-water bath, trimethylaluminum (2.0 M n-hexane solution) (489.90 µL) was added to a solution of 4-(6-ethoxypyrazin-2-yl)aniline **3b** (70.30 mg, 326.60 µmol) in toluene (2 mL). The mixture was stirred at room temperature for 1 hour, and then a solution of methyl 3-(4-(cyclopropanesulfonamido)pyrimidin-2-yl)-8-oxabicyclo[3.2.1]octane-3-carboxylate **105c** (100.00 mg, 272.17 µmol) in toluene was added. The mixture was heated to 100°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol, and purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(4-(6-ethoxypyrazin-2-yl)phenyl)-8-oxabicycl o[3.2.1]octane-3-carboxamide **105** (4 mg) with a yield of 2.12%.

MS m/z (ESI): 551.1 [M+1]
1H NMR (400 MHz, DMSO-d6) δ 11.24 (s, 1H), 9.57 (s, 1H), 8.74 (s, 1H), 8.51 (d, J = 5.6 Hz, 1H), 8.17 (s, 1H), 8.05 (d, J = 8.3 Hz, 2H), 7.78 (d, J = 8.3 Hz, 2H), 6.78 (d, J = 5.7 Hz, 1H), 4.52 - 4.45 (m, 2H), 4.39 (s, 2H), 3.20 (s, 1H), 2.68 (d, J = 13.8 Hz, 2H), 2.40 (s, 2H), 1.92 (d, J = 7.3 Hz, 2H), 1.74 (s, 2H), 1.39 (t, J = 7.0 Hz, 3H), 1.06 (s, 2H), 0.84 (d, J = 8.0 Hz, 2H).

### Example 106

### N-(2-(4-(cyclopropanesulfonamido)pyrimidin-2-yl)-2,3-dihydro-1H-inden-2-yl)-5-(6-ethoxypyra zin-2-yl)thiazole-2-carboxamide

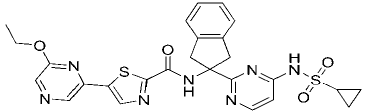

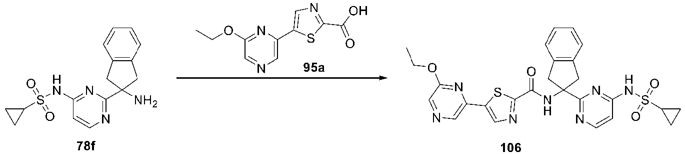

5-(6-Ethoxypyrazin-2-yl)thiazole-2-carboxylic acid **95a** (65 mg, 258.70 µmol), N-(2-(2-amino-2,3-dihydro-1H-inden-2-yl)pyrimidin-4-yl)cyclopropanesulfonamide **78f** (85.47 mg, 258.70 µmol), O-(7-azabenzotriazol-1-yl)-N,N,N',N'-tetramethyluronium hexafluorophosphate (118.04 mg, 310.43 µmol), and N,N-diisopropylethylamine (139.3 µL) were sequentially added to dichloromethane (2 mL). The mixture was stirred at room temperature for 2 hours. The mixture was concentrated under reduced pressure to dryness, and the resulting residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(2-(4-(cyclopropanesulfonamido)pyrimidin-2-yl)-2,3-dihydro-1H-inden-2-yl)-5-(6-ethoxypyra zin-2-yl)thiazole-2-carboxamide **106** (1.3 mg) with a yield of 1%.

MS m/z (ESI): 564.1 [M+1]
1H NMR (400 MHz, DMSO-d6) δ 11.21 (s, 1H), 9.41 (s, 1H), 8.91 (s, 1H), 8.75 (s, 1H), 8.50 (d, J = 5.6 Hz, 1H), 8.27 (s, 1H), 7.35 - 7.07 (m, 4H), 6.78 (d, J = 5.7 Hz, 1H), 4.41 (q, J = 7.0 Hz, 2H), 3.74 (q, J = 16.6 Hz, 4H), 2.76 (s, 1H), 1.38 (t, J = 7.0 Hz, 3H), 1.03 - 0.92 (m, 2H), 0.74 (d, J = 7.5 Hz, 2H).

### Example 107

### 3-(6-(cyclopropanesulfonamido)pyrazin-2-yl)-N-(4-(6-ethoxypyrazin-2-yl)phenyl)-8-oxabicyclo[ 3.2.1]octane-3-carboxamide

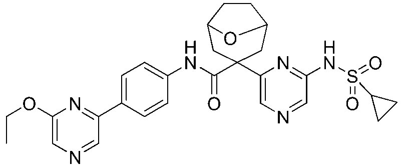

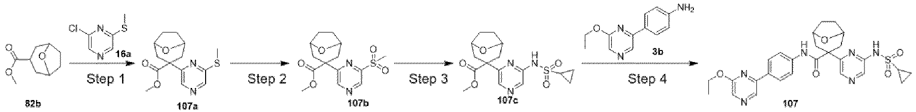

### Step 1

### methyl 3-(6-(methylthio)pyrazin-2-yl)-8-oxabicyclo[3.2.1]octane-3-carboxylate

At -30°C, lithium bis(trimethylsilyl)amide (1 M, 3.53 mL) was added to a solution of 2-chloro-6-(methylthio)pyrazine **16a** (207.62 mg, 1.29 mmol) and methyl 8-oxabicyclo[3.2.1]octane-3-carboxylate **82b** (200 mg, 1.18 mmol) in tetrahydrofuran (46.73 mL). The mixture was heated to room temperature and stirred for 1 hour. Saturated ammonium chloride solution was added to quench the reaction, and the aqueous phase was extracted with ethyl acetate (50 mL × 3). The combined organic phase was washed with saturated sodium chloride solution (30 mL × 2), dried over anhydrous sodium sulfate, filtered, and concentrated under reduced pressure to dryness. The crude product was separated by silica gel column chromatography (eluent: System A) to obtain methyl 3-(6-(methylthio)pyrazin-2-yl)-8-oxabicyclo[3.2.1]octane-3-carboxylate **107a** (190 mg) with a yield of 54.93%.

MS m/z (ESI): 295.1 [M+1]

### Step 2

### methyl 3-(6-(methylsulfonyl)pyrazin-2-yl)-8-oxabicyclo[3.2.1]octane-3-carboxylate

Methyl 3-(6-(methylthio)pyrazin-2-yl)-8-oxabicyclo[3.2.1]octane-3-carboxylate **107a** (200 mg, 679.42 µmol) and meta-chloroperoxybenzoic acid (275.88 mg, 1.36 mmol, 85% purity) were added to dichloromethane (5 mL) and stirred at room temperature for 2 hours. Saturated sodium bicarbonate solution (50 mL) was added, and the mixture was extracted with dichloromethane (50 mL × 3). The combined organic phase was washed with saturated sodium chloride solution (30 mL × 2), dried over anhydrous sodium sulfate, filtered, and concentrated under reduced pressure to dryness to obtain methyl 3-(6-(methylsulfonyl)pyrazin-2-yl)-8-oxabicyclo[3.2.1]octane-3-carboxylate **107b** (150 mg) with a yield of 67.65%. The product was not isolated and was directly used in the next step.

MS m/z (ESI): 327.0 [M+1]

### Step 3

### methyl 3-(6-(cyclopropanesulfonamido)pyrazin-2-yl)-8-oxabicyclo[3.2.1]octane-3-carboxylate

Methyl 3-(6-(methylsulfonyl)pyrazin-2-yl)-8-oxabicyclo[3.2.1]octane-3-carboxylate **107b** (200 mg, 612.81 µmol), cyclopropanesulfonamide (96.52 mg, 796.65 µmol), and cesium carbonate (399.33 mg, 1.23 mmol) were dissolved in N-methylpyrrolidone (2 mL). The mixture was heated to 100°C and stirred for 2 hours. The mixture was cooled to room temperature, and water (50 mL) was added. The mixture was extracted with ethyl acetate (50 mL × 3). The combined organic phase was washed with saturated sodium chloride solution (30 mL × 2), dried over anhydrous sodium sulfate, filtered, and concentrated under reduced pressure to dryness. The crude product was separated by silica gel column chromatography (eluent: System A) to obtain methyl 3-(6-(cyclopropanesulfonamido)pyrazin-2-yl)-8-oxabicyclo[3.2.1]octane-3-carboxylate **107c** (100 mg) with a yield of 44.41%.

MS m/z (ESI): 368.1 [M+1]

### Step 4

### 3-(6-(cyclopropanesulfonamido)pyrazin-2-yl)-N-(4-(6-ethoxypyrazin-2-yl)phenyl)-8-oxabicyclo[ 3.2.1]octane-3-carboxamide

Under ice-water bath, trimethylaluminum (2.0 M n-hexane solution) (532.15 µL) was added to a solution of 4-(6-ethoxypyrazin-2-yl)aniline **3b** (70 mg, 325.20 µmol) in toluene (1 mL). The mixture was stirred at room temperature for 1 hour, and then a solution of methyl 3-(6-(cyclopropanesulfonamido)pyrazin-2-yl)-8-oxabicyclo[3.2.1]octane-3-carboxylate **107c** (108.62 mg, 295.64 µmol) in toluene was added. The mixture was heated to 120°C and stirred for 5 hours. A small amount of methanol was added to quench the reaction, and the solvent was directly evaporated to dryness by rotation to obtain a crude product. Dilute hydrochloric acid was added, and the mixture was stirred for 10 minutes. The crude product was directly evaporated to dryness by rotation, then dissolved in methanol, and separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrazin-2-yl)-N-(4-(6-ethoxypyrazin-2-yl)phenyl)-8-oxabicyclo[ 3.2.1]octane-3-carboxamide 107 (3 mg) with a yield of 1.44%.

MS m/z (ESI): 552.1 [M+1]
1H NMR (400 MHz, DMSO-d6) δ 11.08 (s, 1H), 9.61 (s, 1H), 8.75 (s, 1H), 8.27 (s, 1H), 8.16 (d, J = 10.9 Hz, 2H), 8.07 (d, J = 8.4 Hz, 2H), 7.73 (d, J = 8.4 Hz, 2H), 4.48 - 4.40 (m, 4H), 3.20 (s, 1H), 2.85 (d, J = 14.3 Hz, 2H), 2.24 (s, 2H), 1.92 (d, J = 7.5 Hz, 2H), 1.80 - 1.75 (m, 2H), 1.39 (t, J = 7.1 Hz, 3H), 1.11 (d, J = 5.3 Hz, 2H), 0.97 - 0.92 (m, 2H).

### Example 108

### N-(3-(6-(cyclopropanesulfonamido)pyrazin-2-yl)bicyclo[3.1.0]hexan-3-yl)-5-(6-ethoxypyrazin-2 -yl)thiazole-2-carboxamide

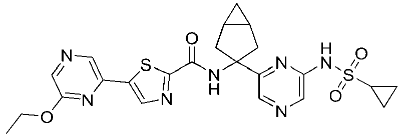

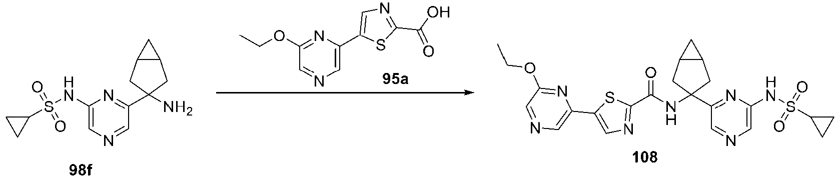

5-(6-Ethoxypyrazin-2-yl)thiazole-2-carboxylic acid **95a** (30 mg, 119.40 µmol), N-(6-(3-aminobicyclo[3.1.0]hexan-3-yl)pyrazin-2-yl)cyclopropanesulfonamide **98f** (35.15 mg, 119.40 µmol), O-(7-azabenzotriazol-1-yl)-N,N,N',N'-tetramethyluronium hexafluorophosphate (54.48 mg, 143.28 µmol), and N,N-diisopropylethylamine (64 µL) were sequentially added to dichloromethane (2 mL) and stirred at room temperature for 2 hours. The mixture was concentrated under reduced pressure to dryness, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrazin-2-yl)bicyclo[3.1.0]hexan-3-yl)-5-(6-ethoxypyrazin-2 -yl)thiazole-2-carboxamide **108** (2.3 mg) with a yield of 3.4%.

MS m/z (ESI): 528.1 [M+1]
1H NMR (400 MHz, DMSO-d6) δ 10.98 (s, 1H), 8.98 (s, 1H), 8.92 (s, 1H), 8.79 (s, 1H), 8.27 (d, J = 5.9 Hz, 2H), 8.14 (s, 1H), 4.41 (q, J = 7.0 Hz, 2H), 3.08 (s, 1H), 2.61 (s, 4H), 1.48 (d, J = 7.7 Hz, 2H), 1.38 (t, J = 7.0 Hz, 3H), 1.11 (p, J = 4.7 Hz, 2H), 1.04-0.98 (m, 2H), 0.63-0.55 (m, 2H).

### Example 109

### N-(3-(2-(cyclopropanesulfonamido)pyrimidin-4-yl)bicyclo[3.1.0]hexan-3-yl)-5-(6-ethoxypyrazin -2-yl)thiazole-2-carboxamide

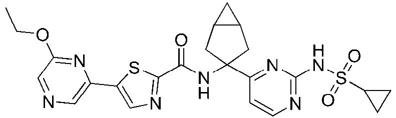

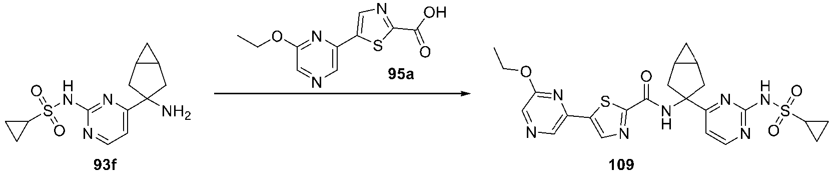

5-(6-Ethoxypyrazin-2-yl)thiazole-2-carboxylic acid **95a** (76.82 mg, 305.74 µmol), N-(4-(3-aminobicyclo[3.1.0]hexan-3-yl)pyrimidin-2-yl)cyclopropanesulfonamide **93f** (75 mg, 254.78 µmol), O-(7-azabenzotriazol-1-yl)-N,N,N',N'-tetramethyluronium hexafluorophosphate (116.25 mg, 305.74 µmol), and N,N-diisopropylethylamine (137 µL) were added sequentially to dichloromethane (3 mL). The mixture was stirred at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(2-(cyclopropanesulfonamido)pyrimidin-4-yl)bicyclo[3.1.0]hexan-3-yl)-5-(6-ethoxypyrazin -2-yl)thiazole-2-carboxamide **109** (1.8 mg) with a yield of 1.2%.

MS m/z (ESI): 528.1 [M+1]
1H NMR (400 MHz, DMSO-d6) δ 9.04 (s, 1H), 8.92 (s, 1H), 8.79 (s, 1H), 8.48 (d, J = 5.4 Hz, 1H), 8.28 (s, 1H), 7.04 (d, J = 5.3 Hz, 1H), 4.41 (q, J = 7.0 Hz, 2H), 3.24 - 3.19 (m, 1H), 2.59 (d, J = 18.0 Hz, 4H), 1.47 (dd, J = 8.1, 4.0 Hz, 2H), 1.38 (t, J = 7.0 Hz, 3H), 1.24 (d, J = 3.7 Hz, 2H), 1.16 - 1.08 (m, 2H), 1.02 (dd, J = 7.8, 2.8 Hz, 2H).

### Example 110

### N-(3-(6-(cyclopropanesulfonamido)pyrimidin-4-yl)bicyclo[3.1.0]hexan-3-yl)-5-(6-ethoxypyrazin -2-yl)picolinamide

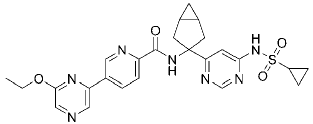

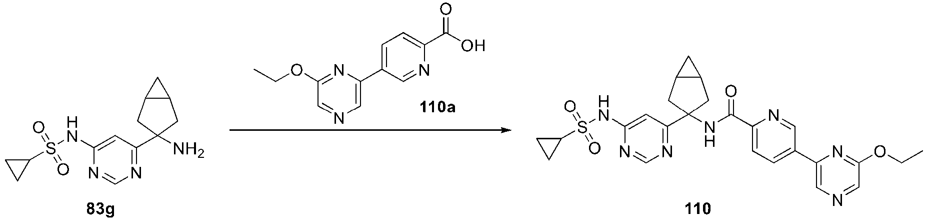

5-(6-Ethoxypyrazin-2-yl)picolinic acid **110a** (100 mg, 407.77 µmol), N-(6-(3-aminobicyclo[3.1.0]hexan-3-yl)pyrimidin-4-yl)cyclopropanesulfonamide **83g** (120.04 mg, 407.77 µmol), O-(7-azabenzotriazol-1-yl)-N,N,N',N'-tetramethyluronium hexafluorophosphate (170.55 mg, 448.55 µmol), and N,N-diisopropylethylamine (219 µL) were added sequentially to dichloromethane (3 mL). The mixture was stirred at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-ethoxypyrazin -2-yl)picolinamide 110 (6.3 mg) with a yield of 2.8%.

MS m/z (ESI): 522.1 [M+1]
1H NMR (400 MHz, DMSO-d6) δ 9.41 (s, 1H), 8.97 (s, 1H), 8.76 (s, 2H), 8.68 (d, J = 8.2 Hz, 1H), 8.36 (s, 1H), 8.10 (d, J = 8.2 Hz, 1H), 7.02 (s, 1H), 4.53 (q, J = 7.0 Hz, 2H), 3.01 (t, J = 6.7 Hz, 1H), 2.72 - 2.51 (m, 4H), 1.49 (dd, J = 8.4, 4.3 Hz, 2H), 1.42 (t, J = 7.0 Hz, 3H), 1.09 - 0.97 (m, 4H), 0.60-0.56 (m, 2H).

### Example 111

### N-(3-(4-(cyclopropanesulfonamido)pyrimidin-2-yl)bicyclo[3.1.0]hexan-3-yl)-5-(6-ethoxypyrazin -2-yl)thiazole-2-carboxamide

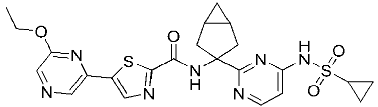

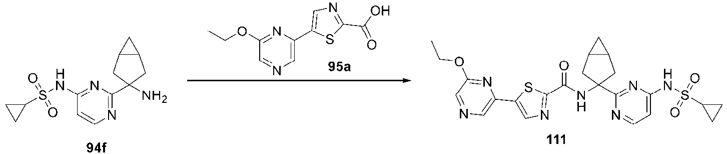

5-(6-Ethoxypyrazin-2-yl)thiazole-2-carboxylic acid **95a** (60 mg, 238.80 µmol), N-(2-(3-aminobicyclo[3.1.0]hexan-3-yl)pyrimidin-4-yl)cyclopropanesulfonamide **94f** (70.29 mg, 238.80 µmol), O-(7-azabenzotriazol-1-yl)-N,N,N',N'-tetramethyluronium hexafluorophosphate (99.88 mg, 262.68 µmol), and N,N-diisopropylethylamine (128 µL) were added sequentially to dichloromethane (5 mL). The mixture was stirred at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(4-(cyclopropanesulfonamido)pyrimidin-2-yl)bicyclo[3.1.0]hexan-3-yl)-5-(6-ethoxypyrazin -2-yl)thiazole-2-carboxamide **111** (2.0 mg) with a yield of 1.5%.

MS m/z (ESI): 528.2 [M+1]

### Example 112

### 5-(6-ethoxypyrazin-2-yl)-N-(3-(6-(ethylsulfonamido)pyrimidin-4-yl)bicyclo[3.1.0]hexan-3-yl)thi azole-2-carboxamide

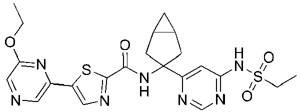

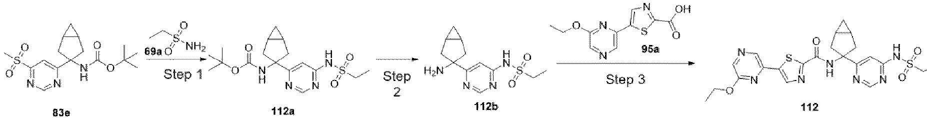

### Step 1

### tert-butyl (3-(6-(ethylsulfonamido)pyrimidin-4-yl)bicyclo[3.1.0]hexan-3-yl)carbamate

Tert-butyl (3-(6-(methylsulfonyl)pyrimidin-4-yl)bicyclo[3.1.0]hexan-3-yl)carbamate **83e** (130 mg, 367.82 µmol), ethanesulfonamide **69a** (44.16 mg, 404.6 µmol), and cesium carbonate (239.68 mg, 735.64 µmol) were sequentially added to N-methylpyrrolidone (2 mL). The mixture was heated to 100°C with stirring for 2 hours. The mixture was concentrated to dryness under reduced pressure, and the resulting residue was purified by silica gel column chromatography (eluent: System A) to obtain tert-butyl (3-(6-(ethylsulfonamido)pyrimidin-4-yl)bicyclo[3.1.0]hexan-3-yl)carbamate **112a** (135 mg) with a yield of 95.5%.

MS m/z (ESI): 383.2 [M+1]

### Step 2

### N-(6-(3-aminobicyclo[3.1.0]hexan-3-yl)pyrimidin-4-yl)ethanesulfonamide

Tert-butyl (3-(6-(ethylsulfonamido)pyrimidin-4-yl)bicyclo[3.1.0]hexan-3-yl)carbamate **112a** (135 mg, 352.96 µmol) was added to 1 M hydrochloric acid dioxane solution (0.5 mL). The mixture was stirred at room temperature for 2 hours. The mixture was concentrated to dryness under reduced pressure to obtain N-(6-(3-aminobicyclo[3.1.0]hexan-3-yl)pyrimidin-4-yl)ethanesulfonamide **112b** (110 mg) with a yield of 97.7%.

MS m/z (ESI): 283.1 [M+1]

### Step 3

### 5-(6-ethoxypyrazin-2-yl)-N-(3-(6-(ethylsulfonamido)pyrimidin-4-yl)bicyclo[3.1.0]hexan-3-yl)thi azole-2-carboxamide

5-(6-Ethoxypyrazin-2-yl)thiazole-2-carboxylic acid **95a** (130 mg, 517.39 µmol), N-(6-(3-aminobicyclo[3.1.0]hexan-3-yl)pyrimidin-4-yl)ethanesulfonamide **112b** (164.96 mg, 517.39 µmol), O-(7-azabenzotriazol-1-yl)-N,N,N',N'-tetramethyluronium hexafluorophosphate (216.40 mg, 569.13 µmol), and N,N-diisopropylethylamine (278 µL) were sequentially added to N,N-dimethylformamide (4 mL). The mixture was stirred at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA+ H2O, mobile phase B: CH3CN) to obtain 5-(6-ethoxypyrazin-2-yl)-N-(3-(6-(ethylsulfonamido)pyrimidin-4-yl)bicyclo[3.1.0]hexan-3-yl)thi azole-2-carboxamide **112** (3.6 mg) with a yield of 1.2%.

MS m/z (ESI): 516.1 [M+1]
1H NMR (400 MHz, DMSO-d6) δ 11.01 (s, 1H), 9.01 (s, 1H), 8.93 (s, 1H), 8.81 (s, 1H), 8.77 (s, 1H), 8.29 (s, 1H), 6.95 (s, 1H), 4.42 (q, J = 7.0 Hz, 2H), 3.46 (s, 2H), 2.58 (d, J = 3.2 Hz, 2H), 1.51 - 1.43 (m, 2H), 1.39 (t, J = 7.0 Hz, 3H), 1.26 - 1.15 (m, 5H), 0.56 (dt, J = 23.5, 3.8 Hz, 2H).

### Example 113

### 5-(6-ethoxypyrazin-2-yl)-N-(3-(6-(methylsulfonamido)pyrimidin-4-yl)bicyclo[3.1.0]hexan-3-yl)t hiazole-2-carboxamide

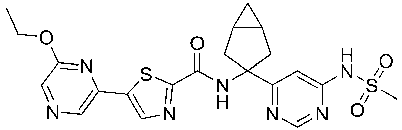

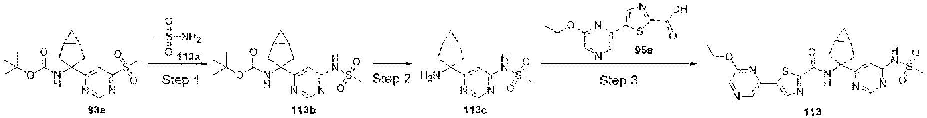

### Step 1

### tert-butyl (3-(6-(methylsulfonamido)pyrimidin-4-yl)bicyclo[3.1.0]hexan-3-yl)carbamate

Tert-butyl (3-(6-(methylsulfonyl)pyrimidin-4-yl)bicyclo[3.1.0]hexan-3-yl)carbamate **83e** (150 mg, 424.41 µmol), methanesulfonamide **113a** (44.41 mg, 466.85 µmol, commercially available), and cesium carbonate (276.56 mg, 848.81 µmol) were sequentially added to N-methylpyrrolidone (2 mL). The mixture was heated to 100°C with stirring for 1 hour. The mixture was concentrated to dryness under reduced pressure, and the resulting residue was purified by silica gel column chromatography (eluent: System A) to obtain tert-butyl (3-(6-(methylsulfonamido)pyrimidin-4-yl)bicyclo[3.1.0]hexan-3-yl)carbamate **113b** (120 mg) with a yield of 76.7%.

MS m/z (ESI): 369.1 [M+1]

### Step 2

### N-(6-(3-aminobicyclo[3.1.0]hexan-3-yl)pyrimidin-4-yl)methanesulfonamide

Tert-butyl (3-(6-(methylsulfonamido)pyrimidin-4-yl)bicyclo[3.1.0]hexan-3-yl)carbamate **113b** (120 mg, 325.69 µmol) was added to 1 M hydrochloric acid dioxane solution (0.5 mL). The mixture was stirred at room temperature for 2 hours. The mixture was concentrated to dryness under reduced pressure to obtain N-(6-(3-aminobicyclo[3.1.0]hexan-3-yl)pyrimidin-4-yl)methanesulfonamide **113c** (95 mg) with a yield of 95.7%.

MS m/z (ESI): 269.1 [M+1]

### Step 3

### 5-(6-ethoxypyrazin-2-yl)-N-(3-(6-(methylsulfonamido)pyrimidin-4-yl)bicyclo[3.1.0]hexan-3-yl)t hiazole-2-carboxamide

5-(6-Ethoxypyrazin-2-yl)thiazole-2-carboxylic acid 95a (90 mg, 358.19 µmol), N-(6-(3-aminobicyclo[3.1.0]hexan-3-yl)pyrimidin-4-yl)methanesulfonamide **113c** (96.12 mg, 358.19 µmol), O-(7-azabenzotriazol-1-yl)-N,N,N',N'-tetramethyluronium hexafluorophosphate (149.82 mg, 394.0 µmol), and N,N-diisopropylethylamine (192 µL) were sequentially added to N,N-dimethylformamide (2 mL). The mixture was stirred at room temperature for 2 hours. The mixture was concentrated to dryness under reduced pressure, and the resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 5-(6-ethoxypyrazin-2-yl)-N-(3-(6-(methylsulfonamido)pyrimidin-4-yl)bicyclo[3.1.0]hexan-3-yl)t hiazole-2-carboxamide **113** (25.0 mg) with a yield of 13.4%.

MS m/z (ESI): 502.1 [M+1]
1H NMR (400 MHz, DMSO-d6) δ 9.02 (s, 1H), 8.93 (s, 1H), 8.81 (s, 1H), 8.78 (s, 1H), 8.29 (s, 1H), 6.92 (s, 1H), 4.42 (q, J = 7.0 Hz, 2H), 3.30(s, 3H), 2.59 (s, 4H), 1.47 (d, J = 7.3 Hz, 2H), 1.39 (t, J = 7.0 Hz, 3H), 0.57 (ddt, J = 21.2, 8.2, 4.7 Hz, 2H).

### Example 114

### N-((1R,5S)-3-(6-(cyclopropanesulfonamido)pyrimidin-4-yl)-8-oxabicyclo[3.2.1]octan-3-yl)-5-(6-ethoxypyrazin-2-yl)thiazole-2-carboxamide

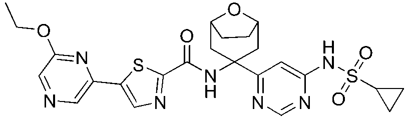

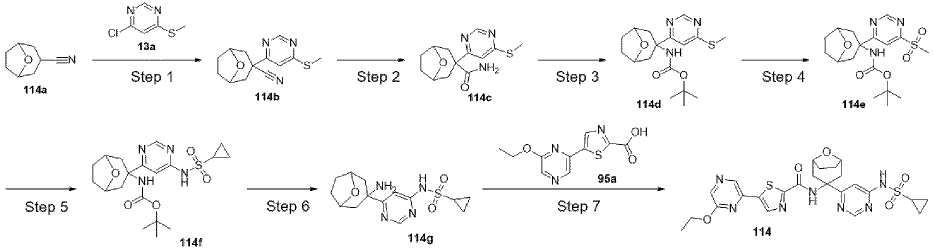

### Step 1

### 3-(6-(methylthio)pyrimidin-4-yl)-8-oxabicyclo[3.2.1]octane-3-carbonitrile

8-Oxabicyclo[3.2.1]octane-3-carbonitrile **114a** (200 mg, 1.46 mmol, commercially available), 4-chloro-6-(methylthio)pyrimidine **13a** (234.18 mg, 1.46 mmol), and lithium hexamethyldisilazide (1 M, 4.37 mL) were sequentially added to tetrahydrofuran (4 mL). The mixture was subjected to argon exchange 3 times, stirred at -30°C for 2 hours, gradually heated to 0°C, and stirred for 0.5 hours. Saturated ammonium chloride aqueous solution was added dropwise to quench the reaction. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL), dried over anhydrous sodium sulfate, filtered, and concentrated to dryness under reduced pressure. The resulting residue was purified by silica gel column chromatography (eluent: System A) to obtain 3-(6-(methylthio)pyrimidin-4-yl)-8-oxabicyclo[3.2.1]octane-3-carbonitrile **114b** (340 mg) with a yield of 89.23%.

MS m/z (ESI): 262.1 [M+1]

### Step 2

### 3-(6-(methylthio)pyrimidin-4-yl)-8-oxabicyclo[3.2.1]octane-3-carboxamide

3-(6-(Methylthio)pyrimidin-4-yl)-8-oxabicyclo[3.2.1]octane-3-carbonitrile **114b** (1.5 g, 6.48 mmol) was dissolved in dimethyl sulfoxide (15 mL). Under an ice bath, 2.5 M sodium hydroxide solution (4 mL) was added sequentially, followed by slow dropwise addition of hydrogen peroxide (2 mL). The mixture was stirred at room temperature for 0.5 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL), dried over anhydrous sodium sulfate, filtered, and concentrated to dryness under reduced pressure. The resulting residue was purified by silica gel column chromatography (eluent: System A) to obtain 3-(6-(methylthio)pyrimidin-4-yl)-8-oxabicyclo[3.2.1]octane-3-carboxamide **114c** (1.5 g) with a yield of 92.77%.

MS m/z (ESI): 280.1 [M+1]

### Step 3

### tert-butyl (3-(6-(methylthio)pyrimidin-4-yl)-8-oxabicyclo[3.2.1]octan-3-yl)carbamate

3-(6-(Methylthio)pyrimidin-4-yl)-8-oxabicyclo[3.2.1]octane-3-carboxamide **114c** (320 mg, 1.15 mmol) and lead tetraacetate (2.03 g, 4.58 mmol) were sequentially added to tert-butanol (3 mL). The mixture was subjected to argon exchange 3 times and heated to 100°C with stirring for 5 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL), dried over anhydrous sodium sulfate, filtered, and concentrated to dryness under reduced pressure. The resulting residue was purified by silica gel column chromatography (eluent: System A) to obtain tert-butyl (3-(6-(methylthio)pyrimidin-4-yl)-8-oxabicyclo[3.2.1]octan-3-yl)carbamate **114d** (150 mg) with a yield of 37.26%.

MS m/z (ESI): 352.2 [M+1]

### Step 4

### tert-butyl (3-(6-(methylsulfonyl)pyrimidin-4-yl)-8-oxabicyclo[3.2.1]octan-3-yl)carbamate

tert-butyl (3-(6-(methylthio)pyrimidin-4-yl)-8-oxabicyclo[3.2.1]octan-3-yl)carbamate **114d** (150 mg, 426.79 µmol) and meta-chloroperoxybenzoic acid (216.62 mg, 1.07 mmol, 85% purity) were added to dichloromethane (2 mL). The mixture was stirred at room temperature for 1.0 hours. Dichloromethane (30 mL) and water (15 mL) were added, and the mixture was partitioned. The aqueous phase was extracted with dichloromethane (30 mL × 2). The combined organic phase was washed with saturated sodium chloride solution (30 mL × 2), dried over anhydrous sodium sulfate, filtered, and concentrated to dryness under reduced pressure to obtain tert-butyl (3-(6-(methylsulfonyl)pyrimidin-4-yl)-8-oxabicyclo[3.2.1]octan-3-yl)carbamate **114e** (150 mg) with a yield of 91.66%, which was directly used in the next step.

MS m/z (ESI): 328.1[M+1-56]

### Step 5

### tert-butyl (3-(6-(cyclopropanesulfonamido)pyrimidin-4-yl)-8-oxabicyclo[3.2.1]octan-3-yl)carbamate

tert-butyl (3-(6-(methylsulfonyl)pyrimidin-4-yl)-8-oxabicyclo[3.2.1]octan-3-yl)carbamate **114e** (130 mg, 339.02 µmol), cyclopropanesulfonamide (45.18 mg, 372.92 µmol), and cesium carbonate (220.92 mg, 678.03 µmol) were sequentially added to N-methylpyrrolidone (2 mL). The mixture was subjected to argon exchange three times and heated to 100°C with stirring for 1 hour. The mixture was concentrated to dryness under reduced pressure. The resulting residue was purified by silica gel column chromatography (eluent: System A) to obtain tert-butyl (3-(6-(cyclopropanesulfonamido)pyrimidin-4-yl)-8-oxabicyclo[3.2.1]octan-3-yl)carbamate 114f (140 mg) with a yield of 97.28%.

**MS** m/z (ESI): 425.2 [M+1]

### Step 6

### N-(6-(3-amino-8-oxabicyclo[3.2.1]octan-3-yl)pyrimidin-4-yl)cyclopropanesulfonamide

Tert-butyl (3-(6-(cyclopropanesulfonamido)pyrimidin-4-yl)-8-oxabicyclo[3.2.1]octan-3-yl)carbamate **114f** (140 mg, 329.79 µmol) was added to a solution of hydrogen chloride in 1,4-dioxane (0.5 mL). The mixture was subjected to argon exchange three times and stirred at room temperature for 1 hour. The mixture was concentrated to dryness under reduced pressure to obtain N-(6-(3-amino-8-oxabicyclo[3.2.1]octan-3-yl)pyrimidin-4-yl)cyclopropanesulfonamide **114g** (100 mg) with a yield of 93.47%. The crude product was directly used in the next step.

**MS** m/z (ESI): 325.1 [M+1]

### Step 7

### N-((1R,5S)-3-(6-(cyclopropanesulfonamido)pyrimidin-4-yl)-8-oxabicyclo[3.2.1]octan-3-yl)-5-(6-ethoxypyrazin-2-yl)thiazole-2-carboxamide

**N-(6-(3-amino-8-oxabicyclo[3.2.1]octan-3-yl)pyrimidin-4-yl)cyclopropanesulfonamide 114g** (103.29 mg, 318.39 µmol), 5-(6-ethoxypyrazin-2-yl)thiazole-2-carboxylic acid 95a (100.00 mg, 318.39 µmol), O-(7-azabenzotriazol-1-yl)-N,N,N',N'-tetramethyluronium hexafluorophosphate (133.17 mg, 350.23 µmol), and N,N-diisopropylethylamine (123.45 mg, 955.18 µmol, 171.45 µL) were sequentially added to N,N-dimethylformamide (2 mL). The mixture was subjected to argon exchange three times and stirred at room temperature for 2 hours. The mixture was concentrated to dryness under reduced pressure. The resulting residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-((1R,5S)-3-(6-(cyclopropanesulfonamido)pyrimidin-4-yl)-8-oxabicyclo[3.2.1]octan-3-yl)-5-(6-ethoxypyrazin-2-yl)thiazole-2-carboxamide **114** (30 mg) with a yield of 16.61%.

**MS** m/z (ESI): 558.2 [M+1]
1H NMR (400 MHz, DMSO-d6) δ 9.19 (s, 1H), 8.93 (s, 1H), 8.85 (s, 1H), 8.78 (s, 1H), 8.30 (s, 1H), 7.06 (s, 1H), 4.43 (q, J = 6.8 Hz, 4H), 3.02 (d, J = 8.5 Hz, 1H), 2.55 (s, 1H), 2.37 (dd, J = 14.8, 4.2 Hz, 2H), 2.13 (d, J = 7.5 Hz, 2H), 1.86 (dd, J = 8.4, 4.4 Hz, 2H), 1.39 (t, J = 7.0 Hz, 3H), 1.04 (dt, J = 14.4, 4.4 Hz, 4H).

### Example 115

### N-(3-(6-(cyclopropanesulfonamido)pyrimidin-4-yl)bicyclo[3.1.0]hexan-3-yl)-5-(6-ethoxypyrazin -2-yl)-1,3,4-thiadiazole-2-carboxamide

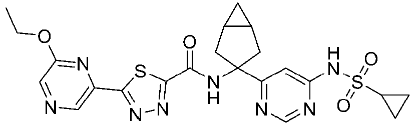

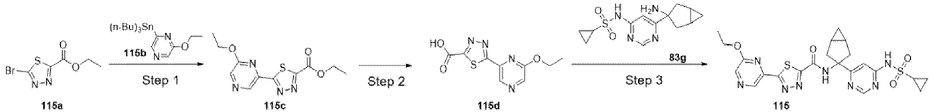

### Step 1

### ethyl 5-(6-ethoxypyrazin-2-yl)-1,3,4-thiadiazole-2-carboxylate

Ethyl 5-bromo-1,3,4-thiadiazole-2-carboxylate **115a** (200 mg, 843.62 µmol, commercially available), 2-ethoxy-6-(tributylstannyl)pyrazine **115b** (633.76 mg, 1.01 mmol, commercially available), and tetrakis(triphenylphosphine)palladium(0) (97.49 mg, 84.36 µmol) were sequentially added to toluene (3 mL). The mixture was subjected to argon exchange three times and heated to 100°C with stirring for 16 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL), dried over anhydrous sodium sulfate, filtered, and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain ethyl 5-(6-ethoxypyrazin-2-yl)-1,3,4-thiadiazole-2-carboxylate **115c** (50 mg) with a yield of 21.14%.

**MS** m/z (ESI): 281.0 [M+1]

### Step 2

### 5-(6-ethoxypyrazin-2-yl)-1,3,4-thiadiazole-2-carboxylic acid

Ethyl 5-(6-ethoxypyrazin-2-yl)-1,3,4-thiadiazole-2-carboxylate **115c** (50 mg, 178.38 µmol) and potassium trimethylsilanolate (27.46 mg, 214.05 µmol) were sequentially added to tetrahydrofuran (2 mL). The mixture was subjected to argon exchange three times and stirred at room temperature for 1 hour. The mixture was concentrated to dryness under reduced pressure to obtain 5-(6-ethoxypyrazin-2-yl)-1,3,4-thiadiazole-2-carboxylic acid **115d** (45 mg) with a yield of 80.01%. The crude product was directly used in the next step.

**MS** m/z (ESI): 253.0 [M+1]

### Step 3

### N-(3-(6-(cyclopropanesulfonamido)pyrimidin-4-yl)bicyclo[3.1.0]hexan-3-yl)-5-(6-ethoxypyrazin -2-yl)-1,3,4-thiadiazole-2-carboxamide

5-(6-Ethoxypyrazin-2-yl)-1,3,4-thiadiazole-2-carboxylic acid **115d** (45 mg, 178.39 µmol), N-(6-(3-aminobicyclo[3.1.0]hexan-3-yl)pyrimidin-4-yl)cyclopropanesulfonamide **83g** (52.51 mg, 178.39 µmol), O-(7-azabenzotriazol-1-yl)-N,N,N',N'-tetramethyluronium hexafluorophosphate (74.61 mg, 196.23 µmol), and N,N-diisopropylethylamine (69.17 mg, 535.18 µmol, 96.07 µL) were sequentially added to N,N-dimethylformamide (2 mL). The mixture was subjected to argon exchange three times and stirred at room temperature for 2 hours. The mixture was concentrated to dryness under reduced pressure, and the residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-ethoxypyrazin -2-yl)-1,3,4-thiadiazole-2-carboxamide **115** (5.0 mg) with a yield of 5.24%.

**MS** m/z (ESI): 529.1 [M+1]
1H NMR (400 MHz, DMSO-d6) δ 9.55 (s, 1H), 9.05 (s, 1H), 8.78 (s, 1H), 8.53 (s, 1H), 7.02 (s, 1H), 4.45 (q, J = 7.0 Hz, 2H), 2.60 (s, 4H), 1.48 (d, J = 7.9 Hz, 2H), 1.40 (t, J = 7.0 Hz, 3H), 1.05 (s, 4H), 0.64 (t, J = 4.4 Hz, 1H), 0.60 - 0.51 (m, 1H).

### Example 116

### N-((1R,5S)-3-(6-(cyclopropanesulfonamido)pyrimidin-4-yl)-8-oxabicyclo[3.2.1]octan-3-yl)-2-(6-ethoxypyrazin-2-yl)thiazole-5-carboxamide

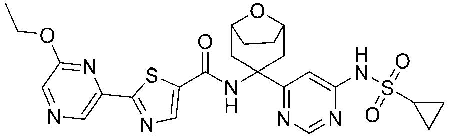

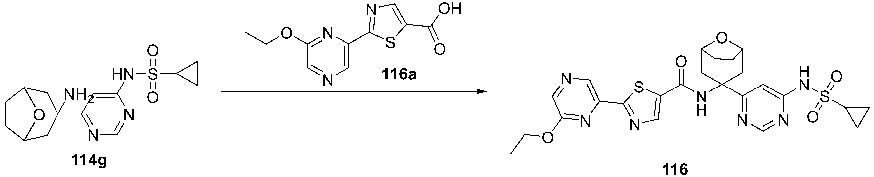

2-(6-Ethoxypyrazin-2-yl)thiazole-5-carboxylic acid **116a** (75 mg, 298.49 µmol, prepared according to published patent WO2022087634), N-(6-(3-amino-8-oxabicyclo[3.2.1]octan-3-yl)pyrimidin-4-yl)cyclopropanesulfonamide **114g** (96.83 mg, 298.49 µmol), O-(7-azabenzotriazol-1-yl)-N,N,N',N'-tetramethyluronium hexafluorophosphate (124.85 mg, 328.34 µmol), and N,N-diisopropylethylamine (115.73 mg, 895.48 µmol, 160.74 µL) were sequentially added to N,N-dimethylformamide (2 mL). The mixture was subjected to argon exchange three times and stirred continuously at room temperature for 2 hours. Ethyl acetate (30 mL) and water (15 mL) were added, and the mixture was separated by liquid separation. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-((1R,5S)-3-(6-(cyclopropanesulfonamido)pyrimidin-4-yl)-8-oxabicyclo[3.2.1]octan-3-yl)-2-(6-ethoxypyrazin-2-yl)thiazole-5-carboxamide **116** (8.7 mg) with a yield of 5.18%.

**MS** m/z (ESI): 558.2 [M+1]
1H NMR (400 MHz, DMSO-d6) δ 9.00 (s, 1H), 8.91 (s, 1H), 8.77 (s, 1H), 8.64 (s, 1H), 8.45 (s, 1H), 7.05 (s, 1H), 4.47 (t, J = 7.0 Hz, 2H), 4.43 (d, J = 6.8 Hz, 2H), 3.03 (s, 1H), 2.46 - 2.31 (m, 4H), 2.14 (t, J = 6.8 Hz, 2H), 1.92 - 1.85 (m, 2H), 1.41 (t, J = 7.0 Hz, 3H), 1.09 - 0.99 (m, 4H).

### Example 117

### 5-(5-chloropyrazolo[1,5-a]pyridin-3-yl)-N-((1R,5S)-3-(6-(cyclopropanesulfonamido)pyrimidin-4 -yl)-8-oxabicyclo[3.2.1]octan-3-yl)thiazole-2-carboxamide

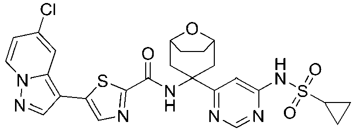

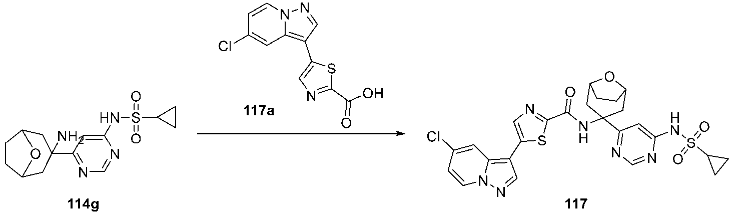

5-(5-Chloropyrazolo[1,5-a]pyridin-3-yl)thiazole-2-carboxylic acid **117a** (30 mg, 107.26 µmol, prepared according to published patent WO2022087634), N-(6-(3-amino-8-oxabicyclo[3.2.1]octan-3-yl)pyrimidin-4-yl)cyclopropanesulfonamide **114g** (34.79 mg, 107.26 µmol), O-(7-azabenzotriazol-1-yl)-N,N,N',N'-tetramethyluronium hexafluorophosphate (48.94 mg, 128.71 µmol), and N,N-diisopropylethylamine (41.59 mg, 321.77 µmol, 57.76 µL) were sequentially added to N,N-dimethylformamide (1 mL). The mixture was subjected to argon exchange three times and stirred continuously at room temperature for 2 hours. Ethyl acetate (30 mL) and water (15 mL) were added, and the mixture was separated by liquid separation. The aqueous phase was extracted with ethyl acetate (30 mL × 3). The combined organic phase was washed with saturated sodium chloride solution (30 mL × 2), dried over anhydrous sodium sulfate, filtered, and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 5-(5-chloropyrazolo[1,5-a]pyridin-3-yl)-N-((1R,5S)-3-(6-(cyclopropanesulfonamido)pyrimidin-4 -yl)-8-oxabicyclo[3.2.1]octan-3-yl)thiazole-2-carboxamide **117** (1.2 mg) with a yield of 1.77%.

**MS** m/z (ESI): 586.0 [M+1]
1H NMR (400 MHz, DMSO-d6) δ 11.17 (s, 1H), 9.09 (s, 1H), 8.86 (d, J = 7.4 Hz, 1H), 8.78 (s, 1H), 8.59 (s, 1H), 8.46 (s, 1H), 8.13 (d, J = 2.3 Hz, 1H), 7.13 (dd, J = 7.4, 2.3 Hz, 1H), 7.05 (s, 1H), 4.41 (s, 2H), 3.01-3.00 (m, 1H), 2.67-2.65 (m, 2H), 2.37 (d, J = 15.1 Hz, 2H), 2.14 (d, J = 7.6 Hz, 2H), 1.86 (s, 2H), 1.05 (s, 4H).

### Example 118

### N-((1R,5S)-3-(4-(cyclopropanesulfonamido)pyrimidin-2-yl)-8-oxabicyclo[3.2.1]octan-3-yl)-5-(6-ethoxypyrazin-2-yl)thiazole-2-carboxamide

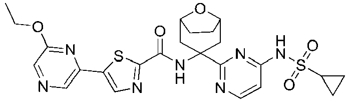

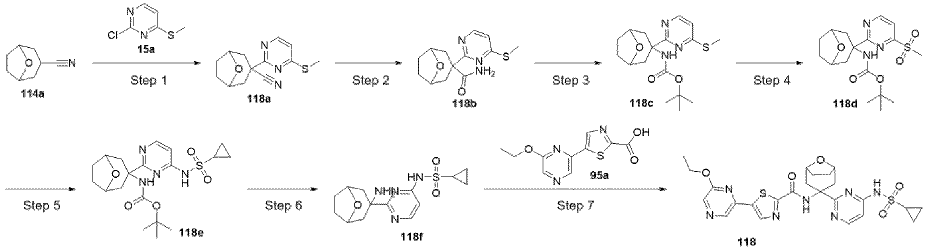

### Step 1

### 3-(4-(methylthio)pyrimidin-2-yl)-8-oxabicyclo[3.2.1]octane-3-carbonitrile

8-Oxabicyclo[3.2.1]octane-3-carbonitrile **114a** (300 mg, 2.19 mmol), 2-chloro-4-(methylthio)pyrimidine **15a** (351.27 mg, 2.19 mmol), and lithium bis(trimethylsilyl)amide (1 M, 6.56 mL) were sequentially added to tetrahydrofuran (6 mL). The mixture was subjected to argon exchange three times, stirred at -30°C for 2 hours, and gradually heated to 0°C, then stirred for 2 hours. Saturated ammonium chloride aqueous solution was added dropwise to quench the reaction. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL), dried over anhydrous sodium sulfate, filtered, and concentrated under reduced pressure to dryness. The residue was purified by silica gel column chromatography (eluent: System A) to obtain 3-(4-(methylthio)pyrimidin-2-yl)-8-oxabicyclo[3.2.1]octane-3-carbonitrile **118a** (380 mg) with a yield of 66.49%.

**MS** m/z (ESI): 262.1 [M+1]

### Step 2

### 3-(4-(methylthio)pyrimidin-2-yl)-8-oxabicyclo[3.2.1]octane-3-carboxamide

3-(4-(Methylthio)pyrimidin-2-yl)-8-oxabicyclo[3.2.1]octane-3-carbonitrile **118a** (380 mg, 1.45 mmol) was added to a mixed solution of dimethyl sulfoxide (2 mL) and 2.5 M sodium hydroxide solution (1 mL). Hydrogen peroxide (0.5 mL) was added dropwise, and the mixture was continuously stirred at room temperature for 1 hour. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL), dried over anhydrous sodium sulfate, filtered, and concentrated under reduced pressure to dryness. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(methylthio)pyrimidin-2-yl)-8-oxabicyclo[3.2.1]octane-3-carboxamide **118b** (380 mg) with a yield of 93.55%.

**MS** m/z (ESI): 280.1 [M+1]

### Step 3

### tert-butyl (3-(4-(methylthio)pyrimidin-2-yl)-8-oxabicyclo[3.2.1]octan-3-yl)carbamate

3-(4-(Methylthio)pyrimidin-2-yl)-8-oxabicyclo[3.2.1]octane-3-carboxamide **118b** (350 mg, 1.25 mmol) and lead tetraacetate (2.22 g, 5.01 mmol) were sequentially added to tert-butanol (5 mL). The mixture was subjected to argon exchange three times and heated to 100°C with continuous stirring for 5 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL), dried over anhydrous sodium sulfate, filtered, and concentrated under reduced pressure to dryness. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain tert-butyl (3-(4-(methylthio)pyrimidin-2-yl)-8-oxabicyclo[3.2.1]octan-3-yl)carbamate **118c** (70 mg) with a yield of 15.90%.

**MS** m/z (ESI): 352.2 [M+1]

### Step 4

### tert-butyl (3-(4-(methylsulfonyl)pyrimidin-2-yl)-8-oxabicyclo[3.2.1]octan-3-yl)carbamate

Tert-butyl (3-(4-(methylthio)pyrimidin-2-yl)-8-oxabicyclo[3.2.1]octan-3-yl)carbamate **118c** (70 mg, 199.17 µmol) and meta-chloroperoxybenzoic acid (101.09 mg, 497.92 µmol, 85% purity) were sequentially added to dichloromethane (2 mL). The mixture was continuously stirred at room temperature for 2 hours. Dichloromethane (30 mL) and water (15 mL) were added, and the mixture was separated. The aqueous phase was extracted with dichloromethane (30 mL × 2). The combined organic phase was washed with saturated sodium chloride solution (30 mL), dried over anhydrous sodium sulfate, filtered, and concentrated under reduced pressure to dryness. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain tert-butyl (3-(4-(methylsulfonyl)pyrimidin-2-yl)-8-oxabicyclo[3.2.1]octan-3-yl)carbamate **118d** (60 mg) with a yield of 78.56%.

MS m/z (ESI): 328.1 [M+1-56]

### Step 5

### tert-butyl (3-(4-(cyclopropanesulfonamido)pyrimidin-2-yl)-8-oxabicyclo[3.2.1]octan-3-yl)carbamate

Tert-butyl (3-(4-(methylsulfonyl)pyrimidin-2-yl)-8-oxabicyclo[3.2.1]octan-3-yl)carbamate **118d** (60 mg, 156.47 µmol), cyclopropanesulfonamide (24.64 mg, 203.41 µmol), and cesium carbonate (101.96 mg, 312.94 µmol) were sequentially added to N-methylpyrrolidone (1 mL). The mixture was heated to 100°C with continuous stirring for 1 hour. The mixture was concentrated under reduced pressure to dryness. The residue was purified by silica gel column chromatography (eluent: System B) to obtain tert-butyl (3-(4-(cyclopropanesulfonamido)pyrimidin-2-yl)-8-oxabicyclo[3.2.1]octan-3-yl)carbamate **118e** (50 mg) with a yield of 75.27%.

**MS** m/z (ESI): 425.2 [M+1]

### Step 6

### N-(2-(3-amino-8-oxabicyclo[3.2.1]octan-3-yl)pyrimidin-4-yl)cyclopropanesulfonamide

Tert-butyl (3-(4-(cyclopropanesulfonamido)pyrimidin-2-yl)-8-oxabicyclo[3.2.1]octan-3-yl)carbamate **118e** (50 mg, 117.78 µmol) was added to a solution of hydrogen chloride in 1,4-dioxane (0.5 mL). The mixture was continuously stirred at room temperature for 1 hour. The mixture was concentrated under reduced pressure to dryness to obtain N-(2-(3-amino-8-oxabicyclo[3.2.1]octan-3-yl)pyrimidin-4-yl)cyclopropanesulfonamide 118f (30 mg) with a yield of 78.52%. The product was directly used in the next step.

**MS** m/z (ESI): 325.0 [M+1]

### Step 7

### N-((1R,5S)-3-(4-(cyclopropanesulfonamido)pyrimidin-2-yl)-8-oxabicyclo[3.2.1]octan-3-yl)-5-(6-ethoxypyrazin-2-yl)thiazole-2-carboxamide

5-(6-Ethoxypyrazin-2-yl)thiazole-2-carboxylic acid 95a (40 mg, 159.20 µmol), N-(2-(3-amino-8-oxabicyclo[3.2.1]oct-3-yl)pyrimidin-4-yl)cyclopropanesulfonamide 118f (51.64 mg, 159.20 µmol), O-(7-azabenzotriazol-1-yl)-N,N,N',N'-tetramethyluronium hexafluorophosphate (72.64 mg, 191.04 µmol), and N,N-diisopropylethylamine (61.72 mg, 477.59 µmol, 85.73 µL) were sequentially added to N,N-dimethylformamide (1 mL). The mixture was stirred continuously at room temperature for 2 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL), dried over anhydrous sodium sulfate, filtered, and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-((1R,5S)-3-(4-(cyclopropanesulfonamido)pyrimidin-2-yl)-8-oxabicyclo[3.2.1]octan-3-yl)-5-(6-ethoxypyrazin-2-yl)thiazole-2-carboxamide 118 (0.95 mg) with a yield of 1.03%.

MS m/z (ESI): 558.2 [M+1]

### Example 119

### (1R,5S)-3-(2-(cyclopropanesulfonamido)pyrimidin-4-yl)-N-(5-(6-ethoxypyrazin-2-yl)pyridin-2-y l)-8-oxabicyclo[3.2.1]octane-3-carboxamide

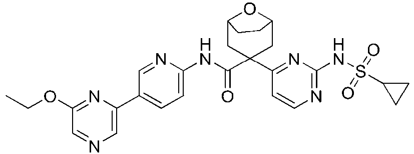

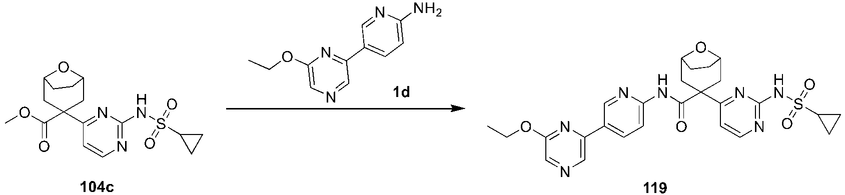

Under ice-water bath, trimethylaluminum (2.0 M n-hexane solution) (2 M, 489.90 µL) was added to a solution of 5-(6-ethoxypyrazin-2-yl)pyridin-2-amine **1d** (70.62 mg, 326.60 µmol) in toluene (2 mL). The mixture was stirred at room temperature for 1 hour. A solution of methyl 3-(2-(cyclopropanesulfonamido)pyrimidin-4-yl)-8-oxabicyclo[3.2.1]octane-3-carboxylate **104c** (100.00 mg, 272.17 µmol) in toluene was added, and the mixture was heated to 100°C and stirred for 5 hours. The mixture was cooled to room temperature, quenched by adding methanol, and adjusted to pH 4-5 with 1 N hydrochloric acid. The mixture was extracted with ethyl acetate (30 mL × 3). The combined organic phase was washed with saturated sodium chloride solution (30 mL), dried over anhydrous sodium sulfate, filtered, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(2-(cyclopropanesulfonamido)pyrimidin-4-yl)-N-(5-(6-ethoxypyrazin-2-yl)pyridin-2-y l)-8-oxabicyclo[3.2.1]octane-3-carboxamide 119 (0.99 mg) with a yield of 0.66%.

**MS** m/z (ESI): [M+1] 552.1
1H NMR (400 MHz, DMSO-d6) δ 10.21 (s, 1H), 9.03 (d, J = 2.4 Hz, 1H), 8.83 (s, 1H), 8.56 (d, J = 5.4 Hz, 1H), 8.50 (dd, J = 8.8, 2.5 Hz, 1H), 8.25 (s, 1H), 8.13 (d, J = 8.8 Hz, 1H), 7.14 (d, J = 5.4 Hz, 1H), 4.48 (d, J = 7.0 Hz, 2H), 4.41 (s, 2H), 3.31 (s, 1H), 2.81 (d, J = 14.4 Hz, 2H), 2.25 - 2.18 (m, 2H), 1.88 (d, J = 7.2 Hz, 2H), 1.77 (dd, J = 8.3, 4.4 Hz, 2H), 1.40 (t, J = 7.0 Hz, 3H), 1.08 (dd, J = 4.7, 2.5 Hz, 2H), 0.94 (dd, J = 7.9, 2.8 Hz, 2H).

### Example 120

### N-(3-(4-(cyclopropanesulfonamido)pyridin-2-yl)bicyclo[3.1.0]hexan-3-yl)-5-(6-ethoxypyrazin-2-yl)thiazole-2-carboxamide

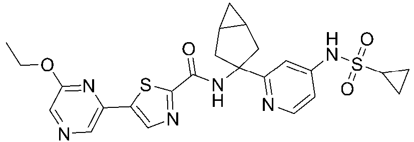

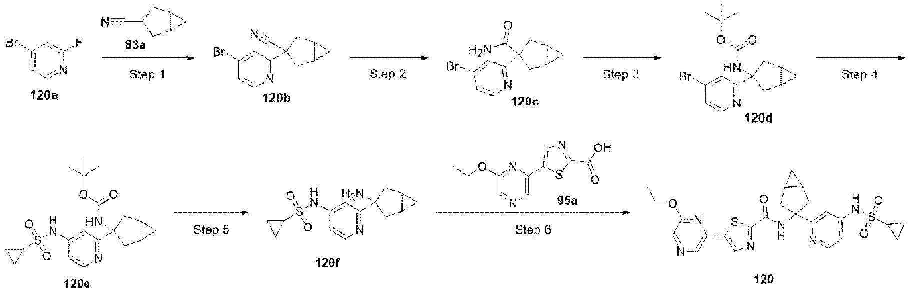

### Step 1

### 3-(4-bromopyridin-2-yl)bicyclo[3.1.0]hexane-3-carbonitrile

At -30°C, lithium bis(trimethylsilyl)amide (1 M, 5.60 mL) was added to a solution of 4-bromo-2-fluoropyridine 120a (492.72 mg, 2.80 mmol, commercially available) and bicyclo[3.1.0]hexane-3-carbonitrile 83a (300 mg, 2.80 mmol) in tetrahydrofuran (5.00 mL). The mixture was heated to room temperature and stirred for 2 hours. After the starting material was completely reacted, saturated ammonium chloride aqueous solution (30 mL) was added to quench the reaction. The mixture was extracted with ethyl acetate (30 mL × 3). The combined organic phase was washed with saturated sodium chloride solution (30 mL), dried over anhydrous sodium sulfate, and concentrated by rotary evaporation. The residue was purified by silica gel column chromatography (eluent: System A) to obtain 3-(4-bromopyridin-2-yl)bicyclo[3.1.0]hexane-3-carbonitrile **120b** (160 mg) with a yield of 21.72%.

MS m/z (ESI): 265.0 [M+1]

### Step 2

### 3-(4-bromopyridin-2-yl)bicyclo[3.1.0]hexane-3-carboxamide

3-(4-Bromopyridin-2-yl)bicyclo[3.1.0]hexane-3-carbonitrile **120b** (160 mg, 608.06 µmol) was added to sodium hydroxide (0.5 mL, 2.5 M) and dimethyl sulfoxide (1 mL). Hydrogen peroxide (0.5 mL) was added dropwise, and the mixture was stirred at room temperature for 1 hour. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was purified by silica gel column chromatography (eluent: System B) to obtain 3-(4-bromopyridin-2-yl)bicyclo[3.1.0]hexane-3-carboxamide **120c** (150 mg) with a yield of 97.7%.

MS m/z (ESI): 283.0 [M+1]

### Step 3

### tert-butyl (3-(4-bromopyridin-2-yl)bicyclo[3.1.0]hexan-3-yl)carbamate

3-(4-Bromopyridin-2-yl)bicyclo[3.1.0]hexane-3-carboxamide **120c** (350 mg, 1.24 mmol) and lead(IV) acetate (2.21 g, 4.98 mmol) were sequentially added to tert-butanol (5 mL). The mixture was subjected to argon exchange three times and heated to 100°C with stirring for 3 hours. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was purified by silica gel column chromatography (eluent: System B) to obtain tert-butyl (3-(4-bromopyridin-2-yl)bicyclo[3.1.0]hexan-3-yl)carbamate **120d** (95 mg) with a yield of 21.60%.

**MS** m/z (ESI): 355.1 [M+1]

### Step 4

### tert-butyl (3-(4-(cyclopropanesulfonamido)pyridin-2-yl)bicyclo[3.1.0]hexan-3-yl)carbamate

Tert-butyl (3-(4-bromopyridin-2-yl)bicyclo[3.1.0]hexan-3-yl)carbamate **120d** (95 mg, 268.93 µmol), cyclopropanesulfonamide (39.10 mg, 322.71 µmol), cesium carbonate (175.24 mg, 537.86 µmol), and allylpalladium chloride (9.84 mg, 26.89 µmol) were sequentially added to 1,4-dioxane (2 mL). The mixture was subjected to argon exchange three times and heated to 100°C with stirring for 6 hours. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was purified by silica gel column chromatography (eluent: System B) to obtain tert-butyl (3-(4-(cyclopropanesulfonamido)pyridin-2-yl)bicyclo[3.1.0]hexan-3-yl)carbamate **120e** (32 mg) with a yield of 30.24%.

**MS** m/z (ESI): 394.2 [M+1]

### Step 5

### N-(2-(3-aminobicyclo[3.1.0]hexan-3-yl)pyridin-4-yl)cyclopropanesulfonamide

Tert-butyl (3-(4-(cyclopropanesulfonamido)pyridin-2-yl)bicyclo[3.1.0]hexan-3-yl)carbamate **120e** (32 mg, 81.32 µmol) was added to a solution of hydrogen chloride in 1,4-dioxane (0.5 mL). The mixture was stirred at room temperature for 2 hours. The mixture was concentrated to dryness under reduced pressure to obtain N-(2-(3-aminobicyclo[3.1.0]hexan-3-yl)pyridin-4-yl)cyclopropanesulfonamide **120f** (20 mg) with a yield of 83.83%. The crude product was directly used in the next step.

MS m/z (ESI): 294.2 [M+1]

### Step 6

### N-(3-(4-(cyclopropanesulfonamido)pyridin-2-yl)bicyclo[3.1.0]hexan-3-yl)-5-(6-ethoxypyrazin-2-yl)thiazole-2-carboxamide

5-(6-Ethoxypyrazin-2-yl)thiazole-2-carboxylic acid 95a (30 mg, 119.40 µmol), N-(2-(3-aminobicyclo[3.1.0]hexan-3-yl)pyridin-4-yl)cyclopropanesulfonamide (35.03 mg, 119.40 µmol), O-(7-azabenzotriazol-1-yl)-N,N,N',N'-tetramethyluronium hexafluorophosphate (49.94 mg, 131.34 µmol), and N,N-diisopropylethylamine (46.29 mg, 358.19 µmol, 64.30 µL) were sequentially added to N,N-dimethylformamide (1.04 mL). The mixture was stirred at room temperature for 2 hours. Ethyl acetate (30 mL) and water (15 mL) were added, and the organic phase was separated. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(4-(cyclopropanesulfonamido)pyridin-2-yl)bicyclo[3.1.0]hexan-3-yl)-5-(6-ethoxypyrazin-2-yl)thiazole-2-carboxamide 120 (10.5 mg) with a yield of 13.45%.

**MS** m/z (ESI): 527.1 [M+1]
1H NMR (400 MHz, DMSO-d6) δ 9.11 (s, 1H), 8.93 (s, 1H), 8.81 (s, 1H), 8.40 (d, J = 6.0 Hz, 1H), 8.29 (s, 1H), 7.35 (d, J = 2.1 Hz, 1H), 7.19 (d, J = 5.9 Hz, 1H), 4.42 (q, J = 7.0 Hz, 2H), 2.91 - 2.80 (m, 1H), 2.59 (d, J = 4.5 Hz, 4H), 1.48 (dd, J = 8.1, 4.0 Hz, 2H), 1.38 (t, J = 7.0 Hz, 3H), 1.09 - 0.96 (m, 4H), 0.63 (dp, J = 8.4, 4.8 Hz, 2H).

### Example 121

### (1R,5S)-3-(2-(cyclopropanesulfonamido)pyrimidin-4-yl)-N-(4-(6-ethoxypyrazin-2-yl)-2-methoxy phenyl)-8-oxabicyclo[3.2.1]octane-3-carboxamide

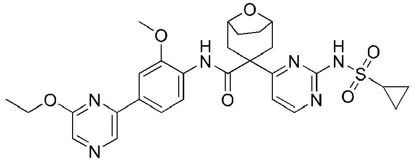

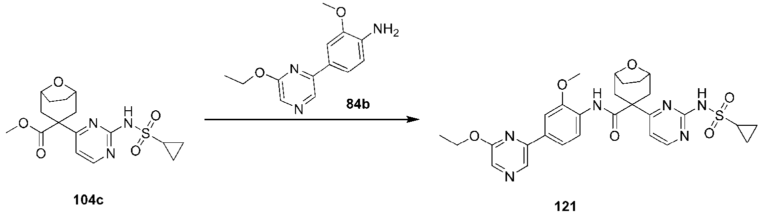

Under ice-water bath, trimethylaluminum (2.0 M n-hexane solution) (2 M, 489.90 µL) was added to a solution of 4-(6-ethoxypyrazin-2-yl)-2-methoxyaniline 84b (80.11 mg, 326.60 µmol) in toluene (2 mL). The mixture was stirred at room temperature for 1 hour. A solution of methyl 3-(2-(cyclopropanesulfonamido)pyrimidin-4-yl)-8-oxabicyclo[3.2.1]octane-3-carboxylate **104c** (100.00 mg, 272.17 µmol) in toluene was added, and the mixture was heated to 120°C and stirred for 8 hours. After the reaction was completed, the mixture was cooled to room temperature. Methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(2-(cyclopropanesulfonamido)pyrimidin-4-yl)-N-(4-(6-ethoxypyrazin-2-yl)-2-methoxy phenyl)-8-oxabicyclo[3.2.1]octane-3-carboxamide 121 (5 mg) with a yield of 2.50%.

**MS** m/z (ESI): 581.1 [M+1]
1H NMR (400 MHz, DMSO-d6) δ 11.32 (s, 1H), 8.93 (d, J = 4.0 Hz, 1H), 8.82 (d, J = 4.1 Hz, 1H), 8.57 (d, J = 5.0 Hz, 1H), 8.21 (d, J = 4.1 Hz, 1H), 7.72 (t, J = 6.2 Hz, 3H), 7.10 (d, J = 5.0 Hz, 1H), 4.49 - 4.39 (m, 4H), 3.88 (d, J = 4.0 Hz, 3H), 3.29 (s, 1H), 2.74 (d, J = 14.4 Hz, 2H), 2.25 (d, J = 14.3 Hz, 2H), 1.97 (d, J = 8.1 Hz, 2H), 1.78 (s, 2H), 1.39 (d, J = 5.6 Hz, 3H), 1.13 (s, 2H), 1.04 (d, J = 7.8 Hz, 2H).

### Example 122

### (1R,5S)-3-(2-(cyclopropanesulfonamido)pyrimidin-4-yl)-N-(4-(6-ethoxypyrazin-2-yl)-2-fluoroph enyl)-8-oxabicyclo[3.2.1]octane-3-carboxamide

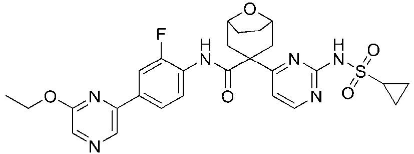

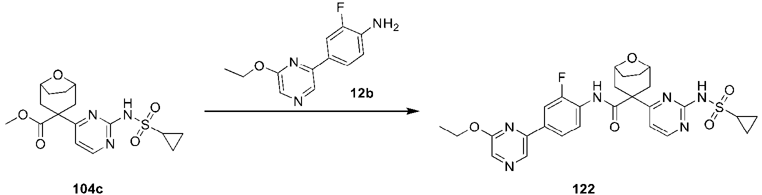

Under ice-water bath, trimethylaluminum (2.0 M n-hexane solution) (2 M, 489.90 µL) was added to a solution of 4-(6-ethoxypyrazin-2-yl)-2-fluoroaniline 12b (76.18 mg, 326.60 µmol) in toluene (2 mL). The mixture was stirred at room temperature for 1 hour, and then a toluene solution of methyl 3-(2-(cyclopropanesulfonamido)pyrimidin-4-yl)-8-oxabicyclo[3.2.1]octane-3-carboxylate **104c** (100.00 mg, 272.17 µmol) was added. The mixture was heated to 120°C and stirred for 8 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(2-(cyclopropanesulfonamido)pyrimidin-4-yl)-N-(4-(6-ethoxypyrazin-2-yl)-2-fluoroph enyl)-8-oxabicyclo[3.2.1]octane-3-carboxamide 122 (1.27 mg) with a yield of 0.75%.

**MS** m/z (ESI): 569.1 [M+1]
1H NMR (400 MHz, DMSO-d6) δ 11.29 (s, 1H), 9.58 (s, 1H), 8.84 (s, 1H), 8.57 (d, J = 5.3 Hz, 1H), 8.26 (s, 1H), 7.98 (t, J = 11.1 Hz, 2H), 7.51 (s, 1H), 7.07 (d, J = 5.3 Hz, 1H), 4.51 - 4.46 (m, 2H), 4.42 (s, 2H), 2.73 (d, J = 14.3 Hz, 2H), 2.27 (dd, J = 14.3, 4.0 Hz, 2H), 1.99 (t, J = 7.0 Hz, 3H), 1.79 (d, J = 7.1 Hz, 2H), 1.39 (d, J = 6.9 Hz, 3H), 1.16 - 1.10 (m, 2H), 1.03 (d, J = 7.7 Hz, 2H).

### Example 123

### 5-(5-chloropyridin-3-yl)-N-(3-(6-(cyclopropanesulfonamido)pyrimidin-4-yl)bicyclo[3.1.0]hexan-3-yl)thiazole-2-carboxamide

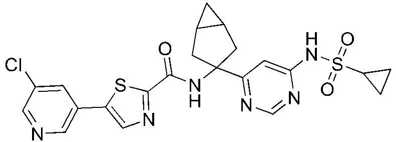

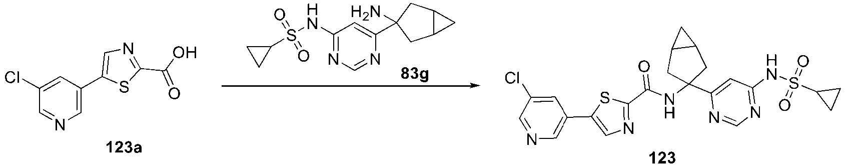

5-(5-Chloropyridin-3-yl)thiazole-2-carboxylic acid **123a** (18 mg, 74.79 µmol, prepared according to published patent "WO2022087634"), N-(6-(3-aminobicyclo[3.1.0]hexan-3-yl)pyrimidin-4-yl)cyclopropanesulfonamide 83g (31.08 mg, 89.75 µmol), O-(7-azabenzotriazol-1-yl)-N,N,N',N'-tetramethyluronium hexafluorophosphate (31.28 mg, 82.27 µmol), and N,N-diisopropylethylamine (29.00 mg, 224.38 µmol, 40.28 µL) were sequentially added to N,N-dimethylformamide (1.96 mL). The mixture was stirred continuously at room temperature for 2 hours. Ethyl acetate (30 mL) and water (15 mL) were added, and the mixture was separated. The aqueous phase was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 5-(5-chloropyridin-3-yl)-N-(3-(6-(cyclopropanesulfonamido)pyrimidin-4-yl)bicyclo[3.1.0]hexan-3-yl)thiazole-2-carboxamide 123 (8.0 mg) with a yield of 13.8%.

MS m/z (ESI): 517.1 [M+1]
1H NMR (400 MHz, DMSO-d6) δ 9.08 (s, 1H), 8.96 (d, J = 1.9 Hz, 1H), 8.76 (s, 1H), 8.68 (d, J = 2.1 Hz, 2H), 8.46 (t, J = 2.2 Hz, 1H), 6.99 (s, 1H), 3.04 (s, 1H), 2.59 (s, 4H), 1.46 (d, J = 7.9 Hz, 2H), 1.08 - 0.98 (m, 4H), 0.58 (dd, J = 9.2, 5.0 Hz, 1H), 0.53 (dt, J = 8.2, 4.1 Hz, 1H).

### Example 124

### (1R,5S)-3-(4-(cyclopropanesulfonamido)pyrimidin-2-yl)-N-(5-(6-ethoxypyrazin-2-yl)pyridin-2-y l)-8-oxabicyclo[3.2.1]octane-3-carboxamide

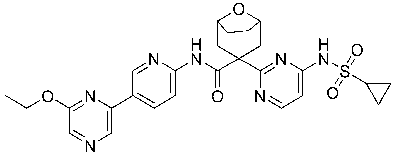

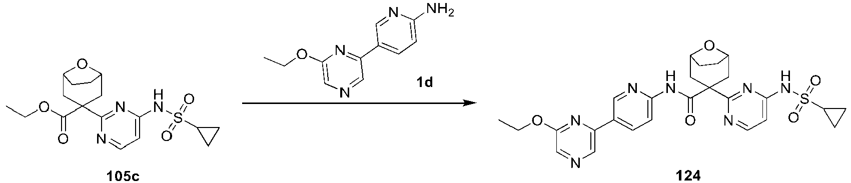

Under ice-water bath, trimethylaluminum (2.0 M toluene solution) (2 M, 489.90 µL) was added to a solution of 5-(6-ethoxypyrazin-2-yl)pyridin-2-amine **1d** (176.56 mg, 816.51 µmol) in toluene (2.5 mL). The mixture was stirred at room temperature for 1 hour, and then a toluene solution of methyl 3-(4-(cyclopropanesulfonamido)pyrimidin-2-yl)-8-oxabicyclo[3.2.1]octane-3-carboxylate **105c** (100.00 mg, 272.17 µmol) was added. The mixture was heated to 100°C and stirred for 5 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(4-(cyclopropanesulfonamido)pyrimidin-2-yl)-N-(5-(6-ethoxypyrazin-2-yl)pyridin-2-y l)-8-oxabicyclo[3.2.1]octane-3-carboxamide 124 (11 mg) with a yield of 6.01%.

**MS** m/z (ESI): 552.1 [M+1]
1H NMR (400 MHz, DMSO-d6) δ 11.24 (s, 1H), 10.12 (s, 1H), 9.01 (s, 1H), 8.83 (s, 1H), 8.51 (d, J = 6.9 Hz, 2H), 8.30 - 8.13 (m, 2H), 6.80 (d, J = 5.7 Hz, 1H), 4.48 (q, J = 7.1 Hz, 2H), 4.38 (s, 2H), 3.17 (s, 1H), 2.65 (d, J = 14.5 Hz, 2H), 2.45 (d, J = 12.5 Hz, 2H), 1.90 (d, J = 7.4 Hz, 2H), 1.75 (s, 2H), 1.40 (t, J = 7.1 Hz, 3H), 1.07 (s, 2H), 0.89 (d, J = 7.8 Hz, 2H).

### Example 125

### (1R,5S)-3-(4-(cyclopropanesulfonamido)pyrimidin-2-yl)-N-(4-(6-ethoxypyrazin-2-yl)-2-fluoroph enyl)-8-oxabicyclo[3.2.1]octane-3-carboxamide

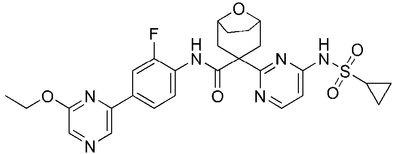

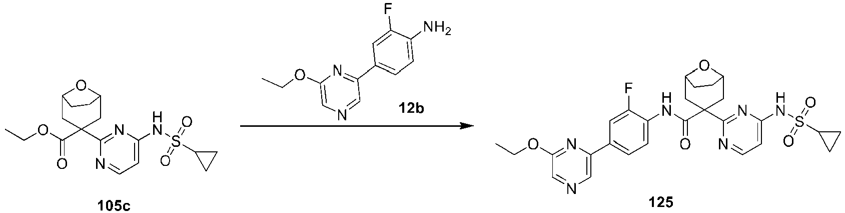

Under ice-water bath, trimethylaluminum (2.0 M toluene solution) (2 M, 489.90 µL) was added to a solution of 4-(6-ethoxypyrazin-2-yl)-2-fluoroaniline 12b (190.44 mg, 816.51 µmol) in toluene (2.5 mL). The mixture was stirred at room temperature for 1 hour, and then a toluene solution of methyl 3-(4-(cyclopropanesulfonamido)pyrimidin-2-yl)-8-oxabicyclo[3.2.1]octane-3-carboxylate 105c (100 mg, 272.17 µmol) was added. The mixture was heated to 120°C and stirred for 8 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(4-(cyclopropanesulfonamido)pyrimidin-2-yl)-N-(4-(6-ethoxypyrazin-2-yl)-2-fluoroph enyl)-8-oxabicyclo[3.2.1]octane-3-carboxamide 125 (20 mg) with a yield of 10.65%.

**MS** m/z (ESI): 569.1 [M+1]
1H NMR (400 MHz, DMSO-d6) δ 11.29 (s, 1H), 9.39 (s, 1H), 8.83 (s, 1H), 8.24 (s, 1H), 7.97 (d, J = 10.6 Hz, 2H), 7.76 - 7.65 (m, 2H), 6.83 (d, J = 6.9 Hz, 1H), 4.51 - 4.47 (m, 2H), 4.40 (s, 2H), 3.22 (s, 1H), 2.69 (d, J = 14.2 Hz, 2H), 2.42 (d, J = 15.2 Hz, 2H), 2.05 - 1.95 (m, 2H), 1.77 (s, 2H), 1.40 (d, J = 7.1 Hz, 3H), 1.13 (s, 2H), 1.01 (d, J = 7.7 Hz, 2H).

### Example 126

### (1R,5S)-3-(4-(cyclopropanesulfonamido)pyrimidin-2-yl)-N-(4-(6-ethoxypyrazin-2-yl)-2-methoxy phenyl)-8-oxabicyclo[3.2.1]octane-3-carboxamide

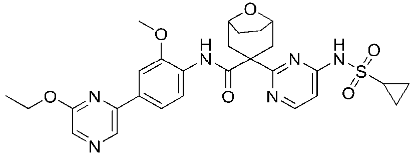

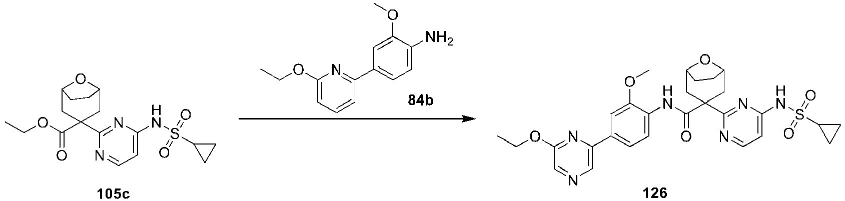

Under ice-water bath, trimethylaluminum (2.0 M toluene solution) (2 M, 489.90 µL) was added to a solution of 4-(6-ethoxypyrazin-2-yl)-2-methoxyaniline 84b (200.27 mg, 816.51 µmol) in toluene (2.5 mL). The mixture was stirred at room temperature for 1 hour. A toluene solution of methyl 3-(4-(cyclopropanesulfonamido)pyrimidin-2-yl)-8-oxabicyclo[3.2.1]octane-3-carboxylate **105c** (100 mg, 272.17 µmol) was added, and the mixture was heated to 100°C and stirred for 18 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain (1R,5S)-3-(4-(cyclopropanesulfonamido)pyrimidin-2-yl)-N-(4-(6-ethoxypyrazin-2-yl)-2-methoxy phenyl)-8-oxabicyclo[3.2.1]octane-3-carboxamide 126 (11 mg) with a yield of 5.37%.

**MS** m/z (ESI): 581.2 [M+1]
1H NMR (400 MHz, DMSO-d6) δ 11.31 (s, 1H), 8.90 (s, 1H), 8.81 (s, 1H), 8.57 (d, J = 5.7 Hz, 1H), 8.19 (s, 1H), 8.05 (d, J = 8.5 Hz, 1H), 7.71 (d, J = 5.2 Hz, 2H), 6.85 (d, J = 5.7 Hz, 1H), 4.47 (d, J = 7.1 Hz, 2H), 4.40 (s, 2H), 3.91 (s, 3H), 3.18 (dd, J = 9.1, 4.8 Hz, 1H), 2.67 (d, J = 14.3 Hz, 2H), 2.46 - 2.39 (m, 2H), 1.93 (d, J = 7.2 Hz, 2H), 1.77 (d, J = 6.6 Hz, 2H), 1.40 (t, J = 7.2 Hz, 3H), 1.17 - 1.09 (m, 2H), 1.03 (d, J = 7.6 Hz, 2H).

### Example 127

### N-(3-(6-(cyclopropanesulfonamido)pyrimidin-4-yl)bicyclo[3.1.0]hexan-3-yl)-5-(6-ethoxypyrazin -2-yl)thiophene-2-carboxamide

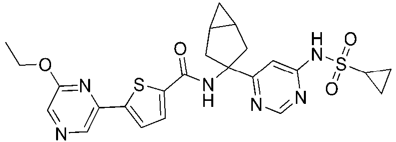

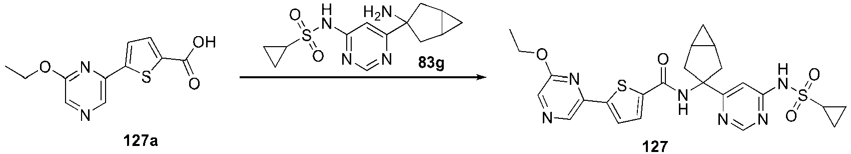

5-(6-Ethoxypyrazin-2-yl)thiophene-2-carboxylic acid **127a** (80 mg, 319.65 µmol, prepared according to published patent WO2022087634), N-(6-(3-aminobicyclo[3.1.0]hexan-3-yl)pyrimidin-4-yl)cyclopropanesulfonamide 83g (132.84 mg, 383.58 µmol), O-(7-azabenzotriazol-1-yl)-N,N,N',N'-tetramethyluronium hexafluorophosphate (133.69 mg, 351.62 µmol), and N,N-diisopropylethylamine (123.93 mg, 958.95 µmol, 172.13 µL) were sequentially added to N,N-dimethylformamide (1.57 mL) and stirred at room temperature for 2 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-ethoxypyrazin -2-yl)thiophene-2-carboxamide 127 (22.3 mg) with a yield of 12.97%.

MS m/z (ESI): 527.2 [M+1]
1H NMR (400 MHz, DMSO-d6) δ 8.80 (s, 1H), 8.75 (s, 1H), 8.67 (s, 1H), 8.21 (s, 1H), 8.03 - 7.93 (m, 2H), 6.97 (s, 1H), 4.42 (q, J = 7.0 Hz, 2H), 3.02 (s, 1H), 2.67 - 2.53 (m, 4H), 1.47 (dt, J = 8.1, 3.8 Hz, 2H), 1.39 (t, J = 7.0 Hz, 3H), 1.08 - 0.97 (m, 4H), 0.57 (dp, J = 13.0, 4.6 Hz, 2H).

### Example 128

### N-(3-(6-(cyclopropanesulfonamido)pyrimidin-4-yl)bicyclo[3.1.0]hexan-3-yl)-5-(6-ethoxypyrazin -2-yl)-4-methylthiazole-2-carboxamide

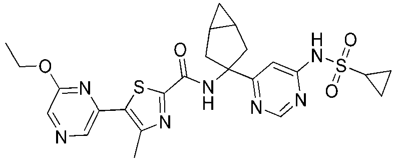

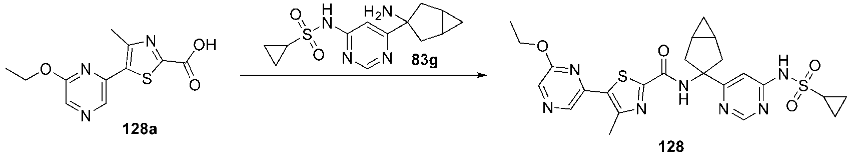

5-(6-Ethoxypyrazin-2-yl)-4-methylthiazole-2-carboxylic acid **128a** (80 mg, 301.56 µmol, prepared according to published patent WO2022087634), N-(6-(3-aminobicyclo[3.1.0]hexan-3-yl)pyrimidin-4-yl)cyclopropanesulfonamide 83g (125.32 mg, 361.87 µmol), O-(7-azabenzotriazol-1-yl)-N,N,N',N'-tetramethyluronium hexafluorophosphate (126.13 mg, 331.71 µmol), and N,N-diisopropylethylamine (116.92 mg, 904.68 µmol, 162.39 µL) were sequentially added to N,N-dimethylformamide (1.84 mL) and stirred at room temperature for 2 hours. Ethyl acetate (30 mL) and water (15 mL) were added, and the mixture was partitioned. The aqueous phase was extracted with ethyl acetate (30 mL × 2). The combined organic phase was washed with saturated sodium chloride solution (30 mL × 2), dried over anhydrous sodium sulfate, filtered, and concentrated to dryness under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-ethoxypyrazin -2-yl)-4-methylthiazole-2-carboxamide 128 (28.8 mg) with a yield of 16.2%.

**MS** m/z (ESI): 542.2 [M+1]
1H NMR (400 MHz, DMSO-d6) δ 8.95 (s, 1H), 8.76 (s, 1H), 8.61 (s, 1H), 8.30 (s, 1H), 6.98 (s, 1H), 4.42 (q, J = 7.0 Hz, 2H), 3.05 (s, 1H), 2.56 (d, J = 18.1 Hz, 7H), 1.47 (d, J = 7.6 Hz, 2H), 1.39 (t, J = 7.0 Hz, 3H), 1.04 (dtt, J = 10.3, 4.8, 2.4 Hz, 4H), 0.57 (dq, J = 16.7, 3.8, 3.3 Hz, 2H).

### Example 129

### N-(5-(6-ethoxypyrazin-2-yl)pyridin-2-yl)-3-(4-(methylsulfonamido)pyrimidin-2-yl)bicyclo[3.1.0] hexane-3-carboxamide

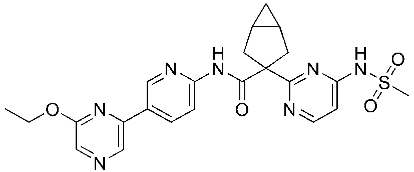

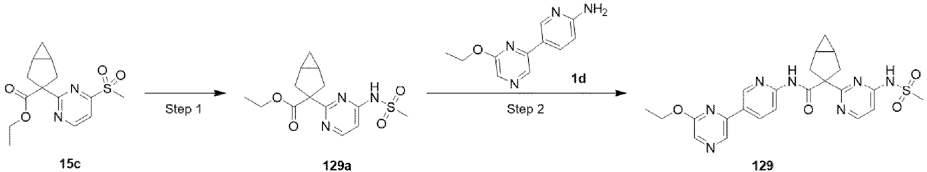

### Step 1

### ethyl 3-(4-(methylsulfonamido)pyrimidin-2-yl)bicyclo[3.1.0]hexane-3-carboxylate

Ethyl 3-(4-(methylsulfonyl)pyrimidin-2-yl)bicyclo[3.1.0]hexane-3-carboxylate **15c** (200 mg, 679.42 µmol), methanesulfonamide (129.25 mg, 1.36 mmol), and cesium carbonate (442.74 mg, 1.36 mmol) were dissolved in N-methylpyrrolidone (1 mL). The mixture was heated to 90°C and stirred for 3 hours. The solvent was removed by concentration under reduced pressure. The residue was separated by silica gel column chromatography (eluent: System A) to obtain ethyl 3-(4-(methylsulfonamido)pyrimidin-2-yl)bicyclo[3.1.0]hexane-3-carboxylate **129a** (180 mg) with a yield of 81.5%.

MS m/z (ESI): 326.0 [M+1]

### Step 2

### N-(5-(6-ethoxypyrazin-2-yl)pyridin-2-yl)-3-(4-(methylsulfonamido)pyrimidin-2-yl)bicyclo[3.1.0] hexane-3-carboxamide

Under ice-water bath, trimethylaluminum (2.0 M toluene solution) (2 M, 489.90 µL) was added to a solution of 5-(6-ethoxypyrazin-2-yl)pyridin-2-amine **1d** (117.71 mg, 544.34 µmol) in toluene (2.5 mL). The mixture was stirred at room temperature for 1 hour, and then a solution of ethyl 3-(4-(methylsulfonamido)pyrimidin-2-yl)bicyclo[3.1.0]hexane-3-carboxylate **129a** (84.74 mg, 272.17 µmol) in toluene was added. The mixture was heated to 100°C and stirred for 5 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(5-(6-ethoxypyrazin-2-yl)pyridin-2-yl)-3-(4-(methylsulfonamido)pyrimidin-2-yl)bicyclo[3.1.0] hexane-3-carboxamide 129 (45 mg) with a yield of 25.58%.

**MS** m/z (ESI): 552.1 [M+1]
1H NMR (400 MHz, DMSO-d6) δ 11.10 (s, 1H), 9.96 (s, 1H), 8.86 (s, 1H), 8.68 (s, 1H), 8.34 (t, J = 6.7 Hz, 2H), 8.09 (s, 1H), 8.05 (d, J = 8.8 Hz, 1H), 6.62 (d, J = 5.7 Hz, 1H), 4.33 (q, J = 7.0 Hz, 2H), 3.20 (s, 3H), 2.66 (d, J = 13.6 Hz, 2H), 2.44 (dd, J = 13.6, 3.3 Hz, 2H), 1.25 (t, J = 6.9 Hz, 5H), 0.25 (d, J = 6.6 Hz, 1H), -0.01 (q, J = 4.3 Hz, 1H).

### Example 130

### N-(3-(6-(cyclopropanesulfonamido)pyrimidin-4-yl)bicyclo[3.1.0]hexan-3-yl)-5-(6-(trifluorometh yl)pyrazin-2-yl)thiazole-2-carboxamide

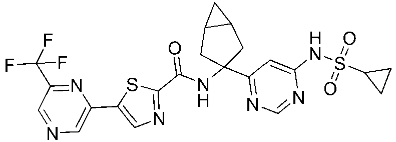

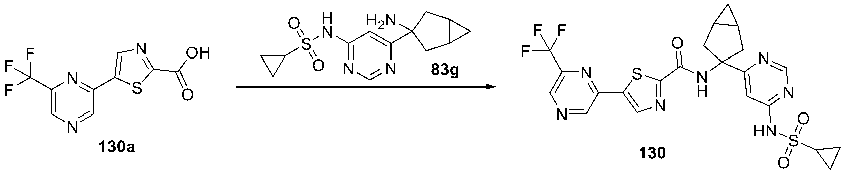

5-(6-(Trifluoromethyl)pyrazin-2-yl)thiazole-2-carboxylic acid 130a (100 mg, 363.36 µmol, prepared according to published patent "WO2022087634"), N-(6-(3-aminobicyclo[3.1.0]hexan-3-yl)pyrimidin-4-yl)cyclopropanesulfonamide 83g (151.01 mg, 436.04 µmol), O-(7-azabenzotriazol-1-yl)-N,N,N',N'-tetramethyluronium hexafluorophosphate (151.98 mg, 399.70 µmol), and N,N-diisopropylethylamine (140.88 mg, 1.09 mmol, 195.67 µL) were sequentially added to N,N-dimethylformamide (2 mL). The mixture was stirred at room temperature for 2 hours. Ethyl acetate (30 mL) and water (15 mL) were added, and the mixture was partitioned. The aqueous phase was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-(trifluorometh yl)pyrazin-2-yl)thiazole-2-carboxamide 130 (19.7 mg) with a yield of 9.27%.

**MS** m/z (ESI): 552.1 [M+1]
1H NMR (400 MHz, DMSO-d6) δ 9.74 (s, 1H), 9.18 (s, 2H), 9.02 (s, 1H), 8.77 (s, 1H), 7.01 (s, 1H), 3.02 (q, J = 6.4, 5.9 Hz, 1H), 2.59 (s, 4H), 1.47 (d, J = 7.9 Hz, 2H), 1.08 - 0.97 (m, 4H), 0.57 (ddt, J = 21.2, 8.2, 4.6 Hz, 2H).

### Example 131

### N-(3-(2-(cyclopropanesulfonamido)pyridin-4-yl)bicyclo[3.1.0]hexan-3-yl)-5-(6-ethoxypyrazin-2-yl)thiazole-2-carboxamide

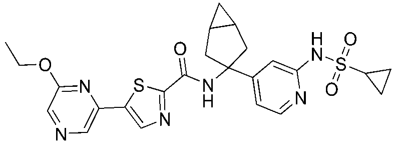

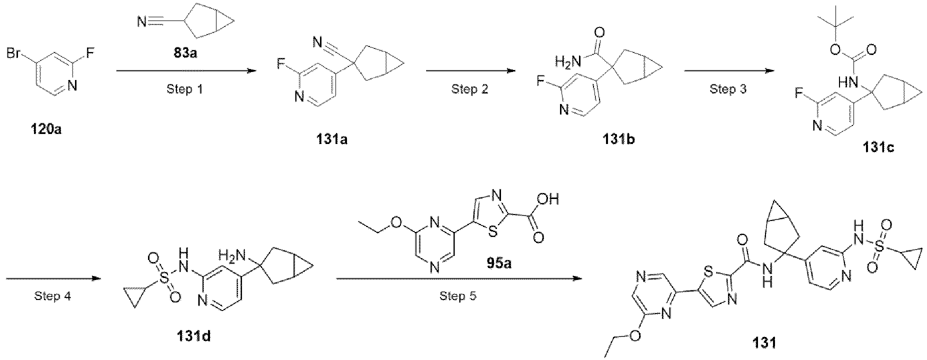

### Step 1

### 3-(2-fluoropyridin-4-yl)bicyclo[3.1.0]hexane-3-carbonitrile

At -30°C, lithium bis(trimethylsilyl)amide (1 M, 5.60 mL) was added to a solution of 4-bromo-2-fluoropyridine **120a** (492.72 mg, 2.80 mmol) and bicyclo[3.1.0]hexane-3-carbonitrile 83a (300 mg, 2.80 mmol) in tetrahydrofuran (5.00 mL). The mixture was heated to room temperature and stirred for 2 hours. After the starting material was completely reacted, saturated ammonium chloride aqueous solution (30 mL) was added to quench the reaction. The mixture was extracted with ethyl acetate (30 mL × 3). The combined organic phase was washed with saturated sodium chloride solution (30 mL), dried over anhydrous sodium sulfate, filtered, and evaporated to dryness by rotation. The residue was separated by silica gel column chromatography (eluent: System A) to obtain 3-(2-fluoropyridin-4-yl)bicyclo[3.1.0]hexane-3-carbonitrile **131a** (240 mg) with a yield of 42.39%.

**MS** m/z (ESI): 203.1 [M+1]

### Step 2

### 3-(2-fluoropyridin-4-yl)bicyclo[3.1.0]hexane-3-carboxamide

Hydrogen peroxide (0.5 mL) was added dropwise to a mixed solution of 3-(2-fluoropyridin-4-yl)bicyclo[3.1.0]hexane-3-carbonitrile **131a** (240 mg, 1.19 mmol) in sodium hydroxide aqueous solution (0.5 mL, 2.5 M) and dimethyl sulfoxide (1 mL). The mixture was stirred at room temperature for 1 hour. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated. The residue was purified by silica gel column chromatography (eluent: System A) to obtain 3-(2-fluoropyridin-4-yl)bicyclo[3.1.0]hexane-3-carboxamide **131b** (250 mg) with a yield of 95.65%.

MS m/z (ESI): 221.0 [M+1]

### Step 3

### tert-butyl (3-(2-fluoropyridin-4-yl)bicyclo[3.1.0]hexan-3-yl)carbamate

3-(2-Fluoropyridin-4-yl)bicyclo[3.1.0]hexane-3-carboxamide **131b** (400 mg, 1.82 mmol) and lead(IV) acetate (3.22 g, 7.26 mmol) were sequentially added to tert-butanol (1 mL). The mixture was heated to 100°C with continuous stirring for 3 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated. The residue was purified by silica gel column chromatography (eluent: System A) to obtain tert-butyl (3-(2-fluoropyridin-4-yl)bicyclo[3.1.0]hexan-3-yl)carbamate **131c** (200 mg) with a yield of 37.6%.

**MS** m/z (ESI): 293.2 [M+1]

### Step 4

### N-(4-(3-aminobicyclo[3.1.0]hexan-3-yl)pyridin-2-yl)cyclopropanesulfonamide

Tert-butyl (3-(2-fluoropyridin-4-yl)bicyclo[3.1.0]hexan-3-yl)carbamate **131c** (130 mg, 444.68 µmol), cyclopropanesulfonamide (107.75 mg, 889.35 µmol), and cesium carbonate (289.77 mg, 889.35 µmol) were sequentially added to dimethyl sulfoxide (2 mL). The mixture was heated in a sealed tube at 150°C with continuous stirring for 5 hours. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(4-(3-aminobicyclo[3.1.0]hexan-3-yl)pyridin-2-yl)cyclopropanesulfonamide **131d** (20 mg) with a yield of 15.33%.

**MS** m/z (ESI): 294.1 [M+1]

### Step 5

### N-(3-(2-(cyclopropanesulfonamido)pyridin-4-yl)bicyclo[3.1.0]hexan-3-yl)-5-(6-ethoxypyrazin-2-yl)thiazole-2-carboxamide

5-(6-Ethoxypyrazin-2-yl)thiazole-2-carboxylic acid 95a (25.69 mg, 102.26 µmol), N-(4-(3-aminobicyclo[3.1.0]hexan-3-yl)pyridin-2-yl)cyclopropanesulfonamide **131d** (30 mg, 102.26 µmol), O-(7-azabenzotriazol-1-yl)-N,N,N',N'-tetramethyluronium hexafluorophosphate (42.77 mg, 112.48 µmol), and N,N-diisopropylethylamine (39.65 mg, 306.77 µmol, 55.06 µL) were sequentially added to N,N-dimethylformamide (1 mL). The mixture was stirred at room temperature for 2 hours. Ethyl acetate (30 mL) and water (15 mL) were added, and the mixture was separated. The aqueous phase was extracted with ethyl acetate (30 mL × 2). The combined organic phase was washed with saturated sodium chloride solution (30 mL × 2), dried over anhydrous sodium sulfate, filtered, and concentrated under reduced pressure to dryness. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(2-(cyclopropanesulfonamido)pyridin-4-yl)bicyclo[3.1.0]hexan-3-yl)-5-(6-ethoxypyrazin-2-yl)thiazole-2-carboxamide 131 (5.6 mg) with a yield of 7.76%.

**MS** m/z (ESI): 527.2 [M+1]
1H NMR (400 MHz, DMSO-d6) δ 9.06 (s, 1H), 8.91 (s, 1H), 8.79 (s, 1H), 8.28 (s, 1H), 8.08 (d, J = 5.7 Hz, 1H), 7.09 (s, 1H), 6.99 (d, J = 5.8 Hz, 1H), 4.41 (q, J = 7.0 Hz, 2H), 2.93 (s, 1H), 2.59 (d, J = 14.3 Hz, 3H), 2.42 - 2.31 (m, 3H), 1.46 (dt, J = 8.2, 4.0 Hz, 2H), 1.38 (t, J = 7.0 Hz, 3H), 1.03 - 0.89 (m, 4H), 0.62 (dt, J = 12.9, 4.6 Hz, 2H).

### Example 132

### N-(3-(6-(cyclopropanesulfonamido)pyrimidin-4-yl)bicyclo[3.1.0]hexan-3-yl)-5-(6-methoxypyraz in-2-yl)thiazole-2-carboxamide

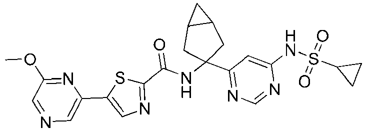

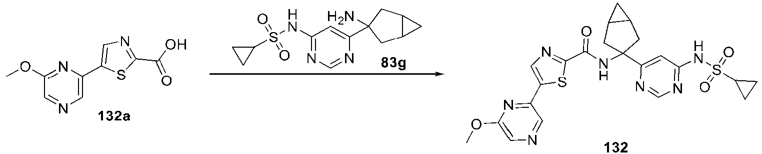

5-(6-Methoxypyrazin-2-yl)thiazole-2-carboxylic acid **132a** (85 mg, 358.29 µmol, prepared according to published patent "WO2022087634"), N-(6-(3-aminobicyclo[3.1.0]hexan-3-yl)pyrimidin-4-yl)cyclopropanesulfonamide 83g (148.90 mg, 429.95 µmol), O-(7-azabenzotriazol-1-yl)-N,N,N',N'-tetramethyluronium hexafluorophosphate (149.86 mg, 394.12 µmol), and N,N-diisopropylethylamine (1.07 mmol, 192.94 µL) were sequentially added to N,N-dimethylformamide (2 mL). The mixture was stirred at room temperature for 2 hours. Ethyl acetate (30 mL) and water (15 mL) were added, and the mixture was separated. The aqueous phase was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(3-(6-(cyclopropanesulfonamido)pyrimidin-4-yl)bicyclo[3.1.0]hexan-3-yl)-5-(6-methoxypyraz in-2-yl)thiazole-2-carboxamide 132 (18.0 mg) with a yield of 9.72%.

**MS** m/z (ESI): 514.1 [M+1]
1H NMR (400 MHz, DMSO-d6) δ 9.07 (s, 1H), 8.95 (s, 1H), 8.83 (s, 1H), 8.77 (s, 1H), 8.32 (s, 1H), 6.99 (s, 1H), 3.98 (s, 3H), 3.04 (s, 1H), 2.59 (s, 4H), 1.50 - 1.43 (m, 2H), 1.10 - 0.99 (m, 4H), 0.59 (dd, J = 10.7, 6.5 Hz, 1H), 0.54 (dt, J = 8.2, 4.1 Hz, 1H).

### Example 133

### N-(5-(6-ethoxypyrazin-2-yl)pyridin-2-yl)-3-(6-(ethylsulfonamido)pyrimidin-4-yl)bicyclo[3.1.0]h exane-3-carboxamide

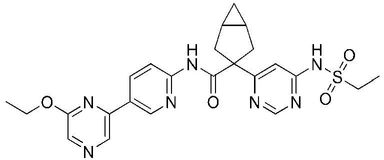

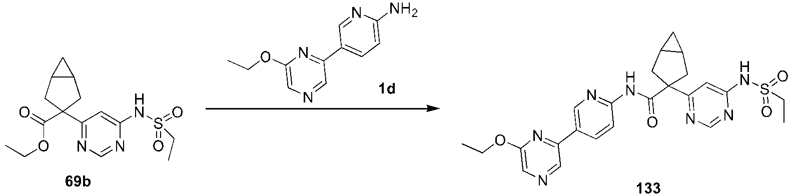

Trimethylaluminum (2.0 M toluene solution) (2 M, 530.33 µL) was added to a solution of 5-(6-ethoxypyrazin-2-yl)pyridin-2-amine **1d** (95.57 mg, 441.94 µmol) in toluene (2 mL). The mixture was stirred at room temperature for 1 hour. Ethyl 3-(6-(ethylsulfonamido)pyrimidin-4-yl)bicyclo[3.1.0]hexane-3-carboxylate **69b** (100 mg, 294.63 µmol) was added, and the mixture was heated to 110°C and stirred for 5 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain N-(5-(6-ethoxypyrazin-2-yl)pyridin-2-yl)-3-(6-(ethylsulfonamido)pyrimidin-4-yl)bicyclo[3.1.0]h exane-3-carboxamide 133 (10 mg) with a yield of 5.12%.

**MS** m/z (ESI): 510.2 [M+1]
1H NMR (400 MHz, DMSO-d6) δ 10.16 (s, 1H), 8.89 (d, J = 2.4 Hz, 1H), 8.70 (s, 1H), 8.62 (s, 1H), 8.36 (dd, J = 8.8, 2.4 Hz, 1H), 8.11 (s, 1H), 8.02 (d, J = 8.7 Hz, 1H), 6.80 (s, 1H), 4.34 (q, J = 7.0 Hz, 2H), 3.36 (m, 2 H), 2.66 (d, J = 13.7 Hz, 2H), 2.26 (dd, J = 13.8, 3.5 Hz, 2H), 1.26 (t, J = 7.0 Hz, 5H), 1.06 (t, J = 7.3 Hz, 3H), 0.31 (d, J = 5.5 Hz, 1H), -0.01 (d, J = 4.4 Hz, 1H).

### Example 134

### 3-(6-(cyclopropanesulfonamido)pyrimidin-4-yl)-N-(4-(6-ethoxypyrazin-2-yl)-2-methoxyphenyl) bicyclo[3.1.0]hexane-3-carboxamide

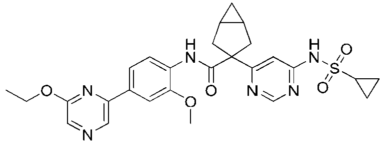

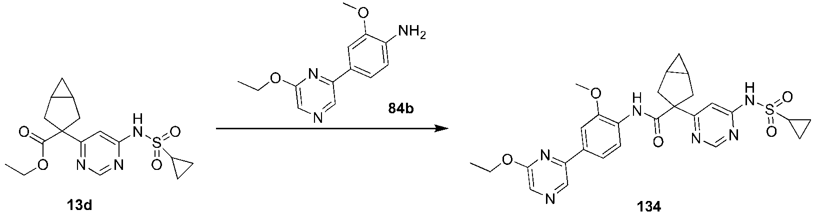

Trimethylaluminum (2.0 M toluene solution) (2 M, 512.21 µL) was added to a solution of 4-(6-ethoxypyrazin-2-yl)-2-methoxyaniline 84b (104.69 mg, 426.84 µmol) in toluene (3.00 mL). The mixture was stirred at room temperature for 1 hour. Ethyl 3-(6-(cyclopropanesulfonamido)pyrimidin-4-yl)bicyclo[3.1.0]hexane-3-carboxylate 13d (100 mg, 284.56 µmol) was added, and the mixture was heated to 110°C and stirred for 5 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(6-(cyclopropanesulfonamido)pyrimidin-4-yl)-N-(4-(6-ethoxypyrazin-2-yl)-2-methoxyphenyl) bicyclo[3.1.0]hexane-3-carboxamide 134 (13 mg) with a yield of 6.08%.

**MS** m/z (ESI): 551.2 [M+1]
1H NMR (400 MHz, DMSO-d6) δ 8.94 (s, 1H), 8.59 (d, J = 6.6 Hz, 2H), 7.96 (s, 1H), 7.71 (d, J = 8.4 Hz, 1H), 7.47 (d, J = 10.2 Hz, 2H), 6.70 (s, 1H), 4.23 (q, J = 7.2 Hz, 2H), 3.70 (s, 3H), 2.85 (s, 1H), 2.58 (d, J = 13.5 Hz, 2H), 2.16 (d, J = 13.1 Hz, 2H), 1.16 (t, J = 7.1 Hz, 5H), 0.91 - 0.73 (m, 4H), 0.29 - 0.14 (m, 1H), -0.00 (s, 1H).

### Example 135

### 3-(4-(cyclopropanesulfonamido)pyridin-2-yl)-N-(4-(6-ethoxypyrazin-2-yl)-2-methoxyphenyl)bic yclo[3.1.0]hexane-3-carboxamide

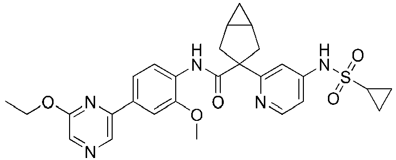

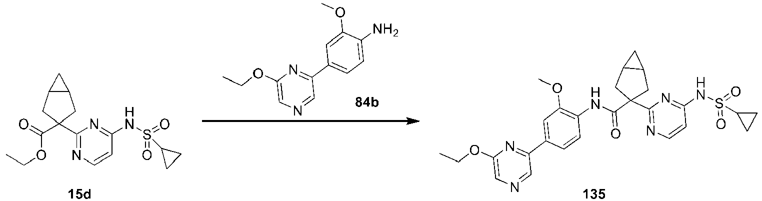

Trimethylaluminum (2.0 M toluene solution) (2 M, 512.21 µL) was added to a solution of 4-(6-ethoxypyrazin-2-yl)-2-methoxyaniline 84b (104.69 mg, 426.84 µmol) in toluene (3 mL). The mixture was stirred at room temperature for 1 hour. Ethyl 3-(4-(cyclopropanesulfonamido)pyrimidin-2-yl)bicyclo[3.1.0]hexane-3-carboxylate 15d (99.72 mg, 284.56 µmol) was added, and the mixture was heated to 110°C and stirred for 5 hours. The mixture was cooled to room temperature, and methanol was added to quench the reaction. The pH was adjusted to 4-5 with 1 N hydrochloric acid. The mixture was extracted with ethyl acetate (30 mL × 3). The combined organic phase was washed with saturated sodium chloride solution (30 mL), dried over anhydrous sodium sulfate, and concentrated under reduced pressure. The residue was separated by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyridin-2-yl)-N-(4-(6-ethoxypyrazin-2-yl)-2-methoxyphenyl)bic yclo[3.1.0]hexane-3-carboxamide **135** (16 mg) with a yield of 7.70%.

**MS** m/z (ESI): 550.2 [M+1]
1H NMR (400 MHz, DMSO-d6) δ 10.55 (s, 1H), 9.22 (s, 1H), 8.80 (s, 1H), 8.47 (d, J = 5.4 Hz, 1H), 8.20 - 8.01 (m, 2H), 7.70 (d, J = 8.2 Hz, 2H), 7.11 (s, 2H), 4.46 (q, J = 7.2 Hz, 2H), 3.95 (s, 3H), 3.34 (s, 1H), 2.83 (d, J = 13.9 Hz, 2H), 2.43 (d, J = 13.4 Hz, 2H), 1.39 (t, J = 7.1 Hz, 5H), 1.05 - 0.94 (m, 4H), 0.43 (d, J = 7.1 Hz, 1H), 0.26 (s, 1H).

### Example 136

### 3-(4-(cyclopropanesulfonamido)pyrimidin-2-yl)-N-(5-(6-ethoxypyrazin-2-yl)thiazol-2-yl)bicyclo [3.1.0]hexane-3-carboxamide

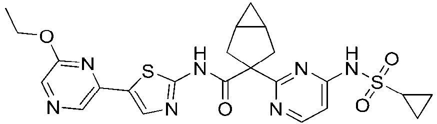

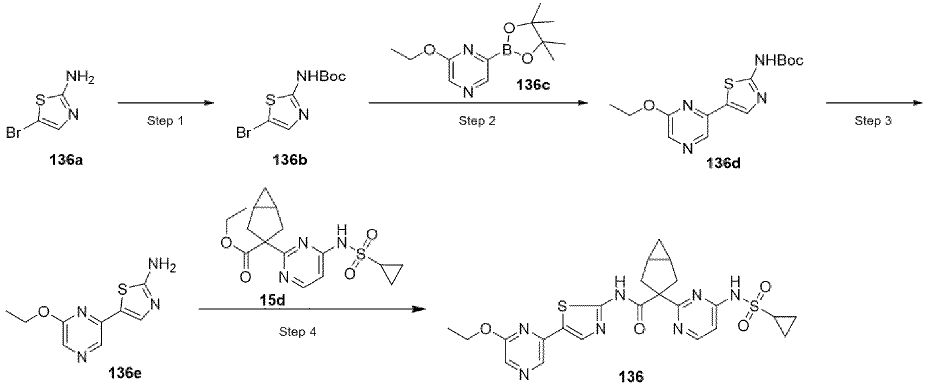

### Step 1

### tert-butyl (5-bromothiazol-2-yl)carbamate

5-Bromothiazol-2-amine 136a (3 g, 16.76 mmol, commercially available), triethylamine (5.09 g, 50.27 mmol, 7.06 mL), di-tert-butyl dicarbonate (4.02 g, 18.43 mmol), and 4-dimethylaminopyridine (204.71 mg, 1.68 mmol) were dissolved in tetrahydrofuran (30 mL) and stirred at room temperature for 2 hours. After the reaction was completed, the solvent was evaporated. Water (30 mL) was added, and the mixture was extracted with dichloromethane (30 mL × 3). The combined organic phase was washed with saturated sodium chloride solution (30 mL), dried over anhydrous sodium sulfate, filtered, and concentrated under reduced pressure to dryness. The crude product was purified by silica gel column chromatography (eluent: System A) to obtain tert-butyl (5-bromothiazol-2-yl)carbamate 136b (4 g) with a yield of 85.51%.

MS m/z (ESI): 223.0 [M+1-56]

### Step 2

### tert-butyl (5-(6-ethoxypyrazin-2-yl)thiazol-2-yl)carbamate

2-Ethoxy-6-(4,4,5,5-tetramethyl-1,3,2-dioxaborolan-2-yl)pyrazine 136c (4.10 g, 11.46 mmol, commercially available), tert-butyl (5-bromothiazol-2-yl)carbamate 136b (1.6 g, 5.73 mmol), [1,1'-bis(diphenylphosphino)ferrocene]palladium(II) dichloride (419.38 mg, 573.16 µmol), and potassium carbonate (2.38 g, 17.19 mmol) were sequentially added to 1,4-dioxane (50 mL) and water (10 mL). The mixture was subjected to argon exchange three times and heated to 100°C with stirring for 2 hours. The solvent was evaporated, and the crude product was purified by silica gel column chromatography (eluent: System A) to obtain tert-butyl (5-(6-ethoxypyrazin-2-yl)thiazol-2-yl)carbamate 136d (500 mg, 1.55 mmol) with a yield of 27.06%.

MS m/z (ESI): 323.1 [M+1]/267.1 [M+1-56]

### Step 3

### 5-(6-ethoxypyrazin-2-yl)thiazol-2-amine

tert-butyl (5-(6-ethoxypyrazin-2-yl)thiazol-2-yl)carbamate 136d (200 mg, 620.38 µmol) was dissolved in 4 M hydrochloric acid in 1,4-dioxane solution (10 mL) and stirred at room temperature for 18 hours. After the reaction was completed, the solvent was evaporated to obtain 5-(6-ethoxypyrazin-2-yl)thiazol-2-amine 136e (130 mg, hydrochloride) with a yield of 94.28%. No workup was performed, and the product was directly used in the next step.

MS m/z (ESI): 223.1 [M+1]

### Step 4

### 3-(4-(cyclopropanesulfonamido)pyrimidin-2-yl)-N-(5-(6-ethoxypyrazin-2-yl)thiazol-2-yl)bicyclo [3.1.0]hexane-3-carboxamide

Trimethylaluminum (2.0 M toluene solution) (2 M, 102.44 µL) was added to a solution of 5-(6-ethoxypyrazin-2-yl)thiazol-2-amine 136e (18.97 mg, 85.37 µmol) in toluene (2 mL), and the mixture was stirred at room temperature for 1 hour. Subsequently, ethyl 3-(4-(cyclopropanesulfonamido)pyrimidin-2-yl)bicyclo[3.1.0]hexane-3-carboxylate 15d (20 mg, 56.91 µmol) was added, and the mixture was heated to 120°C and stirred for 5 hours. The mixture was cooled to room temperature, and methanol was added to quench the reaction. The pH was adjusted to 4-5 with 1 N hydrochloric acid, and the mixture was extracted with ethyl acetate (30 mL × 3). The combined organic phase was washed with saturated sodium chloride solution (30 mL), dried over anhydrous sodium sulfate, and concentrated under reduced pressure. The residue was purified by preparative liquid chromatography (separation column AKZONOBEL Kromasil; 250 × 21.2 mm I.D.; 5 µm, 20 mL/min; mobile phase A: 0.05% TFA + H2O, mobile phase B: CH3CN) to obtain 3-(4-(cyclopropanesulfonamido)pyrimidin-2-yl)-N-(5-(6-ethoxypyrazin-2-yl)thiazol-2-yl)bicyclo [3.1.0]hexane-3-carboxamide 136 (1.02 mg) with a yield of 2.52%.

MS m/z (ESI): 510.2 [M+1]
1H NMR (400 MHz, DMSO-d6) δ 12.15 (s, 1H), 11.22 (s, 1H), 8.74 (s, 1H), 8.48 (s, 1H), 8.26 (s, 1H), 8.12 (s, 1H), 6.79 (s, 1H), 4.42 (d, J = 8.6 Hz, 2H), 3.05 (s, 1H), 2.78 (d, J = 13.4 Hz, 2H), 2.62 (d, J = 13.4 Hz, 2H), 1.53 - 1.34 (m, 5H), 1.04 (s, 2H), 0.84 (s, 2H), 0.38 (s, 1H), 0.00 (s, 1H).

### Biological Evaluation

### Test Example 1: Inhibition on Jurkat cell proliferation by the compounds of the present disclosure

The following method was used to determine the effect of the compounds of the present disclosure on Jurkat cell proliferation. Jurkat (Clone E6-1) cells were purchased from the Cell Resource Center of Shanghai Institutes for Biological Sciences, Chinese Academy of Sciences, and were cultured in RPMI 1640 medium containing 10% fetal bovine serum, 100 U penicillin, and 100 µg/mL streptomycin. Cell viability was measured using a CellTiter-Glo^{®} Luminescent Cell Viability Assay kit (Promega, catalog number G7573).

The experiment was performed according to the kit's manual, briefly summarized as follows: test compounds were first dissolved in DMSO to prepare 10 mM stock solutions, which were then diluted with medium to prepare test samples. The final concentration range of the compounds was from 10000 nM to 1.52 nM. Cells in the logarithmic growth phase were seeded into a 96-well cell culture plate at a density of 1000 cells per well, and test compounds were added. The cells were cultured in a 37°C CO₂ incubator for 72 hours. After culturing, 50 µL of CellTiter-Glo detection reagent was added to each well, the plate was shaken for 5 minutes, and allowed to stand for 10 minutes. The luminescence of each well was then measured using a microplate reader in Luminescence mode. The percentage inhibition at each concentration point was calculated by comparison with the control group (0.1% DMSO). Nonlinear regression analysis of compound concentration logarithm versus inhibition rate was performed using GraphPad Prism 9 software to obtain the IC₅₀ values of the compounds for inhibiting cell proliferation, as shown in Table 1.

**Table 1 IC₅₀ Data for Inhibition on Jurkat Cell Proliferation by the Compounds of the Present Disclosure**

| Example No. | IC₅₀(nM) | Example No. | IC₅₀(nM) | Example No. | IC₅₀(nM) |
|---|---|---|---|---|---|
| 1 | 72.3 | 45 | 16.6 | 102 | 11.9 |
| 2 | 67.4 | 48 | 42.8 | 104 | 13.8 |
| 3 | 69.7 | 50 | 10.6 | 105 | 2.79 |
| 4 | 60.8 | 53 | 27.2 | 106 | 98.7 |
| 7 | 29.8 | 57 | 8.35 | 107 | 22.96 |
| 10 | 48.9 | 58 | 16.9 | 108 | 44.55 |
| 12 | 64.9 | 59 | 12.9 | 109 | 81.4 |
| 13 | 3.59 | 60 | 5.2 | 110 | 26.5 |
| 14 | 21.7 | 61 | 40.84 | 111 | 12.1 |
| 15 | 14.2 | 63 | 28.7 | 112 | 7.42 |
| 16 | 42.1 | 64 | 9.11 | 113 | 10.6 |
| 17 | 1.58 | 65 | 16.5 | 114 | 4.29 |
| 18 | 63.7 | 66 | 15.6 | 115 | 8.30 |
| 20 | 7.24 | 68 | 34.8 | 116 | 37.9 |
| 21 | 8.81 | 69 | 8.48 | 117 | 41.7 |
| 23 | 48.8 | 77 | 40.2 | 119 | 14.79 |
| 24 | 6.96 | 80 | 76.9 | 120 | 2.97 |
| 26 | 11.1 | 82 | 1.37 | 121 | 54.6 |
| 28 | 3.37 | 83 | 31.9 | 122 | 49.4 |
| 29 | 3.3 | 84 | 8.73 | 123 | 86.1 |
| 31 | 30.1 | 85 | 57.2 | 124 | 3.24 |
| 32 | 8.45 | 86 | 33.4 | 125 | 15.18 |
| 33 | 10.69 | 87 | 84.9 | 126 | 14.14 |
| 34 | 25.66 | 89 | 50.6 | 127 | 2.37 |
| 36 | 15.75 | 91 | 26.9 | 128 | 2.49 |
| 37 | 40.81 | 95 | 4.32 | 129 | 11.59 |
| 38 | 2.49 | 97 | 13.4 | 130 | 21.48 |
| 39 | 9.19 | 99 | 5.59 | 132 | 24.38 |
| 41 | 27.7 | 100 | 9.17 | 133 | 4.79 |
| 42 | 24.2 | 101 | 4.1 | 134 | 4.48 |

Conclusion: The compounds of the present disclosure have a good inhibitory effect on Jurkat cell proliferation with an IC₅₀ <100 nM.

### Test Example 2: Inhibition on hCTPS1 enzyme activity by the compounds of the present disclosure

The following method was used to determine the degree of inhibition on human cytidine triphosphate synthase 1 (hCTPS1) enzyme activity by the compounds of the present disclosure *in vitro.* ADP-Glo^{™} Kinase Assay kit (catalog number V9102) from Promega was used in this method. The kit's manual may be referred for a detailed experimental procedure.

The experimental procedure was briefly described as follows: The reaction was performed in a buffer containing 50 mM 4-hydroxyethylpiperazineethanesulfonic acid (HEPES), 10 mM magnesium chloride, 5 mM potassium chloride, 0.01% Pluronic F127, and 4 mM dithiothreitol in a 384-well plate. First, 100 nL of gradient-diluted compound was transferred to the 384-well plate via a nanoliter acoustic liquid transfer system (manufacturer: Labcyte; catalog number: Echo 655), and 2.5 µL of buffer containing hCTPS1 was added, followed by incubation in a 25°C incubator for 10 minutes. Then 2.5 µL of buffer containing ATP and UTP was added, followed by incubation in a 25°C incubator for 10 minutes. Finally, 5 µL of buffer containing GTP and L-glutamine (L-Gln) was added to initiate the reaction. The final reaction system was: 40 nM hCTPS1, 120 µM ATP, 160 µM UTP, 60 µM GTP, 100 µM L-Glutamine, 1% DMSO, with a total assay volume of 10 µL. The reaction was performed for 120 minutes, then 10 µL of ADP-Glo Reagent was added, followed by incubation for 1 hour. Then 20 µL of Kinase Detection Reagent was added, and after 1 hour, the chemiluminescence value was recorded on a Microbeta microplate reader (manufacturer: PerkinElmer; catalog number: Envision). The percentage inhibition rates of the compounds at each concentration were calculated by comparing the chemiluminescence intensity ratio with a control group (0.1% DMSO). The IC₅₀ value of the compounds were obtained by nonlinear regression analysis of compound concentration logarithm-inhibition rate using GraphPad Prism 5 software, as shown in Table 2.

**Table 2 IC₅₀ data for inhibition on hCTPS1 enzyme activity by the compounds of the present disclosure**

| Example No. | IC₅₀(nM) | Example No. | IC₅₀(nM) |
|---|---|---|---|
| 1 | 25.3 | 28 | 5.9 |
| 3 | 18 | 33 | 20.1 |
| 7 | 16.3 | 36 | 14.5 |
| 13 | 11 | 84 | 10.7 |
| 15 | 5.2 | 95 | 9.2 |
| 17 | 5.6 | STP-B | 37.2 |
| 26 | 9.0 | | |

Conclusion: The compounds of the present disclosure have significant inhibitory effect on hCTPS1 enzyme activity with an IC₅₀ <50 nM.

Reference compound STP-B was prepared according to Example P115 of published patent WO2021053403A1, with a structure of:

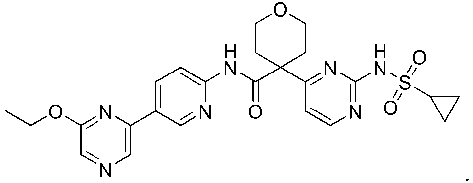

### Test Example 3: Evaluation of selectivity of the compounds of the present disclosure for inhibiting hCTPS1 versus hCTPS2 enzyme activity

The following method was used to determine the degree of inhibition on human cytidine triphosphate synthase 2 (hCTPS2) enzyme activity by the compounds of the present disclosure *in vitro.* ADP-Glo^{™} Kinase Assay kit (catalog number V9102) from Promega was used. The kit's manual may be referred for a detailed experimental procedure.

The experimental procedure was briefly described as follows: The reaction was performed in a buffer containing 50 mM 4-hydroxyethylpiperazineethanesulfonic acid (HEPES), 10 mM magnesium chloride, 5 mM potassium chloride, 0.01% Pluronic F127, and 4 mM dithiothreitol in a 384-well plate. First, 100 nL of gradient-diluted compound was transferred to a 384-well reaction plate via a nanoliter acoustic liquid transfer system (manufacturer: Labcyte; catalog number: Echo 655), and 2.5 µL of buffer containing hCTPS2 was added, followed by incubation in a 25°C incubator for 10 minutes. Then 2.5 µL of buffer containing ATP and UTP was added, followed by incubation in a 25°C incubator for 10 minutes. Finally, 5 µL of buffer containing GTP and L-glutamine (L-Gln) was added to initiate the reaction. The final reaction system was: 40 nM hCTPS2, 120 µM ATP, 160 µM UTP, 60 µM GTP, 100 µM L-Glutamine, 1% DMSO, with a total assay volume of 10 µL. The reaction was performed for 120 minutes, then 10 µL of ADP-Glo Reagent was added to terminate the reaction, followed by incubation for 1 hour. Then 20 µL of Kinase Detection Reagent was added, and after 1 hour, the chemiluminescence value was recorded on an Envision multilabel microplate detector (manufacturer: PerkinElmer; catalog number: Envision 2104). The percentage inhibition rates of the compounds at each concentration were calculated by comparing the chemiluminescence intensity ratio with a control group (0.1% DMSO). The IC₅₀ value of the compounds were obtained by nonlinear regression analysis of compound concentration logarithm-inhibition rate using GraphPad Prism 5 software.

The fold selectivity between CTPS1 and CTPS2 was calculated according to the following equation: $Fold selectivity = hCTPS 2 IC 50 / hCTPS 1 IC 50$

Certain compounds of the present disclosure were tested in the above assay, and the data are shown in Table 3.

**Table 3 Selectivity of the compounds of the present disclosure for inhibiting hCTPS1 enzyme activity**

| Example No. | hCTPS2 IC₅₀(nM) | hCTPS1 IC₅₀(nM) | Fold selectivity |
|---|---|---|---|
| 15 | 766 | 5.2 | ~150 |
| 17 | 6020 | 5.6 | ~1000 |
| 95 | >10000 | 9.2 | >1000 |

Conclusion: The compounds of the present disclosure have more than 100-fold selectivity for CTPS 1.

### Test Example 4: Inhibition on BxPC-3 cell proliferation by the compounds of the present disclosure

The following method was used to determine the effect of the compounds of the present disclosure on the proliferation of BxPC-3 cells (human primary pancreatic cancer cells). BxPC-3 cells were purchased from the Cell Resource Center of Shanghai Institutes for Biological Sciences, Chinese Academy of Sciences, and were cultured in DMEM medium containing 10% fetal bovine serum, 100 U penicillin, and 100 µg/mL streptomycin. Cell viability was determined using CellTiter-Glo^{®} Luminescent Cell Viability Assay kit (Promega, catalog number G7573).

The experimental method was performed according to the steps in the kit's manual, briefly described as follows: The test compound was first dissolved in DMSO to prepare a 10 mM stock solution, then diluted with medium to prepare test samples, with a final concentration range of the compound from 10000 nM to 1.52 nM. Cells in logarithmic growth phase were seeded into a 96-well cell culture plate at a density of 1000 cells per well, then the test compounds were added, and the cells were cultured in a 37°C CO₂ incubator for 120 hours. After culture was completed, 50 µL of CellTiter-Glo detection reagent was added to each well, the plate was shaken for 5 minutes, then allowed to stand for 10 minutes, and then the luminescence value of each well of the sample was read on a microplate reader using Luminescence mode. The percentage inhibition rates of the compounds at each concentration point were calculated by comparing with the value of a control group (0.1% DMSO). Then nonlinear regression analysis was performed on compound concentration logarithm-inhibition rate in GraphPad Prism 9 software to obtain the IC₅₀ values of the compounds for inhibiting cell proliferation, as shown in Table 4.

**Table 4 IC₅₀ data for inhibition on BxPC-3 cell proliferation by the compounds of the present disclosure**

| Example No. | IC₅₀(nM) | Example No. | IC₅₀(nM) |
|---|---|---|---|
| 20 | 5.96 | 39 | 24.08 |
| 21 | 1.54 | 95 | 24.81 |
| 26 | 16.4 | STP-B | 41.3 |
| 38 | 9.64 | | |

Conclusion: The compounds of the present disclosure have good inhibitory effect on BxPC-3 cell proliferation with an IC₅₀ <100 nM.

### Test Example 5: Inhibition of MIA PaCa-2 cell proliferation by the compounds of the present disclosure

The following method was used to determine the effect of the compounds of the present disclosure on the proliferation of MIA PaCa-2 cells (human pancreatic cancer cells). MIA PaCa-2 cells were purchased from the Cell Resource Center of Shanghai Institutes for Biological Sciences, Chinese Academy of Sciences, and were cultured in DMEM medium containing 10% fetal bovine serum, 2.5% heat-inactivated horse serum, 1% sodium pyruvate (Gibco, catalog number 11360070), 100 U penicillin, and 100 µg/mL streptomycin. Cell viability was determined using CellTiter-Glo^{®} Luminescent Cell Viability Assay kit (Promega, catalog number G7573).

The experimental method was performed according to the steps in the kit's manual, briefly described as follows: The test compound was first dissolved in DMSO to prepare a 10 mM stock solution, then diluted with medium to prepare test samples, with a final concentration range of the compound from 10000 nM to 1.52 nM. Cells in logarithmic growth phase were seeded into a 96-well cell culture plate at a density of 1000 cells per well, then the test compounds were added, and the cells were cultured in a 37°C CO₂ incubator for 120 hours. After culture was completed, 50 µL of CellTiter-Glo detection reagent was added to each well, the plate was shaken for 5 minutes, then allowed to stand for 10 minutes, and then the luminescence value of each well of the sample was read on a microplate reader using Luminescence mode. The percentage inhibition rates of the compounds at each concentration point were calculated by comparing with the value of a control group (0.1% DMSO). Then nonlinear regression analysis was performed on compound concentration logarithm-inhibition rate in GraphPad Prism 9 software to obtain the IC₅₀ values of the compounds for inhibiting cell proliferation, as shown in Table 5.

**Table 5 IC₅₀ data for inhibition of MIA PaCa-2 cell proliferation by the compounds of the present disclosure**

| Example No. | IC₅₀(nM) | Example No. | IC₅₀(nM) |
|---|---|---|---|
| 20 | 35.46 | 95 | 45.5 |
| 21 | 15.65 | STP-B | 117.2 |
| 26 | 50.83 | | |
| 38 | 39.71 | | |

Conclusion: The compounds of the present disclosure have good inhibitory effect on MIA PaCa-2 cell proliferation with an IC₅₀<100 nM.

### Test Example 6. Pharmacokinetic testing of compounds of the present disclosure in mice

### 1. Experimental Objective

ICR mice were used as test animals. Plasma concentrations of the test compounds at different time points were measured by LC/MS/MS after intragastrical administration of the reference compound STP-B and the compounds of the present disclosure, and the pharmacokinetic characteristics of the compounds of the present disclosure in mice were studied.

### 2. Experimental Protocol

### 6.1 Experimental Drugs and Animals;

Reference compound STP-B, compounds 13, 15, 17, 20, 21, 26, 38, 39, 41, 50, 57, 65, 69, 84, 95, 99, 104, 112, 113, 125, and 127.

ICR mice, male, 30.8 to 38.0 g, purchased from Vital River Laboratory Animal Technology Co., Ltd.

### 6.2 Drug Preparation

An appropriate amount of the test compound was weighed, and an appropriate amount of DMA (N,N-dimethylacetamide), 30% Solutol HS15 (30% polyethylene glycol-15 hydroxystearate), and saline were added sequentially, mixed by ultrasonication and vortexing to prepare a 1 mg/mL dosing formulation. The ratio of DMA:30% Solutol HS15:saline was 10:10:80 (v/v/v).

### 2.3 Dosing

ICR mice in each test compound injection group (9 mice per group) were fasted overnight and then administered intragastrically (PO, compound dose was 10 mg/kg, dosing volume was 10 mL/kg), and fed 4 hours after dosing.

### 3. Procedure

About 0.1 mL of blood was collected from the orbit before dosing and at 0.25 h, 0.5 h, 1 h, 2 h, 4 h, 8 h, 10 h, and 24 h after dosing, and whole blood samples were placed in anticoagulant tubes containing EDTA-K2. After blood sample collection, samples were placed on ice and centrifuged to separate plasma (centrifugation conditions: 1500 g, 10 minutes). The collected plasma was stored at -40 to -20°C before analysis.

The content of test compounds in mouse plasma after intragastrical administration was determined by LC-MS/MS.

### 4. Pharmacokinetic Parameter Results

The pharmacokinetic parameters of the test compounds are shown in Table 6 below.

**Table 6. Pharmacokinetic parameters of test compounds in mice**

| Example No. | Pharmacokinetic study | | | |
|---|---|---|---|---|
| | Administration route (Dosage) | Plasma concentration Cmax (ng/mL) | AUC AUC₀₋₁(ng·h/mL) | Half-life T_{1/2} (h) |
| STP-B | Oral (10 mg/kg) | 34375 | 37484 | 3.05 |
| 13 | Oral (10 mg/kg) | 13438 | 72611 | 4.52 |
| 15 | Oral (10 mg/kg) | 20300 | 59700 | 2.27 |
| 17 | Oral (10 mg/kg) | 27571 | 125552 | 1.87 |
| 20 | Oral (10 mg/kg) | 28222 | 106895 | 1.06 |
| 21 | Oral (10 mg/kg) | 26392 | 188312 | 2.29 |
| 26 | Oral (10 mg/kg) | 80937 | 651817 | 4.87 |
| 38 | Oral (10 mg/kg) | 34464 | 138092 | 3.32 |
| 39 | Oral (10 mg/kg) | 38390 | 369691 | 6.3 |
| 41 | Oral (10 mg/kg) | 36900 | 142000 | 2.86 |
| 50 | Oral (10 mg/kg) | 36550 | 329711 | 5.32 |
| 57 | Oral (10 mg/kg) | 21738 | 151721 | 5.73 |
| 65 | Oral (10 mg/kg) | 28500 | 111000 | 2.8 |
| 69 | Oral (10 mg/kg) | 14700 | 124000 | 2.15 |
| 84 | Oral (10 mg/kg) | 28700 | 106000 | 2.8 |
| 95 | Oral (10 mg/kg) | 23800 | 118000 | 7.41 |
| 99 | Oral (10 mg/kg) | 15294 | 66475 | 2.06 |
| 104 | Oral (10 mg/kg) | 38635 | 113217 | 2.35 |
| 112 | Oral (10 mg/kg) | 25900 | 92300 | 2.69 |
| 113 | Oral (10 mg/kg) | 15600 | 64400 | 2.6 |
| 125 | Oral (10 mg/kg) | 45501 | 180249 | 2.41 |
| 127 | Oral (10 mg/kg) | 45398 | 59792 | 2.24 |

Conclusion: Compared with the reference compound STP-B, each compound of the present disclosure has a larger area under the curve and significantly improved pharmacokinetic properties.

## Claims

1. A compound of formula (AAI): 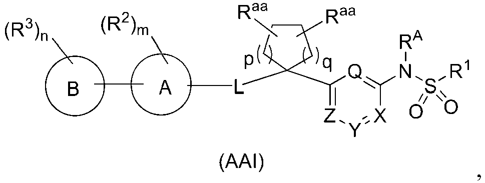 or a stereoisomer, a tautomer, or a pharmaceutically acceptable salt thereof;
wherein:
any two R^{aa}, together with the atoms to which they are attached, form a ring C, wherein ring C is optionally further substituted by one or more R⁴;
ring C is selected from the group consisting of a 6-membered aryl, a 3- to 5- membered cycloalkyl, a 5- to 7- membered heterocyclyl, and a 5- to 6- membered heteroaryl;
R^{A} is selected from the group consisting of a hydrogen atom and a C₁₋₆ alkyl;
ring A is selected from the group consisting of a 6-membered aryl, a 5- to 6- membered heteroaryl, and a 5- to 7- membered heterocyclyl;
ring B is selected from the group consisting of a 6-membered aryl and a 5- to 10- membered heteroaryl;
L is selected from the group consisting of -C(=O)-, -C(=O)NR^{a}-, and -NR^{b}C(=O)-;
R^{a} and R^{b} are each independently selected from the group consisting of a hydrogen atom and a C₁₋₆ alkyl;
X, Y, Z, and Q are each independently selected from the group consisting of N and CR^{c}; and at most two of X, Y, Z, and Q are simultaneously N atoms;
alternatively, X and Y, Z and Y each independently form a 5- to 6- membered heteroaryl or a 5- to 6- membered heterocyclyl; wherein the 5- to 6- membered heteroaryl or 5- to 6- membered heterocyclyl is optionally further substituted by one or more substituents selected from the group consisting of halogen, hydroxy, cyano, and 3- to 5- membered cycloalkyl;
R^{c} is selected from the group consisting of a hydrogen atom, halogen, hydroxy, cyano, C₁₋₆ alkyl, and C₁₋₆ alkoxy; wherein the C₁₋₆ alkyl or C₁₋₆ alkoxy is optionally further substituted by one or more substituents selected from the group consisting of halogen, hydroxy, cyano, and 3- to 5- membered cycloalkyl;
R¹ is selected from the group consisting of C₁₋₆ alkyl and 3- to 5- membered cycloalkyl; wherein the C₁₋₆ alkyl or 3- to 5- membered cycloalkyl is optionally further substituted by one or more substituents selected from the group consisting of halogen, hydroxy, cyano, 3- to 5-membered cycloalkyl, C₁₋₆ alkyl, C₁₋₆ haloalkyl, C₁₋₆ alkoxy, and C₁₋₆ haloalkoxy;
R² is each independently selected from the group consisting of a hydrogen atom, halogen, cyano, hydroxy, C₁₋₆ alkyl, and C₁₋₆ alkoxy; wherein the C₁₋₆ alkyl or C₁₋₆ alkoxy is optionally further substituted by one or more substituents selected from the group consisting of halogen, hydroxy, cyano, C₁₋₆ alkyl, and C₁₋₆ alkoxy;
alternatively, two R², together with the same carbon atom to which they are attached, form a -C(O);
R³ is each independently selected from the group consisting of a hydrogen atom, halogen, cyano, hydroxy, C₁₋₆ alkyl, C₁₋₆ alkoxy, 3- to 5- membered cycloalkyl, C₂₋₆ alkenyl, SF₅, -C(O)R⁵, -C(O)OR⁵, -NHC(O)R⁵, -NHC(O)OR⁵, -NR⁶R⁷, -C(O)NR⁶R⁷, -CH₂NHC(O)OR⁵, -CH₂NR⁶R⁷, and -S(O)ᵣR⁵; wherein the alkyl, alkoxy, alkenyl, or cycloalkyl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
R⁴ is each independently selected from the group consisting of a hydrogen atom, cyano, halogen, alkyl, alkenyl, alkynyl, hydroxy, cycloalkyl, heterocyclyl, aryl, heteroaryl, -OR⁵, -C(O)R⁵, -C(O)OR⁵, -NHC(O)R⁵, -NHC(O)OR⁵, -NR⁶R⁷, -C(O)NR⁶R⁷, -S(O)₂NR⁶R⁷, -CH₂NHC(O)OR⁵, -CH₂NR⁶R⁷, and -S(O)ᵣR⁵; wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
R⁵ is each independently selected from the group consisting of a hydrogen atom, alkyl, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, cyano, alkyl, alkoxy, haloalkyl, haloalkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
R⁶ and R⁷ are each independently selected from the group consisting of a hydrogen atom, hydroxy, alkyl, alkoxy, cycloalkyl, heterocyclyl, aryl, and heteroaryl, wherein the alkyl, alkoxy, cycloalkyl, heterocyclyl, aryl, or heteroaryl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
alternatively, R⁶ and R⁷, together with the atoms to which they are attached, form a 4- to 8-membered heterocyclyl, wherein the 4- to 8- membered heterocyclyl contains one or more N, O, or S(O)r, and the 4- to 8- membered heterocyclyl is optionally further substituted by one or more substituents selected from the group consisting of hydroxy, halogen, nitro, cyano, alkyl, alkoxy, cycloalkyl, heterocyclyl, aryl, heteroaryl, =O, -C(O)R⁸, -C(O)OR⁸, -OC(O)R⁸, -NR⁹R¹⁰, -C(O)NR⁹R¹⁰, -SO₂NR⁹R¹⁰, and -NR⁹C(O)R¹⁰;
R⁸, R⁹, and R¹⁰ are each independently selected from the group consisting of a hydrogen atom, alkyl, amino, cycloalkyl, heterocyclyl, aryl, and heteroaryl, wherein the alkyl, cycloalkyl, heterocyclyl, aryl, or heteroaryl is optionally further substituted by one or more substituents selected from the group consisting of hydroxy, halogen, nitro, amino, cyano, alkyl, alkoxy, cycloalkyl, heterocyclyl, aryl, heteroaryl, carboxy, and carboxylate;
m is selected from the group consisting of 0, 1, and 2;
n is selected from the group consisting of 0, 1, and 2;
p is selected from the group consisting of 0, 1, and 2;
q is selected from the group consisting of 0, 1, and 2; and
r is each independently 0, 1, or 2.

2. The compound or a stereoisomer, a tautomer, or a pharmaceutically acceptable salt thereof according to claim 1, wherein 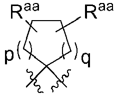 is selected from the group consisting of 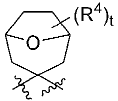 and 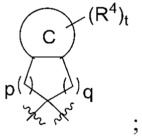 wherein t is selected from the group consisting of 0, 1, and 2; and ring C, R⁴, p, and q are as defined in claim 1.

3. The compound or a stereoisomer, a tautomer, or a pharmaceutically acceptable salt thereof according to claim 1 or 2, which is a compound of formula (AI): 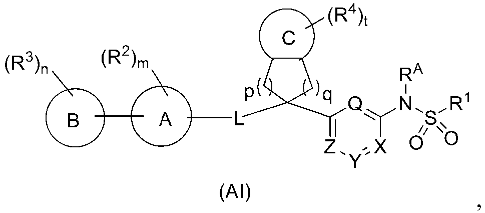 or a stereoisomer, a tautomer, or a pharmaceutically acceptable salt thereof;
wherein:
X, Y, Z, and Q are each independently selected from the group consisting of N and CR^{c}; and at most two of X, Y, Z, and Q are simultaneously N atoms;
alternatively, Z and Y form a 5- to 6- membered heteroaryl, wherein the 5- to 6- membered heteroaryl is optionally further substituted by one or more substituents selected from the group consisting of halogen, hydroxy, cyano, and 3- to 5- membered cycloalkyl;
alternatively, X and Y form a 5- to 6- membered heteroaryl, wherein the 5- to 6- membered heteroaryl is optionally further substituted by one or more substituents selected from the group consisting of halogen, hydroxy, cyano, and 3- to 5- membered cycloalkyl;
t is selected from the group consisting of 0, 1, and 2; and
ring A, ring B, ring C, L, R¹ to R⁴, R^{A}, p, q, m, and n are as defined in claim 1.

4. The compound or a stereoisomer, a tautomer, or a pharmaceutically acceptable salt thereof according to claim 3, which is a compound of formula (I): 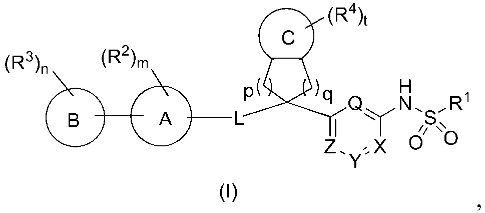
or a stereoisomer, a tautomer, or a pharmaceutically acceptable salt thereof;
wherein: ring A, ring B, ring C, L, R¹ to R⁴, X, Y, Z, Q, p, q, m, n, and t are as defined in claim 3.

5. The compound or a stereoisomer, a tautomer, or a pharmaceutically acceptable salt thereof according to any one of claims 1 to 4, wherein ring A is selected from the group consisting of: 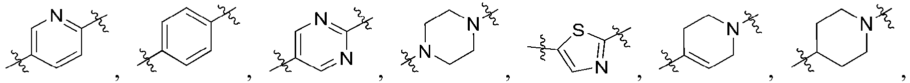 and

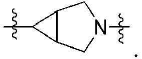

6. The compound or a stereoisomer, a tautomer, or a pharmaceutically acceptable salt thereof according to any one of claims 1 to 5, wherein ring B is selected from the group consisting of:

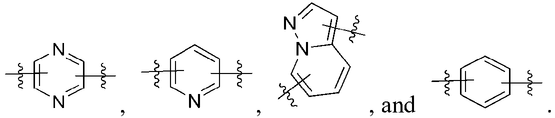

7. The compound or a stereoisomer, a tautomer, or a pharmaceutically acceptable salt thereof according to any one of claims 1 to 6, wherein: L is selected from the group consisting of -C(=O)-, -C(=O)NH-, and -NHC(=O)-.

8. The compound or a stereoisomer, a tautomer, or a pharmaceutically acceptable salt thereof according to any one of claims 1 to 7, which is a compound of formula (IIA) or (IIB): 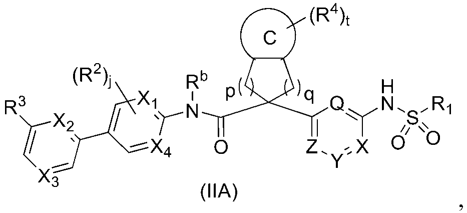 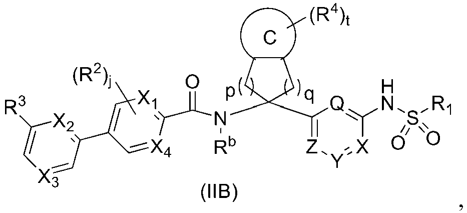
or a stereoisomer, a tautomer, or a pharmaceutically acceptable salt thereof;
wherein each X₁ is independently selected from the group consisting of N and CR^{d};
X₄ is each independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy; and
ring C, X, Y, Z, Q, R¹ to R⁴, p, q, and t are as defined in claim 3.

9. The compound or a stereoisomer, a tautomer, or a pharmaceutically acceptable salt thereof according to any one of claims 1 to 8, which is a compound of formula (II) or (III): 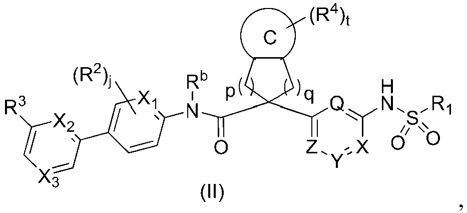 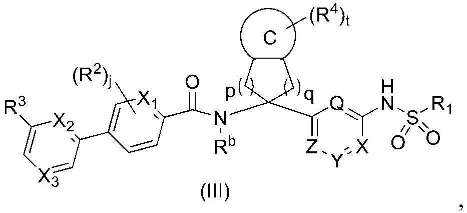
or a stereoisomer, a tautomer, or a pharmaceutically acceptable salt thereof;
wherein each X₁ is independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy; and
ring C, X, Y, Z, Q, R¹ to R⁴, p, q, and t are as defined in claim 3.

10. The compound or a stereoisomer, a tautomer, or a pharmaceutically acceptable salt thereof according to claim 1 or 2, which is a compound of formula (IV-1) or (IV-2): 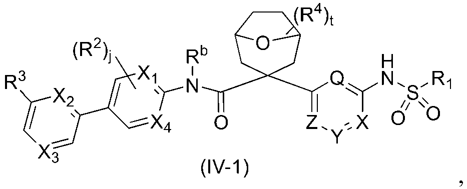 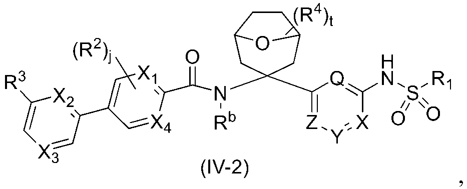
or a stereoisomer, a tautomer, or a pharmaceutically acceptable salt thereof;
wherein each X₁ is independently selected from the group consisting of N and CR^{d};
X₄ is each independently selected from the group consisting of N and CR^{d};
R^{b} is each independently selected from the group consisting of a hydrogen atom and methyl;
R^{d} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
t is selected from the group consisting of 0, 1, and 2; and
X, Y, Z, Q, and R¹ to R⁴ are as defined in claim 1.

11. The compound or a stereoisomer, a tautomer, or a pharmaceutically acceptable salt thereof according to any one of claims 1 to 4, which is a compound of formula (V-A), (V-B), (V-C), or (V-D): 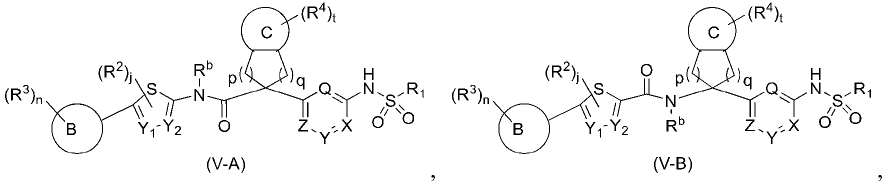 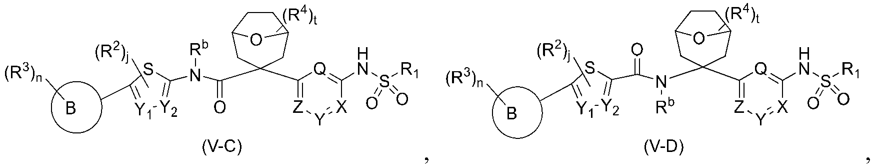
or a stereoisomer, a tautomer, or a pharmaceutically acceptable salt thereof;
wherein each R^{b} is independently selected from the group consisting of a hydrogen atom and methyl;
ring B is each independently selected from the group consisting of 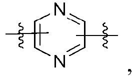 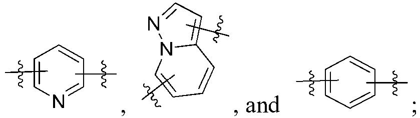
Y₁ and Y₂ are each independently selected from the group consisting of N and CH;
j is each independently selected from the group consisting of 0 and 1;
t is each independently selected from the group consisting of 0, 1, and 2; and
ring C, X, Y, Z, Q, R¹ to R⁴, p, q, and n are as defined in claim 1.

12. The compound or a stereoisomer, a tautomer, or a pharmaceutically acceptable salt thereof according to claim 11, which is a compound of formula (V-1) or (V-2): 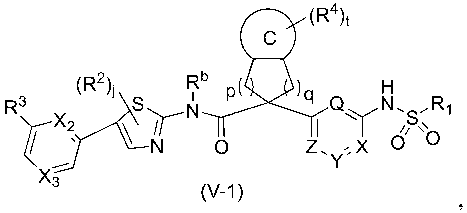 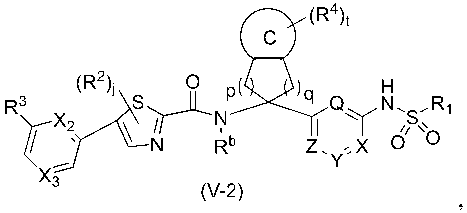
or a stereoisomer, a tautomer, or a pharmaceutically acceptable salt thereof
wherein each R^{b} is independently selected from the group consisting of a hydrogen atom and methyl;
X₂ and X₃ are each independently selected from the group consisting of N and CR^{e};
j is each independently selected from the group consisting of 0 and 1;
R^{e} is each independently selected from the group consisting of a hydrogen atom, halogen, cyano, C₁₋₆ alkyl, C₁₋₆ alkoxy, C₁₋₆ haloalkyl, and C₁₋₆ haloalkoxy;
t is selected from the group consisting of 0, 1, and 2; and
ring C, X, Y, Z, Q, R¹ to R⁴, p, and q are as defined in claim 1.

13. The compound or a stereoisomer, a tautomer, or a pharmaceutically acceptable salt thereof according to any one of claims 1 to 12, wherein 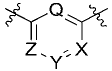 is selected from the group consisting of: 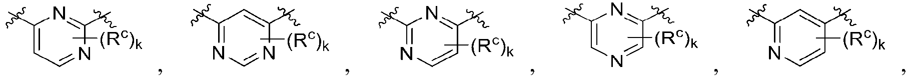 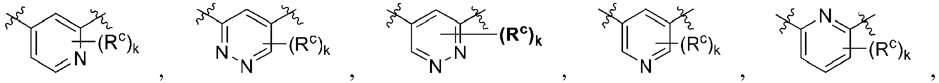 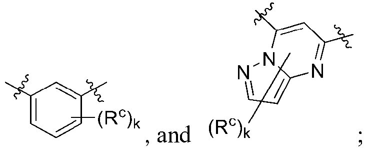
R^{c} is each independently selected from the group consisting of a hydrogen atom, halogen, cyano, C₁₋₆ alkoxy, C₁₋₆ haloalkyl, and C₁₋₆ haloalkoxy; and
k is each independently selected from the group consisting of 0, 1, and 2.

14. The compound or a stereoisomer, a tautomer, or a pharmaceutically acceptable salt thereof according to any one of claims 1 to 13, wherein ring C is selected from the group consisting of:

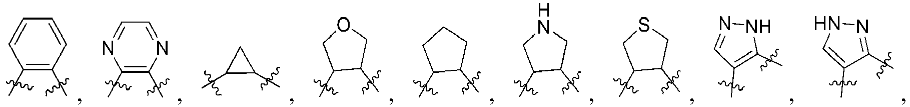

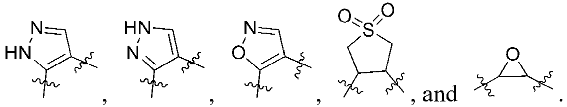

15. The compound or a stereoisomer, a tautomer, or a pharmaceutically acceptable salt thereof according to any one of claims 1 to 14, wherein R⁴ is selected from the group consisting of a hydrogen atom, C₁₋₆ alkyl, -C(O)R⁵, -C(O)NR⁶R⁷, -S(O)₂R⁵, and -S(O)₂NR⁶R⁷; wherein the C₁₋₆ alkyl is optionally further substituted by one or more substituents selected from the group consisting of hydroxy, halogen, cyano, and -C(O)OR⁸;
R⁵ is each independently selected from the group consisting of C₁₋₆ alkyl, 3- to 5- membered cycloalkyl, and 5- to 6- membered heteroaryl, wherein the C₁₋₆ alkyl, 3- to 5- membered cycloalkyl, or 5- to 6- membered heteroaryl is optionally further substituted by one or more substituents selected from the group consisting of halogen and C₁₋₆ alkoxy;
R⁶ and R⁷ are each independently selected from the group consisting of a hydrogen atom and C₁₋₆ alkyl; and
R⁸ is selected from the group consisting of a hydrogen atom and C₁₋₆ alkyl.

16. The compound or a stereoisomer, a tautomer, or a pharmaceutically acceptable salt thereof according to claim 14 or 15, wherein 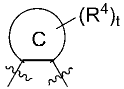 is selected from the group consisting of: 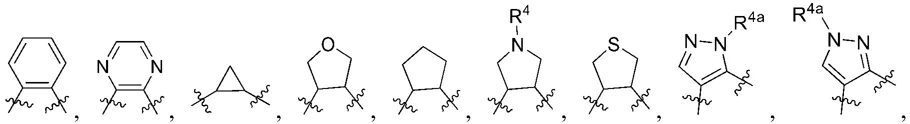 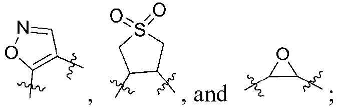
wherein: R^{4a} is each independently selected from the group consisting of a hydrogen atom and C₁₋₆ alkyl; and
R⁴ is as defined in claim 14.

17. The compound or a stereoisomer, a tautomer, or a pharmaceutically acceptable salt thereof according to any one of claims 1 to 16, wherein R¹ is selected from the group consisting of C₁₋₆ alkyl and 3- to 5- membered cycloalkyl, wherein the C₁₋₆ alkyl or 3- to 5- membered cycloalkyl is optionally further substituted by one or more substituents selected from the group consisting of C₁₋₃ alkyl, C₁₋₃ alkoxy, halogen, hydroxy, and cyano.

18. The compound or a stereoisomer, a tautomer, or a pharmaceutically acceptable salt thereof according to claim 17, wherein R¹ is selected from the group consisting of cyclopropyl, methyl, ethyl, isopropyl, difluoromethyl, trifluoromethyl, 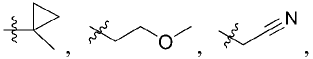 and

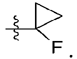

19. The compound or a stereoisomer, a tautomer, or a pharmaceutically acceptable salt thereof according to any one of claims 1 to 18, wherein R² is selected from the group consisting of a hydrogen atom, halogen, cyano, C₁₋₆ alkyl, C₁₋₆ alkoxy, C₁₋₆ haloalkyl, and C₁₋₆ haloalkoxy;
alternatively, two R², together with the same carbon atom to which they are attached, form a -C(O).

20. The compound or a stereoisomer, a tautomer, or a pharmaceutically acceptable salt thereof according to any one of claims 1 to 19, wherein R³ is selected from the group consisting of a hydrogen atom, halogen, cyano, hydroxy, C₁₋₆ alkyl, C₁₋₆ alkoxy, 3 to 5- membered cycloalkyl, SF₅, C₁₋₆ haloalkyl, and C₁₋₆ haloalkoxy.

21. The compound or a stereoisomer, a tautomer, or a pharmaceutically acceptable salt thereof according to claim 20, wherein R³ is selected from the group consisting of chloro, trifluoromethoxy, methyl, ethyl, and ethoxy.

22. The compound or a stereoisomer, a tautomer, or a pharmaceutically acceptable salt thereof according to any one of claims 1 to 21, wherein p is 0 and q is 0.

23. The compound or a stereoisomer, a tautomer, or a pharmaceutically acceptable salt thereof according to any one of claims 1 to 21, wherein p is 1 and q is 1 or 2.

24. The compound or a stereoisomer, a tautomer, or a pharmaceutically acceptable salt thereof according to any one of claims 1 to 21, wherein p is 2 and q is 0, 1, or 2.

25. The compound or a stereoisomer, a tautomer, or a pharmaceutically acceptable salt thereof according to any one of claims 1 to 24, wherein the compound is:

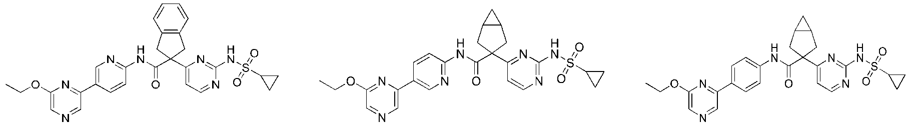

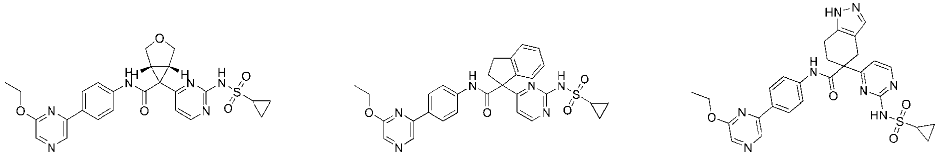

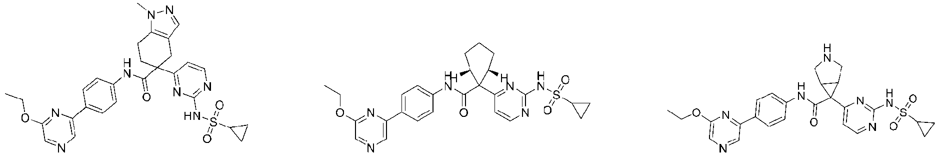

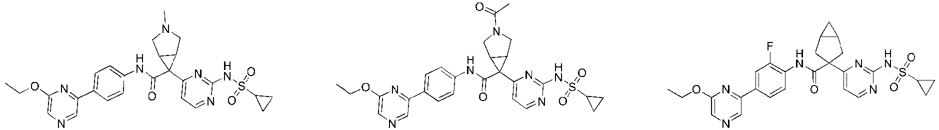

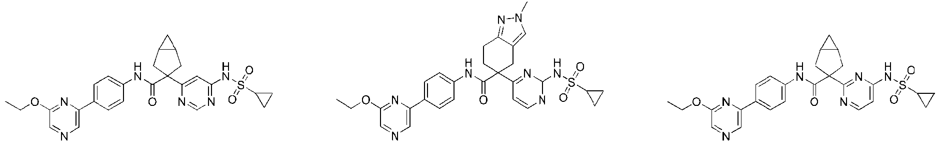

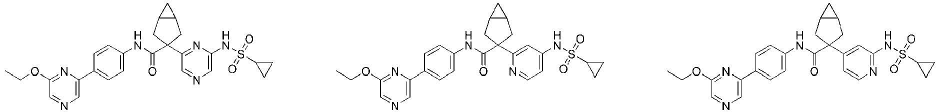

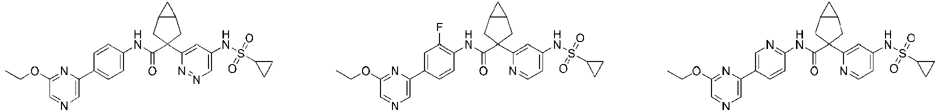

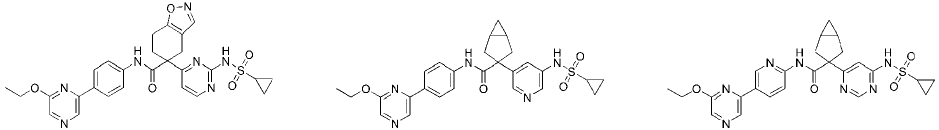

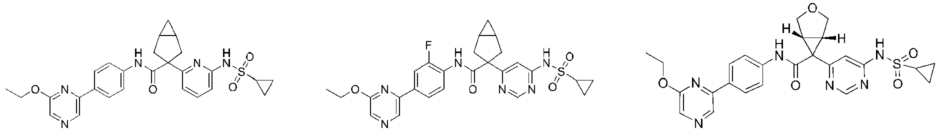

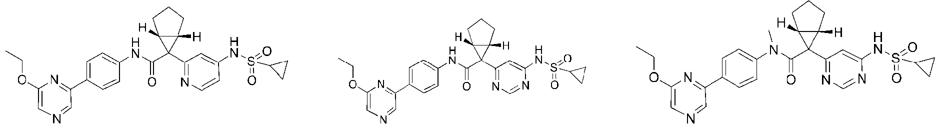

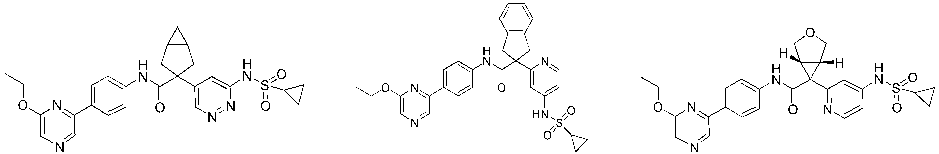

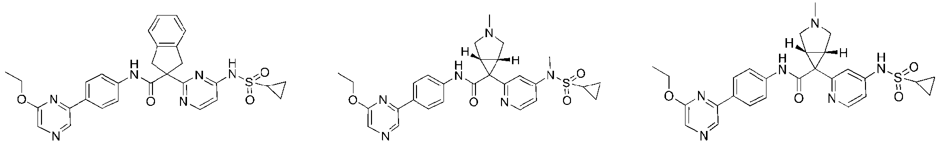

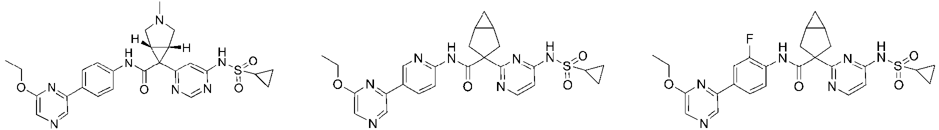

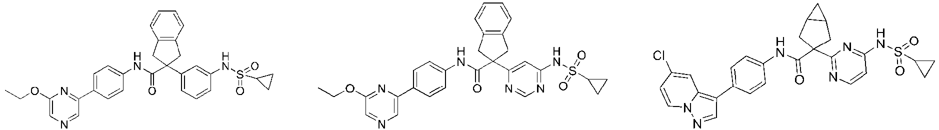

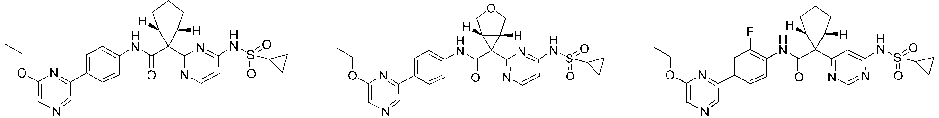

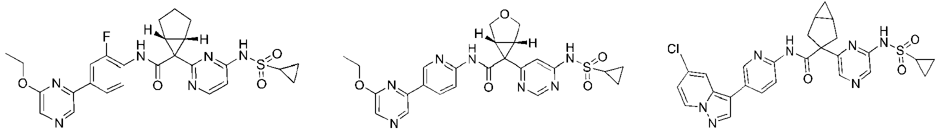

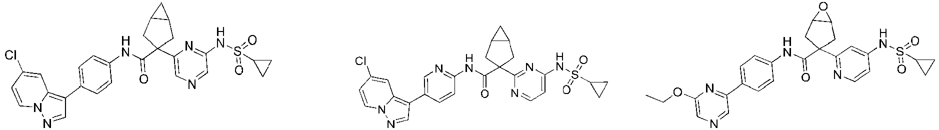

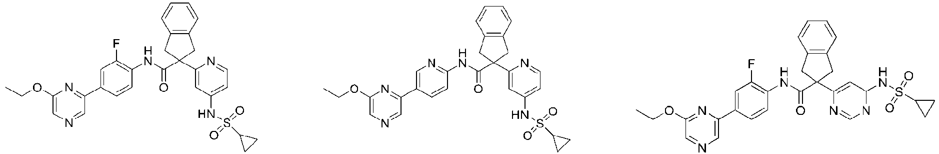

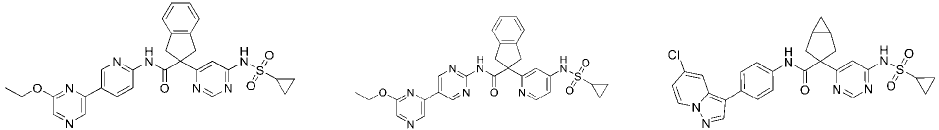

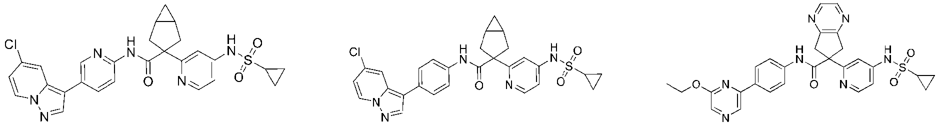

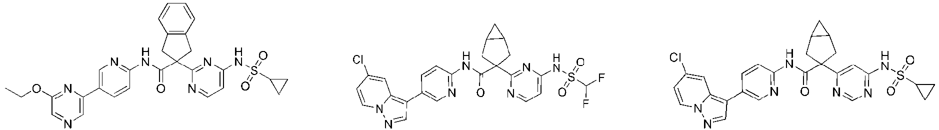

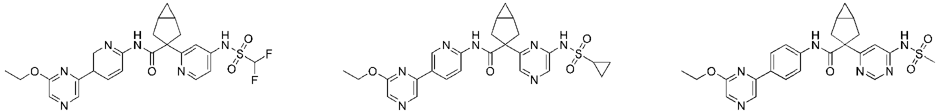

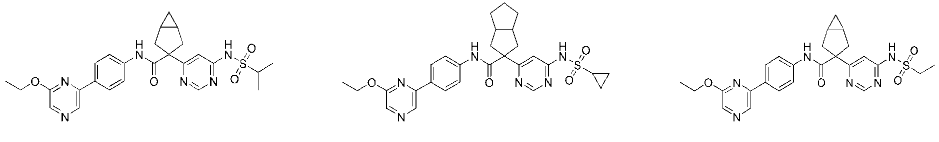

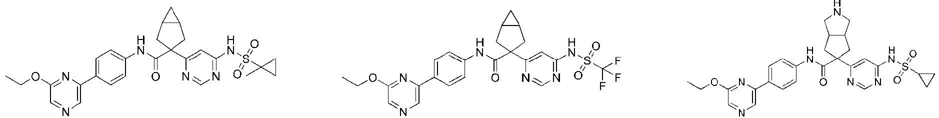

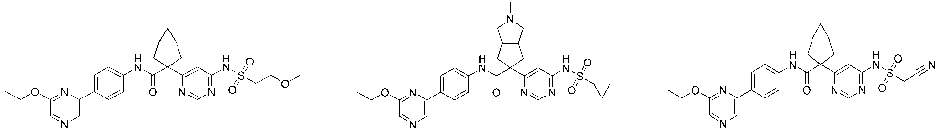

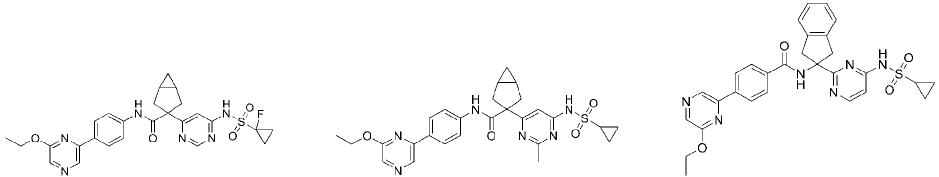

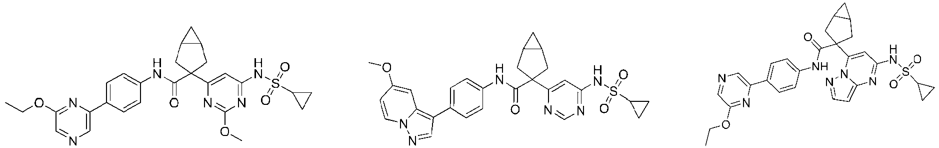

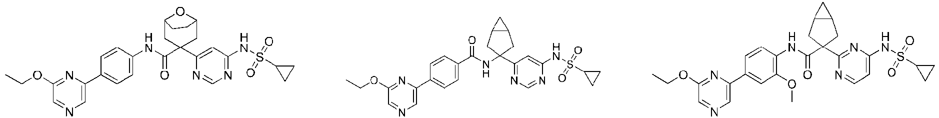

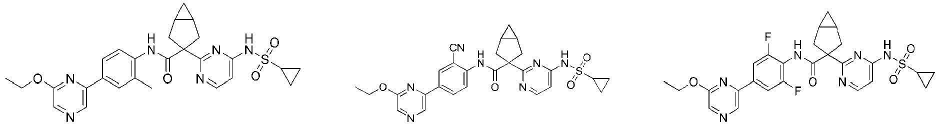

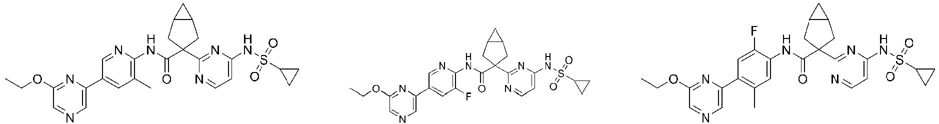

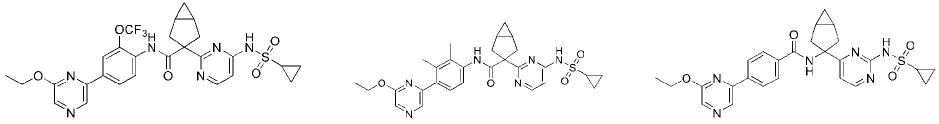

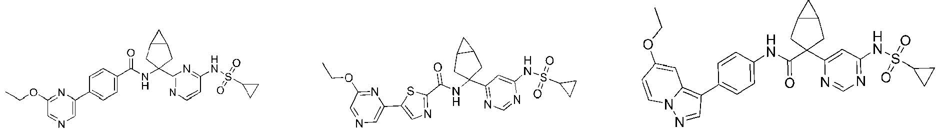

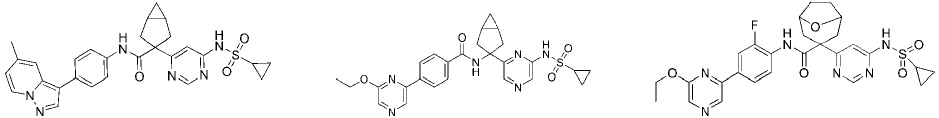

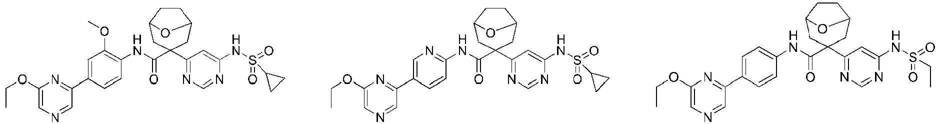

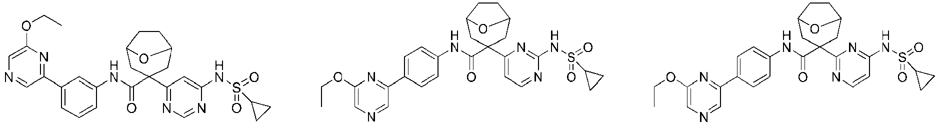

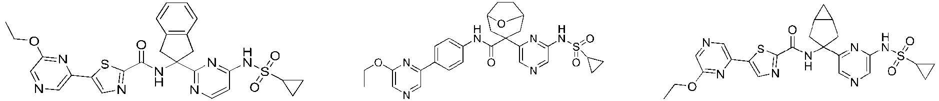

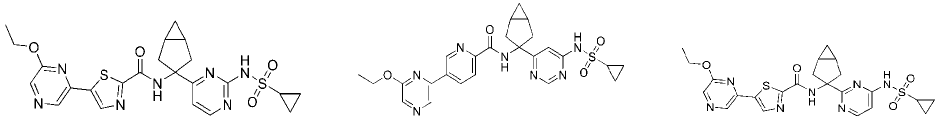

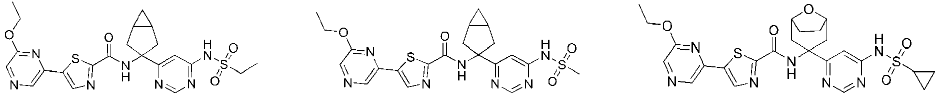

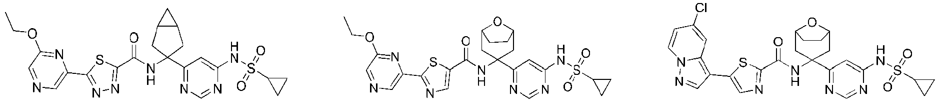

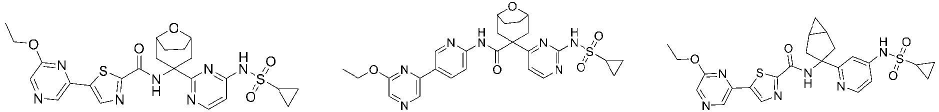

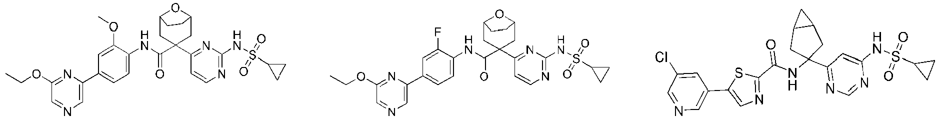

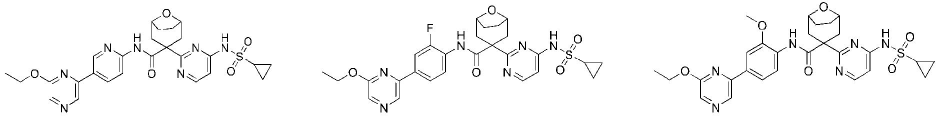

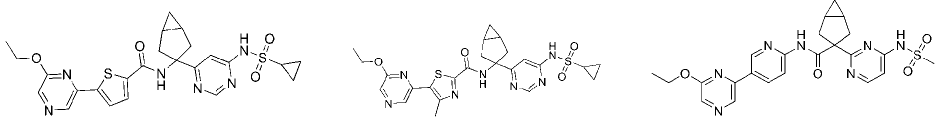

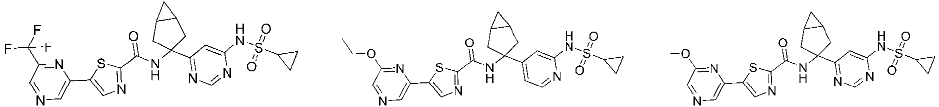

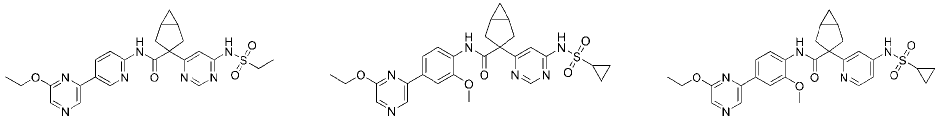

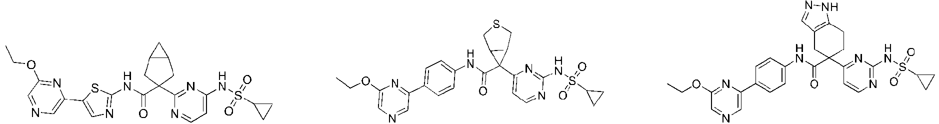

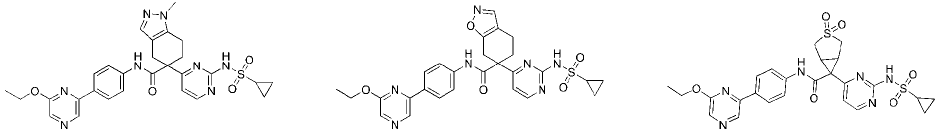

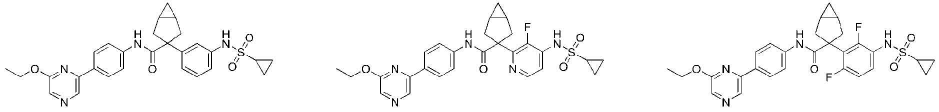

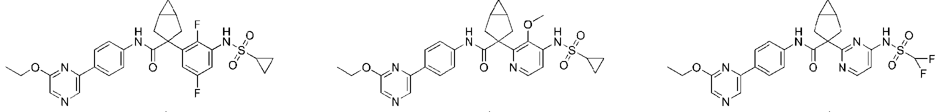

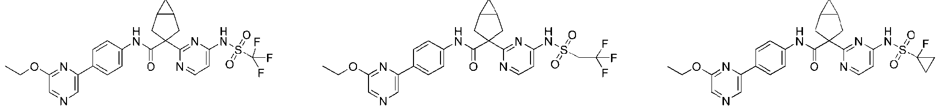

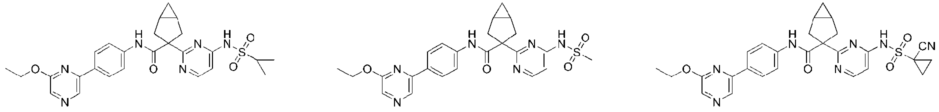

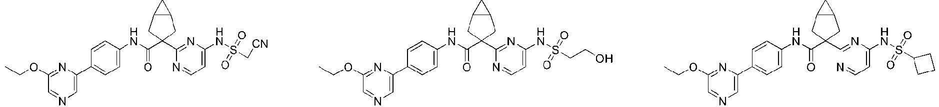

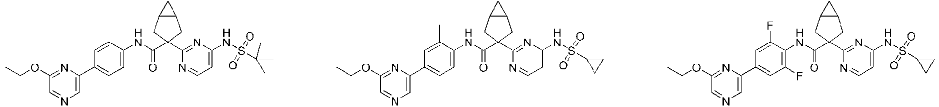

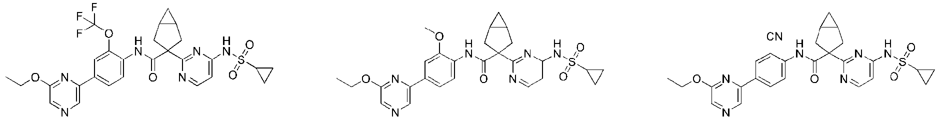

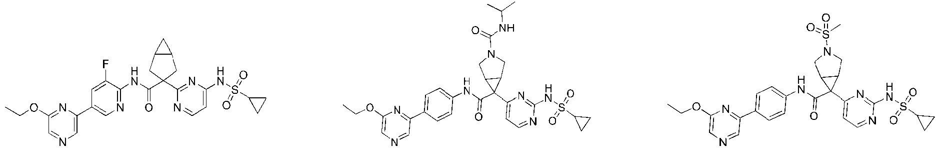

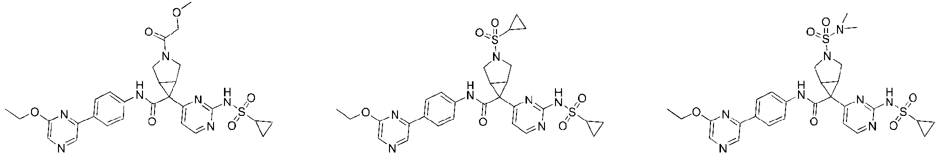

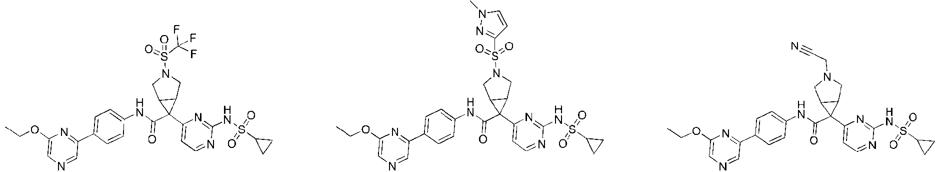

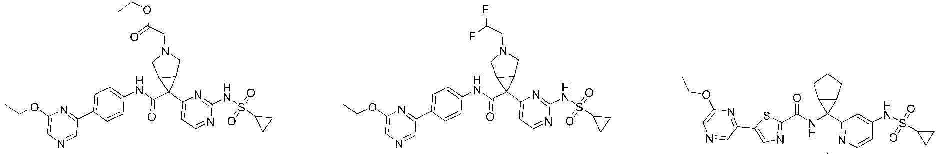

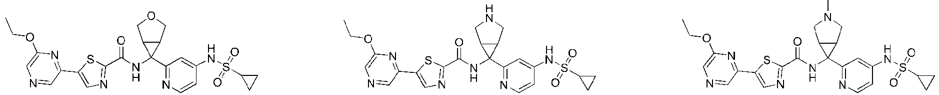

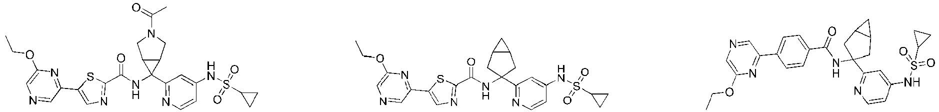

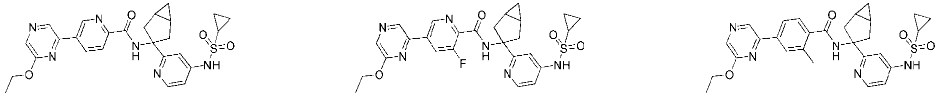

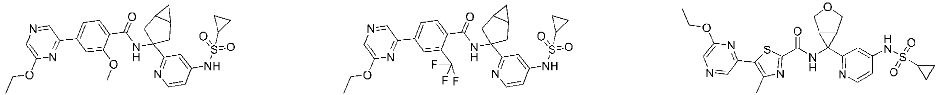

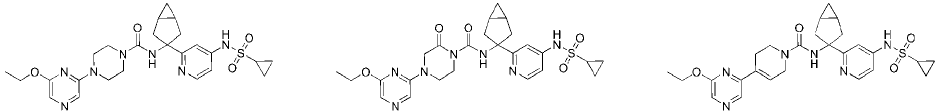

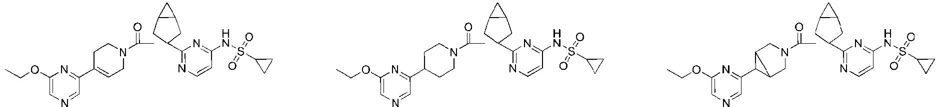

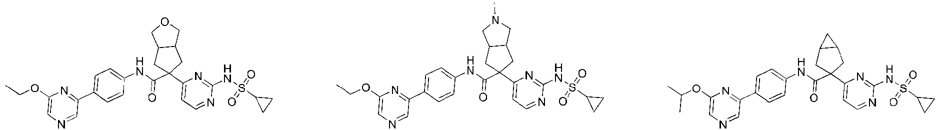

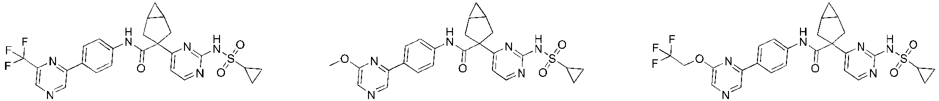

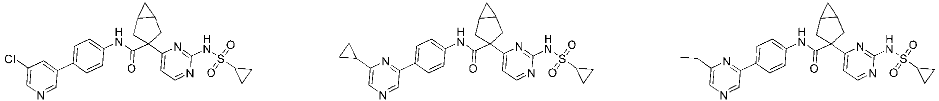

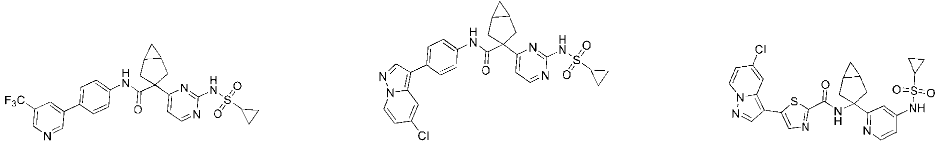

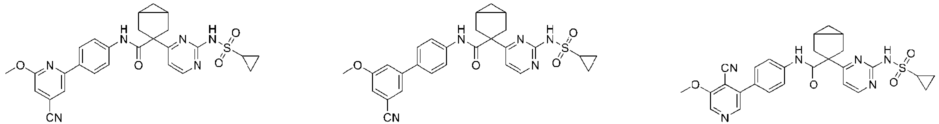

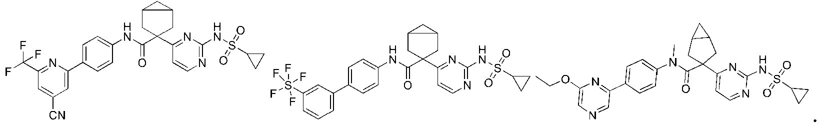

26. A pharmaceutical composition comprising an effective dose of the compound or a stereoisomer, a tautomer, or a pharmaceutically acceptable salt thereof according to any one of claims 1 to 25, and a pharmaceutically acceptable carrier.

27. Use of the compound or a stereoisomer, a tautomer, or a pharmaceutically acceptable salt thereof according to any one of claims 1 to 25, or the pharmaceutical composition according to claim 26, in the manufacture of a CTPS1 inhibitor.

28. Use of the compound or a stereoisomer, a tautomer, or a pharmaceutically acceptable salt thereof according to any one of claims 1 to 25, or the pharmaceutical composition according to claim 26, in the manufacture of a medicament for treating a disease mediated by CTPS1 ; preferably, the disease mediated by CTPS1 is lymphoma or solid tumor.

29. The use according to claim 28, wherein the lymphoma is relapsed/refractory B-cell and T-cell lymphoma.

30. The use according to claim 29, wherein the relapsed/refractory B-cell and T-cell lymphoma is mantle cell lymphoma, diffuse large B-cell lymphoma, peripheral T-cell lymphoma, cutaneous T-cell lymphoma, or indolent B-cell lymphoma.

31. Use of the compound or a stereoisomer, a tautomer, or a pharmaceutically acceptable salt thereof according to any one of claims 1 to 25, or the pharmaceutical composition according to claim 26, in the manufacture of a medicament for treating lymphoma or solid tumor.

32. The use according to claim 31, wherein the lymphoma is relapsed/refractory B-cell and T-cell lymphoma.

33. The use according to claim 32, wherein the relapsed/refractory B-cell and T-cell lymphoma is mantle cell lymphoma, diffuse large B-cell lymphoma, peripheral T-cell lymphoma, cutaneous T-cell lymphoma, or indolent B-cell lymphoma.

34. The use according to claim 28, wherein the solid tumor is preferably pancreatic cancer.
